(19) Europäisches Patentamt
European Patent Office
Office européen des brevets

(11) EP 3 649 119 B1

(12) **EUROPEAN PATENT SPECIFICATION**

(45) Date of publication and mention of the grant of the patent:
**03.11.2021 Bulletin 2021/44**

(21) Application number: **18732777.0**

(22) Date of filing: **21.06.2018**

(51) Int Cl.:
***C07D 401/14*** *(2006.01)* ***C07D 405/14*** *(2006.01)*
***C07D 403/04*** *(2006.01)* ***C07D 403/14*** *(2006.01)*
***C07D 409/14*** *(2006.01)* ***C07D 471/04*** *(2006.01)*
***A61K 31/502*** *(2006.01)* ***A61P 29/00*** *(2006.01)*

(86) International application number:
**PCT/EP2018/066548**

(87) International publication number:
**WO 2019/007696 (10.01.2019 Gazette 2019/02)**

(54) **NOVEL COMPOUNDS AND PHARMACEUTICAL COMPOSITIONS THEREOF FOR THE TREATMENT OF FIBROSIS**

NEUARTIGE VERBINDUNGEN UND PHARMAZEUTISCHE ZUSAMMENSETZUNGEN DAVON ZUR BEHANDLUNG VON FIBROSE

NOUVEAUX COMPOSÉS ET COMPOSITIONS PHARMACEUTIQUES DE CEUX-CI DESTINÉS AU TRAITEMENT DE LA FIBROSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.07.2017 GB 201710851**

(43) Date of publication of application:
**13.05.2020 Bulletin 2020/20**

(73) Proprietor: **Galapagos NV**
**2800 Mechelen (BE)**

(72) Inventors:
- **MAMMOLITI, Oscar**
**2800 Mechelen (BE)**
- **JANSEN, Koen, Karel**
**2300 Turnhout (BE)**
- **MENET, Christel, Jeanne, Marie**
**1050 Brussels (BE)**
- **PALISSE, Adeline, Marie, Elise**
**2800 Mechelen (BE)**
- **TRICARICO, Giovanni, Alessandro**
**2800 Mechelen (BE)**
- **EL BKASSINY, Sandy**
**2800 Mechelen (BE)**
- **JAUNET, Alexis, Patrick, Claude**
**2800 Mechelen (BE)**
- **ALLART, Brigitte**
**2800 Mechelen (BE)**
- **DUTHION, Béranger**
**93230 Romainville (FR)**
- **BREBION, Franck Laurent**
**93230 Romainville (FR)**

(74) Representative: **Bar, Grégory**
**Galapagos NV**
**IP Department**
**Generaal De Wittelaan, L11 A3**
**2800 Mechelen (BE)**

(56) References cited:
**US-A1- 2009 325 970**
**US-A1- 2011 201 607**
**US-A1- 2014 187 550**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 649 119 B1

## Description

### FIELD OF THE INVENTION

The present invention relates to compounds useful in the prophylaxis and/or treatment of fibrotic diseases, inflammatory diseases, respiratory diseases, autoimmune diseases, metabolic diseases, cardiovascular diseases, and/or proliferative diseases. In particular, the compounds of the invention may be sphingosine 1-phosphate (S1P) receptor antagonists, a family of sphingosine receptors that are involved in fibrotic diseases, inflammatory diseases, respiratory diseases, autoimmune diseases, metabolic diseases, cardiovascular diseases, and/or proliferative diseases. The present invention also provides methods for the production of the compounds of the invention, pharmaceutical compositions comprising the compounds of the invention, and methods for the prophylaxis and/or treatment of fibrotic diseases, inflammatory diseases, respiratory diseases, autoimmune diseases, metabolic diseases, cardiovascular diseases, and/or proliferative diseases by administering the compounds of the invention.

### BACKGROUND OF THE INVENTION

Sphingolipids are structural components of all eukaryotic cell membranes. In the plasma membrane, they are commonly believed to protect the cell surface by forming the mechanically stable and chemically resistant outer leaflet of the lipid bilayer. All sphingolipids contain a sphingoid long-chain base (sphingosine) backbone, linked to a fatty acid molecule through an amide bond. Sphingosine-1-phosphate (SIP) is produced from sphingosine (2-amino-4-octadecene-1,3-diol; an aliphatic 18-carbon amino alcohol with an unsaturated hydrocarbon chain), by sphingosine kinases (Takabe et al., 2008).

SIP is a potent bioactive sphingolipid involved in cell proliferation, angiogenesis, inflammation and malignant transformation among other functions. SIP binds with low nano-molar affinity to five related G protein-coupled receptors, named S1P receptors (S1PR1, S1PR2, S1PR3, S1PR4, and S1PR5) (Adada et al., 2013; Milstien and Spiegel, 2006).

The S1PR1, S1PR2, and S1PR3 subtypes are widely expressed within the human body, whereas S1PR4 and S1PR5 show much more restricted tissue expression (Sobel et al., 2013).

However, whereas S1PR1, S1PR2, S1PR3, S1PR4, and S1PR5 are all involved in human physiology, S1PR2 appears to be particularly critical in the immune, nervous, metabolic, cardiovascular, musculoskeletal, and renal systems (Adada et al., 2013; Kitada et al., 2016).

Moreover, the S1PR1 and S1PR2 exerts opposed cellular functions, and undesitrable side effects associated to S1PR1 antagonism have been observed, ranging from immunosuppression, lymphopenia, elevation of blood pressure, to bronchial constriction hereby resulting in a disturbance of the vascular endothelial barrier (Blankenbach et al., 2016) which is a critical problem underlying the development of many diseases or complications of injury. (Yuan and Rigor, 2010)

In addition, there appears to be growing evidence that SIP and S1PR signalling generally plays a role in pro-fibrotic responses in various tissues and isolated cells. Indeed, using various SIP receptor agonists in normal lung fibroblasts, pro-fibrotic responses were observed via activation of S1PR2 and S1PR3, which suggests that antagonists of the specific SIP receptors S1P2R and S1P3R may be particularly beneficial in reducing fibrosis (Sobel et al., 2013). US 2014/187550 discloses S1P2 and S1P3 antagonists for the treatment of e.g. anti-inflammatory or allergic conditions.

Fibrosis is a process that can be triggered by chronic tissue damage because of toxic substances, viral infection, inflammation, or mechanical stress (Nanthakumar et al., 2015); and may be defined as the abnormal or excessive production and accumulation of extracellular matrix (ECM).

In particular, fibrosis is a key driver of progressive organ dysfunction in many inflammatory and metabolic diseases, including idiopathic pulmonary fibrosis, advanced liver disease (e.g. non-alcoholic steatohepatitis (NASH)) and advanced kidney disease. These conditions remain poorly treated despite advances in the understanding of the disease mechanism and, more recently, an increase in the number of clinical trials reflecting the need to identify new treatments, particularly in IPF (Nanthakumar et al., 2015). In the case of IPF for example, only two drugs have been approved despite their undesirable side effects (Brunnemer et al., 2018; Lancaster et al., 2017; Richeldi et al., 2014), and therefore there is clear need for improved therapies (Raghu, 2015).

Therefore current therapies are not satisfactory, and in developing an effective therapeutic arsenal, novel modulators of S1PR, in particular selective S1PR2 would be particularly beneficial for the prevention and or treatment of fibrotic diseases, inflammatory diseases, respiratory diseases, autoimmune diseases, metabolic diseases, cardiovascular diseases, and/or proliferative diseases.

### SUMMARY OF THE INVENTION

The present invention relates to compounds of the invention useful in the prophylaxis and/or treatment of fibrotic diseases, inflammatory diseases, respiratory diseases, autoimmune diseases, metabolic diseases, cardiovascular diseases, and/or proliferative diseases. The present invention also provides methods for the production of these compounds, pharmaceutical compositions comprising these compounds and methods for the prophylaxis and/or treatment of fibrotic diseases, inflammatory diseases, respiratory diseases, autoimmune diseases, metabolic diseases, cardiovascular diseases, and/or proliferative diseases by administering the compounds of the invention.

Accordingly, in a first aspect of the invention, the compounds of the invention are provided having a Formula I:

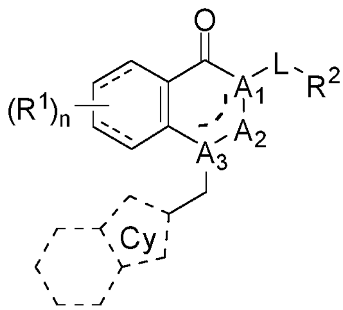

I

wherein

each $A_1$, $A_2$ and $A_3$ is independently selected from C and N provided that $A_1$, $A_2$ and $A_3$ are not simultaneously C or N;
each $R^1$ is independently selected from

- $C_{1-4}$ alkyl,
- $C_{1-4}$ alkoxy,
- $C_{3-6}$ cycloalkyl,
- 4-7 membered monocyclic heteroaryl comprising 1, 2, or 3 heteroatoms independently selected from N, O, or S, optionally substituted with one or two =O groups,
- $-S(O)_2C_{1-4}$ alkyl,
- -CN,
- $-C(=O)NH_2$, and
- halo;

the subscript n is 0, 1 or 2;
Cy is a 9-membered fused 5-6 bicyclic heteroaryl attached as shown in Formula I, comprising 1, 2 or 3 N atoms, which heteroaryl is substituted with one $R^3$ group, one $R^{4a}$ group, and one $R^{4b}$ group; $R^3$ is $C_{1-6}$ alkoxy optionally substituted with one or more independently selected

- halo,
- $C_{1-4}$ alkoxy, or
- $C_{3-7}$ cycloalkyl optionally substituted with one or more independently selected $C_{1-4}$ alkyl, halo, or -CN;

$R^{4a}$ is

- $C_{1-4}$ alkyl optionally substituted with one or more halo,
- halo, or
- -CN;

$R^{4b}$ is H, halo, or OH
L is absent or is $-CR^{5a}R^{5b}-$;
$R^2$ is

- -C(=O)OH,
- $-C(=O)NR^{6a}R^{6b}$,
- $-C(O)NHS(O)_2-C_{1-4}$ alkyl,
- $-C(O)NHS(O)_2-C_{3-7}$ cycloalkyl,
- $-Cy_1$, or

- $-C(=O)Cy_2$;

each $R^{5a}$ and $R^{5b}$ is independently selected from:

- H,
- $C_{1-4}$ alkoxy, and
- $C_{1-4}$ alkyl optionally substituted with one, two or three halo or one $-NR^{8a}R^{8b}$;

each $R^{6a}$ and $R^{6b}$ is independently selected from:

- H,
- $C_{1-6}$ alkyl optionally substituted from one more independently selected
    - OH,
    - -CN,
    - halo,
    - $C_{1-4}$ alkoxy,
    - $-S(O)_2C_{1-4}$ alkyl,
    - $-S(O)_2NH_2$,
    - $-C(O)NR^{9a}R^{9b}$,
    - monocyclic 4-6 membered heterocycloalkyl comprising one or two heteroatoms selected from N, O, and S, optionally substituted with one $-CH_2-OH$, or
    - $C_{3-7}$ cycloalkyl optionally substituted with one or more independently selected OH, or halo;
- $C_{3-7}$ cycloalkyl optionally substituted with one or more OH, and
- monocyclic 4-6 membered heterocycloalkyl comprising one or two heteroatoms selected from N, O, and S, optionally substituted with one or two oxo;

$Cy_1$ is

- $C_{3-7}$ monocyclic cycloalkyl, optionally substituted with one $-C(=O)OH$, or
- monocyclic 4-6 membered heterocycloalkyl comprising one or two heteroatoms selected from N, O, and S, optionally substituted with one or two independently selected $C_{1-4}$ alkyl which alkyl is optionally substituted with one $-C(=O)OH$,

$Cy_2$ is

- N-linked monocyclic 4-7 membered heterocycloalkyl comprising at least one N atom, and optionally one or two heteroatoms selected from N, O, and S, optionally substituted with one or more independently selected:
    - OH,
    - Oxo,
    - -CN,
    - halo,
    - $C_{1-4}$ alkoxy,
    - $C_{1-4}$ alkyl optionally substituted with one or more independently selected
        - halo, or
        - OH,
    - $C_{3-7}$ cycloalkyl,
    - $-S(O)_2C_{1-4}$ alkyl,
    - $-NR^{7a}R^{7b}$;
- N-linked spirocyclic 7-9 membered heterocycloalkyl comprising at least one N atom, and optionally one or two heteroatoms selected from N, O, and S, optionally substituted with one or more halo;

each $R^{7a}$, $R^{7b}$, $R^{8a}$, $R^{8b}$, $R^{9a}$ and $R^{9b}$ is independently selected from H, and $C_{1-4}$ alkyl;

provided that:

- when $A_1$ and $A_2$ are C, $A_3$ is N, L is absent, $R^3$ is unsubstituted $C_{1-6}$ alkoxy or $C_{1-6}$ alkoxy substituted with halogen, then $R^2$ is not COOH; and
- when $A_1$ and $A_2$ are N, $A_3$ is C, and $R^{4a}$ is $-CH_3$ then $R^3$ is not unsubstituted $C_{1-4}$ alkoxy.

In a particular aspect, the compounds of the invention are provided for use in the prophylaxis and/or treatment of fibrotic diseases, inflammatory diseases, respiratory diseases, autoimmune diseases, metabolic diseases, cardiovascular diseases, and/or proliferative diseases.

In a particular aspect the compounds of the invention are sphingosine 1-phosphate receptor (S1PR) modulators. In a more particular embodiment, the compounds of the invention are sphingosine 1-phosphate receptor 2 (S1PR2) antagonists. In a most particular embodiment, the compounds of the invention may show selectivity towards S1PR2, which may be advantageous in reducing undesirable effect associated with non-selective modulation of S1PR.

In yet another aspect, the compounds of the invention may surprisingly show good ADME properties.

In a further aspect, a compound of the invention according to one or more of the embodiments described above may show a good ADME profile, in metabolic stability, bioavailability, and/or low plasma protein binding (PPB), which may result in a lower dose regimen and/or good compliance with dose regimen.

In a further aspect, the present invention provides pharmaceutical compositions comprising a compound of the invention, and a pharmaceutical carrier, excipient or diluent. In a particular aspect, the pharmaceutical composition may additionally comprise further therapeutically active ingredients suitable for use in combination with the compounds of the invention. In a more particular aspect, the further therapeutically active ingredient is an agent for the treatment of fibrotic diseases, inflammatory diseases, respiratory diseases, autoimmune diseases, metabolic diseases, cardiovascular diseases, and/or proliferative diseases.

Moreover, the compounds of the invention, useful in the pharmaceutical compositions and treatment methods disclosed herein, are pharmaceutically acceptable as prepared and used.

In a further aspect of the invention, this invention provides a method of treating a mammal, in particular humans, afflicted with a condition selected from among those listed herein, and particularly fibrotic diseases, inflammatory diseases, respiratory diseases, autoimmune diseases, metabolic diseases, cardiovascular diseases, and/or proliferative diseases, which method comprises administering an effective amount of the pharmaceutical composition or compounds of the invention as described herein.

The present invention also provides pharmaceutical compositions comprising a compound of the invention, and a suitable pharmaceutical carrier, excipient or diluent for use in medicine. In a particular aspect, the pharmaceutical composition is for use in the prophylaxis and/or treatment of fibrotic diseases, inflammatory diseases, respiratory diseases, autoimmune diseases, metabolic diseases, cardiovascular diseases, and/or proliferative diseases.

In additional aspects, this invention provides methods for synthesizing the compounds of the invention, with representative synthetic protocols and pathways disclosed later on herein.

Other objects and advantages will become apparent to those skilled in the art from a consideration of the ensuing detailed description.

It will be appreciated that compounds of the invention may be metabolized to yield biologically active metabolites.

## DETAILED DESCRIPTION OF THE INVENTION

### Definitions

The following terms are intended to have the meanings presented therewith below and are useful in understanding the description and intended scope of the present invention.

When describing the invention, which may include compounds, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein.

The articles 'a' and 'an' may be used herein to refer to one or to more than one (*i.e.* at least one) of the grammatical objects of the article. By way of example 'an analogue' means one analogue or more than one analogue.

'Alkyl' means straight or branched aliphatic hydrocarbon having the specified number of carbon atoms. Particular alkyl groups have 1 to 6 carbon atoms or 1 to 4 carbon atoms. Branched means that one or more alkyl groups such as methyl, ethyl or propyl is attached to a linear alkyl chain. Particular alkyl groups are methyl ($-CH_3$), ethyl ($-CH_2-CH_3$), n-

propyl ($-CH_2-CH_2-CH_3$), isopropyl ($-CH(CH_3)_2$), n-butyl ($-CH_2-CH_2-CH_2-CH_3$), tert-butyl ($-CH_2-C(CH_3)_3$), sec-butyl ($-CH_2-CH(CH_3)_2$), n-pentyl ($-CH_2-CH_2-CH_2-CH_2-CH_3$), n-hexyl ($-CH_2-CH_2-CH_2-CH_2-CH_2-CH_3$), and 1,2-dimethylbutyl ($-CHCH_3)-C(CH_3)H_2-CH_2-CH_3$). Particular alkyl groups have between 1 and 4 carbon atoms.

'Alkoxy' refers to the group O-alkyl, where the alkyl group has the number of carbon atoms specified. In particular the term refers to the group $-O-C_{1-6}$ alkyl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, i.e. with between 1 and 6 carbon atoms. Further particular alkoxy groups have between 1 and 4 carbon atoms.

'Aryl' refers to a monovalent aromatic hydrocarbon group derived by the removal of one hydrogen atom from a single carbon atom of a parent aromatic ring system. In particular aryl refers to an aromatic ring structure, monocyclic or fused polycyclic, with the number of ring atoms specified. Specifically, the term includes groups that include from 6 to 10 ring members. Particular aryl groups include phenyl, and naphthyl.

'Cycloalkyl' refers to a non-aromatic hydrocarbyl ring structure, monocyclic, fused polycyclic, bridged polycyclic, or spirocyclic, with the number of ring atoms specified. A cycloalkyl may have from 3 to 12 carbon atoms, in particular from 3 to 10, and more particularly from 3 to 7 carbon atoms. Such cycloalkyl groups include, by way of example, single ring structures such as cyclopropyl, cyclobutyl, cyclopentyl, cyclohexyl, and cycloheptyl.

'Cyano' refers to the radical -CN.

'Halo' or 'halogen' refers to fluoro (F), chloro (Cl), bromo (Br) and iodo (I). Particular halo groups are either fluoro or chloro.

'Hetero' when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.* heteroalkyl, cycloalkyl, *e.g.* heterocycloalkyl, aryl, *e.g.* heteroaryl, and the like having from 1 to 4, and particularly from 1 to 3 heteroatoms, more typically 1 or 2 heteroatoms, for example a single heteroatom.

'Heteroaryl' means an aromatic ring structure, monocyclic or fused polycyclic, that includes one or more heteroatoms independently selected from O, N and S and the number of ring atoms specified. In particular, the aromatic ring structure may have from 5 to 9 ring members. The heteroaryl group can be, for example, a five membered or six membered monocyclic ring or a fused bicyclic structure formed from fused five and six membered rings or two fused six membered rings or, by way of a further example, two fused five membered rings. Each ring may contain up to four heteroatoms typically selected from nitrogen, sulphur and oxygen. Typically the heteroaryl ring will contain up to 4 heteroatoms, more typically up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general the number of basic nitrogen atoms present in the heteroaryl group, including any amino group substituents of the ring, will be less than five.

Examples of five membered monocyclic heteroaryl groups include but are not limited to pyrrolyl, furanyl, thiophenyl, imidazolyl, furazanyl, oxazolyl, oxadiazolyl, oxatriazolyl, isoxazolyl, thiazolyl, isothiazolyl, pyrazolyl, triazolyl and tctrazolyl groups.

Examples of six membered monocyclic heteroaryl groups include but are not limited to pyridinyl, pyrazinyl, pyridazinyl, pyrimidinyl and triazinyl.

Particular examples of bicyclic heteroaryl groups containing a five membered ring fused to another five-membered ring include but are not limited to imidazothiazolyl and imidazoimidazolyl.

Particular examples of bicyclic heteroaryl groups containing a six membered ring fused to a five membered ring include but are not limited to benzofuranyl, benzothiophenyl, benzoimidazolyl, benzoxazolyl, isobenzoxazolyl, benzisoxazolyl, benzothiazolyl, benzoisothiazolyl, isobenzofuranyl, indolyl, isoindolyl, indolizinyl, purinyl (*e.g.* adenine, guanine), indazolyl, pyrazolopyrimidinyl, triazolopyrimidinyl, and pyrazolopyridinyl groups.

Particular examples of bicyclic heteroaryl groups containing two fused six membered rings include but are not limited to quinolinyl, isoquinolinyl, pyridopyridinyl, quinoxalinyl, quinazolinyl, cinnolinyl, phthalazinyl, naphthyridinyl, and pteridinyl groups. Particular heteroaryl groups are those derived from thiophenyl, pyrrolyl, benzothiophenyl, benzofuranyl, indolyl, pyridinyl, quinolinyl, imidazolyl, oxazolyl and pyrazinyl.

Examples of representative heteroaryls include the following:

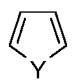 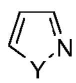 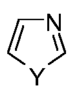 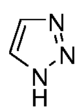 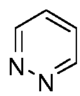 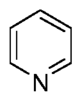

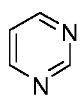 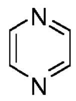 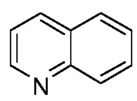 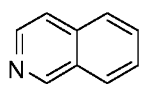

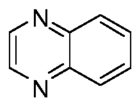 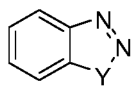 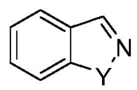 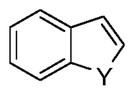

wherein each Y is selected from >C=O, NH, O and S.

'Heterocycloalkyl' means a non-aromatic fully saturated ring structure, monocyclic, fused polycyclic, spirocyclic, or bridged polycyclic, that includes one or more heteroatoms independently selected from O, N and S and the number of ring atoms specified. The heterocycloalkyl ring structure may have from 4 to 12 ring members, in particular from 4 to 10 ring members and more particularly from 4 to 7 ring members. Each ring may contain up to four heteroatoms typically selected from nitrogen, sulphur and oxygen. Typically the heterocycloalkyl ring will contain up to 4 heteroatoms, more typically up to 3 heteroatoms, more usually up to 2, for example a single heteroatom. Examples of heterocyclic rings include, but are not limited to azetidinyl, oxetanyl, thietanyl, pyrrolidinyl (*e.g.* 1-pyrrolidinyl, 2-pyrrolidinyl and 3-pyrrolidinyl), tetrahydrofuranyl (*e.g.* 1-tetrahydrofuranyl, 2-tetrahydrofuranyl and 3-tetrahydrofuranyl), tetrahydrothiophenyl (*e.g.* 1-tetrahydrothiophenyl, 2-tetrahydrothiophenyl and 3-tetrahydrothiophenyl), piperidinyl (*e.g.* 1-piperidinyl, 2-piperidinyl, 3-piperidinyl and 4-piperidinyl), tetrahydropyranyl (*e.g. 4*-tetrahydropyranyl), tetrahydrothiopyranyl (*e.g.* 4-tetrahydrothiopyranyl), morpholinyl, thiomorpholinyl, dioxanyl, or piperazinyl.

Particular examples of monocyclic rings are shown in the following illustrative examples:

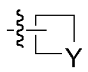 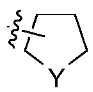 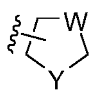 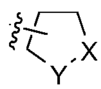

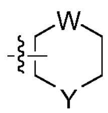 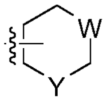 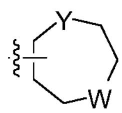

wherein each W and Y is independently selected from $-CH_2-$, -NH-, -O- and -S-.

Particular examples of fused bicyclic rings are shown in the following illustrative examples:

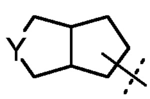 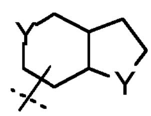 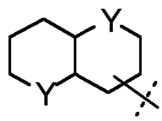 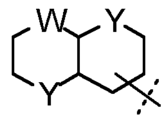

wherein each W and Y is independently selected from $-CH_2-$, -NH-, -O- and -S-.

Particular examples of bridged bicyclic rings are shown in the following illustrative examples:

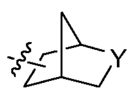 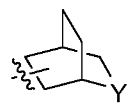 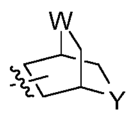 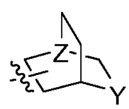

wherein each W and Y is independently selected from $-CH_2-$, -NH-, -O- and -S-, and Z is selected from N and CH.

Particular examples of spirocyclic rings are shown in the following illustrative examples:

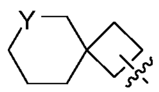 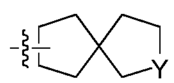 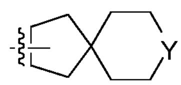

wherein each Y is selected from $-CH_2-$, -NH-, -O- and -S-.

'Hydroxyl' refers to the radical -OH.

'Oxo' refers to the radical =O.

'Substituted' refers to a group in which one or more hydrogen atoms are each independently replaced with the same or different substituent(s).

'Sulfo' or 'sulfonic acid' refers to a radical such as $-SO_3H$.

'Thiol' refers to the group -SH.

As used herein, term 'substituted with one or more' refers to one to four substituents. In one embodiment it refers to one to three substituents. In further embodiments it refers to one or two substituents. In a yet further embodiment it refers to one substituent.

'Thioalkoxy' refers to the group -S-alkyl where the alkyl group has the number of carbon atoms specified. In particular the term refers to the group $-S-C_{1-6}$ alkyl. Particular thioalkoxy groups are thiomethoxy, thioethoxy, n-thiopropoxy, isothiopropoxy, n-thiobutoxy, tert-thiobutoxy, sec-thiobutoxy, n-thiopentoxy, n-thiohexoxy, and 1,2-dimethylthiobutoxy. Particular thioalkoxy groups are lower thioalkoxy, *i.e.* with between 1 and 6 carbon atoms. Further particular alkoxy groups have between 1 and 4 carbon atoms.

One having ordinary skill in the art of organic synthesis will recognize that the maximum number of heteroatoms in a stable, chemically feasible heterocyclic ring, whether it is aromatic or non-aromatic, is determined by the size of the ring, the degree of unsaturation and the valence of the heteroatoms. In general, a heterocyclic ring may have one to four heteroatoms so long as the heteroaromatic ring is chemically feasible and stable.

'Pharmaceutically acceptable' means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

'Pharmaceutically acceptable salt' refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term 'pharmaceutically acceptable cation' refers to an acceptable cationic counter-ion of an acidic functional group. Such cations are exemplified by sodium, potassium, calcium, magnesium, ammonium, tetraalkylammonium cations, and the like.

'Pharmaceutically acceptable vehicle' refers to a diluent, adjuvant, excipient or carrier with which a compound of the invention is administered.

'Prodrugs' refers to compounds, including derivatives of the compounds of the invention,which have cleavable groups and become by solvolysis or under physiological conditions the compounds of the invention which are pharmaceutically active *in vivo.* Such examples include, but are not limited to, choline ester derivatives and the like, N-alkylmorpholine esters and the like.

'Solvate' refers to forms of the compound that are associated with a solvent, usually by a solvolysis reaction. This physical association includes hydrogen bonding. Conventional solvents include water, EtOH, acctic acid and the like. The compounds of the invention may be prepared *e.g.* in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. 'Solvate' encompasses both solution-phase and isolable solvates. Representative solvates include hydrates, ethanolates and methanolates.

'Subject' includes humans. The terms 'human', 'patient' and 'subject' are used interchangeably herein.

'Effective amount' means the amount of a compound of the invention that, when administered to a subject for treating a disease, is sufficient to effect such treatment for the disease. The "effective amount" can vary depending on the compound, the disease and its severity, and the age, weight, etc., of the subject to be treated.

'Preventing' or 'prevention' refers to a reduction in risk of acquiring or developing a disease or disorder (*i.e.* causing at least one of the clinical symptoms of the disease not to develop in a subject that may be exposed to a disease-causing agent, or predisposed to the disease in advance of disease onset).

The term 'prophylaxis' is related to 'prevention', and refers to a measure or procedure the purpose of which is to prevent, rather than to treat or cure a disease. Non-limiting examples of prophylactic measures may include the administration of vaccines; the administration of low molecular weight heparin to hospital patients at risk for thrombosis due, for example, to immobilization; and the administration of an anti-malarial agent such as chloroquine, in advance of a visit to a geographical region where malaria is endemic or the risk of contracting malaria is high.

'Treating' or 'treatment' of any disease or disorder refers, in one embodiment, to ameliorating the disease or disorder (*i.e.* arresting the disease or reducing the manifestation, extent or severity of at least one of the clinical symptoms thereof). In another embodiment 'treating' or 'treatment' refers to ameliorating at least one physical parameter, which may not be discernible by the subject. In yet another embodiment, 'treating' or 'treatment' refers to modulating the disease or disorder, either physically, (*e.g*. stabilization of a discernible symptom), physiologically, (*e.g*. stabilization of a physical parameter), or both. In a further embodiment, 'treating' or 'treatment' relates to slowing the progression of the disease.

As used herein the term 'fibrotic diseases' refers to diseases characterized by excessive scarring due to excessive production, deposition, and contraction of extracellular matrix, and are that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract. In particular, the term fibrotic diseases refers to idiopathic pulmonary fibrosis (IPF); cystic fibrosis, other diffuse parenchymal lung diseases of different etiologies including iatrogenic drug-induced fibrosis, occupational and/or environmental induced fibrosis, granulomatous diseases (sarcoidosis, hypersensitivity pneumonia), collagen vascular disease, alveolar proteinosis, langerhans cell granulomatosis, lymphangioleiomyomatosis, inherited diseases (Hermansky-Pudlak Syndrome, tuberous sclcrosis, neurofibromatosis, metabolic storage diseases, familial interstitial lung disease); radiation induced fibrosis; chronic obstructive pulmonary disease; scleroderma; bleomycin induced pulmonary fibrosis; chronic asthma; silicosis; asbestos induced pulmonary fibrosis; acute respiratory distress syndrome (ARDS); kidney fibrosis; tubulointerstitium fibrosis; glomerular nephritis; diabetic nephropathy, focal segmental glomerular sclerosis; IgA nephropathy; hypertension; Alport; gut fibrosis; liver fibrosis; cirrhosis; alcohol induced liver fibrosis; toxic/drug induced liver fibrosis; hemochromatosis; nonalcoholic steatohepatitis (NASH); biliary duct injury; primary biliary cirrhosis; infection induced liver fibrosis; viral induced liver fibrosis; and autoimmune hepatitis; corneal scarring; hypertrophic scarring; Dupuytren disease, keloids, cutaneous fibrosis; cutaneous scleroderma; systemic sclerosis, spinal cord injury/fibrosis; myelofibrosis; Duchenne muscular dystrophy (DMD) associated musculoskeletal fibrosis, vascular restenosis; atherosclerosis; arteriosclerosis; Wegener's granulomatosis; Peyronie's disease, or chronic lymphocytic. More particularly, the term "fibrotic diseases" refers to idiopathic pulmonary fibrosis (IPF), Dupuytren disease, nonalcoholic steatohepatitis (NASH), portal hypertension, systemic sclerosis, renal fibrosis, and cutaneous fibrosis.

As used herein the term 'inflammatory disease(s)' refers to the group of conditions including, rheumatoid arthritis (RA), osteoarthritis (OA), juvenile idiopathic arthritis, psoriasis, psoriatic arthritis, ankylosing spondylitis, allergic airway disease (*e.g*. asthma, rhinitis), chronic obstructive pulmonary disease (COPD), inflammatory bowel diseases (IBD) (*e.g*. Crohn's disease, ulcerative colitis), endotoxin-driven disease states (*e.g*. complications after bypass surgery or chronic endotoxin states contributing to e.g. chronic cardiac failure), and related diseases involving cartilage, such as that of the joints. Particularly the term refers to rheumatoid arthritis, osteoarthritis, allergic airway disease (*e.g*. asthma), chronic obstructive pulmonary disease and inflammatory bowel diseases. More particularly the term refers to rheumatoid arthritis, osteoarthritis, allergic airway disease, chronic obstructive pulmonary disease and inflammatory bowel diseases.

As used herein , the term 'respiratory disease(s)' refers to disease(s) affecting the organs that are involved in breathing, such as the nose, throat, larynx, eustachian tubes, trachea, bronchi, lungs, related muscles (*e.g*., diaphram and intercostals), and nerves. In particular, examples of respiratory diseases include asthma, adult respiratory distress syndrome and allergic (extrinsic) asthma, non-allergic (intrinsic) asthma, acute severe asthma, chronic asthma, clinical asthma, nocturnal asthma, allerGen-induced asthma, aspirin-sensitive asthma, exercise-induced asthma, isocapnic hyperventilation, child onset asthma, adult-onset asthma, cough-variant asthma, occupational asthma, steroid-resistant asthma, seasonal asthma, seasonal allergic rhinitis, perennial allergic rhinitis, chronic obstructive pulmonary disease, including chronic bronchitis or emphysema, pulmonary hypertension, interstitial lung fibrosis and/or airway inflammation, cystic fibrosis, and hypoxia. More particularly the term refers to asthma.

As used herein the term 'asthma' as used herein refers to any disease of the lungs characterized by variations in pulmonary gas flow associated with airway constriction of whatever cause (intrinsic, extrinsic, or both; allergic or non-allergic). The term asthma may be used with one or more adjectives to indicate the cause.

As used herein the term 'autoimmune discasc(s)' refers to the group of diseases including obstructive airways disease, including conditions such as chronic obstructive pulmonary disease (COPD), asthma (e.g intrinsic asthma, extrinsic asthma, dust asthma, infantile asthma) particularly chronic or inveterate asthma (for example late asthma and airway hyperreponsiveness), bronchitis, including bronchial asthma, systemic lupus erythematosus (SLE), cutaneous lupus erythematosus, lupus nephritis, dermatomyositis, Sjogren's syndrome, multiple sclerosis, psoriasis, dry eye disease, type I diabetes mellitus and complications associated therewith, atopic eczema (atopic dermatitis), thyroiditis (Hashimoto's and autoimmune thyroiditis), contact dermatitis and further eczematous dermatitis, inflammatory bowel disease (*e.g*. Crohn's disease and ulcerative colitis), atherosclerosis and amyotrophic lateral sclerosis. Particularly the term refers to chronic obstructive pulmonary disease, asthma, systemic lupus erythematosus, type I diabetes mellitus and inflammatory bowel disease. More particularly, the term refers to chronic obstructive pulmonary disease, asthma, systemic lupus erythematosus, type I diabetes mellitus and inflammatory bowel disease.

As used herein the term 'metabolic disease(s)' refers to the group of conditions affecting the body's ability to process certain nutrients and vitamins. Examples of metabolic disorders include cystic fibrosis, phenylketonuria (PKU), type II diabetes, hyperlipidemia, gout, obesity and rickets. A particular example of metabolic disorders is type II diabetes and/or obesity.

As used herein the term 'cardiovascular diseases' refers to diseases affecting the heart or blood vessels or both. In particular, cardiovascular disease includes arrhythmia (atrial or ventricular or both); atherosclerosis and its sequelae; angina; cardiac rhythm disturbances; myocardial ischemia; myocardial infarction; cardiac or vascular aneurysm; vasculitis, giant cell arteritis, stroke; peripheral obstructive arteriopathy of a limb, an organ, or a tissue; reperfusion injury following ischemia (for example ischemia of the brain, heart, or kidney); endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue. In particular the term refers to stroke, atherosclerosis, reperfusion injury following ischemia, myocardial ischemia, angina, peripheral obstructive arteriopathy or vasculitis. More particularly, the term refers to stroke, atherosclerosis, reperfusion injury following ischemia, myocardial ischemia, or vasculitis.

As used herein the term 'proliferative disease(s)' refers to conditions such as cancer (e.g. uterine leiomyosarcoma or prostate cancer), myeloproliferative diseases (e.g. polycythemia vera, essential thrombocytosis and myelofibrosis), leukemia (e.g. acute myeloid leukaemia, acute and chronic lymphoblastic leukemia), multiple myeloma, psoriasis, restenosis, scleroderma or fibrosis. In particular the term refers to cancer, leukemia, multiple myeloma, psoriasis, restenosis, or scleroderma.

As used herein, the term 'cancer' refers to a malignant or benign growth of cells in skin or in body organs, for example but without limitation, breast, prostate, lung, kidney, pancreas, stomach or bowel. A cancer tends to infiltrate into adjacent tissue and spread (metastasise) to distant organs, for example to bone, liver, lung or the brain. As used herein the term cancer includes both metastatic tumour cell types (such as but not limited to, melanoma, lymphoma, leukaemia, fibrosarcoma, rhabdomyosarcoma, and mastocytoma) and types of tissue carcinoma (such as but not limited to, colorectal cancer, prostate cancer, small cell lung cancer and non-small cell lung cancer, breast cancer, pancreatic cancer, bladder cancer, renal cancer, gastric cancer, glioblastoma, primary liver cancer, ovarian cancer, prostate cancer and uterine leiomyosarcoma). In particular, the term 'cancer' refers to acute lymphoblastic leukemia, acute myeloidleukemia, adrenocortical carcinoma, anal cancer, appendix cancer, astrocytomas, atypical teratoid/rhabdoid tumor, basal cell carcinoma, bile duct cancer, bladder cancer, bone cancer (osteosarcoma and malignant fibrous histiocytoma), brain stem glioma, brain tumors, brain and spinal cord tumors, breast cancer, bronchial tumors, Burkitt lymphoma, cervical cancer, chronic lymphocytic leukemia, chronic myelogenous leukemia, colon cancer, colorectal cancer, craniopharyngioma, cutaneous T-Cell lymphoma, embryonal tumors, endometrial cancer, ependymoblastoma, ependymoma, esophageal cancer, ewing sarcoma family of tumors, eye cancer, retinoblastoma, gallbladder cancer, gastric (stomach) cancer, gastrointestinal carcinoid tumor, gastrointestinal stromal tumor (GIST), gastrointestinal stromal cell tumor, germ cell tumor, glioma, hairy cell leukemia, head and neck cancer, hepatocellular (liver) cancer, hodgkin lymphoma, hypopharyngeal cancer, intraocular melanoma, islet cell tumors (endocrine pancreas), Kaposi sarcoma, kidney cancer, Langerhans cell histiocytosis, laryngeal cancer, leukemia, Acute lymphoblastic leukemia, acute myeloid leukemia, chronic lymphocytic leukemia, chronic myelogenous leukemia, hairy cell leukemia, liver cancer, non-small cell lung cancer, small cell lung cancer, Burkitt lymphoma, cutaneous T-celllymphoma, Hodgkin lymphoma, non-Hodgkin lymphoma, lymphoma, Waldenstrom macroglobulinemia, medulloblastoma, medulloepithelioma, melanoma, mesothelioma, mouth cancer, chronic myelogenous leukemia, myeloid leukemia, multiple myeloma, asopharyngeal cancer, neuroblastoma, non-Hodgkin lymphoma, non-small cell lung cancer, oral cancer, oropharyngeal cancer, osteosarcoma, malignant fibrous histiocytoma of bone, ovarian cancer, ovarian epithelial cancer, ovarian germ cell tumor, ovarian low malignant potential tumor, pancreatic cancer, papillomatosis, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, primary central nervous system lymphoma, prostate cancer, rectal cancer, renal cell (kidney) cancer, retinoblastoma, rhabdomyosarcoma, salivary gland cancer, sarcoma, Ewing sarcoma family of tumors, sarcoma, kaposi, Sezary syndrome, skin cancer, small cell Lung cancer, small intestine cancer, soft tissue sarcoma, squamous cell carcinoma, stomach (gastric) cancer, supratentorial primitive neuroectodermal tumors, T -cell lymphoma, testicular cancer, throat cancer, thymoma and thymic carcinoma, thyroid cancer, urethral cancer, uterine cancer, uterine sarcoma, vaginal cancer, vulvar cancer, Waldenstrom macroglobulinemia, and Wilms tumor.

As used herein the term 'leukemia' refers to neoplastic diseases of the blood and blood forming organs. Such diseases can cause bone marrow and immune system dysfunction, which renders the host highly susceptible to infection and bleeding. In particular the term leukemia refers to acute myeloid leukaemia (AML), and acute lymphoblastic leukemia (ALL) and chronic lymphoblastic leukaemia (CLL).

'Compound(s) of the invention', and equivalent expressions, are meant to embrace compounds of the Formula(e) as herein described, which expression includes the pharmaceutically acceptable salts, and the solvates, *e.g.* hydrates, and the solvates of the pharmaceutically acceptable salts where the context so permits. Similarly, reference to intermediates, whether or not they themselves are claimed, is meant to embrace their salts, and solvates, where the context so permits.

When ranges are referred to herein, for example but without limitation, $C_{1-8}$ alkyl, the citation of a range should be considered a representation of each member of said range.

Other derivatives of the compounds of this invention have activity in both their acid and acid derivative forms, but in the acid sensitive form often offers advantages of solubility, tissue compatibility, or delayed release in the mammalian organism (Bundgard, H, 1985). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are particularly useful prodrugs. In some cases it is desirable to prepare double ester type prodrugs such as (acyloxy)alkyl esters or ((alkoxycarbonyl)oxy)alkylesters. Particular such prodrugs are the $C_{1-8}$ alkyl, $C_{2-8}$ alkenyl, $C_{6-10}$ optionally substituted aryl, and ($C_{6-10}$ aryl)-($C_{1-4}$ alkyl) esters of the compounds of the invention.

As used herein, the term 'isotopic variant' refers to a compound that contains unnatural proportions of isotopes at one or more of the atoms that constitute such compound. For example, an 'isotopic variant' of a compound can contain one or more non-radioactive isotopes, such as for example, deuterium ($^{2}H$ or D), carbon-13 ($^{13}C$), nitro ($^{15}N$), or the like. It will be understood that, in a compound where such isotopic substitution is made, the following atoms, where present, may vary, so that for example, any hydrogen may be $^{2}H$/D, any carbon may be $^{13}C$, or any nitrogen may be $^{15}N$, and that the presence and placement of such atoms may be determined within the skill of the art. Likewise, the invention may include the preparation of isotopic variants with radioisotopes, in the instance for example, where the resulting compounds may be used for drug and/or substrate tissue distribution studies. The radioactive isotopes tritium, i.e. $^{3}H$, and carbon-14, i.e. $^{14}C$, are particularly useful for this purpose in view of their ease of incorporation and ready means of detection. Further, compounds may be prepared that are substituted with positron emitting isotopes, such as $^{11}C$, $^{18}F$, $^{15}O$ and $^{13}N$, and would be useful in Positron Emission Topography (PET) studies for examining substrate receptor occupancy.

It is also to be understood that compounds that have the same molecular formula but differ in the nature or sequence of bonding of their atoms or the arrangement of their atoms in space are termed 'isomers'. Isomers that differ in the arrangement of their atoms in space are termed 'stereoisomers'.

Stereoisomers that are not mirror images of one another are termed 'diastereomers' and those that are non-superimposable mirror images of each other are termed 'enantiomers'. When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e. as (+) or (-)-isomers respectively). A chiral compound can exist as either individual enantiomer or as a mixture thereof. A mixture containing equal proportions of the enantiomers is called a 'racemic mixture'.

'Tautomers' refer to compounds that are interchangeable forms of a particular compound structure, and that vary in the displacement of hydrogen atoms and electrons. Thus, two structures may be in equilibrium through the movement of $\pi$ electrons and an atom (usually H). For example, enols and ketones are tautomers because they are rapidly interconverted by treatment with either acid or base. Another example of tautomerism is the aci- and nitro- forms of phenylnitromethane, that are likewise formed by treatment with acid or base.

Tautomeric forms may be relevant to the attainment of the optimal chemical reactivity and biological activity of a compound of interest.

The compounds of the invention may possess one or more asymmetric centers; such compounds can therefore be produced as individual (R)- or (S)- stereoisomers or as mixtures thereof.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

It will be appreciated that compounds of the invention may be metabolized to yield biologically active metabolites.

## THE INVENTION

The present invention is based on the identification of novel compounds, and their ability to act as sphingosine 1-phosphate (SIP) receptor antagonists, which may be useful in the prophylaxis and/or treatment of fibrotic diseases, inflammatory diseases, respiratory diseases, autoimmune diseases, metabolic diseases, cardiovascular diseases, and/or proliferative diseases.

The present invention also provides methods for the production of these compounds, pharmaceutical compositions comprising these compounds and methods for the prophylaxis and/or treatment of fibrotic diseases, inflammatory diseases, respiratory diseases, autoimmune diseases, metabolic diseases, cardiovascular diseases, and/or proliferative diseases by administering the compounds of the invention.

Accordingly, in a first aspect of the invention, the compounds of the invention are provided having a Formula I:

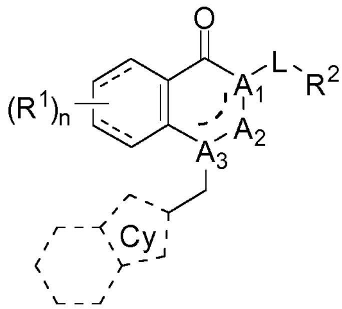

I

wherein

each $A_1$, $A_2$ and $A_3$ is independently selected from C and N provided that $A_1$, $A_2$ and $A_3$ are not simultaneously C or N;
each $R^1$ is independently selected from

- $C_{1-4}$ alkyl,
- $C_{1-4}$ alkoxy,
- $C_{3-6}$ cycloalkyl,
- 4-7 membered monocyclic heteroaryl comprising 1, 2, or 3 heteroatoms independently selected from N, O, or S, optionally substituted with one or two =O groups,
- $-S(O)_2C_{1-4}$ alkyl,
- -CN,
- $-C(=O)NH_2$, and
- halo;

the subscript n is 0, 1 or 2;
Cy is a 9-membered fused 5-6 bicyclic heteroaryl attached as shown in Formula I, comprising 1, 2 or 3 N atoms, which heteroaryl is substituted with one $R^3$ group, one $R^{4a}$ group, and one $R^{4b}$ group; $R^3$ is $C_{1-6}$ alkoxy optionally substituted with one or more independently selected

- halo,
- $C_{1-4}$ alkoxy, or
- $C_{3-7}$ cycloalkyl optionally substituted with one or more independently selected $C_{1-4}$ alkyl, halo, or -CN;

$R^{4a}$ is

- $C_{1-4}$ alkyl optionally substituted with one or more halo,
- halo, or
- -CN;

$R^{4b}$ is H, halo, or OH
L is absent or is $-CR^{5a}R^{5b}-$;
$R^2$ is

- -C(=O)OH,
- $-C(=O)NR^{6a}R^{6b}$,
- $-C(O)NHS(O)_2-C_{1-4}$ alkyl,
- $-C(O)NHS(O)_2-C_{3-7}$ cycloalkyl,
- $-Cy_1$, or
- $-C(=O)Cy_2$;

each $R^{5a}$ and $R^{5b}$ is independently selected from

- H,
- $C_{1-4}$ alkoxy, and
- $C_{1-4}$ alkyl optionally substituted with one, two or three halo or one $-NR^{8a}R^{8b}$;

each $R^{6a}$ and $R^{6b}$ is independently selected from

- H,
- $C_{1-6}$ alkyl optionally substituted from one more independently selected
  - OH,
  - -CN,
  - halo,
  - $C_{1-4}$ alkoxy,
  - $-S(O)_2C_{1-4}$ alkyl,
  - $-S(O)_2NH_2$,
  - $-C(O)NR^{9a}R^{9b}$,
  - monocyclic 4-6 membered heterocycloalkyl comprising one or two heteroatoms selected from N, O, and S, optionally substituted with one $-CH_2-OH$, or
  - $C_{3-7}$ cycloalkyl optionally substituted with one or more independently selected OH, or halo;
- $C_{3-7}$ cycloalkyl optionally substituted with one or more OH, and
- monocyclic 4-6 membered heterocycloalkyl comprising one or two heteroatoms selected from N, O, and S, optionally substituted with one or two oxo;

$Cy_1$ is

- $C_{3-7}$ monocyclic cycloalkyl, optionally substituted with one -C(=O)OH, or
- monocyclic 4-6 membered heterocycloalkyl comprising one or two heteroatoms selected from N, O, and S, optionally substituted with one or two independently selected $C_{1-4}$ alkyl which alkyl is optionally substituted with one -C(=O)OH,

$Cy_2$ is

- N-linked monocyclic 4-7 membered heterocycloalkyl comprising at least one N atom, and optionally one or two heteroatoms selected from N, O, and S, optionally substituted with one or more independently selected
  - OH,
  - Oxo,
  - -CN,
  - halo,
  - $C_{1-4}$ alkoxy,
  - $C_{1-4}$ alkyl optionally substituted with one or more independently selected
    - halo, or
    - OH,
  - $C_{3-7}$ cycloalkyl,
  - $-S(O)_2C_{1-4}$ alkyl, or
  - $-NR^{7a}R^{7b}$; or

- N-linked spirocyclic 7-9 membered heterocycloalkyl comprising at least one N atom, and optionally one or two heteroatoms selected from N, O, and S, optionally substituted with one or more halo;

each $R^{7a}$, $R^{7b}$, $R^{8a}$, $R^{8b}$, $R^{9a}$ and $R^{9b}$ is independently selected from H, and $C_{1-4}$ alkyl;
provided that

- when $A_1$ and $A_2$ are C, $A_3$ is N, L is absent, $R^3$ is unsubstituted $C_{1-6}$ alkoxy or $C_{1-6}$ alkoxy substituted with halogen, then $R^2$ is not COOH; and
- when $A_1$ and $A_2$ are N, $A_3$ is C, and $R^{4a}$ is $-CH_3$ then $R^3$ is not unsubstituted $C_{1-4}$ alkoxy.

In another embodiment, the compound of the invention is according to Formula I, wherein the subscript n is 1 or 2. In a particular embodiment, the subscript n is 1.
In one embodiment, the compound of the invention is according to Formula I, wherein the subscript n is 2 and each $R^1$ is independently selected halo. In a particular embodiment, each $R^1$ is independently selected from F and Cl.
In one embodiment, the compound of the invention is according to Formula I, wherein the subscript n is 1 and $R^1$ is $C_{1-4}$ alkyl. In a particular embodiment, $R^1$ is $-CH_3$.
In one embodiment, the compound of the invention is according to Formula I, wherein the subscript n is 1 and $R^1$ is $C_{1-4}$ alkoxy. In a particular embodiment, $R^1$ is $-OCH_3$.
In one embodiment, the compound of the invention is according to Formula I, wherein the subscript n is 1 and $R^1$ is $C_{3-7}$ cycloalkyl. In a particular embodiment, $R^1$ is cyclopropyl.
In one embodiment, the compound of the invention is according to Formula I, wherein the subscript n is 1 and $R^1$ is 4-7 membered monocyclic heterocycloalkyl comprising 1, 2 or 3 heteroatoms independently selected from N, O, or S, optionally substituted with one or two oxo. In a particular embodiment, $R^1$ is 4-7 membered monocyclic heterocycloalkyl comprising 1, 2 or 3 heteroatoms independently selected from N, O, or S. In a more particular embodiment, $R^1$ is morpholinyl.
In one embodiment, the compound of the invention is according to Formula I, wherein the subscript n is 1 and $R^1$ is -CN or halo. In a particular embodiment, $R^1$ is -CN, F or Cl.
In one embodiment, the compound of the invention is according to Formula I, wherein the subscript n is 0.
In one embodiment, the compound of the invention according to Formula I is according to any of Formula IIa, IIb, IIc, IId, IIe, or IIf:

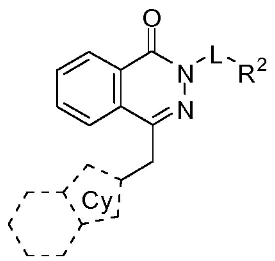
IIa,

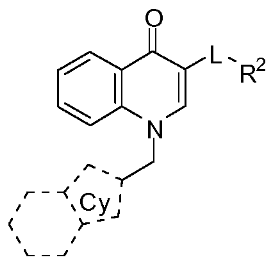
IIb,

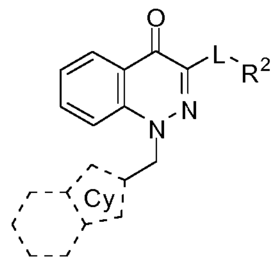
IIc,

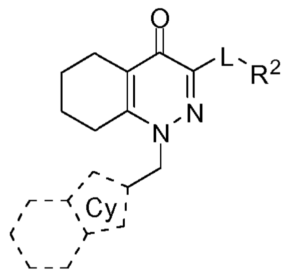
IId,

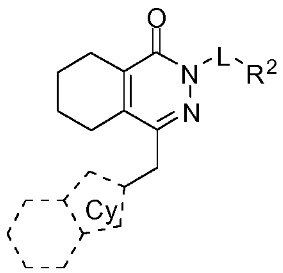
IIe, or

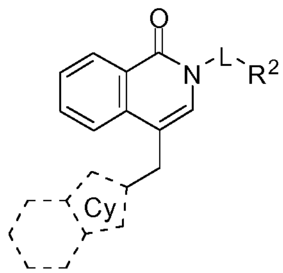
IIf.

wherein $R^2$, L, and Cy are as previously decribed.
In one embodiment, the compound of the invention is according to any one of Formula I-IIf, wherein Cy is a 9-membered fused 5-6 bicyclic heteroaryl linked via the 5-membered ring, comprising 1, 2 or 3 N atoms, which heteroaryl is substituted with one $R^3$ group, one $R^{4a}$ group, and one $R^{4b}$ group. In a particular embodiment, Cy is imidazopyridinyl,benzimidazolyl, indazolyl, indolyl, or pyrazolopyridinyl, each of which is substituted with one $R^3$ group, one $R^{4a}$ group, and one $R^{4b}$ group.

In one embodiment, the compound of the invention is according to any one of Formula I-IIf, wherein Cy is selected from $Cy_A$, $Cy_B$, Cyc, and $Cy_D$:

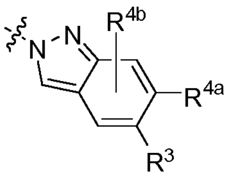

$Cy_A$,

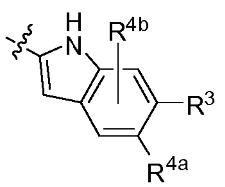

$Cy_B$,

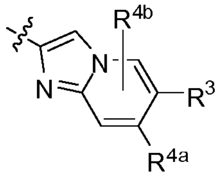

$Cy_C$, and

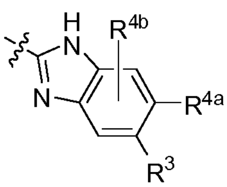

$Cy_D$.

wherein $R^3$, $R^{4a}$, and $R^{4b}$ are as previously described.

In one embodiment, the compound of the invention is according to anyone of Formula I-IIg, $R^3$ is $C_{1-6}$ alkoxy. In a particular embodiment, $R^3$ is $-OCH_3$, $-OCH_2CH_3$, $-OCH_2CH(CH_3)_2$ or $-OCH_2C(CH_3)_3$. In a more particular embodiment, $R^3$ is $-OCH_2CH_3$.

In another embodiment, the compound of the invention is according to any one of Formula I-IIf, $R^3$ is $C_{1-6}$ alkoxy substituted with one or more independently selected halo, $C_{1-4}$ alkoxy, or $C_{3-7}$ cycloalkyl optionally substituted with one or more independently selected $C_{1-4}$ alkyl, halo, or -CN. In a particular embodiment, $R^3$ is $C_{1-6}$ alkoxy substituted with one, two or three independently selected halo, $C_{1-4}$ alkoxy, or $C_{3-7}$ cycloalkyl optionally substituted with one or more independently selected $C_{1-4}$ alkyl, halo, or -CN. In another particular embodiment, $R^3$ is $C_{1-6}$ alkoxy substituted with one halo, $C_{1-4}$ alkoxy, or $C_{3-7}$ cycloalkyl optionally substituted with one or more independently selected $C_{1-4}$ alkyl, halo, or -CN. In a more particular embodiment, $R_3$ is $-OCH_3$, or $-OCH_2CH_3$, each of which is substituted with one, two or three independently selected halo, $C_{1-4}$ alkoxy, or $C_{3-7}$ cycloalkyl optionally substituted with one or more independently selected $C_{1-4}$ alkyl, halo, or -CN. In another more particular embodiment, $R_3$ is $-OCH_3$, or $-OCH_2CH_3$, each of which is substituted with one halo, $C_{1-4}$ alkoxy, or $C_{3-7}$ cycloalkyl optionally substituted with one or more independently selected $C_{1-4}$ alkyl, halo, or -CN. In a most particular embodiment, $R_3$ is $-OCF_3$, $-OCH_2CF_3$, or $-OCH_2CHF_2$. In another more particular embodiment, $R_3$ is $-OCH_3$, or $-OCH_2CH_3$, each of which is substituted with one $-OCH_3$, $-OCH_2CH_3$ or cyclopropyl optionally substituted with one or more independently selected $C_{1-4}$ alkyl, halo, or -CN.

In another embodiment, the compound of the invention is according to any one of Formula I-IIf, $R^3$ is $-OCH_3$, $-OCH_2CH_3$, $-OCF_3$, $-OCH_2CF_3$, $-OCH_2CHF_2$, $-OCH_2CH_2OCH_3$,

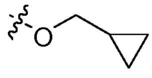
,
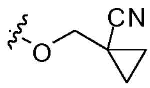
, or
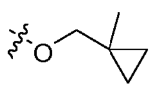
.

In another embodiment, the compound of the invention is according to any one of Formula I-IIf, wherein $R^{4b}$ is H, halo or OH. In a particular embodiment, $R^{4b}$ is H, F, Cl or OH. In a more particular embodiment, $R^{4b}$ is H.

In one embodiment, the compound of the invention is according to Formula IIIa, IIIb, or IIIc:

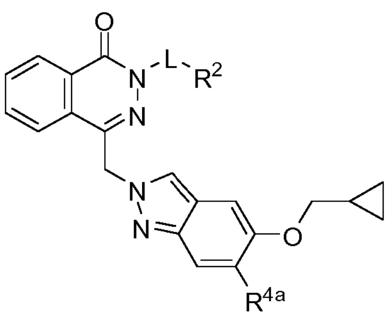

IIIa,

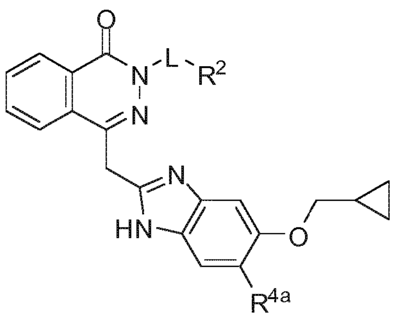

IIIb, or

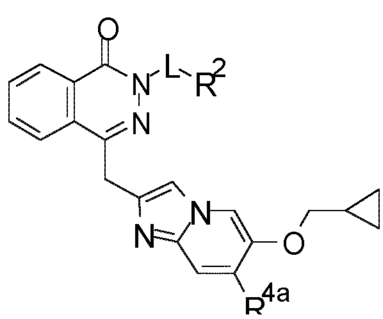

IIIc.

wherein $R^{4a}$, L and $R^2$ are as described previously.

In one embodiment, the compound of the invention is according to Formula IVa, IVb, or IVc:

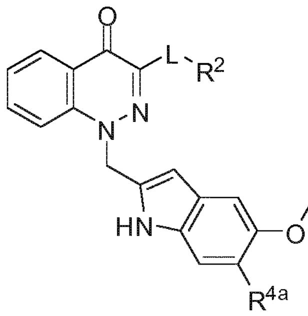

IVa,

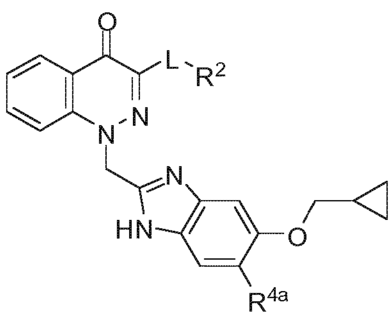

IVb, or

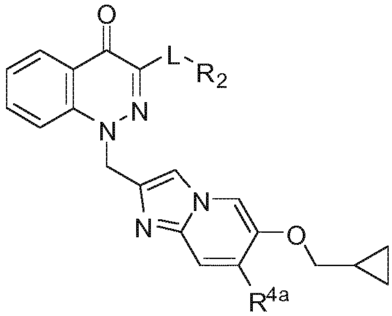

IVc.

wherein $R^{4a}$, L and $R^2$ are as described previously.

In one embodiment, the compound of the invention is according to any one of Formula I-IVc, wherein $R^{4a}$ is halo, -CN, or $C_{1-4}$ alkyl optionally substituted with one or more halo. In a particular embodiment, $R^{4a}$ is F, Cl, -CN, or $-CF_3$. In a more particular embodiment, $R^{4a}$ is Cl.

In one embodiment, the compound of the invention is according to any one of Formula I-IVc, wherein L is absent.

In another embodiment, the compound of the invention is according to any one of Formula I-IVc, wherein L is $-CR^{5a}R^{5b}-$.

In one embodiment, the compound of the invention is according to any one of Formula I-VIc, wherein L is absent and $R^2$ is $-Cy_1$. In a particular embodiment, $Cy_1$ is $C_{3-7}$ monocyclic cycloalkyl, optionally substituted with one -C(=O)OH. In a more particular embodiment, $Cy_1$ is cyclopropyl, cyclobutyl, or cyclopentyl, each of which is optionally substituted with one -C(=O)OH. In a most particular embodiment, $Cy_1$ is cyclopropyl or cyclobutyl. In a further most particular embodiment, $Cy_1$ is

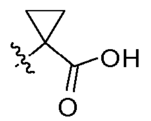 or 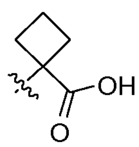.

In one embodiment, the compound of the invention is according to any one of Formula I-VIc, wherein L is absent and $R^2$ is $-Cy_1$ is monocyclic 4-6 membered heterocycloalkyl comprising one or two heteroatoms selected from N, O, and S, optionally substituted with one or two independently selected $C_{1-4}$ alkyl which alkyl is optionally substituted with one -C(=O)OH. In a particular embodiment, $Cy_1$ is azetidinyl, oxetanyl, pyrolidinyl, dioxolanyl, tetrahydrofuranyl, piperidinyl, piperazinyl, morpholinyl, or tetrahydropyranyl, each of which is optionally substituted with one or two independently selected $C_{1-4}$ alkyl which alkyl is optionally substituted with one -C(=O)OH. In another particular embodiment, $Cy_1$ is monocyclic 4-6 membered heterocycloalkyl comprising one or two heteroatoms selected from N, O, and S, optionally substituted with one or two independently selected $-CH_3$, $-CH_2CH_3$, or $-CH_2C(=O)OH$. In a most particular embodiment, $Cy_1$ is azetidinyl, oxetanyl, pyrolidinyl, dioxolanyl, tetrahydrofuranyl, piperidinyl, piperazinyl, morpholinyl, or tetrahydropyranyl, each of which is optionally substituted with one or two independently selected $-CH_3$, $-CH_2CH_3$, or $-CH_2C(=O)OH$.

In one embodiment, the compound of the invention is according to Formula Va, Vb, or Vc:

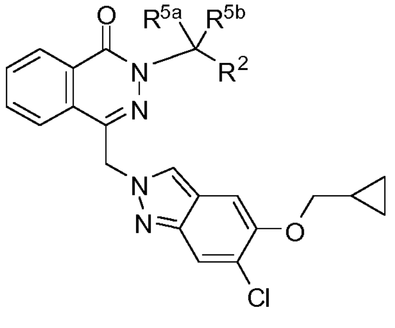

Va,

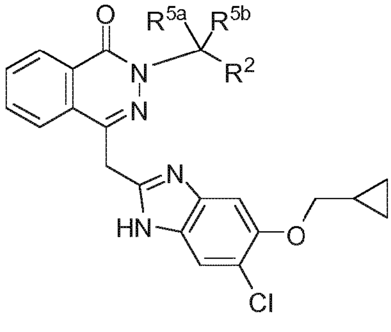

Vb, or

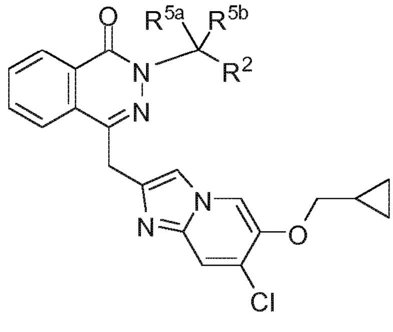

Vc.

wherein $R^{4a}$, L and $R^2$ are as described previously.

In one embodiment, the compound of the invention is according to Formula VIa, VIb, or VIc:

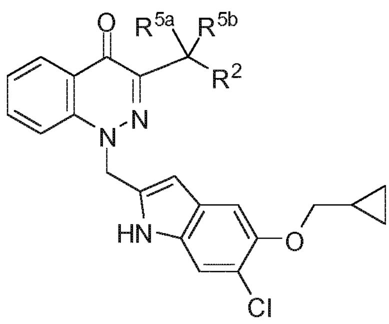

VIa,

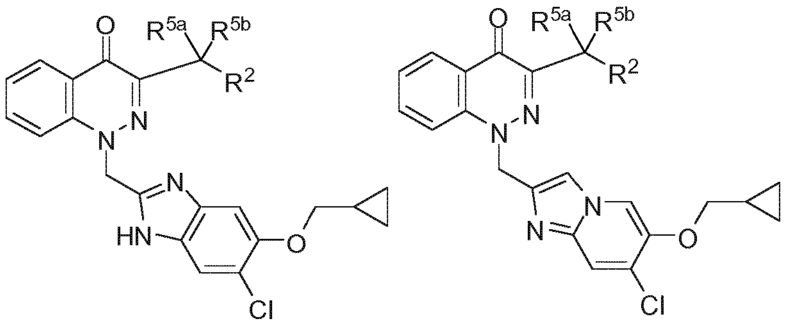

VIb, or

Vic.

wherein $R^{5a}$, $R^{5b}$, L and $R^2$ are as described previously.

In another embodiment, the compound of the invention is according to any one of Formula I-IVc, wherein L is $-CR^{5a}R^{5b}-$, or according to any one of Formula Va-VIc, wherein $R^{5a}$ and $R^{5b}$ are H.

In another embodiment, the compound of the invention is according to any one of Formula I-IVc, wherein L is $-CR^{5a}R^{5b}-$, or according to any one of Formula Va-VIc, wherein each $R^{5a}$ and $R^{5b}$ is independently selected from H, $C_{1-4}$ alkoxy, and $C_{1-4}$ alkyl optionally substituted with one, two or three halo or one $-NR^{8a}R^{8b}$, wherein $R^{8a}$ and $R^{8b}$ are as defined previously. In a more particular embodiment, each $R^{5a}$ and $R^{5b}$ is independently selected from H, $C_{1-4}$ alkoxy, and $C_{1-4}$ alkyl optionally substituted with one, two or three halo or one $-NR^{8a}R^{8b}$, wherein each $R^{8a}$ and $R^{8b}$ is independently selected from H, $-CH_3$, or - $CH_2CH_3$. In a most particular embodiment, each $R^{5a}$ and $R^{5b}$ is independently selected from H, $-OCH_3$, - $OCH_2CH_3$, $-CH_3$, $-CH_2CH_3$, $-CH_3$, $-CH_2CHF_2$, $-CH_2CF_3$, and $-CH_2CH_2-N(CH_3)_2$.

In another embodiment, the compound of the invention is according to any one of Formula I-IVc, wherein L is $-CR^{5a}R^{5b}-$, or according to any one of Formula Va-VIc, wherein $R^{5a}$ is H and $R^{5b}$ is selected from H, $C_{1-4}$ alkoxy, and $C_{1-4}$ alkyl optionally substituted with one, two or three halo or one $-NR^{8a}R^{8b}$, wherein $R^{8a}$ and $R^{8b}$ are as defined previously. In a more particular embodiment, $R^{5a}$ is H and $R^{5b}$ is independently selected from H, $C_{1-4}$ alkoxy, and $C_{1-4}$ alkyl optionally substituted with one, two or three halo or one $-NR^{8a}R^{8b}$, wherein each $R^{8a}$ and $R^{8b}$ independently selected from H, $-CH_3$, or $-CH_2CH_3$. In a most particular embodiment, $R^{5a}$ is H and $R^{5b}$ is selected from H, $-OCH_3$, $-OCH_2CH_3$, $-CH_3$, $-CH_2CH_3$, $-CH_3$, - $CH_2CHF_2$, $-CH_2CF_3$, and $-CH_2CH_2-N(CH_3)_2$. In a further most particular embodiment, $R^{5a}$ is H and $R^{5b}$ is selected from $-CH_3$.

In one embodiment, the compound of the invention is according to Formula VIIa, VIIb, or VIIc:

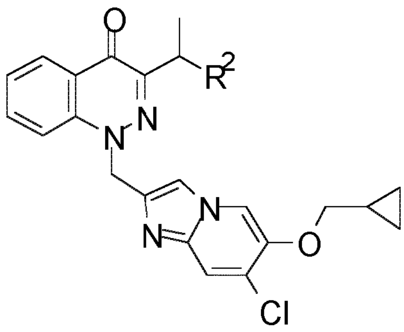

VIIa,

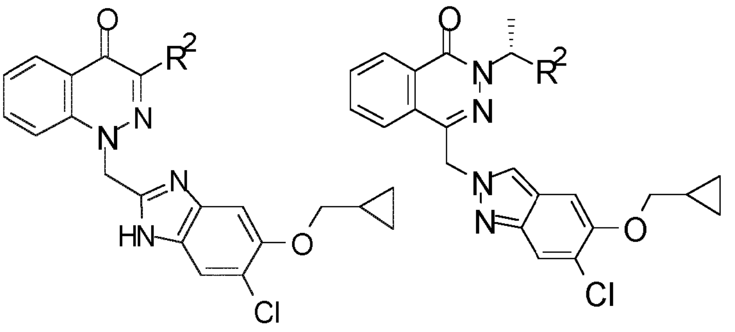

VIIb, or

VIIc.

wherein $R^2$ is as described above.

In one embodiment, the compound of the invention is according to any one of Formula I-VIIc, wherein $R^2$ is $-C(=O)OH$.

In one embodiment, the compound of the invention is according to any one of Formula I-VIIc, wherein $R^2$ is $-C(=O)NR^{6a}R^{6b}$, wherein each $R^{6a}$ and $R^{6b}$ is as previously defined. In a particular embodiment, one of $R^{6a}$ and $R^{6b}$ is H, and the other is as previously defined. In another particular embodiment, $R^{6a}$ and $R^{6b}$ are both H.

In one embodiment, the compound of the invention is according to any one of Formula I-VIIc, $R^2$ is $-C(=O)NR^{6a}R^{6b}$, wherein $R^{6b}$ is as previously described, and $R^{6a}$ is $C_{1-6}$ alkyl. In a particular embodiment, $R^{6b}$ is as

previously described, $R^{6b}$ is as previously described, and $R^{6a}$ is $-CH_3$, or-$CH_2CH_3$.

In one embodiment, the compound of the invention is according to any one of Formula I-VIc, $R^2$ is $-C(=O)NR^{6a}R^{6b}$, wherein $R^{6b}$ is as previously described, and $R^{6a}$ is $C_{1-6}$ alkyl substituted with one or more independently selected OH, CN, halo, $C_{1-4}$ alkoxy, $-S(O)_2C_{1-4}$ alkyl, $-S(O)_2NH_2$, or $-C(O)NR^{9a}R^{9b}$ wherein each $R^{9a}$ and $R^{9b}$ is independently selected from H, and $C_{1-4}$ alkyl. In a particular embodiment, $R^{6b}$ is as previously described, and $R^{6a}$ is $C_{1-6}$ alkyl substituted with one, two or three independently selected OH, CN, halo, $C_{1-4}$ alkoxy, $-S(O)_2C_{1-4}$ alkyl, $-S(O)_2NH_2$, or $-C(O)NR^{9a}R^{9b}$ wherein each $R^{9a}$ and $R^{9b}$ is independently selected from H, and $C_{1-4}$ alkyl. In a more particular embodiment, $R^{6b}$ is as previously described, and $R^{6a}$ is $-CH_3$, $-CH_2CH_3$, $-CH_2CH_2CH_3$, $-CH_2C(CH_3)_3$, $-CH_2CH_2CH_2CH_3$, $-CH_2CH_2C(CH_3)_2CH_3$, $-CH_2C(CH_3)_2CH_3$, each of which is substituted with one, two or three independently selected OH, CN, halo, $C_{1-4}$ alkoxy, $-S(O)_2C_{1-4}$ alkyl, $-S(O)_2NH_2$, or $-C(O)NR^{9a}R^{9b}$ wherein each $R^{9a}$ and $R^{9b}$ is independently selected from H, and $C_{1-4}$ alkyl. In another more particular embodiment, $R^{6b}$ is as previously described, and $R^{6a}$ is $C_{1-6}$ alkyl, each of which is substituted with one, two or three independently selected OH, CN, F, Cl, $-OCH_3$, $-OCH_2CH_3$, $-S(O)_2CH_3$, $-S(O)_2CH_2CH_3$, $-S(O)_2NH_2$, or $-C(O)NR^{9a}R^{9b}$ wherein each $R^{9a}$ and $R^{9b}$ is independently selected from H, $-CH_3$, and - $CH_2CH_3$. In a most particular embodiment, $R^{6b}$ is as previously described, and $R^{6a}$ is selected from:

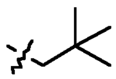 , 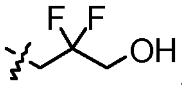 , 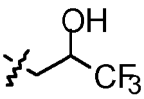 , 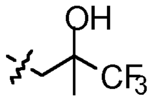 , 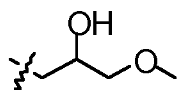 , 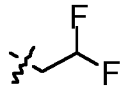 ,

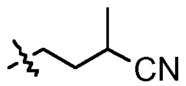 , 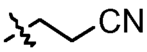 , 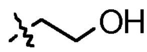 , 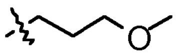 , 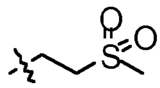 ,

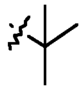 , 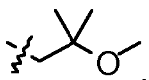 , 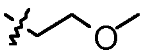 , 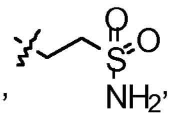 , 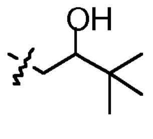 , 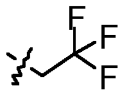 or 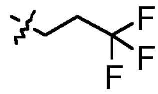 .

In one embodiment, the compound of the invention is according to any one of Formula I-VIIc, $R^2$ is $-C(=O)NR^{6a}R^{6b}$, wherein $R^{6a}$ is as previously described, and $R^{6b}$ is $C_{1-6}$ alkyl. In a particular embodiment, $R^{6b}$ is as previously described, $R^{6a}$ is as previously described, and $R^{6b}$ is $-CH_3$, or-$CH_2CH_3$.

In one embodiment, the compound of the invention is according to any one of Formula I-IVc, $R^2$ is $-C(=O)NR^{6a}R^{6b}$, wherein $R^{6a}$ is as previously described, and $R^{6b}$ is $C_{1-6}$ alkyl substituted with one or more independently selected OH, CN, halo, $C_{1-4}$ alkoxy, $-S(O)_2C_{1-4}$ alkyl, $-S(O)_2NH_2$, or $-C(O)NR^{9a}R^{9b}$ wherein each $R^{9a}$ and $R^{9b}$ is independently selected from H, and $C_{1-4}$ alkyl. In a particular embodiment, $R^{6a}$ is as previously described, and $R^{6b}$ is $C_{1-6}$ alkyl substituted with one, two or three independently selected OH, CN, halo, $C_{1-4}$ alkoxy, $-S(O)_2C_{1-4}$ alkyl, $-S(O)_2NH_2$, or $-C(O)NR^{9a}R^{9b}$ wherein each $R^{9a}$ and $R^{9b}$ is independently selected from H, and $C_{1-4}$ alkyl. In a more particular embodiment, $R^{6a}$ is as previously described, and $R^{6b}$ is $-CH_3$, $-CH_2CH_3$, $-CH_2CH_2CH_3$, $-CH_2C(CH_3)_3$, $-CH_2CH_2CH_2CH_3$, - $CH_2CH_2C(CH_3)_2CH_3$, $-CH_2C(CH_3)_2CH_3$, each of which is substituted with one, two or three independently selected OH, CN, halo, $C_{1-4}$ alkoxy, $-S(O)_2C_{1-4}$ alkyl, $-S(O)_2NH_2$, or $-C(O)NR^{9a}R^{9b}$ wherein each $R^{9a}$ and $R^{9b}$ is independently selected from H, and $C_{1-4}$ alkyl. In another more particular embodiment, $R^{6a}$ is as previously described, and $R^{6b}$ is $C_{1-6}$ alkyl, each of which is substituted with one, two or three independently selected OH, CN, F, Cl, $-OCH_3$, $-OCH_2CH_3$, $-S(O)_2CH_3$, $-S(O)_2CH_2CH_3$, $-S(O)_2NH_2$, or $-C(O)NR^{9a}R^{9b}$ wherein each $R^{9a}$ and $R^{9b}$ is independently selected from H, $-CH_3$, and - $CH_2CH_3$. In a most particular embodiment, $R^{6a}$ is as previously described, and $R^{6b}$ is selected from :

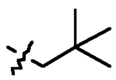 , 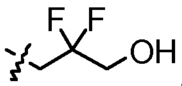 , 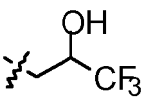 , 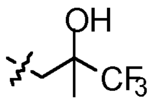 , 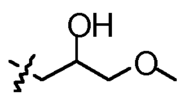 , 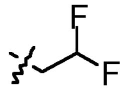 ,

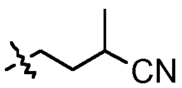 , 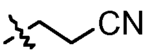 , 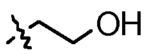 , 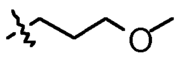 , 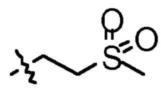 ,

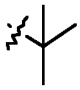, 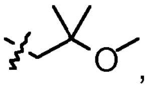, 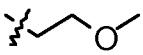, 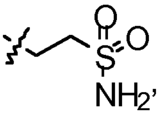 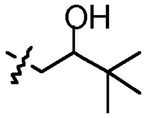, 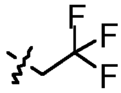 or 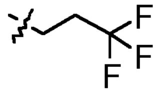

In one embodiment, the compound of the invention is according to any one of Formula I-VIIc, $R^2$ is $-C(=O)NR^{6a}R^{6b}$ wherein $R^{6b}$ is as previously described, and $R^{6a}$ is $C_{1-6}$ alkyl substituted with one or more independently selected monocyclic 4-6 membered heterocycloalkyl comprising one or two heteroatoms selected from N, O, and S, optionally substituted with one $-CH_2-OH$. In a particular embodiment, $R^{6b}$ is as previously described, and $R^{6a}$ is $C_{1-6}$ alkyl substituted with one monocyclic 4-6 membered heterocycloalkyl comprising one or two heteroatoms selected from N, O, and S, optionally substituted with one $-CH_2-OH$. In a more particular embodiment, $R^{6b}$ is as previously described, and $R^{6a}$ is $-CH_3$, $-CH_2CH_3$, each of which is substituted with one monocyclic 4-6 membered heterocycloalkyl comprising one or two heteroatoms selected from N, O, and S, optionally substituted with one $-CH_2-OH$. In another more particular embodiment, $R^{6b}$ is as previously described, and $R^{6a}$ is $C_{1-6}$ alkyl substituted with one oxetanyl, or tetrahydrofuranyl, each of which is optionally substituted with one $-CH_2-OH$. In a most particular embodiment, $R^{6b}$ is as previously described, and $R^{6a}$ is $-CH_3$, or $-CH_2CH_3$, each of which is substituted with one oxetanyl, or tetrahydrofuranyl, each of which is optionally substituted with one $-CH_2-OH$.

In one embodiment, the compound of the invention is according to any one of Formula I-VIIc, $R^2$ is $-C(=O)NR^{6a}R^{6b}$ wherein $R^{6a}$ is as previously described, and $R^{6b}$ is $C_{1-6}$ alkyl substituted with one or more independently selected monocyclic 4-6 membered heterocycloalkyl comprising one or two heteroatoms selected from N, O, and S, optionally substituted with one $-CH_2-OH$. In a particular embodiment, $R^{6a}$ is as previously described, and $R^{6b}$ is $C_{1-6}$ alkyl substituted with one monocyclic 4-6 membered heterocycloalkyl comprising one or two heteroatoms selected from N, O, and S, optionally substituted with one $-CH_2-OH$. In a more particular embodiment, $R^{6a}$ is as previously described, and $R^{6b}$ is $-CH_3$, $-CH_2CH_3$, each of which is substituted with one monocyclic 4-6 membered heterocycloalkyl comprising one or two heteroatoms selected from N, O, and S, optionally substituted with one $-CH_2-OH$. In another more particular embodiment, $R^{6a}$ is as previously described, and $R^{6b}$ is $C_{1-6}$ alkyl substituted with one oxetanyl, or tetrahydrofuranyl, each of which is optionally substituted with one $-CH_2-OH$. In a most particular embodiment, $R^{6a}$ is as previously described, and $R^{6b}$ is $-CH_3$, or $-CH_2CH_3$, each of which is substituted with one oxetanyl, or tetrahydrofuranyl, each of which is optionally substituted with one $-CH_2-OH$.

In one embodiment, the compound of the invention is according to any one of Formula I-VIIc, $R^2$ is $-C(=O)NR^{6a}R^{6b}$ wherein $R^{6b}$ is as previously described, and $R^{6a}$ is $C_{1-6}$ alkyl substituted with one or more independently selected $C_{3-7}$ cycloalkyl optionally substituted with one or more independently selected OH, or halo. In a particular embodiment, $R^{6b}$ is as previously described, and $R^{6a}$ is $C_{1-6}$ alkyl substituted with one $C_{3-7}$ cycloalkyl optionally substituted with one or more independently selected OH, or halo. In a more particular embodiment, $R^{6b}$ is as previously described, and $R^{6a}$ is $-CH_3$, $-CH_2CH_3$, each of which is substituted with one $C_{3-7}$ cycloalkyl optionally substituted with one or more independently selected OH, or halo. In another more particular embodiment, $R^{6b}$ is as previously described, and $R^{6a}$ is $C_{1-6}$ alkyl substituted with one cyclobutyl, cyclopentyl, each of which is optionally substituted with one or more independently selected OH, or halo. In a most particular embodiment, $R^{6b}$ is as previously described, and $R^{6a}$ is $-CH_3$, or $- CH_2CH_3$, each of which is substituted with one cyclobutyl, or cyclopentyl, each of which is optionally substituted with one or more independently selected OH, or halo. In a further most particular embodiment, $R^{6b}$ is as previously described, and $R^{6a}$ is $-CH_3$, or $-CH_2CH_3$, each of which is substituted with one cyclobutyl, or cyclopentyl, each of which is optionally substituted with one or more independently selected OH, or F.

In one embodiment, the compound of the invention is according to any one of Formula I-VIIc, $R^2$ is $-C(=O)NR^{6a}R^{6b}$ wherein $R^{6a}$ is as previously described, and $R^{6b}$ is $C_{1-6}$ alkyl substituted with one or more independently selected $C_{3-7}$ cycloalkyl optionally substituted with one or more independently selected OH, or halo. In a particular embodiment, $R^{6a}$ is as previously described, and $R^{6b}$ $C_{1-6}$ alkyl substituted with one $C_{3-7}$ cycloalkyl optionally substituted with one or more independently selected OH, or halo. In a more particular embodiment, $R^{6a}$ is as previously described, and $R^{6b}$ $-CH_3$, $-CH_2CH_3$, each of which is substituted with one $C_{3-7}$ cycloalkyl optionally substituted with one or more independently selected OH, or halo. In another more particular embodiment, $R^{6a}$ is as previously described, and $R^{6b}$ $C_{1-6}$ alkyl substituted with one cyclobutyl, cyclopentyl, each of which is optionally substituted with one or more independently selected OH, or halo. In a most particular embodiment, $R^{6a}$ is as previously described, and $R^{6b}$ $-CH_3$, or $-CH_2CH_3$, each of which is substituted with one cyclobutyl, or cyclopentyl, each of which is optionally substituted with one or more independently selected OH, or halo. In a further most particular embodiment, $R^{6a}$ is as previously described, and $R^{6b}$ $-CH_3$, or $-CH_2CH_3$, each of which is substituted with one cyclobutyl, or cyclopentyl, each of which is optionally substituted with one or more independently selected OH, or F.

In one embodiment, the compound of the invention is according to any one of Formula I-VIIc, $R^2$ is $-C(=O)NR^{6a}R^{6b}$ wherein $R^{6b}$ is as previously described, and $R^{6a}$ is $C_{3-7}$ cycloalkyl optionally substituted with one or

more OH. In a particular embodiment, $R^{6b}$ is as previously described, and $R^{6a}$ is cyclopropyl, cyclobutyl, or cyclopentyl, each of which is optionally substituted with one or more OH. In a particular embodiment, $R^{6b}$ is as previously described, and $R^{6a}$ is cyclopropyl, cyclobutyl, or cyclopentyl, each of which is optionally substituted with one OH.

In one embodiment, the compound of the invention is according to any one of Formula I-VIIc, $R^2$ is -C(=O)$NR^{6a}R^{6b}$ wherein $R^{6a}$ is as previously described, and $R^{6b}$ is $C_{3-7}$ cycloalkyl optionally substituted with one or more OH. In a particular embodiment, $R^{6a}$ is as previously described, and $R^{6b}$ is cyclopropyl, cyclobutyl, or cyclopentyl, each of which is optionally substituted with one or more OH. In a particular embodiment, $R^{6a}$ is as previously described, and $R^{6b}$ is cyclopropyl, cyclobutyl, or cyclopentyl, each of which is optionally substituted with one OH.

In one embodiment, the compound of the invention is according to any one of Formula I-VIIc, $R^2$ is -C(=O)$NR^{6a}R^{6b}$ wherein $R^{6b}$ is as previously described, and $R^{6a}$ is monocyclic 4-6 membered heterocycloalkyl comprising one or two heteroatoms selected from N, O, and S, optionally substituted with one or two oxo. In a particular embodiment, $R^{6b}$ is as previously described, and $R^{6a}$ is oxetanyl, tetrahydrofuranyl, tetrahydropyranyl, or tetrahydrothiopyranyl, each of which is optionally substituted with one or two oxo. In a particular embodiment, $R^{6b}$ is as previously described, and $R^{6a}$ is oxetanyl, tetrahydrofuranyl, or tetrahydropyranyl.

In one embodiment, the compound of the invention is according to any one of Formula I-VIIc, $R^2$ is -C(=O)$NR^{6a}R^{6b}$ wherein $R^{6a}$ is as previously described, and $R^{6b}$ is monocyclic 4-6 membered heterocycloalkyl comprising one or two heteroatoms selected from N, O, and S, optionally substituted with one or two oxo. In a particular embodiment, $R^{6a}$ is as previously described, and $R^{6b}$ is oxetanyl, tetrahydrofuranyl, tetrahydropyranyl, or tetrahydrothiopyranyl, each of which is optionally substituted with one or two oxo. In a particular embodiment, $R^{6a}$ is as previously described, and $R^{6b}$ is oxetanyl, tetrahydrofuranyl, or tetrahydropyranyl.

In one embodiment, the compound of the invention is according to any one of Formula I-VIIc, wherein $R^2$ is -C(O)NHS(O)$_2$-$C_{1-4}$ alkyl. In a particular embodiment, $R^2$ is -C(O)NHS(O)$_2$-$CH_3$.

In one embodiment, the compound of the invention is according to any one of Formula I-VIIc, wherein $R^2$ is -C(O)NHS(O)$_2$-$C_{3-7}$ cycloalkyl. In a particular embodiment, $R^2$ is -C(O)NHS(O)$_2$-cyclopropyl.

In one embodiment, the compound of the invention is according to any one of Formula I-VIIc, wherein $R^2$ is -$Cy_1$. In a particular embodiment, $Cy_1$ is $C_{3-7}$ monocyclic cycloalkyl, optionally substituted with one -C(=O)OH. In a more particular embodiment, $Cy_1$ is cyclopropyl, cyclobutyl, or cyclopentyl, each of which is optionally substituted with one -C(=O)OH. In a most particular embodiment, $Cy_1$ is cyclopropyl or cyclobutyl. In a further most particular embodiment, $Cy_1$ is

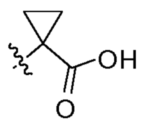 or 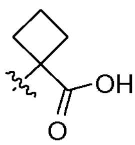.

In one embodiment, the compound of the invention is according to any one of Formula I-VIIc, wherein $R^2$ is -$Cy_1$ is monocyclic 4-6 membered heterocycloalkyl comprising one or two heteroatoms selected from N, O, and S, optionally substituted with one or two independently selected $C_{1-4}$ alkyl which alkyl is optionally substituted with one -C(=O)OH. In a particular embodiment, $Cy_1$ is azetidinyl, oxetanyl, pyrolidinyl, dioxolanyl, tetrahydrofuranyl, piperidinyl, piperazinyl, morpholinyl, or tetrahydropyranyl, each of which is optionally substituted with one or two independently selected $C_{1-4}$ alkyl which alkyl is optionally substituted with one -C(=O)OH. In another particular embodiment, $Cy_1$ is monocyclic 4-6 membered heterocycloalkyl comprising one or two heteroatoms selected from N, O, and S, optionally substituted with one or two independently selected -$CH_3$, -$CH_2CH_3$, or -$CH_2$C(=O)OH. In a most particular embodiment, $Cy_1$ is azetidinyl, oxetanyl, pyrolidinyl, dioxolanyl, tetrahydrofuranyl, piperidinyl, piperazinyl, morpholinyl, or tetrahydropyranyl, each of which is optionally substituted with one or two independently selected -$CH_3$, -$CH_2CH_3$, or -$CH_2$C(=O)OH.

In one embodiment, the compound of the invention is according to any one of Formula I-VIIc, wherein $R^2$ is -C(=O)$Cy_2$ and $Cy_2$ is N-linked monocyclic 4-7 membered heterocycloalkyl comprising at least one N atom, and optionally one or two heteroatoms selected from N, O, and S. In a particular embodiment, $Cy_2$ is azetidinyl, pyrolidinyl, piperidinyl, piperazinyl, or morpholinyl. In a more particular embodiment, $Cy_2$ is morpholinyl.

In one embodiment, the compound of the invention is according to any one of Formula I-VIIc, wherein $R^2$ is -C(=O)$Cy_2$ and $Cy_2$ is N-linked monocyclic 4-7 membered heterocycloalkyl comprising at least one N atom, and optionally one or two heteroatoms selected from N, O, and S, which heterocycloalkyl is substituted with one or more independently selected OH, oxo, -CN, halo, $C_{1-4}$ alkoxy, $C_{1-4}$ alkyl, $C_{1-4}$ alkyl substituted with one or more independently selected halo or OH, $C_{3-7}$ cycloalkyl, -S(O)$_2$$C_{1-4}$ alkyl, or -$NR^{7a}R^{7b}$ wherein $R^{7a}$ and $R^{7b}$ are as previously described. In a particular embodiment, $Cy_2$ is N-linked monocyclic 4-7 membered heterocycloalkyl comprising at least one N atom, and optionally one or two heteroatoms selected from N, O, and S, which heterocycloalkyl is substituted with one, two or three independently selected OH, oxo, -CN, halo, $C_{1-4}$ alkoxy, $C_{1-4}$ alkyl, $C_{1-4}$ alkyl substituted with one or more independently

selected halo or OH, $C_{3-7}$ cycloalkyl, $-S(O)_2C_{1-4}$ alkyl, or $-NR^{7a}R^{7b}$ wherein $R^{7a}$ and $R^{7b}$ are as previously described. In a more particular embodiment, $Cy_2$ is azetidinyl, pyrolidinyl, piperidinyl, piperazinyl, or morpholinyl, each of which is substituted with one, two or three independently selected OH, oxo, -CN, halo, $C_{1-4}$ alkoxy, $C_{1-4}$ alkyl, $C_{1-4}$ alkyl substituted with one or more independently selected halo or OH, $C_{3-7}$ cycloalkyl, $-S(O)_2C_{1-4}$ alkyl, or $-NR^{7a}R^{7b}$ wherein $R^{7a}$ and $R^{7b}$ are as previously described. In another more particular embodiment, $Cy_2$ is N-linked monocyclic 4-7 membered heterocycloalkyl comprising at least one N atom, and optionally one or two heteroatoms selected from N, O, and S, which heterocycloalkyl is substituted with one, two or three independently selected OH, oxo, -CN, F, Cl, $-OCH_3$, $-OCH_2CH_3$, $-CH_3$, $-CH_2CH_3$, $-CH_2OH$, $-C(CH_3)_2OH$, $-CF_3$, $-CH_2CF_3$, cyclopropyl, cyclopropyl, - $S(O)_2CH_3$, $-S(O)_2CH_2CH_3$, $-NH_2$, $-NHCH_3$, or $-N(CH_3)_2$. In most particular embodiment, $Cy_2$ is azetidinyl, pyrolidinyl, piperidinyl, piperazinyl, or morpholinyl, each of which is substituted with one, two or three independently selected OH, oxo, -CN, F, Cl, $-OCH_3$, $-OCH_2CH_3$, $-CH_3$, $-CH_2CH_3$, $-CH_2OH$, $-C(CH_3)_2OH$, $-CF_3$, $-CH_2CF_3$, cyclopropyl, cyclopropyl, $-S(O)_2CH_3$, $-S(O)_2CH_2CH_3$, $-NH_2$, $-NHCH_3$, or $-N(CH_3)_2$.

In one embodiment, the compound of the invention is according to any one of Formula I-VIIc, wherein $R^2$ is $-C(=O)Cy_2$ and $Cy_2$ is N-linked spirocyclic 7-9 membered hctcrocycloalkyl comprising at least one N atom, and optionally one or two heteroatoms selected from N, O, and S, optionally substituted with one or more halo. In a particular embodiment, $Cy_2$ is 7-Oxa-2-aza-spiro[3.5]nonanyl, or 5-Aza-spiro[2.4]heptane, each of which is optionally substituted with one or more halo. In a particular embodiment, $Cy_2$ is 7-Oxa-2-aza-spiro[3.5]nonanyl, 5-Aza-spiro[2.4]heptane, each of which is optionally substituted with one or more F.

In one embodiment, the compound according to Formula I is selected from: 6-(2,2-difluoroethoxy)-2-[[4-oxo-3-(pyrrolidin-2-ylmethyl)phthalazin-1-yl]methyl]-3H-benzimidazole-5-carbonitrile, 2-(azetidin-3-yl)-4-[[6-chloro-5-(cyclopropylmethoxy)indazol-2-yl]methyl]phthalazin-1-one, 2-[4-[[6-chloro-5-(cyclopropylmethoxy)indazol-2-yl]methyl]-1-oxo-phthalazin-2-yl]propanoic acid, 2-[1-[[7-chloro-6-(cyclopropylmethoxy)imidazo[1,2-a]pyridin-2-yl]methyl]-4-oxo-cinnolin-3-yl]propanoic acid, 1-[[6-chloro-5-(cyclopropylmethoxy)-1H-benzimidazol-2-yl]methyl]-4-oxo-cinnoline-3-carboxylic acid, 2-[4-[(5-chloro-6-ethoxy-1H-benzimidazol-2-yl)methyl]-1-oxo-phthalazin-2-yl]acetic acid, 2-[4-[[6-cyano-5-(cyclopropylmethoxy)-1H-benzimidazol-2-yl]methyl]-1-oxo-phthalazin-2-yl]acetic acid, 2-[4-[[6-cyano-5-(2,2-difluoroethoxy)indazol-2-yl]methyl]-1-oxo-phthalazin-2-yl]acetic acid, 2-[4-[[5-chloro-6-(2,2-difluoroethoxy)-1H-indol-2-yl]methyl]-1-oxo-phthalazin-2-yl]acetic acid, 6-(cyclopropylmethoxy)-2-[[3-[2-[(3S,4S)-3,4-dihydroxypyrrolidin-1-yl]-2-oxo-ethyl]-4-oxo-cinnolin-1-yl]methyl]-3H-benzimidazole-5-carbonitrile, 4-[[5-chloro-6-(2,2-difluoroethoxy)-1H-indol-2-yl]methyl]-2-(2-morpholino-2-oxo-ethyl)phthalazin-1-one, 4-[[6-chloro-5-(2,2-difluoroethoxy)-1H-benzimidazol-2-yl]methyl]-2-(2-morpholino-2-oxo-ethyl)-1-oxo-phthalazine-6-carboxamide, 6-(2,2-difluoroethoxy)-2-[[3-[2-[(3S)-3-hydroxypyrrolidin-1-yl]-2-oxo-ethyl]-4-oxo-phthalazin-1-yl]methyl]-3H-benzimidazole-5-carbonitrile, 2-[(1-acetylpyrrolidin-2-yl)methyl]-4-[[5-chloro-6-(trifluoromethoxy)-1H-benzimidazol-2-yl]methyl]phthalazin-1-one, 2-[1-[[7-chloro-6-(cyclopropylmethoxy)imidazo[1,2-a]pyridin-2-yl]methyl]-4-oxo-cinnolin-3-yl]-N-methylsulfonyl-propanamide, 2-[1-[[5-chloro-6-(cyclopropylmethoxy)-1H-indol-2-yl]methyl]-4-oxo-cinnolin-3-yl]acetic acid, ((2R)-2-[4-[[6-chloro-5-(cyclopropylmethoxy)indazol-2-yl]methyl]-1-oxo-phthalazin-2-yl]propanoic acid), (2S)-2-[4-[[6-chloro-5-(cyclopropylmethoxy)indazol-2-yl]methyl]-1-oxo-phthalazin-2-yl]propanoic acid, (2S)-2-[1-[[7-chloro-6-(cyclopropylmethoxy)imidazo[1,2-a]pyridin-2-yl]methyl]-4-oxocinnolin-3-yl]propanoic acid, (2R)-2-[1-[[7-chloro-6-(cyclopropylmethoxy)imidazo[1,2-a]pyridin-2-yl]methyl]-4-oxocinnolin-3-yl]propanoic acid, (2-[1-[[5-cyano-6-(cyclopropylmethoxy)-1H-benzimidazol-2-yl]methyl]-4-oxocinnolin-3-yl]-3-(dimethylamino)propanoic acid), 2-[4-[(7-chloro-6-ethoxy-indazol-2-yl)methyl]-1-oxo-phthalazin-2-yl]acetic acid, 2-[4-[[5-chloro-6-(2,2-difluoroethoxy)-1H-benzimidazol-2-yl]methyl]-6-cyano-1-oxo-phthalazin-2-yl]acetic acid, 4-[[5-chloro-6-(trifluoromethoxy)-1H-benzimidazol-2-yl]methyl]-2-cyclopentyl-phthalazin-1-one, 4-[[5-chloro-6-(trifluoromethoxy)-1H-benzimidazol-2-yl]methyl]-2-cyclopropyl-phthalazin-1-one, 4-[[5-chloro-6-(trifluoromethoxy)-1H-benzimidazol-2-yl]methyl]-2-cyclobutyl-phthalazin-1-one, 4-[[6-chloro-5-(trifluoromethoxy)-1H-benzimidazol-2-yl]methyl]-2-(cyclopropylmethyl)phthalazin-1-one, 4-[[6-chloro-5-(trifluoromethoxy)-1H-benzimidazol-2-yl]methyl]-2-(cyclobutylmethyl)phthalazin-1-one, 4-[[5-chloro-6-(2,2-difluoroethoxy)-1H-benzimidazol-2-yl]methyl]-6-methoxy-2-(2-morpholino-2-oxoethyl)phthalazin-1-one, 4-[[5-chloro-6-(2,2-difluoroethoxy)-1H-benzimidazol-2-yl]methyl]-7-methoxy-2-(2-morpholino-2-oxoethyl)phthalazin-1-one, 4-[[5-chloro-6-(2,2-difluoroethoxy)-1H-benzimidazol-2-yl]methyl]-2-cyclopropyl-phthalazin-1-one, 4-[[5-chloro-6-(2,2-difluoroethoxy)-1H-benzimidazol-2-yl]methyl]-7-morpholino-2-(2-morpholino-2-oxo-ethyl)phthalazin-1-one, 4-[[6-chloro-5-(trifluoromethoxy)-1H-benzimidazol-2-yl]methyl]-2-(THF-2-ylmethyl)phthalazin-1-one, 4-[[6-chloro-5-(trifluoromethoxy)-1H-benzimidazol-2-yl]methyl]-2-(tetrahydropyran-2-ylmethyl)phthalazin-1-one, 4-[[6-chloro-5-(trifluoromethoxy)-1H-benzimidazol-2-yl]methyl]-2-[(3-methyloxetan-3-yl)methyl]phthalazin-1-one, 4-[[6-chloro-5-(trifluoromethoxy)-1H-benzimidazol-2-yl]methyl]-2-[(2,2-dimethyl-1,3-dioxolan-4-yl)methyl]phthalazin-1-one, 4-[[6-chloro-5-(trifluoromethoxy)-1H-benzimidazol-2-yl]methyl]-2-[(1-methyl-3-piperidyl)methyl]phthalazin-1-one, 1-[[5-chloro-6-(2,2-difluoroethoxy)-1H-benzimidazol-2-yl]methyl]-3-(2-morpholino-2-oxo-ethyl)-4-oxo-phthalazine-6-carbonitrile, 4-[[5-chloro-6-(2,2-difluoroethoxy)-1H-benzimidazol-2-yl]methyl]-6-methylsulfonyl-2-(2-morpholino-2-oxo-ethyl)phthalazin-1-one, 6-(cyclopropylmethoxy)-2-[(3-cyclopropyl-4-oxo-phthalazin-1-yl)methyl]-1H-benzimidazole-5-carbonitrile, 4-[[6-chloro-5-(trifluoromethoxy)-1H-benzimidazol-2-yl]methyl]-2-[(2,2-difluorocyclopropyl)methyl]phthalazin-1-one, 4-[[6-chloro-5-(trifluoromethoxy)-1H-benzimidazol-2-yl]methyl]-2-(3,3-dimethyl-2-oxobutyl)phthalazin-1-one, 1-[[4-[[5-chloro-6-(trifluoromethoxy)-1H-benzimidazol-2-yl]methyl]-1-oxo-phthalazin-2-yl]methyl]cyclopropanecarboxylic acid, 4-[[6-chloro-5-(2,2-difluoroethoxy)-1H-benzimidazol-2-yl]methyl]-5-cyclopropyl-2-(2-morpholino-2-oxo-ethyl)phthalazin-1-one, 6-(2,2-Difluoro-ethoxy)-2-[3-(2-morpholin-4-yl-2-oxo-ethyl)-4-oxo-4H-cinnolin-1-ylmethyl]-3H-enzoimidazole-5 -carbonitrile, 1-[5-(2,2-Difluoro-ethoxy)-6-fluoro, -1H-benzoimidazol-2-ylmethyl]-3-(2-morpholin-4-yl-2-oxo-ethyl)-1H-cinnolin-4-one, 6-(2-methoxyethoxy)-2-[[3-(2-morpholino-2-oxo-ethyl)-4-oxo-phthalazin-1-yl]methyl]-3H-benzimidazole-5-carbonitrile, 6-(2,2-difluoropropoxy)-2-[[3-(2-morpholino-2-oxo-ethyl)-4-oxo-phthalazin-1-yl]methyl]-3H-benzimidazole-5-carbonitrile, 7-chloro-6-(2,2-difluoroethoxy)-2-[[3-(2-morpholino-2-oxo-ethyl)-4-oxo-phthalazin-1-yl]methyl]-3H-benzimidazole-5-carbonitrile, 2-[[8-cyclopropyl-3-(2-morpholino-2-oxo-ethyl)-4-oxo-phthalazin-1-yl]methyl]-6-(2,2-difluoroethoxy)-3H-benzimidazole-5-carbonitrile, 4-[[6-chloro-5-(trifluoromethoxy)-1H-benzimidazol-2-yl]methyl]-2-(pyrrolidin-2-ylmethyl)phthalazin-1-one, 4-[[6-chloro-5-(trifluoromethoxy)-1H-benzimidazol-2-yl]methyl]-2-(4-piperidylmethyl)phthalazin-1-one, 4-[[6-chloro-5-(trifluoromethoxy)-1H-benzimidazol-2-yl]methyl]-2-(pyrrolidin-3-ylmethyl)phthalazin-1-one, 4-[[6-chloro-5-(trifluoromethoxy)-1H-benzimidazol-2-yl]methyl]-2-(3-piperidylmethyl)phthalazin-1-one, 4-[[6-chloro-5-(trifluoromethoxy)-1H-benzimidazol-2-yl]methyl]-2-pyrrolidin-3-yl-phthalazin-1-one, 2-(azetidin-3-ylmethyl)-4-[[6-chloro-5-(trifluoromethoxy)-1H-benzimidazol-2-yl]methyl]phthalazin-1-one, 4-[[6-chloro-5-(trifluoromethoxy)-1H-benzimidazol-2-yl]methyl]-2-(4-piperidyl)phthalazin-1-one, 4-[[5-chloro-6-(trifluoromethoxy)-1H-benzimidazol-2-yl]methyl]-2-(morpholin-2-ylmethyl)phthalazin-1-one, 6-(2,2-difluoroethoxy)-2-[(4-oxo-3-pyrrolidin-3-yl-phthalazin-1-yl)methyl]-3H-benzimidazole-5-carbonitrile, 6-(2-methoxyethoxy)-2-[(4-oxo-3-pyrrolidin-3-yl-phthalazin-1-yl)methyl]-3H-benzimidazole-5-carbonitrile, 6-(2,2-difluoroethoxy)-2-[[8-methyl-3-(2-morpholino-2-oxo-ethyl)-4-oxo-phthalazin-1-yl]methyl]-3H-benzimidazole-5-carbonitrile, 4-[[6-chloro-5-(2-methoxyethoxy)-1H-benzimidazol-2-yl]methyl]-2-pyrrolidin-3-yl-phthalazin-1-one, 2-[[3-(azetidin-3-yl)-4-oxo-phthalazin-1-yl]methyl]-6-(2,2-difluoroethoxy)-3H-benzimidazole-5-carbonitrile, 2-[3-[4-[[6-cyano-5-(2,2-difluoroethoxy)-1H-benzimidazol-2-yl]methyl]-1-oxo-phthalazin-2-yl]azetidin-1-yl]acctic acid, 2-[2-[[4-[[6-cyano-5-(2,2-difluoroethoxy)-1H-benzimidazol-2-yl]methyl]-1-oxo-phthalazin-2-yl]methyl]pyrrolidin-1-yl]acetic acid, 2-[3-[4-[[6-cyano-5-(2,2-difluoroethoxy)-1H-benzimidazol-2-yl]methyl]-1-oxo-phthalazin-2-yl]pyrrolidin-1-yl]acetic acid, 6-(2,2-difluoroethoxy)-2-[[3-(1-methylpyrrolidin-3-yl)-4-oxo-phthalazin-1-yl]methyl]-3H-benzimidazole-5-carbonitrile, 2-[3-[4-[[7-cyano-6-(cyclopropylmethoxy)imidazo[1,2-a]pyridin-2-yl]methyl]-1-oxo-phthalazin-2-yl]azetidin-1-yl] acetic acid, 2-[4-[[6-cyano-5-(2,2-difluoroethoxy)-1H-benzimidazol-2-yl]methyl]-l-oxo-phthalazin-2-yl]-3-(dimethylamino)propanoic acid, 3-(azetidin-1-yl)-2- [4-[[6-cyano-5-(2,2-difluoroethoxy)-1H-benzimidazol-2-yl]methyl] -1 -oxo-phthalazin-2-yl]propanoic acid, 2-[4-[[6-chloro-5-(2,2-difluoroethoxy)indazol-2-yl]methyl]-1-oxo-phthalazin-2-yl]propanoic acid, 2-[4-[(6-chloro-5-ethoxy-1H-benzimidazol-2-yl)methyl]-8-methyl-1-oxo-phthalazin-2-yl]acetic acid, {1-[6-Chloro-5-(2,2-difluoro-ethoxy)-1H-benzoimidazol-2-ylmethyl]-4-oxo-1,4-dihydro-cinnolin-3-yl}-acetic acid, 2-[4-[[7-cyano-6-(cyclopropylmethoxy)imidazo[1,2-a]pyridin-2-yl]methyl]-1-oxo-phthalazin-2-yl]acetic acid, 1-[[6-cyano-5-(2,2-difluoroethoxy)-1H-benzimidazol-2-yl]methyl]-4-oxo-quinoline-3-carboxylic acid, 2-[4-[[7-cyano-6-(cyclopropylmethoxy)imidazo[1,2-a]pyridin-2-yl]methyl]-1-oxo-phthalazin-2-yl]propanoic acid, 2-[4-[[7-chloro-6-(cyclopropylmethoxy)imidazo[1,2-a]pyridin-2-yl]methyl]-1-oxo-phthalazin-2-yl]acetic acid, 2-[4-[[7-chloro-6-(cyclopropylmethoxy)imidazo[1,2-a]pyridin-2-yl]methyl]-l-oxo-phthalazin-2-yl]propanoic acid, 1-[[7-chloro-6-(cyclopropylmethoxy)imidazo[1,2-a]pyridin-2-yl]methyl]-4-oxo-quinoline-3-carboxylic acid, 1-[[7-chloro-6-(cyclopropylmethoxy)imidazo[1,2-a]pyridin-2-yl]methyl]-4-oxo-cinnoline-3-carboxylic acid, 2-[4-[[7-chloro-6-(cyclopropylmethoxy)imidazo[1,2-a]pyridin-2-yl]methyl]-6-fluoro-1-oxo-phthalazin-2-yl]propanoic acid, 2-[3-[4-[[7-chloro-6-(cyclopropylmethoxy)imidazo[1,2-a]pyridin-2-yl]methyl]-1-oxo-phthalazin-2-yl]pyrrolidin-1-yl]acetic acid, 2-[1-[[7-chloro-6-(cyclopropylmethoxy)imidazo[1,2-a]pyridin-2-yl]methyl]-4-oxo-cinnolin-3-yl]-2-methyl-propanoic acid, 2-[1-[[7-chloro-6-(cyclopropylmethoxy)imidazo[1,2-a]pyridin-2-yl]methyl]-4-oxo-cinnolin-3-yl]acetic acid, 1-[[6-chloro-5-(cyclopropylmethoxy)-1H-benzimidazol-2-yl]methyl]-4-oxo-quinoline-3-carboxylic acid, 2-[4-[[6-chloro-5-(cyclopropylmethoxy)indazol-2-yl]methyl]-1-oxo-phthalazin-2-yl]cyclobutanecarboxylic acid, 2-[4-[[6-chloro-5-(cyclopropylmethoxy)indazol-2-yl]methyl]-6-cyano-1-oxo-phthalazin-2-yl]propanoic acid, 2-[4-[[6-chloro-5-(cyclopropylmethoxy)indazol-2-yl]methyl]-1-oxo-5,6,7,8-tetrahydrophthalazin-2-yl]propanoic acid, 2-[1-[[7-chloro-6-(cyclopropylmethoxy)imidazo[1,2-a]pyridin-2-yl]methyl]-4-oxo-cinnolin-3-yl]-2-methoxy-acetic acid, 1-[4-[[6-chloro-5-(cyclopropylmethoxy)indazol-2-yl]methyl]-1-oxo-phthalazin-2-yl]cyclopropanecarboxylic acid, 1-[1-[[7-chloro-6-(cyclopropylmethoxy)imidazo[1,2-a]pyridin-2-yl]methyl]-4-oxo-cinnolin-3-yl]cyclopropanecarboxylic acid, 2-[4-[[6-chloro-5-(cyclopropylmethoxy)indazol-2-yl]methyl]-7-methoxy-1-oxo-phthalazin-2-yl]propanoic acid, 2-[1-[[7-chloro-6-(cyclopropylmethoxy)imidazo[1,2-a]pyridin-2-yl]methyl]-4-oxo-cinnolin-3-yl]butanoic acid, 2-[1-[[6-(cyclopropylmethoxy)-7-(trifluoromethyl)imidazo[1,2-a]pyridin-2-yl]methyl]-4-oxo-cinnolin-3-yl]acetic acid, 2-[1-[[6-(cyclopropylmethoxy)-7-(trifluoromethyl)imidazo[1,2-a]pyridin-2-yl]methyl]-4-oxo-cinnolin-3-yl]propanoic acid, 2-[1-[[7-chloro-6-(cyclopropylmethoxy)imidazo[1,2-a]pyridin-2-yl]methyl]-7-fluoro-4-oxo-cinnolin-3-yl]acetic acid, 2-[1-[[7-chloro-6-(cyclopropylmethoxy)imidazo[1,2-a]pyridin-2-yl]methyl]-5-methyl-4-oxo-cinnolin-3-yl]acetic acid, 2-[1-[[6-(cyclopropylmethoxy)-7-(trifluoromethyl)imidazo[1,2-a]pyridin-2-yl]methyl]-4-oxo-cinnolin-3-yl]butanoic acid, (1-[[5-(cyclopropylmethoxy)-6-(trifluoromethyl)-1H-benzimidazol-2-yl]methyl]-4-oxocinnoline-3-carboxylic acid), 2-[1-[[7-chloro-6-(cyclopropylmethoxy)imidazo[1,2-a]pyridin-2-yl]methyl]-4-oxo-5,6,7,8-tetrahydrocinnolin-3-yl]acetic acid, (1-[[5-(cyclopropylmethoxy)-6-(trifluoromethyl)-1H-benzimidazol-2-yl]methyl]-4-oxoquinoline-3-carboxylic acid), 2-[1-[[6-cyano-5-(cyclopropylmethoxy)-1H-benzimidazol-2-yl]methyl]-4-oxo-cinnolin-3-yl]propanoic acid,

2-[1-[[6-cyano-5-(cyclopropylmethoxy)-1H-benzimidazol-2-yl]methyl]-4-oxo-cinnolin-3-yl]acetic acid, 2-[1-[[6-bromo-5-(cyclopropylmethoxy)-1H-benzimidazol-2-yl]methyl]-4-oxo-cinnolin-3-yl]acetic acid, 1-[[5-cyano-6-(cyclopropylmcthoxy)-1H-benzimidazol-2-yl]mcthyl]-4-oxo-cinnoline-3-carboxylic acid, 2-[4-[[6-bromo-5-(cyclopropylmethoxy)indazol-2-yl]methyl]-1-oxo-phthalazin-2-yl]acetic acid, 2-[4-[[6-chloro-5-(trifluoromethoxy)-1H-benzimidazol-2-yl]methyl]-1-oxo-phthalazin-2-yl]acetic acid, 2-[4-[[5-chloro-6-(trifluoromethoxy)-1H-benzimidazol-2-yl]methyl]-1-oxo-5,6,7,8-tetrahydrophthalazin-2-yl]acetic acid, 2-[4-[[6-chloro-5-(cyclopropylmethoxy)indazol-2-yl]methyl]-1-oxo-phthalazin-2-yl]acetic acid, 2-[4-[[5-chloro-6-(cyclopropylmethoxy)-1H-benzimidazol-2-yl]methyl]-1-oxo-phthalazin-2-yl]acetic acid, 2-[4-[[5-chloro-6-(cyclopropylmethoxy)-7-fluoro-1H-benzimidazol-2-yl]methyl]-1-oxo-phthalazin-2-yl]acetic acid, 2-[4-[[5-chloro-6-(cyclopropylmethoxy)-1H-benzimidazol-2-yl]methyl]-1-oxo-5,6,7,8-tetrahydrophthalazin-2-yl] acetic acid, 2-[4-[[5-chloro-6-(2,2-difluoroethoxy)-1H-benzimidazol-2-yl]methyl]-1-oxo-phthalazin-2-yl]acetic acid, 2-[4-[[5-chloro-6-(2,2-difluoroethoxy)-1H-benzimidazol-2-yl]methyl]-1-oxo-5,6,7,8-tetrahydrophthalazin-2-yl] acetic acid, 2-[4-[(5-chloro-6-methoxy-1H-benzimidazol-2-yl)methyl]-1-oxo-5,6,7,8-tetrahydrophthalazin-2-yl]acetic acid, 2-[4-[(5-chloro-6-ethoxy-1H-benzimidazol-2-yl)methyl]-1-oxo-5,6,7,8-tetrahydrophthalazin-2-yl]acetic acid, 2-[4-(6-Chloro-5-cyclopropylmethoxy-1H-benzoimidazol-2-ylmethyl)-1-oxo-1H-phthalazin-2-yl]-propionic acid, 2-[4-[[5-(cyclopropylmethoxy)-6-(trifluoromethyl)-1H-benzimidazol-2-yl]methyl]-1-oxo-phthalazin-2-yl]acetic acid, 2-[4-[[5-(cyclopropylmethoxy)-6-fluoro-1H-benzimidazol-2-yl]methyl]-1-oxo-phthalazin-2-yl]acetic acid, {4-[5-Chloro-6-(2,2-difluoro-ethoxy)-1H-benzoimidazol-2-ylmethyl]-1-oxo-1H-isoquinolin-2-yl}-acetic acid, 2-[4-[[6-chloro-5-(2,2-difluoroethoxy)-1H-benzimidazol-2-yl]methyl]-5,8-difluoro-1-oxo-phthalazin-2-yl]acetic acid, 2-[4-[[5-ethoxy-6-(trifluoromethyl)-1H-benzimidazol-2-yl]methyl]-1-oxo-phthalazin-2-yl]acetic acid, 2-[4-[(5-chloro-6-ethoxy-3-oxo-1H-indazol-2-yl)methyl]-1-oxo-phthalazin-2-yl]acetic acid, 2-[4-[(5-ethoxy-6-fluoro-1H-benzimidazol-2-yl)methyl]-1-oxo-phthalazin-2-yl]acetic acid, 2- [4-[[5-(2,2-difluoroethoxy)-6-fluoro-1H-benzimidazol-2-yl]methyl]-1-oxo-phthalazin-2-yl]acetic acid, 2-[4-[[5-chloro-6-[(1-methylcyclopropyl)methoxy]-1H-benzimidazol-2-yl]methyl]-1-oxo-phthalazin-2-yl]acetic acid, 2-[4-[[5-chloro-6-(2,2-dimethylpropoxy)-1H-benzimidazol-2-yl]methyl]-1-oxo-phthalazin-2-yl]acetic acid, 2-[4-[[5-chloro-6-[(1-cyanocyclopropyl)methoxy]-1H-benzimidazol-2-yl]methyl]-1-oxo-phthalazin-2-yl]acctic acid, 2-[4-[[5-chloro-6-(cyclopropylmethoxy)-3-oxo-1H-indazol-2-yl]methyl]-1-oxo-phthalazin-2-yl]acetic acid, 2-[4-[[6-cyano-5-(cyclopropylmethoxy)indazol-2-yl]methyl]-1-oxo-phthalazin-2-yl]acetic acid, 2-[4-[(6-chloro-5-methoxy-1H-benzimidazol-2-yl)methyl]-1-oxo-phthalazin-2-yl]acetic acid, 2-[4-[(6-bromo-5-ethoxy-indazol-2-yl)methyl]-1-oxo-phthalazin-2-yl]acetic acid, 2-[4-[(5-cyano-6-ethoxy-1H-benzimidazol-2-yl)methyl]-1-oxo-phthalazin-2-yl]acetic acid, 2-[4-[[5-cyano-6-(2,2-difluoroethoxy)-1H-benzimidazol-2-yl]methyl]-1-oxo-phthalazin-2-yl]acetic acid, 2-[4-[[6-chloro-5-(trifluoromethoxy)-1H-benzimidazol-2-yl]methyl]-1-oxo-phthalazin-2-yl]-N-[(1S,2R)-2-hydroxycyclopentyl]acetamide, 2-[4-[[6-chloro-5-(trifluoromethoxy)-1H-benzimidazol-2-yl]methyl]-1-oxo-phthalazin-2-yl]-N-(1,1-dioxothian-4-yl)acetamide, 4-[[6-chloro-5-(trifluoromethoxy)-1H-benzimidazol-2-yl]methyl]-2-(2-morpholino-2-oxoethyl)phthalazin-1-one, 2-[4-[[6-chloro-5-(cyclopropylmethoxy)indazol-2-yl]methyl]-1-oxo-phthalazin-2-yl]-N-[(1S,2S)-2-hydroxycyclopentyl] acetamide, N-tert-butyl-2-[4-[[6-chloro-5-(cyclopropylmethoxy)indazol-2-yl]methyl]-1-oxo-phthalazin-2-yl]acetamide, 4-[[5-chloro-6-(trifluoromethoxy)-1H-benzimidazol-2-yl]methyl]-2-(2-morpholino-2-oxo-ethyl)-5,6,7,8-tetrahydrophthalazin-1-one, 2-[4-[[5-chloro-6-(trifluoromethoxy)-1H-benzimidazol-2-yl]methyl]-1-oxo-5,6,7,8-tetrahydrophthalazin-2-yl]-N-[(1S,2S)-2-hydroxycyclopentyl]acetamide, 2-[4-[[5-chloro-6-(trifluoromethoxy)-1H-benzimidazol-2-yl]methyl]-1-oxo-5,6,7,8-tetrahydrophthalazin-2-yl]-N-[(1S,2S)-2-hydroxycyclopentyl]acetamide, 4-[[6-chloro-5-(cyclopropylmethoxy)indazol-2-yl]methyl]-2-(2-morpholno-2-oxo-ethyl)phthalazin-1-one, 4-[[5-chloro-6-(cyclopropylmethoxy)-7-fluoro-1H-benzimidazol-2-yl]methyl]-2-(2-morpholino-2-oxoethyl)phthalazin-1-one, 2-[4-[[5-chloro-6-(cyclopropylmethoxy)-1H-benzimidazol-2-yl]methyl]-1-oxo-phthalazin-2-yl]-N-cyclopentyl-acetamide, 4-[[5-chloro-6-(2,2-difluoroethoxy)-1H-benzimidazol-2-yl]methyl]-2-(2-morpholino-2-oxoethyl)phthalazin-1-one, 4-[[6-chloro-5-(2,2-difluoroethoxy)-1H-benzimidazol-2-yl]methyl]-5,8-difluoro-2-(2-morpholino-2-oxoethyl)phthalazin-1-one, 4-[[6-chloro-5-(2,2-difluoroethoxy)-1H-benzimidazol-2-yl]methyl]-2-(2-morpholino-2-oxo-ethyl)-1-oxo-phthalazine-6-carbonitrile, 4-[(5-chloro-6-ethoxy-3-oxo-1H-indazol-2-yl)methyl]-2-(2-morpholino-2-oxo-ethyl)phthalazin-1-one, 4-(6-Chloro-5-cyclopropylmethoxy-1H-benzoimidazol-2-ylmethyl)-2-(1-methyl-2-morpholin-4-yl-2-oxoethyl)-2H-phthalazin-1-one, 4-[[5-(2,2-difluoroethoxy)-6-fluoro-1H-benzimidazol-2-yl]methyl]-2-(2-morpholino-2-oxoethyl)phthalazin-1-one, 4-[(5-ethoxy-6-fluoro-1H-benzimidazol-2-yl)methyl]-2-(2-morpholno-2-oxo-ethyl)phthalazin-1-one, 6-(cyclopropylmethoxy)-2-[[3-(2-morpholino-2-oxo-ethyl)-4-oxo-phthalazin-1-yl]methyl]-3H-benzimidazole-5-carbonitrile, 1-[6-Chloro-5-(2,2-difluoro-ethoxy)-1H-benzoimidazol-2-ylmethyl]-3-(2-morpholin-4-yl-2-oxo-ethyl)-1H-cinnolin-4-one, 4-[[5-chloro-6-(2,2-dimethylpropoxy)-1H-benzimidazol-2-yl]methyl]-2-(2-morpholino-2-oxoethyl)phthalazin-1-one, 1-[[6-chloro-2-[[3-(2-morpholino-2-oxo-ethyl)-4-oxo-phthalazin-1-yl]methyl]-3H-benzimidazol-5-yl]oxymethyl]cyclopropanecarbonitrile, 1-[[6-chloro-2-[[3-[2-[(2S,6R)-2,6-dimethylmorpholin-4-yl]-2-oxo-ethyl]-4-oxo-phthalazin-1-yl]methyl]-3H-benzimidazol-5-yl]oxymethyl]cyclopropanecarbonitrile, 4-[[5-chloro-6-[(1-methylcyclopropyl)methoxy]-1H-benzimidazol-2-yl]methyl]-2-(2-morpholino-2-oxoethyl)phthalazin-1-one, 6-(2,2-difluoroethoxy)-2-[[3-(2-morpholino-2-oxo-ethyl)-4-oxo-5,6,7,8-tetrahydrophthalazin-1-yl]methyl]-1H-benzimidazole-5-carbonitrile, 4-[(5-chloro-6-ethoxy-1H-benzimidazol-2-yl)methyl]-2-(2-morpholino-2-oxo-ethyl)phthalazin-1-one, 4-[[5-chloro-6-(cyclopropylmethoxy)-1H-benzimidazol-2-yl]methyl]-2-(2-morpholino-2-oxoethyl)phthalazin-1-one, 6-ethoxy-2-[[3-(2-morpholino-2-oxo-ethyl)-4-oxo-5,6,7,8-tetrahydrophthalazin-1-yl]methyl]-1H-benzimidazole-5 -carbonitrile, 4-[[6-ethoxy-5-(trifluoromethyl)-1H-benzimidazol-2-yl]methyl]-2-(2-morpholino-2-oxo-ethyl)phthalazin-1-one, 4-[6-Chloro-5-(2,2-difluoro-ethoxy)-1H-benzoimidazol-2-ylmethyl]-2-(2-morpholin-4-yl-2-oxo-ethyl)-2H-isoquinolin-1-one, 6-ethoxy-2-[[3-(2-morpholino-2-oxo-ethyl)-4-oxo-phthalazin-1-yl]methyl]-1H-benzimidazole-5-carbonitrile, 6-(2,2-difluoroethoxy)-2-[[3-(2-morpholino-2-oxo-ethyl)-4-oxo-phthalazin-1-yl]methyl]-1H-benzimidazole-5 -carbonitrile, 4-[[5-chloro-6-(2,2-difluoropropoxy)-1H-benzimidazol-2-yl]methyl]-2-(2-morpholino-2-oxoethyl)phthalazin-1-one, 4-[(5-chloro-6-isobutoxy-1H-benzimidazol-2-yl)methyl]-2-(2-morpholino-2-oxo-ethyl)phthalazin-1-one, 4-[[5-chloro-6-(2-methoxyethoxy)-1H-benzimidazol-2-yl]methyl]-2-(2-morpholino-2-oxoethyl)phthalazin-1-one, 2-[[3-(2-morpholino-2-oxo-ethyl)-4-oxo-phthalazin-1-yl]methyl]-6-(2,2,2-trifluoroethoxy)-3H-benzimidazole-5 -carbonitrile, 6-(2,2-difluoroethoxy)-2-[[3-[2-[3-(dimethylamino)pyrrolidin-1-yl]-2-oxo-ethyl]-4-oxo-phthalazin-1-yl]methyl]-3H-benzimidazole-5-carbonitrile, 6-(2,2-difluoroethoxy)-2-[[3-[2-[(2S,6R)-2,6-dimethylmorpholin-4-yl]-2-oxo-ethyl]-4-oxo-phthalazin-1-yl]methyl]-3H-benzimidazole-5-carbonitrile, 2-[4-[[6-cyano-5-(2,2-difluoroethoxy)-1H-benzimidazol-2-yl]methyl]-1-oxo-phthalazin-2-yl]-N-THF-3-yl-acetamide, 4-[[5-(2,2-difluoroethoxy)-6-fluoro-1H-benzimidazol-2-yl]methyl]-2-(2-morpholino-2-oxo-ethyl)-5,6,7,8-tetrahydrophthalazin-1-one, 2-[4-[[6-cyano-5-(2,2-difluoroethoxy)-1H-benzimidazol-2-yl]methyl]-1-oxo-phthalazin-2-yl]-N-methyl-N-(THF-2-ylmethyl)acetamide, 2-[4-[[6-cyano-5-(2,2-difluoroethoxy)-1H-benzimidazol-2-yl]methyl]-1-oxo-phthalazin-2-yl]-N-(2-cyanoethyl)-N-cyclopropyl-acetamide, 2-[4-[[6-cyano-5-(2,2-difluoroethoxy)-1H-benzimidazol-2-yl]methyl]-1-oxo-phthalazin-2-yl]-N-(2-methoxy-2-methyl-propyl)acetamide, 2-[4-[[6-cyano-5-(2,2-difluoroethoxy)-1H-benzimidazol-2-yl]methyl]-1-oxo-phthalazin-2-yl]-N-(2-methoxyethyl)-N-methyl-acetamide, 2-[4-[[6-cyano-5-(2,2-difluoroethoxy)-1H-benzimidazol-2-yl]methyl]-1-oxo-phthalazin-2-yl]-N-tetrahydropyran-3 -yl-acetamide, 6-(2,2-difluoroethoxy)-2-[[3-[2-(4,4-difluoro-1-piperidyl)-2-oxo-ethyl]-4-oxo-5,6,7,8-tetrahydrophthalazin-1-yl]methyl]-1H-benzimidazole-5 -carbonitrile, 6-(2,2-difluoroethoxy)-2-[[3-[2-(3-methoxypyrrolidin-1-yl)-2-oxo-ethyl]-4-oxo-phthalazin-1-yl]methyl]-1H-benzimidazole-5-carbonitrile, 2-[4-[[5-cyano-6-(2,2-difluoroethoxy)-1H-benzimidazol-2-yl]methyl]-1-oxo-phthalazin-2-yl]-N-(2-methoxyethyl)acetamide, 6-(2,2-difluoroethoxy)-2-[[3-[2-(4,4-difluoro-1-piperidyl)-2-oxo-ethyl]-4-oxo-phthalazin-1-yl]methyl]-1H-benzimidazole-5-carbonitrile, 6-(2,2-difluoroethoxy)-2-[[3-[2-(6-oxa-9-azaspiro[3.5]nonan-9-yl)-2-oxo-ethyl]-4-oxo-phthalazin-1-yl]methyl]-1H-benzimidazole-5-carbonitrile, 2-[4-[[5-cyano-6-(2,2-difluoroethoxy)-1H-benzimidazol-2-yl]methyl]-1-oxo-phthalazin-2-yl]-N-(3,3,3-trifluoro-2-hydroxy-2-methyl-propyl)acetamide, 2-[4-[[5-cyano-6-(2,2-difluoroethoxy)-1H-benzimidazol-2-yl]methyl]-1-oxo-phthalazin-2-yl]-N-[(2S)-3,3,3-trifluoro-2-hydroxy-propyl]acetamide, 2-[4-[[5-cyano-6-(2,2-difluoroethoxy)-1H-benzimidazol-2-yl]methyl]-1-oxo-phthalazin-2-yl]-N-(2-hydroxy-3-methoxy-propyl)acetamide, 6-(2,2-difluoroethoxy)-2-[[3-[2-(4-hydroxy-1-piperidyl)-2-oxo-ethyl]-4-oxo-phthalazin-1-yl]methyl]-1H-benzimidazole-5-carbonitrile, 2-[4-[[5-cyano-6-(2,2-difluoroethoxy)-1H-benzimidazol-2-yl]methyl]-1-oxo-phthalazin-2-yl]-N-(2,2-difluoroethyl)-N-(2-hydroxyethyl)acetamide, 2-[4-[[5-cyano-6-(2,2-difluoroethoxy)-1H-benzimidazol-2-yl]methyl]-1-oxo-phthalazin-2-yl]-N-(2-cyanopropyl)-N-methyl-acetamide, 2-[4-[[5-cyano-6-(2,2-difluoroethoxy)-1H-benzimidazol-2-yl]methyl]-1-oxo-phthalazin-2-yl]-N-(cyclopropylmethyl)-N-methyl-acetamide, 2-[4-[[5-cyano-6-(2,2-difluoroethoxy)-1H-benzimidazol-2-yl]methyl]-1-oxo-phthalazin-2-yl]-N-(2,2-difluoroethyl)-N-methyl-acetamide, 6-(2,2-difluoroethoxy)-2-[[3-[2-[(2S)-2-(hydroxymethyl)pyrrolidin-1-yl]-2-oxo-ethyl]-4-oxo-phthalazin-1-yl]methyl]-1H-benzimidazole-5-carbonitrile, 2-[4-[[5-cyano-6-(2,2-difluoroethoxy)-1H-benzimidazol-2-yl]methyl]-1-oxo-phthalazin-2-yl]-N-[[3-(hydroxymethyl)oxetan-3-yl]methyl]acetamide, 2-[[3-(2-morpholino-2-oxo-ethyl)-4-oxo-5,6,7,8-tetrahydrophthalazin-1-yl]methyl]-6-(2,2,2-trifluoroethoxy)-1H-benzimidazole-5-carbonitrile, 6-(2,2-difluoroethoxy)-2-[[3-[2-(3-hydroxy-3-methyl-pyrrolidin-1-yl)-2-oxo-ethyl]-4-oxo-phthalazin-1-yl]methyl]-3H-benzimidazole-5-carbonitrile, 2-[4-[[6-cyano-5-(2,2-difluoroethoxy)-1H-benzimidazol-2-yl]methyl]-1-oxo-phthalazin-2-yl]-N-(2,2-dimethylpropyl)acetamide, N-tert-butyl-2-[4-[[6-cyano-5-(2,2-difluoroethoxy)-1H-benzimidazol-2-yl]methyl]-1-oxo-5,6,7,8-tetrahydrophthalazin-2-yl] acetamide, 6-(2,2-difluoroethoxy)-2-[[3-[2-[3-(dimethylamino)pyrrolidin-1-yl]-2-oxo-ethyl]-4-oxo-5,6,7,8-tetrahydrophthalazin-1-yl]methyl]-1H-benzimidazole-5 -carbonitrile, 5-(2,2-difluoroethoxy)-2-[[3-(2-morpholino-2-oxo-ethyl)-4-oxo-phthalazin-1-yl]methyl]indazole-6-carbonitrile, 5-(2,2-difluoroethoxy)-2-[[3-[2-[(3R)-3-(dimethylamino)pyrrolidin-1-yl]-2-oxo-ethyl]-4-oxo-phthalazin-1-yl]methyl]indazole-6-carbonitrile, 5-(2,2-difluoroethoxy)-2-[[3-[2-[(3S)-3-(dimethylamino)pyrrolidin-1-yl]-2-oxo-ethyl]-4-oxo-phthalazin-1-yl]methyl]indazole-6-carbonitrile, 2-[4-[[6-cyano-5-(2,2-difluoroethoxy)indazol-2-yl]methyl]-1-oxo-phthalazin-2-yl]-N-cyclopentyl-acetamide, 5-(cyclopropylmethoxy)-2-[[3-(2-morpholino-2-oxo-ethyl)-4-oxo-phthalazin-1-yl]methyl]indazole-6-carbonitrile, 6-(cyclopropylmethoxy)-2-[[3-[2-(3-hydroxy-3-methyl-pyrrolidin-1-yl)-2-oxo-ethyl]-4-oxo-phthalazin-1-yl]methyl]imidazo[1,2-a]pyridine-7-carbonitrile, 6-(2,2-difluoroethoxy)-2-[[3-[2-[(3S,4S)-3,4-dihydroxypyrrolidin-1-yl]-2-oxo-ethyl]-4-oxo-phthalazin-1-yl]methyl]-3H-benzimidazole-5-carbonitrile, 6-(2,2-difluoroethoxy)-2-[[3-[2-(3,3-difluoro-4-hydroxy-1-piperidyl)-2-oxo-ethyl]-4-oxo-phthalazin-1-yl]methyl]-3H-benzimidazole-5-carbonitrile, 2-[[3-[2-(4-cyclopropyl-4-hydroxy-1-piperidyl)-2-oxo-ethyl]-4-oxo-phthalazin-1-yl]methyl]-6-(2,2-difluoroethoxy)-3H-benzimidazole-5-carbonitrile, 2-[[3-[2-(3-cyclopropyl-3-hydroxy-pyrrolidin-1-yl)-2-oxo-ethyl], -4-oxo-phthalazin-1-yl]methyl]-6-(2,2-difluoroethoxy)-3H-benzimidazole-5-carbonitrile, 2-[[3-[2-(3-cyclopropyl-3-hydroxy-azetidin-1-yl)-2-oxo-ethyl]-4-oxo-phthalazin-1-yl]methyl]-6-(2,2-difluoroethoxy)-3H-benzimidazole-5-carbonitrile, 6-(2,2-difluoroethoxy)-2-[[3-[2-[3-hydroxy-3-(trifluoromethyl)pyrrolidin-1-yl]-2-oxo-ethyl]-4-oxo-phthalazin-1-yl]methyl]-3H-benzimidazole-5-carbonitrile, 1-[[6-bromo-5-(cyclopropylmethoxy)-1H-benzimidazol-2-yl]methyl]-3-[2-[(3S,4S)-3,4-dihydroxypyrrolidin-1-yl]-2-oxo-ethyl]cinnolin-4-one, 2-[[3-[2-[4-(cyclopropylmethyl)piperazin-1-yl]-2-oxo-ethyl]-4-oxo-phthalazin-1-yl]methyl]-6-(2,2-difluoroethoxy)-3H-benzimidazole-5-carbonitrile, 2-[[3-[2-(4-cyano-1-piperidyl)-2-oxo-ethyl]-4-oxo-phthalazin-1-yl]methyl]-6-(2,2-difluoroethoxy)-3H-benzimidazole-5 -carbonitrile, 2-[4-[[6-cyano-5-(2,2-difluoroethoxy)-1H-benzimidazol-2-yl]methyl]-1-oxo-phthalazin-2-yl]-N-methyl-N-(2-methylsulfonylethyl)acetamide, 6-(2,2-difluoroethoxy)-2-[[3-[2-(3-methylsulfonylpyrrolidin-1-yl)-2-oxo-ethyl]-4-oxo-phthalazin-1-yl]methyl]-3H-benzimidazole-5-carbonitrile, 2-[4-[[6-cyano-5-(2,2-difluoroethoxy)-1H-benzimidazol-2-yl]methyl]-1-oxo-phthalazin-2-yl]-N-(2-hydroxy-3,3 -dimethyl-butyl)acetamide, 2-[4-[[6-cyano-5-(2,2-difluoroethoxy)-1H-benzimidazol-2-yl]methyl]-1-oxo-phthalazin-2-yl]-N-(2,2-difluoroethyl)acetamide, 2-[4-[[6-cyano-5-(2,2-difluoroethoxy)-1H-benzimidazol-2-yl]methyl]-1-oxo-phthalazin-2-yl]-N-(3,3,3-trifluoropropyl)acetamide, 6-(2,2-difluoroethoxy)-2-[[3-[2-[4-(2-hydroxyethyl)piperazin-1-yl]-2-oxo-ethyl]-4-oxo-phthalazin-1-yl]methyl]-3H-benzimidazole-5-carbonitrile, N-[cyano(cyclopropyl)methyl]-2-[4-[[6-cyano-5-(2,2-difluoroethoxy)-1H-benzimidazol-2-yl]methyl]-1-oxo-phthalazin-2-yl] acetamide, 6-(2,2-difluoroethoxy)-2-[[3-[2-(2,2-dimethylmorpholin-4-yl)-2-oxo-ethyl]-4-oxo-phthalazin-1-yl]methyl]-3H-benzimidazole-5-carbonitrile, 2-[4-[[6-cyano-5-(2,2-difluoroethoxy)-1H-benzimidazol-2-yl]methyl]-1-oxo-phthalazin-2-yl]-N-(2,2-difluoro-3-hydroxy-propyl)acetamide, 2-[4-[[6-cyano-5-(2,2-difluoroethoxy)-1H-benzimidazol-2-yl]methyl]-1-oxo-phthalazin-2-yl]-N-(2-sulfamoylethyl)acetamide, 2-[4-[[6-cyano-5-(2,2-difluoroethoxy)-1H-benzimidazol-2-yl]methyl]-1-oxo-phthalazin-2-yl]-N-(2,2,2-trifluoroethyl)acetamide, 6-(2,2-difluoroethoxy)-2-[[3-[2-(3-hydroxy-1-piperidyl)-2-oxo-ethyl]-4-oxo-phthalazin-1-yl]methyl]-3H-benzimidazole-5 -carbonitrile, 2-[4-[[6-cyano-5-(2,2-difluoroethoxy)-1H-benzimidazol-2-yl]methyl]-1-oxo-phthalazin-2-yl]-N-[(3,3-difluorocyclobutyl)methyl]acetamide, 6-(2,2-difluoroethoxy)-2-[[3-[2-(3,3-dimethylpyrrolidin-1-yl)-2-oxo-ethyl]-4-oxo-phthalazin-1-yl]methyl]-3H-benzimidazole-5-carbonitrile, 6-(2,2-difluoroethoxy)-2-[[3-[2-(4,4-dimethyl-1-piperidyl)-2-oxo-ethyl]-4-oxo-phthalazin-1-yl]methyl]-3H-benzimidazole-5-carbonitrile, 2-[[3-[2-(2,2-difluoro-5-azaspiro[2.4]heptan-5-yl)-2-oxo-ethyl]-4-oxo-phthalazin-1-yl]methyl]-6-(2,2-difluoroethoxy)-3H-benzimidazole-5-carbonitrile, 1-[2-[4-[[6-cyano-5-(2,2-difluoroethoxy)-1H-benzimidazol-2-yl]methyl]-1-oxo-phthalazin-2-yl]acetyl]-N,N-dimethyl-piperidine-4-carboxamide, 6-(2,2-difluoroethoxy)-2-[[3-[2-[3-(1-hydroxy-1-methyl-ethyl)-1-piperidyl]-2-oxo-ethyl]-4-oxo-phthalazin-1-yl]methyl]-3H-benzimidazole-5-carbonitrile, 6-(2,2-difluoroethoxy)-2-[[3-[2-(4-morpholino-1-piperidyl)-2-oxo-ethyl]-4-oxo-phthalazin-1-yl]methyl]-3H-benzimidazole-5-carbonitrile, 6-(2,2-difluoroethoxy)-2-[[3-[2-(7-oxa-2-azaspiro[3.5]nonan-2-yl)-2-oxo-ethyl]-4-oxo-phthalazin-1-yl]methyl]-3H-benzimidazole-5-carbonitrile, 6-(2,2-difluoroethoxy)-2-[[3-[2-(4-ethylsulfonyl-1-piperidyl)-2-oxo-ethyl]-4-oxo-phthalazin-1-yl]methyl]-3H-benzimidazole-5-carbonitrile, 2-[4-[[6-cyano-5-(2,2-difluoroethoxy)-1H-benzimidazol-2-yl]methyl]-l-oxo-phthalazin-2-yl]-N-[2-(dimethylamino)-2-oxo-ethyl]-N-methyl-acetamide, 2-[4-[[6-chloro-5-(cyclopropylmethoxy)-1H-benzimidazol-2-yl]methyl]-1-oxo-phthalazin-2-yl]-N-methylsulfonyl-acetamide, 2-[4-[[6-chloro-5-(cyclopropylmethoxy)indazol-2-yl]methyl]-1-oxo-phthalazin-2-yl]-N-methylsulfonyl-acetamide, 2-[4-[[6-chloro-5-(cyclopropylmethoxy)indazol-2-yl]methyl]-1-oxo-phthalazin-2-yl]-N-cyclopropylsulfonyl-acetamide, 2-[4-[[6-chloro-5-(2,2-difluoroethoxy)-1H-benzimidazol-2-yl]methyl]-6-cyano-1-oxo-phthalazin-2-yl]-N-methylsulfonyl-acetamide, N-{2-[4-(6-Chloro-5-cyclopropylmethoxy-1H-benzoimidazol-2-ylmethyl)-1-oxo-1H-phthalazin-2-yl] - propionyl}-methanesulfonamide, 2-[4-[(5-ethoxy-6-fluoro-1H-benzimidazol-2-yl)methyl]-1-oxo-phthalazin-2-yl]-N-methylsulfonyl-acetamide, 2-[4-[[5-(2,2-difluoroethoxy)-6-fluoro-1H-benzimidazol-2-yl]methyl]-1-oxo-phthalazin-2-yl]-N-methylsulfonyl-acetamide, 2-[4-[[6-chloro-5-[(1-cyanocyclopropyl)methoxy]-1H-benzimidazol-2-yl]methyl]-1-oxo-phthalazin-2-yl]-N-methylsulfonyl-acetamide, 2-[4-[[6-chloro-5-[(1-methylcyclopropyl)methoxy]-1H-benzimidazol-2-yl]methyl]-1-oxo-phthalazin-2-yl]-N-methylsulfonyl-acetamide, 2-[4-[(6-chloro-5-ethoxy-indazol-2-yl)methyl]-1-oxo-phthalazin-2-yl]acetic acid, 2-[4-[[6-chloro-5-(2,2,2-trifluoroethoxy)indazol-2-yl]methyl]-1-oxo-phthalazin-2-yl]acetic acid, 2-[4-[[6-chloro-5-(2,2-difluorocthoxy)indazol-2-yl]mcthyl]-1-oxo-phthalazin-2-yl]acctic acid, 2-[4-[(4-chloro-5-ethoxy-indazol-2-yl)methyl]-1-oxo-phthalazin-2-yl]acetic acid, 2-[4-[(5-chloro-6-ethoxy-indazol-2-yl)methyl]-1-oxo-phthalazin-2-yl]acetic acid, 6-(2,2-difluoroethoxy)-2-[[3-(2-morpholino-2-oxo-ethyl)-4-oxo-phthalazin-1-yl]methyl]imidazo[1,2-a]pyridine-7-carbonitrile, and 2-[4-[[6-chloro-5-(cyclopropylmethoxy)-1H-imidazo[4,5-b]pyridin-2-yl]methyl]-1-oxo-phthalazin-2-yl]acetic acid.

In one embodiment a compound of the invention is not an isotopic variant.

In one aspect a compound of the invention according to any one of the embodiments herein described is present as the free base.

In one aspect a compound of the invention according to any one of the embodiments herein described is a pharmaceutically acceptable salt.

In one aspect a compound of the invention according to any one of the embodiments herein described is a solvate of the compound.

In one aspect a compound of the invention according to any one of the embodiments herein described is a solvate of a pharmaceutically acceptable salt of a compound.

While specified groups for each embodiment have generally been listed above separately, a compound of the invention includes one in which several or each embodiment in the above Formula, as well as other formulae presented herein, is selected from one or more of particular members or groups designated respectively, for each variable. Therefore, this invention is intended to include all combinations of such embodiments within its scope.

While specified groups for each embodiment have generally been listed above separately, a compound of the invention may be one for which one or more variables (for example, R groups) is selected from one or more embodiments according to any of the Formula(e) listed above. Therefore, the present invention is intended to include all combinations

of variables from any of the disclosed embodiments within its scope.

Alternatively, the exclusion of one or more of the specified variables from a group or an embodiment, or combinations thereof is also contemplated by the present invention.

In certain aspects, the present description provides prodrugs and derivatives of the compounds according to the formulae above. Prodrugs are derivatives of the compounds of the invention, which have metabolically cleavable groups and become by solvolysis or under physiological conditions the compounds of the invention, which are pharmaceutically active, in vivo. Such examples include, but are not limited to, choline ester derivatives and the like, N-alkylmorpholine esters and the like.

Other derivatives of the compounds of this invention have activity in both their acid and acid derivative forms, but the acid sensitive form often offers advantages of solubility, tissue compatibility, or delayed release in the mammalian organism (Bundgard, H, 1985). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are preferred prodrugs. In some cases it is desirable to prepare double ester type prodrugs such as (acyloxy)alkyl esters or ((alkoxycarbonyl)oxy)alkylesters. Particularly useful are the $C_1$-$C_8$ alkyl, $C_2$-$C_8$ alkenyl, aryl, $C_7$-$C_{12}$ substituted aryl, and $C_7$-$C_{12}$ arylalkyl esters of the compounds of the invention.

## PHARMACEUTICAL COMPOSITIONS

When employed as a pharmaceutical, a compound of the invention is typically administered in the form of a pharmaceutical composition. Such compositions can be prepared in a manner well known in the pharmaceutical art and comprise at least one active compound of the invention according to Formula Ia or Ib. Generally, a compound of the invention is administered in a pharmaceutically effective amount. The amount of compound of the invention actually administered will typically be determined by a physician, in the light of the relevant circumstances, including the condition to be treated, the chosen route of administration, the actual compound of the invention administered, the age, weight, and response of the individual patient, the severity of the patient's symptoms, and the like.

The pharmaceutical compositions of this invention can be administered by a variety of routes including oral, rectal, transdermal, subcutaneous, intra-articular, intravenous, intramuscular, and intranasal. Depending on the intended route of delivery, a compound of the invention is preferably formulated as either injectable or oral compositions or as salves, as lotions or as patches all for transdermal administration.

The compositions for oral administration can take the form of bulk liquid solutions or suspensions, or bulk powders. More commonly, however, the compositions are presented in unit dosage forms to facilitate accurate dosing. The term 'unit dosage forms' refers to physically discrete units suitable as unitary dosages for human subjects and other mammals, each unit containing a predetermined quantity of active material calculated to produce the desired therapeutic effect, in association with a suitable pharmaceutical excipient, vehicle or carrier. Typical unit dosage forms include prefilled, premeasured ampules or syringes of the liquid compositions or pills, tablets, capsules or the like in the case of solid compositions. In such compositions, the compound of the invention according to Formula I is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or non-aqueous vehicle with buffers, suspending and dispensing agents, colorants, flavors and the like. Solid forms may include, for example, any of the following ingredients, or compound of the inventions of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint or orange flavoring.

Injectable compositions are typically based upon injectable sterile saline or phosphate-buffered saline or other injectable carriers known in the art. As before, the active compound of the invention according to Formula I in such compositions is typically a minor component, often being from about 0.05 to 10% by weight with the remainder being the injectable carrier and the like.

Transdermal compositions are typically formulated as a topical ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or the formulation. All such known transdermal formulations and ingredients are included within the scope of this invention.

A compound of the invention can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

A compound of the invention can also be administered in sustained release forms or from sustained release drug delivery systems. A description of representative sustained release materials can be found in Remington's Pharmaceutical Sciences.

The following formulation examples illustrate representative pharmaceutical compositions that may be prepared in accordance with this invention. The present invention, however, is not limited to the following pharmaceutical compositions.

**Formulation 1 - Tablets**

A compound of the invention according to Formula I may be admixed as a dry powder with a dry gelatin binder in an approximate 1:2 weight ratio. A minor amount of magnesium stearate may be added as a lubricant. The mixture may be formed into 240-270 mg tablets (80-90 mg of active compound of the invention according to Formula I per tablet) in a tablet press.

**Formulation 2 - Capsules**

A compound of the invention according to Formula I may be admixed as a dry powder with a starch diluent in an approximate 1:1 weight ratio. The mixture may be filled into 250 mg capsules (125 mg of active compound of the invention according to Formula I per capsule).

**Formulation 3 - Liquid**

A compound of the invention according to Formula I (125 mg), may be admixed with sucrose (1.75 g) and xanthan gum (4 mg) and the resultant mixture may be blended, passed through a No. 10 mesh U.S. sieve, and then mixed with a previously made solution of microcrystalline cellulose and sodium carboxymethyl cellulose (11:89, 50 mg) in water. Sodium benzoate (10 mg), flavor, and color may be diluted with water and added with stirring. Sufficient water may then be added with stirring. Further sufficient water may be then added to produce a total volume of 5 mL.

**Formulation 4 - Tablets**

A compound of the invention according to Formula I may be admixed as a dry powder with a dry gelatin binder in an approximate 1:2 weight ratio. A minor amount of magnesium stearate may be added as a lubricant. The mixture may be formed into 450-900 mg tablets (150-300 mg of active compound of the invention according to Formula I) in a tablet press.

**Formulation 5 - Injection**

A compound of the invention according to Formula I may be dissolved or suspended in a buffered sterile saline injectable aqueous medium to a concentration of approximately 5 mg/mL.

**Formulation 6 - Topical**

Stearyl alcohol (250 g) and a white petrolatum (250 g) may be melted at about 75°C and then a mixture of A compound of the invention according to Formula I (50 g) methylparaben (0.25 g), propylparaben (0.15 g), sodium lauryl sulfate (10 g), and propylene glycol (120 g) dissolved in water (about 370 g) may be added and the resulting mixture may be stirred until it congeals.

## METHODS OF TREATMENT

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the

prophylaxis and/or treatment of fibrotic diseases, inflammatory diseases, respiratory diseases, autoimmune diseases, metabolic diseases, cardiovascular diseases, and/or proliferative diseases.

In another embodiment, the present invention provides compounds of the invention, or pharmaceutical compositions comprising a compound of the invention for use in the manufacture of a medicament for use in the prophylaxis and/or treatment of fibrotic diseases, inflammatory diseases, respiratory diseases, autoimmune diseases, metabolic diseases, cardiovascular diseases, and/or proliferative diseases.

In additional method of treatment aspects, this invention provides methods of prophylaxis and/or treatment of a mammal afflicted with fibrotic diseases, inflammatory diseases, respiratory diseases, autoimmune diseases, metabolic diseases, cardiovascular diseases, and/or proliferative diseases, which methods comprise the administration of an effective amount of a compound of the invention or one or more of the pharmaceutical compositions herein described for the treatment or prophylaxis of said condition.

In one embodiment, the present invention provides pharmaceutical compositions comprising a compound of the invention, and another therapeutic agent. In a particular embodiment, the other therapeutic agent is a fibrotic diseases, inflammatory diseases, respiratory diseases, autoimmune diseases, metabolic diseases, cardiovascular diseases, and/or proliferative diseases.

In one embodiment, the present invention provides compounds of the invention or pharmaceutical compositions comprising a compound of the invention, for use in the prophylaxis and/or treatment of fibrotic diseases. In a more particular embodiment, the fibrotic disease is selected from idiopathic pulmonary fibrosis, Dupuytren disease, nonalcoholic steatohepatitis, portal hypertension, systemic sclerosis, renal fibrosis, and cutaneous fibrosis. In a most particular embodiment, the fibrotic disease is idiopathic pulmonary fibrosis. In another most particular embodiment, the fibrotic disease is nonalcoholic steatohepatitis (NASH).

In another embodiment, the present invention provides compounds of the invention, or pharmaceutical compositions comprising a compound of the invention for use in the manufacture of a medicament for use in the prophylaxis and/or treatment of fibrotic diseases. In a more particular embodiment, the fibrotic disease is selected from idiopathic pulmonary fibrosis, Dupuytren disease, nonalcoholic steatohepatitis, portal hypertension, systemic sclerosis, renal fibrosis, and cutaneous fibrosis. In a most particular embodiment, the fibrotic disease is idiopathic pulmonary fibrosis. In another most particular embodiment, the fibrotic disease is nonalcoholic steatohepatitis (NASH).

In additional method of treatment aspects, this invention provides methods of prophylaxis and/or treatment of a mammal afflicted with fibrotic diseases, which methods comprise the administration of an effective amount of a compound of the invention or one or more of the pharmaceutical compositions herein described for the treatment or prophylaxis of said condition. In a more particular embodiment, the fibrotic disease is selected from idiopathic pulmonary fibrosis, Dupuytren disease, nonalcoholic steatohepatitis, portal hypertension, systemic sclerosis, renal fibrosis, and cutaneous fibrosis. In a most particular embodiment, the fibrotic disease is idiopathic pulmonary fibrosis. In another most particular embodiment, the fibrotic disease is nonalcoholic steatohepatitis (NASH).

In one embodiment, the present invention provides pharmaceutical compositions comprising a compound of the invention, and another therapeutic agent. In a particular embodiment, the other therapeutic agent is a fibrotic diseases treatment agent. In a more particular embodiment, the fibrotic disease is selected from idiopathic pulmonary fibrosis, Dupuytren disease, nonalcoholic steatohepatitis, portal hypertension, systemic sclerosis, renal fibrosis, and cutaneous fibrosis. In a most particular embodiment, the fibrotic disease is idiopathic pulmonary fibrosis. In another most particular embodiment, the fibrotic disease is nonalcoholic steatohepatitis (NASH).

In a particular embodiment, the present invention provides compounds of the invention or pharmaceutical compositions comprising a compound of the invention, for use in the prophylaxis and/or treatment of inflammatory diseases. In a particular embodiment, the inflammatory disease is selected from rheumatoid arthritis, ostcoarthritis, allergic airway disease, chronic obstructive pulmonary disease and inflammatory bowel diseases.

In another embodiment, the present invention provides compounds of the invention, or pharmaceutical compositions comprising a compound of the invention for use in the manufacture of a medicament for use in the prophylaxis and/or treatment of inflammatory diseases. In a particular embodiment, the inflammatory disease is selected from rheumatoid arthritis, osteoarthritis, allergic airway disease, chronic obstructive pulmonary disease and inflammatory bowel diseases.

In additional method of treatment aspects, this invention provides methods of prophylaxis and/or treatment of a mammal afflicted with inflammatory diseases, which methods comprise the administration of an effective amount of a compound of the invention or one or more of the pharmaceutical compositions herein described for the treatment or prophylaxis of said condition. In a particular embodiment, the inflammatory disease is selected from rheumatoid arthritis, osteoarthritis, allergic airway disease, chronic obstructive pulmonary disease and inflammatory bowel diseases.

In one embodiment, the present invention provides pharmaceutical compositions comprising a compound of the invention, and another therapeutic agent. In a particular embodiment, the other therapeutic agent is an agent for the prophylaxis and/or treatment of inflammatory diseases. In a particular embodiment, the inflammatory disease is selected from rheumatoid arthritis, osteoarthritis, allergic airway disease, chronic obstructive pulmonary disease and inflammatory

bowel diseases.

In a particular embodiment, the present invention provides compounds of the invention or pharmaceutical compositions comprising a compound of the invention, for use in the prophylaxis and/or treatment of respiratory diseases. In a particular embodiment, the respiratory disease is selected from asthma.

In another embodiment, the present invention provides compounds of the invention, or pharmaceutical compositions comprising a compound of the invention for use in the manufacture of a medicament for use in the prophylaxis and/or treatment of repiratory diseases. In a particular embodiment, the respiratory disease is selected from asthma.

In additional method of treatment aspects, this invention provides methods of prophylaxis and/or treatment of a mammal afflicted with respiratory diseases, which methods comprise the administration of an effective amount of a compound of the invention or one or more of the pharmaceutical compositions herein described for the treatment or prophylaxis of said condition. In a particular embodiment, the respiratory disease is selected from asthma.

In one embodiment, the present invention provides pharmaceutical compositions comprising a compound of the invention, and another therapeutic agent. In a particular embodiment, the other therapeutic agent is an agent for the prophylaxis and/or treatment of respiratory diseases. In a particular embodiment, the respiratory disease is selected from asthma.

In a particular embodiment, the present invention provides compounds of the invention or pharmaceutical compositions comprising a compound of the invention, for use in the prophylaxis and/or treatment of autoimmune diseases. In a particular embodiment, the autoimmune disease is selected from chronic obstructive pulmonary disease, asthma, systemic lupus erythematosus, type I diabetes mellitus and inflammatory bowel disease.

In another embodiment, the present invention provides compounds of the invention, or pharmaceutical compositions comprising a compound of the invention for use in the manufacture of a medicament for use in the prophylaxis and/or treatment of autoimmune diseases. In a particular embodiment, the autoimmune disease is selected from chronic obstructive pulmonary disease, asthma, systemic lupus erythematosus, type I diabetes mellitus and inflammatory bowel disease.

In additional method of treatment aspects, this invention provides methods of prophylaxis and/or treatment of a mammal afflicted with autoimmune diseases, which methods comprise the administration of an effective amount of a compound of the invention or one or more of the pharmaceutical compositions herein described for the treatment or prophylaxis of said condition. In a particular embodiment, the autoimmune disease is selected from chronic obstructive pulmonary disease, asthma, systemic lupus erythematosus, type I diabetes mellitus and inflammatory bowel disease.

In one embodiment, the present invention provides pharmaceutical compositions comprising a compound of the invention, and another therapeutic agent. In a particular embodiment, the other therapeutic agent is an autoimmune diseases treatment agent. In a particular embodiment, the autoimmune disease is selected from chronic obstructive pulmonary disease, asthma, systemic lupus erythematosus, type I diabetes mellitus and inflammatory bowel disease.

In a particular embodiment, the present invention provides compounds of the invention or pharmaceutical compositions comprising a compound of the invention, for use in the prophylaxis and/or treatment of metabolic diseases. In a particular embodiment, the metabolic disease is type II diabetes and/or obesity.

In another embodiment, the present invention provides compounds of the invention, or pharmaceutical compositions comprising a compound of the invention for use in the manufacture of a medicament for use in the prophylaxis and/or treatment of metabolic diseases. In a particular embodiment, the metabolic disease is type II diabetes and/or obesity.

In additional method of treatment aspects, this invention provides methods of prophylaxis and/or treatment of a mammal afflicted with metabolic diseases, which methods comprise the administration of an effective amount of a compound of the invention or one or more of the pharmaceutical compositions herein described for the treatment or prophylaxis of said condition. In a particular embodiment, the metabolic disease is type II diabetes and/or obesity.

In one embodiment, the present invention provides pharmaceutical compositions comprising a compound of the invention, and another therapeutic agent. In a particular embodiment, the other therapeutic agent is a metabolic diseases treatment agent. In a particular embodiment, the metabolic disease is type II diabetes and/or obesity.

In a particular embodiment, the present invention provides compounds of the invention or pharmaceutical compositions comprising a compound of the invention, for use in the prophylaxis and/or treatment of cardiovascular diseases. In a particular embodiment, the cardiovascular disease is selected from stroke, atherosclerosis, reperfusion injury following ischemia, myocardial ischemia, angina, peripheral obstructive arteriopathy and/or vasculitis. In a more particular embodiment, the cardiovascular disease is stroke and/or vasculitis.

In another embodiment, the present invention provides compounds of the invention, or pharmaceutical compositions comprising a compound of the invention for use in the manufacture of a medicament for use in the prophylaxis and/or treatment of cardiovascular diseases. In a particular embodiment, the cardiovascular disease is selected from stroke, atherosclerosis, reperfusion injury following ischemia, myocardial ischemia, angina, peripheral obstructive arteriopathy and/or vasculitis. In a more particular embodiment, the cardiovascular disease is stroke and/or vasculitis.

In additional method of treatment aspects, this invention provides methods of prophylaxis and/or treatment of

a mammal afflicted with cardiovascular diseases, which methods comprise the administration of an effective amount of a compound of the invention or one or more of the pharmaceutical compositions herein described for the treatment or prophylaxis of said condition. In a particular embodiment, the cardiovascular disease is selected from stroke, atherosclerosis, reperfusion injury following ischemia, myocardial ischemia, angina, peripheral obstructive arteriopathy and/or vasculitis. In a more particular embodiment, the cardiovascular disease is stroke and/or vasculitis.

In one embodiment, the present invention provides pharmaceutical compositions comprising a compound of the invention, and another therapeutic agent. In a particular embodiment, the other therapeutic agent is a cardiovascular diseases treatment agent. In a particular embodiment, the cardiovascular disease is selected from stroke, atherosclerosis, reperfusion injury following ischemia, myocardial ischemia, angina, peripheral obstructive arteriopathy or vasculitis. In a more particular embodiment, the cardiovascular disease is stroke or vasculitis.

In a particular embodiment, the present invention provides compounds of the invention or pharmaceutical compositions comprising a compound of the invention, for use in the prophylaxis and/or treatment of proliferative diseases. In a particular embodiment, the proliferative disease is selected from Wilm's tumor, glioblastoma, lung cancer, breast cancer, ovarian cancer, melanoma, multiple myeloma, psoriasis, restenosis, and scleroderma. In a particular embodiment, the proliferative disease is scleroderma.

In another embodiment, the present invention provides compounds of the invention, or pharmaceutical compositions comprising a compound of the invention for use in the manufacture of a medicament for use in the prophylaxis and/or treatment of proliferative diseases. In a particular embodiment, the proliferative disease is selected from Wilm's tumor, glioblastoma, lung cancer, breast cancer, ovarian cancer, melanoma, multiple myeloma, psoriasis, restenosis, and scleroderma. In a particular embodiment, the proliferative disease is scleroderma.

In additional method of treatment aspects, this invention provides methods of prophylaxis and/or treatment of a mammal afflicted with proliferative diseases, which methods comprise the administration of an effective amount of a compound of the invention or one or more of the pharmaceutical compositions herein described for the treatment or prophylaxis of said condition. In a particular embodiment, the proliferative disease is selected from Wilm's tumor, glioblastoma, lung canccr, breast canccr, ovarian cancer, melanoma, multiple myeloma, psoriasis, restenosis, and scleroderma. In a particular embodiment, the proliferative disease is scleroderma.

In one embodiment, the present invention provides pharmaceutical compositions comprising a compound of the invention, and another therapeutic agent. In a particular embodiment, the other therapeutic agent is a proliferative diseases treatment agent. In a particular embodiment, the proliferative disease is selected from Wilm's tumor, glioblastoma, lung cancer, breast cancer, ovarian cancer, melanoma, multiple myeloma, psoriasis, restenosis, and scleroderma. In a particular embodiment, the proliferative disease is scleroderma.

Injection dose levels range from about 0.1 mg/kg/h to at least 10 mg/kg/h, all for from about 1 to about 120 h and especially 24 to 96 h. A preloading bolus of from about 0.1 mg/kg to about 10 mg/kg or more may also be administered to achieve adequate steady state levels. The maximum total dose is not expected to exceed about 1 g/day for a 40 to 80 kg human patient.

For the prophylaxis and/or treatment of long-term conditions, such as degenerative conditions, the regimen for treatment usually stretches over many months or years so oral dosing is preferred for patient convenience and tolerance. With oral dosing, one to four (1-4) regular doses daily, especially one to three (1-3) regular doses daily, typically one to two (1-2) regular doses daily, and most typically one (1) regular dose daily are representative regimens. Alternatively for long lasting effect drugs, with oral dosing, once every other week, once weekly, and once a day are representative regimens. In particular, dosage regimen can be every 1-14 days, more particularly 1-10 days, even more particularly 1-7 days, and most particularly 1-3 days.

Using these dosing patterns, each dose provides from about 1 to about 1000 mg of a compound of the invention, with particular doses each providing from about 10 to about 500 mg and especially about 30 to about 250 mg.

Transdermal doses are generally selected to provide similar or lower blood levels than are achieved using injection doses.

When used to prevent the onset of a condition, a compound of the invention will be administered to a patient at risk for developing the condition, typically on the advice and under the supervision of a physician, at the dosage levels described above. Patients at risk for developing a particular condition generally include those that have a family history of the condition, or those who have been identified by genetic testing or screening to be particularly susceptible to developing the condition.

A compound of the invention can be administered as the sole active agent or it can be administered in combination with other therapeutic agents, including other compound of the inventions that demonstrate the same or a similar therapeutic activity and that are determined to be safe and efficacious for such combined administration. In a specific embodiment, co-administration of two (or more) agents allows for significantly lower doses of each to be used, thereby reducing the side effects seen.

In one embodiment, a compound of the invention or a pharmaceutical composition comprising a compound of the invention is administered as a medicament. In a specific embodiment, said pharmaceutical composition additionally

comprises a further activc ingredient.

In one embodiment, a compound of the invention is co-administered with one or more further therapeutic agents for the treatment and/or prophylaxis of a fibrotic disease. In a particular embodiment, a compound of the invention is co-administered with one or two further therapeutic agents for the treatment and/or prophylaxis of a fibrotic disease. In a more particular embodiment, a compound of the invention is co-administered with one further therapeutic agent for the treatment and/or prophylaxis of a fibrotic disease.

In one embodiment, the further therapeutic agent for the treatment and/or prophylaxis of a fibrotic disease include, but are not limited to 5-methyl-1-phenyl-2-(1H)-pyridone (Pirfenidone ®); Nintedanib (Ofev® or Vargatef®); STX-100 (ClinicalTrials.gov Identifier NCT01371305), FG-3019 (ClinicalTrials.gov Identifier NCT01890265), Lebrikizumab (CAS n# 953400-68-5); Tralokinumab (CAS n# 1044515-88-9), PRM-151 (ClinicalTrials.gov Identifier NCT02550873) and PBI-4050 (ClinicalTrials.gov Identifier NCT02538536). In another particular embodiment, the further therapeutic agent for the treatment and/or prophylaxis of a fibrotic disease is an autotaxin (or ectonucleotide pyrophosphatase/phosphodiesterase 2 or NPP2 or ENPP2) inhibitor, examples of which are described in WO 2014/139882.

In one embodiment, a compound of the invention is co-administered with another therapeutic agent for the treatment and/or prophylaxis of a disease involving inflammation, particular agents include, but are not limited to, immunoregulatory agents e.g. azathioprine, corticosteroids (e.g. prednisolone or dexamethasone), cyclophosphamide, cyclosporin A, tacrolimus, mycophenolate, mofetil, muromonab-CD3 (OKT3, e.g. Orthocolone®), ATG, aspirin, acetaminophen, ibuprofen, naproxen, and piroxicam.

In one embodiment, a compound of the invention is co-administered with another therapeutic agent for the treatment and/or prophylaxis of arthritis (e.g. rheumatoid arthritis), particular agents include but are not limited to analgesics, non-steroidal anti-inflammatory drugs (NSAIDS), steroids, synthetic DMARDS (for example but without limitation methotrexate, leflunomide, sulfasalazine, auranofin, sodium aurothiomalate, penicillamine, chloroquine, hydroxychloroquine, azathioprine, tofacitinib, baricitinib, fostamatinib, and cyclosporin), and biological DMARDS (for example but without limitation infliximab, etanercept, adalimumab, rituximab, and abatacept).

In one embodiment, a compound of the invention is co-administered with another therapeutic agent for the treatment and/or prophylaxis of proliferative diseases, particular agents include but are not limited to: methotrexate, leukovorin, adriamycin, prednisone, bleomycin, cyclophosphamide, 5-fluorouracil, paclitaxel, docetaxel, vincristine, vinblastine, vinorelbine, doxorubicin, tamoxifen, toremifene, megestrol acetate, anastrozole, goserelin, anti-HER2 monoclonal antibody (e.g. HerceptinTM), capecitabine, raloxifene hydrochloride, EGFR inhibitors (e.g. Iressa®, Tarceva™, Erbitux™), VEGF inhibitors (e.g. Avastin™), proteasome inhibitors (e.g. Velcade™), Glivec® and hsp90 inhibitors (e.g. 17-AAG). Additionally, the compound of the invention according to Formula I may be administered in combination with other therapies including, but not limited to, radiotherapy or surgery. In a specific embodiment the proliferative disease is selected from cancer, myeloproliferative disease or leukaemia.

In one embodiment, a compound of the invention is co-administered with another therapeutic agent for the treatment and/or prophylaxis of autoimmune diseases, particular agents include but arc not limited to: glucocorticoids, cytostatic agents (e.g. purine analogs), alkylating agents, (e.g nitrogen mustards (cyclophosphamide), nitrosoureas, platinum compound of the inventions, and others), antimetabolites (e.g. methotrexate, azathioprine and mercaptopurine), cytotoxic antibiotics (e.g. dactinomycin anthracyclines, mitomycin C, bleomycin, and mithramycin), antibodies (e.g. anti-CD20, anti-CD25 or anti-CD3 (OTK3) monoclonal antibodies, Atgam® and Thymoglobuline®), cyclosporin, tacrolimus, rapamycin (sirolimus), interferons (e.g. IFN-β), TNF binding proteins (e.g. infliximab, etanercept, or adalimumab), mycophenolate, fingolimod and myriocin..

In one embodiment, a compound of the invention is co-administered with another therapeutic agent for the treatment and/or prophylaxis of transplant rejection, particular agents include but are not limited to: calcineurin inhibitors (e.g. cyclosporin or tacrolimus (FK506)), mTOR inhibitors (e.g. sirolimus, everolimus), anti-proliferatives (e.g. azathioprine, mycophenolic acid), corticosteroids (e.g. prednisolone, hydrocortisone), antibodies (e.g. monoclonal anti-IL-2Rα receptor antibodies, basiliximab, daclizumab), polyclonal anti-T-cell antibodies (e.g. anti-thymocyte globulin (ATG), anti-lymphocyte globulin (ALG)).

In one embodiment, a compound of the invention is co-administered with another therapeutic agent for the treatment and/or prophylaxis of asthma and/or rhinitis and/or chronic obstructive pulmonary disease, particular agents include but are not limited to: beta2-adrenoceptor agonists (e.g. salbutamol, levalbuterol, terbutaline and bitolterol), epinephrine (inhaled or tablets), anticholinergics (e.g. ipratropium bromide), glucocorticoids (oral or inhaled). Long-acting β2-agonists (e.g. salmeterol, formoterol, bambuterol, and sustained-release oral albuterol), combinations of inhaled steroids and long-acting bronchodilators (e.g. fluticasone/salmeterol, budesonide/formoterol), leukotriene antagonists and synthesis inhibitors (e.g. montelukast, zafirlukast and zileuton), inhibitors of mediator release (e.g. cromoglycate and ketotifen), biological regulators of IgE response (e.g. omalizumab), antihistamines (e.g. ceterizine, cinnarizine, fexofenadine) and vasoconstrictors (e.g. oxymethazoline, xylomethazoline, nafazoline and tramazoline).

Additionally, a compound of the invention may be administered in combination with emergency therapies for asthma and/or chronic obstructive pulmonary disease, such therapies include oxygen or heliox administration, nebulized

salbutamol or terbutaline (optionally combined with an anticholinergic (e.g. ipratropium), systemic steroids (oral or intravenous, e.g. prednisone, prednisolone, methylprednisolone, dexamethasone, or hydrocortisone), intravenous salbutamol, non-specific beta-agonists, injected or inhaled (e.g. epinephrine, isoetharine, isoproterenol, metaproterenol), anticholinergics (IV or nebulized, e.g. glycopyrrolate, atropine, ipratropium), methylxanthines (theophylline, aminophylline, bamiphylline), inhalation anesthetics that have a bronchodilatory effect (e.g. isoflurane, halothane, enflurane), ketamine and intravenous magnesium sulfate.

In one embodiment, a compound of the invention is co-administered with another therapeutic agent for the treatment and/or prophylaxis of inflammatory bowel disease (IBD), particular agents include but are not limited to: glucocorticoids (e.g. prednisone, budesonide) synthetic disease modifying, immunomodulatory agents (e.g. methotrexate, leflunomide, sulfasalazine, mesalazine, azathioprine, 6-mercaptopurine and cyclosporin) and biological disease modifying, immunomodulatory agents (infliximab, adalimumab, rituximab, and abataccpt).

In one embodiment, a compound of the invention is co-administered with another therapeutic agent for the treatment and/or prophylaxis of SLE, particular agents include but are not limited to: human monoclonal antibodies (belimumab (Benlysta)), Disease-modifying antirheumatic drugs (DMARDs) such as antimalarials (e.g. plaquenil, hydroxychloroquine), immunosuppressants (e.g. methotrexate and azathioprine), cyclophosphamide and mycophenolic acid, immunosuppressive drugs and analgesics, such as nonsteroidal anti-inflammatory drugs, opiates (e.g. dextropropoxyphene and co-codamol), opioids (e.g. hydrocodone, oxycodone, MS Contin, or methadone) and the fentanyl duragesic transdermal patch.

In one embodiment, a compound of the invention is co-administered with another therapeutic agent for the treatment and/or prophylaxis of psoriasis, particular agents include but are not limited to: topical treatments such as bath solutions, moisturizers, medicated creams and ointments containing coal tar, dithranol (anthralin), corticosteroids like desoximetasone (Topicort™), fluocinonide, vitamin D3 analogues (for example, calcipotriol), argan oil and retinoids (etretinate, acitretin, tazarotene), systemic treatments such as methotrexate, cyclosporine, retinoids, tioguanine, hydroxyurea, sulfasalazine, mycophenolate mofetil, azathioprine, tacrolimus, fumaric acid esters or biologics such as Amevive™, Enbrel™, Humira™, Remicade™, Raptiva™ and ustekinumab (a IL-12 and IL-23 blocker). Additionally, a compound of the invention may be administered in combination with other therapies including, but not limited to phototherapy, or photochemotherapy (e.g. psoralen and ultraviolet A phototherapy (PUVA)).

In one embodiment, a compound of the invention is co-administered with another therapeutic agent for the treatment and/or prophylaxis of allergic reaction, particular agents include but are not limited to: antihistamines (e.g. cetirizine, diphenhydramine, fexofenadine, levocetirizine), glucocorticoids (e.g. prednisone, betamethasone, beclomethasone, dexamethasone), epinephrine, theophylline or anti-leukotrienes (e.g. montelukast or zafirlukast), anti-cholinergics and decongestants.

By co-administration is included any means of delivering two or more therapeutic agents to the patient as part of the same treatment regime, as will be apparent to the skilled person. Whilst the two or more agents may be administered simultaneously in a single formulation, i.e. as a single pharmaceutical composition, this is not essential. The agents may be administered in different formulations and at different times.

## CHEMICAL SYNTHETIC PROCEDURES

### General

The compound of the invention can be prepared from readily available starting materials using the following general methods and procedures. It will be appreciated that where typical or preferred process conditions *(i.e.* reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art (Greene, T W; Wuts, P G M;, 1991).

The following methods are presented with details as to the preparation of a compound of the invention as defined hereinabove and the comparative examples. A compound of the invention may be prepared from known or commercially available starting materials and reagents by one skilled in the art of organic synthesis.

All reagents were of commercial grade and were used as received without further purification, unless otherwise stated. Commercially available anhydrous solvents were used for reactions conducted under inert atmosphere. Reagent grade solvents were used in all other cases, unless otherwise specified. Column chromatography was performed on silica gel 60 (35-70 μm). Thin layer chromatography was carried out using pre-coated silica gel F-254 plates (thickness 0.25 mm). $^1H$ NMR spectra were recorded on a Bruker DPX 400 NMR spectrometer (400 MHz or a Bruker Advance

300 NMR spectrometer (300 MHz). Chemical shifts (δ) for H NMR spectra are reported in parts per million (ppm) relative to tetramethylsilane (δ 0.00) or the appropriate residual solvent peak, i.e. $CHCl_3$ (δ 7.27), as internal reference. Multiplicities are given as singlet (s), doublet (d), triplet (t), quartet (q), quintuplet (quin), multiplet (m) and broad (br). Electrospray MS spectra were obtained on a Waters platform LC/MS spectrometer or with Waters Acquity H-Class UPLC coupled to a Waters Mass detector 3100 spectrometer. Columns used: Waters Acquity UPLC BEH C18 1.7μm, 2.1mm ID x 50mm L, Waters Acquity UPLC BEH C18 1.7 μm, 2.1mm ID x 30 mm L, or Waters Xterra MS 5μm C18, 100 x 4.6mm. The methods are using either MeCN/$H_2O$ gradients ($H_2O$ contains either 0.1% TFA or 0.1% $NH_3$) or MeOH /$H_2O$ gradients ($H_2O$ contains 0.05% TFA). Microwave heating was performed with a Biotage Initiator.

**Table I. List of abbreviations used in the experimental section:**

| Abbreviation | Definition | Abbreviation | Definition |
|---|---|---|---|
| μL | micro litre | d | doublet |
| ALL | acute lymphoblastic leukemia | DCM | Dichloromethane |
| AML | acute myeloid leukaemia | eq. | Equivalent |
| aq | aqueous | DMF | Dimethylformamide |
| br s | broad singlet | DMA | Dimethylacetamide |
| BINAP | 2,2'-bis(diphenylphosphino)-1,1'-binaphtyl | DMAP | Dimethylaminopyridine |
| BID | Twice daily | EtOAc | Ethyl acetate |
| BLM | Bleomycin | g | Gram |
| bt | Broad triplet | GTPγS | guanosine 5'-O-[gamma-thio] triphosphate) |
| BSA | Bovine serum albumin | h | Hour |
| Cat. | Catalytic amount | HBSS | Hank's Balanced Salt Solution |
| CLL | chronic lymphoblastic leukaemia | Int | Intermediate |
| COPD | chronic obstructive pulmonary disease | IPF | idiopathic pulmonary fibrosis |
| Cpd | Compound | iPrOH | Isopropanol |
| $Cs_2CO_3$ | Cesium carbonate | $K_2CO_3$ | Potassium carbonate |
| CV | Column volumes | L | Liter |

(continued)

| Abbreviation | Definition |
|---|---|
| LiOH | Lithium hydroxide |
| m | multiplet |
| MeCN | Acetonitrile |
| MeOH | Methanol |
| mg | milligram |
| min | minute |
| mL | millilitre |
| MTBE | Methyl tButyl ether |
| MW | Molecular weight |
| MC | Methylcellulose |
| NaH | Sodium hydride |
| $NaHCO_3$ | Sodium bicarbonate |
| NASH | nonalcoholic steatohepatitis |
| $NH_4Cl$ | Ammonium chloride |
| PBS | Phosphate buffered saline |
| Pd/C | Palladium on Carbon 10% |
| $Pd_2(allyl)_2Cl_2$ | Bis(allyl)dichloropalladium (II) |
| $Pd_2(dba)_3$ | Tris(dibenzylideneacetone) dipalladium(0) |
| $PdCl_2$(dppf). DCM | [1,1'-Bis(diphenylphosphino) ferrocene] dichloropalladium( II), complex with DCM |
| $PdCl_2$dppf | [1,1'-Bis(diphenylphosphino) ferrocene] dichloropalladium(II) |
| Petr. Eth. | Petroleum ether |
| p.o. | Orally *(per os)* |
| ppm | part-per-million |
| $PPh_3$ | Triphenylphosphine |
| PK | Pharmacokinetic |
| q | quadruplet |
| q.d. | Once a day *(quo die)* |
| RT | Room temperature |
| Rpm | Rotation per minute |
| s | singlet |
| sat | saturated |
| SCX column | ion exchange sulfonic acid cross linked columns |
| SLE | systemic lupus erythematosus |
| $SiO_2$ | silica |
| SPhos Pd G2 | Chloro(2-dicyclohexyl phosphino- 2', 6' -dimethoxy-1,1'-biphenyl)[2-(2'-amino-1,1 '-biphenyl)]palladium(II) |
| t | triplet |
| Tetramethyl tBuX PhosPdG3 | CAS n# 1447963-75-8 |
| TFA | Trifluoroacetic acid |
| THF | THF |
| XantPhos | 4,5-Bis(diphenylphosphino)-9,9-dimethylxanthene |
| v/v | Volume/volume |
| MW (calc) | molecular weight calculated |
| MW (obs) | molecular weight observed |

## SYNTHETIC PREPARATION OF THE COMPOUNDS OF THE INVENTION

### General synthetic methods

### Example 1. Synthesis of intermediates towards illustratives compounds of the invention

#### *1.1. Intermediate 1: 4-methyl-2H-phthalazin-1-one*

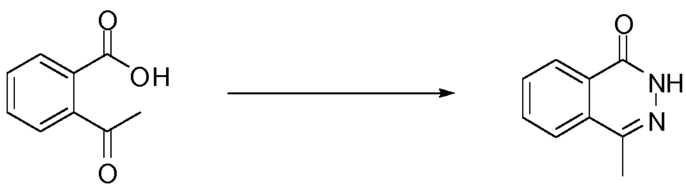

Hydrazine hydrate 78% (41 mL, 635 mmol, 1.3 eq) is added to a solution of 2-acetylbenzoic acid (80 g, 488 mmol, 1 eq) in *i*PrOH (488 mL). The mixture is stirred at 85° C for 1 h. A precipitate is formed and filtered off. The filtrate

is concentrated to give a precipitate which is filtered off. The two precipitates are combined and the resulting solid is washed abundantly with $H_2O$ (3 x 3 L). To remove the residual water, the solid is dissolved in THF and the solvent is removed under reduced pressure (2 x 1L) to yield the desired product.

### *1.2. General method A: alkylation of 4-methyl phthalazinones derivatives*

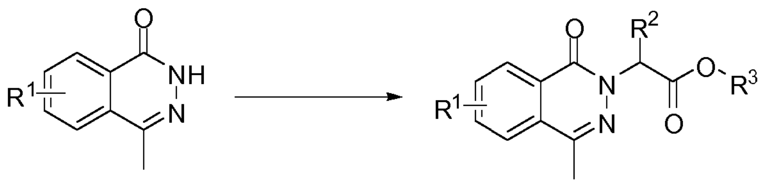

A mixture of 4-methyl-2H-phthalazin-1-one derivative (1 eq), $Cs_2CO_3$ (2 eq) and a 2-bromo ester derivative (1.1 eq) in DMF is stirred at room temperature for 2 h. The reaction mixture is diluted with an organic solvent, the mixture undergoes an aqueous work up and the organic layer is concentrated. The residue is triturated with an appropriate solvent and the resulting solid is dried under reduced pressure to give the desired product.

*Illustrative example of method A: synthesis of intermediate 2, ethyl 2-(4-methyl-1-oxo-phthalazin-2-yl)propanoate*

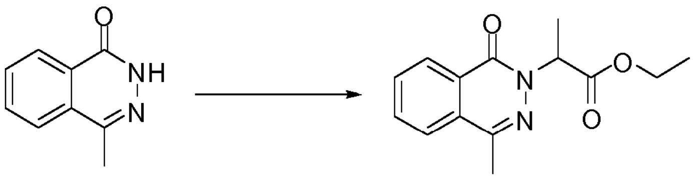

A mixture of 4-methyl-2H-phthalazin-1-one (40 g, 250 mmol, 1 eq), $Cs_2CO_3$ (163 g, 500 mmol, 2 eq) and ethyl 2-bromopropanoate (35.8 mL, 275 mmol, 1.1 eq) in dry DMF (625 mL) is stirred at room temperature for 2 h. The reaction mixture is diluted with ethyl acetate, washed ($H_2O$, $NaHCO_3$ and brine), dried ($Na_2SO_4$) and concentrated. The residue is triturated with *t*-butyl methyl ether. The resulting solid is dried under reduced pressure to give the desired product.

### *1.3. General method B: bromination of 4-methyl phthalazinones derivatives*

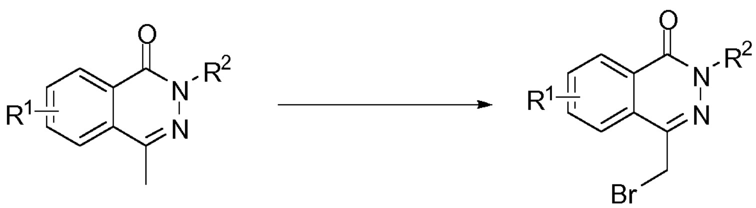

A mixture of 4-methyl phthalazinone derivative (1 eq), N-bromosuccinimide (1 eq) and benzoyl peroxide (typically 0.1 to 0.2 eq) in $CCl_4$ is stirred at reflux for 1 h. The reaction mixture is diluted with an organic solvent, the mixture undergoes an aqueous work up and the organic layer is concentrated. The residue is triturated with an appropriate solvent and the resulting solid is dried under reduced pressure to give the desired product. Flash column chromatography may be applied to further purify the desired product.

*Illustrative example of method B: synthesis of intermediate 3, ethyl 2-[4-(bromomethyl)-1-oxo-phthalazin-2-yl]propanoate*

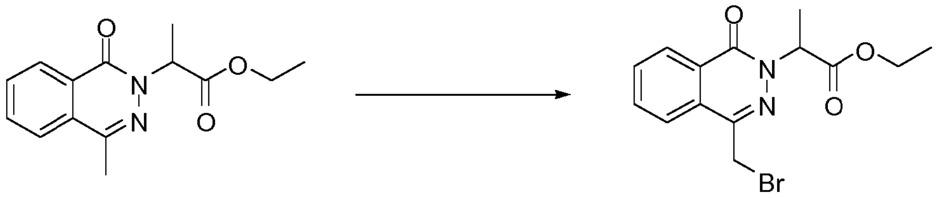

A mixture of ethyl 2-(4-methyl-1-oxo-phthalazin-2-yl)propanoate (20 g, 77 mmol, 1 eq), N-bromosuccinimide (14 g, 78 mmol, 1 eq) and benzoyl peroxide (2.5 g, 10 mmol, 0.13 eq) in $CCl_4$ (167 mL) is stirred at reflux for 1 h. The reaction mixture is washed with aqueous $NaHCO_3$ and concentrated to a quarter of its original volume. The mixture is diluted with ethyl acetate and the resulting mixture is washed with $H_2O$. The organic layer is dried ($Na_2SO_4$) and concentrated. The residue is triturated with t-butyl methyl ether and purified by flash column chromatography ($SiO_2$, petroleum ether / ethyl acetate 90:10 to 87:13) to obtain the desired product.

### *1.4. General method C: alkylation of indazolol derivatives*

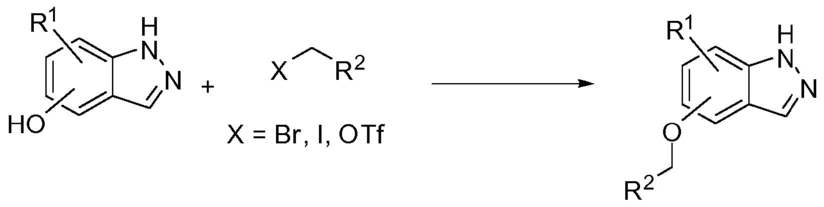


A mixture of indazolol (1 eq), alkyl halide (or alkyl triflate) (1 to 1.1 eq) and $K_2CO_3$ (1 to 2 eq) in DMF is stirred at room temperature or at 60 to 70°C for 16 h. The reaction mixture is diluted with an organic solvent, the mixture undergoes an aqueous work up and the organic layer is concentrated. The residue is triturated with an appropriate solvent and the resulting solid is dried under reduced pressure to give the desired product. Flash column chromatography may be applied to further purify the desired product.

*Illustrative example of method C: synthesis of intermediate 4, 6-bromo-5-(cyclopropylmethoxy)-1H-indazole*

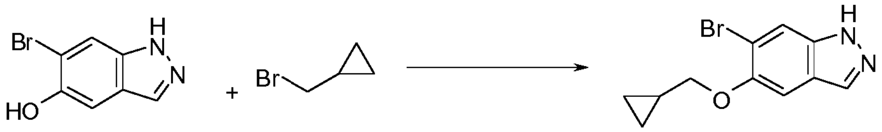

A mixture of 6-bromo-1H-indazol-5-ol (300 mg, 1.4 mmol, 1 eq), 1-(bromomethyl)cyclopropane (155 µl, 1.55 mmol, 1.1 eq) and $K_2CO_3$ (389 mg, 2.8 mmol, 2 eq) in DMF (1.9 mL) is stirred at 60°C for approximately 16 h. The reaction is diluted with ethyl acetate and washed with water. The aqueous layer is extracted with ethyl acetate and the combined organic layers are washed (water and brine), dried ($Na_2SO_4$) and concentrated. The residue is purified by flash column chromatography ($SiO_2$, DCM/methanol 100:0 to 97:3) to afford the desired product.

*Intermediate 5: 6-chloro-5-(cyclopropylmethoxy)-1H-indazole*

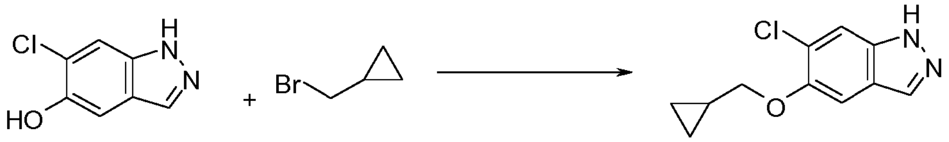

A mixture of 6-chloro-1H-indazol-5-ol (17.9 g, 106 mmol, 1 eq), 1-(bromomethyl)cyclopropane (10.3 mL, 106

mmol, 1 eq) and $K_2CO_3$ (14.7 g, 106 mmol, 1 eq) in dry DMF (150 mL) is stirred at room temperature for 16 h. The mixture is stirred at 60°C for 2 h. NaI (1.59 g, 10.6 mmol, 0.1 eq) is added and the mixture is stirred at 70°C for 6 h. The reaction is diluted with ethyl acetate and washed with water. The aqueous layer is extracted with ethyl acetate and the combined organic layers are washed (brine), dried ($Na_2SO_4$) and concentrated. The residue is purified by trituration and flash column chromatography ($SiO_2$, petroleum ether/ethyl acetate 80:20 to 70:30) to afford the desired product.

*Alternative synthesis of Intermediate 5: 6-chloro-5-(cyclopropylmethoxy)-1H-indazole*

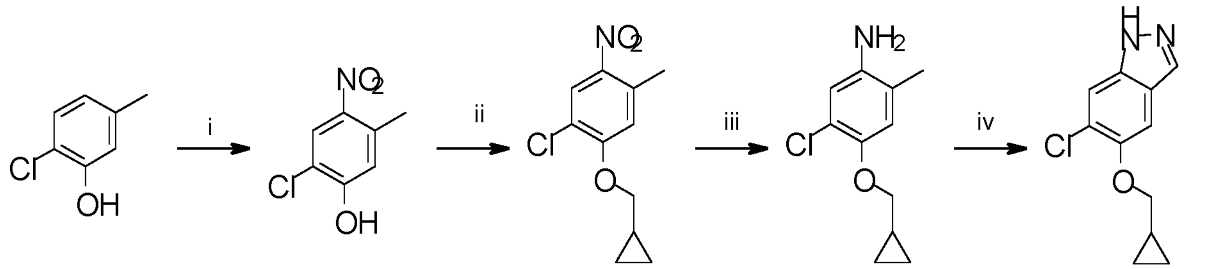


*Step i: 2-chloro-5-methyl-4-nitro-phenol*

Sodium nitrite (1.0 eq, 13.8g, 0.20 moles) dissolved in water (40 mL) is slowly added to a solution of 5-methyl-2-chlorophenol (1.0 eq, 28.5g, 0.20 moles) in acetic acid (60mL) and $H_2SO_4$ (0.75 eq, 8 mL, 0.15 moles) cooled with an iced bath. The addition is exothermic and so the addition is performed over 2 h while keeping the temperature below 10 °C. Once the addition iscompleted, the reaction mixture is stirred for 30 min at 0-10 °C. The reaction mixture is then poured into a large volume of iced water (500 mL). The suspension is filtered and the orange residue washed with water. The solid is dried on the sintered glass funnel. The orange solid is then added portionwise to a solution of nitric acid 70% (1.4 eq, 18 mL, 0.28 moles) in water (60 mL). The thick suspension is stirred at 45-50 °C for 3 h. The reaction mixture is poured into a large volume of iced water (500 mL). The suspension is then filtered and the yellow solid washed with water. The resulting material is dried to afford the expected product.Step ii: 1-chloro-2-(cyclopropylmethoxy)-4-methyl-5-nitro-benzene (Bromomethyl)cyclopropane (1.1 eq, 17.5mL, 0.18 moles) is added to a suspension of 2-chloro-5-methyl-4-nitro-phenol (1.0 eq, 30.7g, 0.164 moles) and potassium carbonate (1.5 eq, 33.9g, 0.245 moles) in NMP (150mL). The reaction mixture is then heated at 80 °C for 2 h. The reaction mixture is cooled to room temperature and poured into iced water (600 mL). The suspension is filtered and the solid washed successively with water and heptane (60 mL). The solid is dried to afford the expected product.

*Step iii: 5-chloro-4-(cyclopropylmethoxy)-2-methyl-aniline*

Zinc dust (6.0 eq, 60.0g, 0.916 moles) is added portionwise to a suspension of 1-chloro-2-(cyclopropylmethoxy)-4-methyl-5-nitro-benzene (1.0 eq, 36.9g, 0.153 moles) and ammonium chloride (10 eq, 136g, 1.527 moles) in MeOH/EtOAc/water (180mL, 180mL, 180mL). During the addition of Zinc dust, the reaction temperature is kept below 30°C with an iced-water bath. The reaction mixture is stirred at 20 °C for 20 min. The reaction mixture is filtered on Celite. The cake is washed with EtOAc (200 mL). The aqueous phase is extracted with EtOAc (100 mL), the combined organic phases are washed with aqueous NaCl, dried on $Na_2SO_4$, filtered and concentrated. The crude residue is re-slurried in methanol/water (20mL/5mL) for 1 hour at room temperature. The suspension is filtered and the solid washed with heptane (50mL). The solid is then dried to afford the expected product.

*Step iv: 6-chloro-5-(cyclopropylmethoxy)-1H-indazole*

5-chloro-4-(cyclopropylmethoxy)-2-methyl-aniline (1.0 eq, 21.0g, 0.099 moles) is dissolved in toluene (100mL). Acetic anhydride (1.5 eq, 14.1mL, 0.149 moles) and tert-butyl nitrite (1.5 eq, 17.7mL, 0.149 moles) are successively added. The reaction mixture is stirred at 95 °C for 2h30. The reaction mixture is cooled to room temperature and concentrated. Methanol (50 mL) is added to the residue. Ammonia 7M in MeOH (3.0 eq, 43 mL, 0.300 moles) is then added to the suspension. The addition being exothermic, an ice bath is used to cool down the reaction mixture. Following the addition, the reaction mixture is stirred at room temperature for 2 h until completion. Water (50 mL) is added and precipitation occurred after few minutes. The suspension is stirred at room temperature for 20 minutes and filtered. The solid is washed with water (50 mL) and dried on the sintered glass funnel overnight. A second crop is obtained from the filtrate. The two crops are combined and re-slurried in MTBE/heptane 1:1 (100 mL) for 30 min at room temperature. The suspension is filtered and the resulting solid is washed with heptane and dried to afford the expected product.

### 1.5. General method D: N alkylation of indazole derivatives with bicyclic electophiles

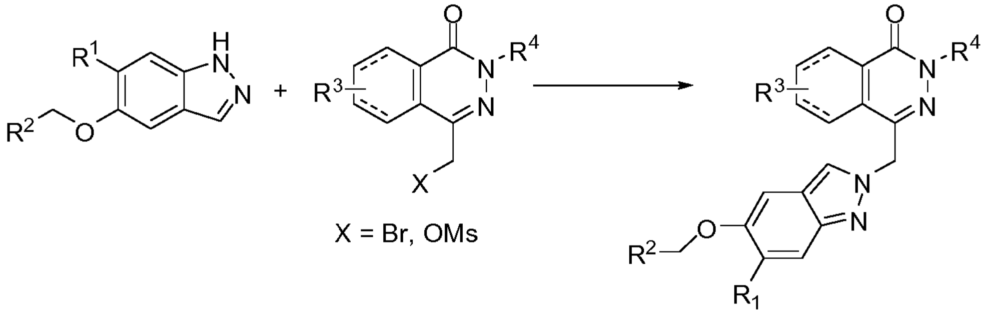

A mixture of indazole derivative (1 eq), bicyclic electrophile (1 eq) and $NaHCO_3$ (3 eq) in 1,4-dioxane is stirred for 24 to 70 h at 100°C. The reaction mixture is diluted with an organic solvent, the mixture undergoes an aqueous work up and the organic layer is concentrated. The residue is triturated with an appropriate solvent and the resulting solid is dried under reduced pressure to give the desired product. Flash column chromatography may be applied to further purify the desired product.

*Illustrative example of method D: synthesis of intermediate 6, ethyl 2-[4-[[6-chloro-5-(cyclopropylmethoxy)indazol-2-yl]methyl]-1-oxo-phthalazin-2-yl] propanoate*

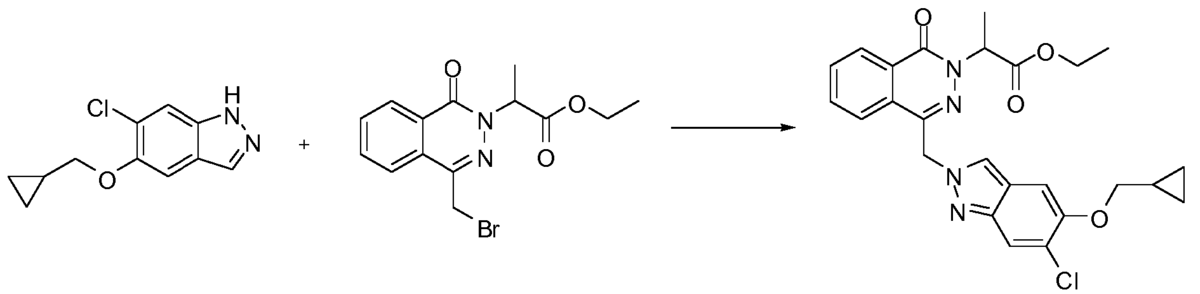

A mixture of 6-chloro-5-(cyclopropylmethoxy)-1H-indazole (7.54 g, 34 mmol, 1 eq), ethyl 2-[4-(bromomethyl)-1-oxo-phthalazin-2-yl]propanoate (11.5 g, 34 mmol, 1 eq) and $NaHCO_3$ (8.57 g, 102 mmol, 3 eq) in 1,4-dioxane (85 mL) is stirred at 100°C for approximately 70 h. The mixture is diluted with ethyl acetate, washed (water and brine), dried ($Na_2SO_4$) and concentrated. The residue is purified by flash column chromatography ($SiO_2$, petroleum ether/ethyl acetate 85:15 to 70:30) to afford the desired product.

### 1.6. General method E: bromination of isobenzofuran-1-one derivatives

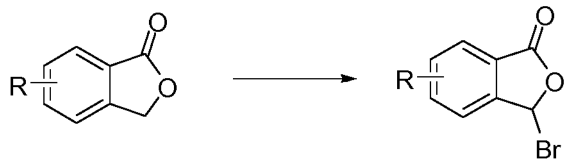

A mixture of isobenzofuran-1-one derivative (1 eq), N-bromosuccinimide (1.05 eq) and dibenzoyl peroxide (0.05 to 0.1 eq) in $CCl_4$ are stirred at 80 to 110°C for 2 to 6 h. A precipitate may be formed and filtered out. The organic mixture is concentrated and the residue is partitioned between DCM and water. The two phases are separated and the organic layer is dried and the organic layer is concentrated to afford the desired product.

*Illustrative example of method E: synthesis of intermediate 7, 3-bromo-5-fluoro-3H-isobenzofuran-1-one*

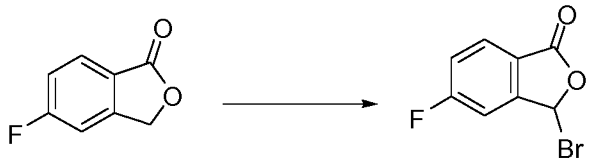

A mixture of 5-fluoro-3H-isobenzofuran-1-one (1 g, 6.6 mmol, 1 eq), N-bromosuccinimide (1.2 g, 6.8 mmol, 1.03 eq) and dibenzoyl peroxide (111 mg, 0.5 mmol, 0.07 eq) in $CCl_4$ is stirred at 80°C for 1.5 h. The precipitated is filtered and the filtrate is concentrated. The residue is partitioned between DCM and water. The two layers are separated and the aqueous layer is extracted with DCM. The combined organic layers are dried (filtered through phase separator) and concentrated to afford the desired product.

### *1.7. General method F: formation of phosponium salts of isobenzofuranone derivatives*

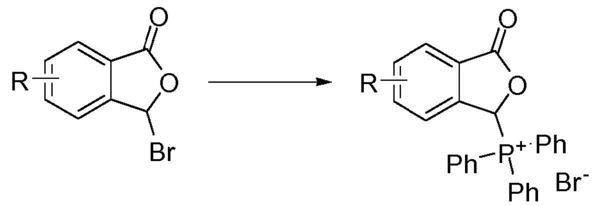

A mixture of the isobenzofuranone derivative (1 eq) and $PPh_3$ (0.95 eq) in acetonitrile is stirred at 65 to 85°C for 6 h. The reaction mixture is concentrated and the desired product is triturated with the appropriate solvent to afford the desired product.

*Illustrative example of method F: synthesis of intermediate 8, (6-fluoro-3-oxo-1H-isobenzofuran-1-yl)-triphenyl-phosphonium bromide*

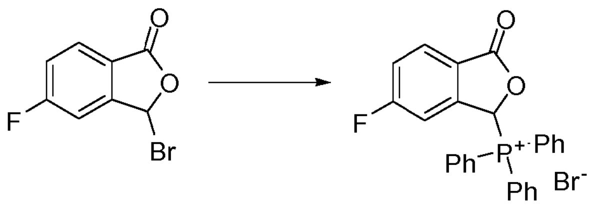

A mixture of 3-bromo-5-fluoro-3H-isobenzofuran-1-one (1.5 g, 6.4 mmol, 1 eq) and $PPh_3$ (1.6 g, 6.1 mmol, 0.95 eq) in acetonitrile (18 mL) is stirred at 65°C for 6 h. The reaction mixture is concentrated and the residue is triturated with MTBE to afford the desired product.

*Intermediate 9: 5-bromo-3-hydroxy-3H-isobenzofuran-1-one*

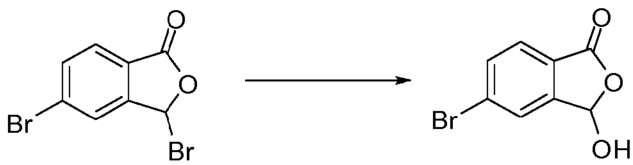

To a suspension of 5-bromo-3-bromo-3H-isobenzofuran-1-one (7 g, 24.1 mmol, 1 eq) in water (40 mL), is added powdered potassium hydroxide (2.7 g, 49 mmol, 2 eq), and the mixture is refluxed for 2 h. After cooling, potassium bisulfate (2 g) is added, and the aqueous layer is extracted with ethyl acetate (150 mL). Then the aqueous is acidified with HCl to pH 2-3 and the water layer is extracted twice more with ethyl acetate. The combined organic layers are dried (filtered through phase separator) and concentrated to afford the desired product.

### *Intermediate 10: 5-bromo-3-dimethoxyphosphoryl-3H-isobenzofuran-1-one*

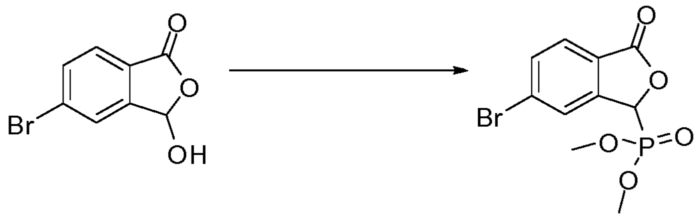

To a solution of sodium methoxide (25%) in methanol (20 mL) is added dimethyl phosphite at 0°C, and the solution is stirred at 0°C for 10 min. A suspension of 5-bromo-3-hydroxy-3H-isobenzofuran-1-one (5.3 g, 23.2 mmol, 1 eq) in anhydrous methanol (30 mL) is slowly added and the reaction mixture allowed warming to room temperature over a period of 1 h. The solution is then cooled to 0°C and methanesulfonic acid (3.31 mL, 51 mmol, 2.2 eq) is added dropwise. After the addition, the mixture is concentrated on a rotary evaporator. The concentrate is partitioned between DCM (200 mL) and water (100 mL). The organics were washed with brine (50 mL), dried (filtered through phase separator) and concentrated. The residue is dried under vacuum to yield the title compound.

### *Intermediate 11: ethyl (2Z)-2-(6-bromo-3-oxo-isobenzofuran-1-ylidene)acetate*

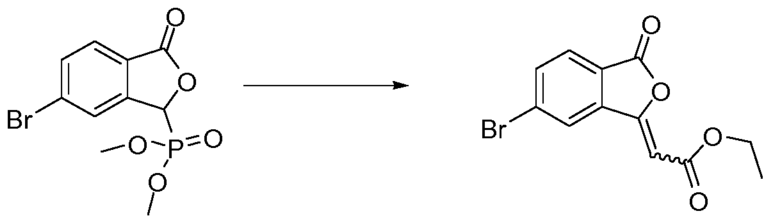

5-bromo-3-dimethoxyphosphoryl-3H-isobenzofuran-1-one (7.36 g, 23 mmol, 1 eq) is dissolved in THF, followed by addition of ethyl glyoxylate, 50% (4.69 mL, 23 mmol, 1.0 eq) and then triethylamine (3.5 mL, 25.3 mmol, 1.1 eq). The reaction is stirred at room temperature for 4 h. The mixture is evaporated partially; water (100 mL) and ethyl acetate (200 mL) were added. The layers were separated and the organic layer is washed with aqueous citric acid solution (1M) and brine. The organic layer is dried over sodium sulphate, filtered and evaporated.

## *1.8. General method G: Wittig reaction of phosponium salts of isobenzofuranone derivatives*

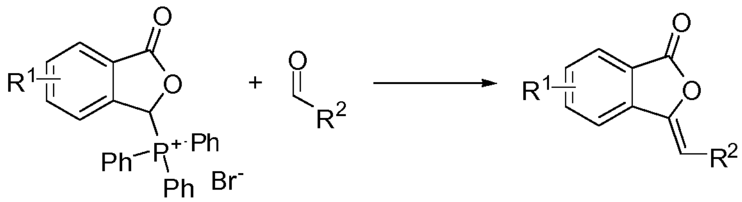

Potassium tert-butoxide (1.1 eq) is added to a cooled solution of the aldehyde (1 eq) and the phosphonium salt (1 eq) in dry acetonitrile. The resulting mixture is allowed to reach room temperature and is stirred for approximately 1.5 h. The reaction is quenched with water, diluted with an organic solvent, the mixture undergoes an aqueous work up and the organic layer is concentrated. The residue is triturated with an appropriate solvent and the resulting solid is dried under reduced pressure to give the desired product.

*Illustrative example of method G: synthesis of intermediate 12, (3Z)-3-[[5-chloro-6-(2,2-difluoroethoxy)-1-(2-trimethylsilylethoxymethyl)indol-2-yl]methylene]isobenzofuran-1-one*

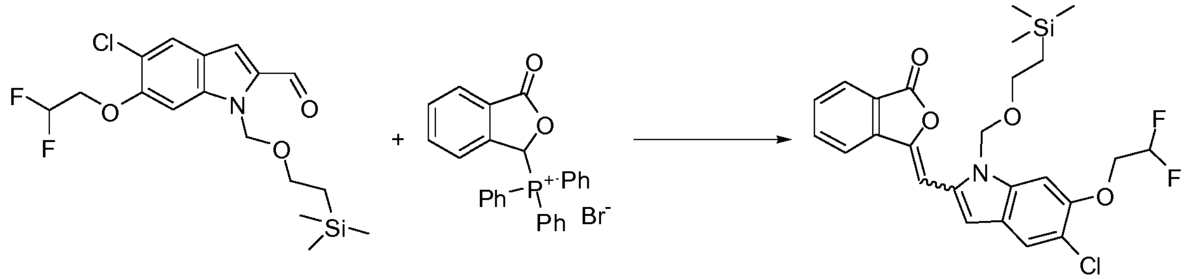

Potassium tert-butoxide (187 mg, 1.67 mmol, 1.1 eq) is added to a cooled solution of the 5-chloro-6-(2,2-difluoroethoxy)-1-(2-trimethylsilylethoxymethyl)indole-2-carbaldehyde (591 mg, 1.5 mmol, 1 eq) and (3-oxo-1H-isobenzofuran-1-yl)-triphenyl-phosphonium bromide (720 mg, 1.5 mmol, 1 eq) in dry acetonitrile (48 mL). The resulting mixture is allowed to reach room temperature and is stirred for 1.5 h. The reaction is quenched with water and extracted with ethyl acetate. The organic layer is washed (water and brine), dried ($Na_2SO_4$) and concentrated to afford the desired product.

*Intermediate 13: ethyl (2Z)-2-(7-bromo-3-oxo-isobenzofuran-1-ylidene)acetate*

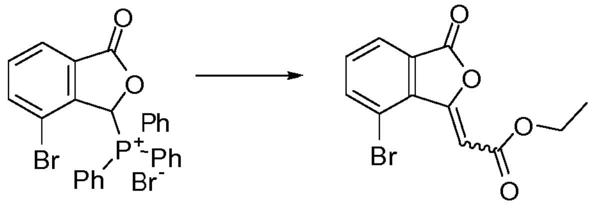

Triethylamine (1.5 mL, 10.8 mmol, 1.2 eq) is added to a cooled solution of the ethyl glyoxylate (2.4 mL, 11.7 mmol, 1.3 eq, 50% in toluene) and the (7-bromo-3-oxo-1H-isobenzofuran-1-yl)-triphenylphosphonium (5g, 9.02 mmol, 1 eq) in dry DCM (90 mL). The resulting mixture is allowed to reach room temperature and is stirred for approximately 4 h. The solvent is removed under reduced pressure. The residue is purified by flash column chromatography ($SiO_2$, petroleum ether/diethyl ether 80:20 to 50:50) to afford the desired product.

*Intermediate 14: 2-(3-oxoisobenzofuran-1-ylidene)acetic acid*

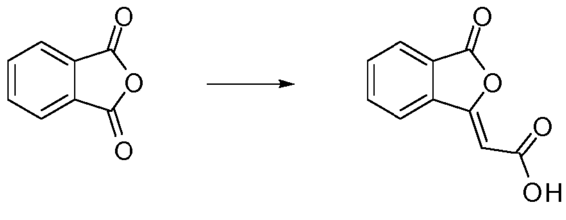

Phthalic anhydride (275 g, 1.85 mol, 1 eq) , potassium acetate (182 g, 1.85 mol, 1 eq) and acetic anhydride (369 mL) are stirred at 145-150°C for 10 min and then at 140°C for 20 min. The mixture is allowed to reach 80°C in approximately 1 h. 3 volumes of water are added to the mixture. The precipitate is filtered, washed with warm water and dried for 30 min. The solid is further washed with acetone and ethanol. The solid is dried under vacuum to afford the desired product.

### 1.9. General method H: Wittig reaction of anhydride derivatives

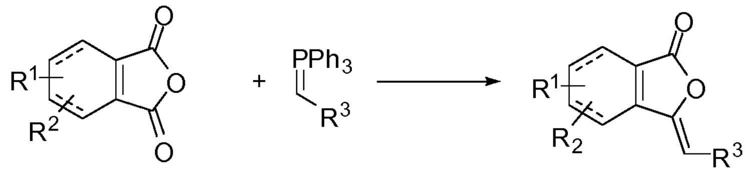

A solution of the anhydride derivative (1 eq) and the ylide (1.1 eq) in DCM is refluxed for 3 to 16 h. The solvent is removed under reduced pressure to afford the desired product. The desired product may be further purified by trituration or by flash column chromatography.

*Illustrative example of method H: synthesis of intermediate 15, tert-butyl 2-(3-oxoisobenzofuran-1-ylidene)acetate*

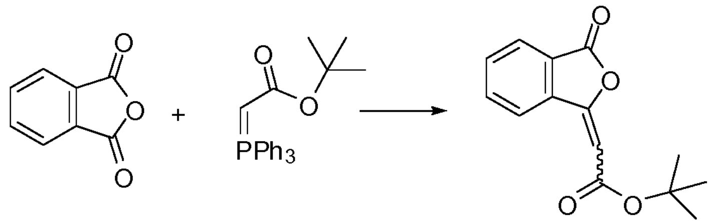

A solution of phthalic anhydride (1.7 g, 12.09 mmol, 1 eq) and tert-butyl 2-(triphenyl-λ5-phosphanylidene)acetate (5 g, 13.3 mmol, 1.1 eq) in DCM is refluxed for 3 h. The solvent is removed under reduced pressure. The residue is purified by flash column chromatography ($SiO_2$, petroleum ether/diethyl ether 80:20 to 50:50) to afford the desired product.

*Intermediate 16: 5-bromo-3-methylene-isobenzofuran-1-one*

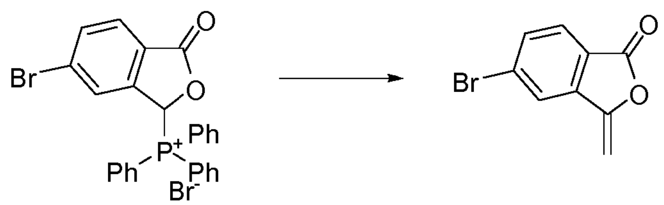

A solution of phthalic anhydride (1.7 g, 12.09 mmol, 1 eq) and tert-butyl 2-(triphenyl-λ5-phosphanylidene)acetate (5 g, 13.3 mmol, 1.1 eq) in DCM is refluxed for 3 h. The solvent is removed under reduced pressure. The residue is purified by flash column chromatography ($SiO_2$, petroleum ether/diethyl ether 80:20 to 50:50) to afford the desired product.

### 1.10. General method I: formation of phthalzinone and phthalazine derivatives

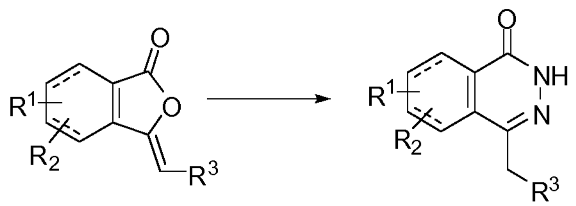

A solution of the isobenzofurane derivative (1 eq) and hydrazine (1.5 to 3 eq) in ethanol or 2-isopropanol is stirred at 80 to 110°C to approximately 16 h. The precipitated desired product is filtered off and washed with the appropriate solvent. Alternatively, the reaction is diluted with an organic solvent, the mixture undergoes an aqueous work up involving an acid wash and the organic layer is concentrated. The residue is triturated with the appropriate solvent to afford the desired product.

*Illustrative example of method I: synthesis of intermediate 17, 2-(4-oxo-3H-phthalazin-1-yl)acetic acid*

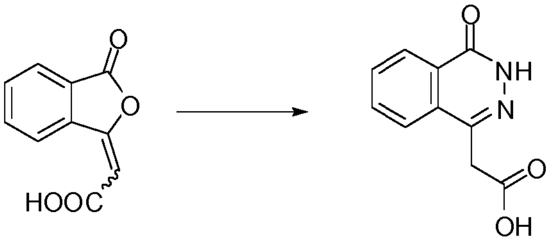

A solution of 2-(3-oxoisobenzofuran-1-ylidene)acetic acid (20 g, 105 mmol, 1 eq) and hydrazine monohydrate (13 mL, 260 mmol, 2.5 eq) in ethanol is stirred at 85°C for 2 h and then at room temperature for approximately 16 h. The precipitate is filtered and washed with ethanol to afford the desired product, which is further dried by dissolution in and subsequent concentration from THF.

*Intermediate 18: methyl 2-(4-oxo-3H-phthalazin-1-yl)acetate*

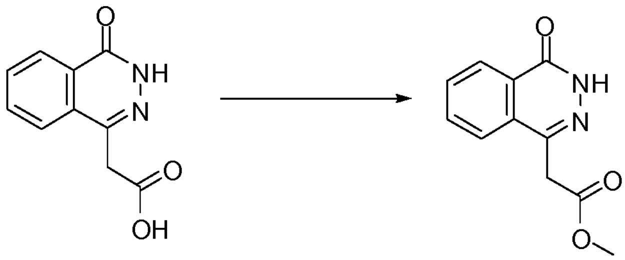

$SOCl_2$ (9.6 mL, 132 mmol, 1.2 eq) is added dropwise over 10-15 min to a solution of 2-(4-oxo-3H-phthalazin-1-yl)acetic acid (110 mmol, 1 eq) in methanol (320 mL). The mixture is stirred for 6 h at 75°C. The precipitate is filtered off and suspended in an ethyl acetate / saturated $NaHCO_3$ mixture. The resulting mixture is stirred vigorously and the two layers are separated. The organic layer is washed (1 M HCl), dried ($Na_2SO_4$) and concentrated to afford the desired product.

### *1.11. General method J: alkylation of phthalzinone, phthalazine and isoquinolinone derivatives*

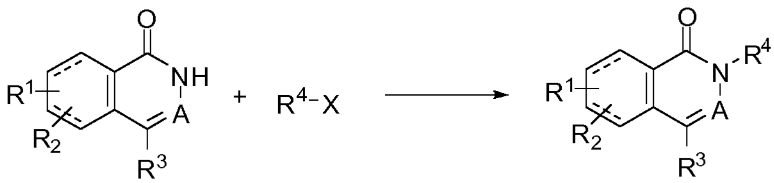

A = CH, N

A mixture of the phthalazinone, phthalazine or isoquinolinone derivative (1 eq), alkylating agent (1 eq, typically alkyl halide) and $Cs_2CO_3$ or $K_2CO_3$ (1 to 2 eq) in DMF or DMA, is stirred at 25 to 120°C for 1 to 16 h. The reaction mixture is diluted with an organic solvent, the mixture undergoes an aqueous work up and the organic layer is concentrated. The residue is typically triturated with an appropriate solvent to afford the desired product.

*Illustrative example of method J: synthesis of intermediate 19, methyl 2-[3-(2-tert-butoxy-2-oxo-ethyl)-4-oxo-phthalazin-1-yl]acetate*

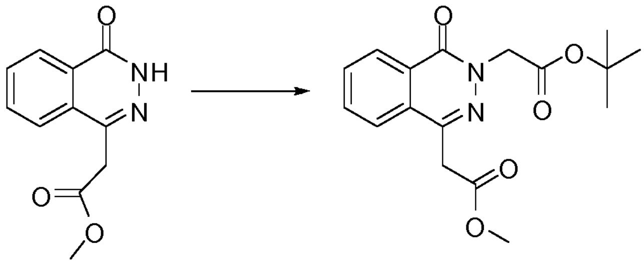

A mixture of methyl 2-(4-oxo-3H-phthalazin-1-yl)acetate (5 g, 23 mmol, 1 eq), tert-butyl bromoacetate (3.4 mL, 23 mmol, 1 eq) and $Cs_2CO_3$ (15 g, 46 mmol, 2 eq) in DMF (100 mL) is stirred at room temperature for 1.5 h. The reaction mixture is diluted with ethyl. The resulting mixture is washed (sat. $NaHCO_3$, water) and concentrated to afford the desired product.

### 1.12. General method K: alkylation of phthalzinone derivatives

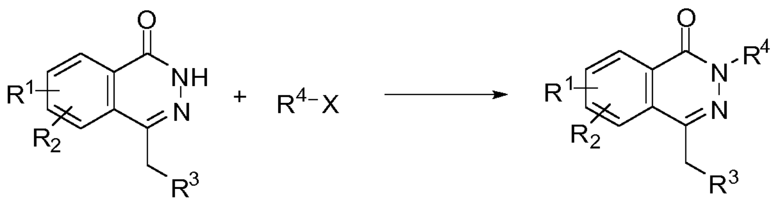

NaH (1 to 2.1 eq) is added to a mixture of the phthalazinone derivative (1 eq) in dry DMF at 0°C. The resulting mixture is let to reach room temperature and is stirred for 10 min. The alkylating agent (1 to 2 equivalents) is added and the reaction is stirred at room temperature for approximately 2 h. The reaction is quenched with water and the resulting mixture is diluted with an organic solvent and the mixture undergoes an aqueous work up. The organic layer is dried and concentrated. The residue is triturated with an appropriate solvent to afford the desired product. The product may be further purified by flash column chromatography.

*Illustrative example of method K: synthesis of intermediate 20, ethyl 2-[4-[[7-chloro-6-(cyclopropylmethoxy)imidazo[1,2-a]pyridin-2-yl]methyl]-1-oxo-phthalazin-2-yl]acetate*

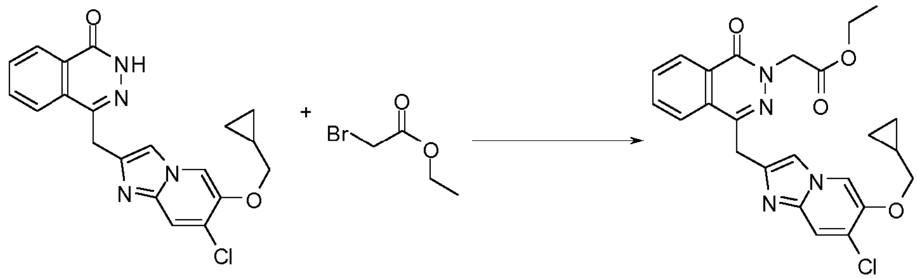

NaH 60% mineral oil (10 mg, 0.26 mmol, 2.1 eq) is added to a mixture of 4-[[7-chloro-6-(cyclopropylmethoxy)imidazo[1,2-a]pyridin-2-yl]methyl]-2H-phthalazin-1-one (50 mg, 0.12 mmol, 1 eq) in dry DMF (2 mL) at 0°C. The resulting mixture is let to reach room temperature and is stirred for 10 min. Ethyl bromoacetate (28 µl, 0.25 mmol, 2.0 eq) is added and the reaction is stirred at room temperature for 2 h. The reaction is quenched with water and the resulting mixture is extracted with ethyl acetate. The organic layer is washed (brine), dried ($Na_2SO_4$) and concentrated. The residue is purified by flash column chromatography ($SiO_2$, DCM/methanol 100:0 to 98.5:1.5) to afford the desired product.

*Intermediate 21: 2-bromo-1-morpholino-ethanone*

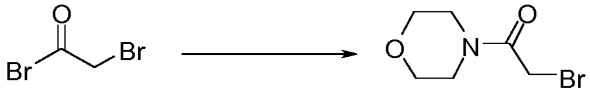

Morpholine (1.3 mL, 87 mmol, 2 eq) is added dropwise over 15 min to a cooled solution (-46°C) of 2-bromoacetyl bromide (0.65 mL, 7.4 mmol, 1 eq) in dry DCM (35 mL). After the addition, the reaction mixture is left to warm up to room temperature and stirred for 1 h. The mixture is washed (sat. $NH_4Cl$, $NaHCO_3$ and brine), dried ($Na_2SO_4$) and concentrated to afford the desired product.

## 1.13. General method L: basic hydrolysis of phthalzinone and phthalazine carboxylic esters derivatives

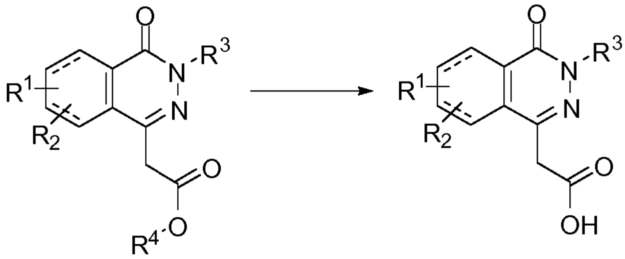

$R^4$ = Me, Et

A mixture of the ester derivative (1 eq) and $LiOH.H_2O$ (1 to 2 eq) in 1:1 methanol/water is stirred for 1 to 2 h at room temperature. The aqueous layer is acidified to pH 1-5 and extracted with an organic solvent. The organic layer is dried and concentrated to afford the desired product. Alternatively the desired product is obtained from precipitation and filtration of the acidic solution.

*Illustrative example of method L: synthesis of intermediate 22, 2-[3-(2-tert-butoxy-2-oxo-ethyl)-4-oxo-phthalazin-1-yl]acetic acid*

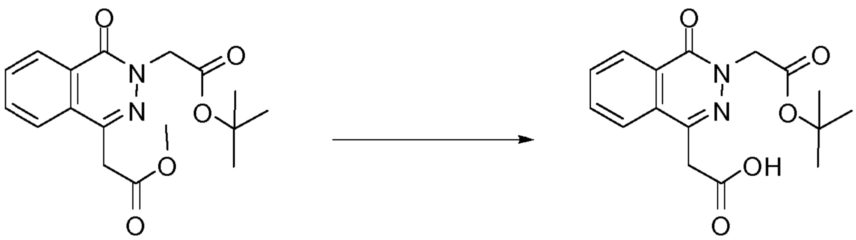

A mixture of methyl 2-[3-(2-tert-butoxy-2-oxo-ethyl)-4-oxo-phthalazin-1-yl]acetate (7.6 g, 23 mmol, 1 eq) and $LiOH.H_2O$ (1.4 g, 34.5 mmol, 1.5 eq) in 1:1 methanol/water (60 mL) is stirred at room temperature for 2 h. The mixture may be diluted (water) and washed (diethyl ether). The aqueous layer is acidified to pH 2-3 and extracted with ethyl acetate. The organic layer is dried and concentrated to afford the desired product.

### *1.14. General method M: basic hydrolysis of phthalzinone, phthalazine and isoquinolinone carboxylic esters derivatives*

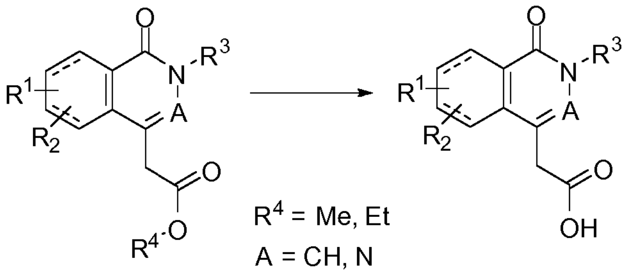


A mixture of the ester derivative (1 eq) and $LiOH.H_2O$ (1 to 2 eq) in 1:1 water/THF is stirred for 1 to 5 h at 0°C to room temperature. The aqueous layer is acidified to pH 1-5 and extracted with an organic solvent. The organic layer is dried and concentrated to afford the desired product.

*Illustrative example of method M: synthesis of intermediate 23, 2-[3-(1-tert-butoxycarbonylpyrrolidin-3-yl)-4-oxo-phthalazin-1-yl]acetic acid*

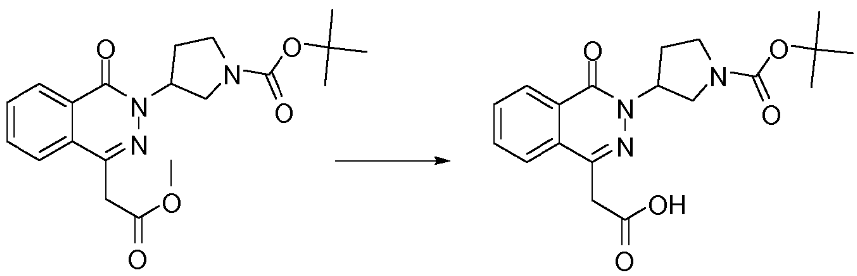

A mixture of tert-butyl 3-[4-(2-methoxy-2-oxo-ethyl)-1-oxo-phthalazin-2-yl]pyrrolidine-1-carboxylate (2.3 g, 5.95 mmol, 1 eq) and $LiOH.H_2O$ (500 mg, 11.9 mmol, 2 eq) in 1:1 methanol/THF (34 mL) is stirred for 2 h at room temperature. The reaction is quenched with saturated $NH_4Cl$ and the mixture is extracted with ethyl acetate. The two layers were separated and the organic layer was dried (filtration through phase separator) and concentrated. The aqueous layer was further acidified with 1 M HCl and extracted with ethyl acetate. The organic layer was washed (brine), dried ($Na_2SO_4$) and concentrated. The two residues were combined to afford the desired product.

*Intermediate 24: 2-[6-methoxy-3-(2-morpholino-2-oxo-ethyl)-4-oxo-phthalazin-1-yl]acetic acid*

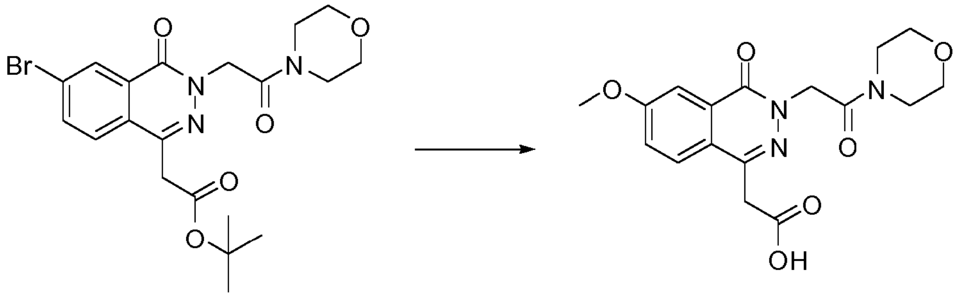

To a mixture of tert-butyl 2-[6-bromo-3-(2-morpholino-2-oxo-ethyl)-4-oxo-phthalazin-1-yl]acetate (200 mg, 0.5 mmol, 1 eq), tetramethyl tBuXPhosPdG3 (42 mg, 0.05 mmol, 0.1 eq) and $Cs_2CO_3$ (244 mg, 0.75 mmol, 1.5 eq) is added dry methanol (0.3 mL) and toluene (3 mL). The mixture is degassed with $N_2$ and then heated overnight at 70°C. Water and ethyl acetate were added. The water layer is acidified and extracted twice with ethyl acetate. The combined organic layers were washed twice with brine, dried over sodium sulphate, filtered and evaporated.

### *1.15. General method N: acidic hydrolysis of phthalzinone tert-butyl carboxylic esters derivatives*

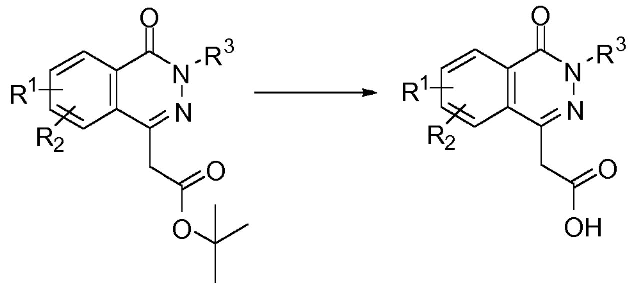

A solution of the tert-butyl carboxylic acid derivative in 3:1 to 4:1 DCM/TFA is stirred for 5 to 72 h. The mixture is concentrated and the residue is partitioned between an aqueous phase and an organic solvent. After work up, the organic layer is dried and concentrated to afford the desired product.

*Illustrative example of method N: synthesis of intermediate 25, 2-[6-morpholino-3-(2-morpholino-2-oxo-ethyl)-4-oxo-phthalazin-1-yl]acetic acid*

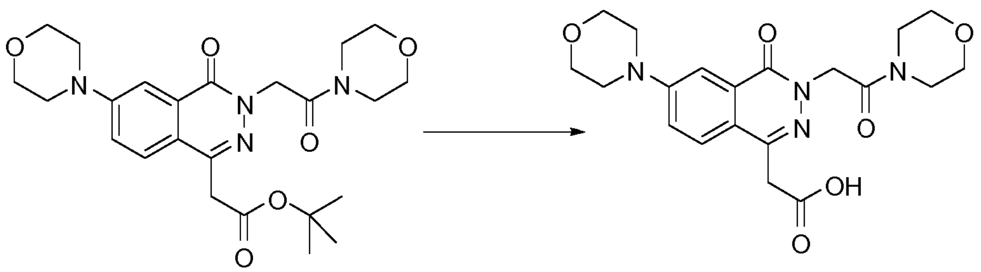

A solution of tert-butyl 2-[6-morpholino-3-(2-morpholino-2-oxo-ethyl)-4-oxo-phthalazin-1-yl]acetate (150 mg) in 3:1 DCM/TFA (4 mL) is stirred at room temperature for approximately 16 h. The mixture is concentrated and the residue is partitioned between diethyl ether and saturated $NaHCO_3$. The water layer was acidified and extracted (ethyl acetate). The organic layer was dried ($Na_2SO_4$) and concentrated to afford the desired product.

### *1.16. General method O: formation of substituted phthalazinone derivatives by reaction of isobenzofuranones with substituted hydrazines*

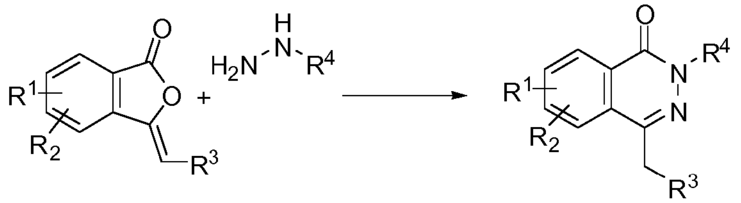

A solution of the isobenzofurane derivative (1 eq) and substituted hydrazine (2 eq) in ethanol is stirred at 80 to 90°C for 2 h. The solvent is removed under reduced pressure and the residue is purified by flash column chromatography to afford the desired product.

*Illustrative example of method O: synthesis of intermediate 26, 2-(3-cyclopropyl-4-oxo-phthalazin-1-yl)acetic acid*

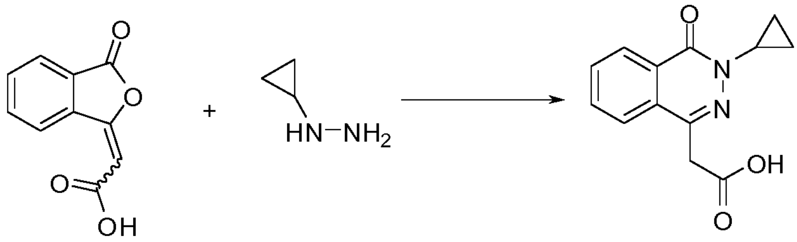

A solution of (2E)-2-(3-oxoisobenzofuran-1-ylidene)acetic acid (300 mg, 1.58 mmol, 1 eq) and cyclopropylhydrazine hydrochloride (342 mg, 3.16 mmol, 2 eq) in ethanol (15 mL) is stirred at 85°C for 2 h. The mixture is concentrated and the residue is purified by flash column chromatography ($SiO_2$, DCM/methanol 100:0 to 80:20) to afford the desired product.

*Intermediate 27: methyl 2-(7-methoxy-4-oxo-3H-phthalazin-1-yl)acetate*

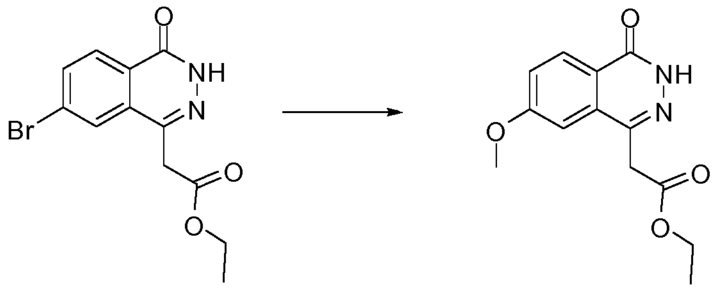

To a mixture of *ethyl* 2-(7-bromo-4-oxo-3H-phthalazin-1-yl)acetate (200 mg, 0.5 mmol, 1 eq), Tetramethyl tBuXPhosPdG3 (42 mg, 0.05 mmol, 0.1 eq) and $Cs_2CO_3$ (244 mg, 0.75 mmol, 1.5 eq) is added dry methanol (0.3 mL) and toluene (3 mL). The mixture is degassed with $N_2$ and then heated overnight at 70°C. Water and ethyl acetate were added. The layers were separated and the water layer is extracted with ethyl acetate (2x). The combined organic layers were washed twice with brine, dried over sodium sulfate, filtered and evaporated.

*Intermediate 28: methyl 2-acetyl-5-methoxy-benzoate*

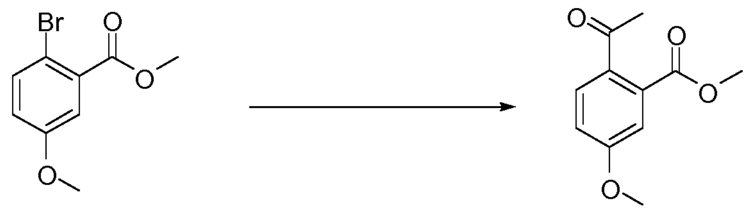

Palladium actetate (6 mg, 0.03 mmol, 0.07 eq) is added to a degassed solution of methyl 2-bromo-5-methoxybenzoate (100 mg, 0.408 mmol, 1 eq), n-butyl vinyl ether (0.26 mL, 2.04 mmol, 5.0 eq), triethylamine (0.07 mL, 0.530 mmol, 1.3 eq) and triphenylphosphine (16 mg, 0.061 mmol, 0.15 eq) in dry acetonitrile (1 mL). The mixture is stirred for 15 h at 100°C. The solvent is removed under reduced pressure. The residue is dissolved in THF (2 mL) and 2N HCl (2 mL) is added. The mixture is stirred for 2 h at room temperature. The reaction is quenched with water and extracted with DCM. The organic layer is washed with water, filtered through a phase separator and concentrated to afford the desired product.

***Intermediate 29: 7-methoxy-4-methyl-2H-phthalazin-1-one***

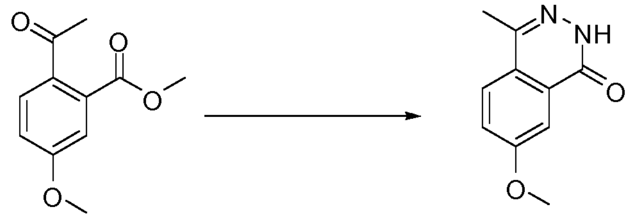

A solution of the methyl 2-acetyl-5-methoxy-benzoate (1 eq) and hydrazine hydrate (0.08 mL, 1.22 mmol, 2 eq) in isopropanol (1 mL) is stirred at 100°C for 2.5 h. The precipitate is filtered and washed with MTBE to afford the desired product.

*Intermediate 30: 2,3,5,6,7,8-hexahydrophthalazine-1,4-dione*

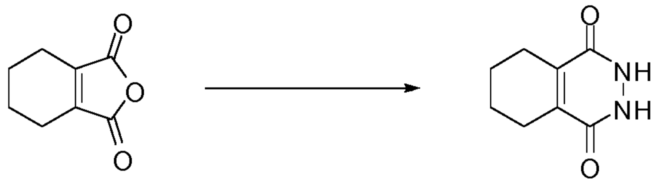

Hydrazine hydrate 78% (1.6 mL, 25.61 mmol,, 1.3 eq) is added slowly to a solution of 3,4,5,6-Tetrahydrophthalic anhydride (3 g, 19.7 mmol, 1eq) in iPrOH (24 mL) at 0° C. The mixture is allowed to warm at room temperature for 5 min and then it is heated at 90° C for 3 h. A precipitate is formed and filtered off. The precipitate is washed with $H_2O$ (3 x 30 mL). To remove the residual water, the solid is dissolved in THF and the solvent is removed under reduced pressure to yield the desired product.

*Intermediate 31: 1,4-dichloro-5,6,7,8-tetrahydrophthalazine*

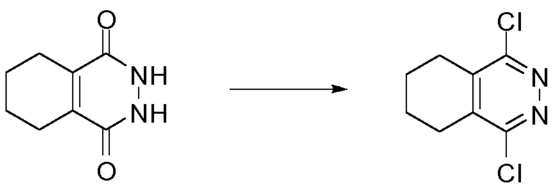

A solution of phosphoryl chloride (8.8 mL) and 2,3,5,6,7,8-hexahydrophthalazine-1,4-dione (3.2 g, 19.28 mmol, 1eq) is stirred at 90°C for 4h. The excess of phosphoryl chloride is removed under reduced pressure. The residue is dissolved in DCM (10 mL) then slowly added to an ice-water mixture. The pH is adjusted to 7-8 by the addition of solid $NaHCO_3$. The aqueous layer is extracted with DCM. The organic layer is dried ($Na_2SO_4$), filtered and concentrated to afford the desired product.

*Intermediate 32: 4-chloro-5,6,7,8-tetrahydro-2H-phthalazin-1-one*

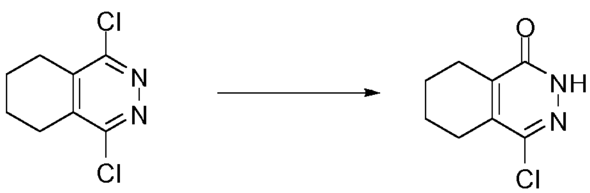

A solution of 1,4-dichloro-5,6,7,8-tetrahydrophthalazine (2.8g, 13.9 mmol, 1.0 eq), acetic acid (30 mL) and water (7 mL) is stirred at 110°C for 4h. The reaction is cooled down to room temperature. A white precipitate is formed and filtered off then washed with water. The solid is dried on high vacuum line to afford the desired product.

*Intermediate 33: 4-oxo-5,6,7,8-tetrahydro-3H-phthalazine-1-carbonitrile*

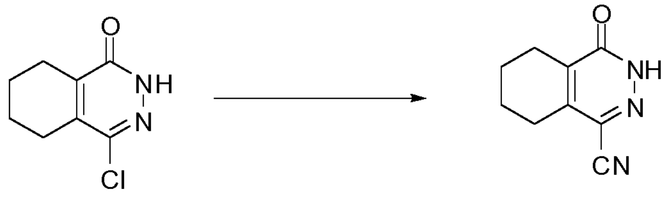

To a degassed solution of 4-chloro-5,6,7,8-tetrahydro-2H-phthalazin-1-one (300 mg, 1.6 mmol, 1eq) in dry DMF (2 mL) is added zinc cyanide (249 mg, 2.12 mmol, 1.3eq) tris(dibenzylideneacetone)dipalladium(0) (75 mg, 0.08 mmol, 0.05 eq), 1,1'-bis(diphenylphosphino) ferrocene (72 mg, 0.13 mmol, 0.08 eq). The reaction is stirred at 135°C for 2h. The mixture is filtered on celite. The filtrate is diluted with ethyl acetate (50 mL) and washed with saturated solution of $NaHCO_3$ (3x50 mL). The organic layer is washed (brine), dried ($Na_2SO_4$), filtered and concentrated to afford the desired product.

*Intermediate 34: 4-oxo-5,6,7,8-tetrahydro-3H-phthalazine-1-carboxylic acid*

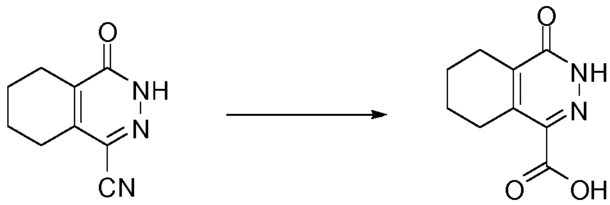

To a solution of 4-oxo-5,6,7,8-tetrahydro-3H-phthalazine-1-carbonitrile (75 mg, 0.4 mmol, 1eq) in ethanol (1 mL) is added barium hydroxide octahydrate (326 mg, 1.03 mmol, 2.4eq). The reaction is stirred at 85°C for 8h. A brown precipitate is filtered off and washed with ethanol. The formed solid is redissolved in ethyl acetate and $H_2O$. The aqueous layer is acidified with HCl 6M till pH 1 then extracted with ethyl acetate. The organic layer is dried ($Na_2SO_4$), filtered and concentrated to afford the desired product.

*Intermediate 35: ethyl 4-oxo-5,6,7,8-tetrahydro-3H-phthalazine-1-carboxylate*

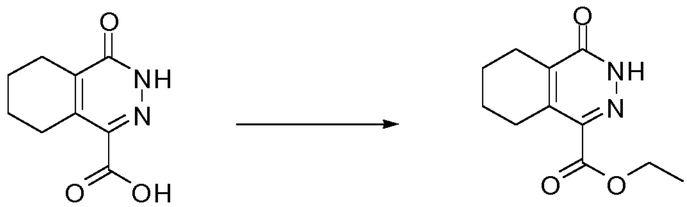

To a solution of 4-oxo-5,6,7,8-tetrahydro-3H-phthalazine-1-carboxylic acid (83 mg, 0.4 mmol, 1eq) in ethanol (0.7 mL) is added sulfuric acid (0.2 mL). The reaction is stirred at 85°C overnight. The reaction is concentrated in vacuo. The reisdue is diluted with ethyl acetate and $H_2O$. The aqueous layer is extracted with ethyl acetate. The organic layer is dried ($Na_2SO_4$), filtered and concentrated to afford the desired product.

*Intermediate 36: 4-(hydroxymethyl)-5,6,7,8-tetrahydro-2H-phthalazin-1-one*

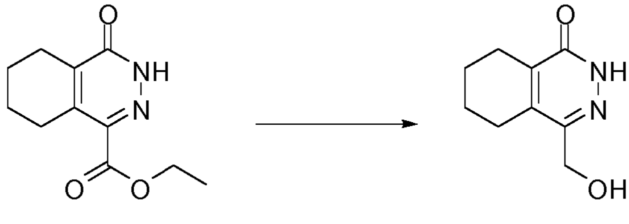

To a solution of ethyl 4-oxo-5,6,7,8-tetrahydro-3H-phthalazine-1-carboxylate (95 mg, 0.4 mmol, 1eq) in ethanol (2 mL) is added sodium borohydride (59 mg, 1.29 mmol, 3.0 eq). The reaction is stirred at room temperature for 3h. The reaction is partitioned between ethyl acetate and $H_2O$. The organic layer is dried ($Na_2SO_4$), filtered and concentrated to afford the desired product.

*Intermediate 37: ethyl 2-[4-(methylsulfonyloxymethyl)-1-oxo-5,6,7,8-tetrahydrophthalazin-2-yl]propanoate*

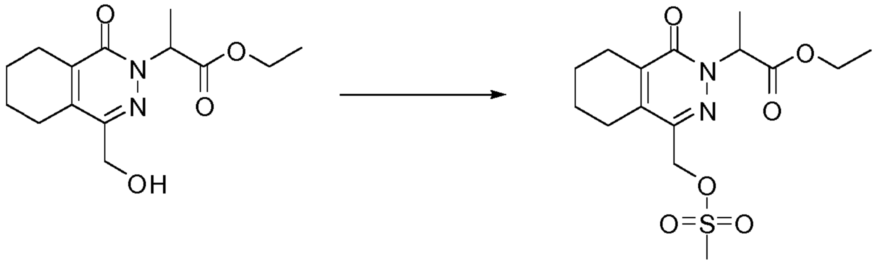

Mesylchloride (0.06 mL, 0.856 mmol, 1.2 eq) is added to a cooled solution of the ethyl 2-[4-(hydroxymethyl)-1-oxo-5,6,7,8-tetrahydrophthalazin-2-yl]propanoate (200 mg, 0.713 mmol, 1.0 eq) and the triethylamine (0.15 mL, 1.07 mmol, 1.5 eq) in dry DCM (2.4 mL). The reaction is quenched with water; DCM and saturated $NaHCO_3$ are added. The organic layer is dried and concentrated to afford the desired product.

*Intermediate 38: ethyl 2-[7-methylsulfonyl-3-(2-morpholino-2-oxo-ethyl)-4-oxo-phthalazin-1-yl]acetate*

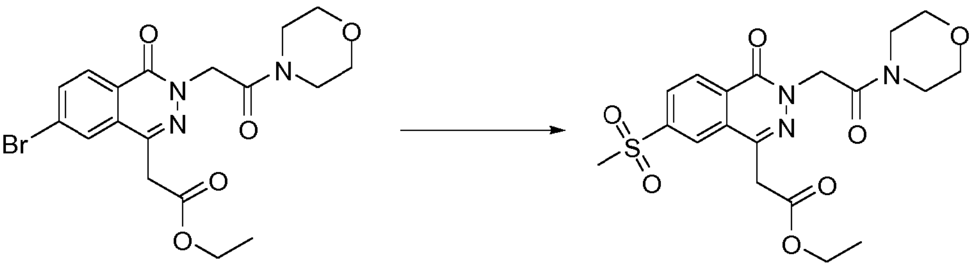

The following reagents were combined in a 5 mL vial with a stir bar: potassium metabisulfite (202 mg, 0.91 mmol, 2eq), tetraethylammonium bromide (105 mg, 0.5 mmol, 1.1 eq), sodium formate (68 mg, 1 mmol, 2.2eq), palladium acetate (5 mg, 0.023 mmol, 0.05 eq), triphenylphosphine (18 mg, 0.068 mmol, 0.15 eq), 1,10- phenanthroline (12.2 mg, 0.068 mmol, 0.15 eq) , and DMSO (2.0 mL). The mixture is degassed under $N_2$. Then *ethyl 2-[7-bromo-3-(2-morpholino-2-oxo-ethyl)-4-oxo-phthalazin-1-yl]acetate* (200 mg, 0.45 mmol, 1 eq) is added, the mixture is stirred at 75°C (external temperature) for 3.25 h and then cooled to room temperature. Methanol is added and the resulting mixture is filtered through celite. The filtrate is concentrated under reduced pressure. To the resulting crude DMSO solution is added MeI (30μL, 0.5 mmol, 1.1 eq) and the mixture is stirred overnight at room temperature. The mixture is diluted with water (10 mL) and extracted with EtOAc (3 x 20 mL), the combined organic extract is washed with brine, dried over $MgSO_4$, filtered and concentrated under reduced pressure.

*Intermediate 39: 2-[6-cyano-3-(2-morpholino-2-oxo-ethyl)-4-oxo-phthalazin-1-yl]acetic acid*

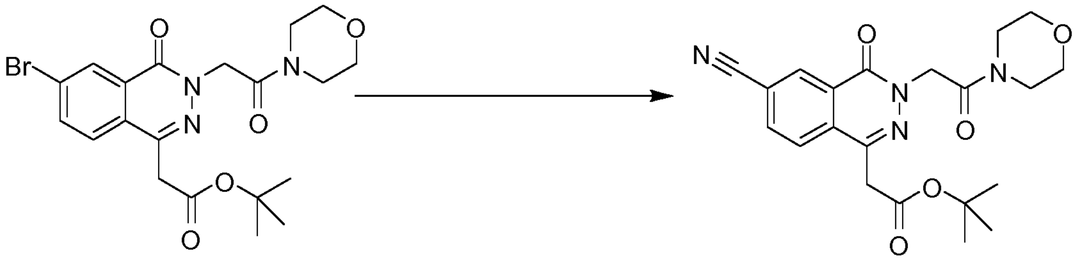

To a 2 mL flask were added tert-butyl *2-[6-bromo-3-(2-morpholino-2-oxo-ethyl)-4-oxo-phthalazin-1-yl]acetate*

(100 mg, 0.17 mmol, 1.0 eq), zinc cyanide (Caution: highly toxic) (21 mg, 0.17 mmol, 1.0 eq), Pddppf (16 mg, 0.02 mmol, 0.11 eq), zinc formate dehydrate (1.5 mg, 0.01 mmol, 0.05 eq) and DMA (1 mL). The resulting slurry is heated under nitrogen to 150°C for 10 min in the μw. The reaction mixture is cooled to rt and diluted with 10 mL of EtOAc. The resulting slurry is filtered over celite and the cake is rinsed with EtOAc (15 mL). The product is isolated by washing the filtrate with 5% $NH_4OH$ (1 x 10 mL) and water. The organic layer is dried with $Na_2SO_4$, filtered and evaporated to dryness to afford the desired product.

*Intermediate 40: tert-butyl 2-[6-morpholino-3-(2-morpholino-2-oxo-ethyl)-4-oxo-phthalazin-1-yl]acetate*

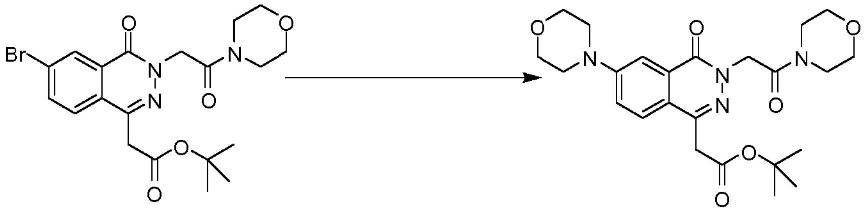

A vial is charged with morpholine (104 μL, 1.2 mmol, eq), RuPhosprecatG2 (20 mg, 0.026 mmol, 0.06 eq) and $Cs_2CO_3$ (390 mg, 1.2 mmol, 3.0 eq) and tert-butyl 2-[6-bromo-3-(2-morpholino-2-oxo-ethyl)-4-oxo-phthalazin-1-yl]acetate (200 mg, 0.43 mmol, 1.0 eq). The test tube is sealed with a cap lined with a disposable teflon septum, evacuated, purged and degassed with $N_2$. The mixture is stirred at 100°C for 3h. The reaction mixture is diluted with water and extracted with EtOAc. The combined organic layers were washed with brine and dried ($MgSO_4$). The solvent is filtered and evaporated to afford the desired product.

*Intermediate 41: 4-chloro-5-ethoxy-2-nitro-aniline*

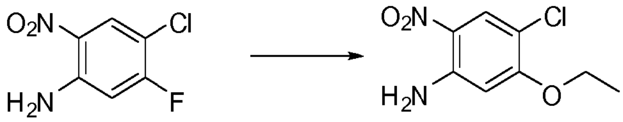

A solution of 4-chloro-5-fluoro-2-nitro-aniline (1g, 5.25 mmol) in absolute EtOH (10 mL) is added at room temperature to a solution of sodium ethoxide (20% solution in ethanol, 2 mL, 1.1 eq) in absolute EtOH (15 mL). The reaction mixture is heated to reflux. After 3 h, 310 μL of NaOEt (20% solution in EtOH, 0.2 eq) is added to the mixture, and the heating is continued for 3 h more. After cooling down to room temperature, the pH of the reaction mixture is adjusted to ~pH 3-4 via the addition of a few drops of 2N aqueous HCl. The resulting precipitate is isolated and dried under vacuum, to afford the desired product.

*Intermediate 42: 4-amino-3-chloro-2-(2,2-difluoroethoxy)-5-nitro-benzonitrile*

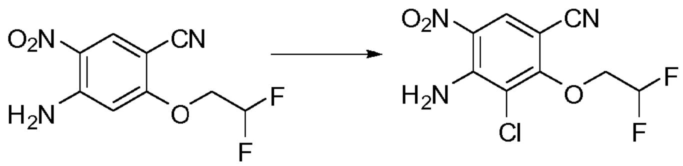

N-chloro-succinimide (147 mg, 2.16 mmol, 1.05 eq) is added at room temperature under nitrogen atmosphereto a solution of 4-amino-2-(2,2-difluoroethoxy)-5-nitro-benzonitrile (500 mg, 2.06 mmol) in dry acetonitrile (100 mL). The reaction mixture is stirred at room temperature for 1 h. The mixture is heated at 60°C for 3.5 h. The solvent is removed. The residue is partitioned between ethyl acetate (100 mL) and saturated aqueous $NaHCO_3$. The aqueous layer is once more extracted with ethyl acetate; the combined organic layer is washed with brine, dried over $Na_2SO_4$ and evaporated under vacuum, to the desired product.

### *1.17. General method P: $SN_{Ar}$ reaction of alcholates on activated aromatic substrates*

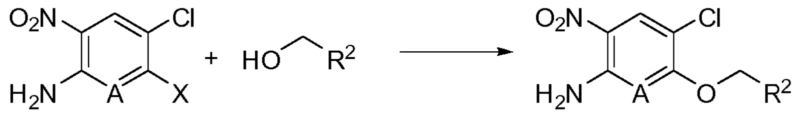

X = F, A = CH, CF
X = Cl, A = N

A mixture of potassium tert-butoxide (1.2 eq) and the alcohol (11 to 13 eq) in dry THF is stirred for 15 to 30 min at 0°C. A solution of the activated aromatic compound (1 eq) in THF is added dropwise while maintaining the temperature at 0°C. The reaction is stirred at 70°C for 1 to approximately 16 h. The mixture is partitioned between water and an organic solvent. The pH is adjusted to 4 and the two layers are separated. The organic layer is dried ($Na_2SO_4$) and concentrated to afford the desired product. The desired product may be further purified by flash column chromatography.

*Illustrative example of method P: synthesis of intermediate 43, 4-chloro-5-(cyclopropylmethoxy)-2-nitro-aniline*

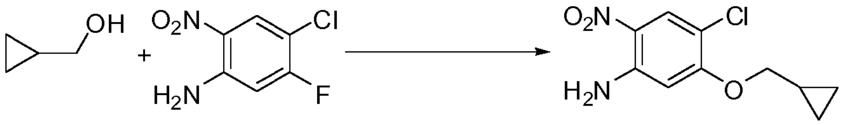

A mixture of potassium tert-butoxide (3.5 g, 31.5 mmol, 1.2 eq) and cyclopropyl methanol (23 mL, 289 mmol, 11 eq) in dry THF (25 mL) is stirred for 30 min at 0°C. A solution of 4-chloro-5-fluoro-2-nitroaniline (5 g, 26 mmol, 1 eq) in dry THF (25 mL) is added dropwise over 30 min while maintaining the temperature at 0°C. The reaction is stirred at 70°C for 2 h. The mixture is partitioned between water and ethyl acetate. The mixture is stirred vigorously at 0°C while the pH is adjusted to 4 by adding 2 N HCl. The two layers are separated and the aqueous layer is further extracted with ethyl acetate. The organic layer is dried ($Na_2SO_4$) and concentrated. The residue is purified by flash column chromatography ($SiO_2$, petroleum ether/ethyl acetate 90:10 to 70:30) to afford the desired product.

*Intermediate 44: (4-Methoxycarbonylmethyl-1-oxo-1H-isoquinolin-2-yl)-acetic acid tert-butyl ester*

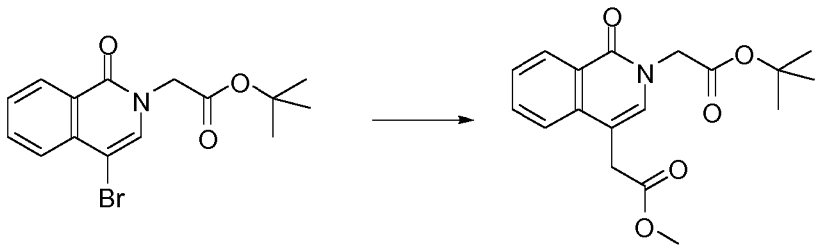

A mixture of $Pd_2(allyl)_2Cl_2$ (7.3 mg, 0.02 mmol, 0.02 eq), BINAP (37 mg, 0.06 mmol, 0.02 eq), DMAP (12 mg, 0.10 mmol, 0.10 eq) and methyl potassium malonate (234 mg, 1.50 mmol, 1.50 eq) are mixed under $N_2$ in a glass reactor. The reaction mixture is purged 3 times by vacuum/$N_2$ cycles. The sonicated suspension of (4-Bromo-1-oxo-1H-isoquinolin-2-yl)-acetic acid tert-butyl ester (338 mg, 1.0 mmol, 1.0 eq) in 2 mL mesitylene is added. The mixture is stirred and purged by vacuum/$N_2$ once. After stirring for 10 min at RT, the reactor is heated in a metal block kept at 140°C. After 22 h LCMS shows complete conversion of the limiting reagent. The reaction mixture is cooled to RT, partitioned between EtOAc and satd. $NaHCO_3$, the org. layer is washed with satd. NaCl, dried on $Na_2SO_4$, filtered and evaporated in vacuo to yield a brown oil. The crude is loaded onto a 20 g Flashmaster silica column pre-equilibrated with 6% EtOAc in petroleum ether; elution is carried out with 6% to 50% EtOAc in petroleum ether over 12 CV, to yield the desired product.

*Intermediate 45: (3-Methoxycarbonylmethyl-4-oxo-4H-cinnolin-1-yl)-acetic acid tert-butyl ester*

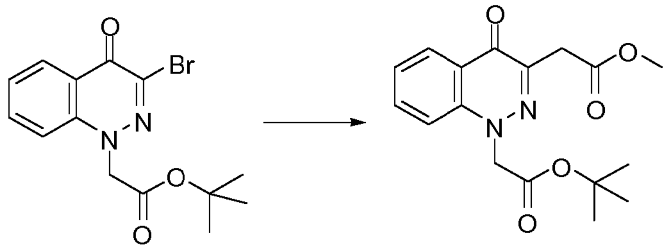

$Pd_2(allyl)_2Cl_2$ (33 mg, 0.09 mmol, 0.03 eq), Xantphos (156 mg, 0.27 mmol, 0.09 eq),, DMAP (367 mg, 3.0 mmol, 1.00 eq) and methyl potassium malonate (1.41 g, 9.0 mmol, 3.0 eq) are mixed in a MW tube under $N_2$. The tube is sealed and purged 3 times by vacuum/$N_2$ cycles. (3-Bromo-4-oxo-4H-cinnolin-1-yl)-acetic acid tert-butyl ester (1.02 g, 3.0 mmol, 1.0 eq) and 9 mL mesitylene are added. The mixture is stirred and purged by vacuum/$N_2$ once. After stirring for 10 min at RT, the mixture is heated in a metal block kept at 150°C. After 6 h, LCMS shows incomplete conversion of the limiting reagent, but no apparent increase of product. The reaction mixture is cooled to RT, treated with 150 mL EtOAc, 50 mL $H_2O$ and 50 mL satd. $NaHCO_3$. The resulting biphasic mixture is stirred. Some tars form. The mixture is filtered on a Pall Seitz 300 thick paper filter. The filtrate is separated, the org. layer is washed with 50 mL satd. NaCl, dried on $Na_2SO_4$, filtered and evaporated in vacuo to yield a brown oil with black tarry residues.

The crude is loaded onto a 70 g Flashmaster silica column pre-equilibrated with 8% EtOAc in Petr. Eth.; elution is carried out with 8% to 100% EtOAc in Petr. Eth. over 12 CV, to yield the desired product.

*Intermediate 46: (3-Bromo-4-oxo-4H-cinnolin-1-yl)-acetic acid tert-butyl ester*

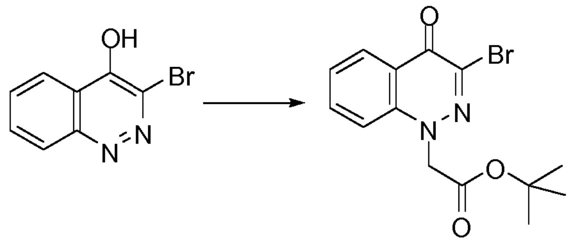

3-bromocinnolin-4-ol (2.25 g, 10.0 mmol, 1.0 eq) is mixed with 50 mL dry THF in a RB flask under $N_2$, and stirred at RT. KOtBu (1.74 g, 15.5 mmol, 1.55 eq) is added. tBu-bromoacetate (2.42 g, 12.4 mmol, 1.24 eq) is added dropwise over 2 min. The mixture is heated in a metal block kept at 60°C.

After 1 h LCMS shows complete conversion of the limiting reagent. The reaction mixture is cooled to RT, evaporated in vacuo, the residue is treated with 150 mL DCM, 50 mL satd. aq. $NaHCO_3$ and 50 mL $H_2O$. After extraction and partition the aq. layer is further extracted with 50 mL DCM. The combined org. layer is washed with 30 mL satd. aq. NaCl, dried on $Na_2SO_4$, filtered and evaporated in vacuo to yield 3.89 g brown solid/oil. The crude is left in vacuo at RT overnight.

The crude is loaded onto a 70 g Flashmaster silica column pre-equilibrated with 10% EtOAc in Petr. Eth.; elution is carried out with 16% to 66% EtOAc in Petr. Eth. , to yield the desired product.

*Intermediate 47: (1-tert-Butoxycarbonylmethyl-4-oxo-1,4-dihydro-cinnolin-3-yl)-acetic acid*

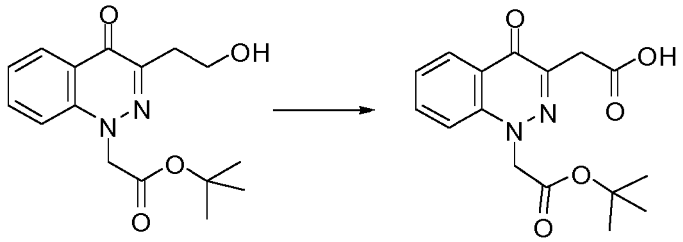

[3-(2-Hydroxy-ethyl)-4-oxo-4H-cinnolin-1-yl]-acetic acid tert-butyl ester (1.296 g, 4.26 mmol, 1.00 eq) is mixed with 21 mL DCM in a RB flask under $N_2$. Dess-Martin periodinane (2.71 g, 6.39 mmol, 1.50 eq) is added in one go, and

the mixture is stirred at RT. After 2 h, LCMS shows complete conversion to the expected aldehyde intermediate. The reaction mixture is diluted with 200 mL EtOAc, the resulting suspension is washed with 50 mL satd. $NaHCO_3$, 50 mL 5% $Na_2S_2O_3$, 25 mL satd. NaCl, dried on $Na_2SO_4$, filtered and evaporated in vacuo to yield the crude product. The material is dissolved in 34 mL tBuOH + 8.5 mL $H_2O$. $NaH_2PO_4.H_2O$ (899 mg, 6.52 mmol, 1.53 eq) and 2-methyl-2-butene (1.81 mL, 17.0 mmol, 4.00 eq) are added. The mixture is stirred at RT. NaOClO (1.16 g, 12.78 mmol, 3.00 eq) is added in one go. The mixture is further stirred at RT. After 1 h, LCMS shows complete conversion to a peak showing the expected MS of the final product (carboxylic acid). The reaction mixture is treated with 25 mL 10% aq. NaHSO3, 2M citric acid (~6 mL) to pH ~3, and extracted with 2x100 mL EtOAc. The org. layer is washed with 25 mL satd. NaCl (treated with 1 drop 2M citric acid to make it acidic), dried on $Na_2SO_4$, filtered and evaporated in vacuo to yield the desired product.

*Intermediate 48: [3-(2-Hydroxy-ethyl)-4-oxo-4H-cinnolin-1-yl]-acetic acid tert-butyl ester*

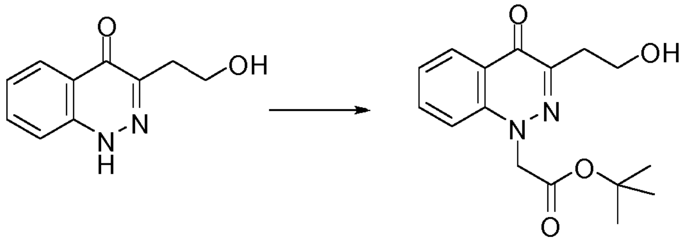

3-(2-Hydroxy-ethyl)-1H-cinnolin-4-one (951 mg, 5.00 mmol, 1.00 eq) is mixed with 10 mL DMF and $K_2CO_3$ (760 mg, 5.50 mg, 1.10 eq) under $N_2$, and stirred for 5 min at RT. tBu-bromoacetate (1024 mg, 5.25 mmol, 1.05 eq) is added in one go. The mixture is further stirred at RT. After 1h, LCMS shows complete conversion to a peak showing the MS of the desired product. The reaction mixture is diluted with 70 mL $H_2O$, the resulting suspension is stirred, cooled to 0÷5°C and filtered on Buchner. The solid is washed with 20 mL $H_2O$, dried under suction and then in vacuo at 42°C to yield the desired product.

*Intermediate 49: methyl 5-chloro-2-fluoro-4-hydroxy-benzoate*

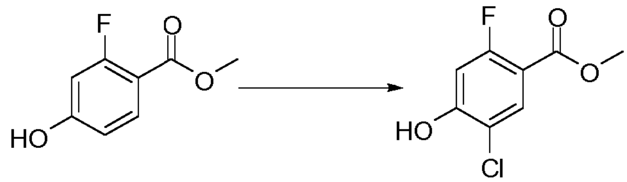

*Methyl 2-fluoro-4-hydroxy-benzoate* (1.0g, 5 mmol, 1.0 eq) is dissolved in MeCN (20 mL) and NCS (732 mg, 5.5 mmol, 1.1 eq) is added. The mixture is stirred at 60°C for 1h. Then, another portion of NCS (333 mg, 2.5 mmol, 0.5 eq) is added. Water and ethyl acetate were added to the mixture. The ethyl acetate layer is separated and washed with 0.5M citric acid and brine. The organic layer is dried over sodium sulphate, filtered and evaporated to yield 1000 mg of crude yellowish oil, containing mixture of 3- and 5-chloro isomers. The crude is used as such in the next step.

*Intermediate 50: methyl 5-chloro-4-ethoxy-2-fluoro-benzoate*

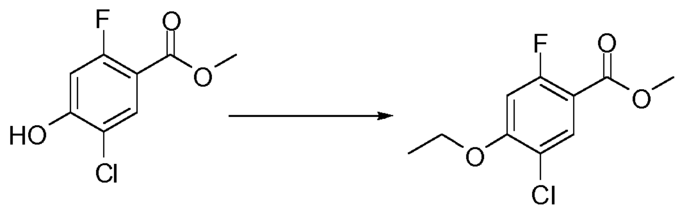

methyl 5-chloro-4-ethoxy-2-fluoro-benzoate (crude 1000 mg, 5 mmol, 1 eq), $Cs_2CO_3$ (1625 mg, 6 mmol, 1.2 eq) were added to a flask containing DMF (10 mL). Then, iodoethane (433μL, 5.5 mmol, 1.1 eq) is added and the mixture is stirred for 10 min at 0°C and for 1h at rt. Water and ethyl acetate were added to the mixture. The ethyl acetate layer is separated, washed with 0.5M citric acid and brine. It is dried over sodium sulphate, filtered and evaporated. Purification is done on a 24 g silica column, from 0 to 33% EtOAc in Petr. Eth. over 15 column volumes to afford the desired product.

*Intermediate 51: 5-chloro-6-ethoxy-1,2-dihydroindazol-3-one*

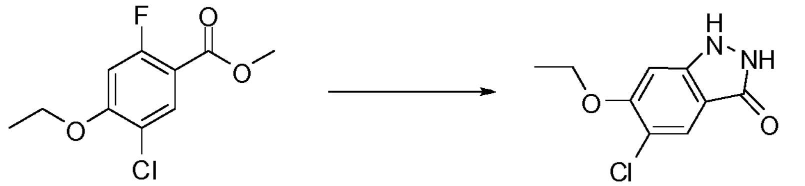

Methyl 5-chloro-4-ethoxy-2-fluoro-benzoate (232 mg, 1.0 mmol, 1.0 eq) is dissolved in n-butanol (2 mL), hydrazine hydrate (80%) (156 μL, 2.5 mmol, 2.5 eq) is added, and the mixture is stirred for 50 min at 160 °C in the microwave in a sealed reaction vessel. After cooling, the precipitate of the reaction solution is filtered and washed with n- butanol, to give the title compound.

*Intermediate 52: tert-butyl 2-[4-[(5-chloro-6-ethoxy-3-oxo-1H-indazol-2-yl)methyl]-1-oxo-phthalazin-2-yl]acetate*

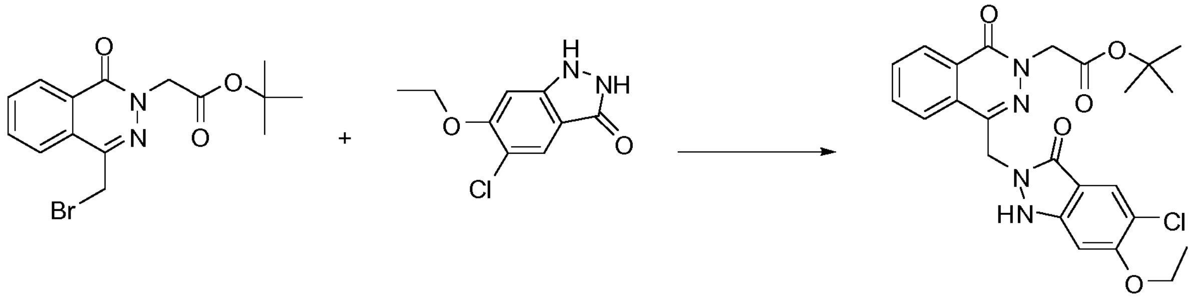

5-chloro-6-ethoxy-1,2-dihydroindazol-3-one (90 mg, 0.42 mmol, 1.0 eq) and tert-butyl 2-[4-(bromomethyl)-1-oxo-phthalazin-2-yl]acetate (150 mg, 0.42 mmol, 1.0 eq) were dissolved in DMA (1.5 mL) in a vial. The vial is capped and heated to 90°C for 3h. The mixture is diluted with ethyl acetate, water and aqueous sodium bicarbonate. The ethyl acetate layer is washed with brine, dried over sodium sulphate, filtered and evaporated to give the title compound.

*Intermediate 53: methyl 5-chloro-4-(cyclopropylmethoxy)-2-fluoro-benzoate*

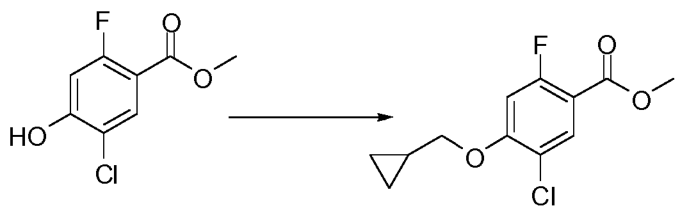

Methyl 5-chloro-4-ethoxy-2-fluoro-benzoate (crude 1000mg, 5 mmol, 1 eq), $Cs_2CO_3$ (1625 mg, 6 mmol, 1.2 eq) were added to a flask containing DMF (10 mL). Then, bromomethyl cyclopropane (590 μL, 5.5 mmol, 1.1 eq) is added and the mixture is stirred for 10 min at 0°C and for 1h at rt. Water and ethyl acetate were added to the mixture. The ethyl acetate layer is separated, washed with 0.5M citric acid and brine. It is dried over sodium sulphate, filtered and evaporated. Purification is on a 24g silica column, from 5% to 10% diethyl ether in Petr.Eth over 15 column volumes to afford the desired product.

*Intermediate 54: 5-chloro-6-(cyclopropylmethoxy)-1,2-dihydroindazol-3-one*

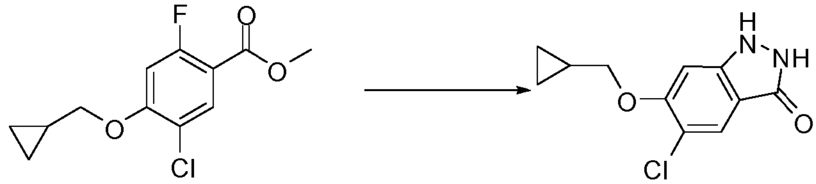

Methyl 5-chloro-4-(cyclopropylmethoxy)-2-fluoro-benzoate as a yellowish oil (200 mg, 0.77 mmol, 1.0 eq) is dissolved in n-butanol (2 mL), hydrazine hydrate (80%) (120 μL, 1.92 mmol, 2.5 eq) is added, and the mixture is stirred for 40 min at 150 °C in the microwave in a sealed reaction vessel. After cooling, citric acid and DCM were added, then a precipitate formed. After filtering the precipitate of the reaction solution, it is washed with ethyl acetate, to give the title compound with traces of the dechlorinated product.

*Intermediate 55: tert-butyl 2-[4-[[5-chloro-6-(cyclopropylmethoxy)-3-oxo-1H-indazol-2-yl]methyl]-1-oxo-phthalazin-2-yl]acetate*

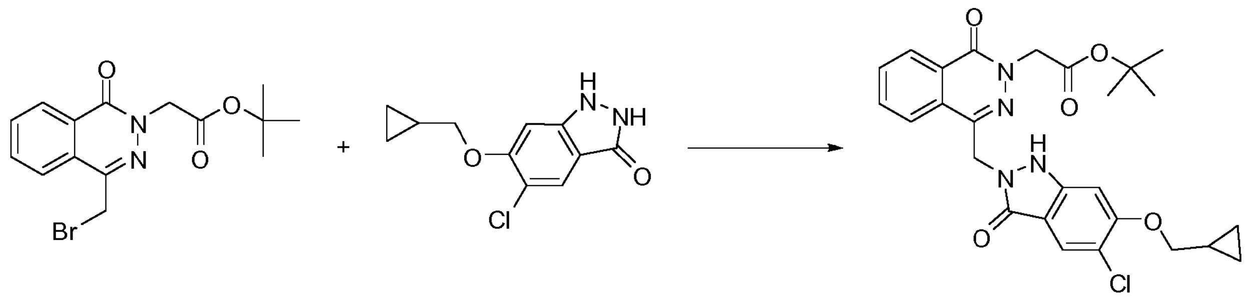

A mixture of 5-chloro-6-(cyclopropylmethoxy)-1,2-dihydroindazol-3-one (50 mg, 0.21 mmol, 1.0 eq), tert-butyl 2-[4-(bromomethyl)-1-oxo-phthalazin-2-yl]acetate (75 mg, 0.21 mmol, 1.0 eq) and sodium bicarbonate (27 mg, 0.31 mmol, 1.5 eq) is dispersed in dioxane (1.5 mL) in a vial. The vial is capped and heated to 110°C for 8h. The mixture is diluted with ethyl acetate, water and aqueous sodium bicarbonate. The ethyl acetate layer is washed with brine, dried over sodium sulphate, filtered and evaporated. The compound is purified by preparative HPLC.

*Intermediate 56: ethyl 2-(2-bromoacetyl)benzoate*

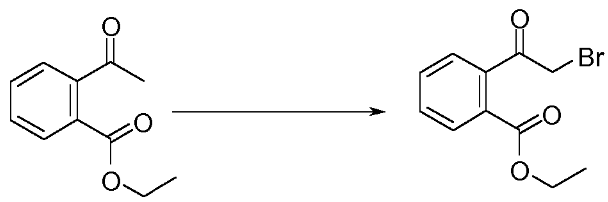

A mixture of ethyl 2-(2-bromoacetyl)benzoate (53 mg, 0.26 mmol, 1 eq), N-bromosuccinimide (48 mg, 0.27 mmol, 1.02 eq) and p-TsOH.$H_2O$ (50 mg, 0.26 mmol, 1 eq) in acetonitrile (140.0 μL) is stirred at 50°C for 6 h. The reaction mixture is diluted (ethyl acetate), washed (saturated $NaHCO_3$ and brine), dried ($Na_2SO_4$) and concentrated. The residue is purified by flash column chromatography ($SiO_2$, cyclohexane/DCM 100:0 to 20:80) to afford the desired product.

*Intermediate 57: ethyl 2-[2-[6-chloro-5-(cyclopropylmethoxy)indazol-2-yl]acetyl]benzoate*

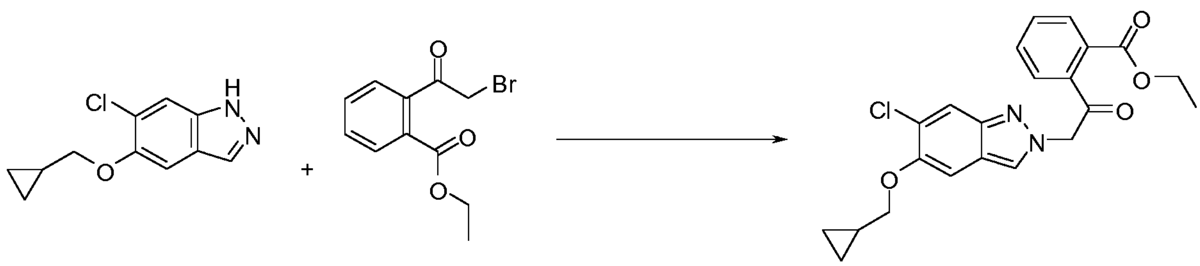

A mixture of ethyl 2-(2-bromoacetyl)benzoate (79 mg, 0.29 mmol, 1 eq), 6-chloro-5-(cyclopropylmethoxy)-lH-indazole (65 mg, 0.29 mg, 1 eq) and $NaHCO_3$ (37 mg, 0.44 mmol, 1.5 eq) in dry 1,4-dioxane (800 μL) is stirred at 105°C for 60 h. The reaction mixture is diluted (ethyl acetate), washed (saturated $NaHCO_3$ and brine), dried ($Na_2SO_4$) and concentrated. The residue is purified by flash column chromatography ($SiO_2$, DCM/ethyl acetate 100:0 to 99:1) to afford

the desired product.

*Intermediate 58: 4-[[6-chloro-5-(cyclopropylmethoxy)indazol-2-yl]methyl]-2H-phthalazin-1-one*

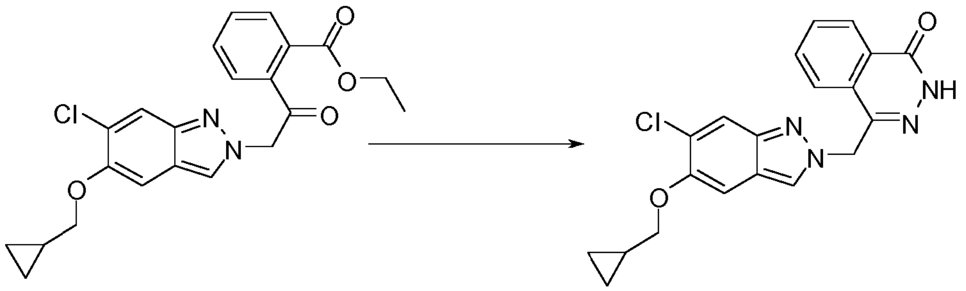

A solution of ethyl 2-[2-[6-chloro-5-(cyclopropylmethoxy)indazol-2-yl]acetyl]benzoate (31 mg, 0.08 mmol, 1 eq) and hydrazine monohydrate (7.5 μL, 0.151 mmol, 2 eq) in 2-isopropanol (180.0 μL) is stirred at 85°C for 3 h. The reaction mixture is concentrated and the residue is taken up in ethyl acetate. The organic mixture is washed (water and brine), dried ($Na_2SO_4$) and concentrated to afford the desired product.

*Alternative synthesis of Intermediate 58: 4-[[6-chloro-5-(cyclopropylmethoxy)indazol-2-yl]methyl]-2H-phthalazin-1-one*

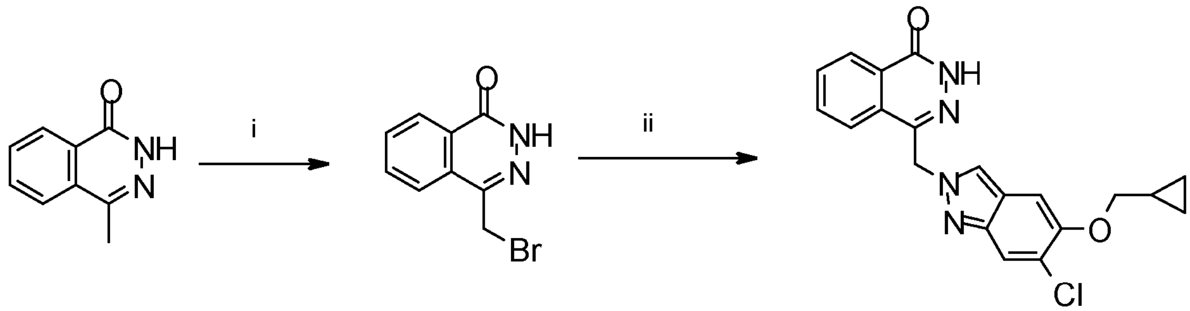


*Step i: 4-(bromomethyl)-2H-phthalazin-1-one*

N-bromosuccinimide (1.5 eq, 1167g, 6.56 moles) is added to a suspension of 4-methyl-2H-phthalazin-1-one (1.0 eq, 700g) in acetonitrile (7L). The reaction mixture is stirred at 20°C under nitrogen atmosphere for 5minutes. Benzoyl peroxide 75% in water (0.1 eq, 142g, 0.44 moles) is added to the suspension in one portion. The reaction mixture is then heated at reflux for 16h30. The reaction mixture is cooled down to 20°C and the suspension is filtered. The solid is triturated in acetonitrile (2100mL) and the suspension is filtered on a sintered glass funnel. The solid is washed with acetonitrile (1400mL), water (4200mL), acetonitrile (1400mL) and finally collected and dried to afford the desired product.

*Step ii: 4-[[6-chloro-5-(cyclopropylmethoxy)indazol-2-yl]methyl]-2H-phthalazin-1-one*

4-(bromomethyl)-2H-phthalazin-1-one (1.1 eq, 415g, 1.73 moles) and 6-chloro-5-(cyclopropylmethoxy)-IH-indazole (1.0 eq, 351g, 1.58 moles) are successively added in n-butyl acetate (3510mL). Lithium carbonate $Li_2CO_3$ (2 eq, 233g, 3.15 moles) is added in one portion and the heterogeneous reaction mixture is heated at reflux for 15h. The suspension is cooled down to 20-25°C, filtered on a sintered glass funnel and washed with acetonitrile (3L). The solid is dried under suction on the fritted funnel and then suspended in water/ACN (3.5L/0.7L). Acetic acid (337mL, 1.25 eq/residual base, 5.9 moles) is slowly added to the suspension. At the end of the addition, the suspension is stirred at 20°C for 1h. The suspension is filtered and the solid is washed with water (1.5L) and dried. The solid is re-slurried in water/acetonitrile (3L/0.3L) for 2 h and the suspension is filtered. The solid obtained is dried at 50°C in a vacuum oven to afford the desired product.

### *1.18. General method Q: Sonogashira reaction between 2-iodo-aniline and but-3-yn-1-ol*

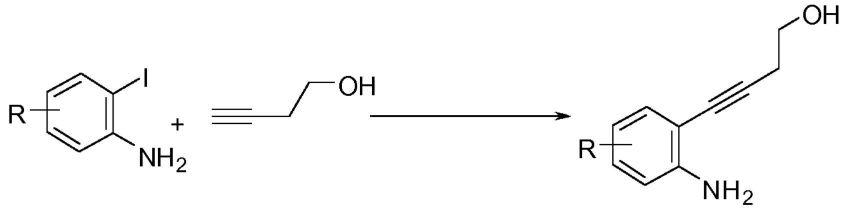

A degassed solution of the 2-iodo-aniline (1 eq), the alkyne (1.75 eq), CuI (0.02 eq), $Pd(PPh_3)_4$ (0.01 eq) and $Et_3N$ (1.5 eq) in water is stirred under inert atmosphere at 80°C typically for 1.5 h. The mixture undergoes an aqueous work up and the organic layer is concentrated to afford the desired product.

*Illustrative example of method Q: synthesis of intermediate 59, 4-(2-aminophenyl)but-3-yn-1-ol*

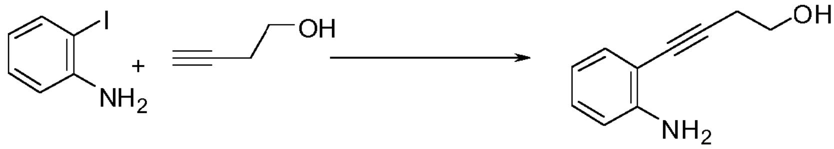

A degassed solution of 2-iodo-aniline (10.0 g, 44.745 mmol, 1 eq), but-3-yn-1-ol (6.11 mL, 78.303 mmol, 1.75 eq), CuI (170.4 mg, 0.895 mmol, 0.02 eq), $Pd(PPh_3)_4$ (527.6 mg, 0.447 mmol, 0.01 eq) and $Et_3N$ (9.35 mL, 67.117 mmol, 1.5 eq) in water (217 mL) is stirred under inert atmosphere at 80°C for 1.5 h. The mixture is extracted with diethyl ether and the organic layer is washed (water and brine). The organic layer is dried ($Na_2SO_2$) and concentrated to afford the desired product.

### *1.19. General method R: synthesis of cinnolones by cyclization*

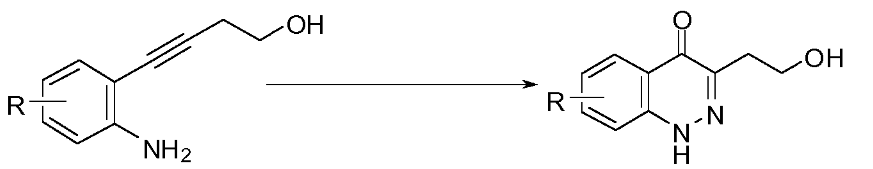

A solution of $H_2SO_4$ (4 eq) in water is added to a flask containing the aniline (1 eq). The mixture is cooled down to 0°C and a solution of $NaNO_2$ (1.5 eq) in water is added dropwise over 10 min. The resulted mixture is stirred at room temperature for 5 h. The product is typically isolated by precipitation.

*Illustrative example of method R: synthesis of intermediate 60, 3-(2-hydroxyethyl)-1H-cinnolin-4-one*

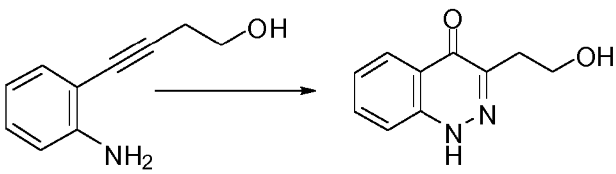

A solution of $H_2SO_4$ (10 mL, 179 mmol, 4 eq) in water (90 mL) is added to a flask containing 4-(2-aminophenyl)but-3-yn-1-ol (7.7 g, 45 mmol, 1 eq). The mixture is cooled down to 0°C and a solution of $NaNO_2$ (4.7 g, 67 mmol, 1.5 eq) in water (136 mL) is added dropwise over 10 min. The resulted mixture is stirred at room temperature for 5 h. The mixture is cooled to 0°C and the resulting precipitate is filtered off, washed (water) and dried under reduced pressure at 45°C to afford the desired product.

### 1.20. General method S: SEM protection of cinnolone derivatives

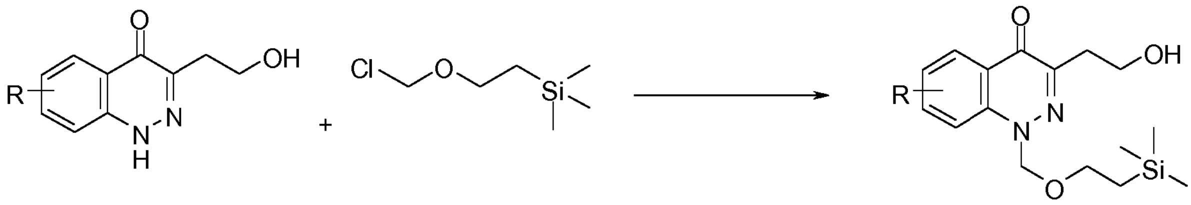

NaH (1.2 eq) is added to a solution of the cinnolone derivative (1 eq) in DMF at 0°C. The reaction mixture is stirred for 30 min at 0°C. 2-(Trimethylsilyl)ethoxymethyl chloride (1.1 eq) is added and the mixture is stirred at room temperature typically for 1.5 h. The reaction is quenched with water and the resulting mixture is extracted with an organic solvent. The organic layer is dried and concentrated to afford the desired product, which may be further purified by flash column chromatography.

*Illustrative example of method S: synthesis of intermediate 61, 3-(2-hydroxyethyl)-1-(2-trimethylsilylethoxymethyl)cinnolin-4-one*

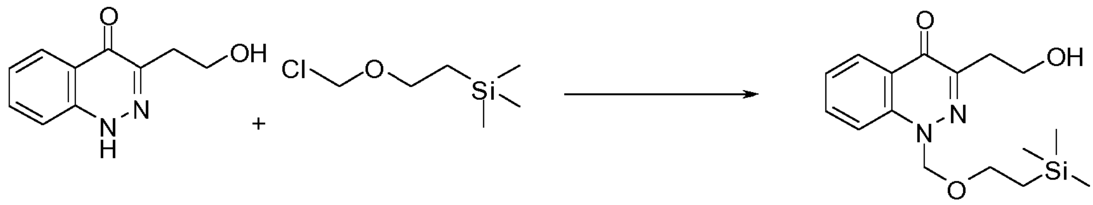

NaH (60% dispersion in mineral oil, 3.29 g, 82.3 mmol 1.2 eq) is added to a solution of 3-(2-hydroxyethyl)-1H-cinnolin-4-one (13.6 g, 71.6 mmol, 1 eq) in DMF (270 mL) at 0°C. The reaction mixture is stirred for 30 min at 0°C. 2-(Trimethylsilyl)ethoxymethyl chloride (14.5 mL, 78.7 mmol 1.1 eq) is added and the mixture is stirred at room temperature typically for 1.5 h. The reaction is quenched with water and extracted with ethyl acetate. The organic layer is washed (5% aq LiCl), dried ($Na_2SO_4$) and concentrated. The residue is purified by flash column chromatography ($SiO_2$, DCM/methanol 100:0 to 97.5:2.5) to afford the desired product.

### 1.21. General method T: oxidation of alcohols to aldehydes

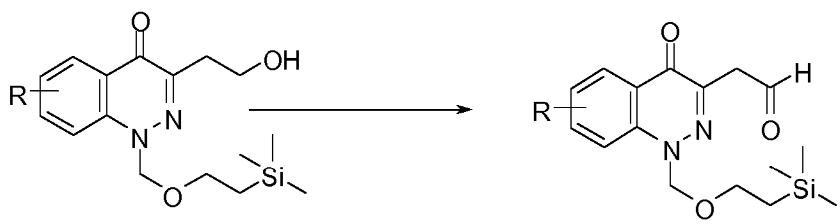

A solution of the alcohol (1 eq) and Dess-Martin periodinane (1.2 eq) in DCM is stirred at room temperature for typically 2 h. The mixture undergoes aqueous work up which typically includes a wash with a $NaHCO_3/Na_2S_2O_3 \times 5H_2O$ solution. The organic layer is dried and concentrated to afford the desired product.

*Illustrative example of method T: synthesis of intermediate 62, 2-[4-oxo-1-(2-trimethylsilylethoxymethyl)cinnolin-3-yl]acetaldehyde*

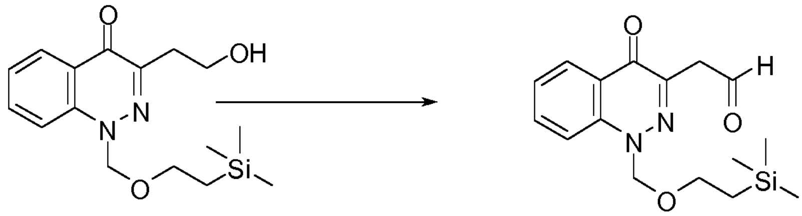

A solution of 3-(2-hydroxyethyl)-1-(2-trimethylsilylethoxymethyl)cinnolin-4-one (7.76 g, 24.2 mmol, 1 eq) and Dess-Martin periodinane (11.9 g, 26.6 mmol, 1.2 eq) in DCM (37 mL) is stirred at room temperature for 2 h. A solution of $NaHCO_3$ (3.3 eq, 6.7 g, 80 mmol) and $Na_2S_2O_3$×5H2O (6 eq, 36 g, 145 mmol) in water (37 mL) is added and the resulting mixture is stirred for 5 min. The two phases are separated and the aqueous layer is extracted with DCM (3 × 100 mL). The organic layers are combined, washed (water, 2 × 100 mL and brine, 100 mL), dried over $Na_2SO_4$ and concentrated under reduced pressure to afford the desired product.

### *1.22. General method U: oxidation of aldehydes to carboxylic acids*

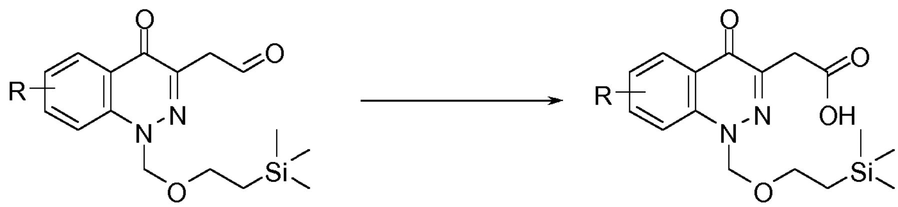

$NaClO_2$ (3 eq) is added to a solution of the aldehyde (1.0 eq), 2 M THF 2-methylbut-2-ene (4.0 eq) and $NaH_2PO_4$x2$H_2O$ (1.5 eq) in 5:1 t-BuOH/water. The reaction mixture is stirred at room temperature for 1 h. The reaction is typically quenched with $NaHSO_3$ (10 %) water solution and adjusting the pH to 2. The mixture is extracted with an organic solvent and the resulting mixture undergoes aqueous work up. The residue is typically purified by flash column chromatography to afford the desired product.

*Illustrative example of method U: synthesis of intermediate 63, 2-[4-oxo-1-(2-trimethylsilylethoxymethyl)cinnolin-3-yl]acetic acid*

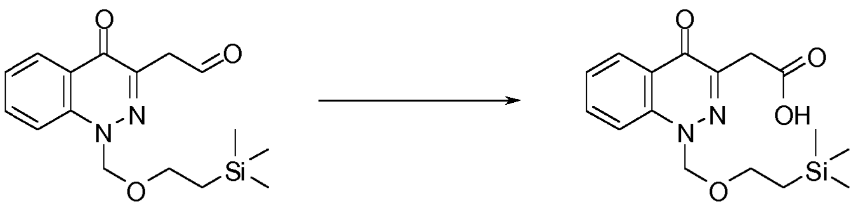

$NaClO_2$ (11.5 g, 102 mmol, 3 eq) is added to a solution of 2-[4-oxo-1-(2-trimethylsilylethoxymethyl)cinnolin-3-yl]acetaldehyde (11.5 g, 36 mmol, 1.0 eq), 2 M THF 2-methylbut-2-ene (68 mL, 136 mmol, 4.0 eq) and $NaH_2PO_4$x2$H_2O$ (8.04 g, 51 mmol, 1.5 eq) in 5:1 t-BuOH/water (340 mL). The reaction mixture is stirred at room temperature for 1 h. The reaction is quenched with aqueous $NaHSO_3$ (10 %) and the pH is adjusted to 2 with citric acid. The mixture is extracted with ethyl acetate. The organic layer is dried ($Na_2SO_4$) and concentrated. The residue is purified by flash column chromatography ($SiO_2$, DCM/methanol 100:0 to 93:7) to afford the desired product.

*Intermediate 63: 4-[[6-chloro-5-(cyclopropylmethoxy)indazol-2-yl]methyl]-2H-phthalazin-1-one (from oxidation of intermediate 60)*

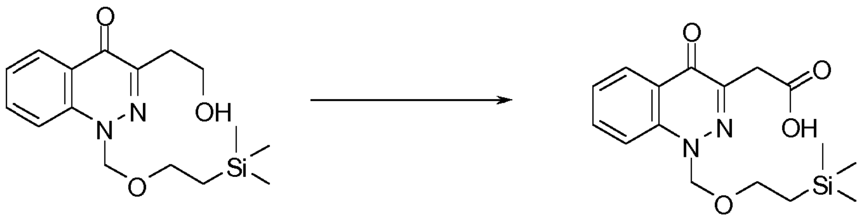

Sodium periodate, NaIO4 (2.68 g, 4.1 eq)) is added to a solution of 3-(2-hydroxyethyl)-1-(2-trimethylsilylethoxymethyl)cinnolin-4-one (1 g, 1.0 eq, 3.03 mmol) in 21 mL of acetonitrile/water/ethyl acetate (6/9/6). The mixture is stirred for 5 min. RuCl3 (6.3 mg, 0.01 eq) is added and the temperature of the reaction is raised to about 45°C. The mixture is let to cool to room temperature and stirred for 2 h. The mixture is diluted with 20 mL of ethyl acetate and filtered over a celite pad. The flask is rinsed with 2x20 mL of ethyl acetate and the washings are used to rinse the pad. The gathered filtrates are washed with 2x20 mL of $Na_2S_2O_3$ 5 % water solution. After washing with 30 mL of brine the solvent is evaporated to afford the desired product.

### *1.23. General method V: esterification of carboxylic acids*

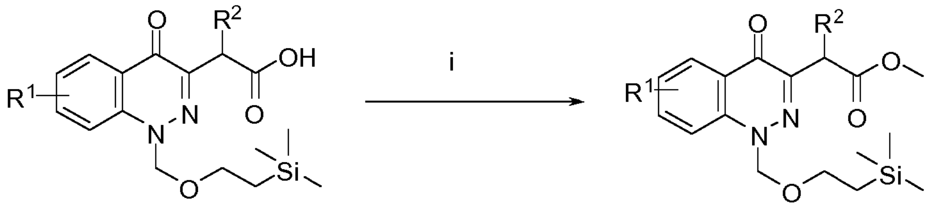


$SOCl_2$ (1.2 eq) is added slowly to a solution of the carboxylic acid (1 eq) in dry methanol. The reaction mixture is stirred at room temperature typically for 90 min. The reaction mixture is concentrated and the residue undergoes aqueous work up. After concentration the residue is typically purified by flash column chromatography to afford the desired product.

*Illustrative example of method V: synthesis of intermediate 64, methyl 2-[4-oxo-1-(2-trimethylsilylethoxymethyl)cinnolin-3-yl]acetate*

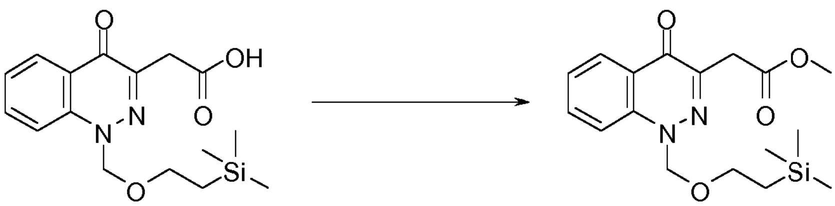

$SOCl_2$ (1.22 mL, 16.8 mmol, 1.2 eq) is added slowly to a solution of 2-[4-oxo-1-(2-trimethylsilylethoxymethyl)cinnolin-3-yl]acetic acid (4.7 g, 14 mmol, 1 eq) in dry methanol (39 mL). The reaction mixture is stirred at room temperature for 90 min. The reaction mixture is concentrated and diluted (ethyl acetate). The organic layer is washed (saturated $NaHCO_3$ and brine), dried ($Na_2SO_4$) and concentrated. The residue is purified by flash column chromatography ($SiO_2$, DCM/methanol 100:0 to 98.5:1.5) to afford the desired product.

### *1.24. General method W: alkylation of cinnolones derivatives*

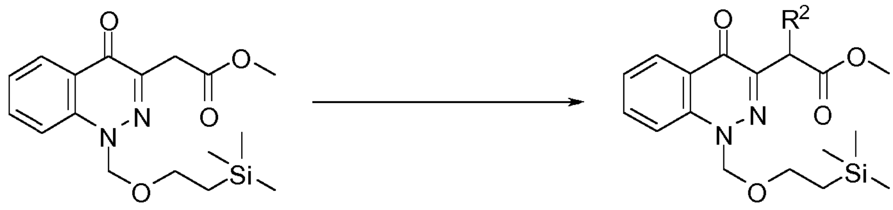

NaH (1.05 eq) is added to a mixture of ester (1 eq) in DMF at 0°C. The mixture is stirred for 10 min then the alkylating agent (typically an alkyl iodide, 1.05 eq) is added. The mixture is stirred at room temperature typically for 1 h. The mixture is quenched with water and extracted with an organic solvent. The organic layer undergoes an aqueous work up, dried and concentrated to afford the desired product.

*Illustrative example of method W: synthesis of intermediate 65, methyl 2-[4-oxo-1-(2-trimethylsilylethoxymethyl)cinnolin-3-yl]propanoate*

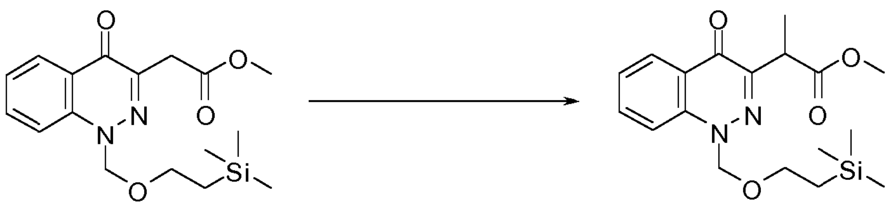

NaH (60% mineral oil, 181 mg, 4.5 mmol, 1.05 eq) is added to a mixture of methyl 2-[4-oxo-1-(2-trimethylsilylethoxymethyl)cinnolin-3-yl]acetate (1.50 g, 4.3 mmol, 1 eq) in DMF (8.6 mL) at 0°C. The mixture is stirred for 10 min and iodomethane (0.28 mL, 4.5 mmol, 1.05 eq) is added. The mixture is stirred at room temperature for 1 h. The mixture is quenched with water and extracted with ethyl acetate. The organic layer is washed (saturated $NaHCO_3$ and 5% aq LiCl), dried ($Na_2SO_4$) and concentrated to afford the desired product.

*Intermediate 66: methyl 2-methyl-2-[4-oxo-1-(2-trimethylsilylethoxymethyl)cinnolin-3-yl]propanoate*

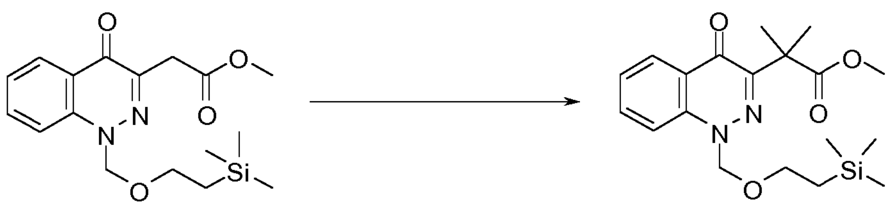

NaH (60% mineral oil, 52 mg, 1.3 mmol, 3 eq) is added to a mixture of methyl 2-[4-oxo-1-(2-trimethylsilylethoxymethyl)cinnolin-3-yl]acetate (150 mg, 0.43 mmol, 1 eq) and iodomethane (0.082 mL, 1.3 mmol, 3 eq) in DMF (0.86 mL) at 0°C. The mixture is stirred for 30 min and 0°C and for 1 h at room temperature. The mixture is quenched with water and extracted with ethyl acetate. The organic layer is washed (saturated $NaHCO_3$), dried ($Na_2SO_4$) and concentrated. The residue is purified by flash column chromatography ($SiO_2$, DCM/methanol 100:0 to 98.5:1.5) to afford the desired product.

*Intermediate 67: methyl 2-[4-oxo-1-(2-trimethylsilylethoxymethyl)cinnolin-3-yl]prop-2-enoate*

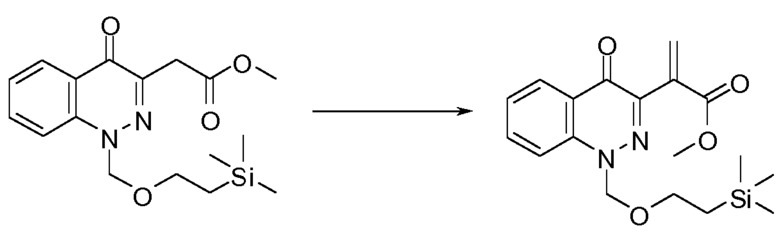

NaH (60% mineral oil, 46 mg, 1.15 mmol, 1.2 eq) is added to a cooled mixture of methyl 2-[4-oxo-1-(2-trimethylsilylethoxymethyl)cinnolin-3-yl]acetate (367 mg, 0.96 mmol, 1 eq) and paraformaldehyde (910 mg, 29 mmol, 30 eq) in 1,4-dioxane (5 mL). The reaction mixture is stirred at room temperature for 4 h and at 45°C for 16 h. Methanol is added to the reaction and the mixture is stirred for 15 min. The solvent is removed under reduced pressure and the residue is taken up in DCM. The organic layer is washed (0.1 M HCl), dried (filtered through phase separator) and concentrated. The residue is purified by flash column chromatography ($SiO_2$, DCM/ethyl acetate 100:0 to 85:15) to afford the desired product.

*Intermediate 68: methyl 1-[4-oxo-1-(2-trimethylsilylethoxymethyl)cinnolin-3-yl]cyclopropanecarboxylate*

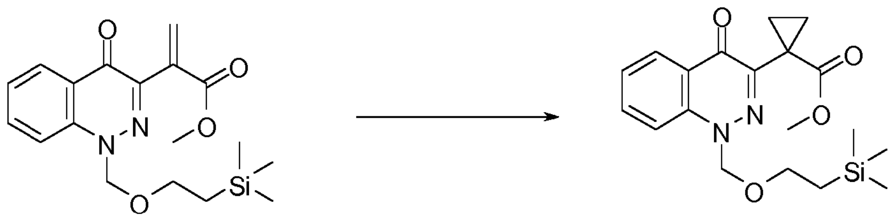

NaH (60% mineral oil, 4.8 mg, 0.12 mmol, 1.5 eq) is added to a solution of trimethylsulfoxonium iodide (29 mg, 0.13 mmol, 1.6 eq) in dry DMSO (0.7 mL). The mixture is stirred for 30 min. A solution of methyl 2-[4-oxo-1-(2-trimethylsilylethoxymethyl)cinnolin-3-yl]prop-2-enoate (29 mg, 0.08 mmol, 1 eq) in dry DMSO (0.7 mL) is added dropwise during 3 min. The resulting reaction mixture is stirred for 1 h. The reaction is quenched by addition of ice. The resulting mixture is partitioned between ethyl acetate and water. Brine is added to increase phase separation. The two layers are separated and the organic layer is dried ($Na_2SO_4$) and concentrated. The residue is purified by flash column chromatography ($SiO_2$, DCM/ethyl acetate 100:0 to 85:15) to afford the desired product.

*Intermediate 69: 2-methoxy-2-[4-oxo-1-(2-trimethylsilylethoxymethyl)cinnolin-3-yl]acetic acid*

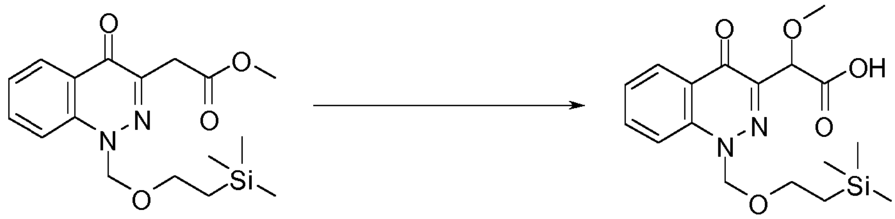

(Diacetoxyiodo)benzene (197.1 mg, 0.593 mmol, 1 eq) and NaOMe (30% in methanol, 339.2 µL, 1.780 mmol, 3 eq) are added to a solution of methyl 2-[4-oxo-1-(2-trimethylsilylethoxymethyl)cinnolin-3-yl]acetate (206.8 mg, 0.593 mmol, 1 eq) in dry methanol (3.93 mL). The mixture is stirred at room temperature for 18 h. The reaction mixture is diluted with water and the pH is adjusted to 5.3 using 2 N HCl. The aqueous layer is extracted with DCM. The organic layer is dried ($Na_2SO_4$) and concentrated to afford the desired product.

### 1.25. General method X: SEM deprotection of cinnolones derivatives

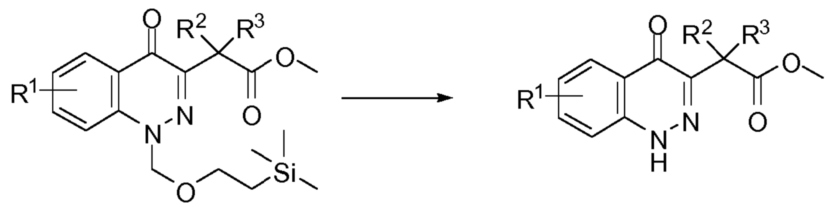

A solution of the SEM-protected cinnolone derivative (1 eq) in 4:1 DCM / TFA is stirred at room temperature typically for 16 h. The reaction mixture is concentrated (typically using toluene to form an azeotrope). The residue undergoes aqueous work up and after drying and concentration of the organic layer the residue is typically purified by flash column chromatography.

*Illustrative example of method X: synthesis of intermediate 70, methyl 2-(4-oxo-1H-cinnolin-3-yl)propanoate*

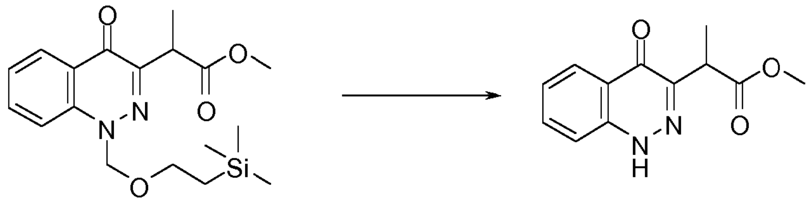

A solution of methyl 2-[4-oxo-1-(2-trimethylsilylethoxymethyl)cinnolin-3-yl]propanoate (1.5 g, 4.1 mmol, 1 eq) in 4:1 DCM / TFA (75 mL) is stirred at room temperature for 16 h. The reaction mixture is concentrated using toluene to form an azeotrope. The residue is taken up in ethyl acetate. The organic layer is washed (saturated $NaHCO_3$ and brine), dried ($Na_2SO_4$) and concentrated. The residue is purified by flash column chromatography ($SiO_2$, dicholormethane/methanol 99.75:0.25 to 96.5:3.5) to afford the desired product.

*Intermediate 71: methyl 2-(4-oxo-5,6,7,8-tetrahydro-1H-cinnolin-3-yl)acetate*

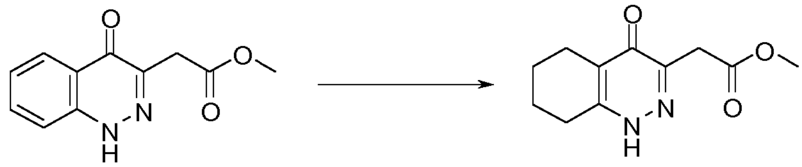

Platinum (IV)oxide, $PtO_2$ (37 mg, 0.16 mmol, 0.1 eq) is added to a solution of methyl 2-(4-oxo-1H-cinnolin-3-yl)acetate (370 mg, 1.61, 1 eq) in TFA (4 mL). The resulting mixture is stirred at room temperature under an atmosphere of 3.5 bar of $H_2$ for 16 h. The reaction mixture is diluted with water and the pH is adjusted to about 8 with $Na_2CO_3$. The aqueous layer is extracted with DCM/isopropanol 90:10. The organic layer is washed (brine), dried ($Na_2SO_4$) and concentrated to afford the desired product.

### *1.26. General method Y: bis-PMB protection of 2-aminopyridines*

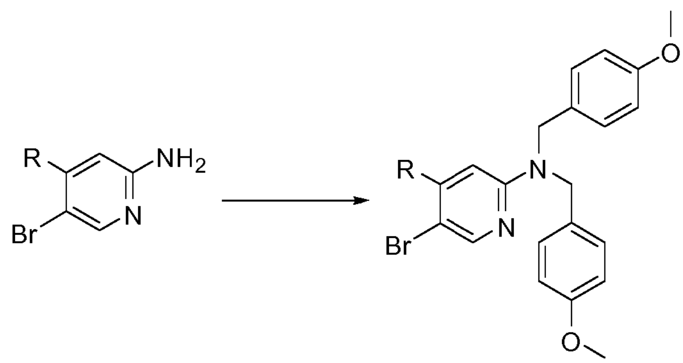

NaH (2.6 eq) is added to a solution of the 2-aminopyridine (1eq) in dry DMF at 0°C. The mixture is stirred typically for 15 min at 0°C. 1-(chloromethyl)-4-methoxy-benzene (2.1 eq) is added and the mixture is stirred at 0°C for typically 90 min. The reaction mixture is partitioned between water and an organic solvent. The two layers are separated and the organic layer is further washed, dried ($Na_2SO_4$) and concentrated. The residue typically undergoes another work up procedure or is purified by flash column chromatography.

*Illustrative example of method Y: synthesis of intermediate 72, 5-bromo-4-chloro-N,N-bis[(4-methoxyphenyl)methyl]pyridin-2-amine*

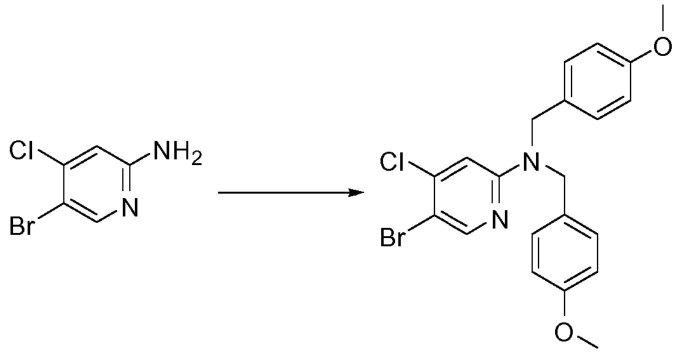

NaH (60 % dispersion on mineral oil, 9.7 g, 243 mmol, 2.6 eq) is added to a solution of 5-bromo-4-chloro-pyridin-2-amine (20 g, 93.5 mmol, 1 eq) in dry DMF (160 mL) at 0°C. The mixture is stirred for 15 min at 0°C. 1-(chloromethyl)-4-methoxy-benzene (31.4 g, 196 mmol, 2.1 eq) is added and the mixture is stirred at 0°C for 90 min. The mixture is partitioned between diethyl ether and water. The aqueous later is further extracted and the organic layers are combined and further washed (water and brine), dried ($Na_2SO_4$) and concentrated. The residue is taken up in acetonitrile and the mixture is washed with cyclohexane. The mixture in acetonitrile is dried ($Na_2SO_4$), concentrated and suspended in diethyl ether. The solvent is concentrated and the residue is dried under reduced pressure to afford the desired product.

## *1.27. General method Z: formation of phenols from aryl bromides*

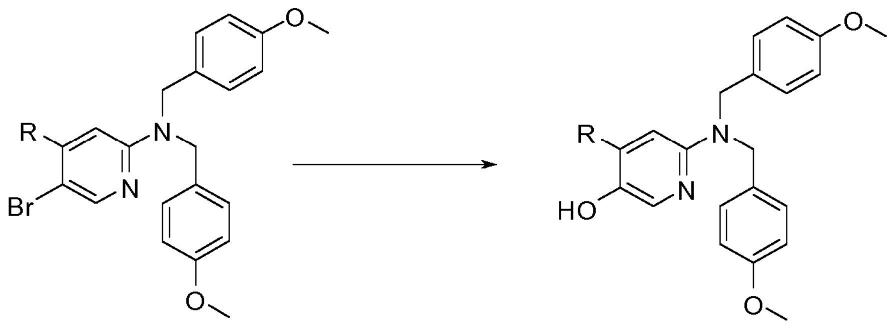

*N*-butyl lithium (2.5 M in hexanes, 1.25 eq) is added dropwise at -78°C to a solution of the aryl bromide (1 eq) in dry THF. The mixture is stirred typically for 90 min. 2-isopropoxy-4,4,5,5-tetramethyl-1,3,2-dioxaborolane (2.0 eq) is added and the mixture is stirred at -78°C typically for 45 min. The mixture is left to reach -10°C typically in 30 min. Hydrogen peroxide, 30 % water solution (4.0 eq) is added. The mixture is left to reach room temperature and stirred for 1 h. The mixture is diluted with an organic solvent and undergoes aqueous work up. The organic layer is dried and concentrated. The residue typically is dissolved in a basic aqueous solution. The aqueous mixture is washed with an organic solvent, neutralized and extracted. The organic layer is dried and concentrated to afford the desired product.

*Illustrative example of method Z: synthesis of intermediate 73, 6-[bis[(4-methoxyphenyl)methyl]amino]-4-chloro-pyridin-3-ol*

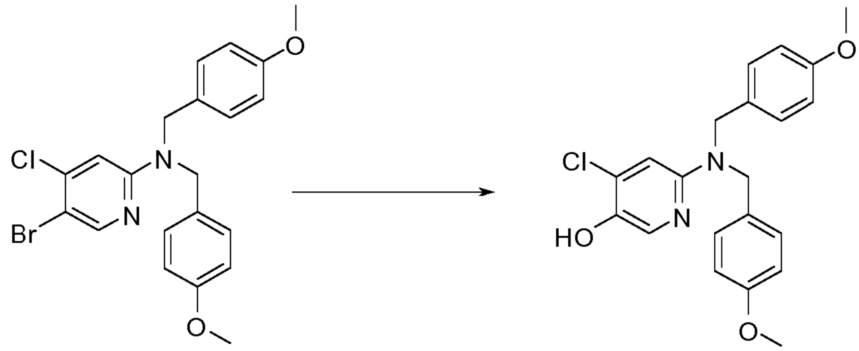

*N*-butyl lithium (2.5 M in hexanes, 22 mL, 55 mmol, 1.25 eq) is added dropwise at -78°C to a solution of 5-bromo-4-chloro-N,N-bis[(4-methoxyphenyl)methyl]pyridin-2-amine (21 g, 44.6 mmol, 1 eq) in dry THF (460 mL). The mixture is stirred for 90 min. 2-isopropoxy-4,4,5,5-tetramethyl-1,3,2-dioxaborolane (18.6 mL, 89 mmol, 2.0 eq) is added and the mixture is stirred at -78°C for 45 min. The mixture is left to reach -10°C in 30 min. Hydrogen peroxide, 30 % water solution (18 mL, 178 mmol, 4.0 eq) is added. The mixture is left to reach room temperature and stirred for 1 h. The mixture is partitioned between ethyl acetate and a NaCl aqueous solution. The layers are separated and the organic layer is dried ($Na_2SO_4$) and concentrated. The mixture is concentrated and the residue is taken up in aqueous 2 M NaOH (700 mL). The aqueous mixture is washed (diethyl ether), neutralized (conc. HCl) and extracted (ethyl acetate). The organic layer is dried ($Na_2SO_4$) and concentrated to afford the desired product, which is further dried under reduced pressure at 40°C.

*Intermediate 74: 2-[bis[(4-methoxyphenyl)methyl]amino]-5-hydroxy-pyridine-4-carbonitrile*

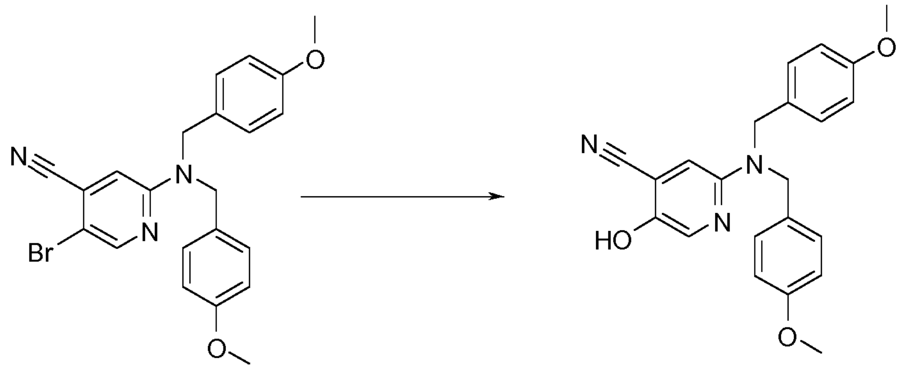

A degassed solution of 2-[bis[(4-methoxyphenyl)methyl]amino]-5-bromo-pyridine-4-carbonitrile (960 mg, 1.0 eq, 2.15 mmol), KOAc (639 mg, 3.0 eq), bis(pinacolatodiboron) (780 mg, 1.40 eq) and $PdCl_2$(dppf).$CH_2Cl_2$ (90 mg, 0.05 eq) in 18 mL of dry 1,4-dioxane is is stirred in a sealed vial at 115°C for 2 h under argon. The reaction is cooled to 0°C. Maintaining the temperature, hydrogen peroxide, 30 % water solution (1.32 mL, 6.0 eq) is added to the mixture. The reaction mixture is left to warm up to room temperature and stirred for 2 h. The mixture is diluted with 100 mL of DCM and the mixture is poured into 150 mL of water. The two phases are separated and the organic layer is washed with 150 mL of brine. After drying over $Na_2SO_4$ and filtration the solvent is evaporated to afford crude material. The residue is purified by flash column chromatography $SiO_2$, cyclohexane/ethyl acetate 95:5 to 65:35) to afford the desired product.

### *1.28. General method AA: synthesis of aminopyridines*

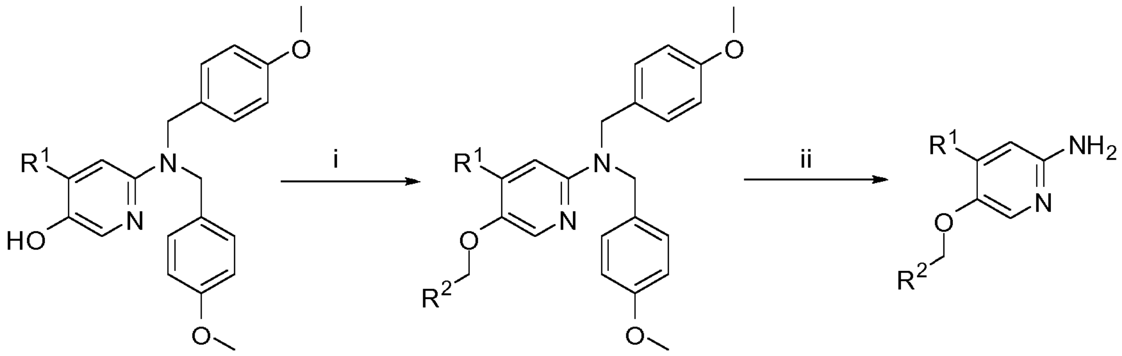


*1.28.1. Step i:*

A mixture of the phenol (1 eq), $Cs_2CO_3$ (1.5 eq) and the alkylating agent (typically an alkyl halide, 1.25 eq) in dry DMF is stirred typically at 80°C for 1 h. The mixture undergoes aqueous work up. The organic layer is dried and concentrated.

*1.28.2. Step ii:*

The residue from step i is stirred in in DCM/TFA 4:1 typically for 18 h. The mixture is partitioned between water and an organic solvent. The resulting mixture is carefully basified and the two layers are separated. The organic layer is dried and concentrated. The residue is typically purified by flash column chromatography to afford the desired product.

*Illustrative example of method AA: synthesis of intermediate 75, 4-chloro-5-(cyclopropylmethoxy)pyridin-2-amine*

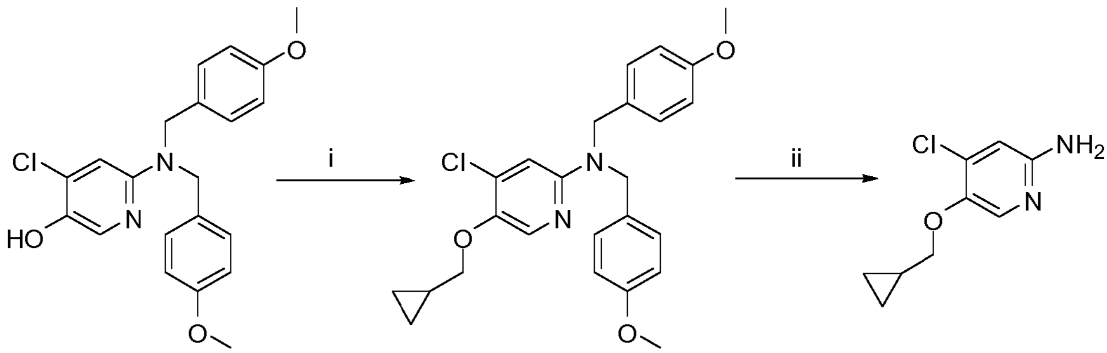


*1.28.3. Step i: 4-chloro-5-(cyclopropylmethoxy)-N,N-bis[(4-methoxyphenyl)methyl]pyridin-2-amine*

A mixture of 6-[bis[(4-methoxyphenyl)methyl]amino]-4-chloro-pyridin-3-ol (27.01 g, 1.0 eq, 64.59 mmol), $Cs_2CO_3$ (31.9 g, 97 mmol, 1.5 eq) and (bromomethyl)cyclopropane (8.1 mL, 81 mmol, 1.25 eq) in dry DMF (200 mL) is stirred at 80°C for 1 h. The mixture is partitioned between diethyl ether and aqueous NaCl. The two layers are separated. The aqueous layer is extracted with diethyl ether. The organic layers are combined, dried ($Na_2SO_4$) and concentrated to afford 4-chloro-5-(cyclopropylmethoxy)-N,N-bis[(4-methoxyphenyl)methyl]pyridin-2-amine.

*1.28.4. Step ii: 4-chloro-5-(cyclopropylmethoxy)pyridin-2-amine*

The residue from step i is stirred in in DCM/TFA 4:1 (500 mL) for 18 h. The mixture is poured into 2.5 l of water/ice mixture. 500 mL of DCM are added and the mixture is cooled. The mixture is basified until approximately pH 10 by adding $Na_2CO_3$. The layers are separated and the aqueous layer is extracted with DCM. The organic layers are combined, dried ($Na_2SO_4$) and concentrated. The residue is purified by flash column chromatography ($SiO_2$, DCM/ethyl acetate 95:5 to 80:20) to afford the desired product.

*Intermediate 76: 2-amino-5-hydroxy-pyridine-4-carbonitrile*

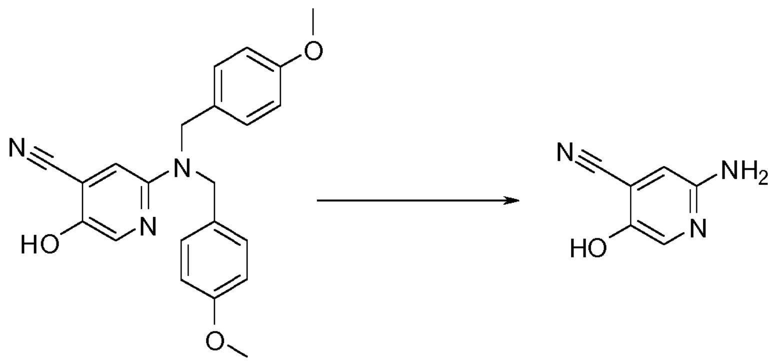

A mixture of 2-[bis[(4-methoxyphenyl)methyl]amino]-5-hydroxy-pyridine-4-carbonitrile (3.1 g, 6.7 mmol, 1 eq) in DCM / TFA 6:1 (70 mL) is stirred at room temperature for 16 h. The mixture is concentrated and taken up in an aqueous solution made from 150 mL saturated $NaHCO_3$ and 50 mL of water. The aqueous mixture is washed (DCM) and the pH is adjusted to about 4 (conc. HCl). The aqueous layer is extracted (ethyl acetate/THF 90:10). The organic layer is dried ($Na_2SO_4$) and concentrated to afford the desired product.

*Intermediate 77: 2-amino-5-(cyclopropylmethoxy)pyridine-4-carbonitrile*

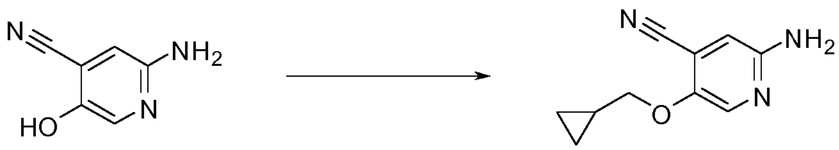

A mixture of 2-amino-5-hydroxy-pyridine-4-carbonitrile (790 mg, 5.6 mmol, 1 eq) and NaH (60% mineral oil, 266 mg, 6.7 mmol, 1.2 eq) in dry DMF (15 mL) is stirred at 0°C for 15 min. (bromomethyl)cyclopropane (0.61 mL, 6.1 mmol, 1.1 eq) is added and the mixture is stirred at room temperature for 16 h. The mixture is diluted (ethyl acetate/THF 90:10), washed (water and brine), dried ($Na_2SO_4$) and concentrated. The residue is purified by flash column chromatography ($SiO_2$, DCM/ethyl acetate 100:0 to 40:60) to afford the desired product.

### *1.29. General method AB: synthesis of imidazopyridines methyl acetate*

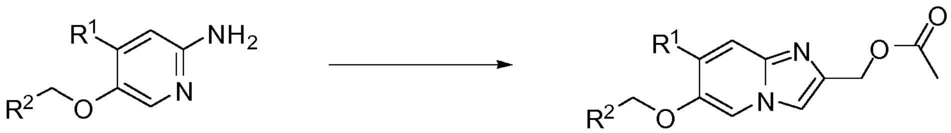

A suspension of the aminopyridine (1 eq) and 1-acetoxy-3-chloroacetone (5 eq) is stirred typically in sealed vial at 55°C for 24 h. The mixture is typically purified by flash column chromatography to afford the desired product.

*Illustrative example of method AB: synthesis of intermediate 78, [7-cyano-6-(cyclopropylmethoxy)imidazo[1,2-a]pyridin-2-yl]methyl acetate*

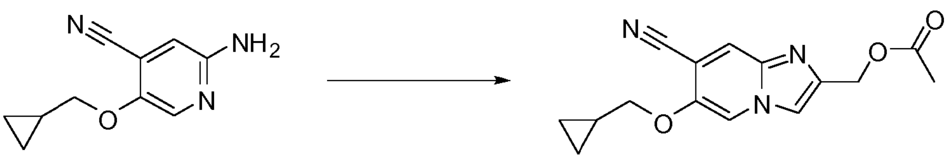

A suspension of 2-amino-5-(cyclopropylmethoxy)pyridine-4-carbonitrile (625 mg, 3.1 mmol, 1 eq) and 1-acetoxy-3-chloroacetone (2 mL, 16 mmol, 5 eq) is stirred in sealed vial at 55°C for 24 h. The mixture is typically purified by flash column chromatography ($SiO_2$, DCM/methanol 100:0 to 95:5) to afford the desired product.

*Intermediate 79, 6-(2,2-difluoroethoxy)-2-(hydroxymethyl)-3H-benzimidazole-5-carbonitrile*

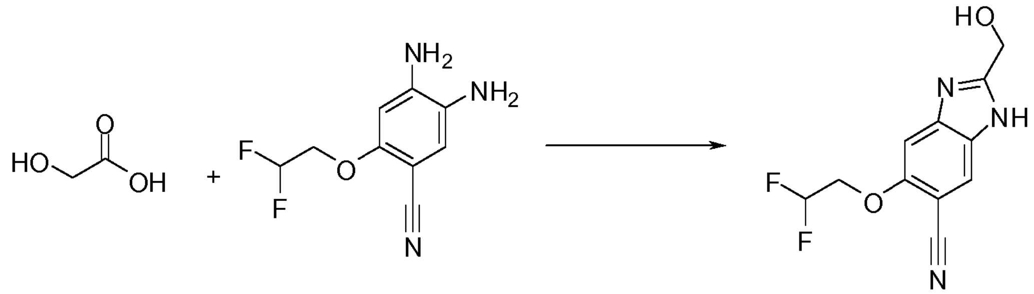

A solution of 4,5-diamino-2-(2,2-difluoroethoxy)benzonitrile (10 g, 47 mmol, 1 eq) and glycolic acid (5.77 g, 75 mmol, 1.4 eq) in 4 N HCl (47 mL) is refluxed for 14 h. The reaction is quenched with saturated $Na_2CO_3$. The aqueous mixture is extracted with 2:1 DCM 2-isopropanol. The organic layer is dried ($Na_2SO_4$) and concentrated. The residue is triturated with DCM to afford the desired product.

### 1.30. General method AC: formation of alcohols by removal of acetate group

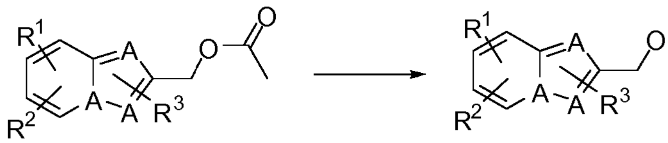

A mixture of the acetate ester (1 eq) and $K_2CO_3$ or $Cs_2CO_3$ (2.2 eq) in dry methanol is stirred at room temperature typically for 1 h. The reaction mixture is diluted with an organic solvent and the resulting mixture undergoes an aqueous work up. The organic layer is dried and concentrated to afford the desired product.

*Illustrative example of method AC: synthesis of intermediate 80, 6-(cyclopropylmethoxy)-2-(hydroxymethyl)imidazo[1,2-a]pyridine-7-carbonitrile*

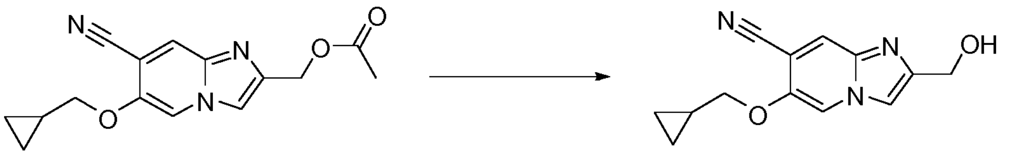

A mixture of [7-cyano-6-(cyclopropylmethoxy)imidazo[1,2-a]pyridin-2-yl]methyl acetate (428 mg, 1.0 eq, 1.35 mmol) and $K_2CO_3$ (415 mg, 3 mmol, 2.2 eq) in dry methanol (25 mL) is stirred at room temperature for 1 h. The mixture is diluted with ethyl acetate. The resulting mixture is washed (water & brine), dried ($Na_2SO_4$) and concentrated. The residue is suspended in diethyl ether, which is removed under reduced pressure to afford the desired product.

### 1.31. General method AD: oxidation of primary alcohols to aldehydes

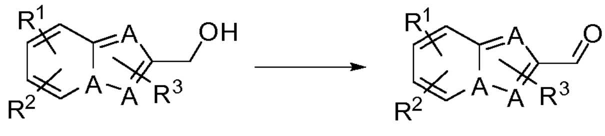

1,1,1-Tris(acetyloxy)-1,1-dihydro-1,2-benziodoxol-3-(1H)-one, Dess-Martin periodinane (1.1 eq) is added to a solution of the alcohol (1 eq) in dry DCM at 0°C. The reaction is stirred at room temperature typically for 1 h. The mixture is carefully concentrated. The residue is dissolved in an organic solvent and the mixture undergoes aqueous work up. The organic layer is dried and concentrated. The residue is typically purified by flash column chromatography to afford the desired product.

*Illustrative example of method AD: synthesis of intermediate 81, 7-chloro-6-(cyclopropylmethoxy)imidazo[1,2-a]pyridine-2-carbaldehyde*

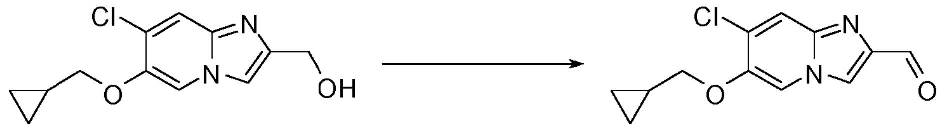

1,1,1-Tris(acetyloxy)-1,1-dihydro-1,2-benziodoxol-3-(1H)-one, Dess-Martin periodinane (1 g, 2.3 mmol, 1.1 eq) is added to a solution of [7-chloro-6-(cyclopropylmethoxy)imidazo[1,2-a]pyridin-2-yl]methanol (560 mg, 2.1 mmol,1 eq) in dry DCM (100 mL) at 0°C. The reaction is stirred at room temperature typically for 1 h. The mixture is concentrated at a temperature of 27°C. The residue is taken up in ethyl acetate. The organic mixture is washed (saturated $NaHCO_3$ and brine), dried ($Na_2SO_4$) and concentrated. The residue is purified by flash column chromatography ($SiO_2$, DCM/methanol 100:0 to 95.5:4.5) to afford the desired product.

### *1.32. General method AE: synthesis of chloromethyl imidazopyridines*

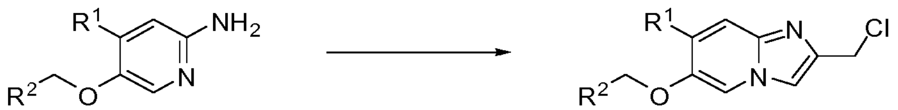

A mixture of the 2-aminopyridine (1 eq) and 1,3-dichloropropan-2-one (1.05 eq) in dry 1,2-dimethoxyethane is stirred typically at room temperature for 4 h. The mixture is concentrated and the residue is taken up in absolute ethanol. The reaction is stirred typically at 90°C for 16 h. The reaction mixture is quenched with saturated $NaHCO_3$. The aqueous mixture is extracted with an organic solvent. The organic layer is dried and concentrated. The residue is typically purified by flash column chromatography to afford the desired product.

*Illustrative example of method AE: synthesis of intermediate 82, 7-chloro-2-(chloromethyl)-6-(cyclopropylmethoxy)imidazo[1,2-a]pyridine*

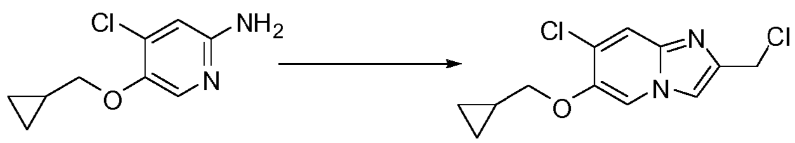

A mixture of 4-chloro-5-(cyclopropylmethoxy)pyridin-2-amine (9 g, 1.0 eq, 45 mmol) and 1,3-dichloropropan-2-one (6.35 g, 47.5 mmol, 1.05 eq) in 1,2-dimethoxyethane (25 mL) is stirred at room temperature for 4 h. The mixture is concentrated and the residue is taken up in absolute ethanol (75 mL). The reaction mixture is stirred at 90°C for 16 h. The reaction mixture is quenched with saturated $NaHCO_3$ (480 mL). The aqueous mixture is extracted (ethyl acetate). The organic layer is dried ($Na_2SO_4$) and concentrated. The residue is purified by flash column chromatography ($SiO_2$, DCM/ethyl acetate 97:3 to 40:60) to afford the desired product.

### *1.33. General method AF: synthesis of chloromethyl derivatives from primary alcohols*

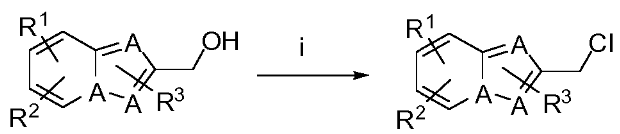


Methanesulfonyl chloride (1.2 eq) is added to a solution of the alcohol derivative (1 eq), 4-dimethylaminopyridine (0.1 eq) and triethylamine (1.2 eq) in dry DCM at 0°C. The resulting mixture is stirred at room temperature typically for 16 h. The mixture undergoes an aqueous work up. After removal of the solvent, the residue is typically purified by flash column chromatography to yield the desired product.

*Illustrative example of method AF: synthesis of intermediates 83, 2-(chloromethyl)-6-(cyclopropylmethoxy)-1-(2-trimethylsilylethoxymethyl)benzimidazole-5-carbonitrile & 2-(chloromethyl)-6-(cyclopropylmethoxy)-3-(2-trimethylsilylethoxymethyl)benzimidazole-5-carbonitrile*

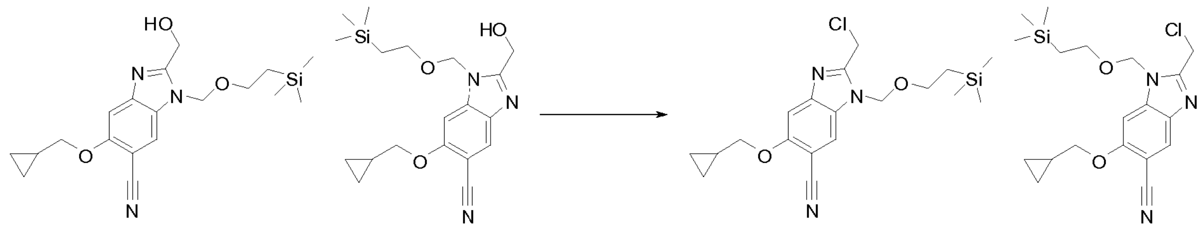

Methanesulfonyl chloride (0.272 mL, 3.5 mmol, 1.2 eq) is added to a solution of 6-(cyclopropylmethoxy)-2-(hydroxymethyl)-1-(2-trimethylsilylethoxymethyl)benzimidazole-5-carbonitrile & 6-(cyclopropylmethoxy)-2-(hydroxymethyl)-3-(2-trimethylsilylethoxymethyl)benzimidazole-5-carbonitrile (2.9 mmol in total, 1 eq), 4-dimethylaminopyridine (36 mg, 0.29 mmol 0.1 eq) and triethylamine (0.49 mmol, 3.5 mmol, 1.2 eq) in DCM (31 mL) at 0°C. The resulting mixture is stirred at room temperature for 16 h. The reaction is diluted with dicholoromethane. The resulting mixture is washed (1 N HCl, saturated $NaHCO_3$ and brine), dried ($Na_2SO_4$) and concentrated. The residue is purified by flash column chromatography ($SiO_2$, DCM/ethyl acetate 100:0 to 93:7) to yield the desired product.

### *1.34. General method AG: formation of bis-aniline derivatives by reduction of nitro derivatives*

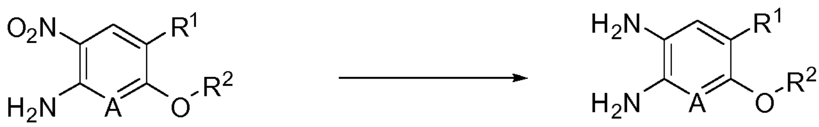

A = CH, CF, CCl, N

Zinc dust (12 eq) is added to a mixture of the nitro derivative (1 eq) and $NH_4Cl$ (12 eq) in methanol at 0°C followed by formic acid (2 eq). The mixture is typically stirred at room temperature for 24 h. The solids are filtered off and the filtrate is concentrated. The residue is taken up in an organic solvent. The mixture undergoes an aqueous work up. The organic layer is dried and concentrated to afford the desired product.

*Illustrative example of method AG: synthesis of intermediate 84, 4-chloro-5-(cyclopropylmethoxy)benzene-1,2-diamine*

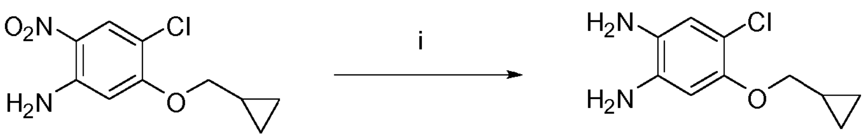


Zinc dust (6.5 g, 99 mmol, 12 eq) is added to a mixture of 4-chloro-5-(cyclopropylmethoxy)-2-nitro-aniline (2 g, 8.3 mmol, 1 eq) and $NH_4Cl$ (5.3 g, 99 mmol, 12 eq) in dry methanol (55 mL) at 0°C followed by formic acid (0.62 mL, 16.5 mmol, 2 eq). The mixture is stirred at room temperature for 24 h. The solids are filtered off and the filtrate is concentrated. The residue is taken up in ethyl acetate. The mixture is washed (saturated $NaHCO_3$ and saturated $NH_4Cl$), dried ($Na_2SO_4$) and concentrated to afford the desired product.

*Intermediate 85, [5-cyano-6-(cyclopropylmethoxy)-1-(2-trimethylsilylethoxymethyl)benzimidazol-2-yl]methyl acetate*

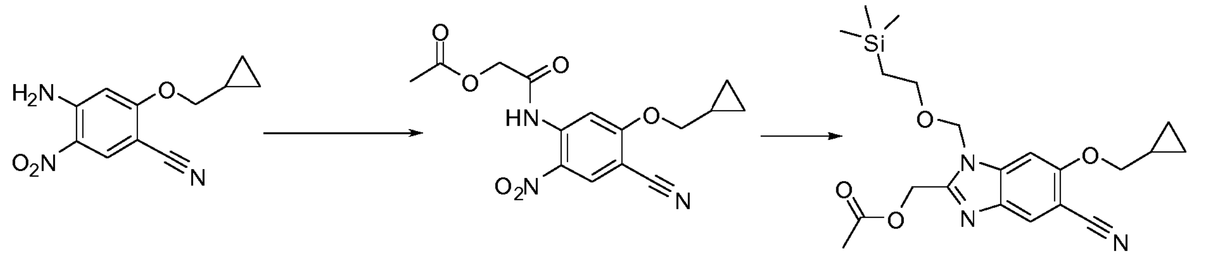

*Step i: [2-[4-cyano-5-(cyclopropylmethoxy)-2-nitro-anilino]-2-oxo-ethyl] acetate*

(2-chloro-2-oxo-ethyl) acetate (2.19 mL, 20 mmol 1.6 eq) is added to a solution of 4-amino-2-(cyclopropylmethoxy)-5-nitro-benzonitrile (3 g, 12.4 mmol, 1 eq) and triethylamine (1.74 mL, 12.4 mmol, 1 eq) in dry DCM (30 mL) at 0°C. The mixture is stirred at room temperature for 24 h. The reaction mixture is diluted (DCM). The resulting mixture is washed ($NaHCO_3$, 5% citric acid in water and brine), dried ($Na_2SO_4$) and concentrated to afford [2-[4-cyano-5-(cyclopropylmethoxy)-2-nitro-anilino]-2-oxo-ethyl] acetate.

*Step ii: [5-cyano-6-(cyclopropylmethoxy)-1-(2-trimethylsilylethoxymethyl)benzimidazol-2-yl]methyl acetate*

NaH (60% mineral oil, 470 mg, 1.3 eq) is added to a solution of [2-[4-cyano-5-(cyclopropylmethoxy)-2-nitro-anilino]-2-oxo-ethyl] acetate (3.0 g, 1.0 eq, 9.0 mmol) in dry DMF (50 mL) at 0°C. The mixture is stirred at 0°C for 10 min. 2-(trimethylsilyl)ethoxymethyl chloride (2.39 mL, 1.5 eq) is added dropwise. The mixture is stirred at room temperature for 16 h. Standard work up is done using extraction between ethyl acetate and $NaHCO_3$ saturated water solution. The gathered organic layers are washed with brine and dried over $Na_2SO_4$. The solvent is removed under reduced pressure. The residue is dissolved in 50 mL of dry DMF. The mixture is cooled to 0°C. To the mixture, another portion of sodium hydride, NaH 60 % dispersion on mineral oil (120 mg, 0.33 eq) is added followed by 2-(trimethylsilyl)ethoxymethyl chloride (0.58 mL, 0.34 eq) and the mixture is stirred at room temperature for 3 h. Standard work up is done using extraction between ethyl acetate and $NaHCO_3$ saturated water solution. The gathered organic layers are washed with brine and dried over $Na_2SO_4$. The solvent is removed under reduced pressure. The residue is dissolved in 40 mL of glacial acetic acid. To the mixture Iron, Fe, powder (2.03 g, 4.0 eq) is added and the mixture is stirred at 70°C for 2 h. The mixture is cooled and diluted with 100 mL of DCM. The resulting mixture is filtered over a celite pad. The filtrate is slowly added to 300 mL of a stirred saturated $NaHCO_3$ aqueous solution. After the addition the layers are separated. The organic layer is washed with 100 mL of saturated $NaHCO_3$ water solution followed by 200 mL of brine. After drying over $Na_2SO_4$ and filtration the solvent is removed under reduced pressure. The residue is purified by flash column chromatography ($SiO_2$, DCM/ethyl acetate 100:0 to 75:25) to yield the desired product.

*Intermediate 86, 3-chloro-4-(2,2-difluoroethoxy)benzaldehyde*

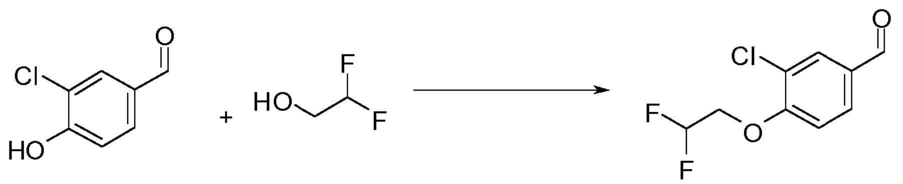

Diisopropyl azodicarboxylate (2.99 mL, 14.9 mmol, 1.2 eq) is added dropwise to a solution of 3-chloro-4-hydroxybenzaldehyde (2.0 g, 12.4 mmol, 1 eq), 2,2-difluoroethanol (0.951 mL, 14.9 mmol, 1.2 eq) and $PPh_3$ (4.9 g, 18.6 mmol, 1.5 eq) in dry THF (55 mL) 0° C. The reaction mixture is stirred at room temperature for 30 min and at 60°C for 2 h. The mixture is concentrated and the residue is purified by flash column chromatography ($SiO_2$, cyclohexane/ethyl acetate 100:0 to 75:25 and then DCM) to afford the desired product.

*Intermediate 87, ethyl-2-azido-3-[3-chloro-4-(2,2-difluoroethoxy)phenyl]prop-2-enoate*

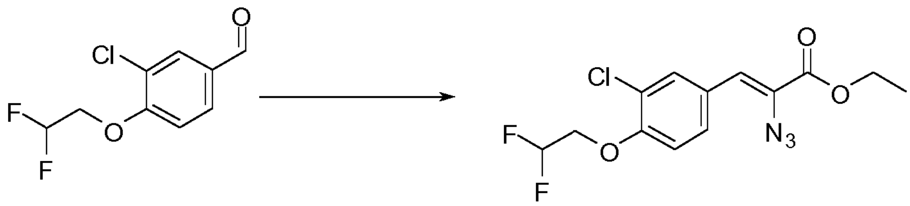

NaOEt (20% in ethanol, 8.9 mL, 22.7 mmol, 3 eq) is added dropwise to a solution of 3-chloro-4-(2,2-difluoroethoxy)benzaldehyde (1.67 g, 7.6 mmol, 1 eq) and ethyl 2-azidoacetate (2.7 mL, 22.7 mmol, 3 eq) in dry ethanol (31 mL) at -10°C. During the addition the temperature is kept at -10°C. The mixture is stirred at -10°C for 3 h. The mixture is quenched with water and the resulting mixture is extracted (DCM). The organic layer is washed (brine), dried ($Na_2SO_4$) and concentrated. The residue is purified by flash column chromatography ($SiO_2$, cyclohexane/ethyl acetate 100:0 to 83:17) to yield the desired product.

*Intermediate 88, ethyl 5-chloro-6-(2,2-difluoroethoxy)-1H-indole-2-carboxylate*

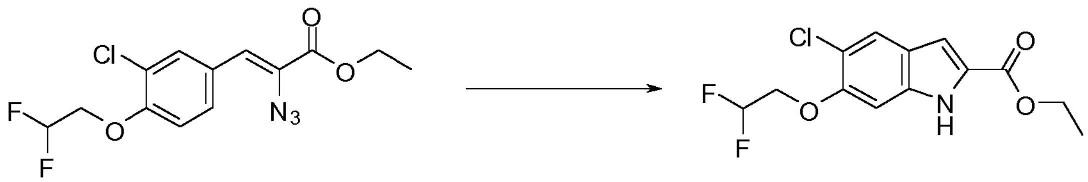

A mixture of ethyl-2-azido-3-[3-chloro-4-(2,2-difluoroethoxy)phenyl]prop-2-enoate (1.56 g, 7.57 mmol) in dry p-xylene (31 mL) is stirred at 150°C for 2 h. The mixture is concentrated and the residue is purified by flash column chromatography ($SiO_2$, cyclohexane/ethyl acetate 100:0 to 83:17) to yield the desired product.

### *1.35. General method AH: SEM protection of bicyclic derivatives*

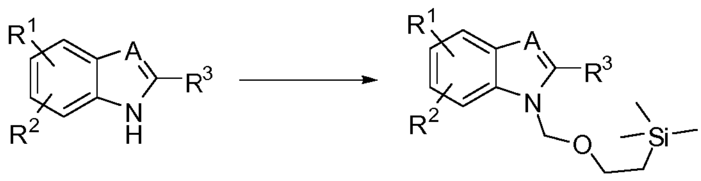

NaH (1.1 eq) is added to a mixture of nucleophile (1 eq) in DMF at 0°C and the mixture is stirred typically for 15 min. 2-(trimethylsilyl)ethoxymethyl chloride (1.1 eq) is added and the mixture is stirred at room temperature typically for 90 min. The mixture is quenched with an aqueous solution and the resulting mixture is extracted with an organic solvent. The organic layer is washed, dried and concentrated. The residue is typically purified by flash column chromatography to afford the desired product.

*Illustrative example of method AH: synthesis of intermediate 89, ethyl 5-chloro-6-(2,2-difluoroethoxy)-1-(2-trimethylsilylethoxymethyl)indole-2-carboxylate*

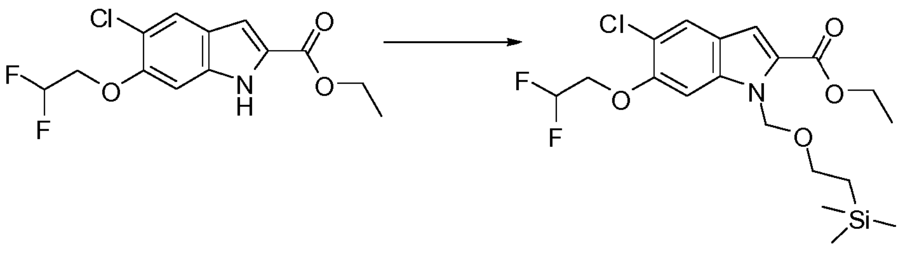

NaH (60% dispersion in mineral oil, 141 mg, 3.5 mmol, 1.1 eq) is added to a mixture of ethyl 5-chloro-6-(2,2-difluoroethoxy)-1H-indole-2-carboxylate (972 mg, 3.2 mmol, 1 eq) at 0°C in DMF (7 mL) and the mixture is stirred for 15 min. 2-(trimethylsilyl)ethoxymethyl chloride (0.65 mL, 3.52 mmol, 1.1 eq) is added and the mixture is stirred at room temperature for 90 min. The reaction mixture is quenched with saturated $NH_4Cl$. The resulting mixture is extracted with ethyl acetate. The organic mixture is washed (water and brine), dried ($Na_2SO_4$) and concentrated. The residue is purified by flash column chromatography ($SiO_2$, cyclohexane/ethyl acetate 99:1 to 88:12) to yield the desired product.

### *1.36. General method AI: reduction of esters to alcohols*

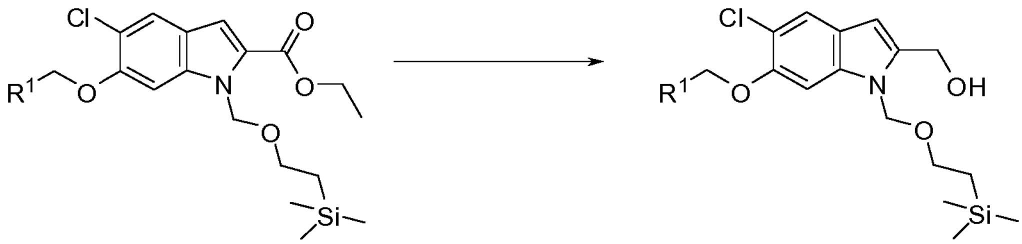

$LiAlH_4$ (1.6 eq) is added to a solution of the ester (1 eq) in dry THF at -10°C. The reaction mixture is stirred at -10°C typically for 1 h. The reaction is quenched by careful addition of water followed by NaOH. The byproducts are typically separated by precipitation and the desired product is obtained after concentration of the solvent.

*Illustrative example of method AI: synthesis of intermediate 90, [5-chloro-6-(2,2-difluoroethoxy)-1-(2-trimethylsilylethoxymethyl)indol-2-yl]methanol*

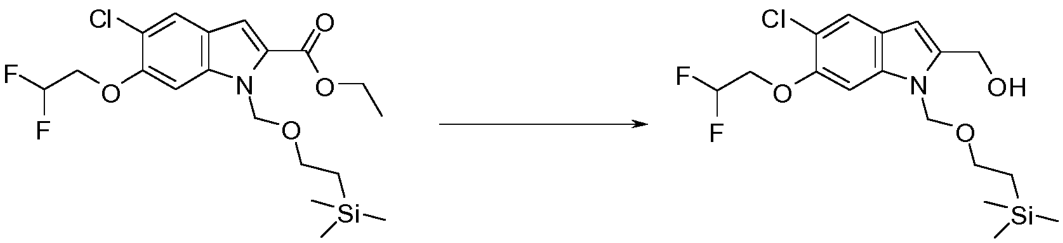

$LiAlH_4$ (2,4 M solution in THF, 1.55 mL, 3.7 mmol, 1.6 eq) is added to a solution of ethyl 5-chloro-6-(2,2-difluoroethoxy)-1-(2-trimethylsilylethoxymethyl)indole-2-carboxylate (1 g, 2.33 mmol, 1 eq) in dry THF (9 mL) at -10°C. The reaction mixture is stirred at -10°C for 1 h. The reaction is quenched by careful addition of water (0.2 mL) followed by NaOH (15% aq, 0.2 mL) and again water (0.6 mL). The resulting mixture is stirred at room temperature for 1 h. A precipitate is formed and filtered off. The precipitate is concentrated to yield the desired product.

### *1.37. General method AJ: synthesis of benzimidazoles*

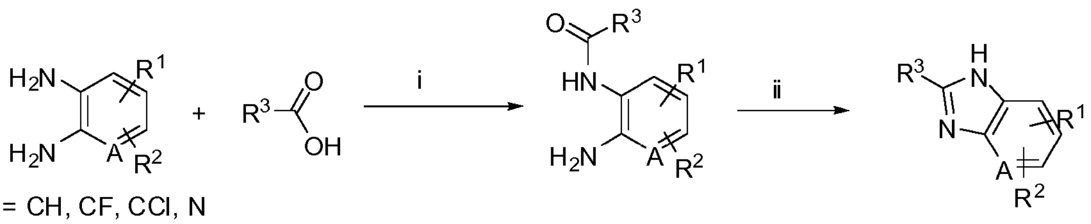


A = CH, CF, CCl, N

*Step i:*

The acid (1 eq), a base, typically diisopropylethylamine (2.5 eq) or triethylamine (3 eq) and a coupling agent, typically HATU (1.1 eq) or EDC.HCl/HOBt (1.5 and 0.15 eq) are mixed in an organic solvent, typically DMF or THF at 0°C. The bis-aniline (1 to 1.5 eq) is added and the mixture is stirred typically at room temperature for 2 to 16 h. The mixture is diluted with an organic solvent and the resulting mixture undergoes an aqueous work up. The mixture is

concentrated to afford the desired intermediate, which may be further purified by flash column chromatography.

*Step ii:*

The amide from the previous step is stirred in acetic acid typically at 70°C for 4 h. The mixture is concentrated and the residue is taken up in an organic solvent. The mixture typically undergoes an organic work up. The organic layer is dried and concentrated to afford the desired product.

*Illustrative example of method AJ: synthesis of intermediate 91, ethyl 1-[[6-chloro-5-(cyclopropylmethoxy)-1H-benzimidazol-2-yl]methyl]-4-oxo-cinnoline-3-carboxylate*

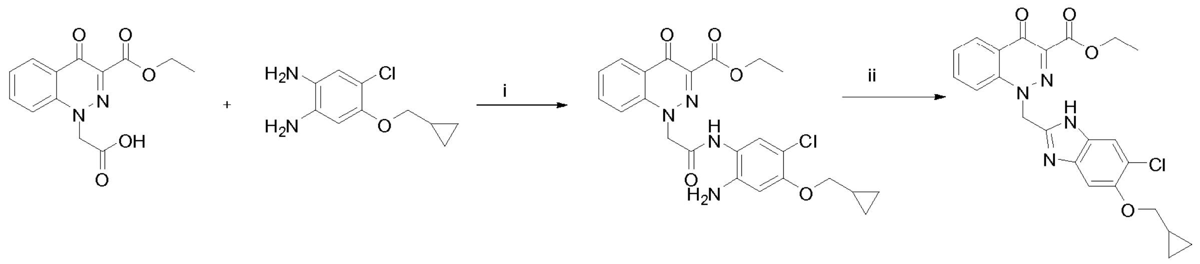


*Step i: ethyl 1-[2-[2-amino-5-chloro-4-(cyclopropylmethoxy)anilino]-2-oxo-ethyl]-4-oxo-cinnoline-3-carboxylate*

A mixture of 2-(3-ethoxycarbonyl-4-oxo-cinnolin-1-yl)acetic acid (200 mg, 0.72 mmol, 1 eq), HOBt (15 mg, 0.11 mmol, 0.15 eq), EDC.HCl (207 mg, 1.08 mmol, 1.5 eq) and triethylamine (0.3 mL, 2.16 eq, 3 eq) in THF is stirred at 0°C for 15 min. A solution of 4-chloro-5-(cyclopropylmethoxy)benzene-1,2-diamine (153 mg, 0.72 mmol, 1 eq) in THF (5.5 mL total volume of THF) is added and the mixture is stirred at room temperature for 2 h. The mixture is diluted with ethyl acetate. The organic layer is washed (saturated citric acid, water, saturated $NaHCO_3$ and brine), dried ($Na_2SO_4$) and concentrated to yield ethyl 1-[2-[2-amino-5-chloro-4-(cyclopropylmethoxy)anilino]-2-oxo-ethyl]-4-oxo-cinnoline-3-carboxylate.

*Step ii: ethyl 1-[[6-chloro-5-(cyclopropylmethoxy)-1H-benzimidazol-2-yl]methyl]-4-oxo-cinnoline-3-carboxylate*

A mixture of ethyl 1-[2-[2-amino-5-chloro-4-(cyclopropylmethoxy)anilino]-2-oxo-ethyl]-4-oxo-cinnoline-3-carboxylate (338 mg, 0.72 mg, 1 eq) in acetic acid (6.6 mL) is stirred at 70°C for 4 h. The mixture is concentrated. The residual acetic acid is removed by co-evaporation with heptane to afford the desired product.

## *1.38. General method AK: N-alkylation of bicyclic systems*

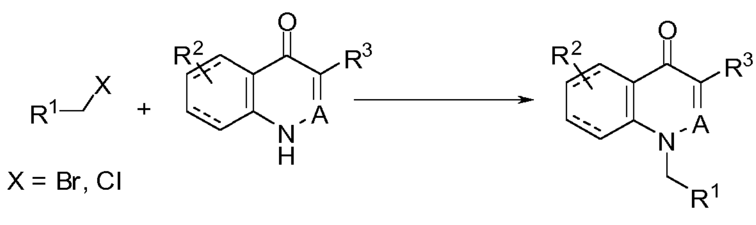

A mixture of the alkylating agent (typically alkyl chloride or bromide, 1 eq), the nucleophile (1 eq) and $K_2CO_3$ (2 eq) in DMF is stirred typically at 60°C for 2.5 h. The mixture is diluted with an organic solvent and the mixture undergoes aqueous work up. The mixture is dried and concentrated. The residue is typically purified by flash column chromatography to afford the desired product.

*Illustrative example of method AK: synthesis of intermediate 92, methyl 2-[1-[[7-chloro-6-(cyclopropylmethoxy)imidazo[1,2-a]pyridin-2-yl]methyl]-4-oxo-cinnolin-3-yl]propanoate*

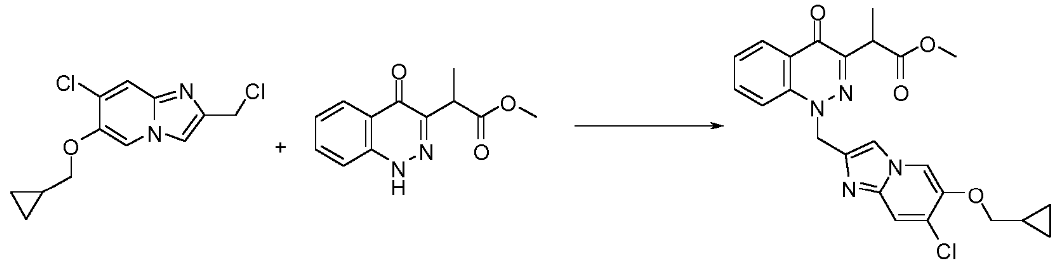

A mixture of 7-chloro-2-(chloromethyl)-6-(cyclopropylmethoxy)imidazo[1,2-a]pyridine (920 mg, 3.4 mmol, 1.03 eq), methyl 2-(4-oxo-1H-cinnolin-3-yl)propanoate (765 mg, 3.3 mmol, 1 eq) and $K_2CO_3$ (910 mg, 6.6 mmol, 2 eq) in DMF (13 mL) is stirred at 60°C for 2.5 h. The reaction mixture is diluted (ethyl acetate), washed (water, saturated $NaHCO_3$ and LiCl), dried ($Na_2SO_4$) and concentrated. The residue is purified by flash column chromatography ($SiO_2$, DCM/methanol 99.75:0.25 to 97.5:2.5) to afford the desired product.

## 1.39. General method AL: SEM deprotection of benzimidazoles

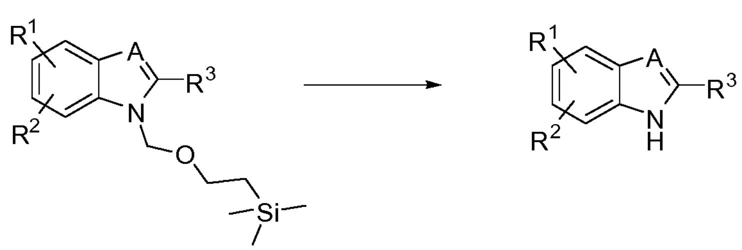

A solution of the benzimidazole in an acidic mixture (typically 2:1 DCM/TFA or 3:1 ethanol/0.5 HCl in methanol) is stirred typically at 20 to 60°C for 2 to 72 h. The desired product is isolated after precipitation or the mixture is concentrated and the residue typically undergoes an aqueous work up. The organic layer is dried and concentrated. The residue may be further purified by flash column chromatography or preparative HPLC.

*Illustrative example of method AL: synthesis of intermediate 93, ethyl 1-[[6-cyano-5-(2,2-difluoroethoxy)-1H-benzimidazol-2-yl]methyl]-4-oxo-quinoline-3-carboxylate*

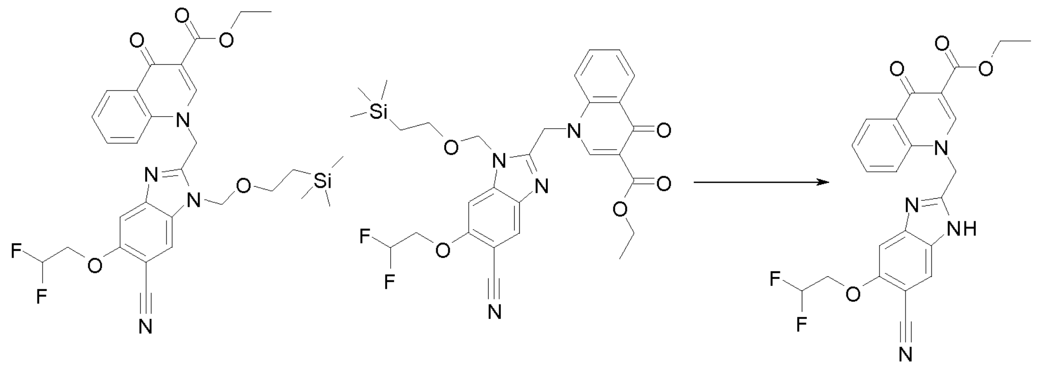

A mixture of ethyl 1-[[6-cyano-5-(2,2-difluoroethoxy)-1-(2-trimethyl silylethoxymethyl)benzimidazol-2-yl]methyl]-4-oxo-quinoline-3-carboxylate & ethyl 1-[[5-cyano-6-(2,2-difluoroethoxy)-1-(2-trimethylsilylethoxymethyl)benzimidazol-2-yl]methyl]-4-oxo-quinoline-3-carbo xylate (69 mg in total, 0.12 mmol, 1 eq) in 3:1 ethanol/0.5 HCl in methanol (4 mL) is stirred at 60°C for 8 h. The mixture is cooled to room temperature and a precipitated is formed. The solid is filtered off and washed with diethyl ether to afford the desired product.

### 1.40. General method AM: simultaneous Boc and SEM deprotection

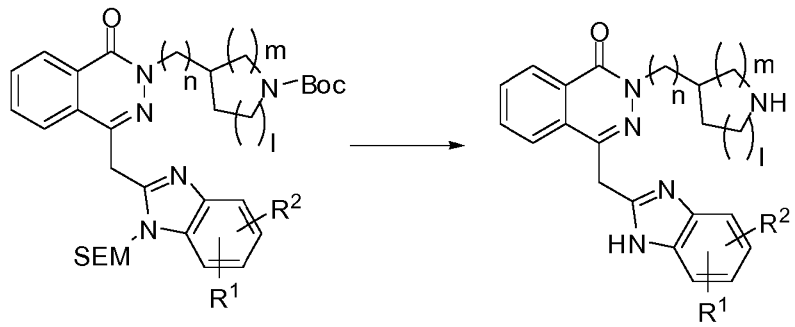

A mixture of the derivative with a simultaneous Boc and SEM protecting groups (1 eq) in 2:1 DCM/TFA is stirred at 40°C typically for 2 h. The mixture is concentrated. The residue is typically purified by preparative HPLC to afford the desired product.

*Illustrative example of method AM: synthesis of final compound 1, 6-(2,2-difluoroethoxy)-2-[[4-oxo-3-(pyrrolidin-2-ylmethyl)phthalazin-1-yl]methyl]-3H-benzimidazole-5-carbonitrile*

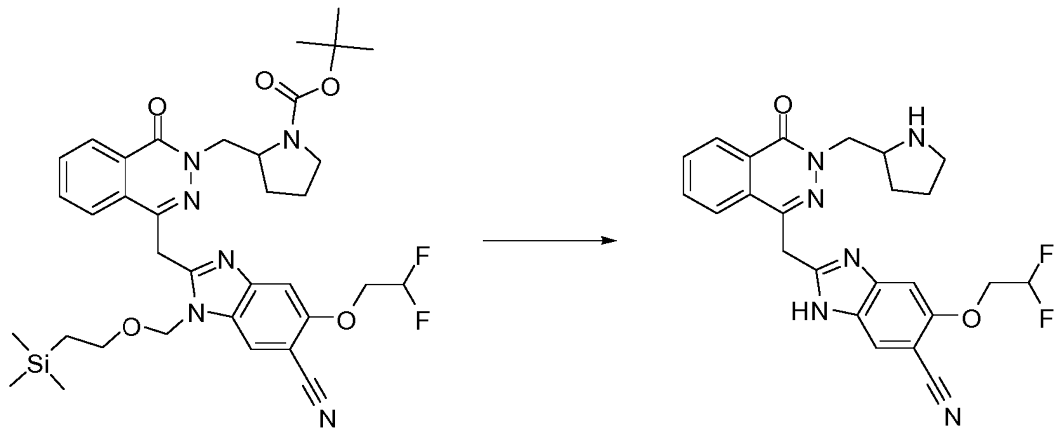

To a solution of tert-butyl 2-[[4-[[6-cyano-5-(2,2-difluoroethoxy)-1-(2-trimethylsilylethoxymethyl)benzimidazol-2-yl]methyl]-1-oxo-phthalazin-2-yl]methyl]pyrrolidine-1-carboxylate (39 mg, 0.06 mmol, 1eq) in DCM (0.18 mL) is added trifluoroacetic acid (0.11 mL). The reaction is stirred at 40°C for 1h. The reaction is The reaction is coevaporated in toluene and sent to preparative HPLC.

### 1.41. General method AN: Boc deprotection in presence of SEM protecting group

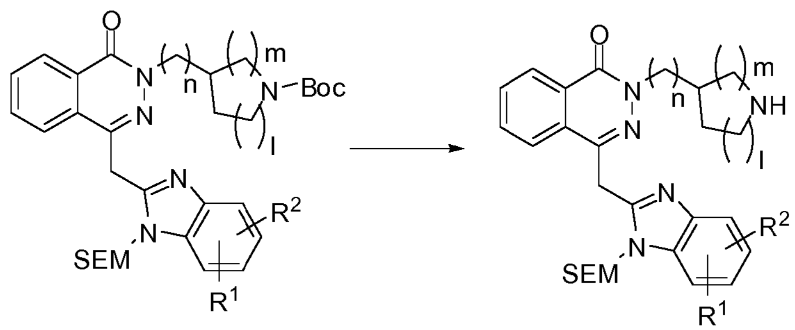

A mixture of the derivative with a simultaneous Boc and SEM protecting groups (1 eq) in 7:1 DCM/TFA is stirred at room temperature typically for 1 h. The mixture is concentrated to afford the desired product.

*Illustrative example of method AN: synthesis of intermediate 94, 6-(2,2-difluoroethoxy)-2-[(4-oxo-3-pyrrolidin-3-yl-phthalazin-1-yl)methyl]-3-(2-trimethylsilylethoxymethyl)benzimidazole-5-carbonitrile*

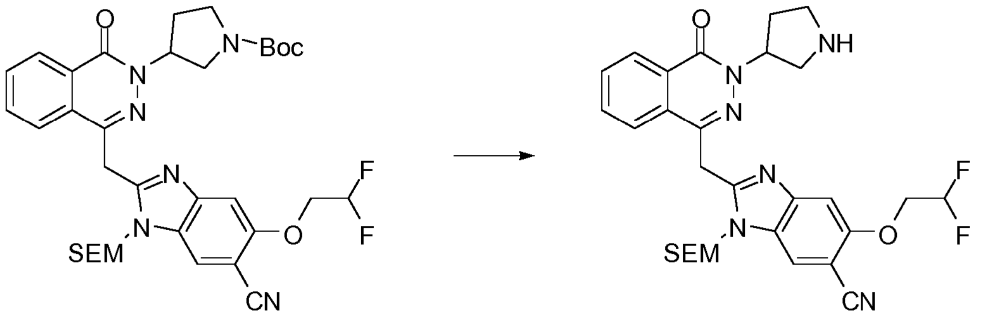

To a solution of tert-butyl 3-[4-[[6-cyano-5-(2,2-difluoroethoxy)-1-(2-trimethylsilylethoxymethyl)benzimidazol-2-yl]methyl]-1-oxo-phthalazin-2-yl]pyrrolidine-1-carboxylate (152 mg, 0.22 mmol, 1eq) in DCM (7 mL) is added trifluoroacetic acid (1.1 mL). The reaction is stirred at room temperature for 1h. The reaction is coevaporated in toluene and sent to preparative HPLC.

### 1.42. General method AO: Boc deprotection in presence of SEM protecting group

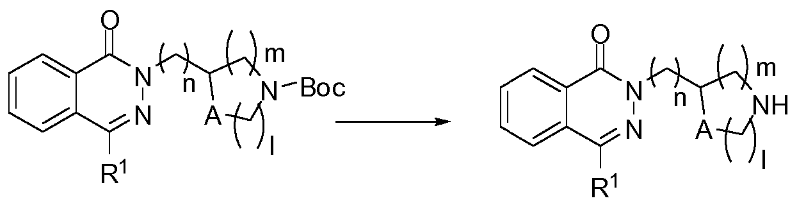

A = $CH_2$, O

The Boc-protected amine (1 eq) is stirred a room temperature in an acidic medium (typically 4:1 DCM/TFA or 1:1 acetonitrile/4 M HCl in 1,4-dioxane or 10:1 methanol/acetyl chloride) for 1 to 16 h. The reaction mixture typically undergoes one or multiple purification techniques, such as aqueous work up, flash column chromatography, SCX resin exchange or preparative HPLC to afford the desired product.

*Illustrative example of method AO: synthesis of final compound 2, 2-(azetidin-3-yl)-4-[[6-chloro-5-(cyclopropylmethoxy)indazol-2-yl]methyl]phthalazin-1-one*

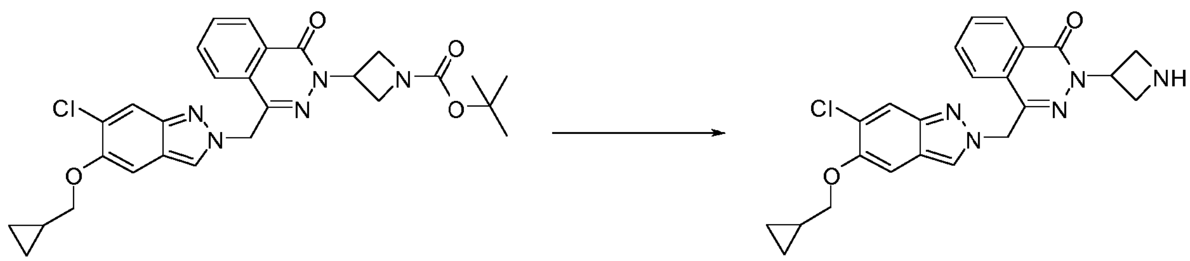

A mixture of tert-butyl 3-[4-[[6-chloro-5-(cyclopropylmethoxy)indazol-2-yl]methyl]-1-oxo-phthalazin-2-yl]azetidine-1-carboxylate (16 mg, 0.03 mmol, 1 eq) in DCM/TFA 4:1 (0.5 mL) is stirred at room temperature for 1 h. The reaction mixture was loaded onto an SCX column. The column is washed with methanol and the compound is eluted with 1:4 7 N $NH_3$ in methanol/methanol. The resulting solution is concentrated to afford the desired product.

*Intermediate 95, 1-chloro-2-(cyclopropylmethoxy)-4-nitro-benzene*

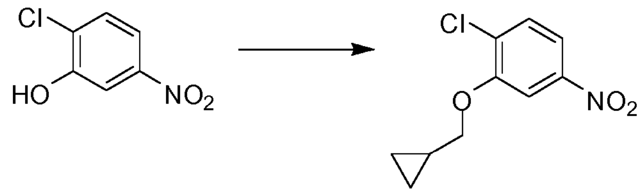

To a solution of 2-chloro-5-nitrophenol (5 g, 29 mmol, 1eq) in dry dimethylformamide (48 mL) is added potassium carbonate (6 g, 44 mmol, 1.5 eq) and cyclopropyl methyl bromide (3.1 mL, 32 mmol, 1.1 eq). The reaction is stirred at room temperature overnight. The reaction is diluted with ethyl acetate and $H_2O$. The aqueous layer is extracted with ethyl acetate. The organic layer is washed (brine) and dried ($Na_2SO_4$), filtered and concentrated to afford the desired product.

*Intermediate 96, 4-chloro-3-(cyclopropylmethoxy)aniline*

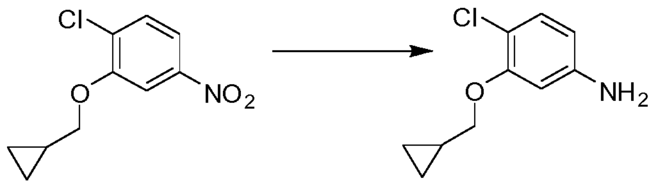

To a solution of 1-chloro-2-(cyclopropylmethoxy)-4-nitro-benzene (6.4 g, 29 mmol, 1eq) in dry methanol (187 mL) is added ammonium chloride (18 g, 336 mmol, 12 eq). Zinc dust (22 g, 336 mmol, 12eq) is then added to the reaction mixture followed by a dropwise addition of formic acid at 0°C. The reaction was stirred at 0°C for 10 min then is warmed up to room temperature. After 5 h, an additional amount of zinc (4.4 g), ammonium chloride (3.6 g) and formic acid (0.4 mL) are added to the reaction mixture. After 2 h, the zinc is filtered off on celite and the filtrate is evaporated. The residue is then dissolved in ethyl acetate (12 mL) and quenched with a saturated solutions of $NaHCO_3$ (2x10 mL) and ammonium chloride (2x10 mL). The organic layer is dried ($Na_2SO_4$), filtered and concentrated to afford the desired product.

*Intermediate 97, 4-chloro-5-(cyclopropylmethoxy)-2-iodo-aniline*

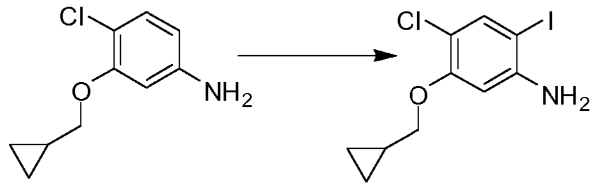

To a solution of 4-chloro-3-(cyclopropylmethoxy)aniline (5.5 g, 28 mmol, 1eq) in dry acetonitrile (70 mL) is added N-iodosuccinimide (7.6 g, 34 mmol, 1.2 eq). The reaction is stirred at room temperature for 30 min. 10% of an aqueous solution of sodium thiosulfate is added to the reaction mixture that is stirred at room temperature for 15 min. Ethyl acetate and a saturated solution of $NaHCO_3$ are then added. The aqueous layer is extracted with ethyl acetate. The organic layer is washed (brine) and dried ($Na_2SO_4$), filtered and concentrated to afford the desired product.

*Intermediate 98, 5-chloro-6-(cyclopropylmethoxy)-1H-indole-2-carboxylic acid*

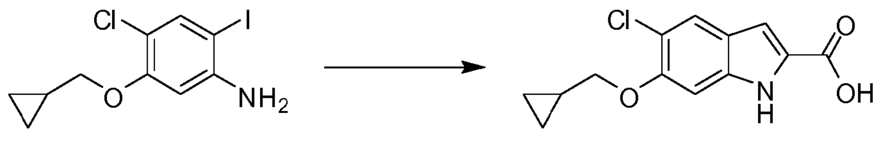

To a solution of 4-chloro-5-(cyclopropylmethoxy)-2-iodo-aniline (6 g, 18.6 mmol, 1eq) in dry dimethylformamide (37 mL) is added 1,4-diazabicyclo[2.2.2]octane (6.2 g, 55.8 mmol, 3.0 eq). The reaction is stirred at room temperature for 20 min. The reaction is degassed. Pyruvic acid (5.2 mL, 56 mmol, 3eq) is added to the reaction mixture. The reaction is heated at 40°C for 30 min. Palladium (II) acetate (42 mg, 0.19 mmol, 0.01 eq) is then added to the degassed reaction.

The reaction is stirred at 100°C overnight. The reaction is diluted with ethyl acetate and washed with $H_2O$. The aqueous layer is acidified with HCl pH 3 and extracted with ethyl acetate. The organic layer is washed (brine) and dried ($Na_2SO_4$), filtered and concentrated to afford the desired product that is used as such in the next step.

*Intermediate 99, methyl 5-chloro-6-(cyclopropylmethoxy)-1H-indole-2-carboxylate*

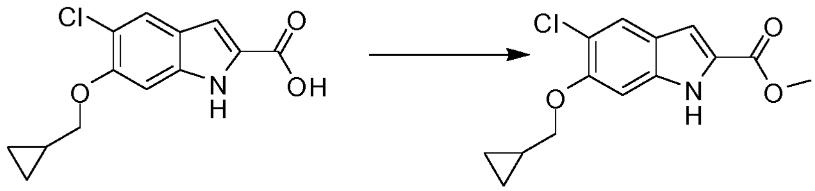

To a solution of 5-chloro-6-(cyclopropylmethoxy)-1H-indole-2-carboxylic acid (4.9 g, 18.6 mmol, 1eq) in dry dimethylformamide (47 mL) is added carbonyldiimidazole (3.6 g, 22.3 mmol, 1.2 eq). The reaction is stirred at room temperature for 1 h. Methanol (21 mL) is then added to the reaction that is stirred at 40°C for 4 h. Water and brine are added. The aqueous layer is extracted with ethyl acetate. The organic layer is washed (brine) and dried ($Na_2SO_4$), filtered and concentrated to afford the desired product that is used as such in the next step.

*Intermediate 100, methyl 2-(4-methyl-1-oxo-phthalazin-2-yl)cyclobutanecarboxylate*

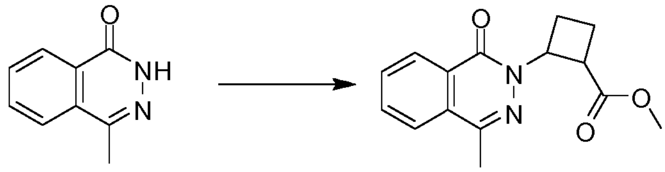

To a solution of 4-methylphtalazin-1(2H)-one (500 mg, 3.13 mmol, 1eq) in dry dimethylacetamide (10 mL) are added potassium carbonate (519 mg, 3.76 mmol, 1.2eq) and methyl-1-bromocyclobutanecarboxylate (0.3 mL, 3.44 mmol, 1.1eq). The reaction is stirred at 150°C for 6 h. An additional amount of methyl-1-bromocyclobutanecarboxylate (0.1 mL) and potassium carbonate (140 mg) are then added to the reaction that is stirred at 150°C for 2 h. The reaction is diluted with ethyl acetate. The organic layer is washed with a saturated solution of $NaHCO_3$ and brine then dried ($Na_2SO_4$), filtered and concentrated. The residue is purified by flash chromatography by using a gradient of petroleum ether: ethyl acetate 9:1 till 7:1 to give the product.

*Intermediate 101, methyl 2-(4-methyl-1-oxo-phthalazin-2-yl)prop-2-enoate*

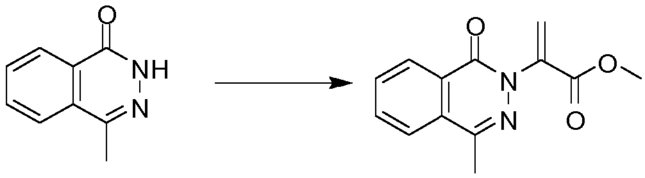

To a solution of 4-methylphtalazin-1(2H)-one (400 mg, 2.5 mmol, 1eq) in dry toluene (25 mL) arc added methyl propiolate (210 mg, 2.5 mmol, 1eq), triphenylphosphine (66 mg, 0.25 mmol, 0.1eq), acetic acid (0.07 mL, 1.25 mmol, 0.5eq) and sodium acetate (103 mg, 1.25 mmol, 0.5eq). The reaction is stirred at 110°C for 2 h. The reaction is quenched with water. The aqueous layer is extracted with ethyl acetate. The organic layer is dried ($Na_2SO_4$) filtered and concentrated to give the desired product that is used as such in the next step.

*Intermediate 102, methyl 1-(4-methyl-1-oxo-phthalazin-2-yl)cyclopropanecarboxylate*

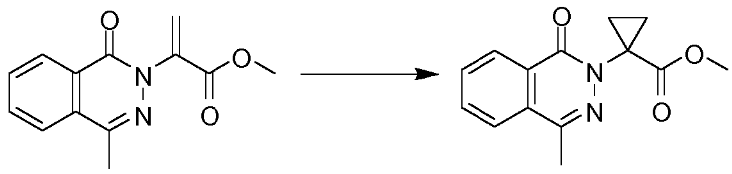

To a solution of trimethylsulfoxonium (1.1 g, 5 mmol, 2eq) in dimethylsulfoxide (5 mL) is added sodium hydride (200 mg, 5 mmol, 2eq). The reaction is stirred at room temperature for 45 min. A solution of methyl 2-(4-methyl-1-oxo-phthalazin-2-yl)prop-2-enoate (610 mg, 2.5 mmol, 1eq) in dimethylsulfoxide (4 mL) is added to the reaction mixture. The reaction is stirred at room temperature for 2 h. The reaction is quenched with water. The organic layer is washed (water) and dried ($Na_2SO_4$) filtered and concentrated. The residue is purified by flash chromatography by using a gradient of petroleum ether: ethyl acetate 95:5 till 100% ethyl acetate to give the desired product.

*Intermediate 103, 6-(2,2-difluoroethoxy)-2-[[8-methyl-3-(2-morpholino-2-oxo-ethyl)-4-oxo-phthalazin-1-yl]methyl]-3-(2-trimethylsilylethoxymethyl)benzimidazole-5-carbonitrile*

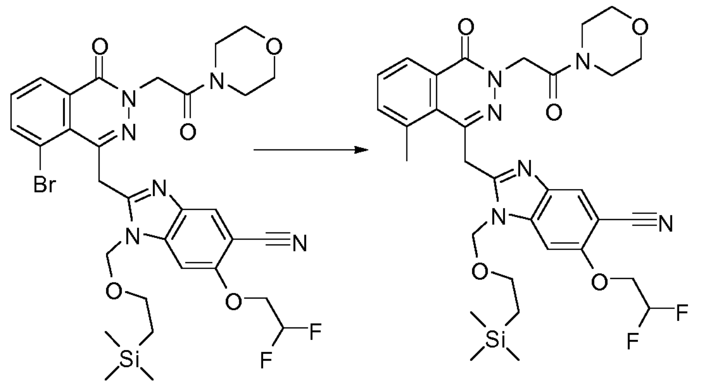

*Step i: 6-(2,2-difluoroethoxy)-2-[[8-methyl-3-(2-morpholino-2-oxo-ethyl)-4-oxo-phthalazin-1-yl]methyl]-3-(2-trimethylsilylethoxymethyl)benzimidazole-5-carbonitrile*

SPhos G3 (5 mg, 0.007 mmol, 0.05 eq) is added to a degassed solution of 2-[[8-bromo-3-(2-morpholino-2-oxo-ethyl)-4-oxo-phthalazin-1-yl]methyl]-6-(2,2-difluoroethoxy)-1-(2-trimethylsilyl ethoxymethyl)benzimidazole-5-carbonitrile (100 mg, 0.139 mmol, 1 eq), methyl boronic acid (25 mg, 0.418 mmol, 3 eq), and cesium carbonate (226 mg, 0.695 mmol, 5 eq) in dry dioxane (1 mL). The mixture is stirred for 2 h at 90°C. The reaction is quenched with water and extracted with DCM. The organic layer is filtered through a phase separator and concentrated to afford the desired product.

*Intermediate 104, ethyl 2-[4-[[6-chloro-5-(cyclopropylmethoxy)indazol-2-yl]methyl]-6-cyano-1-oxo-phthalazin-2-yl]propanoate*

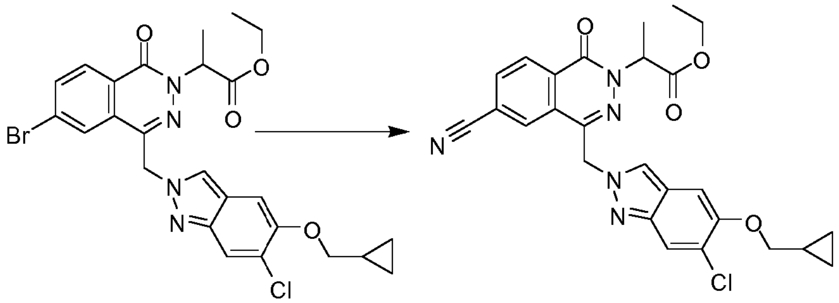

A mixture of ethyl 2-[6-bromo-4-[[6-chloro-5-(cyclopropylmethoxy)indazol-2-yl]methyl]-1-oxo-phthalazin-2-yl]propanoate (110 mg, 0.196 mmol, 1 eq), zinc cyanide (23 mg, 0.196 mmol, 1 eq) and $Pd(PPh_3)_4$ (18 mg, 0.020 mmol,

0.1eq) in DMF (1 mL) is stirred at 150°C for 5 min under microwave conditions. The mixture is diluted with ethyl acetate, washed (sat. $NaHCO_3$ solution and brine), dried ($Na_2SO_4$) and concentrate to afford the desired product.

*Intermediate 105: ethyl 2-[8-cyclopropyl-3-(2-morpholino-2-oxo-ethyl)-4-oxo-phthalazin-1-yl]acetate*

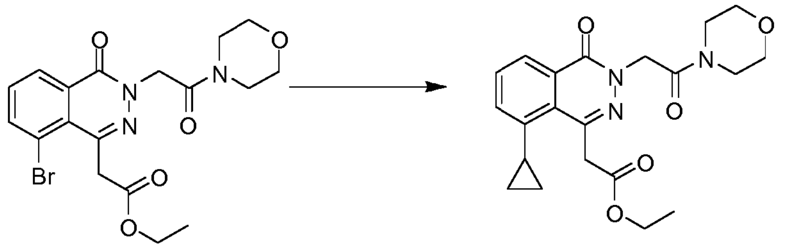

Under a nitrogen atmosphere, palladium (II) acetate is added to a mixture of ethyl 2-[8-bromo-3-(2-morpholino-2-oxo-ethyl)-4-oxo-phthalazin-1-yl]acetate (358 mg, 0.82 mmol, 1.0 eq), cyclopropyl boronic acid (175 mg, 2.04 mmol, 2.5 eq), tricyclohexylphosphine (344 mg, 1.23 mmol, 1.5 eq) and anhydrous potassium triphosphate (1.04 g, 4.90 mmol, 6.0 eq) in anhydrous toluene (10 mL). Water (54 µL) was added; the mixture is degassed for 10 min under nitrogen atmosphere. Then, the mixture is heated at 105°C for 4 h. Then, the reaction mixture is partitioned between ethyl acetate and water; the organic layer is dried over $Na_2SO_4$, and evaporated under vacuum. The resulting crude product is triturated in diethyl ether (insoluble materials are removed); the filtrate is adsorbed on silica and purified on silica using a gradient from ethyl acetate/ petroleum ether (5:95) to (100:0), to afford ethyl 2-[8-cyclopropyl-3-(2-morpholino-2-oxo-ethyl)-4-oxo-phthalazin-1-yl]acetate.

## *1.43. General method AP: formation of α,β-unsaturated esters followed by 1,4-addition of amines*

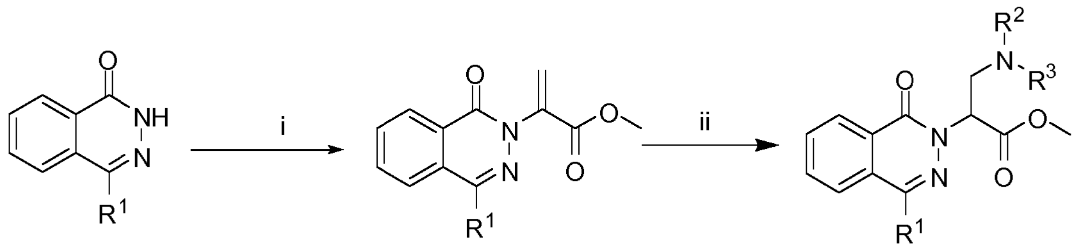


*Step i:*

A mixture of methyl prop-2-ynoate (1 eq), the phthalazinone derivative (1 eq), $PPh_3$ (0.1 eq), AcOH (0.5 eq) and NaOAc (0.5 eq) in toluene is stirred at 110°C for 2 h. The mixture is typically quenched with water and extracted with an organic solvent. The organic layer is dried and concentrated.

*Step ii:*

A mixture of the α,β-unsaturated ester (1 eq) and the amine (8 eq) in THF is stirred at room temperature for 1 h. The mixture is concentrated to afford the desired product.

*Illustrative example of method AP: intermediate 106, methyl 2-[4-[[6-cyano-5-(2,2-difluoroethoxy)-1-(2-trimethylsilylethoxymethyl)benzimidazol-2-yl]methyl]-1-oxo-phthalazin-2-yl]-3-(dimethylamino)propanoate*

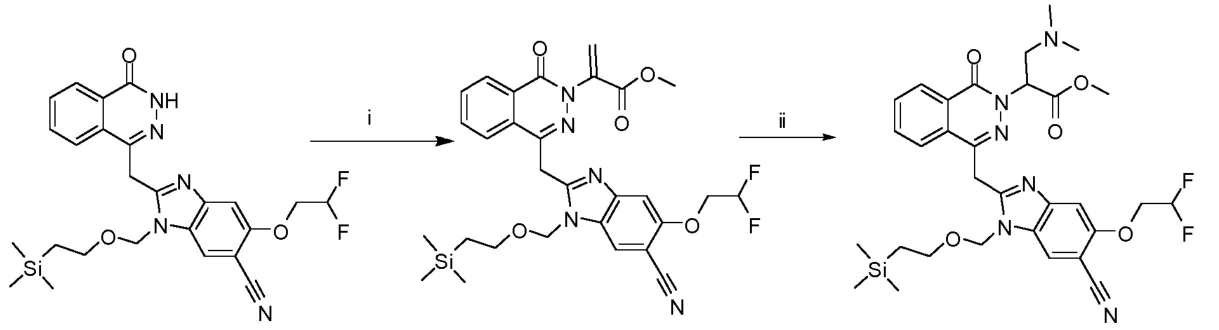


*Step i: methyl 2-[4-[[6-cyano-5-(2,2-difluoroethoxy)-1-(2-trimethylsilyl ethoxymethyl)benzimidazol-2-yl]methyl]-1-oxo-phthalazin-2-yl]-3-(dimethylamino)propanoate*

A mixture of methyl prop-2-ynoate (33 mg, 0.39 mmol,1 eq), 6-(2,2-difluoroethoxy)-2-[(4-oxo-3H-phthalazin-1-yl)methyl]-3-(2-trimethylsilylethoxymethyl)benzimidazole-5-carbonitrile (200 mg, 0.39 mmol,1 eq), $PPh_3$ (10 mg, 0.04 mmol, 0.1 eq), AcOH (0.012 mL, 0.2 mmol, 0.5 eq) and NaOAc (16 mg, 0.2 mmol, 0.5 eq) in toluene (3.9 mL) is stirred at 110°C for 2 h. The mixture is quenched with water and extracted with ethyl acetate. The organic layer is dried (filtered through a phase separator) and concentrated.

*Step ii: methyl 2-[4-[[6-cyano-5-(2,2-difluoroethoxy)-1-(2-trimethylsilylethoxymethyl) benzimidazol-2-yl]methyl]-1-oxo-phthalazin-2-yl]prop-2-enoate*

A mixture of methyl 2-[4-[[6-cyano-5-(2,2-difluoroethoxy)-1-(2-trimethylsilylethoxymethyl)benzimidazol-2-yl]methyl]-1-oxo-phthalazin-2-yl]-3-(dimethylamino) propanoate (0.12 mmol) and dimethylamine (30% solution in ethanol, 0.1 mL, 1 mmol, 8 eq) is stirred at room temperature for 1 h. The mixture is concentrated to yield the desired product.

## 1.44. General method AQ: reductive alkylation of cyclic amines

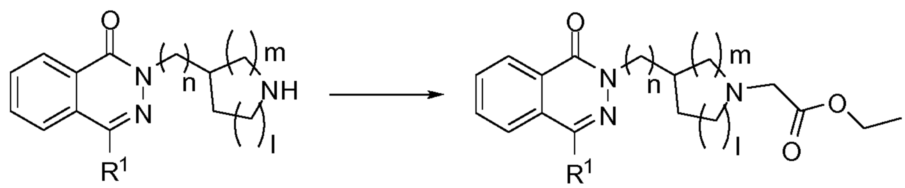

A mixture of the amine (1 eq), ethyl 2-oxoacetate (3 eq) and acetic acid (2 eq) in 1:1 DCM/methanol is stirred at room temperature for 10 min. $NaBH_3CN$ (1.2 eq) is added and the reaction mixture is stirred at room temperature for typically for 3 h. The mixture is quenched with a saturated solution of $NaHCO_3$ and extracted with an organic solvent. The organic layer is dried and concentrated to afford the desired product.

### *1.45. Illustrative example of method AQ: intermediate 107, ethyl 2-[3-[4-[[6-cyano-5-(2,2-difluoroethoxy)-1-(2-trimethylsilylethoxymethyl)benzimidazol-2-yl]methyl]-1-oxo-phthalazin-2-yl]azetidin-1-yl]acetate*

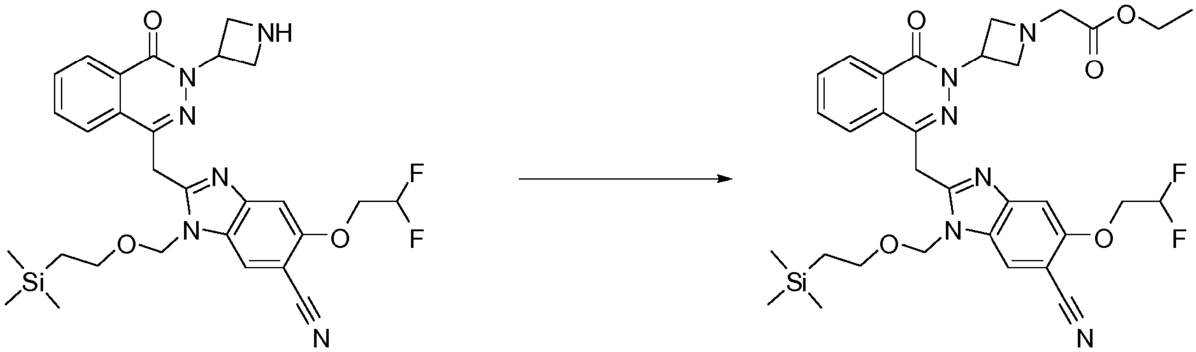

A mixture of 2-[[3-(azetidin-3-yl)-4-oxo-phthalazin-1-yl]methyl]-6-(2,2-difluoroethoxy)-3-(2-trimethylsilylethoxymethyl)benzimidazole-5-carbonitrile (102 mg, 0.18 mmol, 1 eq), ethyl 2-oxoacetate (0.11 mL, 0.54 mmol, 3 eq) and acetic acid (0.02 mL, 0.36 mmol, 2 eq) in 1:1 DCM/methanol (3 mL) is stirred at room temperature for 10 min. $NaBH_3CN$ (14 mg, 0.22 mmol, 1.2 eq) is added and the reaction mixture is stirred at room temperature for for 3 h. The mixture is quenched with a saturated solution of $NaHCO_3$ and extracted with DCM. The organic layer is dried and concentrated to afford the desired product.

### *1.46. General method AR: basic hydrolysis of methyl and ethyl esters*

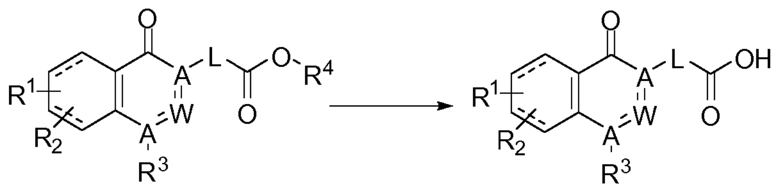

A = C, N
W = CH, N
$R^4$ = Me, Et

A mixture of the acid (1 eq) and $LiOH.H_2O$ (or LiOH) (typically 5 eq) in 1:1 (or 1:2) water/THF is stirred at room temperature typically for 2 to 72 h. The THF is removed and the aqueous mixture is acidified and extracted with an organic solvent. The organic layer is dried and concentrated to afford the desired product. The product may be further purified by preparative HPLC.

*Illustrative example of method AR: final compound 3, 2-[4-[[6-chloro-5-(cyclopropylmethoxy)indazol-2-yl]methyl]-1-oxo-phthalazin-2-yl]propanoic acid*

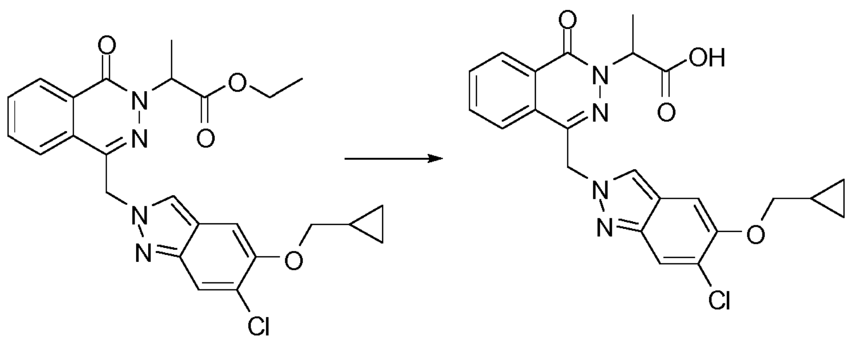

A mixture of ethyl 2-[4-[[6-chloro-5-(cyclopropylmethoxy)indazol-2-yl]methyl]-1-oxo-phthalazin-2-yl]propanoate (12.9 g, 27 mmol, 1 eq) and LiOH.$H_2O$ (5.67 g, 135 mmol, 5 eq) in 1:1 water/THF (416 mL) is stirred at room temperature for 2 h. The mixture is concentrated and diluted with water. The aqueous mixture is acidified to pH 2 with 1 N HCl. The mixture is extracted with 2:1 DCM/2-isopropanol.The organic layer is dried ($Na_2SO_4$) and concentrated to afford the desired product.

*Illustrative example of method AR: final compound 4, 2-[1-[[7-chloro-6-(cyclopropylmethoxy)imidazo[1,2-a]pyridin-2-yl]methyl]-4-oxo-cinnolin-3-yl]propanoic acid*

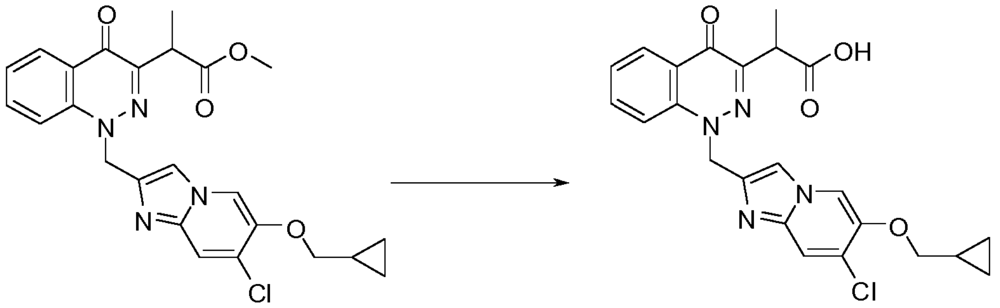

A mixture of ethyl methyl 2-[1-[[7-chloro-6-(cyclopropylmethoxy)imidazo[1,2-a]pyridin-2-yl]methyl]-4-oxo-cinnolin-3-yl]propanoate (1.31 g, 2.8 mmol, 1 eq) and LiOH (342 mg, 14 mmol, 5 eq) in 1:2 water/THF (60 mL) is stirred at room temperature for 18 h. The mixture is concentrated and diluted with 50 mL of water. The aqueous mixture is washed with 50 mL of diethyl ether and acidified to pH 4 with 2 N HCl. A precipitate is formed and filtered off. The solid is washed with water (3x5 mL) and dried in a vacuum oven at 40°C to afford the desired product.

*Illustrative example of method AR: final compound 5, 1-[[6-chloro-5-(cyclopropylmethoxy)-1H-benzimidazol-2-yl]methyl]-4-oxo-cinnoline-3-carboxylic acid*

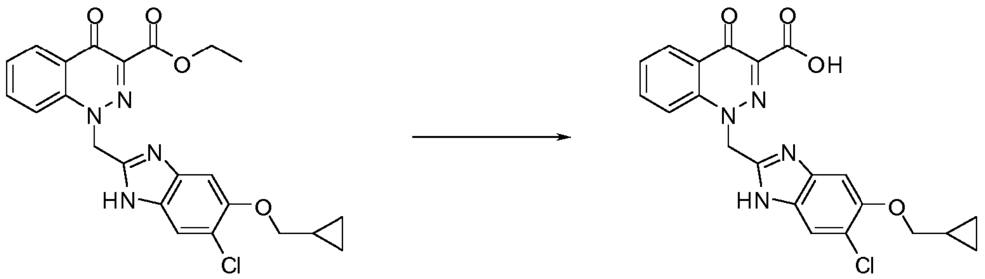

A mixture of ethyl 1-[[6-chloro-5-(cyclopropylmethoxy)-1H-benzimidazol-2-yl]methyl]-4-oxo-cinnoline-3-carboxylate (170 mg, 0.36 mmol, 1 eq) and LiOH.$H_2O$ (76 mg, 1.8 mmol, 5 eq) in 1:1 water/THF (5.6 mL) is stirred at room temperature for 2 h. The mixture is concentrated and diluted with 60 mL of water. The aqueous mixture is acidified to pH 2 with 1 N HCl. The mixture is extracted with 2:1 DCM/2-isopropanol (3x100 mL). The organic layer is concentrated and the residue is partitioned between 4:1 DCM/2-isopropanol and an aqueous solution at pH 10. The phases are separated and the aqueous layer is washed with DCM/2-isopropanol. The aqueous layer is acidified to pH 2 with 1 N HCl. A precipitate is formed and filtered off. The solid is purified by preparative HPLC to afford the desired product.

### *1.47. General method AS: acidic hydrolysis of tert-butyl esters*

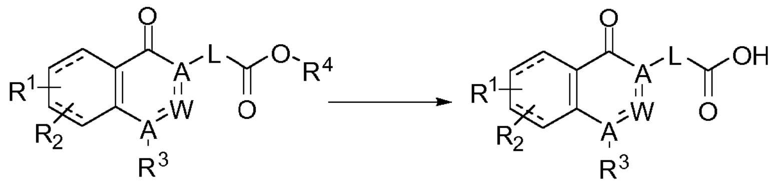

A = C, N
W = CH, N
$R^4$ = tert-Butyl

A mixture of the acid (1 eq) in 2:1 DCM/TFA is stirred typically for 3 h. The mixture is concentrated and the residue typically is triturated to afford the desired product. The product can be further purified by preparative HPLC.

*Illustrative example of method AS: final compound 6, 2-[4-[(5-chloro-6-ethoxy-1H-benzimidazol-2-yl)methyl]-1-oxo-phthalazin-2-yl]acetic acid*

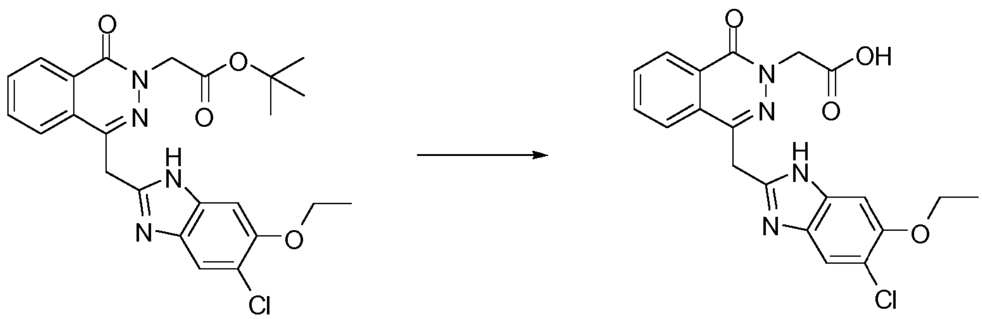

A mixture of tert-butyl 2-[4-[(6-chloro-5-ethoxy-1H-benzimidazol-2-yl)methyl]-1-oxo-phthalazin-2-yl]acetate (73 mg, 0.16 mmol, 1 eq) in 2:1 DCM/TFA (5 mL) is stirred at room temperature for 3 h. The mixture is concentrated and the residue is triturated with 5:1 diethyl ether/ethanol and diethyl ether to afford the desired product.

### *1.48. General method AT: amide coupling followed by formation of benzimidazole and hydrolysis of tert-butyl ester*

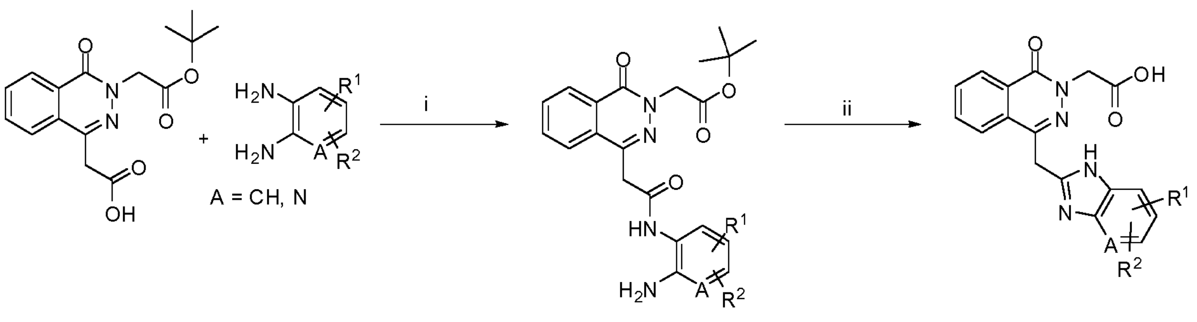


*Step i:*

The acid (1 eq), a base, typically diisopropylethylamine (2.5 eq) or triethylamine (3 eq) and a coupling agent, typically HATU (1.1 eq) or EDC.HCl/HOBt (1.5 and 0.15 eq) are mixed in an organic solvent, typically DMF or THF at 0°C. The bis-aniline (1 to 1.5 eq) is added and the mixture is stirred typically at room temperature for 2 to 16 h. The mixture is diluted with an organic solvent and the resulting mixture undergoes an aqueous work up. The mixture is concentrated to afford the desired intermediate, which may be further purified by flash column chromatography.

*Step ii:*

The amide from the previous step is stirred in acetic acid at 100 to 105°C for 16 h. The mixture is cooled and the desired product is typically isolated by precipitation and trituration.

*Illustrative example of method AT: final compound 7, 2-[4-[[6-cyano-5-(cyclopropylmethoxy)-1H-benzimidazol-2-yl]methyl]-1-oxo-phthalazin-2-yl]acetic acid*

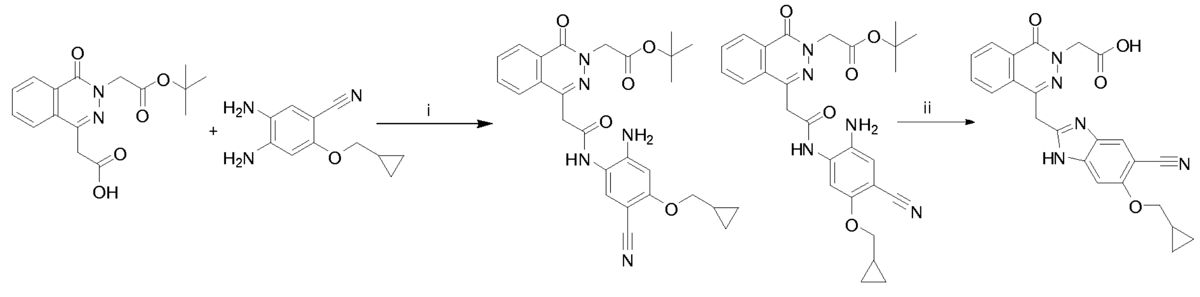


*Step i: tert-butyl 2-[4-[2-[2-amino-5-cyano-4-(cyclopropylmethoxy)anilino]-2-oxo-ethyl]-1-oxo-phthalazin-2-yl]acetate & tert-butyl 2-[4-[2-[2-amino-4-cyano-5-(cyclopropylmethoxy)anilino]-2-oxo-ethyl]-1-oxo-phthalazin-2-yl]acetate*

A mixture of 2-[3-(2-tert-butoxy-2-oxo-ethyl)-4-oxo-phthalazin-1-yl]acetic acid (500 mg, 1.57 mmol, 1 eq), EDC.HCl (452 mg, 2.36 mmol, 1.5 eq) and diisopropylethyl amine (0.67 mL, 3.9 mmol, 2.5 eq) in THF (5 mL) is stirred at 0°C for 15 min. 4,5-diamino-2-(cyclopropyl methoxy)benzonitrile is added and the mixture is stirred at room temperature for 18 h. The mixture is diluted (ethyl acetate) and the organic mixture is washed (saturated $NH_4Cl$, brine and saturated $NaHCO_3$). During the work up, an emulsion is obtained. The emulsion is filtered off and the solid is collected to afford the desired product.

*Step ii: 2-[4-[[6-cyano-5-(cyclopropylmethoxy)-1H-benzimidazol-2-yl]methyl]-1-oxo-phthalazin-2-yl]acetic acid*

A mixture of tert-butyl 2-[4-[2-[2-amino-5-cyano-4-(cyclopropylmethoxy)anilino]-2-oxo-ethyl]-1-oxo-phthalazin-2-yl]acetate & tert-butyl 2-[4-[2-[2-amino-4-cyano-5-(cyclopropylmethoxy)anilino]-2-oxo-ethyl]-1-oxo-phthalazin-2-yl]acetate (655 mg, 1.3 mmol in total) in acetic acid (6 mL) is stirred at 105°C for 16 h. The mixture is cooled and the desired product is isolated by precipitation and trituration (diethyl ether).

*Illustrative compound -, 2-[4-[[6-cyano-5-(2,2-difluoroethoxy)indazol-2-yl]methyl]-1-oxo-phthalazin-2-yl]acetic acid*

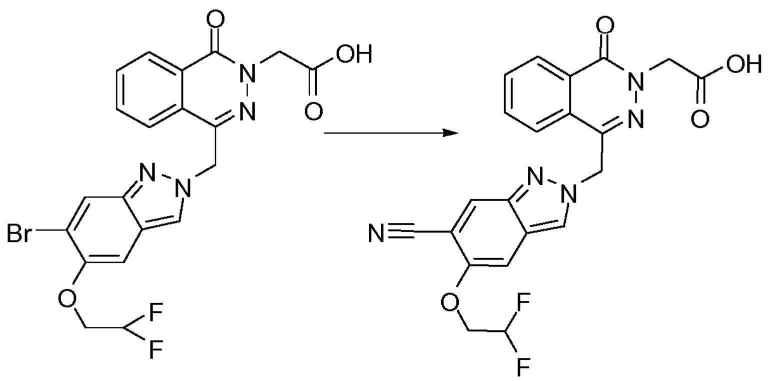

A mixture of 2-[4-[[6-bromo-5-(2,2-difluoroethoxy)indazol-2-yl]methyl]-1-oxo-phthalazin-2-yl]acetic acid (100 mg, 0.2 mmol, 1 eq), $Zn(CN)_2$ (24 mg, 0.2 mmol, 1 eq), $Zn(COOH)_2.2H_2O$ (0.3 mg, 0.002 mmol, 0.01 eq) and $Pd(dppf)Cl_2.CH_2Cl_2$ (3.3 mg, 0.004 mmol, 0.02 eq) in DMA (1 mL) is stirred at 150°C for 15 min in microwave conditions. The mixture is diluted with ethyl acetate and the resulting mixture is filtered over celite. Further purification with preparative HPLC yields the desired product.

*Illustrative compound 9, 2-[4-[[5-chloro-6-(2,2-difluoroethoxy)-1H-indol-2-yl]methyl]-1-oxo-phthalazin-2-yl]acetic acid*

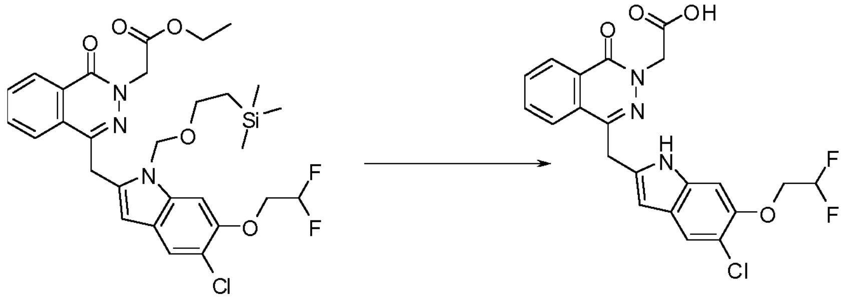

0.76 mL of TBAF (1 M in THF) are concentrated to dryness to yield solid TBAF (0.76 mmol, 3 eq). A solution of ethyl 2-[4-[[5-chloro-6-(2,2-difluoroethoxy)-1-(2-trimethylsilylethoxymethyl)indol-2-yl]methyl]-1-oxo-phthalazin-2-yl]acetate (147 mg, 0.24 mmol, 1 eq) in degassed dry DMF (2 mL) is added under argon to the solid TBAF followed by degassed ethylenediamine (0.10 mL, 1.5 mmol, 6.4 eq). The resulting mixture is stirred at 80°C for 3.5 h. The mixture is diluted with ethyl acetate (50 mL) and the organic solution is washed (water and brine), dried ($Na_2SO_4$) and concentrated. The residue is filtered through an ion exchange resin (SCX) with methanol. The fractions are concentrated and the residue is purified by flash column chromatography ($SiO_2$, DCM/9:1:0.03 DCM/methanol/formic acid 100:0 to 50:50) to afford the desired product.

### *1.49. General method AU: amide coupling*

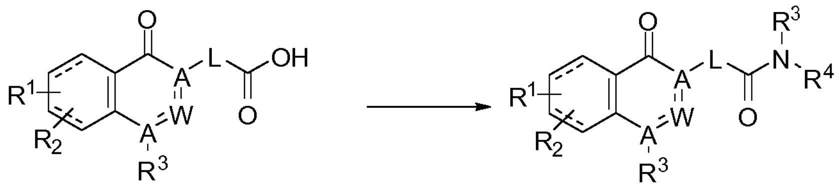

A = C, N
W = CH, N

The acid (1 eq), a base, typically diisopropylethylamine (2.5 eq) or triethylamine (3 eq) and a coupling agent, typically HATU (1.1 eq) or EDC.HCl/HOBt (1.5 and 0.15 eq) are mixed in an organic solvent, typically DMF or THF at 0°C. The bis-aniline (1 to 1.5 eq) is added and the mixture is stirred typically at room temperature for 0.5 to 16 h. The mixture is diluted with an organic solvent and the resulting mixture undergoes an aqueous work up. The mixture is concentrated to afford the desired product, which may be further purified by flash column chromatography or preparative HPLC.

*Illustrative example of method AU: Illustrative compound 10, 6-(cyclopropylmethoxy)-2-[[3-[2-[(3S,4S)-3,4-dihydroxypyrrolidin-1-yl]-2-oxo-ethyl]-4-oxo-cinnolin-1-yl]methyl]-3H-benzimidazole-5-carbonitrile*

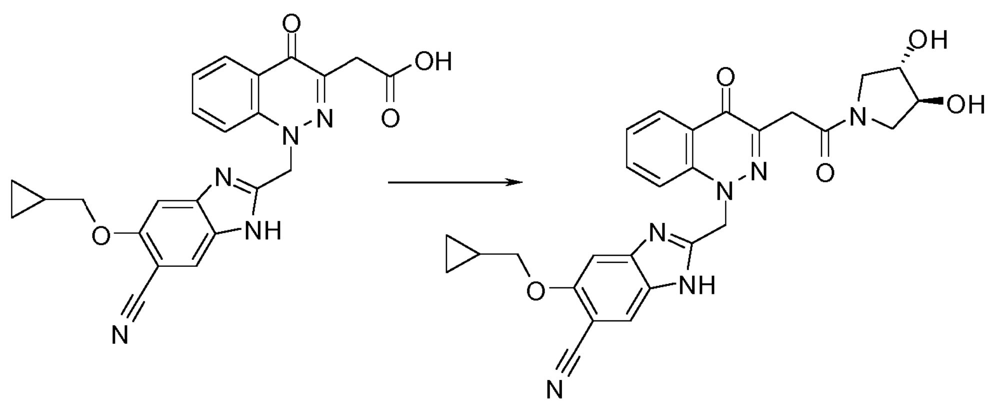

A mixture of 2-[1-[[6-cyano-5-(cyclopropylmethoxy)-1H-benzimidazol-2-yl]methyl]-4-oxo-cinnolin-3-yl]acetic acid (201 mg, 0.47 mmol, 1 eq), (3R,4R)-pyrrolidine-3,4-diol (59 mg, 0.56 mmol, 1.2 eq), diisopropylethylamine (0.183 mL, 1.0 mmol, 2.2 eq) and HATU (196 mg, 0.52 mmol, 1.1 eq) in DMF (4.7 mL) is stirred at room temperature for 30 min. The mixture is diluted (ethyl acetate). The organic layer is washed (water and 5% aq LiCl), dried ($Na_2SO_4$) and concentrated. The residue is purified by flash column chromatography ($SiO_2$, DCM/methanol 99.9:0.1 to 99:1) to obtain the desired product.

*Illustrative compound 11, 4-[[5-chloro-6-(2,2-difluoroethoxy)-1H-indol-2-yl]methyl]-2-(2-morpholino-2-oxo-ethyl)phthalazin-1-one*

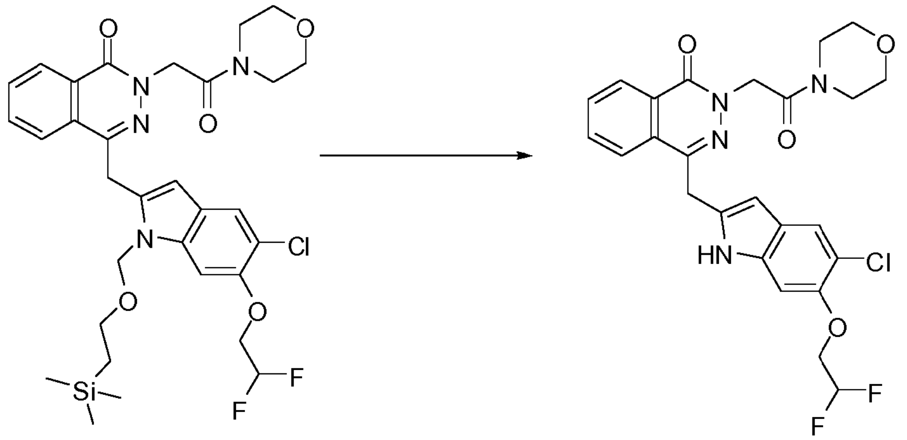

TBAF (1 M in THF, 0.26 m) is concentrated to dryness to yield solid TBAF (0.26mmol, 3 eq). A solution of 4-[[5-chloro-6-(2,2-difluoroethoxy)-1-(2-trimethylsilylethoxymethyl)indol-2-yl]methyl]-2-(2-morpholino-2-oxo-ethyl)phthalazin-1-one (56 mg, 0.09 mmol, 1 eq) in dry DMF (0.7 mL) is added to the solid TBAF followed by ethylenediamine (0.04 mL, 0.58 mmol, 6.4 eq). The resulting mixture is stirred at 80°C for 1.5 h. The mixture is diluted with ethyl acetate (50 mL) and the organic solution is washed (water and brine), dried ($Na_2SO_4$) and concentrated. The residue is purified by flash column chromatography ($SiO_2$, cyclohexane/9:1:0.1 ethyl acetate/cyclohexane/DEA 90:10 to 0:100) to afford the desired product.

*Illustrative compound 12, 4-[[6-chloro-5-(2,2-difluoroethoxy)-1H-benzimidazol-2-yl]methyl]-2-(2-morpholino-2-oxo-ethyl)-1-oxo-phthalazine-6-carboxamide*

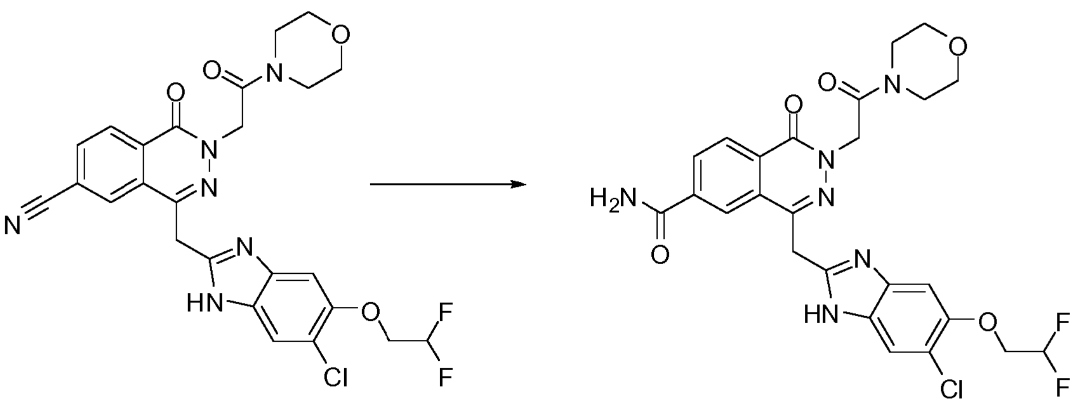

A mixture of 4-[[6-chloro-5-(2,2-difluoroethoxy)-1H-benzimidazol-2-yl]methyl]-2-(2-morpholino-2-oxo-ethyl)-1-oxo-phthalazine-6-carbonitrile (60 mg, 0.19 mmol, 1 eq) and KOtBu (63 mg, 0.57 mmol, 3 eq) in toluene (1.5 mL) and tert-butanol (2 mL) is stirred at room temperature for 16 h. The mixture is quenched with saturated $NH_4Cl$ and extracted (ethyl acetate). The organic layer is dried ($Na_2SO_4$) and concentrated. The residue is purified by preparative HPLC.

### *1.50. General method AV: amide coupling by flow chemistry*

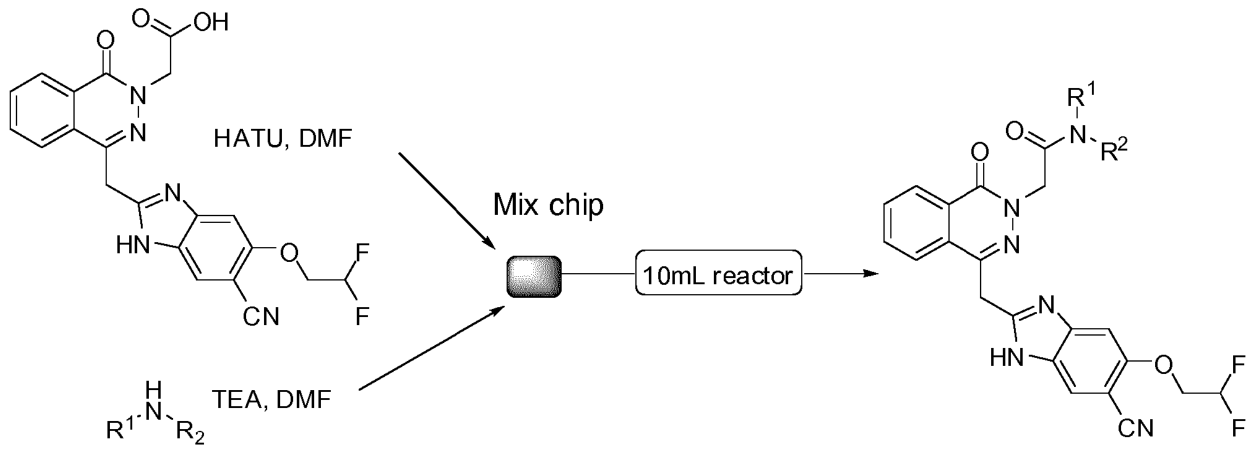


The Vapourtec R2+/R4 platform is used for the synthesis of the amide. One solution of carboxylic acid (68.3 mM) and HATU (109.3 mM) in DMF is placed in a container (bottle A). A series of solutions containing the amines (2.0 eq for each solution) and DIPEA (2.0 eq for salt free amines and 4.0 eq in case the amine is in the form of an HCl salt) in DMF is injected through the reagent sample loop B (1.1 mL). A bottle of DMF is connected to both pumps A and B and the flow rate is fixed at 2.04 mL/min (1.02 mL/min + 1.02 mL/min). After switching, the bottle reagent A (1.0 mL) and the sample loop, the solutions exited are mixed with a mixing chip (0.2 mL), entered in a PTFE coil reactor (10 mL) warmed at 50°C, fitted with the back pressure regulator (15 bar) and the output is recovered in a fraction collector. Products are purified by suitable preparative HPLC methods.

*Illustrative example of method AV: final compound 13, 6-(2,2-difluoroethoxy)-2-[[3-[2-[(3S)-3-hydroxypyrrolidin-1-yl]-2-oxo-ethyl]-4-oxo-phthalazin-1-yl]methyl]-3H-benzimidazole-5-carbonitrile*

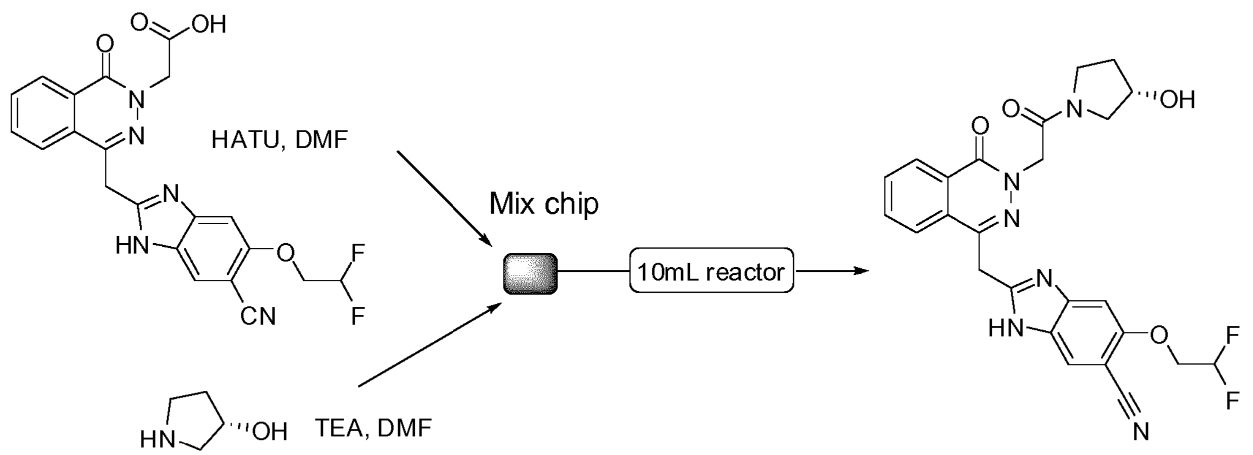


Reagent bottle A: a solution of 2-[4-[[6-cyano-5-(2,2-difluoroethoxy)-1H-benzimidazol-2-yl]methyl]-1-oxo-phthalazin-2-yl]acetic acid (30 mg; 1.0 equiv) and HATU (41.5 mg; 1.6 equiv) in DMF (1.0 mL). Reagent stock solution B : (3S)-pyrrolidin-3-ol (17.0 μL; 2.0 equiv) and DIPEA (35.7 μL; 2.0 equiv) in DMF (1.5 mL). Feeds A and B (1 mL each) are injected simultaneously into the mixing chip

(0.2mL) and passed through PTFE coil reactor (10 mL) at a flow rate of 2.04 mL/min (1.02 mL/min + 1.02 mL/min), warmed at 50°C, fitted with the back pressure regulator (15 bar). The reaction mixture is collected from the output. Product is purified by suitable preparative UPLC methods.

*Final compound 14, 2-[(1-acetylpyrrolidin-2-yl)methyl]-4-[[5-chloro-6-(trifluoromethoxy)-1H-benzimidazol-2-yl]methyl]phthalazin-1-one*

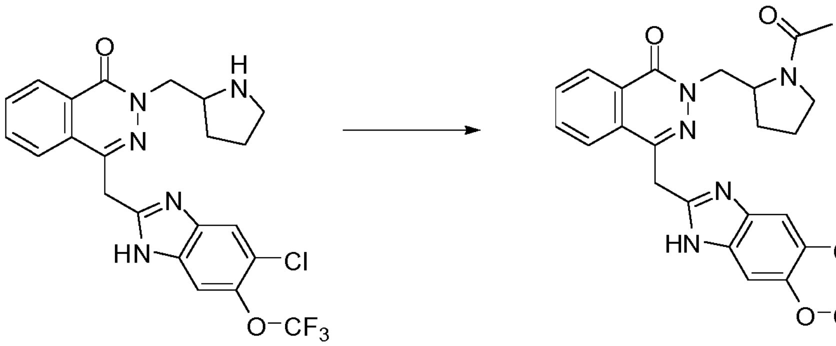

AcCl (14 μl, 192.7 μmol, 1.01 eq) is added under nitrogen to a solution of 4-[[6-chloro-5-(trifluoromethoxy)-1H-benzimidazol-2-yl]methyl]-2-(pyrrolidin-2-ylmethyl)phthalazin-1-one (91 mg, 191.0 μmol, 1 eq) and triethylamine (66 μl, 477 μmol, 2.5 eq) in DCM (1 mL) at 0°C. The reaction is stirred at 0°C for 1 h. The mixture is quenched with saturated $NH_4Cl$. The mixture is extracted with ethyl acetate. The organic layer is dried ($MgSO_4$) and concentrated. The residue is purified by preparative HPLC.

### 1.51. General method AW: formation of acyl sulfonamides

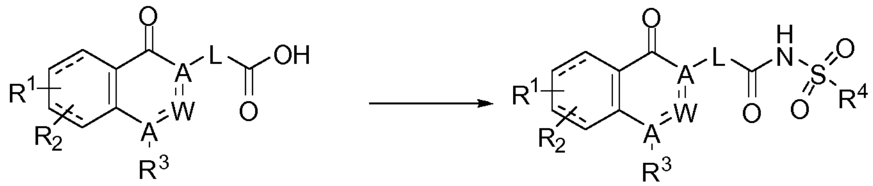

A = C, N
W = CH, N

A mixture of the acid (1 eq) and activating agent such as CDI (1.1 eq) in an organic solvent is stirred at room temperature for 90 to 120 min. The primary sulphonamide (1.1 eq) is added to the mixture followed by DBU (1.1 eq). Alternatively EDC and DMAP are used as activating agents and the primary sulphonamide (1 eq) is added after 10 min. The mixture is stirred at room temperature for 16 h. The reaction is diluted with an organic solvent and undergoes an aqueous work up. The organic layer is dried and concentrated to afford the desired product, which can be further purified by trituration, preparative HPLC or flash column chromatography.

*Illustrative example of method AW: Illustrative compound 15, 2-[1-[[7-chloro-6-(cyclopropylmethoxy)imidazo[1,2-a]pyridin-2-yl]methyl]-4-oxo-cinnolin-3-yl]-N-methylsulfonyl-propanamide*

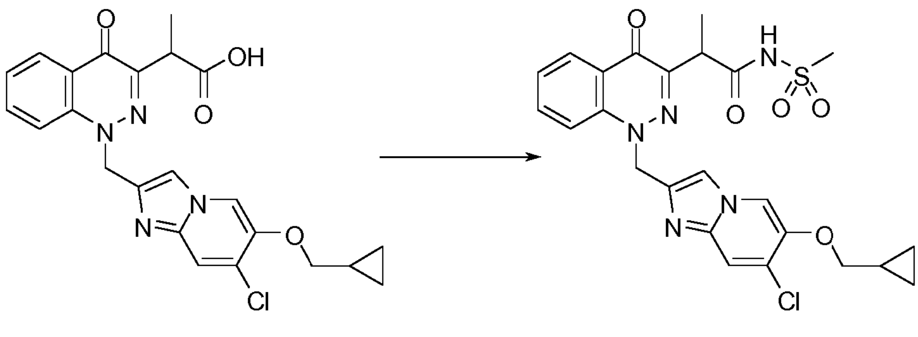

A mixture of 2-[1-[[7-chloro-6-(cyclopropylmethoxy)imidazo[1,2-a]pyridin-2-yl]methyl]-4-oxo-cinnolin-3-yl]propanoic acid (44 mg, 0.1 mmol, 1 eq) and CDI (18 mg, 0.11 mmol, 1.1 eq) in DCM (0.28 mL) is stirred at room temperature for 90 min. Methanesulfonamide (10 mg, 0.11 mmol, 1.1 eq) and DBU (0.016 mL, 0.11 mmol, 1.1 eq) are added and the resulting mixture is stirred at room temperature for 16 h. The mixture is diluted (DCM). The resulting mixture is washed (0.5 N HCl, $NaH_2PO_4$ and water), dried ($Na_2SO_4$) and concentrated. The residue is purified by flash column chromatography ($SiO_2$, DCM/methanol 100:0 to 95:5) to afford the desired product.

***Illustrative compound 16, 2-[1-[[5-chloro-6-(cyclopropylmethoxy)-1H-indol-2-yl]methyl]-4-oxo-cinnolin-3-yl]acetic acid***

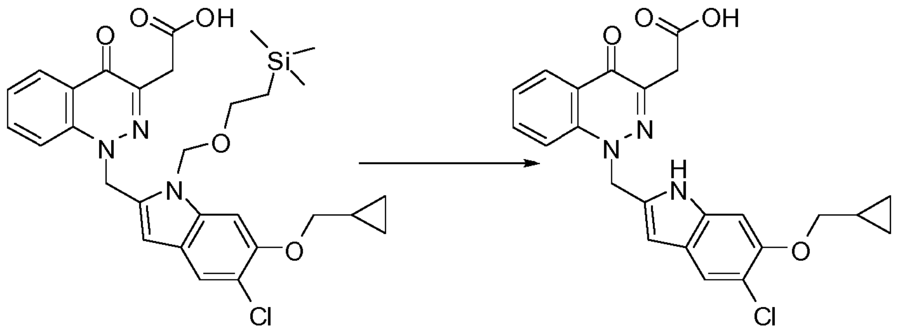

TBAF (1 M in THF, 0.39 mL) is concentrated to dryness to yield solid TBAF (0.31 mmol, 3 eq). A solution of 2-[1-[[5-chloro-6-(cyclopropylmethoxy)-1-(2-trimethylsilylethoxymethyl)indol-2-yl]methyl]-4-oxo-cinnolin-3-yl]acetic acid (74 mg, 0.13 mmol, 1 eq) in dry DMF (1.1 mL) is added to the solid TBAF followed by ethylenediamine (0.055 mL, 0.83 mmol, 6.4 eq). The resulting mixture is stirred at 80°C for 6 h. The mixture is diluted with ethyl acetate (50 mL) and the organic solution is washed (water and brine), dried ($Na_2SO_4$) and concentrated. The residue is filtered through an ion exchange resin (SCX) with methanol. The fractions are concentrated and the residue is purified by flash column chromatography ($SiO_2$, DCM/methanol 100:0 to 80:20) to afford the desired product.

*Illustrative compound 17, ((2R)-2-[4-[[6-chloro-5-(cyclopropylmethoxy)indazol-2-yl]methyl]-1-oxo-phthalazin-2-yl]propanoic acid) and 18, ((2S)-2-[4-[[6-chloro-5-(cyclopropylmethoxy)indazol-2-yl]methyl]-1-oxo-phthalazin-2-yl]propanoic acid)*

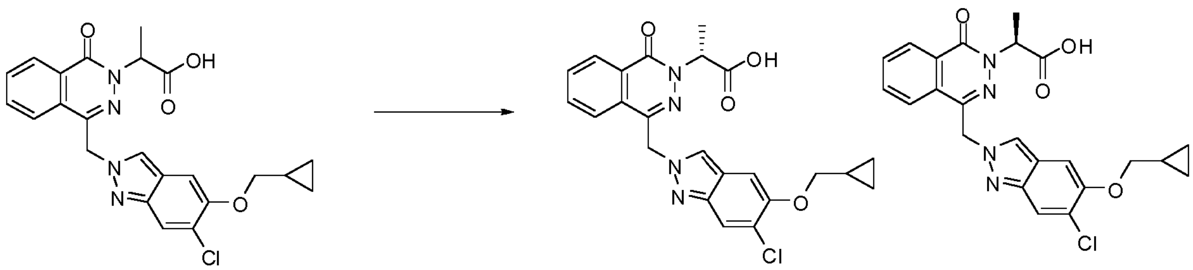

**17** **18**

2-[4-[[6-chloro-5-(cyclopropylmethoxy)indazol-2-yl]methyl]-1-oxo-phthalazin-2-yl]propanoic acid (10.47 g) undergoes a chiral separation (column: CHIRALPAK® IC 5 μm - 250 x 50 mm, mobile phase: Carbon Dioxide/Methanol 60/40) to afford ((2R)-2-[4-[[6-chloro-5-(cyclopropylmethoxy)indazol-2-yl]methyl]-1-oxo-phthalazin-2-yl]propanoic acid) and ((2S)-2-[4-[[6-chloro-5-(cyclopropylmethoxy) indazol-2-yl]methyl]-1-oxo-phthalazin-2-yl]propanoic acid).

*Alternative synthesis of compound 17 ((2R)-2-[4-[[6-chloro-5-(cyclopropylmethoxy)indazol-2-yl]methyl]-1-oxo-phthalazin-2-yl]propanoic acid)*

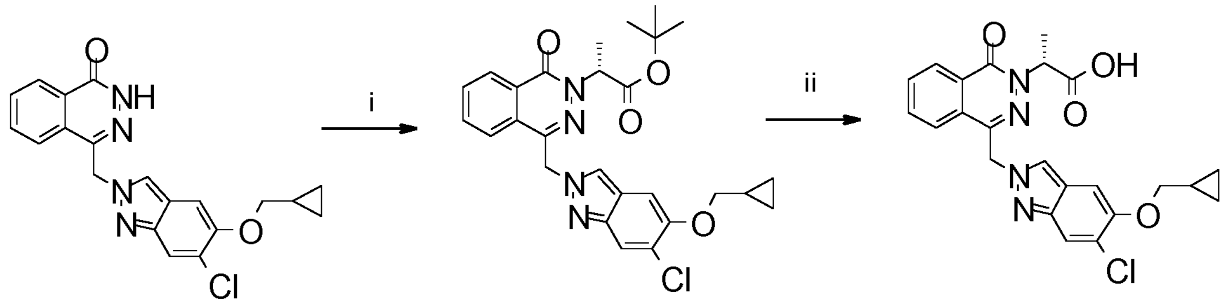


*Step i: tert-butyl (2R)-2-[4-[[6-chloro-5-(cyclopropylmethoxy)indazol-2-yl]methyl]-1-oxophthalazin-2-yl]propanoate*

tert-butyl (2S)-2-(4-methylphenyl)sulfonyloxypropanoate (1.1 eq, 133g, 0.442 moles) is added to a suspension of 4-[[6-chloro-5-(cyclopropylmethoxy)indazol-2-yl]methyl]-2H-phthalazin-1-one (1.0 eq, 153g) and potassium carbonate (2.0 eq, 111g, 0.803 moles) in nBuOAc (1530mL). The heterogeneous reaction mixture is refluxed at 126°C for 7h. The reaction mixture is cooled down to 20°C and the suspension is filtered on Celite (200g). The cake is washed with EtOAc (300mL). The filtrate is washed (in a separatory funnel) with water (760mL) and the organic phase is concentrated to a weight of around 300g. To the resulting solution is slowly added (1h30 of addition) heptane (800mL) to give a suspension which is stirred at 40°C for 15minutes and at 20°C for 30 minutes. The suspension is finally filtered and the solid is washed with heptane (100mL) to afford a first crop of the desired product. The filtrate is concentrated to dryness and dissolved in MTBE (50mL). Heptane (100mL) is slowly added and the resulting suspension is stirred at room temperature for 30 minutes.The suspension is filtered to afford a second crop of the desired product.

*Step ii: ((2R)-2-[4-[[6-chloro-5-(cyclopropylmethoxy)indazol-2-yl]methyl]-1-oxo-phthalazin-2-yl] propanoic acid)*

tert-butyl (2R)-2-[4-[[6-chloro-5-(cyclopropylmethoxy)indazol-2-yl]methyl]-1-oxophthalazin-2-yl]propanoate (646g, 1.0 eq) is suspended in ACN (3000mL). HCl 37% (3.5 eq, 370mL, 4.44 moles) is added to the reaction mixture. The reaction mixture is heated at 47-50°C for about 2h. The reaction mixture is cooled down to 20°C and NaOH 2M (2.5 eq, 1600mL) is added to the reaction mixture. The aqueous phase is removed and the organic phase is stirred at 22-23°C for 2h. The suspension is filtered on a sintered glass funnel and the solid is washed with ACN (400mL then 200mL). The solid is dried under vacuum at 40°C to afford the desired product.

*Final compound 19, ((2S)-2-[1-[[7-chloro-6-(cyclopropylmethoxy)imidazo[1,2-a]pyridin-2-yl]methyl]-4-oxocinnolin-3-yl]propanoic acid) and 20, ((2R)-2-[1-[[7-chloro-6-(cyclopropylmethoxy)imidazo[1,2-a]pyridin-2-yl]methyl]-4-oxocinnolin-3-yl]propanoic acid)*

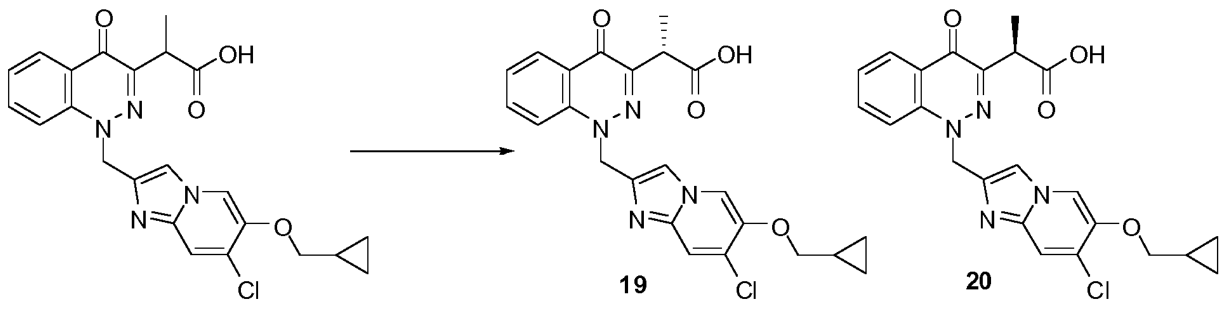

19 20

2-[1-[[7-chloro-6-(cyclopropylmethoxy)imidazo[1,2-a]pyridin-2-yl]methyl]-4-oxo-cinnolin-3-yl]propanoic acid (586 mg) undergoes a chiral separation (column: CHIRALPAK® IC 5 μm - 250 x 30 mm, mobile phase: n-Heptane/DCM/Isopropanol/Formic Acid 35/43/22/0.1) to afford ((2S)-2-[1-[[7-chloro-6-(cyclopropylmethoxy)imidazo[1,2-a]pyridin-2-yl]methyl]-4-oxocinnolin-3-yl]propanoic acid) and ((2R)-2-[1-[[7-chloro-6-(cyclopropylmethoxy)imidazo[1,2-a]pyridin-2-yl]methyl]-4-oxocinnolin-3-yl]propanoic acid).

*Intermediate 108, tert-butyl 2-(4-oxo-1H-cinnolin-3-yl)acetate*

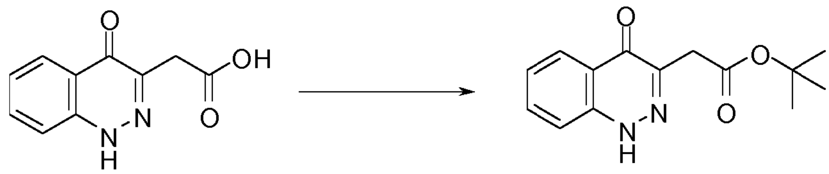

N,N-Dimethylformamide di-tert-butyl acetal (9.3 mL, 35 mmol, 4 eq) is added to a mixture of 2-(4-oxo-1H-cinnolin-3-yl)acetic acid (2.1 g, 8.74 mmol, 1 eq) in toluene (30 mL) at 85°C. The mixture is stirred at reflux for 1 h. The mixture is diluted with 9:1 ethyl acetate/THF and the resulting organic mixture is washed (saturated $NaHCO_3$ and brine), dried ($Na_2SO_4$) and concentrated. The residue is purified by flash column chromatography ($SiO_2$, DCM/ethyl acetate 97:3 to 65:35) to afford the desired product.

*Final compound 21, (2-[1-[[5-cyano-6-(cyclopropylmethoxy)-1H-benzimidazol-2-yl]methyl]-4-oxocinnolin-3-yl]-3-(dimethylamino)propanoic acid)*

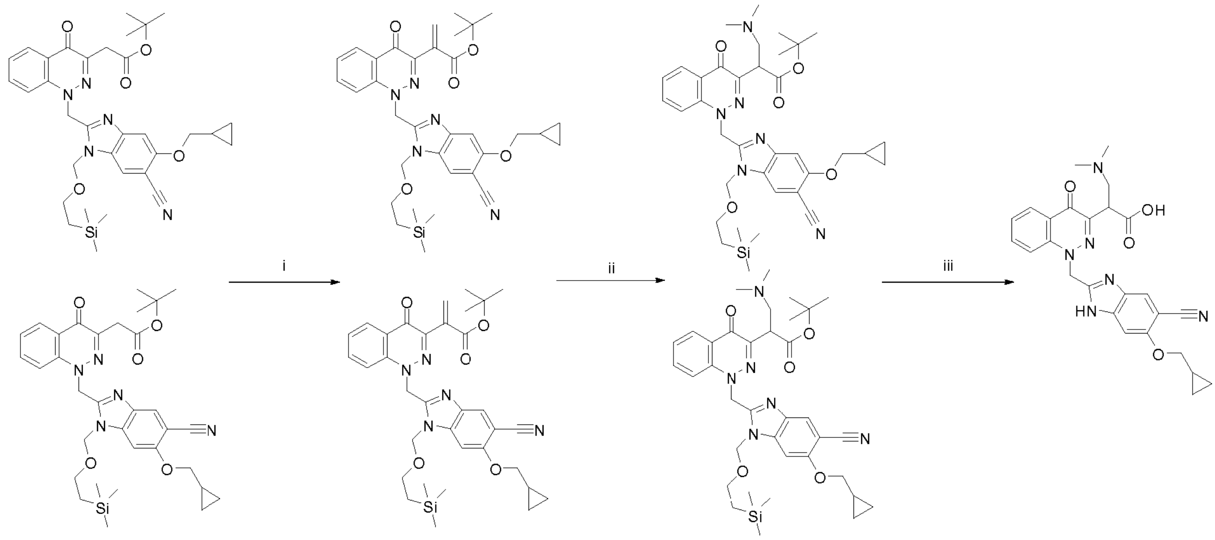


*Step i: tert-butyl 2-[1-[[6-cyano-5-(cyclopropylmethoxy)-1-(2-trimethylsilylethoxymethyl)benzimidazol-2-yl]methyl]-4-oxo-cinnolin-3-yl]prop-2-enoate & tert-butyl 2-[1-[[5-cyano-6-(cyclopropylmethoxy)-1-(2-trimethylsilylethoxymethyl)benzimidazol-2-yl]methyl]-4-oxo-cinnolin-3-yl]prop-2-enoate*

NaH (60% mineral oil, 73 mg, 1.83 mmol, 2.5 eq) is added to a mixture of tert-butyl 2-[1-[[6-cyano-5-(cyclopropylmethoxy)-1-(2-trimethylsilylethoxymethyl)benzimidazol-2-yl]methyl]-4-oxo-cinnolin-3-yl] acetate & tert-butyl 2-[1-[[5-cyano-6-(cyclopropylmethoxy)-1-(2-trimethylsilylethoxymethyl)benzimidazol-2-yl]methyl]-4-oxo-cinnolin-3-yl]acetate (468 mg, 0.73 mmol in total, 1 eq) and paraformaldehyde (692 mg, 21.9 mmol, 30 eq) in dry 1,4-dioxane (4.5 mL). The mixture is stirred at 45°C for 16 h. The mixture is diluted with methanol and concentrated. The residue is taken up in DCM. The organic mixture is washed (0.1 N HCl), dried (phase separator) and concentrated. The residue is purified by flash column chromatography ($SiO_2$, DCM/ethyl acetate 100:0 to 80:20) to afford the desired product.

*Step ii: tert-butyl 2-[1-[[6-cyano-5-(cyclopropylmethoxy)-1-(2-trimethylsilylethoxymethyl)benzimidazol-2-yl]methyl]-4-oxo-cinnolin-3-yl]-3-(dimethylamino)propanoate & tert-butyl 2-[1-[[5-cyano-6-(cyclopropylmethoxy)-1-(2-trimethylsilylethoxymethyl)benzimidazol-2-yl]methyl]-4-oxo-cinnolin-3-yl]-3-(dimethylamino)propanoate*

A mixture of tert-butyl 2-[1-[[6-cyano-5-(cyclopropylmethoxy)-1-(2-trimethylsilylethoxymethyl)benzimidazol-2-yl]methyl]-4-oxo-cinnolin-3-yl]prop-2-enoate & tert-butyl 2-[1-[[5-cyano-6-(cyclopropylmethoxy)-1-(2-trimethylsilylethoxymethyl)benzimidazol-2-yl]methyl]-4-oxo-cinnolin-3-yl]prop-2-enoate (75 mg, 0.12 mmol in total, 1 eq), dimethylamine HCl salt (10 mg, 0.12 mmol, 1 eq) and triethylamine (0.023 mL, 0.18 mmol, 1.5 eq) in THF (1.2 mL) is stirred at room temperature for 72 h. The mixture is diluted with water and extracted with ethyl acetate. The organic layer is

concentrated to afford the desired product.

*Step iii: (2-[1-[[5-cyano-6-(cyclopropylmethoxy)-1H-benzimidazol-2-yl]methyl]-4-oxocinnolin-3-yl]-3-(dimethylamino)propanoic acid)*

A mixture of tert-butyl 2-[1-[[6-cyano-5-(cyclopropylmethoxy)-1-(2-trimethylsilylethoxymethyl)benzimidazol-2-yl]methyl]-4-oxo-cinnolin-3-yl]-3-(dimethylamino)propanoate & tert-butyl 2-[1-[[5-cyano-6-(cyclopropylmethoxy)-1-(2-trimethylsilylethoxymethyl)benzimidazol-2-yl]methyl]-4-oxo-cinnolin-3-yl]-3-(dimethylamino)propanoate (81 mg, 0.12 mmol in total, 1 eq) in 3:2 DCM/TFA (0.6 mL) is stirred at 40°C for 4 h. The reaction mixture is concentrated using toluene to form an azeotrope and the residue is purified by preparative HPLC to afford the desired product.

*Final compound 22, 2-[4-[(7-chloro-6-ethoxy-indazol-2-yl)methyl]-1-oxo-phthalazin-2-yl]acetic acid*

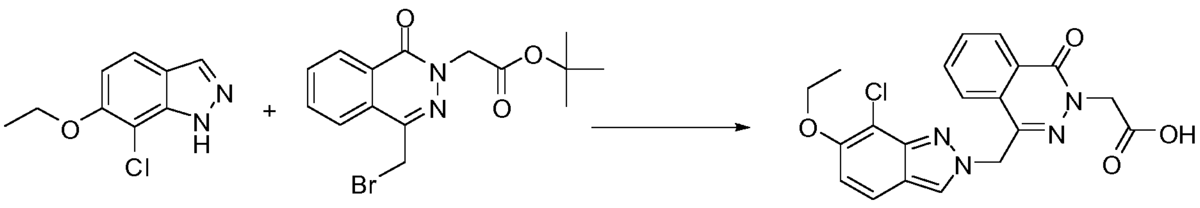

7-chloro-6-ethoxy-1H-indazole (50 mg, 0.22 mmol, 1.0 eq) and tert-butyl 2-[4-(bromomethyl)-1-oxo-phthalazin-2-yl]acetate (77 mg, 0.22 mmol, 1.0 eq) and sodium bicarbonate (18.2 mg, 0.22 mmol, 1.0 eq) are added to a 2 mL vial. Dioxane (1 mL) and a few drops of water are added; the vial is capped and heated to 150°C in the microwave for 1h. The volatiles are evaporated. The residue is redissolved in 5% aq $NaHCO_3$ and washed with MTBE. After acidification it is extracted with ethyl acetate (2x) and the combined ethyl acetate layers are dried over sodium sulphate, filtered and evaporated. The residue is dissolved in acetonitrile and given to preparative HPLC for purification.

*Intermediate 109, methyl 2-[1-[[5-chloro-6-(cyclopropylmethoxy)-1-(2-trimethylsilylethoxymethyl)indol-2-yl]methyl]-4-oxo-cinnolin-3-yl]acetate*

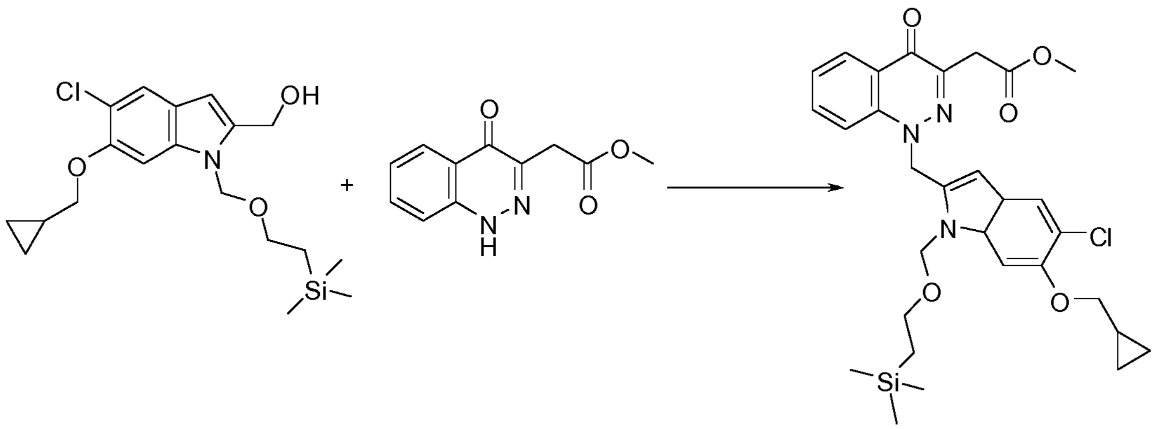

Diisopropyl azodicarboxylate (0.013 mL, 0.063 mmol, 1.2 eq) is added to a mixture of [5-chloro-6-(cyclopropylmethoxy)-1-(2-trimethylsilylethoxymethyl)indol-2-yl]methanol (20 mg, 0.052 mmol, 1 eq), methyl 2-(4-oxo-1H-cinnolin-3-yl)acetate (11.4 mg, 0.052 mmol, 1 eq) and $PPh_3$ (20.8 mg, 0.079 mmol, 1.5 eq) in dry THF (0.23 mL) at 0°C. The resulting mixture is stirred at room temperature for 3 h. $PPh_3$ (6.2 mg, 0.024 mmol) and Diisopropyl azodicarboxylate (3.8 μL, 0.019 mmol) are added to the mixture and the reaction is stirred for 17 h. The mixture is diluted with ethyl acetate, washed (saturated $NH_4Cl$, saturated $NaHCO_3$ and brine), dried ($Na_2SO_4$) and concentrated. The residue is purified by silica chromatography (DCM/ethyl acetate: 100/0 to 92/8) to afford the desired product.

*Final compound 23, 2-[4-[[5-chloro-6-(2,2-difluoroethoxy)-1H-benzimidazol-2-yl]methyl]-6-cyano-1-oxo-phthalazin-2-yl]acetic acid*

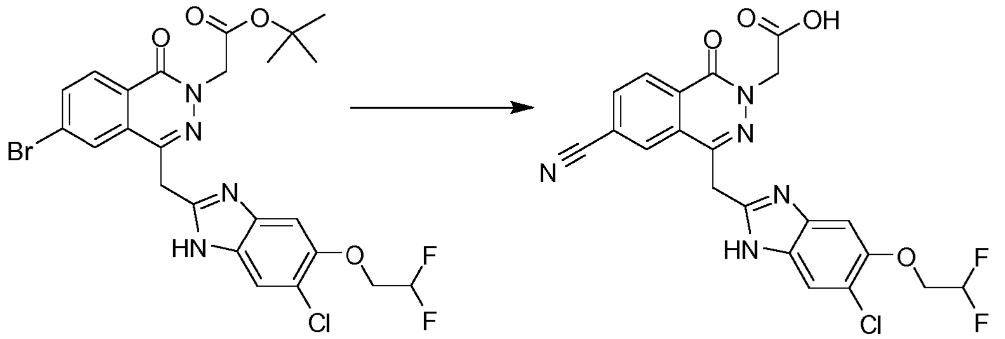

A mixture of 2 tert-butyl 2-[6-bromo-4-[[6-chloro-5-(2,2-difluoroethoxy)-1H-benzimidazol-2-yl]methyl]-1-oxo-phthalazin-2-yl]acetate (100 mg, 0.17 mmol, 1 eq), $Zn(CN)_2$ (24 mg, 0.17 mmol, 1 eq), $Zn(COOH)_2.2H_2O$ (3 mg, 0.02 mmol, 0.1 eq) and $Pd(dppf)Cl_2.CH_2Cl_2$ (16 mg, 0.02 mmol, 0.1 eq) in DMA (1 mL) is stirred at 150°C for 10 min in microwave conditions. The mixture is diluted with ethyl acetate and the resulting mixture is filtered over celite. The filtrate is extracted with water and 5% $NH_4OH$ in water. The aqueous layer is acidified with citric acid to pH 4-5 and extracted with ethyl acetate. The organic layer is dried ($Na_2SO_4$) and concentrated. The residue is purified by flash column chromatography ($SiO_2$, DCM/acetic acid/methanol 98:0.2/2 to 90:0.2:10) to obtain the desired product.

*Intermediate 110, tert-butyl 2-[4-[[6-cyano-5-(cyclopropylmethoxy)indazol-2-yl]methyl]-1-oxo-phthalazin-2-yl]acetate*

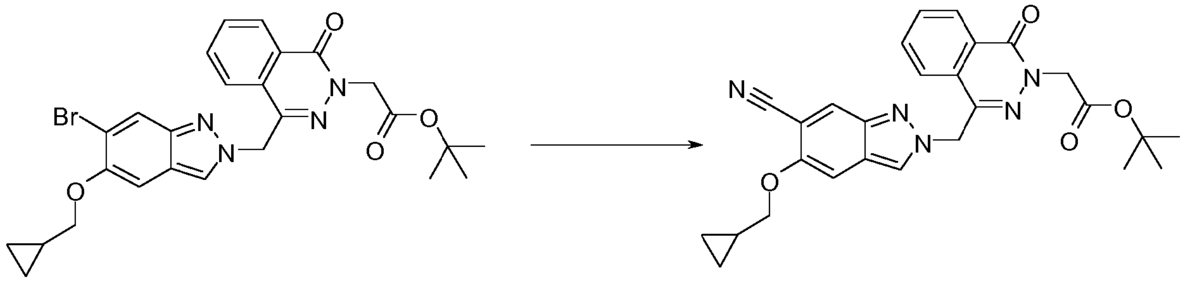

A mixture of tert-butyl 2-[4-[[6-bromo-5-(cyclopropylmethoxy)indazol-2-yl]methyl]-1-oxo-phthalazin-2-yl]acetate (250 mg, 0.46 mmol, 1 eq), $Zn(CN)_2$ (33.3 mg, 0.28 mmol, 0.6 eq), DPPF (21 mg, 0.037 mmol, 0.08 eq) and $Pd_2(dba)_3$ (17.3 mg, 0.02 mmol, 0.04 eq) in DMF (1 mL) is stirred at 135°C for 2 h. The mixture is diluted with ethyl acetate. The resulting mixture is washed (saturated $NaHCO_3$ and brine), dried ($Na_2SO_4$) and concentrated. The residue is purified by flash column chromatography ($SiO_2$, cyclohexane/ethyl acetate 100:0 to 60:40) to obtain the desired product.

*Intermediate 450: tert-butyl (2S)-2-(4-methylphenyl)sulfonyloxypropanoate*

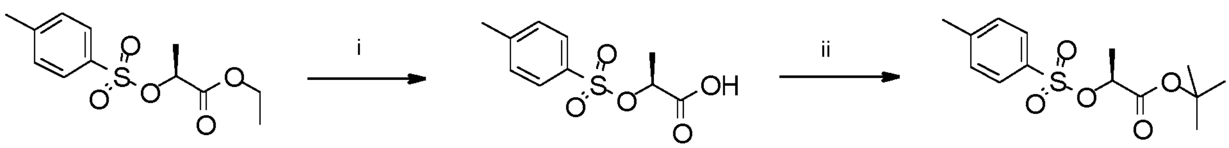


*Step i: (2S)-2-(4-methylphenyl)sulfonyloxypropanoic acid*

L-(-)-O-Tosyllactic acid ethyl ester (1.0 eq, 980g, 3.598 moles) is added to THF (980mL) and the resulting solution is cooled at 13°C. Under stirring, an aqueous solution of sodium hydroxide (NaOH 2M, 1.1 eq, 1.98L, 3.958 moles) is added over 1h20, keeping the reaction temperature below 20°C. The reaction mixture is stirred between 14-20°C until completion. The reaction mixture is cooled down to 10-12°C and an aqueous solution of HCl 2M (around

2L) is added in 30 minutes, until pH=1.5 is reached. The reaction mixture is extracted with MTBE (2L), washed once with an aqueous solution of NaCl (1L). The organic phase is concentrated to dryness and the resulting material is further dried to afford the expected product.

*Step ii: tert-butyl (2S)-2-(4-methylphenyl)sulfonyloxypropanoate*

In a 2L Schott Duran flask is added (2S)-2-(4-methylphenyl)sulfonyloxypropanoic acid (1.0 eq, 250g, 1.023 moles) in dichloromethane (DCM, 1L). The flask is cooled at -20°C and isobutene gas is condensed for 20-30 minutes. About 200g of isobutene are added (3.4 eq). Sulfuric acid (0.3 eq, 16mL) is then added.The flask is sealed and the reaction mixture is warmed to 20°C and stirred for 2h30. The solution is quenched by addition of NaOH 2M (1.25L) over 15-20 minutes. The reaction mixture is stirred vigorously at 20°C for 20-30 minutes. The organic phase is collected, washed with aqueous NaCl 20% w/w (500mL), and concentrated. The residue is dissolved in DCM and filtered on a small pad of Celite to afford, after removal of the solvents, the expected product.

**Table II. Intermediates towards illustrative compounds of the invention**

| Int # | Structure | Starting material | Mtd | MW (calc) | MW (obs) |
|---|---|---|---|---|---|
| 1 | 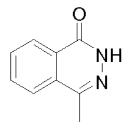 | 2-acetylbenzoic acid | NA | 160 | 161 |
| 2 | 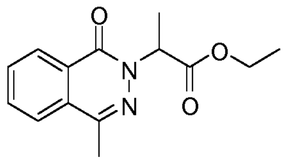 | Int.1 | A | 260 | 261 |
| 3 | 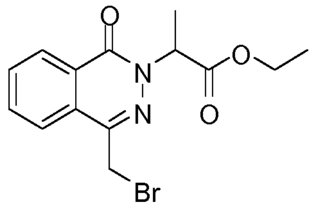 | Int.2 | B | 339 | 339 |
| 4 | 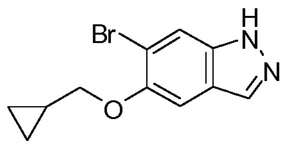 | 6-bromo-1H-indazol-5-ol | C | 267 | 267 |
| 5 | 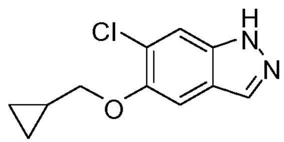 | 6-chloro-1H-indazol-5-ol or 5-methyl-2-chlorophenol | NA | 223 | 223 |

(continued)

| Int # | Structure | Starting material | Mtd | MW (calc) | MW (obs) |
|---|---|---|---|---|---|
| 6 | 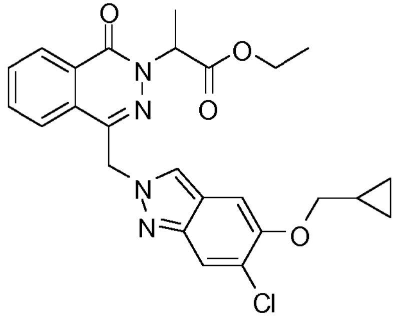 | Int.3 | D | 481 | 481 |
| 7 | 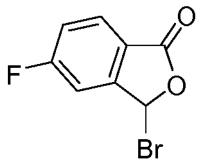 | 5-fluoro-3H-isobenzofuran-1-one | E | 231 | NA |
| 8 | 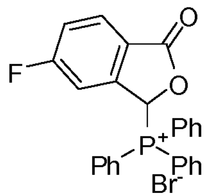 | Int.7 | F | 493 | 413 |
| 9 | 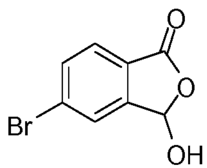 | Int.279 | NA | 229 | 229 |

(continued)

| Int # | Structure | Starting material | Mtd | MW (calc) | MW (obs) |
|---|---|---|---|---|---|
| 10 | 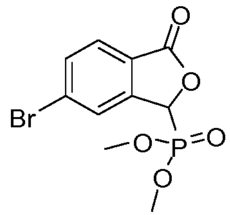 | Int.9 | NA | 321 | 321 |
| 11 | 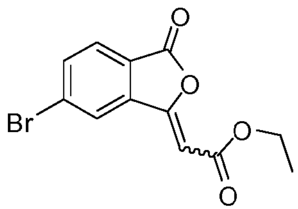 E/Z | Int.10 | NA | 297 | NA |
| 12 | 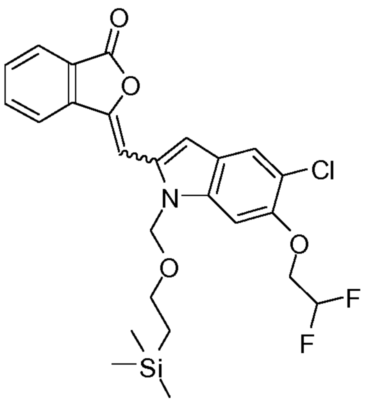 E/Z | Int.281 | G | 506 | 506 |

(continued)

| Int # | Structure | Starting material | Mtd | MW (calc) | MW (obs) |
|---|---|---|---|---|---|
| 13 | 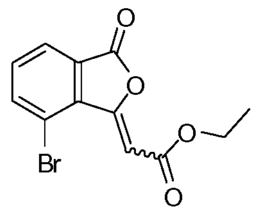 E/Z | Int.283 | NA | 297 | 299 |
| 14 | 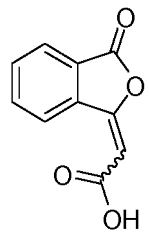 E/Z | isobenzofuran-1,3-dione | NA | 190 | 191 |
| 15 | 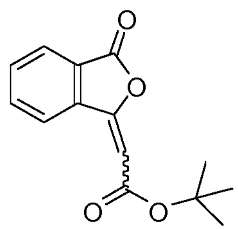 E/Z | isobenzofuran-1,3-dione | H | 246 | 247 |
| 16 | 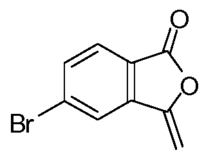 | Int.282 | NA | 225 | NA |

(continued)

| Int # | Structure | Starting material | Mtd | MW (calc) | MW (obs) |
|---|---|---|---|---|---|
| 17 | 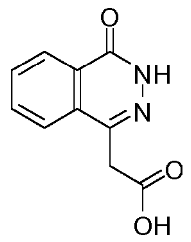 | Int.14 | I | 204 | 205 |
| 18 | 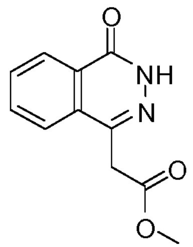 | Int.17 | NA | 218 | 219 |
| 19 | 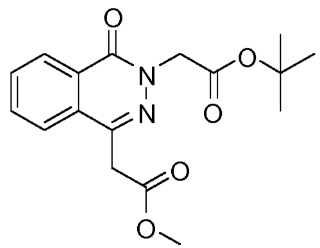 | Int.18 | J | 332 | 333 |

(continued)

| Int # | Structure | Starting material | Mtd | MW (calc) | MW (obs) |
|---|---|---|---|---|---|
| 20 | 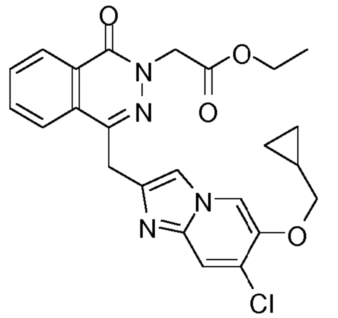 | Int.286 | K | 467 | 468 |
| 21 | 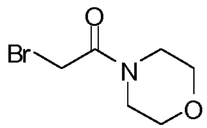 | 2-bromoacetyl bromide | NA | 208 | 208 |
| 22 | 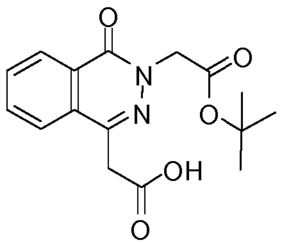 | Int.19 | L | 318 | 319 |
| 23 | 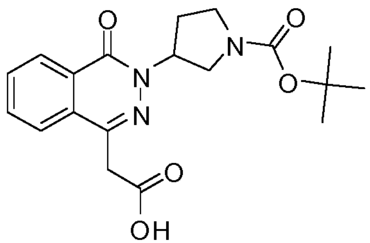 | Int.344 | M | 373 | 396 |

(continued)

| Int # | Structure | Starting material | Mtd | MW (calc) | MW (obs) |
|---|---|---|---|---|---|
| 24 | 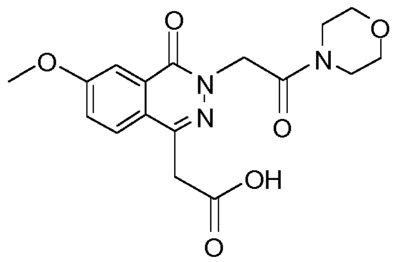 | Int.339 | NA | 361 | 362 |
| 25 | 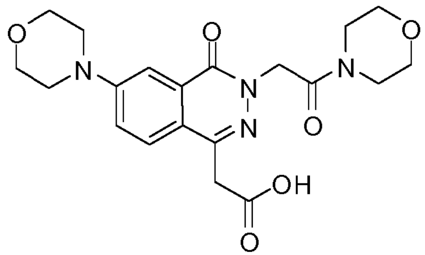 | Int.40 | N | 416 | 417 |
| 26 | 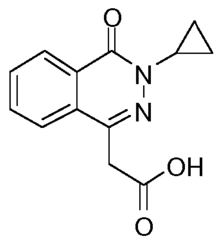 | Int.14 | O | 244 | 245 |
| 27 | 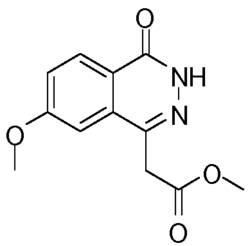 | Int.298 | NA | 248 | 249 |

(continued)

| Int # | Structure | Starting material | Mtd | MW (calc) | MW (obs) |
|---|---|---|---|---|---|
| 28 | 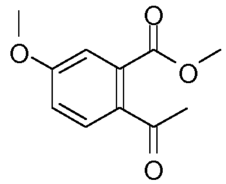 | methyl 2-bromo-5-methoxybenzoate | NA | 208 | 209 |
| 29 | 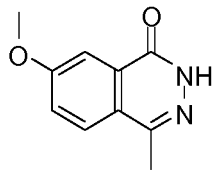 | Int.28 | NA | 190 | 191 |
| 30 | 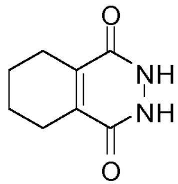 | 4,5,6,7-tetrahydroisob enzofuran-1,3-dione | NA | 166 | 167 |
| 31 | 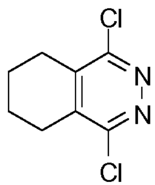 | Int.30 | NA | 203 | 203 |
| 32 | 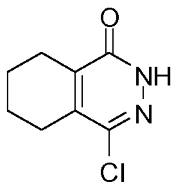 | Int.31 | NA | 185 | 185 |

(continued)

| Int # | Structure | Starting material | Mtd | MW (calc) | MW (obs) |
|---|---|---|---|---|---|
| 33 | 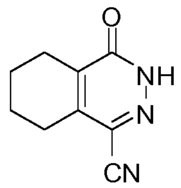 | Int.32 | NA | 175 | 176 |
| 34 | 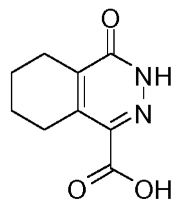 | Int.33 | NA | 194 | 195 |
| 35 | 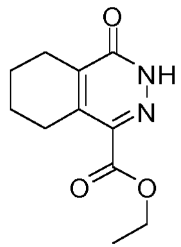 | Int.34 | NA | 222 | 223 |
| 36 | 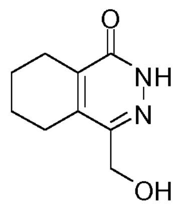 | Int.35 | NA | 180 | 181 |

(continued)

| Int # | Structure | Starting material | Mtd | MW (calc) | MW (obs) |
|---|---|---|---|---|---|
| 37 | 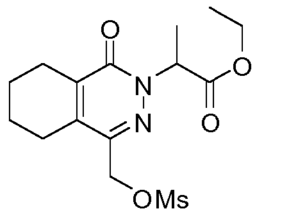 | Int.307 | NA | 358 | 359 |
| 38 | 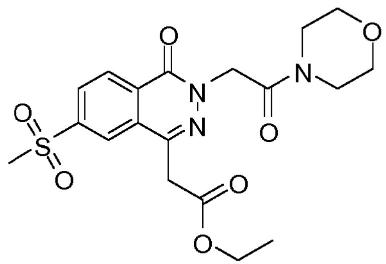 | Int.311 | NA | 437 | 438 |
| 39 | 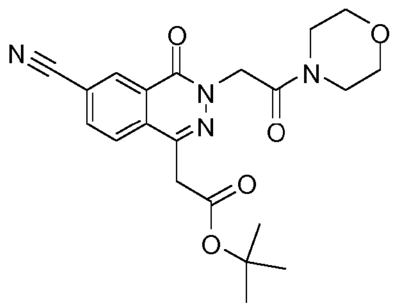 | Int.339 | NA | 412 | 413 |

(continued)

| Int # | Structure | Starting material | Mtd | MW (calc) | MW (obs) |
|---|---|---|---|---|---|
| 40 | 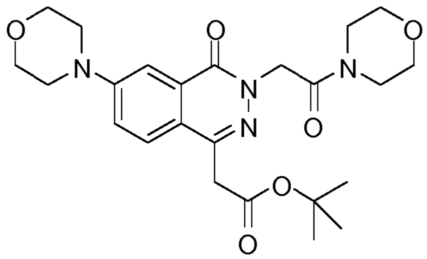 | Int.339 | NA | 473 | 473 |
| 41 | 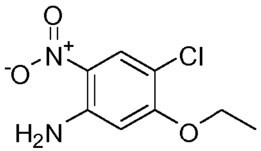 | 4-chloro-5-fluoro-2-nitroaniline | NA | 217 | 217 |
| 42 | 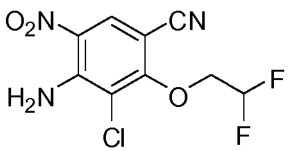 | Int. 441 | NA | 278 | 279 |
| 43 | 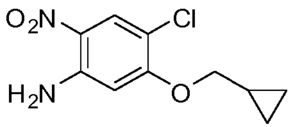 | 4-chloro-5-fluoro-2-nitroaniline | P | 243 | 243 |
| 44 | 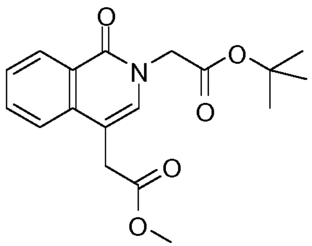 | Int.332 | NA | 331 | 354 [M+Na]+ |

(continued)

| Int # | Structure | Starting material | Mtd | MW (calc) | MW (obs) |
|---|---|---|---|---|---|
| 45 | 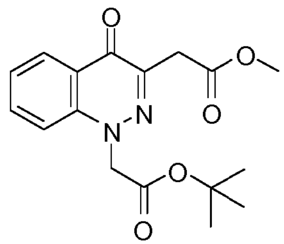 | Int.46 | NA | 332 | 333 |
| 46 | 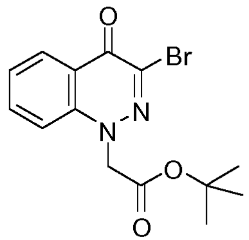 | 3-bromocinnolin -4-ol | NA | 339 | 285 |
| 47 | 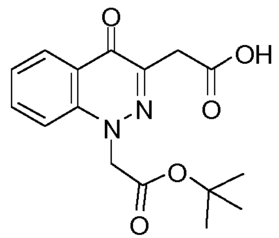 | Int.48 | NA | 318 | 319 |
| 48 | 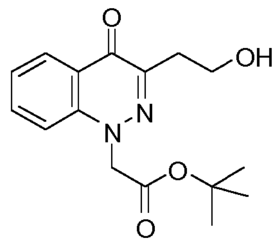 | Int.60 | NA | 304 | 305 |

(continued)

| Int # | Structure | Starting material | Mtd | MW (calc) | MW (obs) |
|---|---|---|---|---|---|
| 49 | 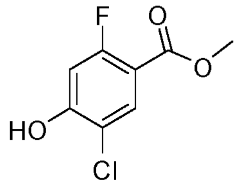 | methyl 2-fluoro-4-hydroxybenzoate | NA | 205 | 205 |
| 50 | 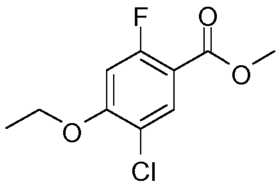 | Int.49 | NA | 233 | 233 |
| 51 | 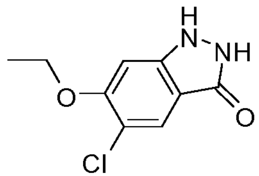 | Int.259 | NA | 213 | 213 |
| 52 | 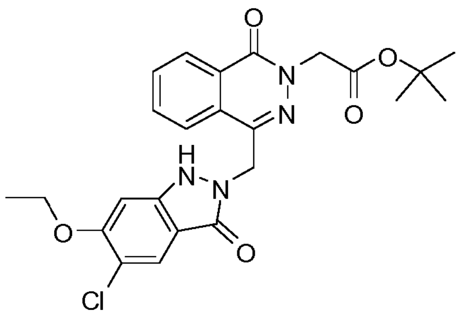 | Int.51 | NA | 485 | 485 |

(continued)

| Int # | Structure | Starting material | Mtd | MW (calc) | MW (obs) |
|---|---|---|---|---|---|
| 53 | 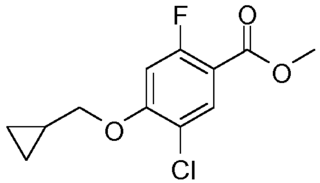 | Int.49 | NA | 259 | 259 |
| 54 | 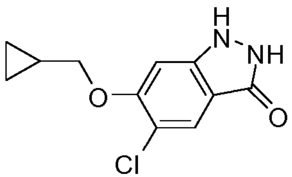 | Int.53 | NA | 239 | 239 |
| 55 | 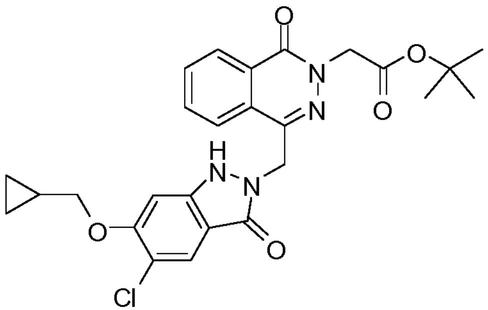 | Int.259 | NA | 511 | 511 |
| 56 | 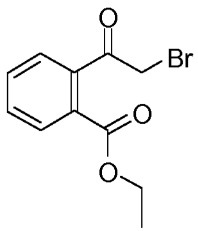 | ethyl 2-acetylbenzoate | NA | 271 | 271 |

(continued)

| Int # | Structure | Starting material | Mtd | MW (calc) | MW (obs) |
|---|---|---|---|---|---|
| 57 | 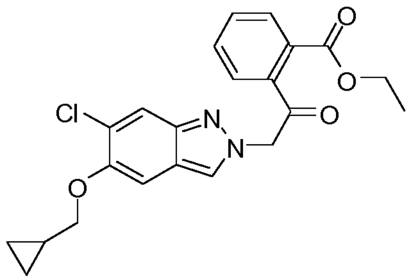 | Int.56 | NA | 413 | 414 |
| 58 | 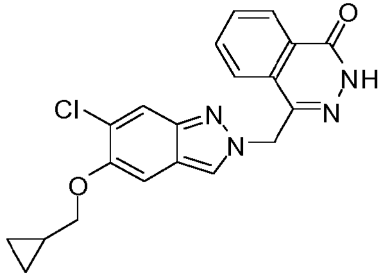 | Int.57 or Int 1+ Int 5 | NA | 381 | 382 |
| 59 | 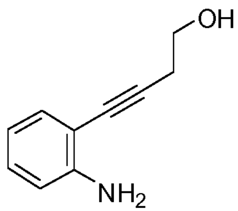 | 2-iodoaniline | Q | 161 | 162 |
| 60 | 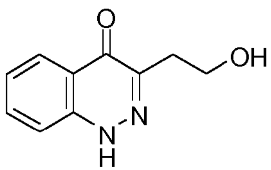 | Int.59 | R | 190 | 191 |

(continued)

| Int # | Structure | Starting material | Mtd | MW (calc) | MW (obs) |
|---|---|---|---|---|---|
| 61 | 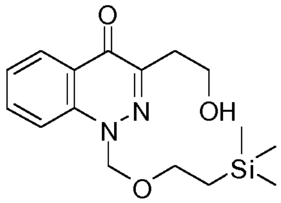 | Int.60 | S | 320 | 322 |
| 62 | 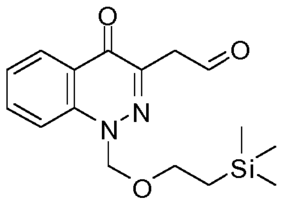 | Int.61 | T | 318 | 319 |
| 63 | 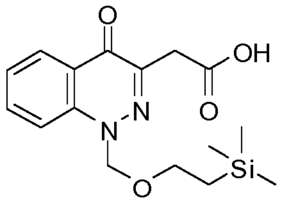 | Int.62 | U | 334 | 335 |
| 64 | 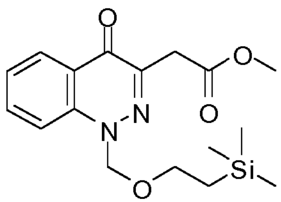 | Int.63 | V | 348 | 350 |

(continued)

| Int # | Structure | Starting material | Mtd | MW (calc) | MW (obs) |
|---|---|---|---|---|---|
| 65 | 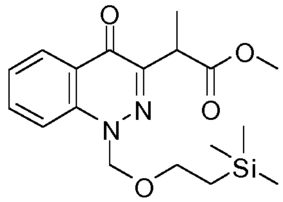 | Int.64 | W | 363 | 364 |
| 66 | 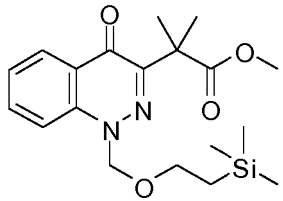 | Int.64 | NA | 377 | 378 |
| 67 | 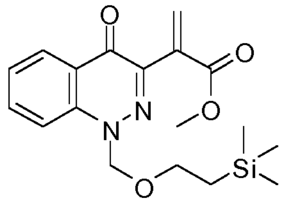 | Int.64 | NA | 360 | 362 |
| 68 | 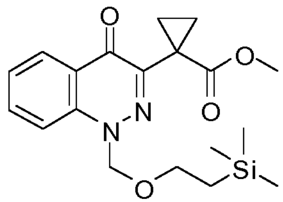 | Int.67 | NA | 375 | 376 |

(continued)

| Int # | Structure | Starting material | Mtd | MW (calc) | MW (obs) |
|---|---|---|---|---|---|
| 69 | 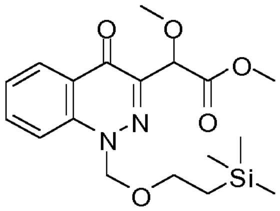 | Int.395 | V | 379 | 379 |
| 70 | 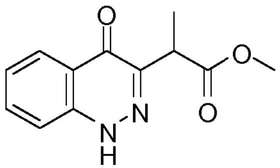 | Int.65 | X | 232 | 233 |
| 71 | 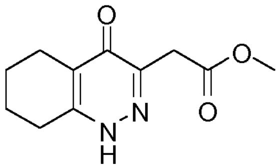 | Int.431 | NA | 222 | 223 |
| 72 | 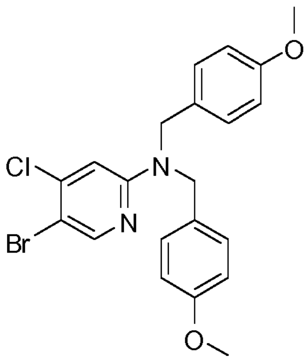 | 5-bromo-4-chloro-pyridin-2-amine | Y | 448 | 447 |

(continued)

| Int # | Structure | Starting material | Mtd | MW (calc) | MW (obs) |
|---|---|---|---|---|---|
| 73 | 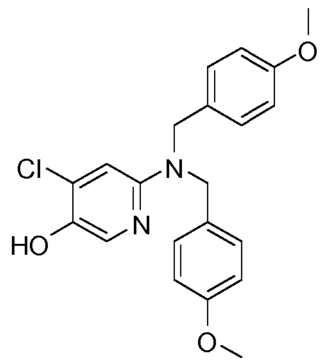 | Int.72 | Z | 385 | 386 |
| 74 | 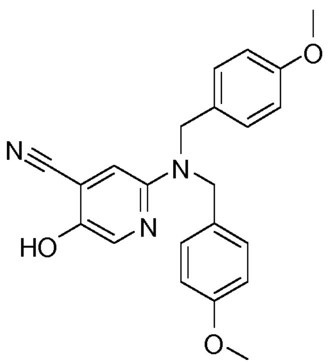 | Int.438 | NA | 375 | 376 |
| 75 | 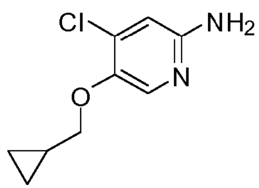 | Int.73 | AA | 199 | 199 |

(continued)

| Int # | Structure | Starting material | Mtd | MW (calc) | MW (obs) |
|---|---|---|---|---|---|
| 76 | 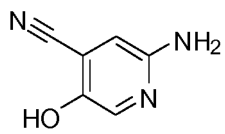 | Int.74 | NA | 135 | 136 |
| 77 | 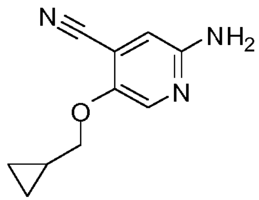 | Int.76 | NA | 189 | 190 |
| 78 | 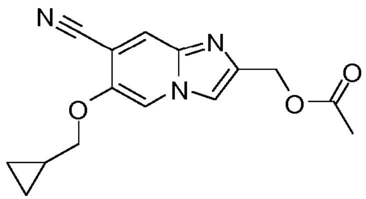 | Int.77 | AB | 285 | 286 |
| 79 | 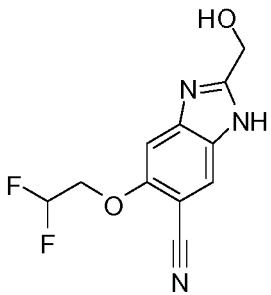 | Int.134 | NA | 253 | 254 |

(continued)

| Int # | Structure | Starting material | Mtd | MW (calc) | MW (obs) |
|---|---|---|---|---|---|
| 80 | 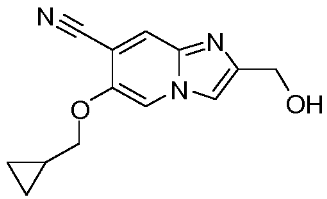 | Int.78 | AC | 243 | 244 |
| 81 | 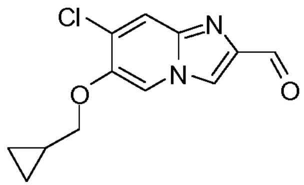 | Int.117 | AD | 251 | 251 |
| 82 | 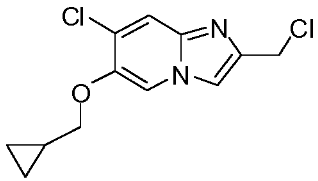 | Int.75 | AE | 271 | 271 |
| 83 | 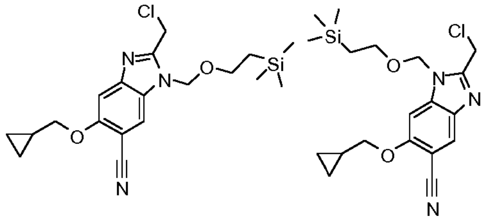 | Int.120 | AF | 391 | 393 |
| 84 | 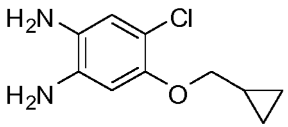 | Int.43 | AG | 213 | 213 |

(continued)

| Int # | Structure | Starting material | Mtd | MW (calc) | MW (obs) |
|---|---|---|---|---|---|
| 85 | 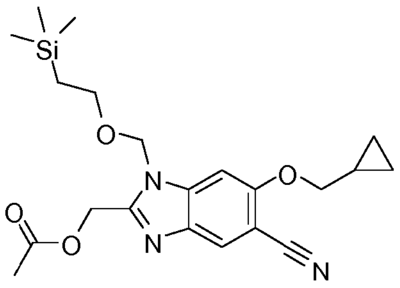 | Int.400 | NA | 416 | 416 |
| 86 | 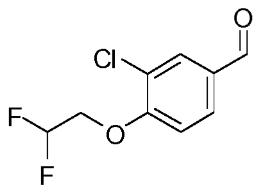 | 3-chloro-4-hydroxybenzaldehyde | NA | 221 | NA |
| 87 | 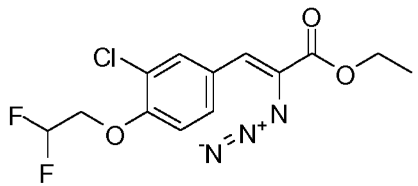 | Int.86 | NA | 332 | NA |
| 88 | 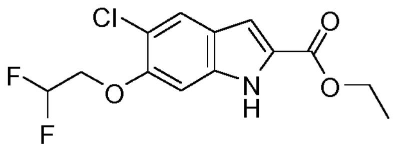 | Int.87 | NA | 304 | 302 [M-1] |

(continued)

| Int # | Structure | Starting material | Mtd | MW (calc) | MW (obs) |
|---|---|---|---|---|---|
| 89 | 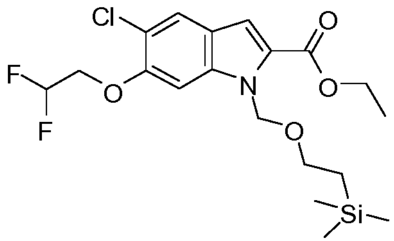 | Int.88 | AH | 434 | NA |
| 90 | 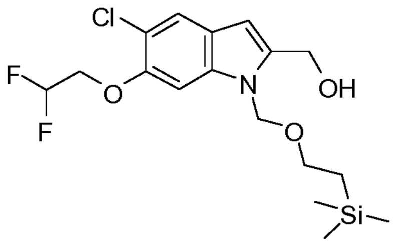 | Int.89 | AI | 392 | NA |
| 91 | 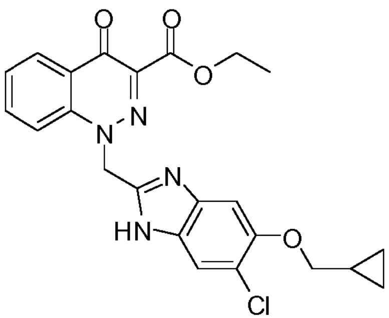 | Int.390 | AJ | 453 | 453 |

(continued)

| Int # | Structure | Starting material | Mtd | MW (calc) | MW (obs) |
|---|---|---|---|---|---|
| 92 | 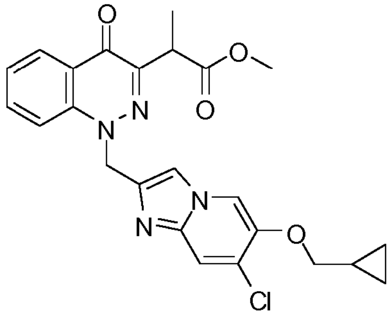 | Int.70 | AK | 467 | 467 |
| 93 | 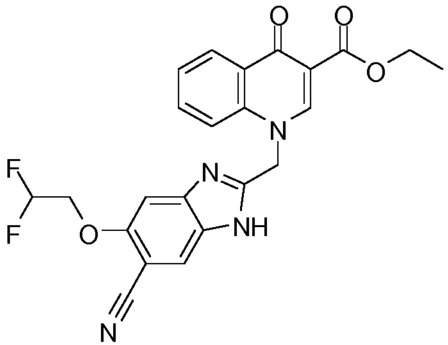 | Int.232 | AL | 452 | 454 |
| 94 | 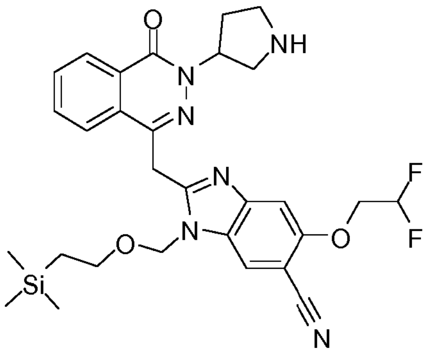 | Int.347 | AN | 581 | 581 |

(continued)

| Int # | Structure | Starting material | Mtd | MW (calc) | MW (obs) |
|---|---|---|---|---|---|
| 95 | 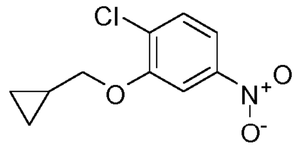 | 2-chloro-5-nitro-phenol | NA | 228 | NA |
| 96 | 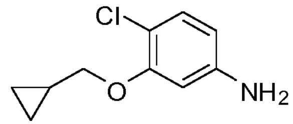 | Int.95 | NA | 198 | 198 |
| 97 | 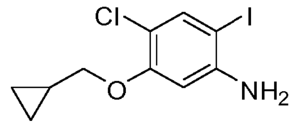 | Int.96 | NA | 324 | 321.9 [M-1] |
| 98 | 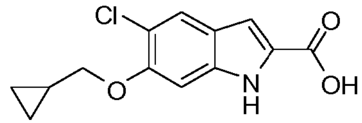 | Int.97 | NA | 266 | 264 [M-1] |
| 99 | 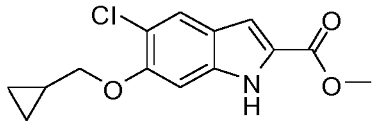 | Int.98 | NA | 280 | 278 [M-1] |
| 100 | 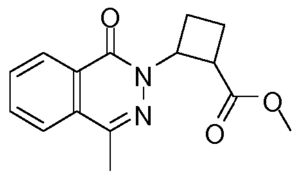 | Int.1 | NA | 272 | 273 |

(continued)

| Int # | Structure | Starting material | Mtd | MW (calc) | MW (obs) |
|---|---|---|---|---|---|
| 101 | 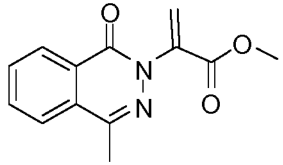 | Int.1 | NA | 244 | 245 |
| 102 | 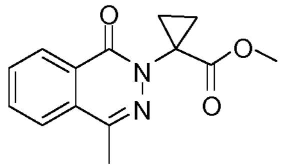 | Int.101 | NA | 258 | 259 |
| 103 | 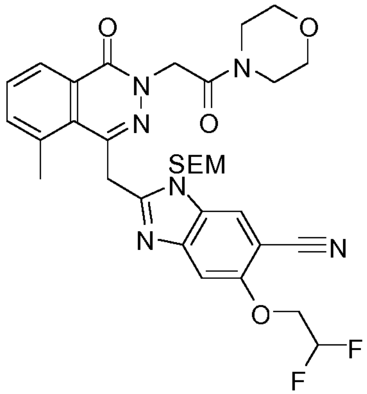 | Int.154 | NA | 653 | 653 |

(continued)

| Int # | Structure | Starting material | Mtd | MW (calc) | MW (obs) |
|---|---|---|---|---|---|
| 104 | 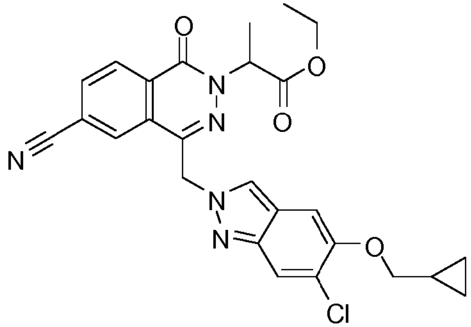 | Int.271 | NA | 506 | 506 |
| 105 | 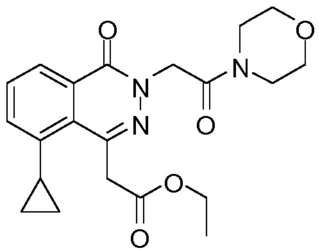 | Int.313 | NA | 399 | 400 |
| 106 | 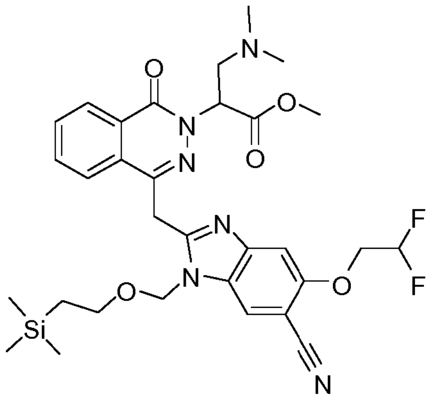 | Int. 303 | AP | 641 | 641 |

(continued)

| Int # | Structure | Starting material | Mtd | MW (calc) | MW (obs) |
|---|---|---|---|---|---|
| 107 | 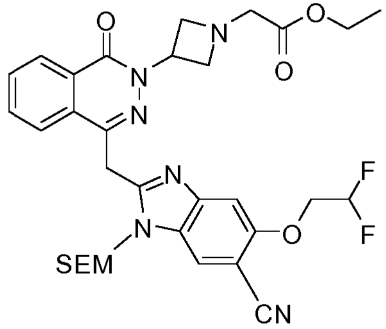 | Int.239 | AQ | 653 | 653 |
| 108 | 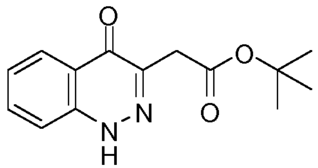 | Int.437 | NA | 260 | 259.51 [M-H]- |
| 109 | 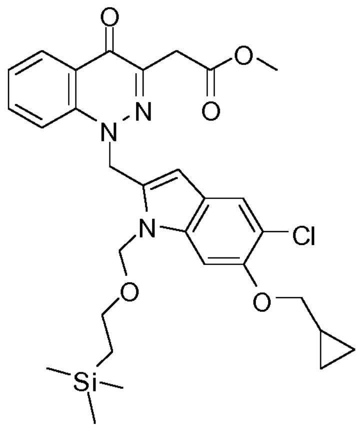 | Int.159 | NA | 582 | 583 |

(continued)

| Int # | Structure | Starting material | Mtd | MW (calc) | MW (obs) |
|---|---|---|---|---|---|
| 110 | 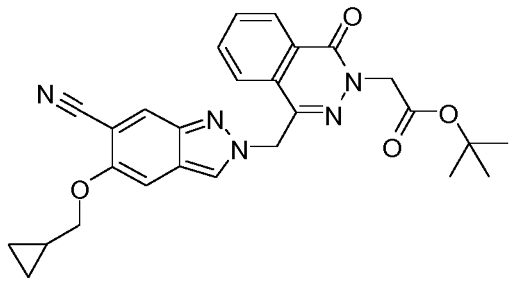 | Int.276 | NA | 486 | 486 |
| 111 | 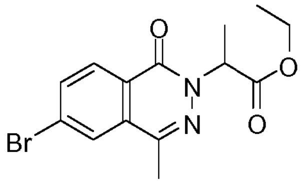 | Int.296 | A | 339 | 341 |
| 112 | 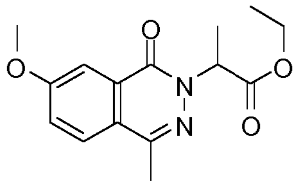 | Int.29 | A | 290 | 291 |
| 113 | 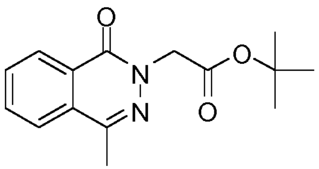 | Int.1 | A | 274 | 219 |

(continued)

| Int # | Structure | Starting material | Mtd | MW (calc) | MW (obs) |
|---|---|---|---|---|---|
| 114 | 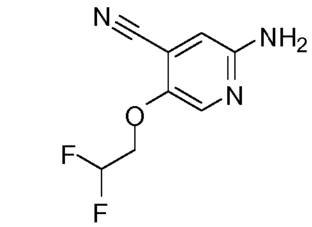 | 2-[bis[(4-methoxypheny l)methyl]amin o]-5-(2,2-difluoroethoxy )pyridine-4-carbonitrile | AA | 199 | 200 |
| 115 | 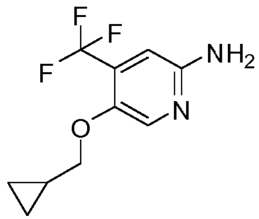 | Int.440 | AA | 232 | 233 |
| 116 | 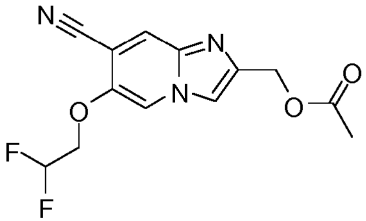 | Int.114 | AB | 295 | 296 |
| 117 | 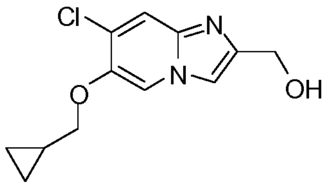 | Int.75 | AB & AC | 253 | 253 |

(continued)

| Int # | Structure | Starting material | Mtd | MW (calc) | MW (obs) |
|---|---|---|---|---|---|
| 118 | 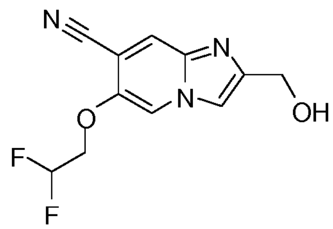 | Int.116 | AC | 253 | 254 |
| 119 | 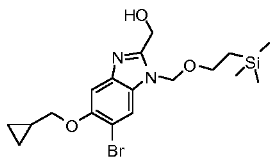 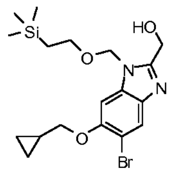 | Int.156 | AC | 426 | 428 |
| 120 | 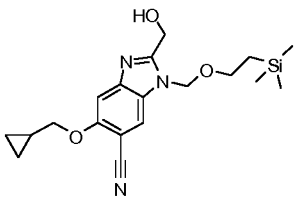 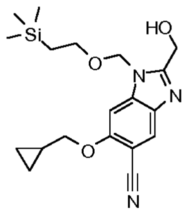 | Int.85 | AC | 373 | 375 |
| 121 | 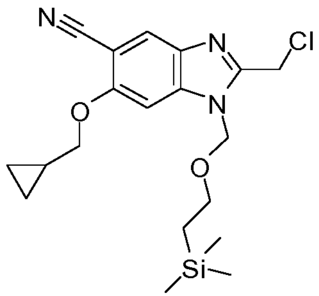 | Int.85 | AC & AF | 392 | 393 |

(continued)

| Int # | Structure | Starting material | Mtd | MW (calc) | MW (obs) |
|---|---|---|---|---|---|
| 122 | 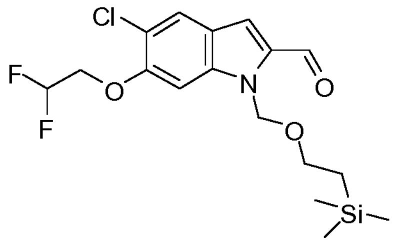 | Int.90 | AD | 390 | NA |
| 123 | 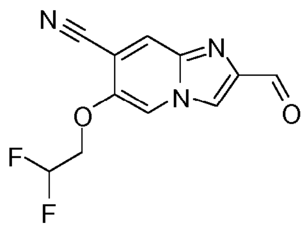 | Int.118 | AD | 251 | 252 |
| 124 | 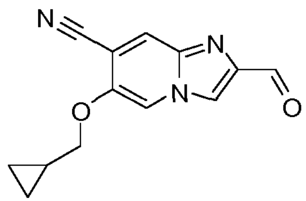 | Int.80 | AD | 241 | 242 |
| 125 | 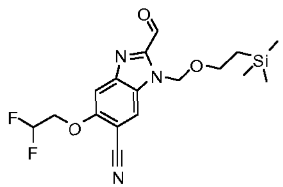 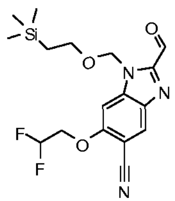 | Int. 158 | AD | 381 | 382 |

(continued)

| Int # | Structure | Starting material | Mtd | MW (calc) | MW (obs) |
|---|---|---|---|---|---|
| 126 | 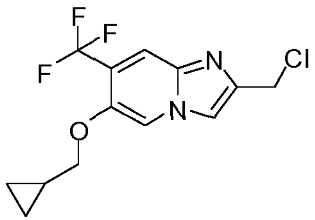 | Int.115 | AE | 305 | 305 |
| 127 | 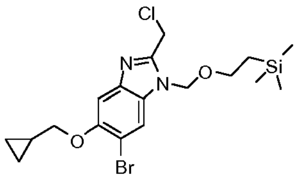 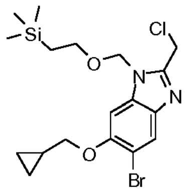 | Int.119 | AF | 444 | 446 |
| 128 | 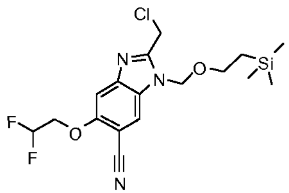 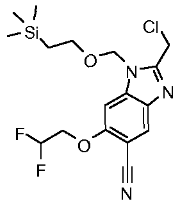 | Int.120 | AF | 402 | 402 |
| 129 | 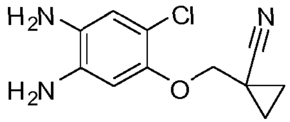 | Int.397 | AG | 238 | 238 |
| 130 | 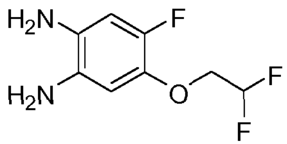 | Int.411 | AG | 206 | 207 |

(continued)

| Int # | Structure | Starting material | Mtd | MW (calc) | MW (obs) |
|---|---|---|---|---|---|
| 131 | 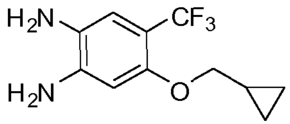 | Int.412 | AG | 246 | 247 |
| 132 | 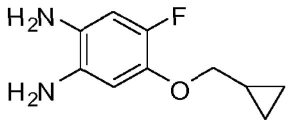 | Int.413 | AG | 196 | 197 |
| 133 | 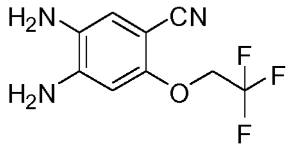 | Int.402 | AG | 231 | 232 |
| 134 | 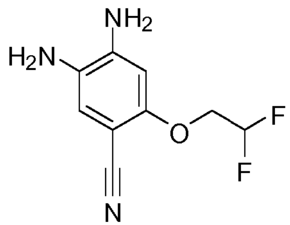 | Int. 441 | AG | 213 | 214 |
| 135 | 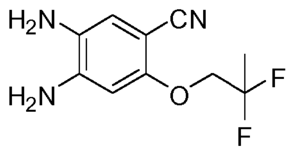 | Int.398 | AG | 227 | 228 |
| 136 | 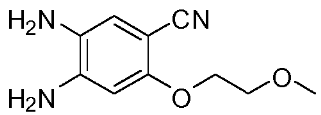 | Int.399 | AG | 207 | 208 |

(continued)

| Int # | Structure | Starting material | Mtd | MW (calc) | MW (obs) |
|---|---|---|---|---|---|
| 137 | 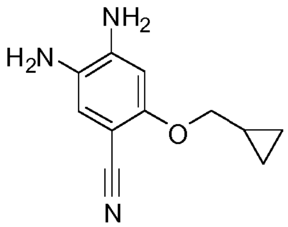 | Int.400 | AG | 203 | 204 |
| 138 | 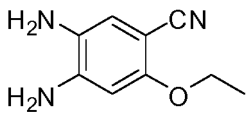 | Int.401 | AG | 177 | 178 |
| 139 | 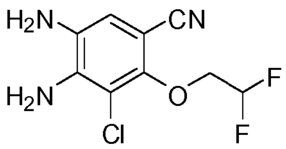 | Int.42 | AG | 248 | 248 |
| 140 | 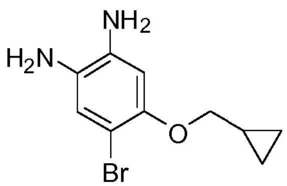 | Int.403 | AG | 257 | 257 |
| 141 | 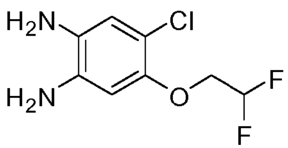 | Int.405 | AG | 223 | 223 |
| 142 | 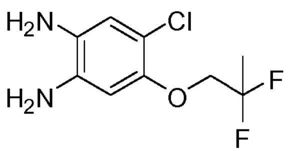 | Int.406 | AG | 237 | 237 |

(continued)

| Int # | Structure | Starting material | Mtd | MW (calc) | MW (obs) |
|---|---|---|---|---|---|
| 143 | 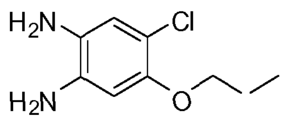 | Int.407 | AG | 229 | 229 |
| 144 | 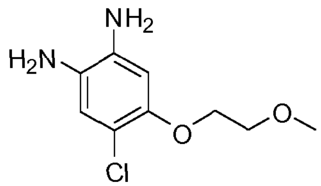 | Int.408 | AG | 217 | 217 |
| 145 | 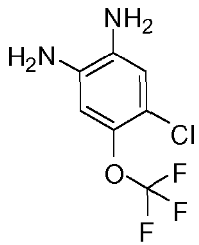 | 5-chloro-2-nitro-4-(trifluorometh oxy)aniline | AG | 227 | 227 |
| 146 | 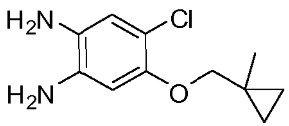 | Int.409 | AG | 227 | 227 |
| 147 | 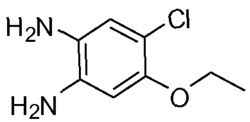 | Int.41 | AG | 187 | 187 |
| 148 | 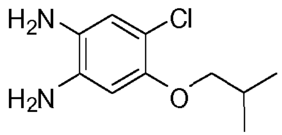 | Int.410 | AG | 215 | 215 |

(continued)

| Int # | Structure | Starting material | Mtd | MW (calc) | MW (obs) |
|---|---|---|---|---|---|
| 149 | 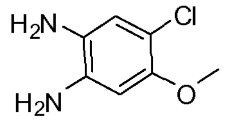 | 5-chloro-4-methoxy-2-nitro-aniline | AG | 173 | 173 |
| 150 | 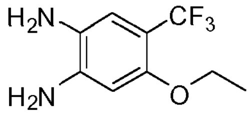 | Int.415 | AG | 220 | 221 |
| 151 | 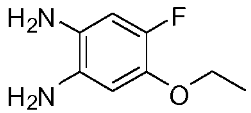 | Int.416 | AG | 170 | 171 |
| 152 | 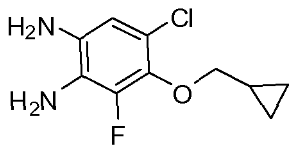 | Int.404 | AG | 231 | 231 |
| 153 | 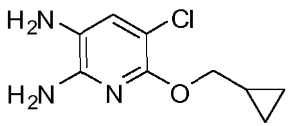 | Int.414 | AG | 214 | 214 |

(continued)

| Int # | Structure | Starting material | Mtd | MW (calc) | MW (obs) |
|---|---|---|---|---|---|
| 154 | 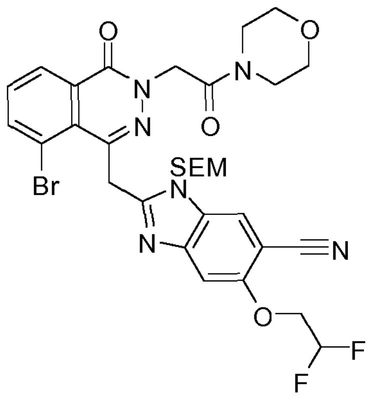 | Int.162 | AH | 718 | 719 |
| 155 | 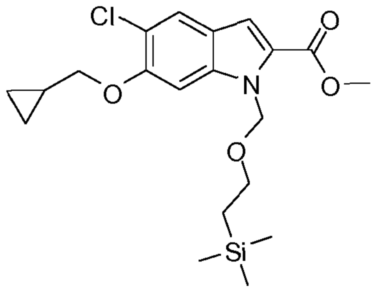 | Int.99 | AH | 410 | NA |
| 156 | 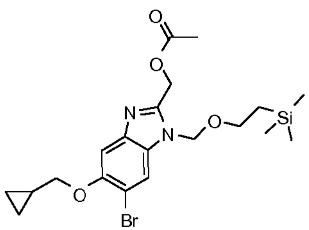 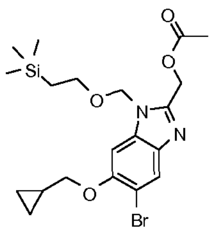 | Int.160 | AH | 468 | 470 |

(continued)

| Int # | Structure | Starting material | Mtd | MW (calc) | MW (obs) |
|---|---|---|---|---|---|
| 157 | 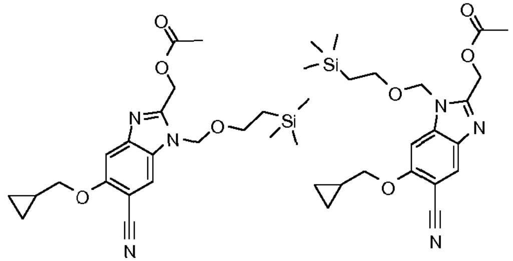 | Int.161 | AH | 415 | 416 |
| 158 | 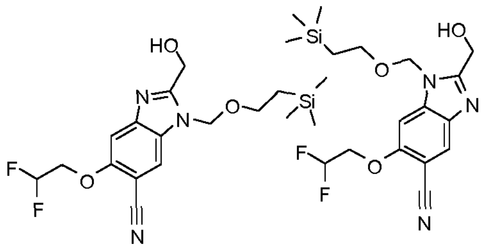 | Int.79 | AH | 383 | 384 |
| 159 | 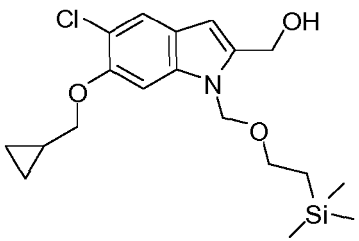 | Int.155 | AI | 382 | 382 |

(continued)

| Int # | Structure | Starting material | Mtd | MW (calc) | MW (obs) |
|---|---|---|---|---|---|
| 160 | 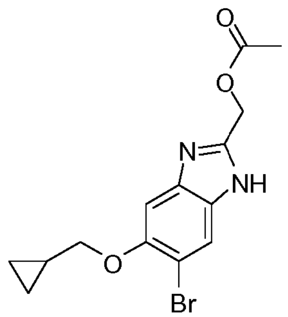 | Int.140 | AJ | 339 | 339 |
| 161 | 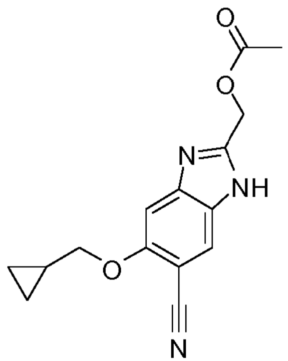 | Int.137 | AJ | 285 | 287 |

(continued)

| Int # | Structure | Starting material | Mtd | MW (calc) | MW (obs) |
|---|---|---|---|---|---|
| 162 | 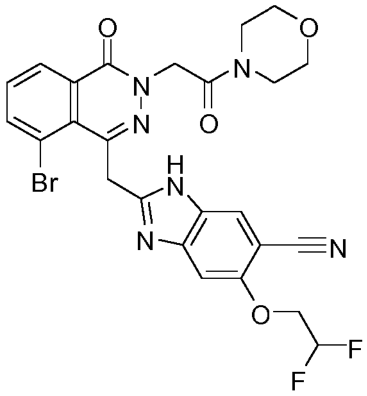 | Int.387 | AJ | 587 | 589 |
| 163 | 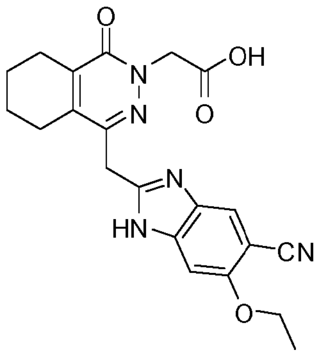 | Int.356 | AJ | 407 | 408 |

(continued)

| Int # | Structure | Starting material | Mtd | MW (calc) | MW (obs) |
|---|---|---|---|---|---|
| 164 | 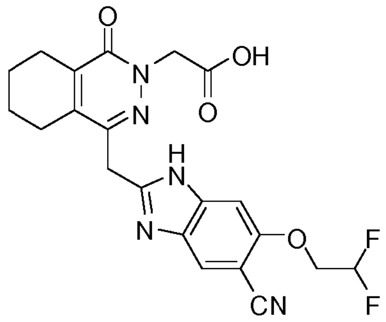 | Int.356 | AJ | 443 | 444 |
| 165 | 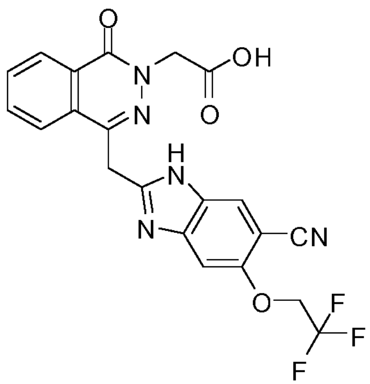 | Int.22 | AJ | 457 | 458 |

(continued)

| Int # | Structure | Starting material | Mtd | MW (calc) | MW (obs) |
|---|---|---|---|---|---|
| 166 | 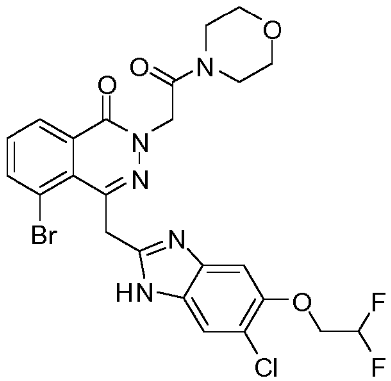 | Int.387 | AJ | 597 | 596 |
| 167 | 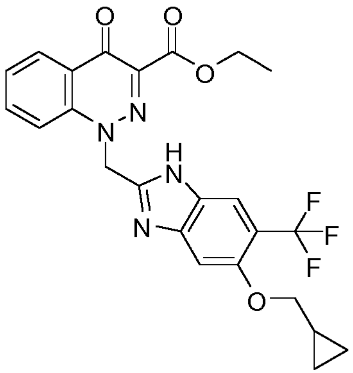 | Int.390 | AJ | 486 | 487 |

(continued)

| Int # | Structure | Starting material | Mtd | MW (calc) | MW (obs) |
|---|---|---|---|---|---|
| 168 | 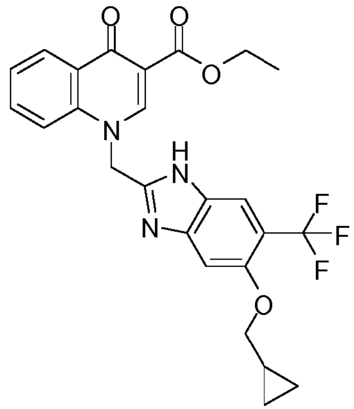 | Int.389 | AJ | 485 | 486 |
| 169 | 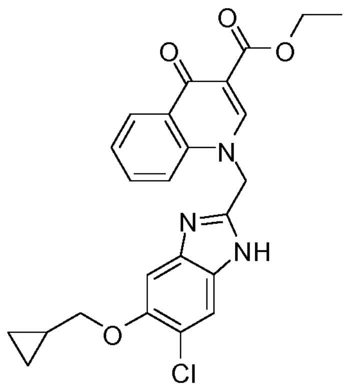 | Int.389 | AJ | 452 | 453 |

(continued)

| Int # | Structure | Starting material | Mtd | MW (calc) | MW (obs) |
|---|---|---|---|---|---|
| 170 | 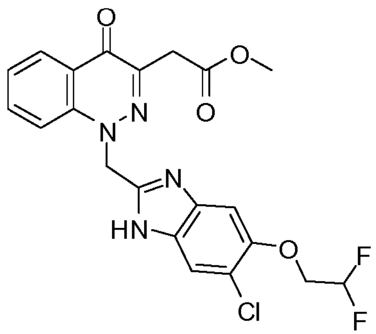 | Int.391 | AJ | 463 | 463 |
| 171 | 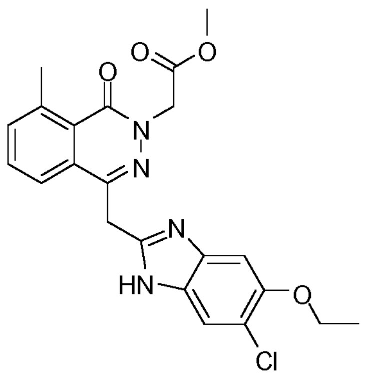 | Int.392 | AJ | 441 | 441 |

(continued)

| Int # | Structure | Starting material | Mtd | MW (calc) | MW (obs) |
|---|---|---|---|---|---|
| 172 | 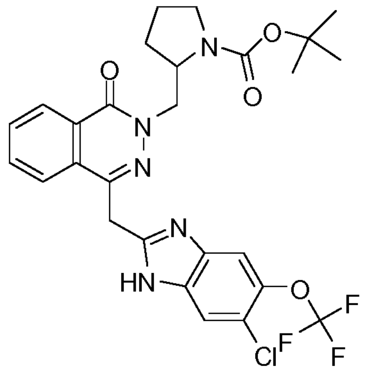 | Int.372 | AJ | 578 | 578 |
| 173 | 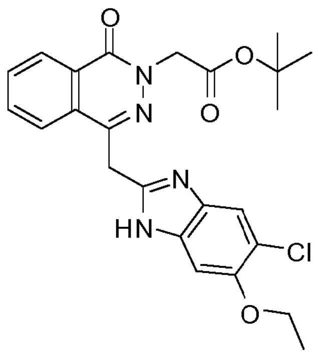 | Int.22 | AJ | 469 | 469 |

(continued)

| Int # | Structure | Starting material | Mtd | MW (calc) | MW (obs) |
|---|---|---|---|---|---|
| 174 | 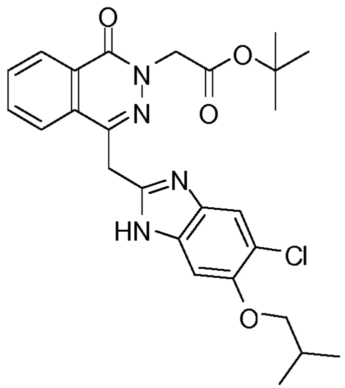 | Int.22 | AJ | 497 | 497 |
| 175 | 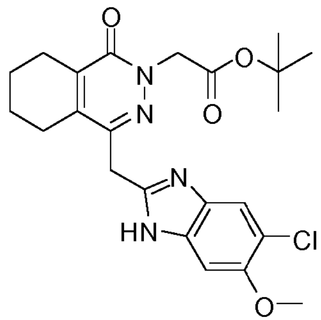 | Int.356 | AJ | 459 | 459 |
| 176 | 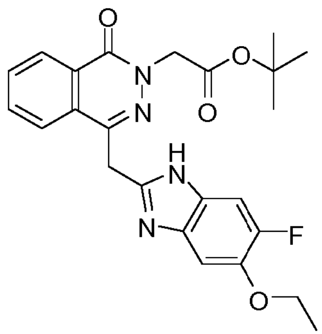 | Int.22 | AJ | 452 | 453 |

(continued)

| Int # | Structure | Starting material | Mtd | MW (calc) | MW (obs) |
|---|---|---|---|---|---|
| 177 | 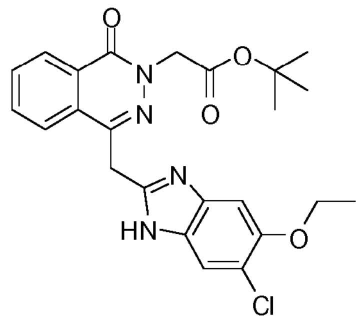 | Int.22 | AJ | 469 | 469 |
| 178 | 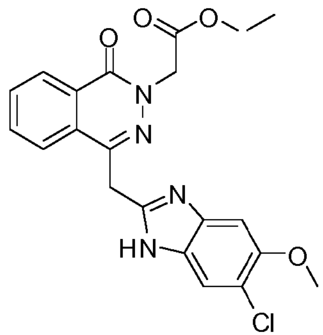 | Int.22 | AJ | 455 | 455 |
| 179 | 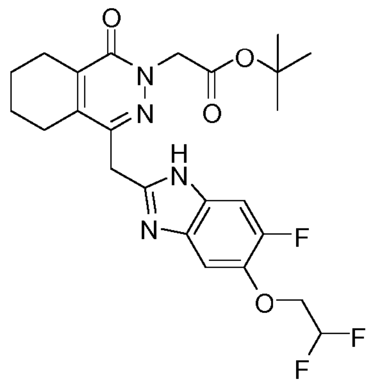 | Int.356 | AJ | 492 | 493 |

(continued)

| Int # | Structure | Starting material | Mtd | MW (calc) | MW (obs) |
|---|---|---|---|---|---|
| 180 | 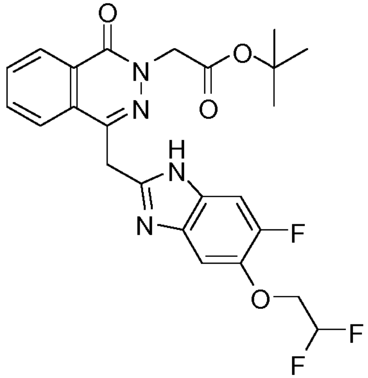 | Int.22 | AJ | 488 | 489 |
| 181 | 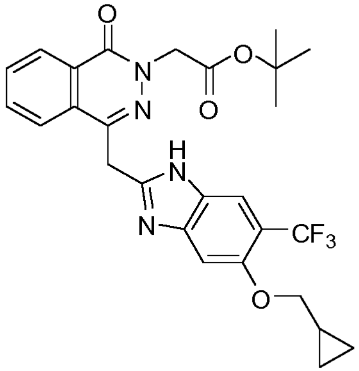 | Int.22 | AJ | 529 | 529 |

(continued)

| Int # | Structure | Starting material | Mtd | MW (calc) | MW (obs) |
|---|---|---|---|---|---|
| 182 | 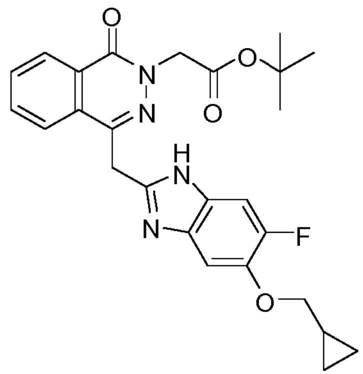 | Int.22 | AJ | 479 | 479 |
| 183 | 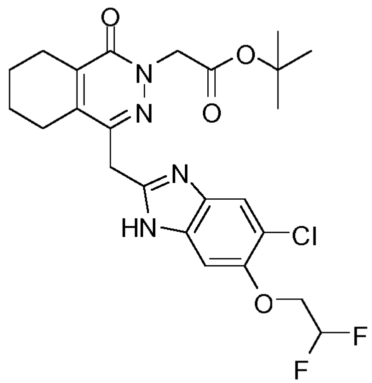 | Int.356 | AJ | 509 | 509 |

(continued)

| Int # | Structure | Starting material | Mtd | MW (calc) | MW (obs) |
|---|---|---|---|---|---|
| 184 | 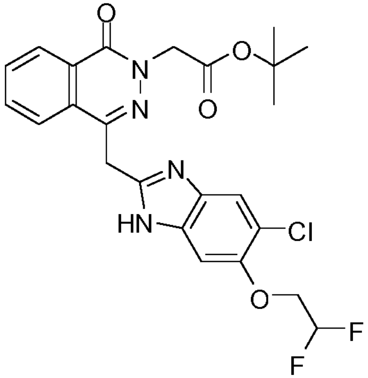 | Int.22 | AJ | 505 | 449 |
| 185 | 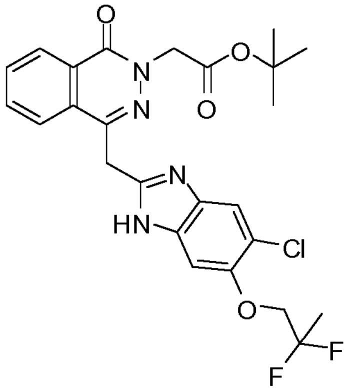 | Int.22 | AJ | 519 | 519 |

(continued)

| Int # | Structure | Starting material | Mtd | MW (calc) | MW (obs) |
|---|---|---|---|---|---|
| 186 | 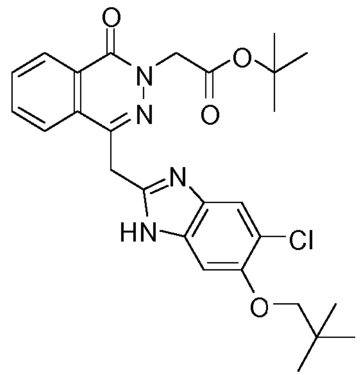 | Int.22 | AJ | 511 | 511 |
| 187 | 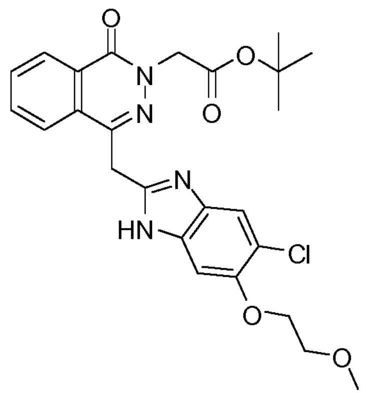 | Int.22 | AJ | 499 | 499 |

(continued)

| Int # | Structure | Starting material | Mtd | MW (calc) | MW (obs) |
|---|---|---|---|---|---|
| 188 | 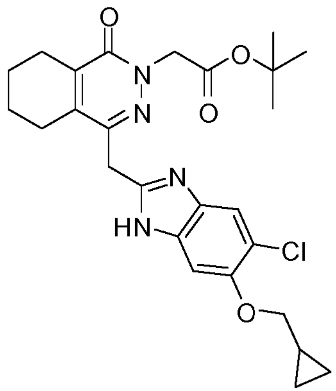 | Int.356 | AJ | 499 | 499 |
| 189 | 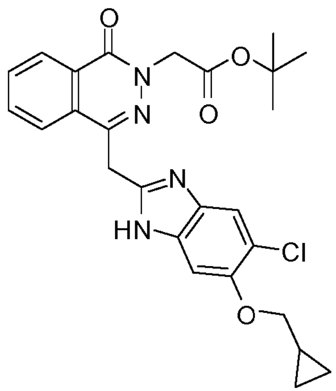 | Int.22 | AJ | 495 | 495 |

(continued)

| Int # | Structure | Starting material | Mtd | MW (calc) | MW (obs) |
|---|---|---|---|---|---|
| 190 | 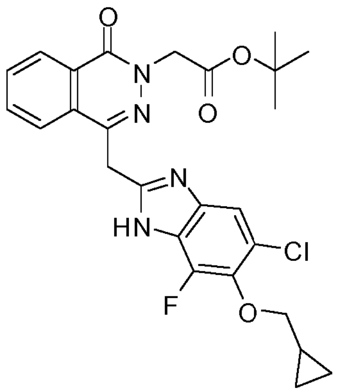 | Int.22 | AJ | 513 | 513 |
| 191 | 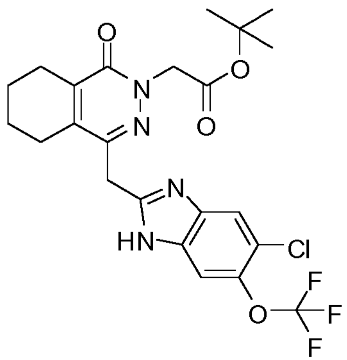 | Int.356 | AJ | 513 | 513 |

(continued)

| Int # | Structure | Starting material | Mtd | MW (calc) | MW (obs) |
| --- | --- | --- | --- | --- | --- |
| 192 | 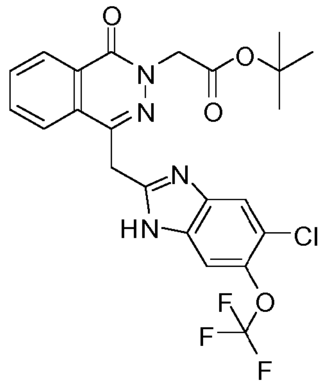 | Int.22 | AJ | 509 | 509 |
| 193 | 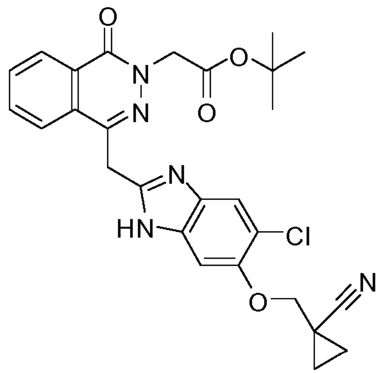 | Int.22 | AJ | 520 | 520 |

(continued)

| Int # | Structure | Starting material | Mtd | MW (calc) | MW (obs) |
|---|---|---|---|---|---|
| 194 | 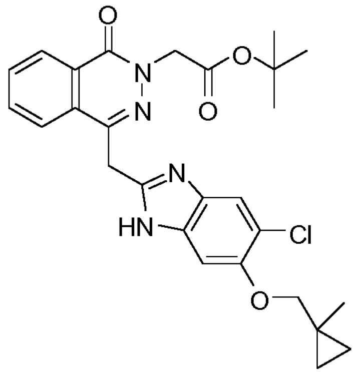 | Int.22 | AJ | 509 | 509 |
| 195 | 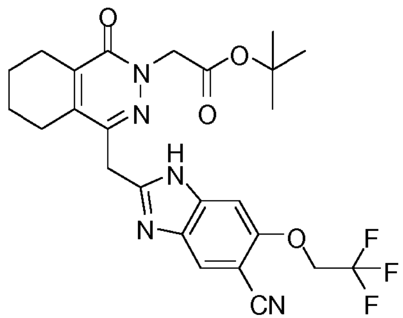 | Int.356 | AJ | 518 | 518 |
| 196 | 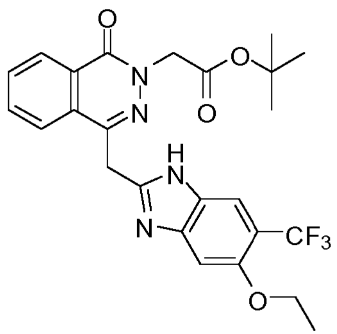 | Int.22 | AJ | 502 | 503 |

(continued)

| Int # | Structure | Starting material | Mtd | MW (calc) | MW (obs) |
|---|---|---|---|---|---|
| 197 | 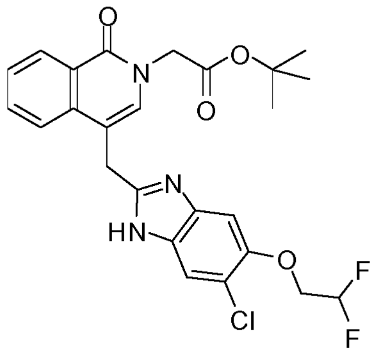 | Int.360 | AJ | 504 | 504 |
| 198 | 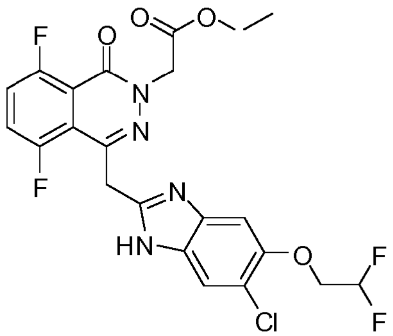 | Int.357 | AJ | 541 | 541 |

(continued)

| Int # | Structure | Starting material | Mtd | MW (calc) | MW (obs) |
|---|---|---|---|---|---|
| 199 | 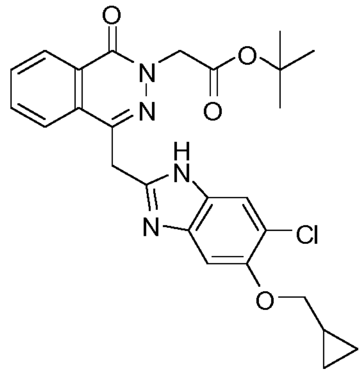 | Int.22 | AJ | 495 | 495 |
| 200 | 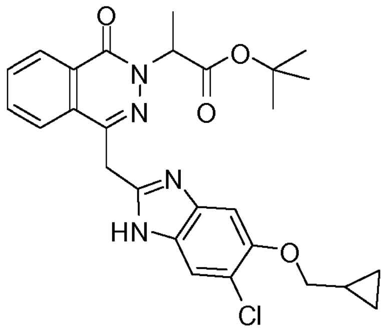 | Int.364 | AJ | 509 | 509 |
| 201 | 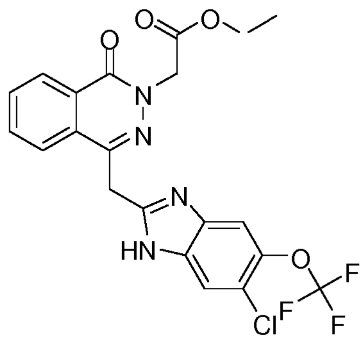 | Int.22 | AJ | 509 | 509 |

(continued)

| Int # | Structure | Starting material | Mtd | MW (calc) | MW (obs) |
|---|---|---|---|---|---|
| 202 | 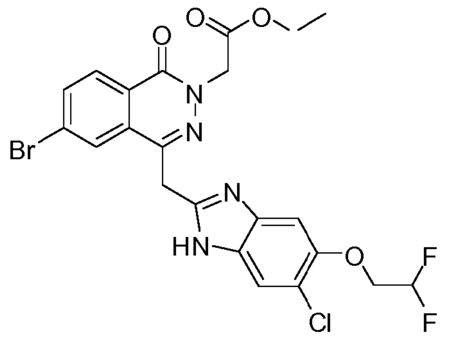 | Int.358 | AJ | 584 | 583 |
| 203 | 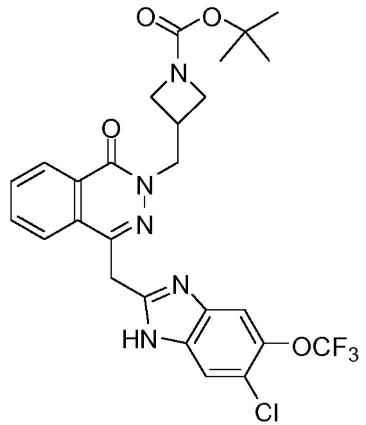 | Int.371 | AJ | 564 | 564 |

(continued)

| Int # | Structure | Starting material | Mtd | MW (calc) | MW (obs) |
|---|---|---|---|---|---|
| 204 | 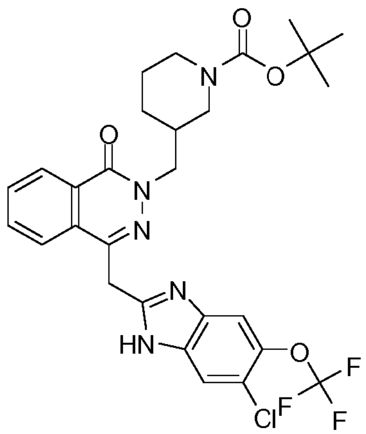 | Int.369 | AJ | 592 | 592 |
| 205 | 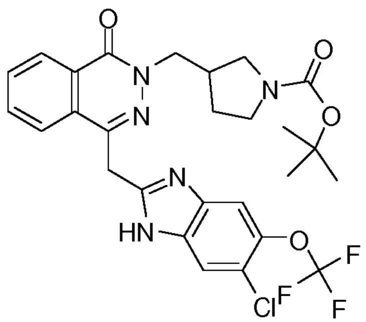 | Int.373 | AJ | 578 | 578 |

(continued)

| Int # | Structure | Starting material | Mtd | MW (calc) | MW (obs) |
|---|---|---|---|---|---|
| 206 | 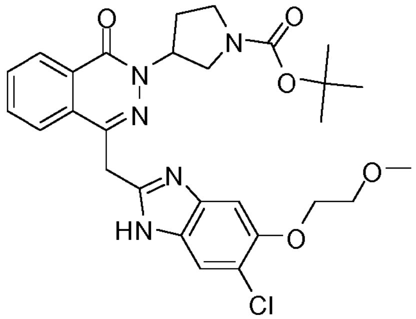 | Int.23 | AJ | 554 | 554 |
| 207 | 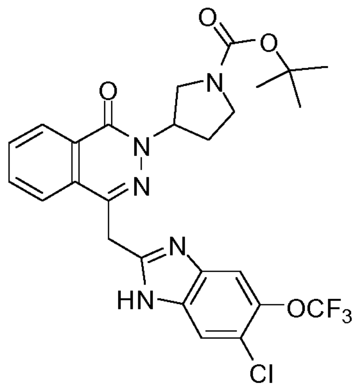 | Int.23 | AJ | 564 | 564 |

(continued)

| Int # | Structure | Starting material | Mtd | MW (calc) | MW (obs) |
|---|---|---|---|---|---|
| 208 | 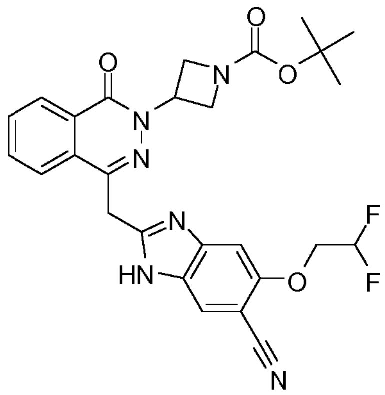 | Int.362 | AJ | 537 | 537 |
| 209 | 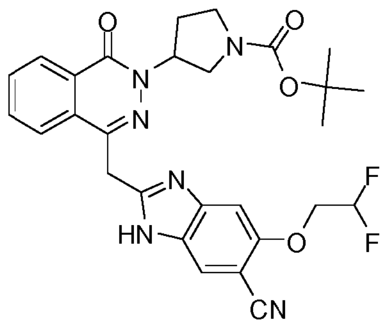 | Int.23 | AJ | 551 | 551 |
| 210 | 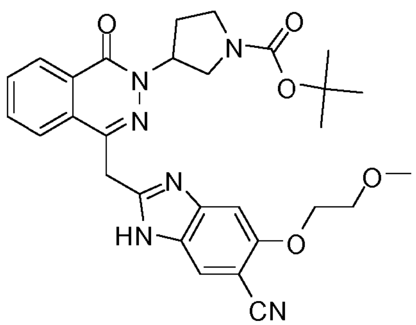 | Int.23 | AJ | 545 | 545 |

(continued)

| Int # | Structure | Starting material | Mtd | MW (calc) | MW (obs) |
|---|---|---|---|---|---|
| 211 | 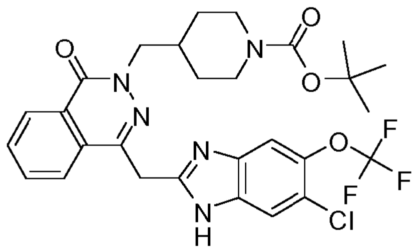 | Int.370 | AJ | 592 | 592 |
| 212 | 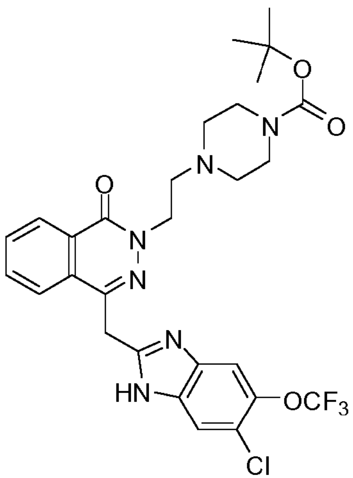 | Int.380 | AJ | 607 | 607 |

(continued)

| Int # | Structure | Starting material | Mtd | MW (calc) | MW (obs) |
|---|---|---|---|---|---|
| 213 | 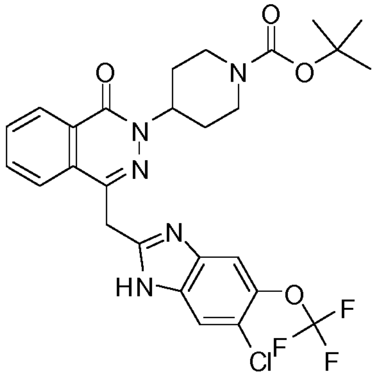 | Int.361 | AJ | 578 | 578 |
| 214 | 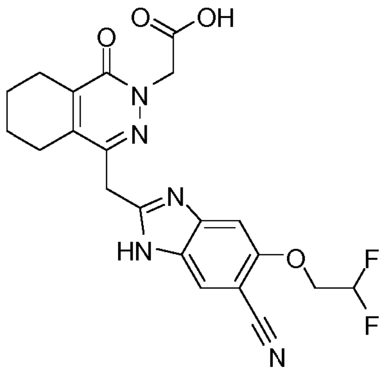 | Int.356 | AJ | 443 | 444 |

(continued)

| Int # | Structure | Starting material | Mtd | MW (calc) | MW (obs) |
|---|---|---|---|---|---|
| 215 | 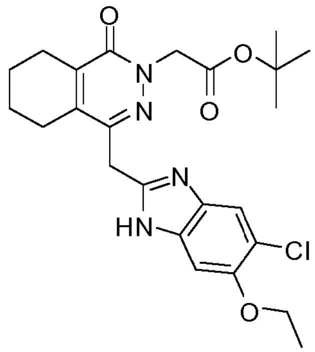 | Int.356 | AJ | 473 | 473 |
| 216 | 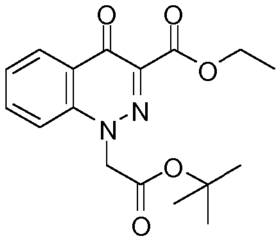 | ethyl 4-oxo-1H-cinnoline-3-carboxylate | AK | 332 | 334 |
| 217 | 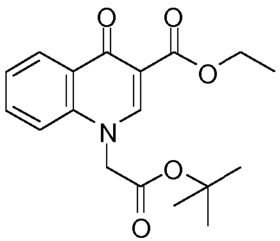 | ethyl 4-oxo-1H-quinoline-3-carboxylate | AK | 331 | 333 |

(continued)

| Int # | Structure | Starting material | Mtd | MW (calc) | MW (obs) |
|---|---|---|---|---|---|
| 218 | 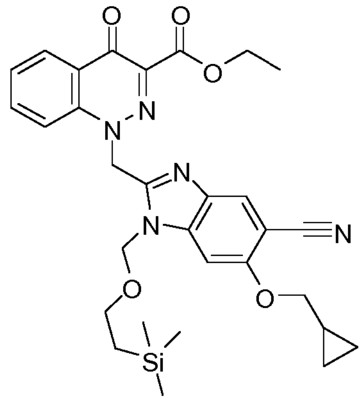 | ethyl 4-oxo-1H-cinnoline-3-carboxylate | AK | 574 | 575 |
| 219 | 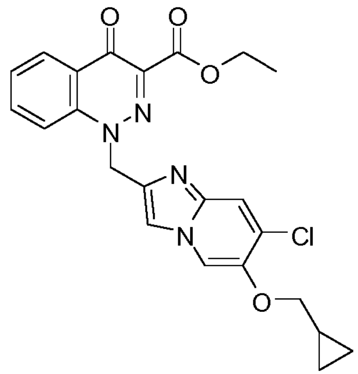 | ethyl 4-oxo-1H-cinnoline-3-carboxylate | AK | 453 | 454 |

(continued)

| Int # | Structure | Starting material | Mtd | MW (calc) | MW (obs) |
|---|---|---|---|---|---|
| 220 | 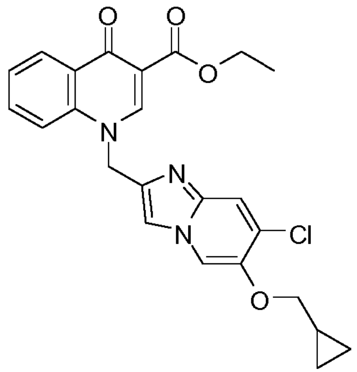 | ethyl 4-oxo-1H-quinoline-3-carboxylate | AK | 452 | 453 |
| 221 | 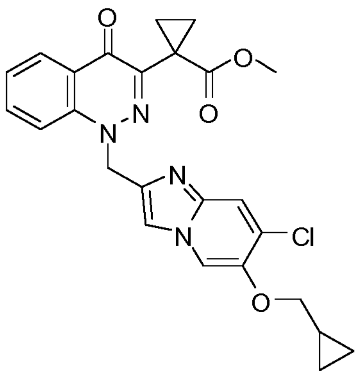 | Int.43 0 | AK | 479 | 480 |

(continued)

| Int # | Structure | Starting material | Mtd | MW (calc) | MW (obs) |
|---|---|---|---|---|---|
| 222 | 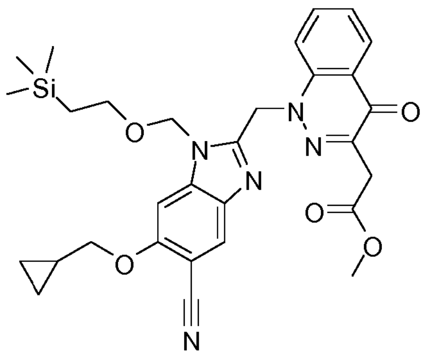 | Int.64 | AK | 574 | 445 |
| 223 | 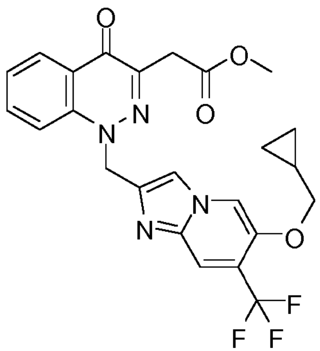 | Int.431 | AK | 486 | 487 |

(continued)

| Int # | Structure | Starting material | Mtd | MW (calc) | MW (obs) |
|---|---|---|---|---|---|
| 224 | 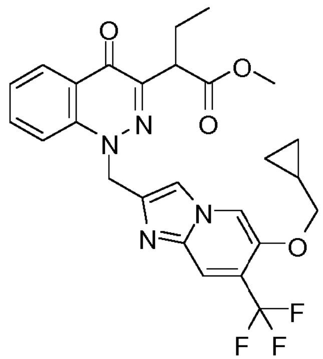 | Int.432 | AK | 515 | 515 |
| 225 | 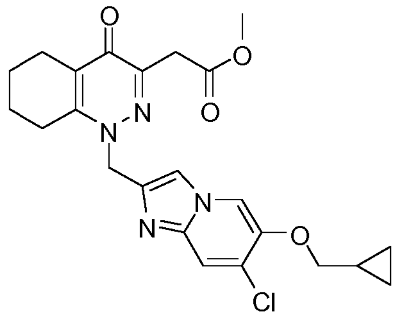 | Int.71 | AK | 457 | 457 |

(continued)

| Int # | Structure | Starting material | Mtd | MW (calc) | MW (obs) |
|---|---|---|---|---|---|
| 226 | 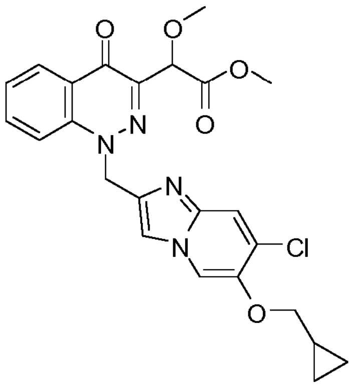 | Int.435 | AK | 483 | 484 |
| 227 | 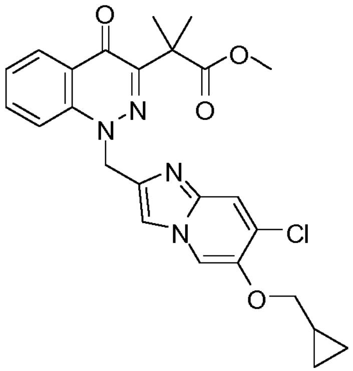 | Int.436 | AK | 481 | 482 |

(continued)

| Int # | Structure | Starting material | Mtd | MW (calc) | MW (obs) |
|---|---|---|---|---|---|
| 228 | 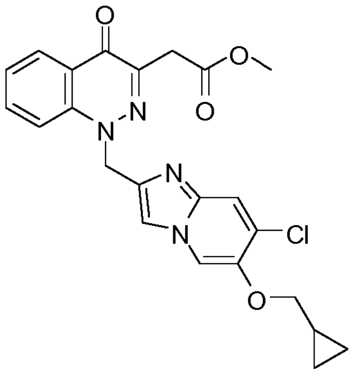 | Int.431 | AK | 453 | 454 |
| 229 | 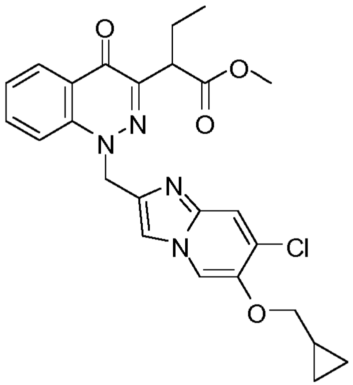 | Int.432 | AK | 481 | 481 |

(continued)

| Int # | Structure | Starting material | Mtd | MW (calc) | MW (obs) |
|---|---|---|---|---|---|
| 230 | 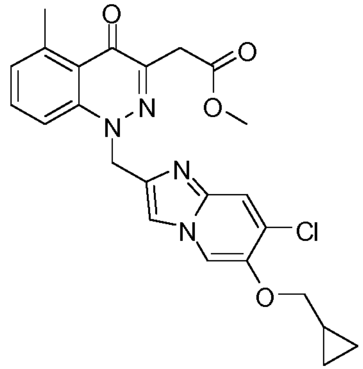 | Int.433 | AK | 467 | 467 |
| 231 | 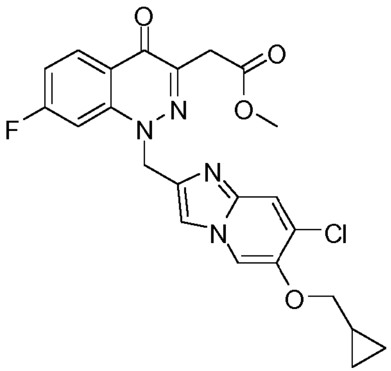 | Int.434 | AK | 471 | 471 |
| 232 | 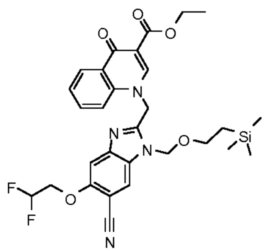 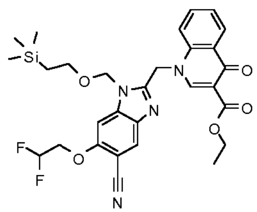 | Int.128 | AK | 583 | 584 |

(continued)

| Int # | Structure | Starting material | Mtd | MW (calc) | MW (obs) |
|---|---|---|---|---|---|
| 233 | 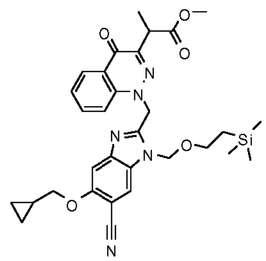 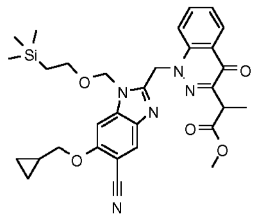 | Int.83 | AK | 587 | 589 |
| 234 | 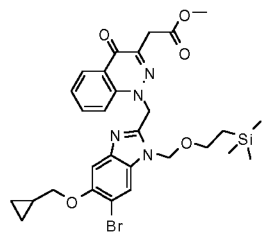 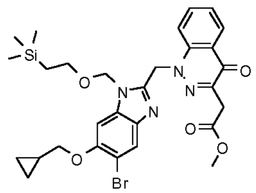 | Int.127 | AK | 628 | 628 |
| 235 | 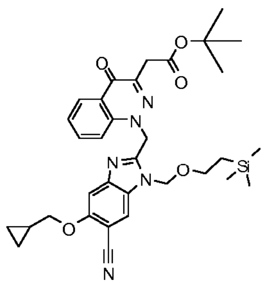 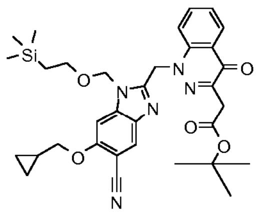 | Int.83 | AK | 616 | 617 |

(continued)

| Int # | Structure | Starting material | Mtd | MW (calc) | MW (obs) |
|---|---|---|---|---|---|
| 236 | 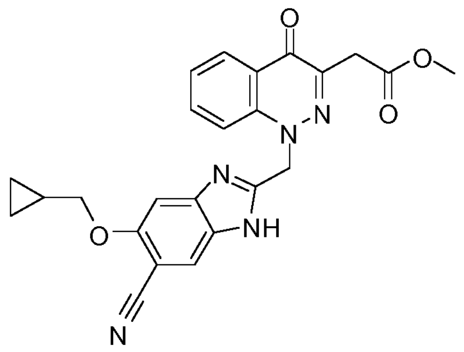 | Int.222 | AL | 443 | 445 |
| 237 | 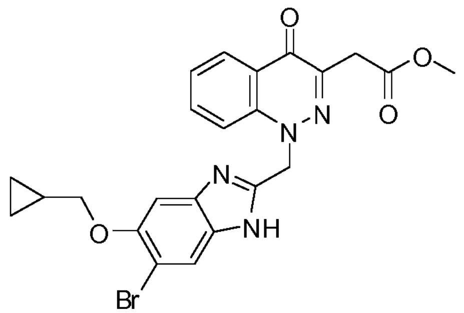 | Int.234 | AL | 497 | 498 |
| 238 | 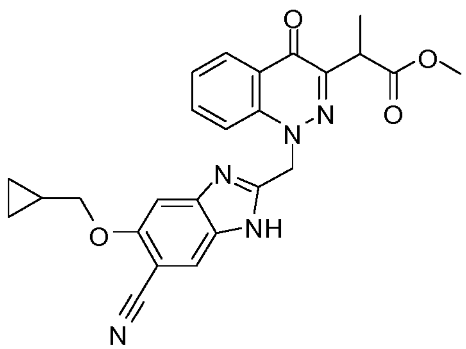 | methyl 2-[1-[[5-cyano-6-(cyclopropylm ethoxy)-1-(2-trimethylsilyle thoxymethyl)b enzimidazol-2-yl]methyl]-4-oxo-cinnolin-3-yl]propanoate | AL | 457 | 459 |

(continued)

| Int # | Structure | Starting material | Mtd | MW (calc) | MW (obs) |
|---|---|---|---|---|---|
| 239 | 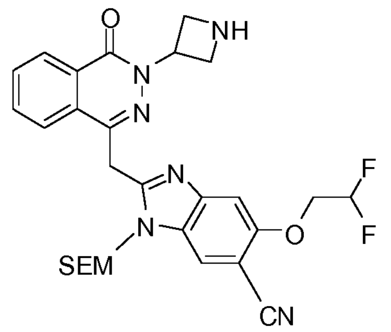 | Int.346 | AN | 567 | 567 |
| 240 | 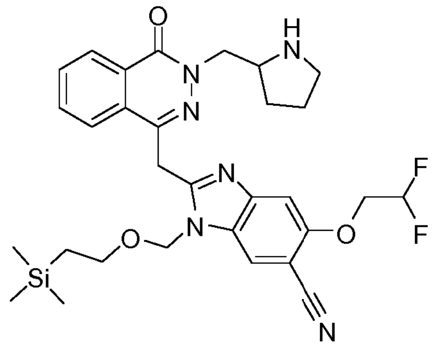 | Int.335 | AN | 595 | 595 |
| 241 | 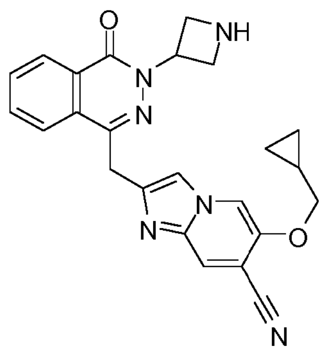 | Int.349 | AO | 426 | 428 |

(continued)

| Int # | Structure | Starting material | Mtd | MW (calc) | MW (obs) |
|---|---|---|---|---|---|
| 242 | 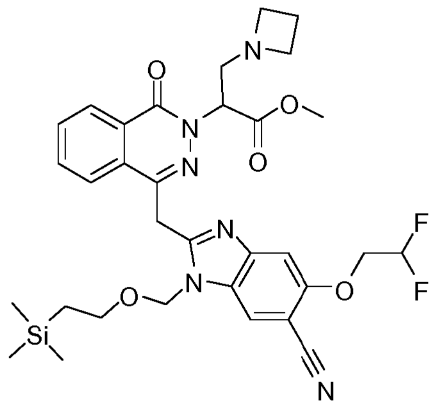 | Int. 303 | AP | 653 | 654 |
| 243 | 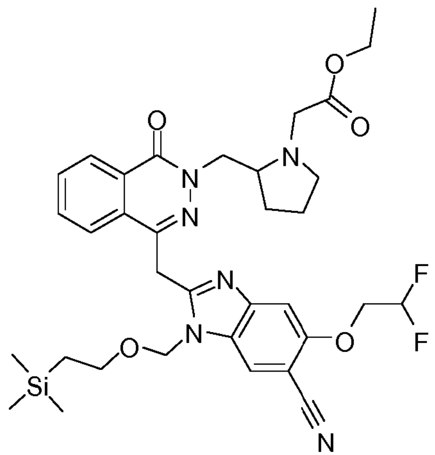 | Int.240 | AQ | 681 | 682 |

(continued)

| Int # | Structure | Starting material | Mtd | MW (calc) | MW (obs) |
|---|---|---|---|---|---|
| 244 | 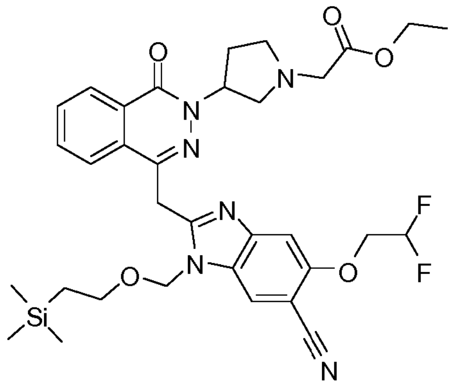 | Int.94 | AQ | 667 | 668 |
| 245 | 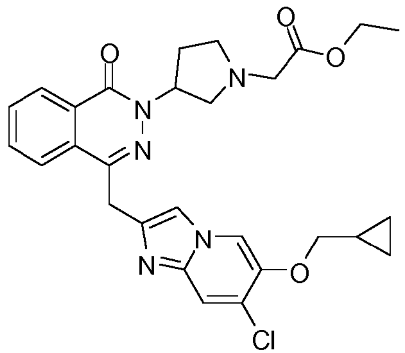 | Int. 442 | AQ | 536 | 536 |

(continued)

| Int # | Structure | Starting material | Mtd | MW (calc) | MW (obs) |
|---|---|---|---|---|---|
| 246 | 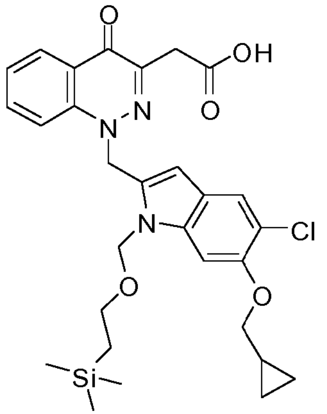 | Int.109 | AR | 568 | 569 |
| 247 | 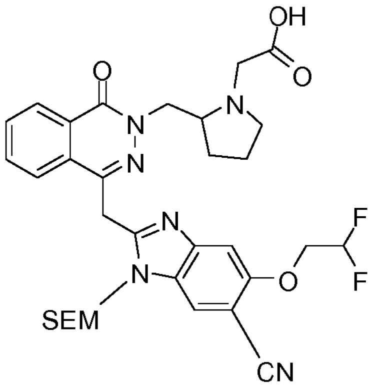 | Int.243 | AR | 653 | 654 |

(continued)

| Int # | Structure | Starting material | Mtd | MW (calc) | MW (obs) |
|---|---|---|---|---|---|
| 248 | 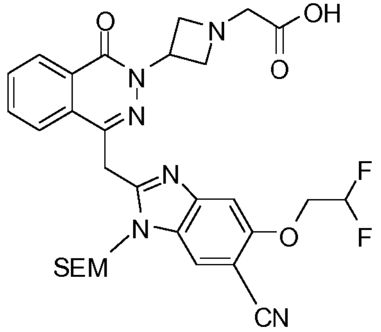 | Int.107 | AR | 625 | 625 |
| 249 | 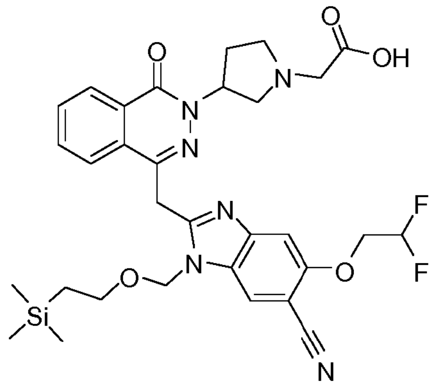 | Int.244 | AR | 639 | 639 |

(continued)

| Int # | Structure | Starting material | Mtd | MW (calc) | MW (obs) |
|---|---|---|---|---|---|
| 250 | 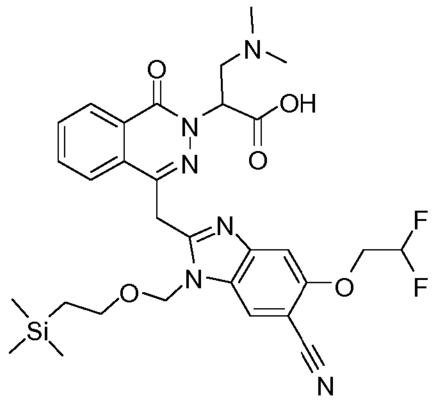 | Int.106 | AR | 627 | 627 |
| 251 | 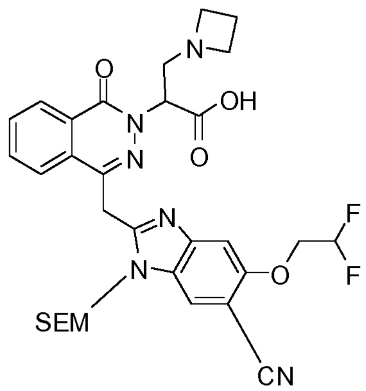 | Int.242 | AR | 639 | 640 |

(continued)

| Int # | Structure | Starting material | Mtd | MW (calc) | MW (obs) |
|---|---|---|---|---|---|
| 252 | 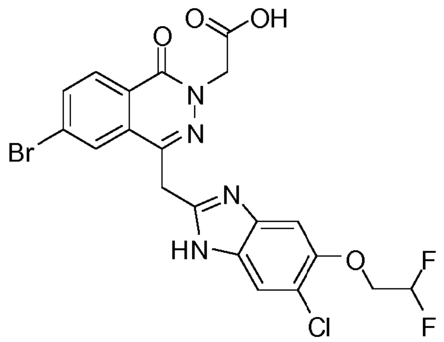 | Int.202 | AS | 528 | 527 |
| 253 | 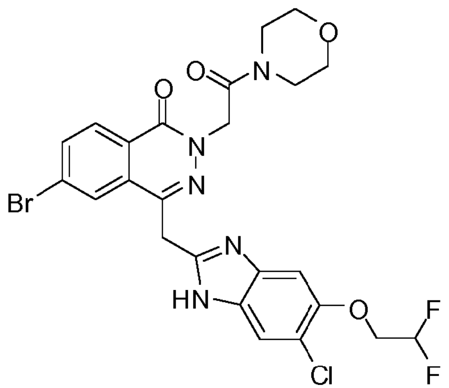 | Int.252 | AU | 597 | 596 |
| 254 | 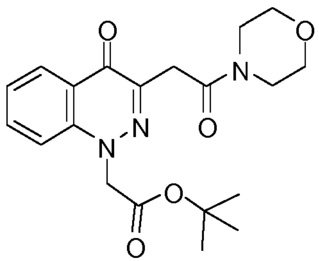 | Int.47 | AU | 387 | 388 |

(continued)

| Int # | Structure | Starting material | Mtd | MW (calc) | MW (obs) |
|---|---|---|---|---|---|
| 255 | 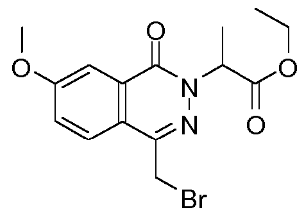 | Int.112 | B | 369 | 371 |
| 256 | 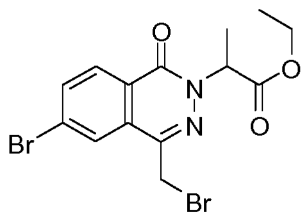 | Int.111 | B | 418 | 419 |
| 257 | 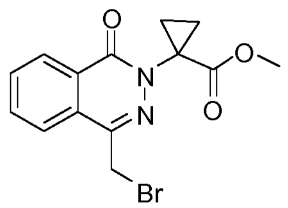 | Int.102 | B | 337 | 337 |
| 258 | 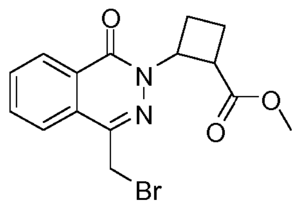 | Int.100 | B | 351 | 351 |

(continued)

| Int # | Structure | Starting material | Mtd | MW (calc) | MW (obs) |
|---|---|---|---|---|---|
| 259 | 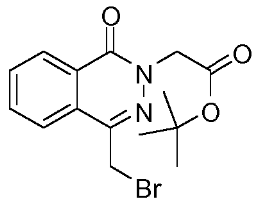 | Int.113 | B | 353 | 353 |
| 260 | 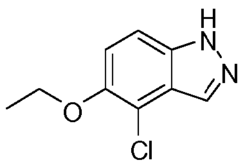 | 6-bromo-1H-indazol-5-ol | C | 197 | 197 |
| 261 | 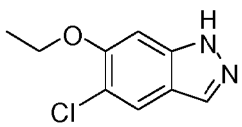 | 6-chloro-1H-indazol-5-ol | C | 197 | 197 |
| 262 | 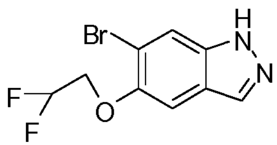 | 6-bromo-1H-indazol-5-ol | C | 277 | 277 |
| 263 | 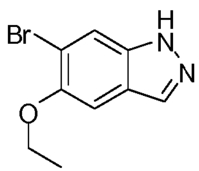 | 6-bromo-1H-indazol-5-ol | C | 241 | 239 |
| 264 | 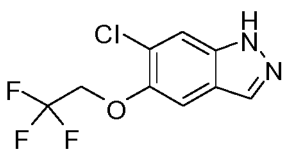 | 6-bromo-1H-indazol-5-ol | C | 251 | 251 |

(continued)

| Int # | Structure | Starting material | Mtd | MW (calc) | MW (obs) |
|---|---|---|---|---|---|
| 265 | 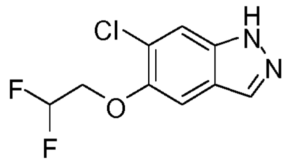 | 6-chloro-1H-indazol-5-ol | C | 233 | 233 |
| 266 | 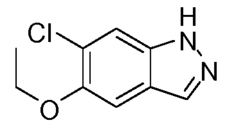 | 6-chloro-1H-indazol-5-ol | C | 197 | 197 |
| 267 | 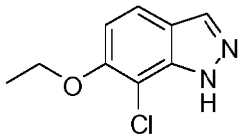 | 7-chloro-1H-indazol-6-ol | C | 197 | 197 |
| 268 | 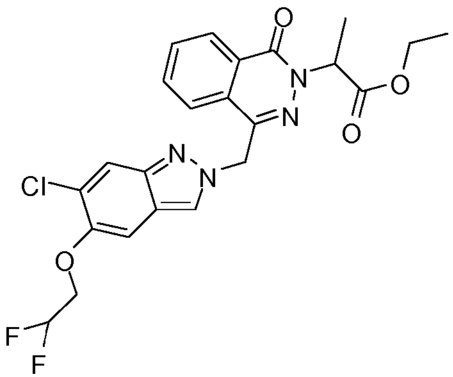 | Int.3 | D | 491 | 491 |

(continued)

| Int # | Structure | Starting material | Mtd | MW (calc) | MW (obs) |
|---|---|---|---|---|---|
| 269 | 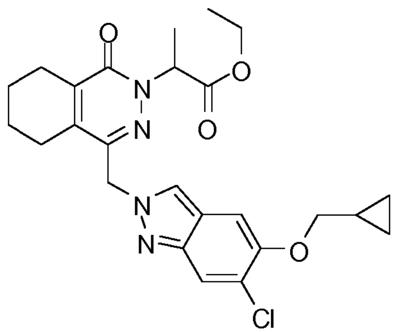 | Int.37 | D | 485 | 485 |
| 270 | 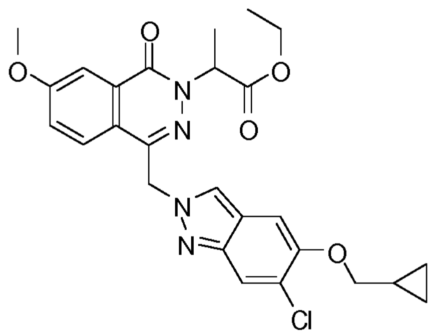 | Int.255 | D | 511 | 511 |
| 271 | 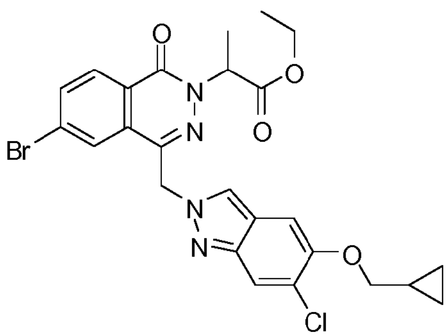 | Int.256 | D | 560 | 561 |

(continued)

| Int # | Structure | Starting material | Mtd | MW (calc) | MW (obs) |
|---|---|---|---|---|---|
| 272 | 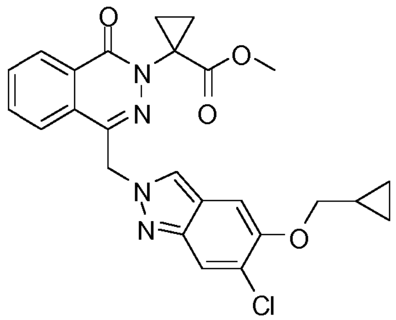 | Int.257 | D | 479 | 479 |
| 273 | 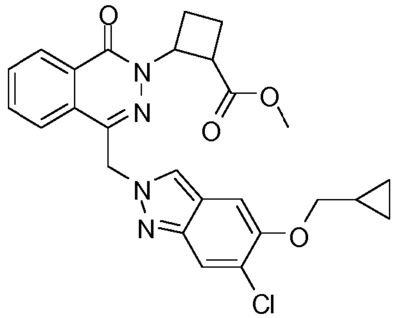 | Int.258 | D | 493 | 493 |
| 274 | 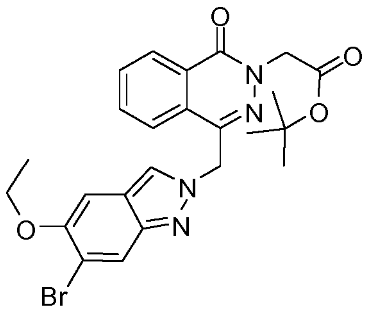 | Int.259 | D | 513 | 513 |

(continued)

| Int # | Structure | Starting material | Mtd | MW (calc) | MW (obs) |
|---|---|---|---|---|---|
| 275 | 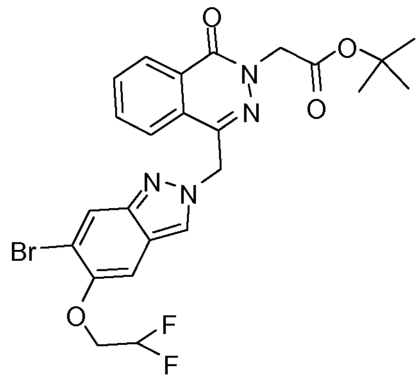 | Int.259 | D | 549 | 549 |
| 276 | 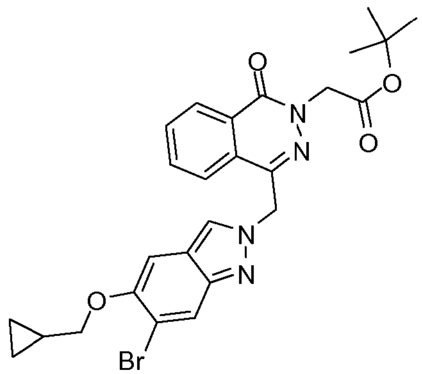 | Int.259 | D | 539 | 539 |

(continued)

| Int # | Structure | Starting material | Mtd | MW (calc) | MW (obs) |
|---|---|---|---|---|---|
| 277 | 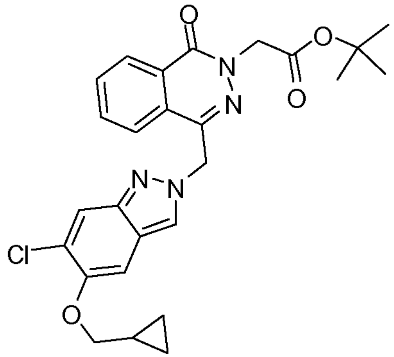 | Int.259 | D | 495 | 495 |
| 278 | 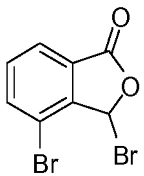 | 4-bromo-3H-isobenzofuran-1-one | E | 292 | 291 |
| 279 | 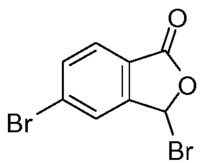 | 5-bromo-3H-isobenzofuran-1-one | E | 292 | NA |
| 280 | 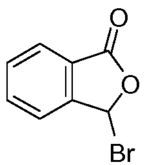 | 3H-isobenzofuran-1-one | E | 213 | NA |

(continued)

| Int # | Structure | Starting material | Mtd | MW (calc) | MW (obs) |
|---|---|---|---|---|---|
| 281 | 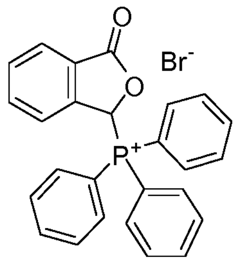 | Int.280 | F | 475 | 395 |
| 282 | 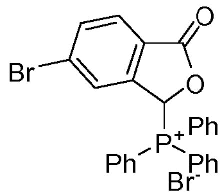 | Int.279 | F | 554 | 475 |
| 283 | 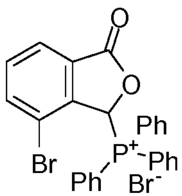 | Int.278 | F | 554 | 475 |
| 284 | 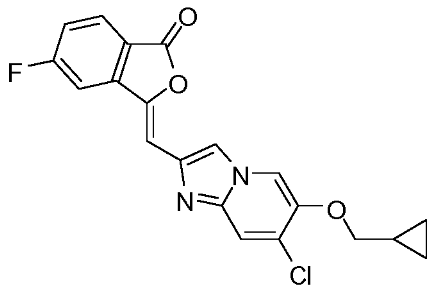 | Int. 8 | G | 385 | 385 |

(continued)

| Int # | Structure | Starting material | Mtd | MW (calc) | MW (obs) |
|---|---|---|---|---|---|
| 285 | 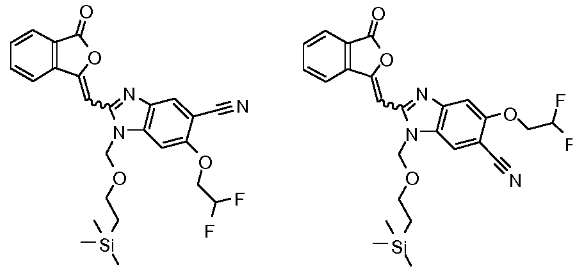 E/Z | Int.125 | G | 498 | 498 |
| 286 | 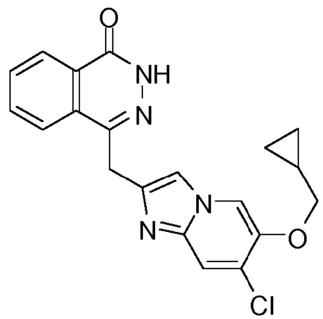 | Int.281 | G&I | 381 | 382 |
| 287 | 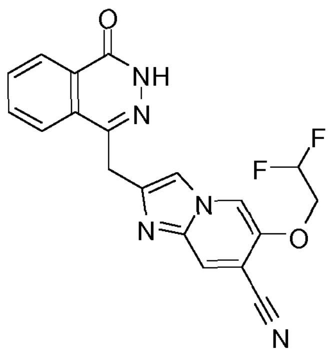 | Int.281 | G&I | 381 | 382 |

(continued)

| Int # | Structure | Starting material | Mtd | MW (calc) | MW (obs) |
|---|---|---|---|---|---|
| 288 | 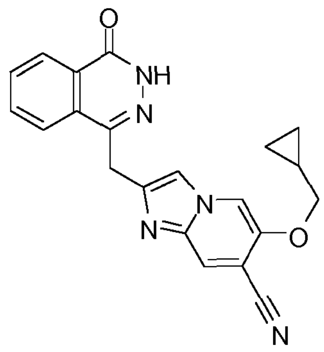 | Int.281 | G&I | 371 | 373 |
| 289 | 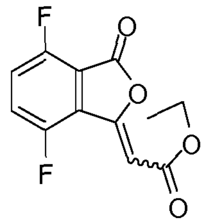 E/Z | 4,7-difluoroisoben zofuran-1,3-dione | H | 254 | 255 |
| 290 | 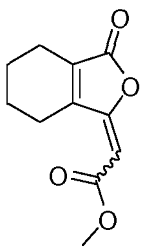 E/Z | 4,5,6,7-tetrahydroisob enzofuran-1,3-dione | H | 208 | 209 |

(continued)

| Int # | Structure | Starting material | Mtd | MW (calc) | MW (obs) |
|---|---|---|---|---|---|
| 291 | 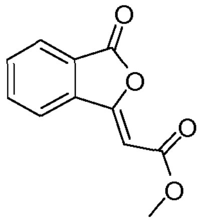 | isobenzofuran-1,3-dione | H | 204 | NA |
| 292 | 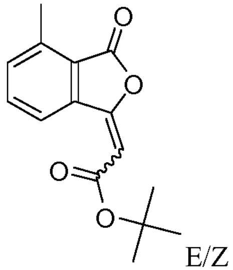 E/Z | 4-methylisobenz ofuran-1,3-dione | H | 260 | 205 |
| 293 | 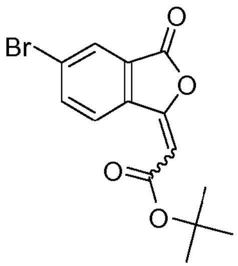 E/Z | 5-bromoisobenz ofuran-1,3-dione | H | 325 | 325 |

(continued)

| Int # | Structure | Starting material | Mtd | MW (calc) | MW (obs) |
|---|---|---|---|---|---|
| 294 | 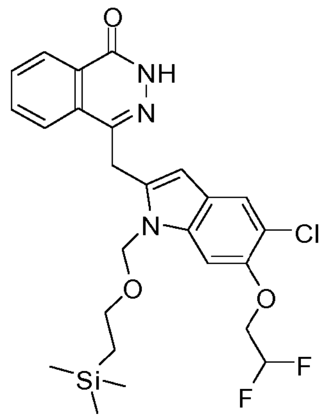 | Int.12 | I | 520 | 520 |
| 295 | 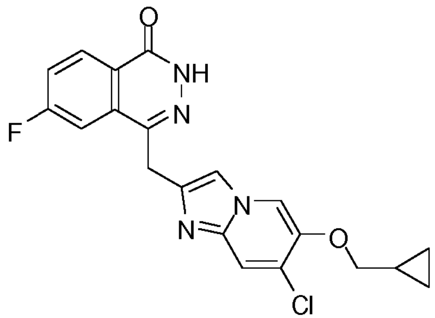 | Int.284 | I | 399 | 399 |
| 296 | 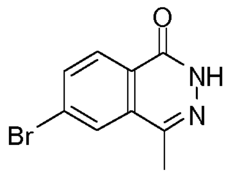 | Int.16 | I | 239 | 241 |

(continued)

| Int # | Structure | Starting material | Mtd | MW (calc) | MW (obs) |
|---|---|---|---|---|---|
| 297 | 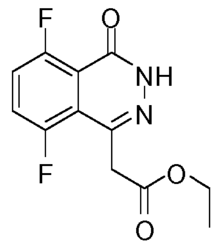 | Int.289 | I | 268 | 269 |
| 298 | 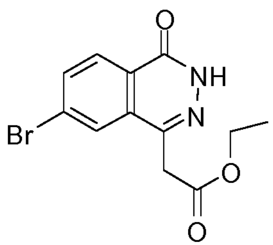 | Int.11 | I | 311 | 311 |
| 299 | 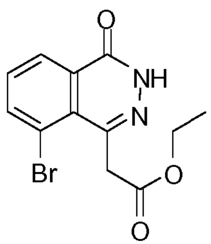 | Int.13 | I | 311 | 311 |
| 300 | 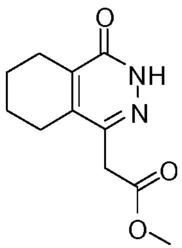 | Int.290 | I | 222 | 223 |

(continued)

| Int # | Structure | Starting material | Mtd | MW (calc) | MW (obs) |
|---|---|---|---|---|---|
| 301 | 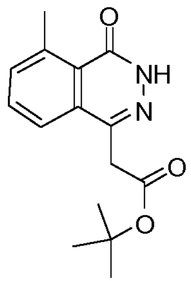 | Int.292 | I | 274 | 275 |
| 302 | 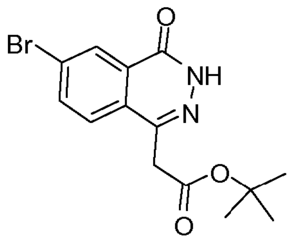 | Int.293 | I | 339 | 339 |
| 303 | 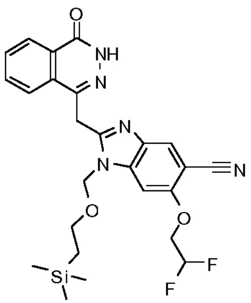 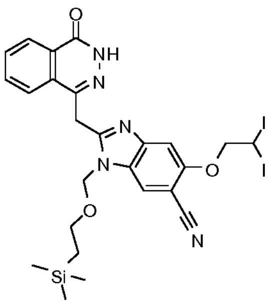 | Int.285 | I | 512 | 512 |

(continued)

| Int # | Structure | Starting material | Mtd | MW (calc) | MW (obs) |
|---|---|---|---|---|---|
| 304 | 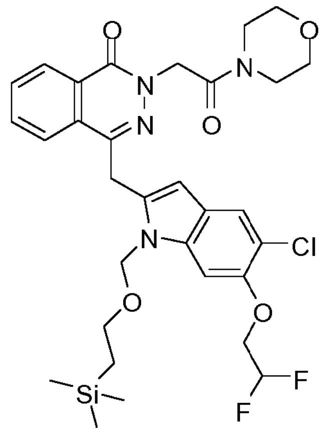 | Int.294 | J | 647 | 647 |
| 305 | 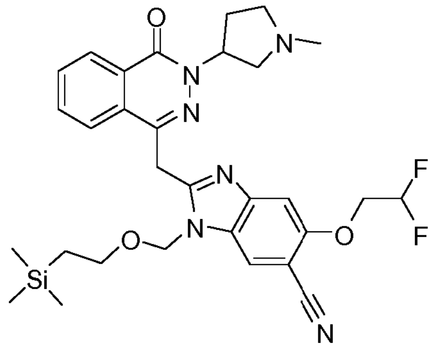 | Int. 303 | J | 595 | 595 |
| 306 | 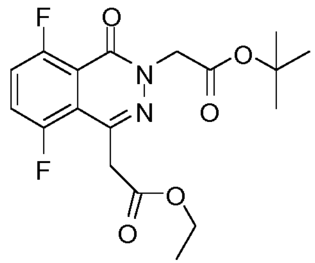 | Int.297 | J | 382 | 383 |

(continued)

| Int # | Structure | Starting material | Mtd | MW (calc) | MW (obs) |
|---|---|---|---|---|---|
| 307 | 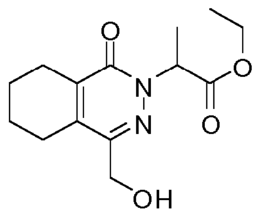 | Int.36 | J | 280 | 281 |
| 308 | 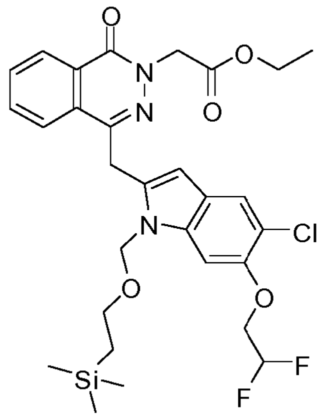 | Int.294 | J | 606 | 606 |
| 309 | 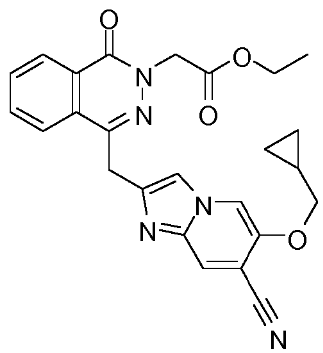 | Int.288 | J | 457 | 459 |

(continued)

| Int # | Structure | Starting material | Mtd | MW (calc) | MW (obs) |
|---|---|---|---|---|---|
| 310 | 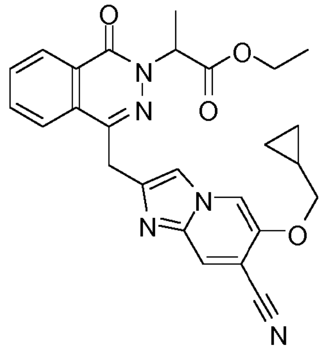 | Int.288 | J | 472 | 473 |
| 311 | 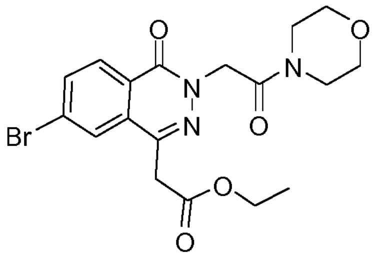 | Int.298 | J | 438 | 438 |
| 312 | 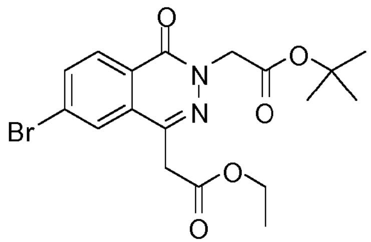 | Int.298 | J | 425 | 425 |

(continued)

| Int # | Structure | Starting material | Mtd | MW (calc) | MW (obs) |
|---|---|---|---|---|---|
| 313 | 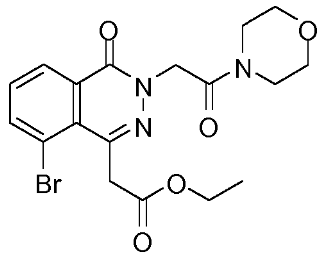 | Int.299 | J | 438 | 440 |
| 314 | 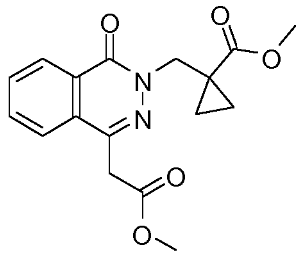 | Int.18 | J | 330 | 331 |
| 315 | 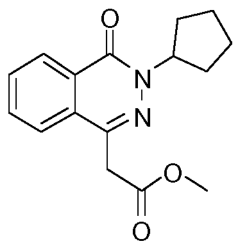 | Int.18 | J | 286 | 287 |
| 316 | 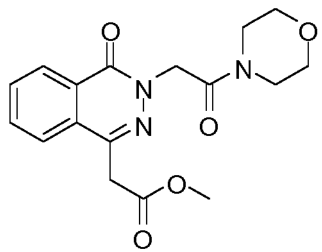 | Int.18 | J | 345 | 346 |

(continued)

| Int # | Structure | Starting material | Mtd | MW (calc) | MW (obs) |
|---|---|---|---|---|---|
| 317 | 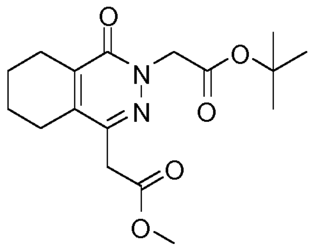 | Int.300 | J | 336 | 337 |
| 318 | 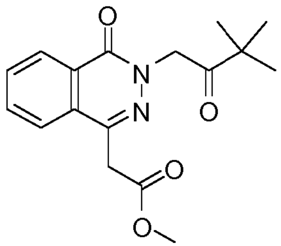 | Int.18 | J | 316 | 317 |
| 319 | 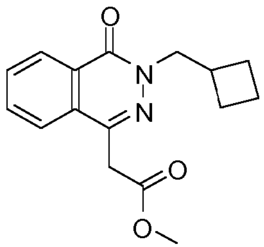 | Int.18 | J | 286 | 287 |
| 320 | 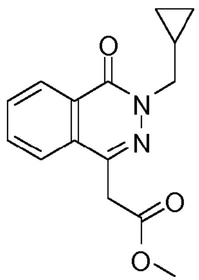 | Int.18 | J | 272 | 273 |

(continued)

| Int # | Structure | Starting material | Mtd | MW (calc) | MW (obs) |
|---|---|---|---|---|---|
| 321 | 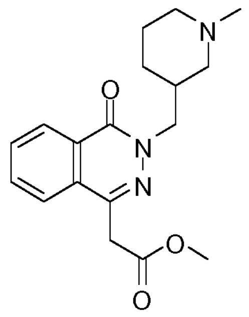 | Int.18 | J | 329 | 330 |
| 322 | 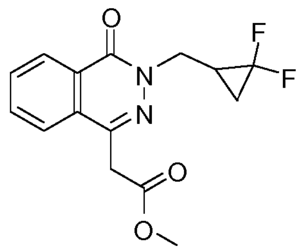 | Int.18 | J | 308 | 309 |
| 323 | 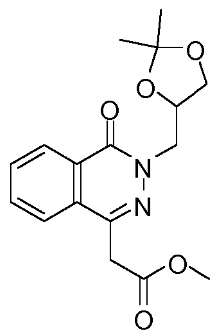 | Int.18 | J | 332 | NA |

(continued)

| Int # | Structure | Starting material | Mtd | MW (calc) | MW (obs) |
|---|---|---|---|---|---|
| 324 | 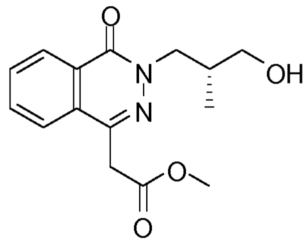 | Int.18 | J | 290 | 291 |
| 325 | 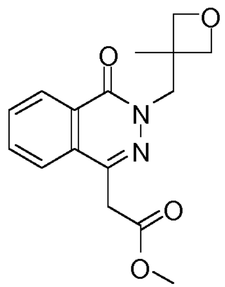 | Int.18 | J | 302 | 303 |
| 326 | 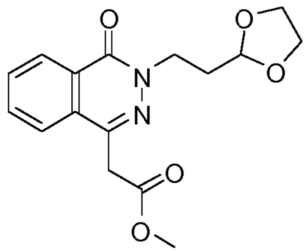 | Int.18 | J | 318 | 319 |

(continued)

| Int # | Structure | Starting material | Mtd | MW (calc) | MW (obs) |
|---|---|---|---|---|---|
| 327 | 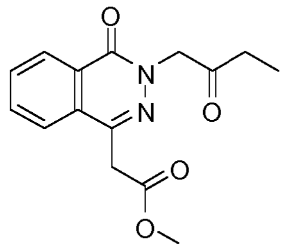 | Int.18 | J | 288 | 289 |
| 328 | 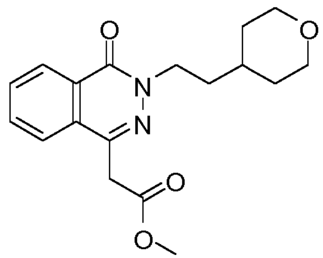 | Int.18 | J | 330 | 331 |
| 329 | 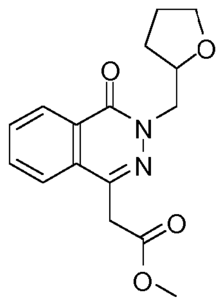 | Int.18 | J | 302 | 303 |

(continued)

| Int # | Structure | Starting material | Mtd | MW (calc) | MW (obs) |
|---|---|---|---|---|---|
| 330 | 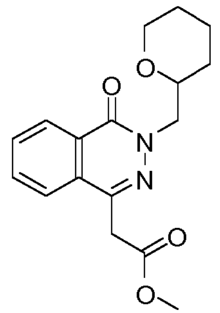 | Int.18 | J | 316 | 317 |
| 331 | 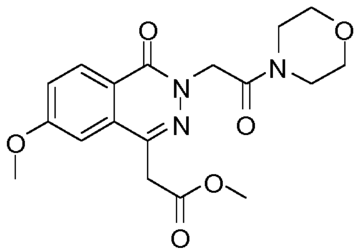 | Int.27 | J | 375 | 376 |
| 332 | 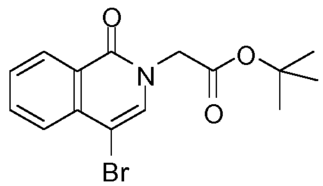 | 4-bromo-2H-isoquinolin-1-one | J | 338 | 282 [-tBu] |

(continued)

| Int # | Structure | Starting material | Mtd | MW (calc) | MW (obs) |
|---|---|---|---|---|---|
| 333 | 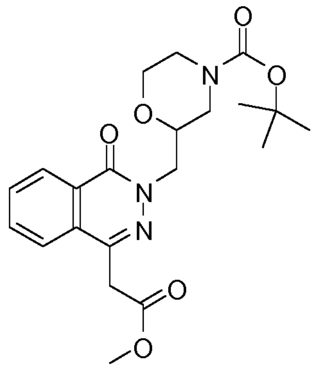 | Int.18 | J | 417 | 418 |
| 334 | 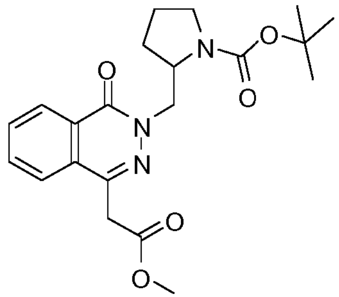 | Int.18 | J | 401 | 402 |

(continued)

| Int # | Structure | Starting material | Mtd | MW (calc) | MW (obs) |
|---|---|---|---|---|---|
| 335 | 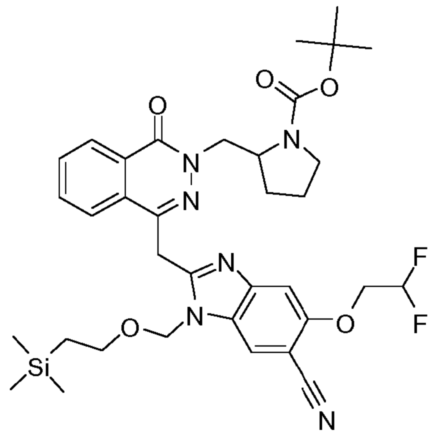 | Int. 303 | J | 695 | 696 |
| 336 | 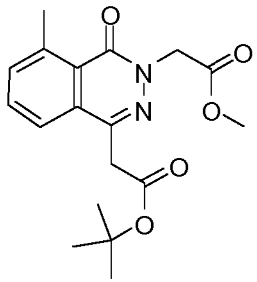 | Int.301 | J | 346 | 347 |
| 337 | 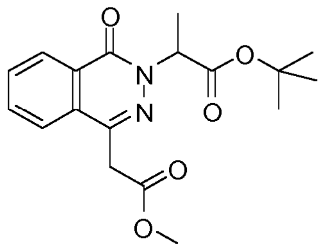 | Int.18 | J | 346 | 347 |

(continued)

| Int # | Structure | Starting material | Mtd | MW (calc) | MW (obs) |
|---|---|---|---|---|---|
| 338 | 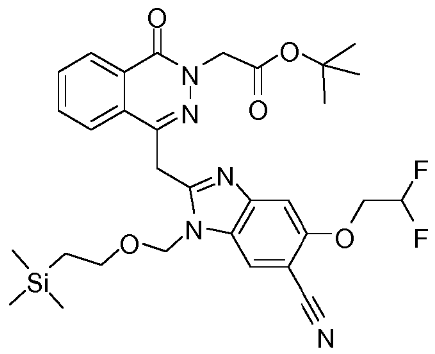 | Int. 303 | J | 626 | 626 |
| 339 | 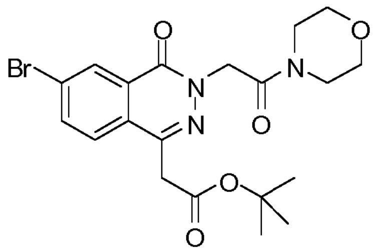 | Int.339 | J | 466 | 438 |
| 340 | 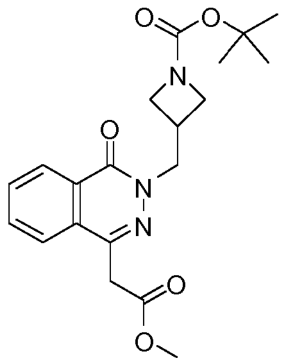 | Int.18 | J | 387 | 388 |

(continued)

| Int # | Structure | Starting material | Mtd | MW (calc) | MW (obs) |
|---|---|---|---|---|---|
| 341 | 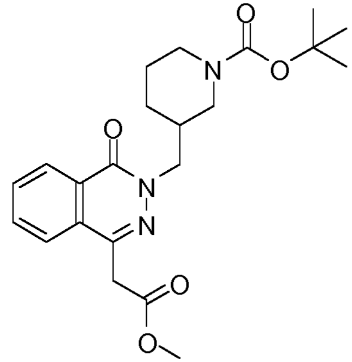 | Int.18 | J | 415 | 416 |
| 342 | 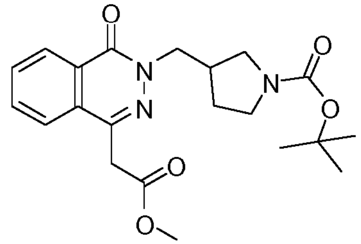 | Int.18 | J | 401 | 402 |
| 343 | 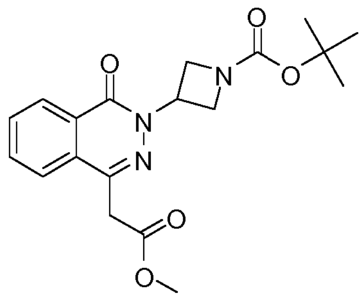 | Int.18 | J | 373 | 374 |

(continued)

| Int # | Structure | Starting material | Mtd | MW (calc) | MW (obs) |
|---|---|---|---|---|---|
| 344 | 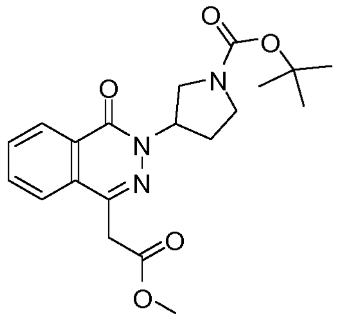 | Int.18 | J | 387 | 388 |
| 345 | 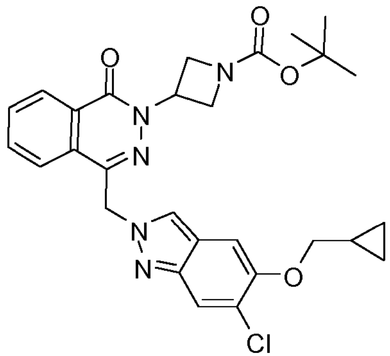 | Int.58 | J | 536 | 537 |

(continued)

| Int # | Structure | Starting material | Mtd | MW (calc) | MW (obs) |
|---|---|---|---|---|---|
| 346 | 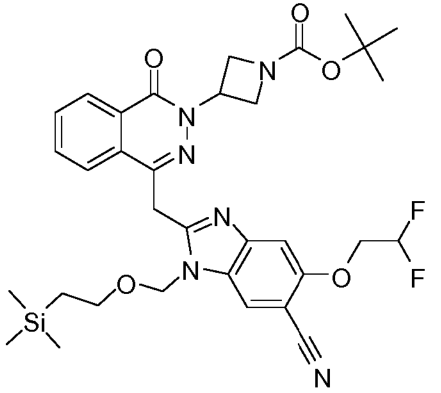 | Int. 303 | J | 667 | 667 |
| 347 | 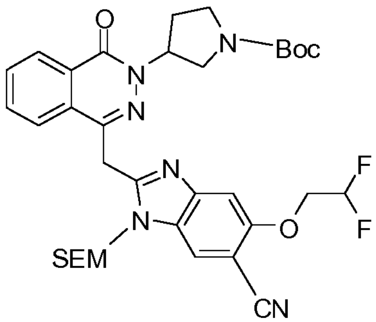 | Int. 303 | J | 681 | 682 |
| 348 | 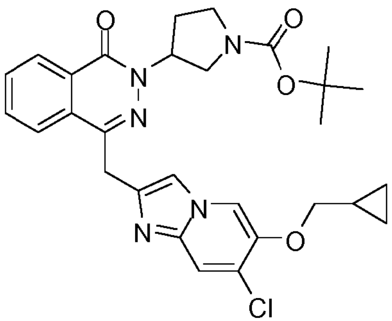 | Int.286 | J | 550 | 550 |

(continued)

| Int # | Structure | Starting material | Mtd | MW (calc) | MW (obs) |
|---|---|---|---|---|---|
| 349 | 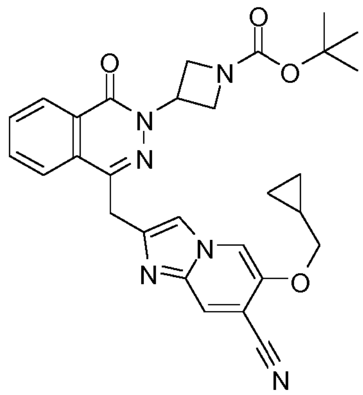 | Int.288 | J | 527 | 528 |
| 350 | 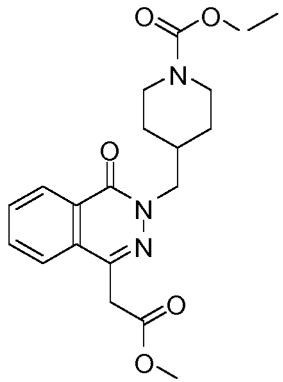 | Int.18 | J | 415 | 416 |

(continued)

| Int # | Structure | Starting material | Mtd | MW (calc) | MW (obs) |
|---|---|---|---|---|---|
| 351 | 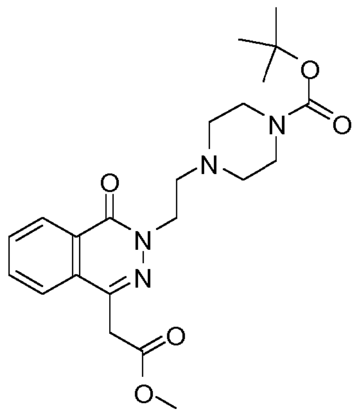 | Int.18 | J | 431 | 431 |
| 352 | 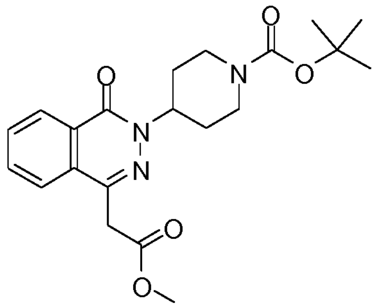 | Int.18 | J | 401 | 402 |
| 353 | 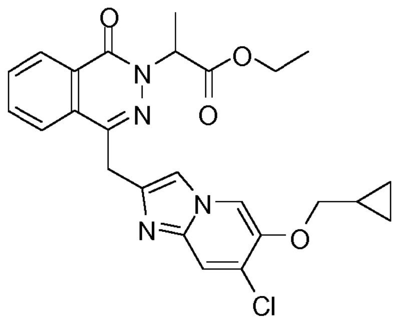 | Int.286 | K | 481 | 482 |

(continued)

| Int # | Structure | Starting material | Mtd | MW (calc) | MW (obs) |
|---|---|---|---|---|---|
| 354 | 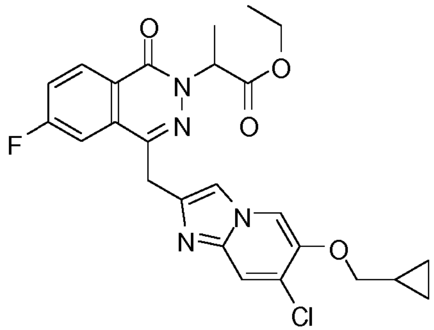 | Int.295 | K | 499 | 499 |
| 355 | 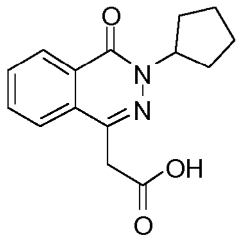 | Int.315 | L | 272 | 273 |
| 356 | 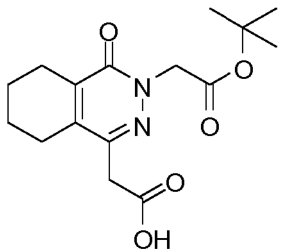 | Int.317 | L | 322 | 323 |

(continued)

| Int # | Structure | Starting material | Mtd | MW (calc) | MW (obs) |
|---|---|---|---|---|---|
| 357 | 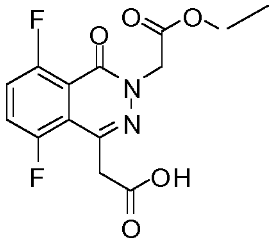 | Int.306 | L | 354 | 299 |
| 358 | 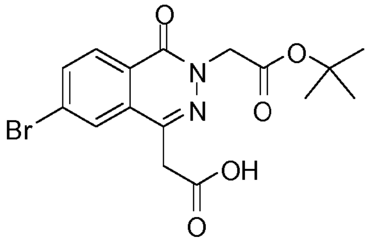 | Int.312 | L | 397 | 341 |
| 359 | 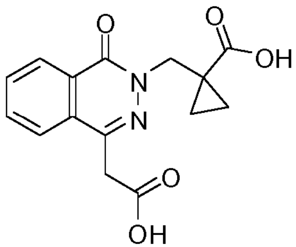 | Int.314 | M | 302 | 303 |
| 360 | 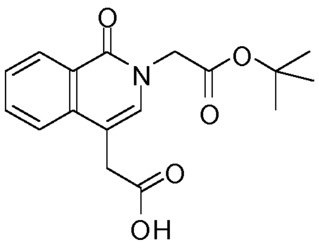 | Int.44 | M | 317 | 340 [M+Na]+ |

(continued)

| Int # | Structure | Starting material | Mtd | MW (calc) | MW (obs) |
|---|---|---|---|---|---|
| 361 | 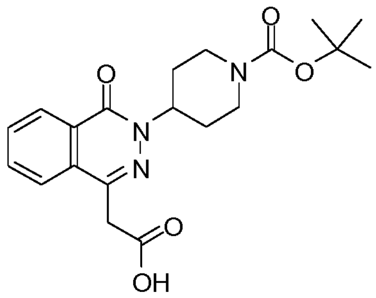 | Int.352 | M | 387 | 388 |
| 362 | 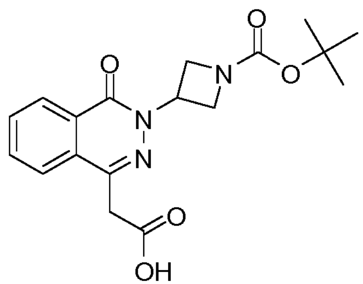 | Int.343 | M | 359 | 360 |
| 363 | 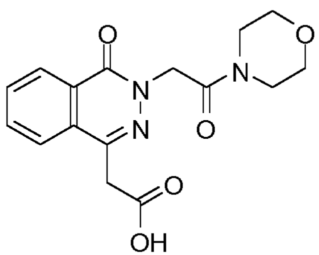 | Int.316 | M | 331 | 332 |

(continued)

| Int # | Structure | Starting material | Mtd | MW (calc) | MW (obs) |
|---|---|---|---|---|---|
| 364 | 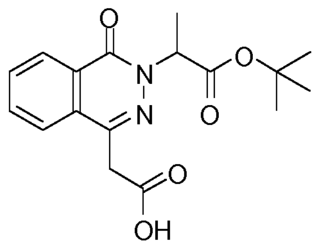 | Int.337 | M | 332 | 355 [M+Na]+ |
| 365 | 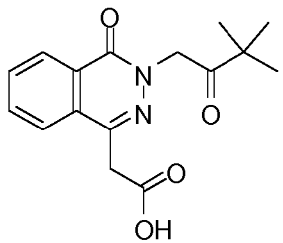 | Int.318 | M | 302 | 303 |
| 366 | 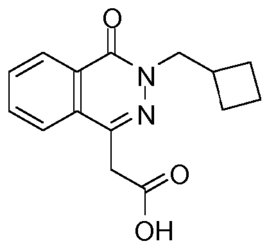 | Int.319 | M | 272 | 273 |
| 367 | 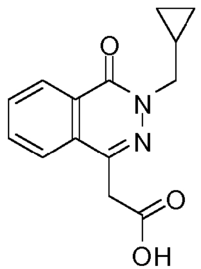 | Int.320 | M | 258 | 259 |

(continued)

| Int # | Structure | Starting material | Mtd | MW (calc) | MW (obs) |
|---|---|---|---|---|---|
| 368 | 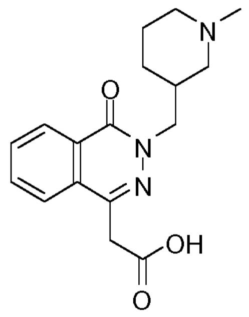 | Int.321 | M | 315 | 316 |
| 369 | 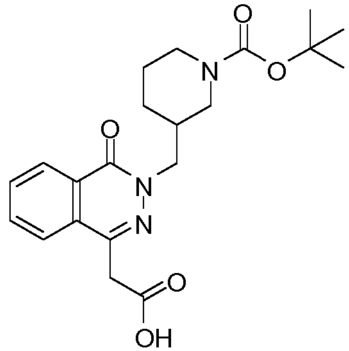 | Int.341 | M | 401 | 402 |
| 370 | 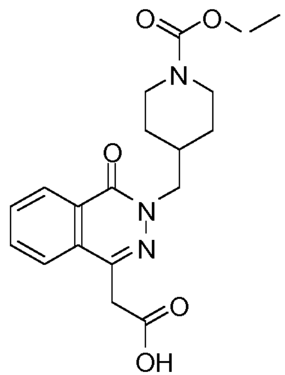 | Int.350 | M | 401 | 402 |

(continued)

| Int # | Structure | Starting material | Mtd | MW (calc) | MW (obs) |
|---|---|---|---|---|---|
| 371 | 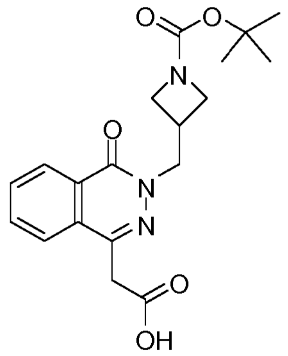 | Int.340 | M | 373 | 374 |
| 372 | 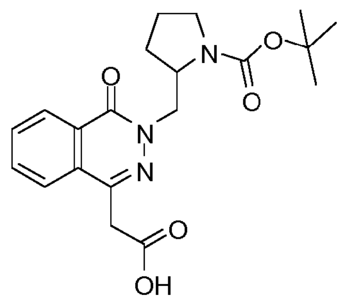 | Int.334 | M | 387 | 388 |
| 373 | 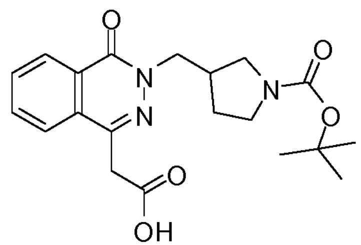 | Int.342 | M | 387 | 388 |

(continued)

| Int # | Structure | Starting material | Mtd | MW (calc) | MW (obs) |
|---|---|---|---|---|---|
| 374 | 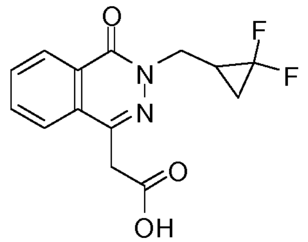 | Int.322 | M | 294 | 295 |
| 375 | 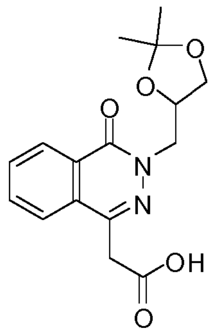 | Int.323 | M | 318 | 319 |
| 376 | 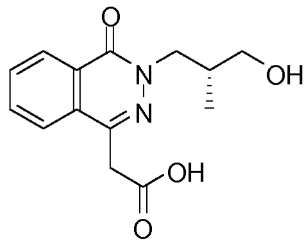 | Int.324 | M | 276 | 277 |

(continued)

| Int # | Structure | Starting material | Mtd | MW (calc) | MW (obs) |
|---|---|---|---|---|---|
| 377 | 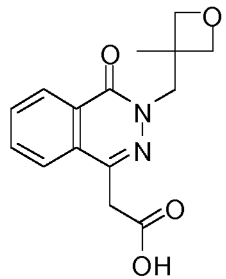 | Int.325 | M | 288 | 289 |
| 378 | 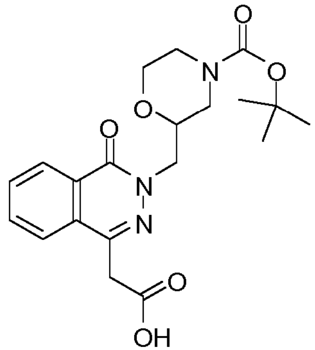 | Int.333 | M | 403 | NA |
| 379 | 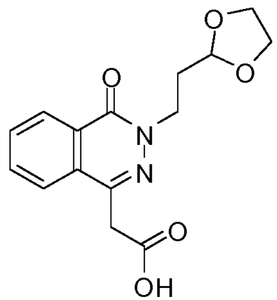 | Int.326 | M | 304 | 305 |

(continued)

| Int # | Structure | Starting material | Mtd | MW (calc) | MW (obs) |
|---|---|---|---|---|---|
| 380 | 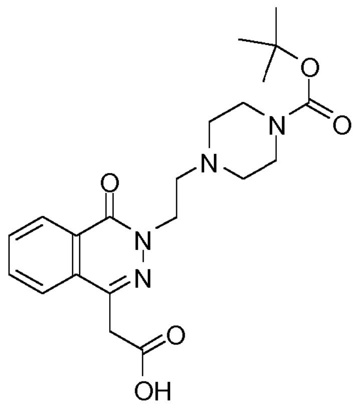 | Int.351 | M | 416 | 417 |
| 381 | 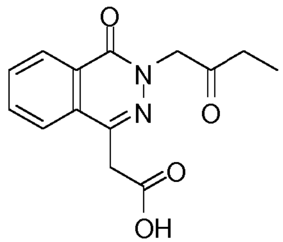 | Int.327 | M | 274 | 275 |
| 382 | 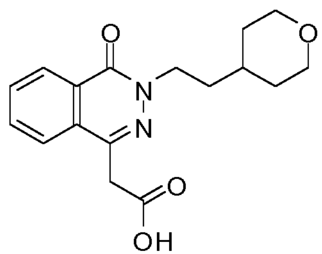 | Int.328 | M | 316 | 317 |

(continued)

| Int # | Structure | Starting material | Mtd | MW (calc) | MW (obs) |
|---|---|---|---|---|---|
| 383 | 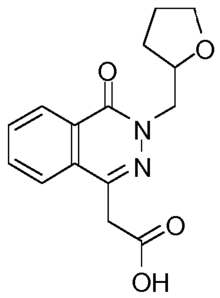 | Int.329 | M | 288 | 289 |
| 384 | 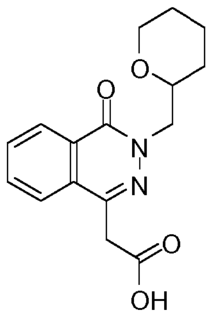 | Int.330 | M | 302 | 303 |
| 385 | 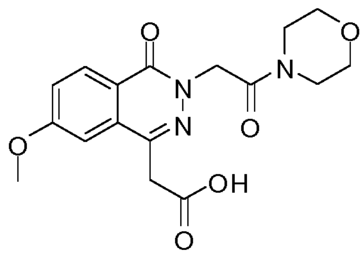 | Int.331 | M | 361 | 362 |

(continued)

| Int # | Structure | Starting material | Mtd | MW (calc) | MW (obs) |
|---|---|---|---|---|---|
| 386 | 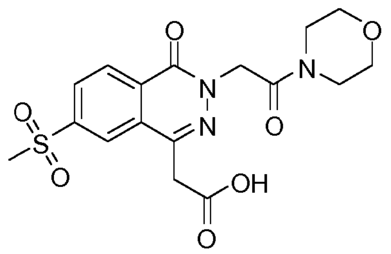 | Int.38 | M | 409 | 410 |
| 387 | 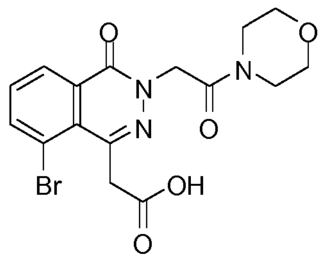 | Int.313 | M | 410 | 410 |
| 388 | 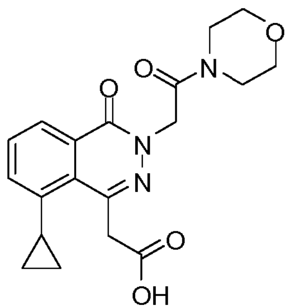 | Int.105 | M | 371 | 372 |

(continued)

| Int # | Structure | Starting material | Mtd | MW (calc) | MW (obs) |
|---|---|---|---|---|---|
| 389 | 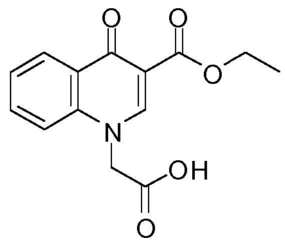 | Int.217 | N | 275 | 277 |
| 390 | 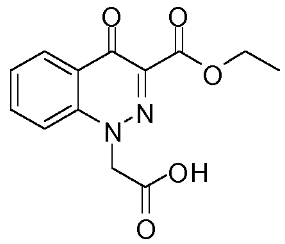 | Int.216 | N | 276 | 277 |
| 391 | 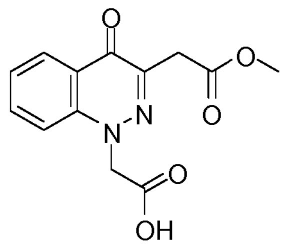 | Int.45 | N | 276 | 277 |
| 392 | 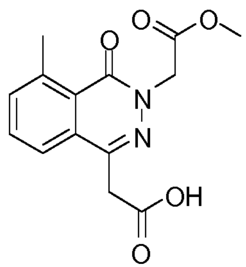 | Int.336 | N | 290 | 291 |

(continued)

| Int # | Structure | Starting material | Mtd | MW (calc) | MW (obs) |
|---|---|---|---|---|---|
| 393 | 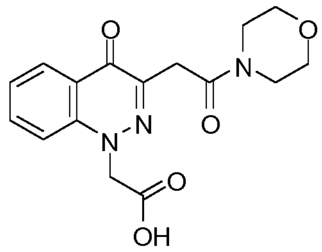 | Int.254 | N | 331 | 332 |
| 394 | 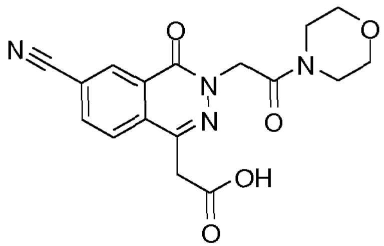 | Int.39 | N | 356 | 357 |
| 395 | 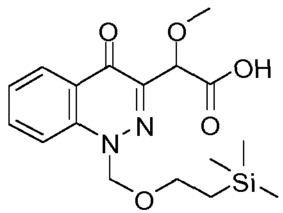 | Int.64 | NA | 364 | 365 |
| 396 | 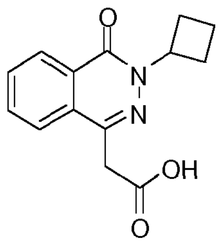 | Int.14 | O | 258 | 259 |

(continued)

| Int # | Structure | Starting material | Mtd | MW (calc) | MW (obs) |
|---|---|---|---|---|---|
| 397 | 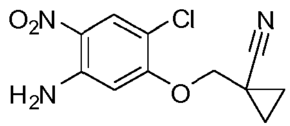 | 4-chloro-5-fluoro-2-nitro-aniline | P | 268 | 268 |
| 398 | 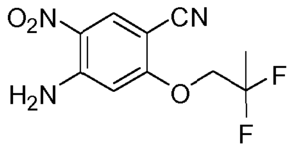 | 4-amino-2-fluoro-5-nitrobenzonitrile | P | 257 | 258 |
| 399 | 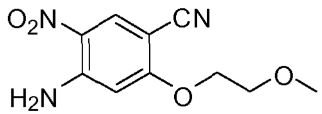 | 4-amino-2-fluoro-5-nitrobenzonitrile | P | 237 | 238 |
| 400 | 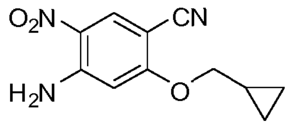 | 4-amino-2-fluoro-5-nitrobenzonitrile | P | 233 | 234 |
| 401 | 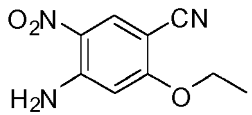 | 4-amino-2-fluoro-5-nitrobenzonitrile | P | 207 | NA |
| 402 | 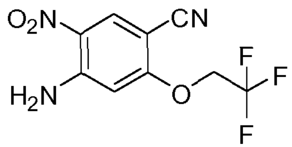 | 4-amino-2-fluoro-5-nitrobenzonitrile | P | 261 | 261 |

(continued)

| Int # | Structure | Starting material | Mtd | MW (calc) | MW (obs) |
|---|---|---|---|---|---|
| 403 | 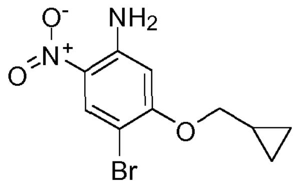 | 4-bromo-5-fluoro-2-nitro-aniline | P | 287 | 287 |
| 404 | 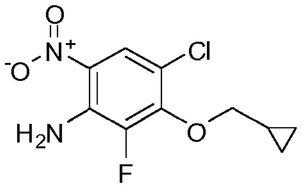 | 4-chloro-2,3-difluoro-6-nitro-aniline | P | 261 | 261 |
| 405 | 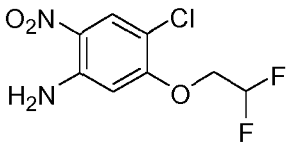 | 4-chloro-5-fluoro-2-nitro-aniline | P | 253 | 253 |
| 406 | 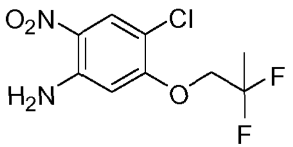 | 4-chloro-5-fluoro-2-nitro-aniline | P | 267 | 267 |
| 407 | 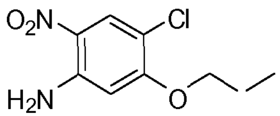 | 4-chloro-5-fluoro-2-nitro-aniline | P | 259 | 259 |
| 408 | 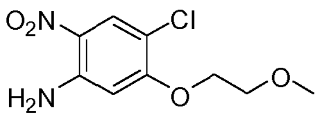 | 4-chloro-5-fluoro-2-nitro-aniline | P | 247 | 247 |

(continued)

| Int # | Structure | Starting material | Mtd | MW (calc) | MW (obs) |
|---|---|---|---|---|---|
| 409 | 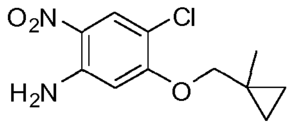 | 4-chloro-5-fluoro-2-nitro-aniline | P | 257 | 257 |
| 410 | 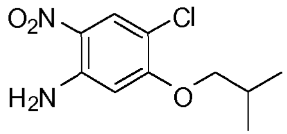 | 4-chloro-5-fluoro-2-nitro-aniline | P | 245 | 245 |
| 411 | 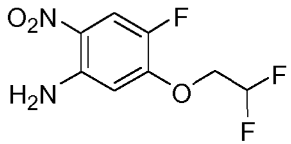 | 4,5-difluoro-2-nitro-aniline | P | 236 | 237 |
| 412 | 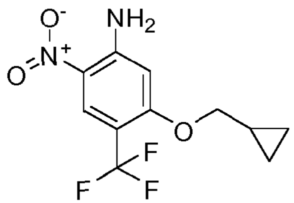 | 5-fluoro-2-nitro-4-(trifluorometh yl)aniline | P | 276 | 275 |
| 413 | 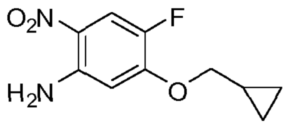 | 4,5-difluoro-2-nitro-aniline | P | 226 | 227 |
| 414 | 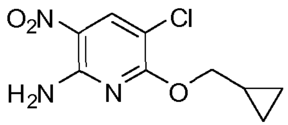 | 5,6-dichloro-3-nitro-pyridin-2-amine | P | 244 | 244 |

(continued)

| Int # | Structure | Starting material | Mtd | MW (calc) | MW (obs) |
|---|---|---|---|---|---|
| 415 | 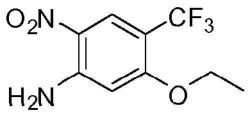 | 5-fluoro-2-nitro-4-(trifluorometh yl)aniline | P | 250 | NA |
| 416 | 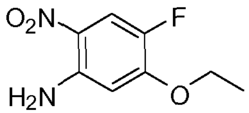 | 4,5-difluoro-2-nitro-aniline | P | 200 | 201 |
| 417 | 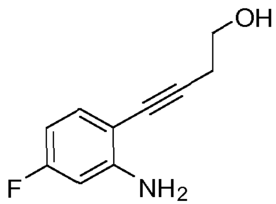 | 5-fluoro-2-iodo-aniline | Q | 179 | 180 |
| 418 | 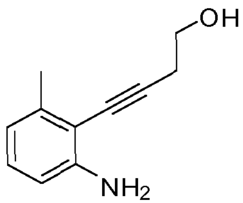 | 2-iodo-3-methyl-aniline | Q | 175 | 176 |
| 419 | 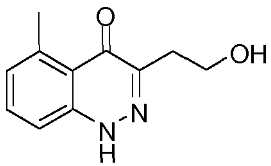 | Int.418 | R | 204 | 205 |
| 420 | 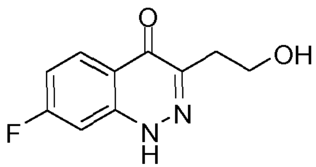 | Int.417 | R | 208 | 209 |

(continued)

| Int # | Structure | Starting material | Mtd | MW (calc) | MW (obs) |
|---|---|---|---|---|---|
| 421 | 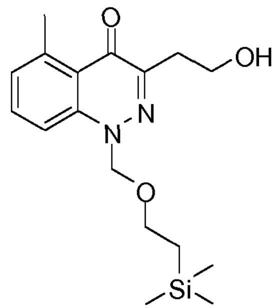 | Int.419 | S | 334 | 335 |
| 422 | 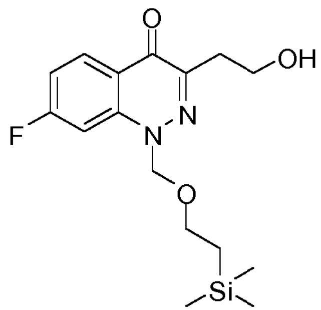 | Int.420 | S | 338 | 339 |
| 423 | 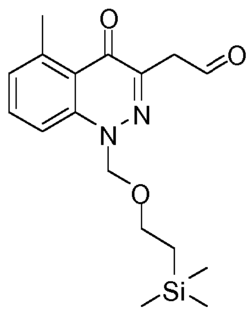 | Int.421 | T | 332 | 333 |

(continued)

| Int # | Structure | Starting material | Mtd | MW (calc) | MW (obs) |
|---|---|---|---|---|---|
| 424 | 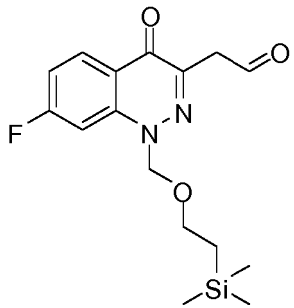 | Int.422 | T | 336 | 337 |
| 425 | 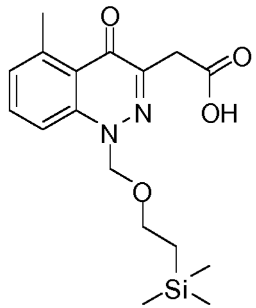 | Int.423 | U | 348 | 349 |
| 426 | 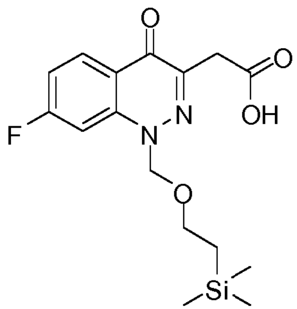 | Int.424 | U | 352 | 353 |

(continued)

| Int # | Structure | Starting material | Mtd | MW (calc) | MW (obs) |
|---|---|---|---|---|---|
| 427 | 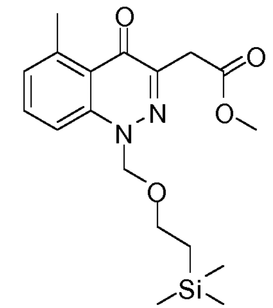 | Int.425 | V | 363 | 363 |
| 428 | 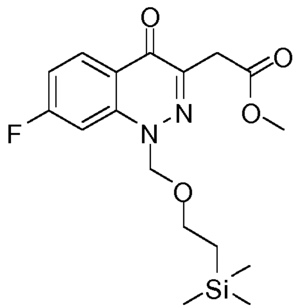 | Int.426 | V | 366 | 367 |
| 429 | 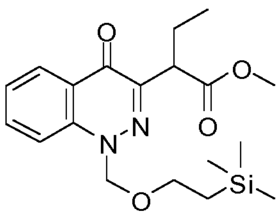 | Int.64 | W | 377 | 378 |
| 430 | 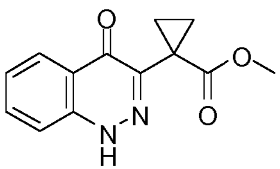 | Int.68 | X | 244 | 245 |

(continued)

| Int # | Structure | Starting material | Mtd | MW (calc) | MW (obs) |
|---|---|---|---|---|---|
| 431 | 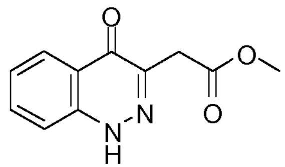 | Int.64 | X | 218 | 219 |
| 432 | 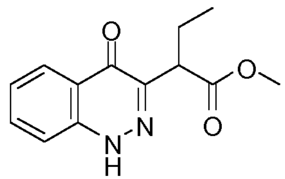 | Int.429 | X | 246 | 247 |
| 433 | 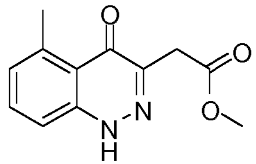 | Int.427 | X | 232 | 219 |
| 434 | 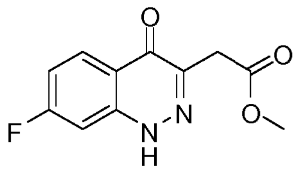 | Int.428 | X | 236 | 237 |
| 435 | 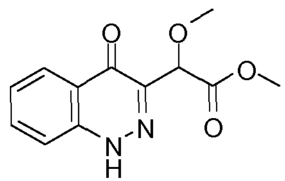 | Int.69 | X | 248 | 250 |

(continued)

| Int # | Structure | Starting material | Mtd | MW (calc) | MW (obs) |
|---|---|---|---|---|---|
| 436 | 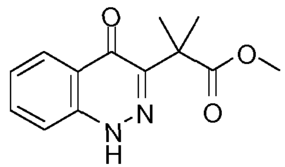 | Int.66 | X | 246 | 247 |
| 437 | 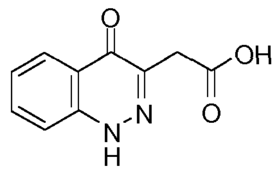 | Int.64 | X& AR | 204 | 205 |
| 438 | 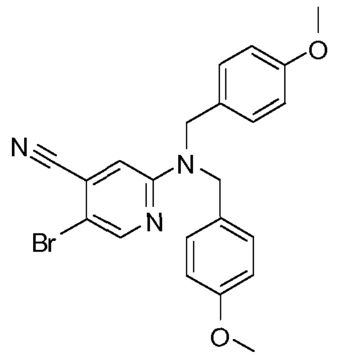 | 2-amino-5-bromopyridine-4-carbonitrile | Y | 438 | 438 |

(continued)

| Int # | Structure | Starting material | Mtd | MW (calc) | MW (obs) |
|---|---|---|---|---|---|
| 439 | 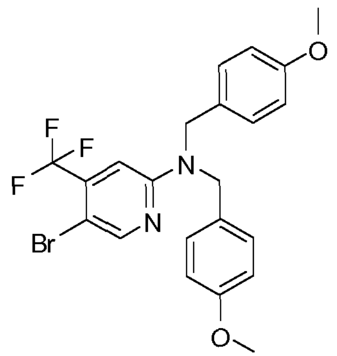 | 5-bromo-4-(trifluorometh yl)pyridin-2-amine | Y | 481 | 481 |
| 440 | 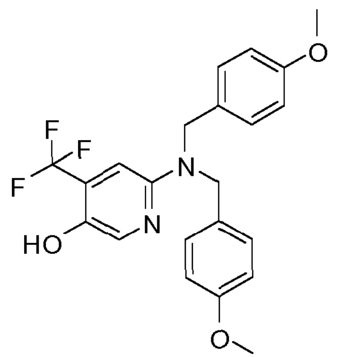 | Int.439 | Z | 418 | 420 |
| 441 | 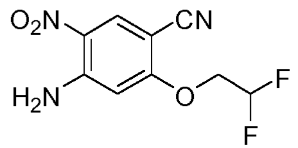 | 4-amino-2-fluoro-5-nitrobenzonitrile | P | 243 | NA |

(continued)

| Int # | Structure | Starting material | Mtd | MW (calc) | MW (obs) |
|---|---|---|---|---|---|
| 442 | 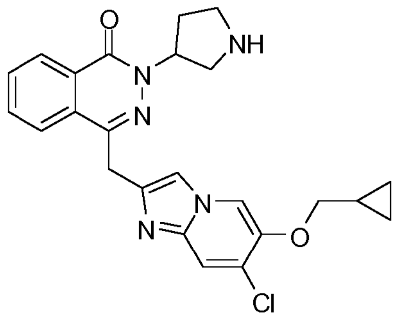 | Int. 348 | AO | 450 | 450 |
| 443 | 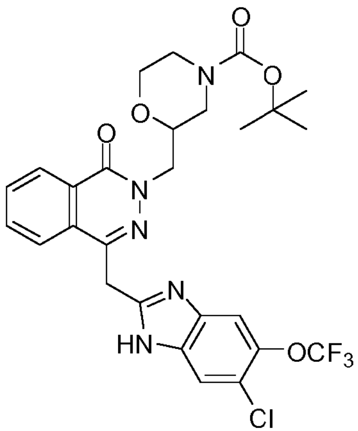 | Int. 378 | AJ | 594 | 594 |

(continued)

| Int # | Structure | Starting material | Mtd | MW (calc) | MW (obs) |
|---|---|---|---|---|---|
| 444 | 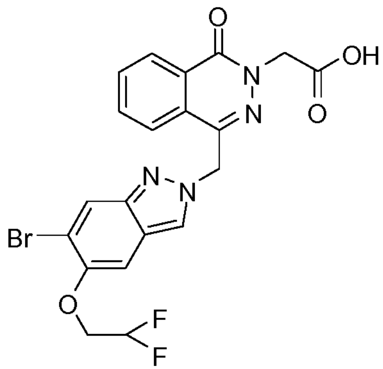 | Int. 275 | AS | 493 | 495 |
| 445 | 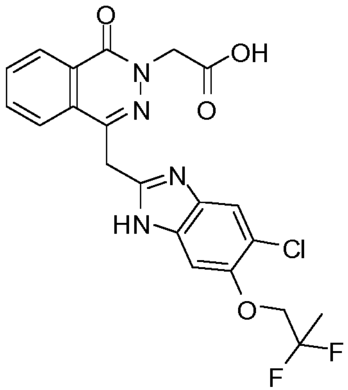 | Int. 185 | AS | 463 | 463 |

(continued)

| Int # | Structure | Starting material | Mtd | MW (calc) | MW (obs) |
|---|---|---|---|---|---|
| 446 | 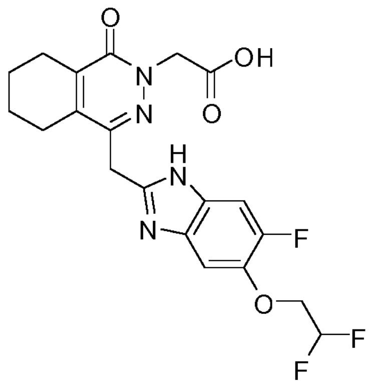 | Int. 179 | AS | 436 | 437 |
| 447 | 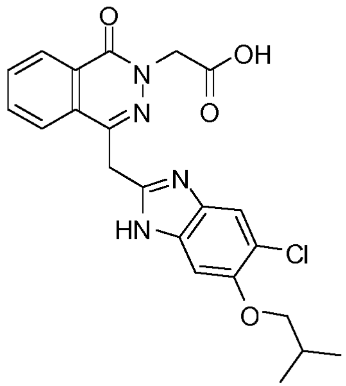 | Int. 174 | AS | 441 | 441 |

(continued)

| Int # | Structure | Starting material | Mtd | MW (calc) | MW (obs) |
|---|---|---|---|---|---|
| 448 | 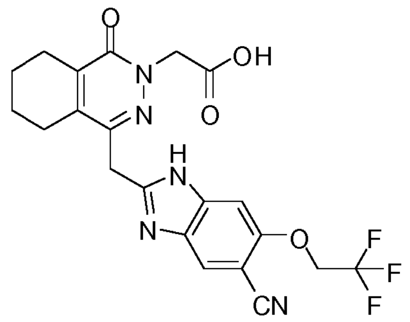 | Int. 195 | AS | 461 | 462 |
| 449 | 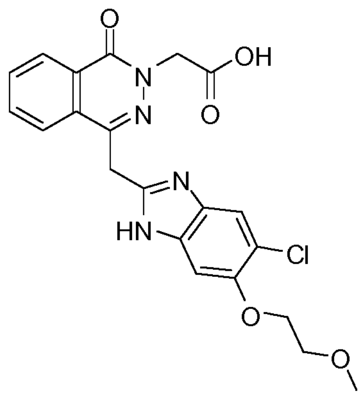 | Int. 187 | AS | 443 | 443 |
| 450 | 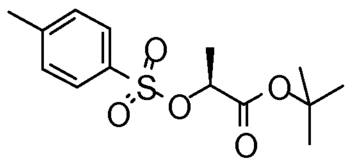 | L-(-)-O-Tosyllactic acid ethyl ester | NA | 300 | 318, 322 |

**Table III. Illustrative compounds of the invention** (Cpd# 80 is a reference compound)

| Cpd # | Structure | Name | Int | Mtd | MW (calc) | MW (obs) |
|---|---|---|---|---|---|---|
| 1 | 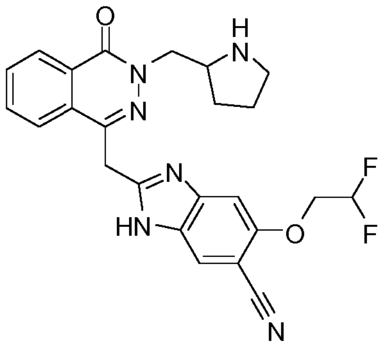 | 6-(2,2-difluoroethoxy)-2-[[4-oxo-3-(pyrrolidin-2-ylmethyl) phthalazin-1-yl]methyl]-3H-benzimidazole-5-carbonitrile | Int.335 | AM | 464 | 465 |
| 2 | 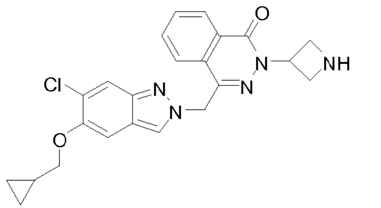 | 2-(azetidin-3-yl)-4-[[6-chloro-5-(cyclopropylmeth oxy)indazol-2-yl] methyl]phthala zin-1-one | Int.345 | AO | 436 | 437 |
| 3 | 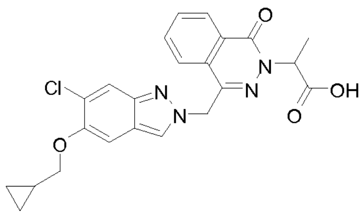 | 2-[4-[[6-chloro-5-(cyclopropylmeth oxy)indazol-2-yl]methyl]-1-oxo-phthalazin-2-yl]propanoic acid | Int.6 | AR | 453 | 453 |

(continued)

| Cpd # | Structure | Name | Int | Mtd | MW (calc) | MW (obs) |
|---|---|---|---|---|---|---|
| 4 | 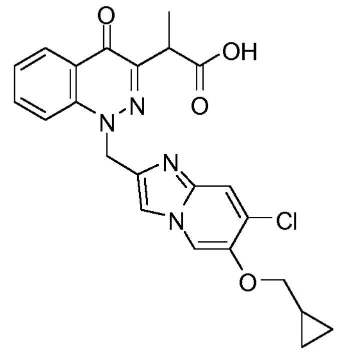 | 2-[1-[[7-chloro-6-(cyclopropylmeth oxy)imidazo [1,2-a]pyridin-2-yl] methyl]-4-oxo-cinnolin-3-yl]propanoic acid | Int.92 | AR | 453 | 453 |
| 5 | 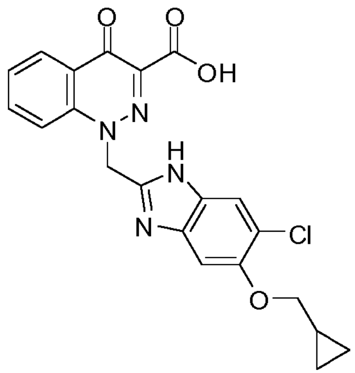 | 1-[[6-chloro-5-(cyclopropylmeth oxy)-1H-benzimidazol-2-yl]methyl]-4-oxo-cinnoline-3-carboxylic acid | Int.91 | AR | 425 | 425 |
| 6 | 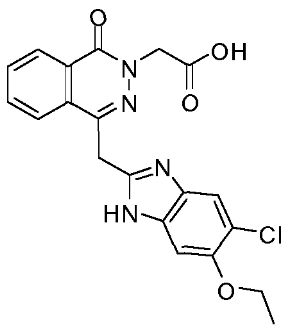 | 2-[4-[(5-chloro-6-ethoxy-1H-benzimidazol-2-yl)methyl]-1-oxo-phthalazin-2-yl]acetic acid | Int.177 | AS | 413 | 413 |

(continued)

| Cpd # | Structure | Name | Int | Mtd | MW (calc) | MW (obs) |
|---|---|---|---|---|---|---|
| 7 | 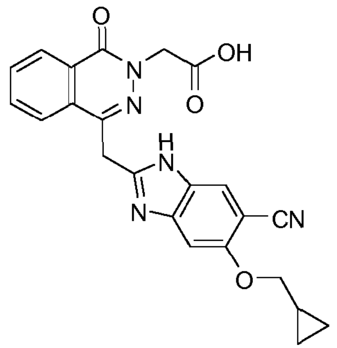 | 2-[4-[[6-cyano-5-(cyclopropylmeth oxy)-1H-benzimidazol-2-yl]methyl]-1-oxo-phthalazin-2-yl]acetic acid | Int.22 | AT | 429 | 430 |
| 8 | 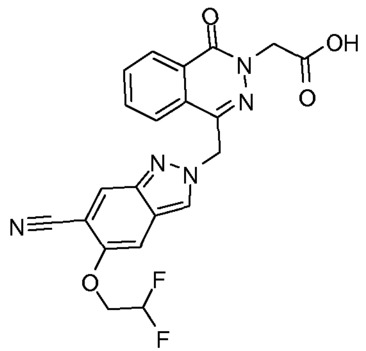 | 2-[4-[[6-cyano-5-(2,2-difluoroethoxy)in dazol-2-yl]methyl]-1-oxo-phthalazin-2-yl]acetic acid | Int.444 | NA | 439 | 440 |

(continued)

| Cpd # | Structure | Name | Int | Mtd | MW (calc) | MW (obs) |
|---|---|---|---|---|---|---|
| 9 | 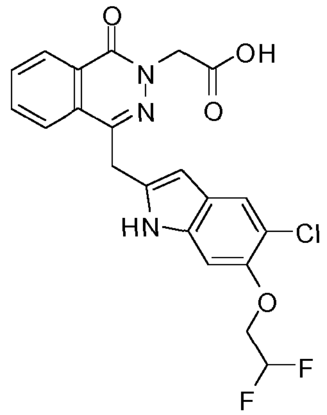 | 2-[4-[[5-chloro-6-(2,2-difluoroethoxy)-1H-indol-2-yl]methyl]-1-oxo-phthalazin-2-yl]acetic acid | Int.308 | NA | 448 | 449 |
| 10 | 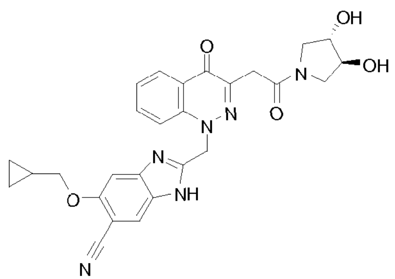 | 6-(cyclopropylmeth oxy)-2-[[3-[2-[(3S,4S)-3,4-dihydroxypyrroli din-1-yl]-2-oxoethyl]-4-oxo-cinnolin-1-yl]methyl]-3H-benzimidazole-5-carbonitrile | Final compoun d 108 | AU | 515 | 515 |

(continued)

| Cpd # | Structure | Name | Int | Mtd | MW (calc) | MW (obs) |
|---|---|---|---|---|---|---|
| 11 | 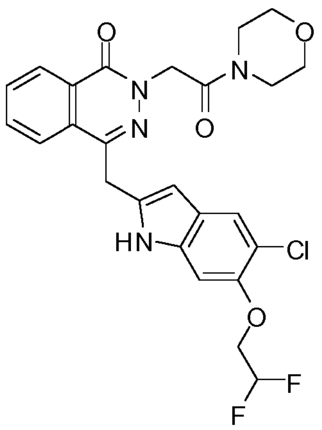 | 4-[[5-chloro-6-(2,2-difluoroethoxy)-1H-indol-2-yl]methyl]-2-(2-morpholino-2-oxoethyl)phthalazin-1-one | Int.3 04 | NA | 517 | 517 |
| 12 | 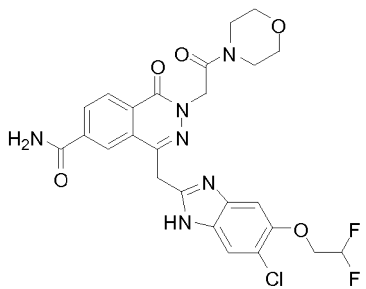 | 4-[[6-chloro-5-(2,2-difluoroethoxy)-1H-benzimidazol-2-yl]methyl]-2-(2-morpholino-2-oxo-ethyl)-1-oxo-phthalazine-6-carboxamide | Final compoun d 153 | NA | 561 | 561 |

(continued)

| Cpd # | Structure | Name | Int | Mtd | MW (calc) | MW (obs) |
|---|---|---|---|---|---|---|
| 13 | 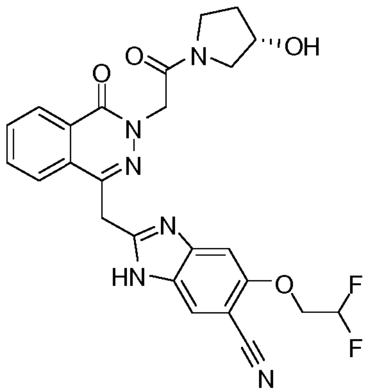 | 6-(2,2-difluoroethoxy)-2-[[3-[2-[(3S)-3-hydroxypyrrolidi n-1-yl]-2-oxoethyl]-4-oxo-phthalazin-1-yl]methyl]-3H-benzimidazole-5-carbonitrile | Final compoun d 139 | AV | 508 | 509 |
| 14 | 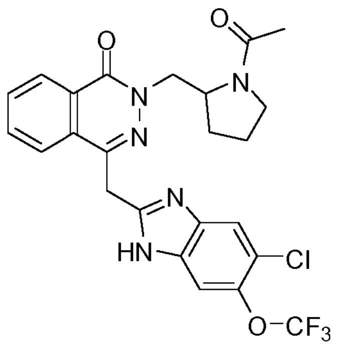 | 2-[(1-acetylpyrrolidin-2-yl)methyl]-4-[[5-chloro-6-(trifluoromethoxy )-1H-benzimidazol-2-yl]methyl]phthala zin-1-one | Final compoun d 55 | NA | 520 | 520 |

(continued)

| Cpd # | Structure | Name | Int | Mtd | MW (calc) | MW (obs) |
|---|---|---|---|---|---|---|
| 15 | 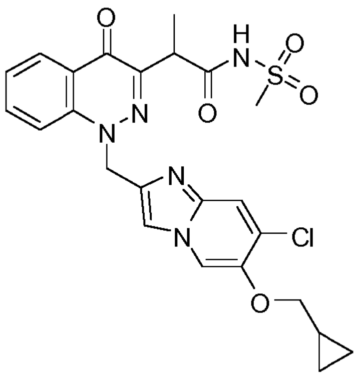 | 2-[1-[[7-chloro-6-(cyclopropylmeth oxy)imidazo [1,2-a]pyridin-2-yl] methyl]-4-oxo-cinnolin-3-yl]-N-methylsulfonyl-propanamide | Final compoun d 4 | AW | 530 | 530 |
| 16 | 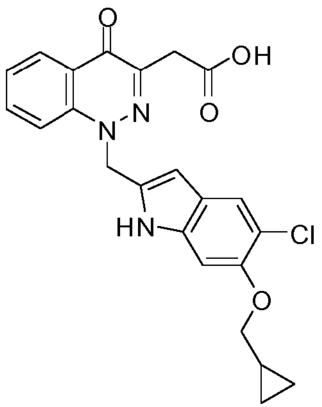 | 2-[1-[[5-chloro-6-(cyclopropylmeth oxy)-1H-indol-2-yl]methyl]-4-oxo-cinnolin-3 - yl]acetic acid | Int.246 | NA | 438 | 439 |

(continued)

| Cpd # | Structure | Name | Int | Mtd | MW (calc) | MW (obs) |
|---|---|---|---|---|---|---|
| 17 | 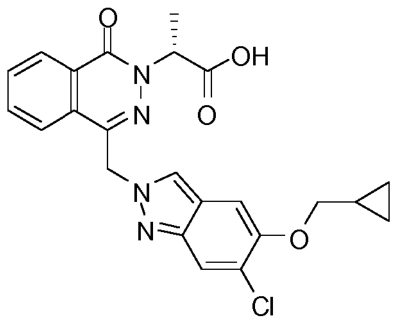 | ((2R)-2-[4-[[6-chloro-5-(cyclopropylmeth oxy)indazol-2-yl]methyl]-1-oxo-phthalazin-2-yl]propanoic acid) | Final compoun d 3 or (Int.58 + Int.450) | NA | 453 | 453 |
| 18 | 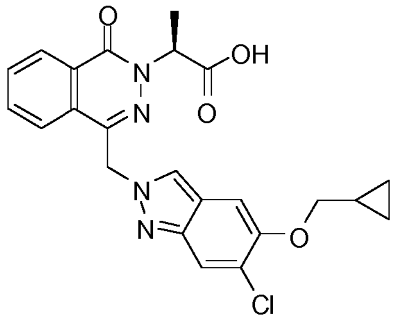 | (2S)-2-[4-[[6-chloro-5-(cyclopropylmeth oxy)indazol-2-yl]methyl]-1-oxo-phthalazin-2-yl]propanoic acid | Final compoun d 3 | NA | 453 | 453 |
| 19 | 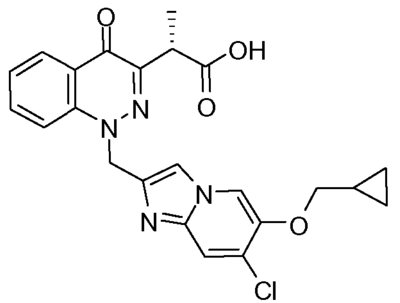 | (2S)-2-[1-[[7 - chloro-6-(cyclopropylmeth oxy)imidazo [1,2-a]pyridin-2-yl]methyl]-4-oxocinnolin-3-yl]propanoic acid | Final compoun d 4 | NA | 453 | 453 |

(continued)

| Cpd # | Structure | Name | Int | Mtd | MW (calc) | MW (obs) |
|---|---|---|---|---|---|---|
| 20 | 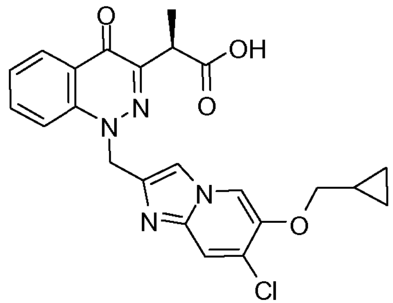 | (2R)-2-[1-[[7-chloro-6-(cyclopropylmeth oxy)imidazo [1,2-a]pyridin-2-yl] methyl]-4-oxocinnolin-3-yl]propanoic acid | Final compoun d 4 | NA | 453 | 453 |
| 21 | 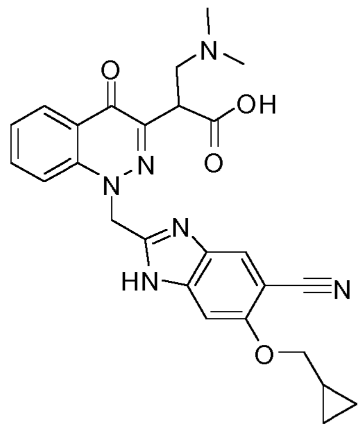 | (2-[1-[[5-cyano-6-(cyclopropylmeth oxy)-1H-benzimidazol-2-yl]methyl]-4-oxocinnolin-3-yl]-3-(dimethylamino) propanoic acid) | Int.235 | NA | 487 | 487 |
| 22 | 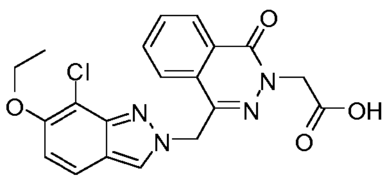 | 2-[4-[(7-chloro-6-ethoxy-indazol-2-yl)methyl]-1-oxo-phthalazin-2-yl] acetic acid | Int.259 | NA | 413 | 413 |

(continued)

| Cpd # | Structure | Name | Int | Mtd | MW (calc) | MW (obs) |
|---|---|---|---|---|---|---|
| 23 | 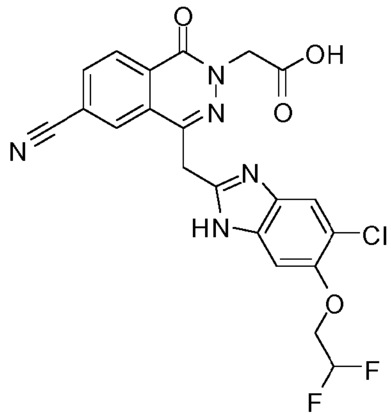 | 2-[4-[[5-chloro-6-(2,2-difluoroethoxy)-1H-benzimidazol-2-yl]methyl]-6-cyano-1-oxo-phthalazin- 2-yl]acetic acid | Int.202 | NA | 474 | 474 |
| 24 | 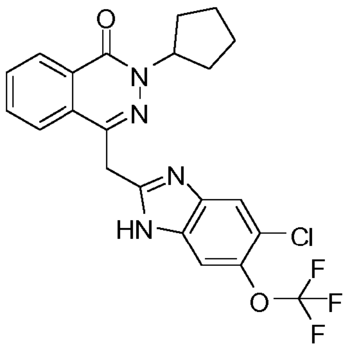 | 4-[[5-chloro-6-(trifluoromethoxy )-1H-benzimidazol-2-yl]methyl]-2-cyclopentyl-phthalazin-1-one | Int.355 | AJ | 463 | 463 |

(continued)

| Cpd # | Structure | Name | Int | Mtd | MW (calc) | MW (obs) |
|---|---|---|---|---|---|---|
| 25 | 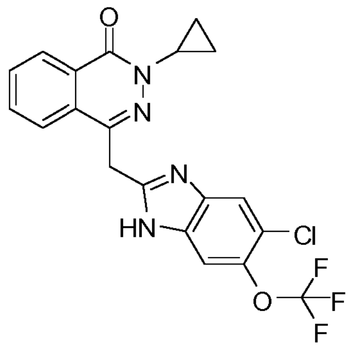 | 4-[[5-chloro-6-(trifluoromethoxy )-1H-benzimidazol-2-yl]methyl]-2-cyclopropyl-phthalazin-1-one | Int.26 | AJ | 435 | 534 |
| 26 | 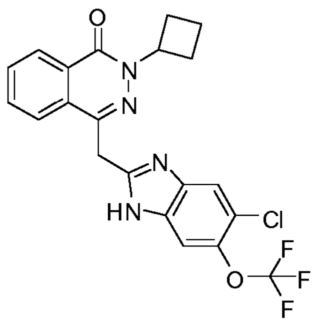 | 4-[[5-chloro-6-(trifluoromethoxy )-1H-benzimidazol-2-yl]methyl]-2-cyclobutyl-phthalazin-1-one | Int.396 | AJ | 449 | 449 |
| 27 | 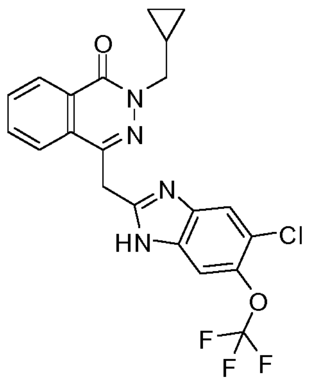 | 4-[[6-chloro-5-(trifluoromethoxy )-1H-benzimidazol-2-yl]methyl]-2-(cyclopropylmeth yl)phthalazin-1-one | Int.367 | AJ | 449 | 449 |

(continued)

| Cpd # | Structure | Name | Int | Mtd | MW (calc) | MW (obs) |
|---|---|---|---|---|---|---|
| 28 | 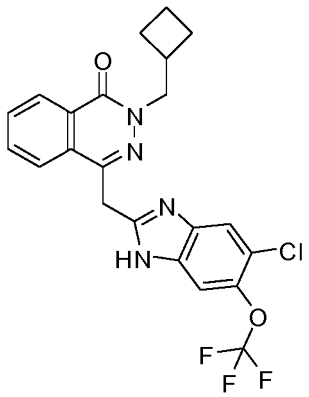 | 4-[[6-chloro-5-(trifluoromethoxy )-1H-benzimidazol-2-yl]methyl]-2-(cyclobutylmethy l)phthalazin-1-one | Int.366 | AJ | 463 | 463 |
| 30 | 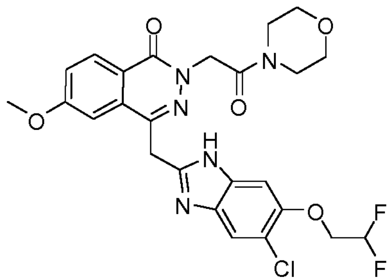 | 4-[[5-chloro-6-(2,2-difluoroethoxy)-1H-benzimidazol-2-yl]methyl]-6-methoxy-2-(2-morpholino-2-oxoethyl)phthalazin-1-one | Int.385 | AJ | 548 | 548 |
| 31 | 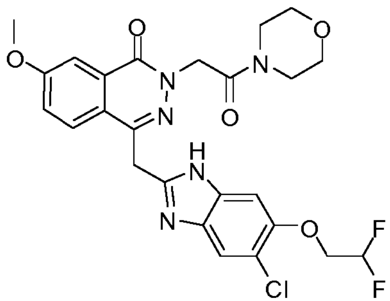 | 4-[[5-chloro-6-(2,2-difluoroethoxy)-1H-benzimidazol-2-yl]methyl]-7-methoxy-2-(2-morpholino-2-oxoethyl)phthalazin-1-one | Int.24 | AJ | 548 | 548 |

(continued)

| Cpd # | Structure | Name | Int | Mtd | MW (calc) | MW (obs) |
|---|---|---|---|---|---|---|
| 32 | 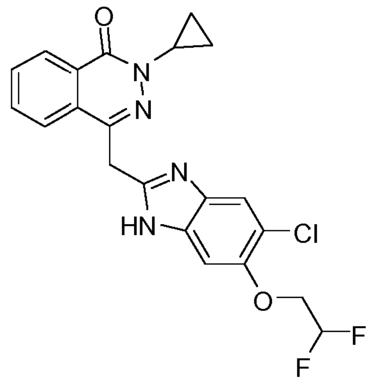 | 4-[[5-chloro-6-(2,2-difluoroethoxy)-1H-benzimidazol-2-yl]methyl]-2-cyclopropyl-phthalazin-1-one | Int.26 | AJ | 431 | 431 |
| 33 | 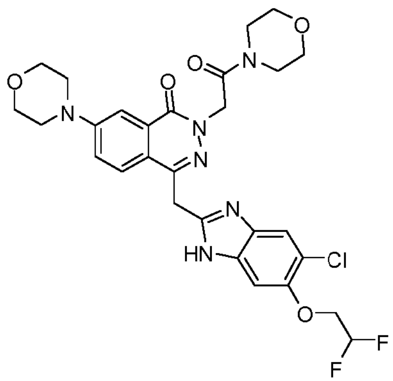 | 4-[[5-chloro-6-(2,2-difluoroethoxy)-1H-benzimidazol-2-yl]methyl]-7-morpholino-2-(2-morpholino-2-oxoethyl)phthalazin-1-one | Int.25 | AJ | 603 | 603 |

(continued)

| Cpd # | Structure | Name | Int | Mtd | MW (calc) | MW (obs) |
|---|---|---|---|---|---|---|
| 34 | 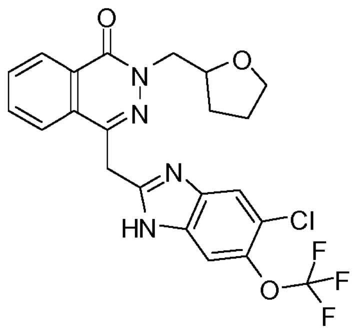 | 4-[[6-chloro-5-(trifluoromethoxy )-1H-benzimidazol-2-yl]methyl]-2-(THF-2-ylmethyl)phthala zin-1-one | Int.383 | AJ | 479 | 479 |
| 35 | 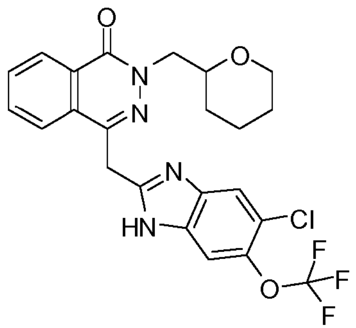 | 4-[[6-chloro-5-(trifluoromethoxy )-1H-benzimidazol-2-yl]methyl]-2-(tetrahydropyran-2-ylmethyl)phthala zin-1-one | Int.384 | AJ | 493 | 493 |
| 36 | 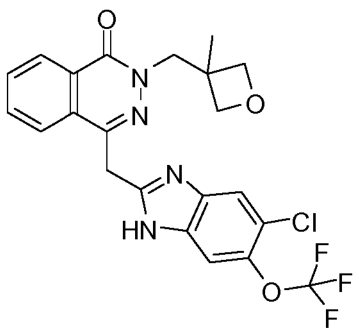 | 4-[[6-chloro-5-(trifluoromethoxy )-1H-benzimidazol-2-yl]methyl]-2-[(3-methyloxetan-3-yl)methyl]phthala zin-1-one | Int.377 | AJ | 479 | 479 |

(continued)

| Cpd # | Structure | Name | Int | Mtd | MW (calc) | MW (obs) |
|---|---|---|---|---|---|---|
| 38 | 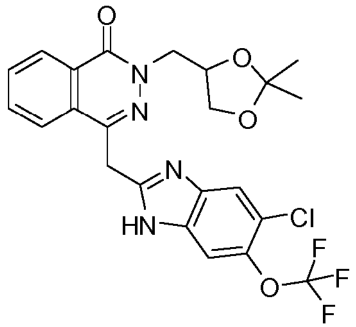 | 4-[[6-chloro-5-(trifluoromethoxy )-1H-benzimidazol-2-yl]methyl]-2-[(2,2-dimethyl-1,3-dioxolan-4-yl)methyl]phthala zin-1-one | Int.375 | AJ | 509 | 509 |
| 39 | 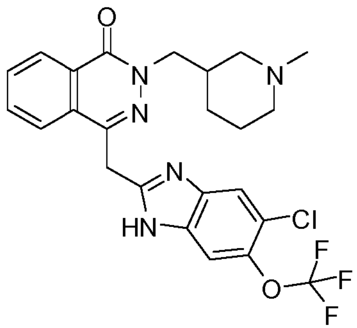 | 4-[[6-chloro-5-(trifluoromethoxy )-1H-benzimidazol-2-yl]methyl]-2-[(1-methyl-3-piperidyl)methyl] phthalazin-1-one | Int.368 | AJ | 506 | 507 |
| 40 | 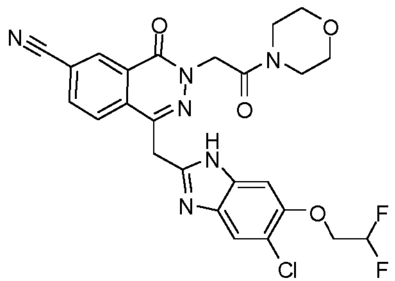 | 1-[[5-chloro-6-(2,2-difluoroethoxy)-1H-benzimidazol-2-yl]methyl]-3-(2-morpholino-2-oxo-ethyl)-4-oxo-phthalazine-6-carbonitrile | Int.394 | AJ | 543 | 543 |

(continued)

| Cpd # | Structure | Name | Int | Mtd | MW (calc) | MW (obs) |
|---|---|---|---|---|---|---|
| 41 | 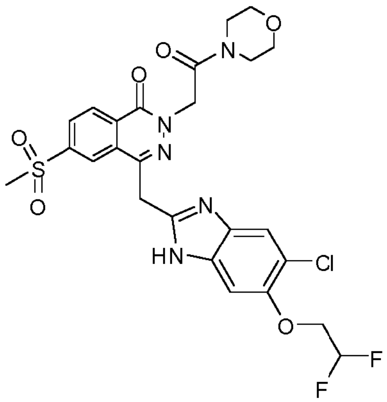 | 4-[[5-chloro-6-(2,2-difluoroethoxy)-1H-benzimidazol-2-yl]methyl]-6-methylsulfonyl-2-(2-morpholino-2-oxoethyl)phthalazin-1-one | Int.386 | AJ | 596 | 596 |
| 42 | 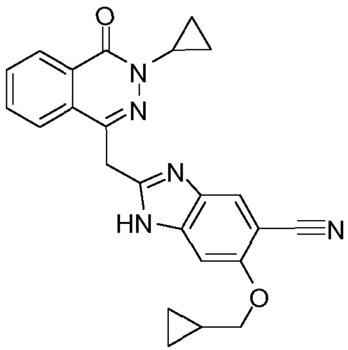 | 6-(cyclopropylmeth oxy)-2-[(3-cyclopropyl-4-oxo-phthalazin-1-yl) methyl]-1H-benzimidazole-5-carbonitrile | Int.26 | AJ | 411 | 412 |

(continued)

| Cpd # | Structure | Name | Int | Mtd | MW (calc) | MW (obs) |
|---|---|---|---|---|---|---|
| 43 | 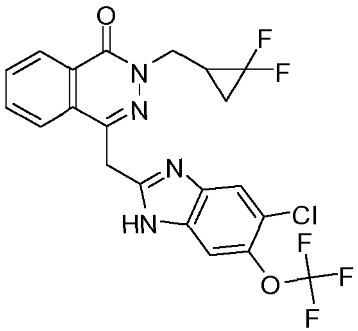 | 4-[[6-chloro-5-(trifluoromethoxy )-1H-benzimidazol-2-yl]methyl]-2-[(2,2-difluorocyclopro pyl)methyl]phtha lazin-1-one | Int.374 | AJ | 485 | 485 |
| 44 | 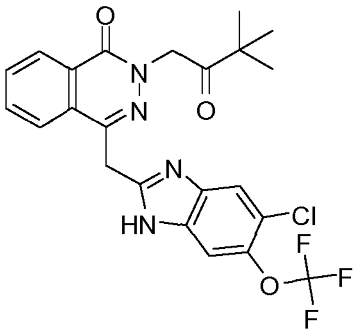 | 4-[[6-chloro-5-(trifluoromethoxy )-1H-benzimidazol-2-yl]methyl]-2-(3,3-dimethyl-2-oxobutyl)phthalazin-1-one | Int.365 | AJ | 493 | 492 |
| 47 | 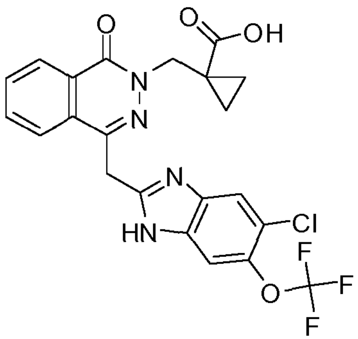 | 1-[[4-[[5-chloro-6-(trifluoromethoxy )-1H-benzimidazol-2-yl]methyl]-1-oxo-phthalazin-2-yl]methyl]cyclop ropanecarboxylic acid | Int.359 | AJ | 493 | 493 |

(continued)

| Cpd # | Structure | Name | Int | Mtd | MW (calc) | MW (obs) |
|---|---|---|---|---|---|---|
| 48 | 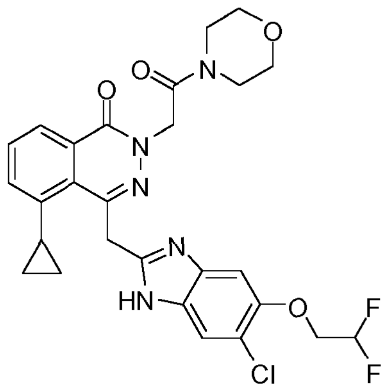 | 4-[[6-chloro-5-(2,2-difluoroethoxy)-1H-benzimidazol-2-yl]methyl]-5-cyclopropyl-2-(2-morpholino-2-oxoethyl)phthalazin-1-one | Int.388 | AJ | 558 | 558 |
| 49 | 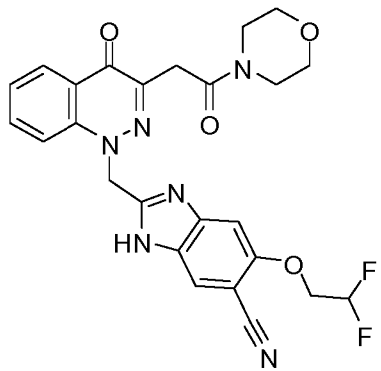 | 6-(2,2-Difluoroethoxy)-2-[3-(2-morpholin-4-yl-2-oxo-ethyl)-4-oxo-4H-cinnolin-1-ylmethyl]-3H-enzoimidazole-5-carbonitrile | Int.393 | AJ | 508 | 509 |

(continued)

| Cpd # | Structure | Name | Int | Mtd | MW (calc) | MW (obs) |
|---|---|---|---|---|---|---|
| 50 | 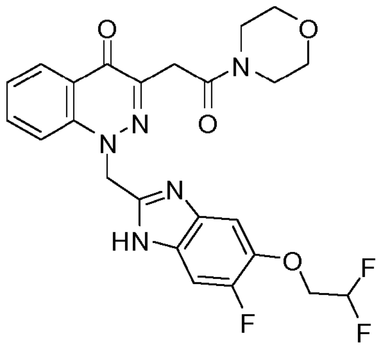 | 1-[5-(2,2-Difluoro-ethoxy)-6-fluoro -1H-benzoimidazol-2-ylmethyl]-3-(2-morpholin-4-yl-2-oxo-ethyl)-1H-cinnolin-4-one | Int.393 | AJ | 501 | 502 |
| 51 | 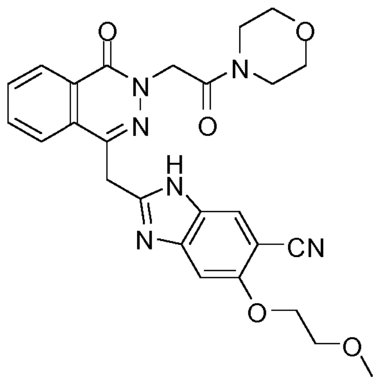 | 6-(2-methoxyethoxy)-2-[[3-(2-morpholino-2-oxo-ethyl)-4-oxo-phthalazin-1-yl]methyl]-3H-benzimidazole-5-carbonitrile | Int.363 | AJ | 503 | 503 |

(continued)

| Cpd # | Structure | Name | Int | Mtd | MW (calc) | MW (obs) |
|---|---|---|---|---|---|---|
| 52 | 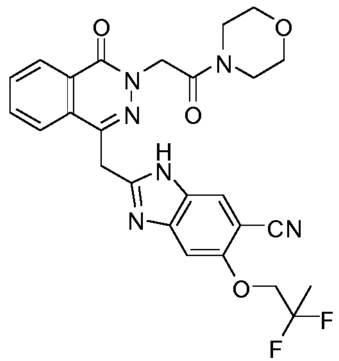 | 6-(2,2-difluoropropoxy) -2-[[3-(2-morpholino-2-oxo-ethyl)-4-oxo-phthalazin-1-yl]methyl]-3H-benzimidazole-5-carbonitrile | Int.363 | AJ | 523 | 523 |
| 53 | 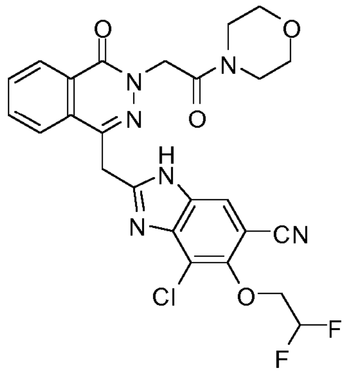 | 7-chloro-6-(2,2-difluoroethoxy)-2-[[3-(2-morpholino-2-oxo-ethyl)-4-oxo-phthalazin-1-yl]methyl]-3H-benzimidazole-5-carbonitrile | Int.363 | AJ | 543 | 543 |

(continued)

| Cpd # | Structure | Name | Int | Mtd | MW (calc) | MW (obs) |
|---|---|---|---|---|---|---|
| 54 | 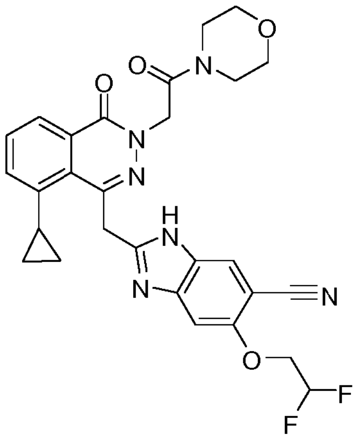 | 2-[[8-cyclopropyl-3-(2-morpholino-2-oxo-ethyl)-4-oxo-phthalazin-1-yl] methyl]-6-(2,2-difluoroethoxy)-3H-benzimidazole-5-carbonitrile | Int.388 | AJ | 549 | 549 |
| 55 | 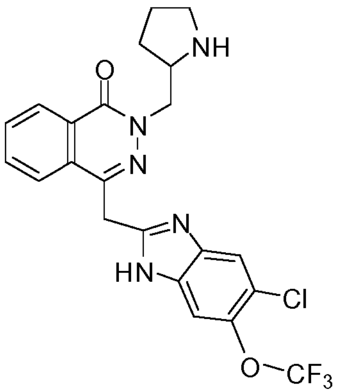 | 4-[[6-chloro-5-(trifluoromethoxy )-1H-benzimidazol-2-yl]methyl]-2-(pyrrolidin-2-ylmethyl)phthala zin-1-one | Int.172 | AO | 478 | 478 |

(continued)

| Cpd # | Structure | Name | Int | Mtd | MW (calc) | MW (obs) |
|---|---|---|---|---|---|---|
| 56 | 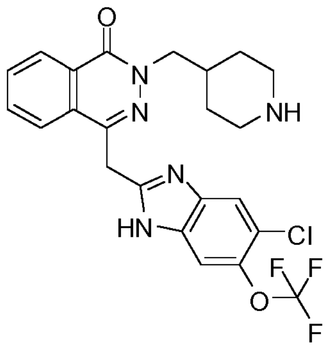 | 4-[[6-chloro-5-(trifluoromethoxy )-1H-benzimidazol-2-yl]methyl]-2-(4-piperidylmethyl) phthalazin-1-one | Int.211 | AO | 492 | 493 |
| 57 | 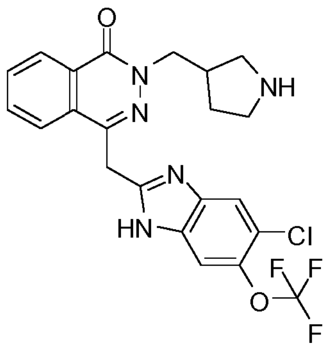 | 4-[[6-chloro-5-(trifluoromethoxy )-1Hbenzimidazol-2-yl]methyl]-2-(pyrrolidin-3-ylmethyl)phthala zin-1-one | Int.205 | AO | 478 | 478 |
| 58 | 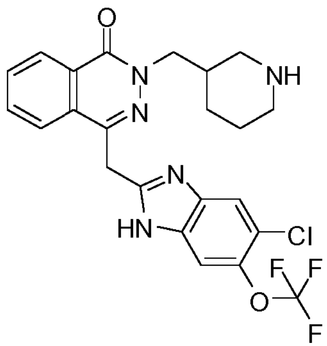 | 4-[[6-chloro-5-(trifluoromethoxy )-1H-benzimidazol-2-yl]methyl]-2-(3-piperidylmethyl) phthalazin-1-one | Int.204 | AO | 492 | 492 |

(continued)

| Cpd # | Structure | Name | Int | Mtd | MW (calc) | MW (obs) |
|---|---|---|---|---|---|---|
| 59 | 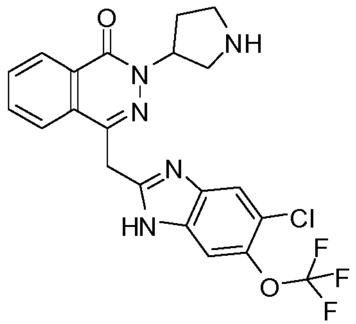 | 4-[[6-chloro-5-(trifluoromethoxy )-1H-benzimidazol-2-yl]methyl]-2-pyrrolidin-3-ylphthalazin-1-one | Int.207 | AO | 464 | 464 |
| 61 | 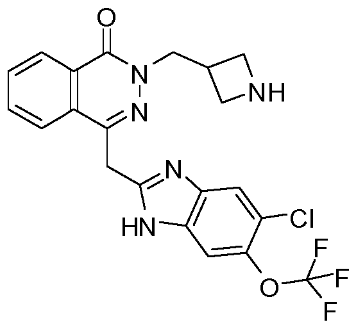 | 2-(azetidin-3-ylmethyl)-4-[[6-chloro-5-(trifluoromethoxy benzimidazol-2-yl]methyl]phthala zin-1-one | Int.203 | AO | 464 | 464 |
| 62 | 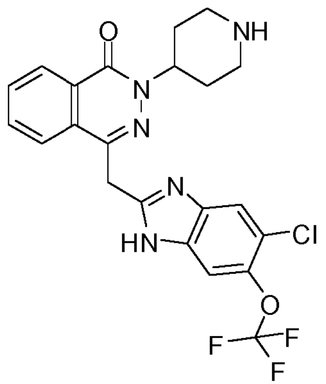 | 4-[[6-chloro-5-(trifluoromethoxy )-1H-benzimidazol-2-yl]methyl]-2-(4-piperidyl)phthala zin-1-one | Int.213 | AO | 478 | 478 |

(continued)

| Cpd # | Structure | Name | Int | Mtd | MW (calc) | MW (obs) |
|---|---|---|---|---|---|---|
| 63 | 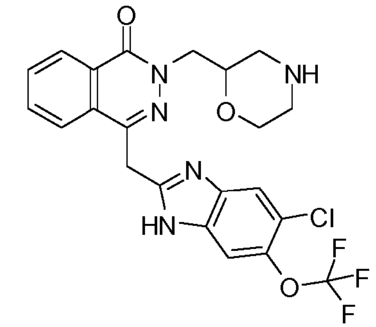 | 4-[[5-chloro-6-(trifluoromethoxy )-1H-benzimidazol-2-yl]methyl]-2-(morpholin-2-ylmethyl)phthala zin-1-one | Int.443 | AO | 494 | 494 |
| 64 | 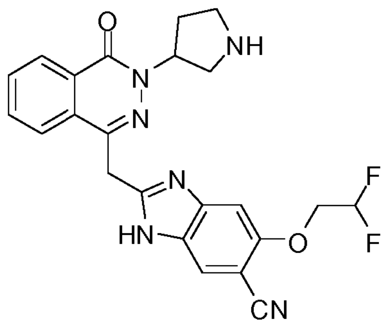 | 6-(2,2-difluoroethoxy)-2-[(4-oxo-3-pyrrolidin-3-ylphthalazin -1-yl) methyl]-3H-benzimidazole-5-carbonitrile | Int.209 | AO | 450 | 451 |
| 65 | 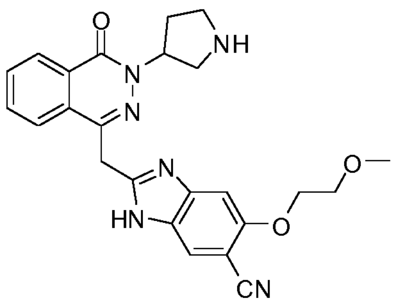 | 6-(2-methoxyethoxy)-2-[(4-oxo-3-pyrrolidin-3-ylphthalazin -1-yl) methyl]-3H-benzimidazole-5-carbonitrile | Int.210 | AO | 444 | 445 |

(continued)

| Cpd # | Structure | Name | Int | Mtd | MW (calc) | MW (obs) |
|---|---|---|---|---|---|---|
| 66 | 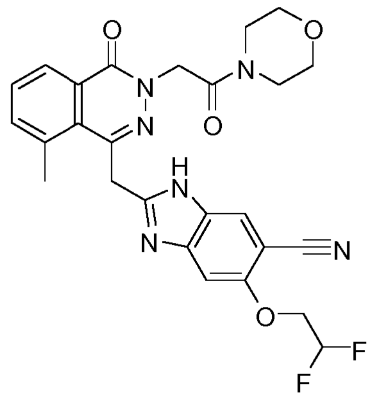 | 6-(2,2-difluoroethoxy)-2-[[8-methyl-3-(2-morpholino-2-oxo-ethyl)-4-oxo-phthalazin-1-yl]methyl]-3H-benzimidazole-5-carbonitrile | Int.103 | AL | 523 | 523 |
| 67 | 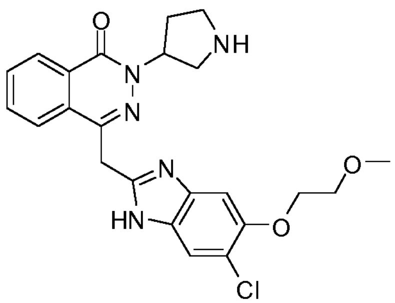 | 4-[[6-chloro-5-(2-methoxyethoxy)-1H-benzimidazol-2-yl] methyl]-2-pyrrolidin-3-ylphthalazin-1-one | Int.206 | AO | 454 | 451 |
| 68 | 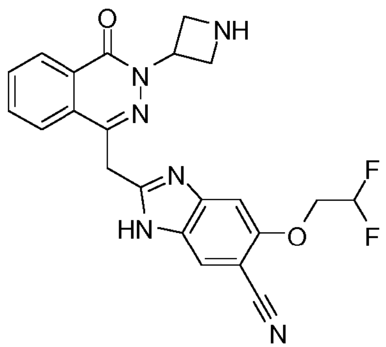 | 2-[[3-(azetidin-3-yl)-4-oxo-phthalazin-1-yl]methyl] -6-(2,2-difluoroethoxy)-3H-benzimidazole-5-carbonitrile | Int.346 | AM | 436 | 437 |

(continued)

| Cpd # | Structure | Name | Int | Mtd | MW (calc) | MW (obs) |
|---|---|---|---|---|---|---|
| 69 | 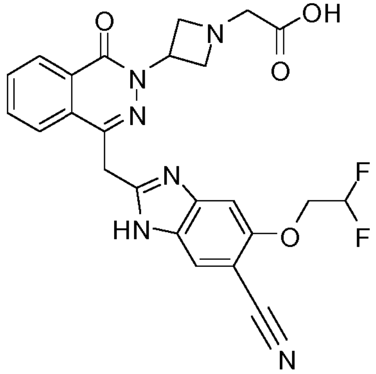 | 2-[3-[4-[[6-cyano-5-(2,2-difluoroethoxy)-1H-benzimidazol -2-yl]methyl]-1-oxo-phthalazin-2-yl]azetidin-1-yl]acetic acid | Int.248 | AM | 494 | 495 |
| 70 | 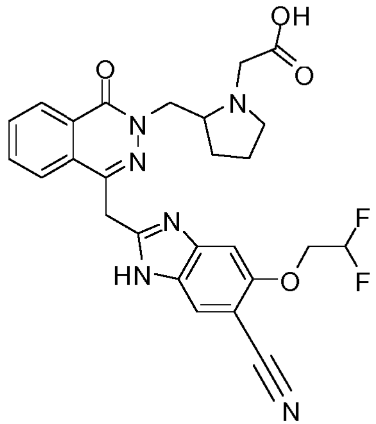 | 2-[2-[[4-[[6-cyano-5-(2,2-difluoroethoxy)-1H-benzimidazol -2-yl] methyl]-1-oxo-phthalazin-2-yl]methyl]pyrroli din-1-yl] acetic acid | Int.247 | AM | 523 | 523 |

(continued)

| Cpd # | Structure | Name | Int | Mtd | MW (calc) | MW (obs) |
|---|---|---|---|---|---|---|
| 71 | 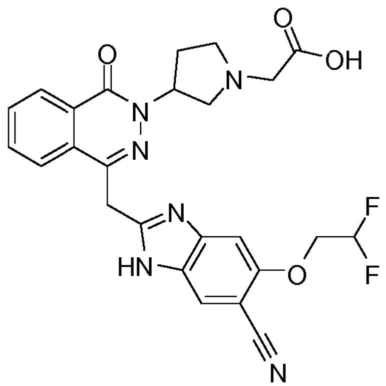 | 2-[3-[4-[[6-cyano-5-(2,2-difluoroethoxy)-1H-benzimidazol -2-yl]methyl]-1-oxo-phthalazin-2-yl]pyrrolidin-1-yl]acetic acid | Int.249 | AM | 508 | 509 |
| 72 | 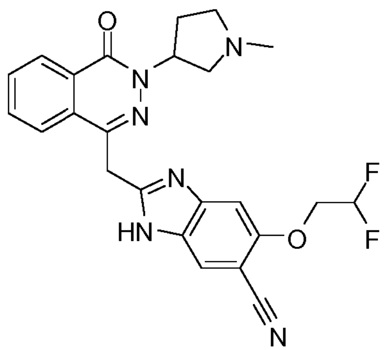 | 6-(2,2-difluoroethoxy)-2-[[3-(1-methylpyrrolidin-3-yl)-4-oxo -phthalazin-1-yl]methyl]-3H-benzimidazole-5-carbonitrile | Int.305 | AL | 464 | 465 |

(continued)

| Cpd # | Structure | Name | Int | Mtd | MW (calc) | MW (obs) |
|---|---|---|---|---|---|---|
| 73 | 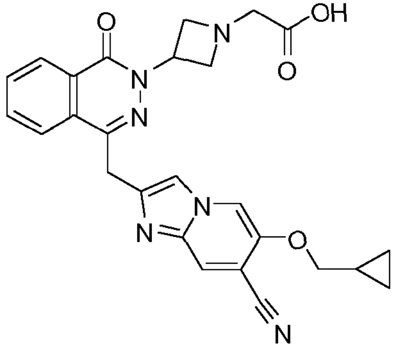 | 2-[3-[4-[[7-cyano-6-(cyclopropylmeth oxy)imidazo[1,2-a]pyridin-2-yl] methyl]-1-oxo-phthalazin- 2-yl]azetidin-1-yl]acetic acid | Int.241 | AQ & AR | 485 | 486 |
| 74 | 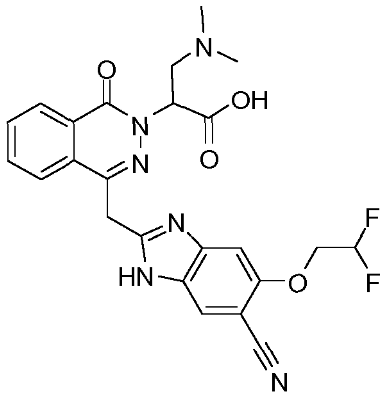 | 2-[4-[[6-cyano-5-(2,2-difluoroethoxy)-1H-benzimidazol -2-yl]methyl]-1-oxo-phthalazin- 2-yl]-3-(dimethylamino) propanoic acid | Int.250 | AM | 496 | 497 |

(continued)

| Cpd # | Structure | Name | Int | Mtd | MW (calc) | MW (obs) |
|---|---|---|---|---|---|---|
| 75 | 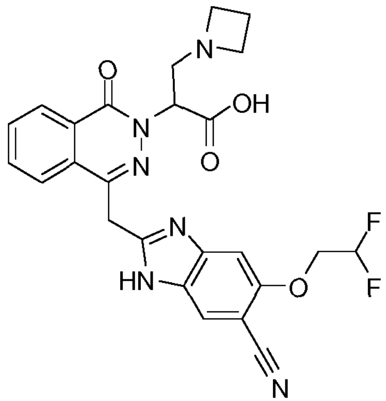 | 3-(azetidin-1-yl)-2-[4-[[6-cyano-5-(2,2-difluoroethoxy) -1H-benzimidazol-2-yl]methyl]-1-oxo-phthalazin-2-yl]propanoic acid | Int.251 | AM | 508 | 509 |
| 76 | 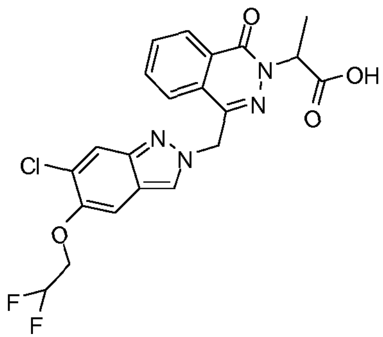 | 2-[4-[[6-chloro-5-(2,2-difluoroethoxy)in dazol-2-yl]methyl]-1-oxo-phthalazin-2-yl]propanoic acid | Int.268 | AR | 463 | 463 |

(continued)

| Cpd # | Structure | Name | Int | Mtd | MW (calc) | MW (obs) |
|---|---|---|---|---|---|---|
| 77 | 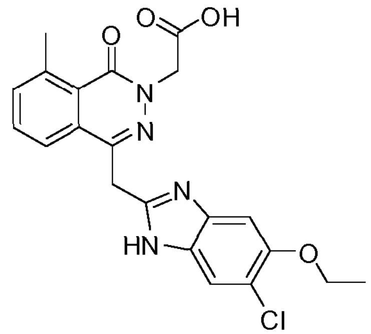 | 2-[4-[(6-chloro-5-ethoxy-1H-benzimidazol-2-yl)methyl]-8-methyl-1-oxo-phthalazin- 2-yl]acetic acid | Int.171 | AR | 427 | 427 |
| 78 | 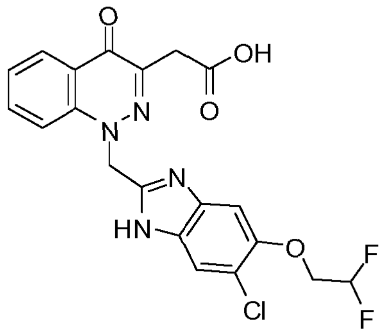 | {1-[6-Chloro-5-(2,2-difluoroethoxy)-1H-benzoimidazol-2-ylmethyl]-4-oxo-1,4-dihydrocinnolin-3-yl}acetic acid | Int.170 | AR | 449 | 449 |
| 79 | 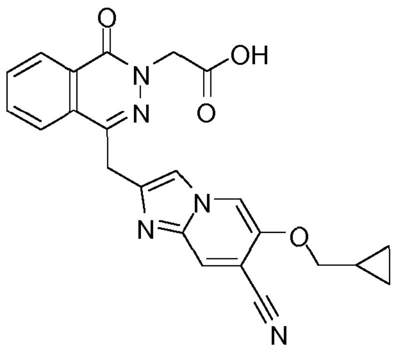 | 2-[4-[[7-cyano-6-(cyclopropylmeth oxy)imidazo[1,2-a]pyridin-2-yl] methyl]-1-oxo-phthalazin-2-yl]acetic acid | Int.309 | AR | 429 | 431 |

(continued)

| Cpd # | Structure | Name | Int | Mtd | MW (calc) | MW (obs) |
|---|---|---|---|---|---|---|
| 80 | 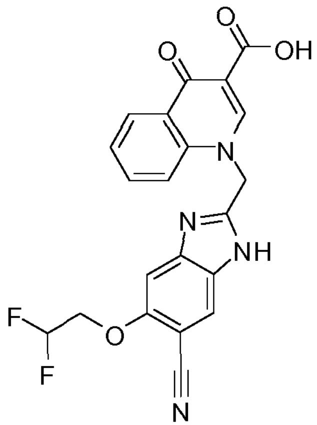 | 1-[[6-cyano-5-(2,2-difluoroethoxy)-1H-benzimidazol-2-yl]methyl]-4-oxo-quinoline-3-carboxylic acid | Int.93 | AR | 424 | 426 |
| 81 | 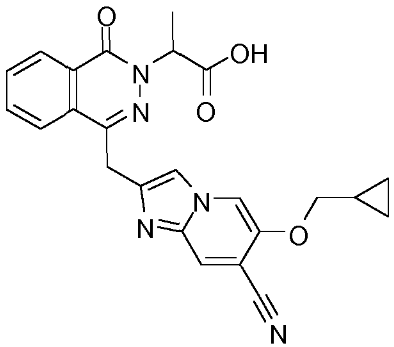 | 2-[4-[[7-cyano-6-(cyclopropylmeth oxy)imidazo [1,2-a]pyridin-2-yl] methyl]-1-oxo-phthalazin-2-yl]propanoic acid | Int.310 | AR | 443 | 445 |

(continued)

| Cpd # | Structure | Name | Int | Mtd | MW (calc) | MW (obs) |
|---|---|---|---|---|---|---|
| 82 | 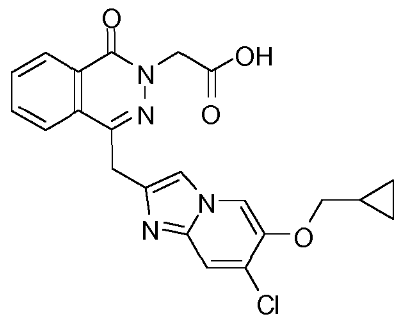 | 2-[4-[[7-chloro-6-(cyclopropylmeth oxy)imidazo[1,2-a]pyridin-2-yl] methyl]-1-oxo-phthalazin-2-yl]acetic acid | Int.20 | AR | 439 | 440 |
| 83 | 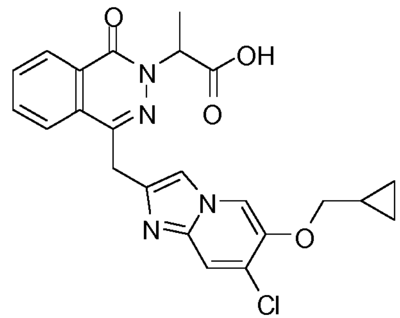 | 2-[4-[[7-chloro-6-(cyclopropylmeth oxy)imidazo [1,2-a]pyridin-2-yl] methyl]-1-oxo-phthalazin-2-yl]propanoic acid | Int.353 | AR | 453 | 454 |
| 84 | 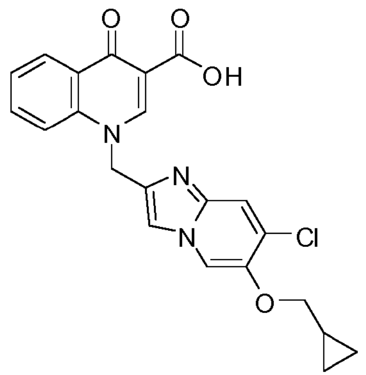 | 1-[[7-chloro-6-(cyclopropylmeth oxy)imidazo [1,2-a]pyridin-2-yl]methyl]-4-oxo-quinoline- 3-carboxylic acid | Int.220 | AR | 424 | 425 |

(continued)

| Cpd # | Structure | Name | Int | Mtd | MW (calc) | MW (obs) |
|---|---|---|---|---|---|---|
| 85 | 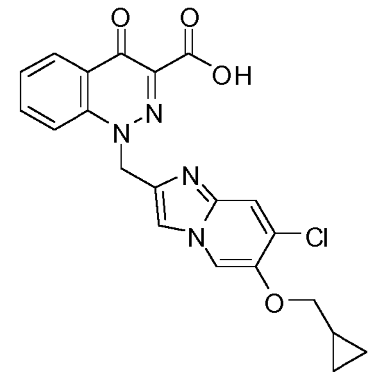 | 1-[[7-chloro-6-(cyclopropylmeth oxy)imidazo [1,2-a]pyridin-2-yl]methyl]-4-oxo-cinnoline-3-carboxylic acid | Int.219 | AR | 425 | 426 |
| 86 | 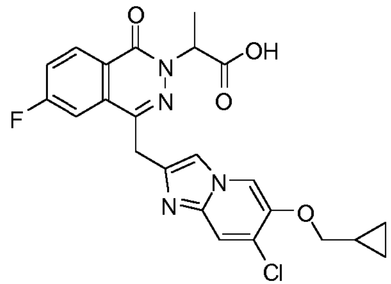 | 2-[4-[[7-chloro-6-(cyclopropylmeth oxy)imidazo [1,2-a]pyridin-2-yl] methyl]-6-fluoro-1-oxo-phthalazin- 2-yl]propanoic acid | Int.354 | AR | 471 | 471 |
| 87 | 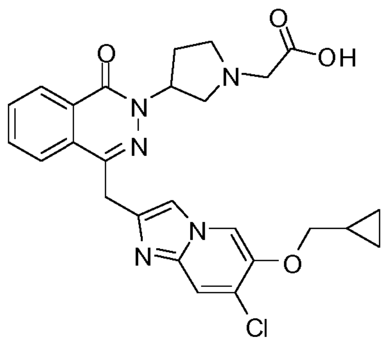 | 2-[3-[4-[[7-chloro-6-(cyclopropylmeth oxy)imidazo [1,2-a] pyridin-2-yl] methyl]-1-oxo-phthalazin-2-yl]pyrrolidin-1-yl]acetic acid | Int.245 | AR | 508 | 508 |

(continued)

| Cpd # | Structure | Name | Int | Mtd | MW (calc) | MW (obs) |
|---|---|---|---|---|---|---|
| 88 | 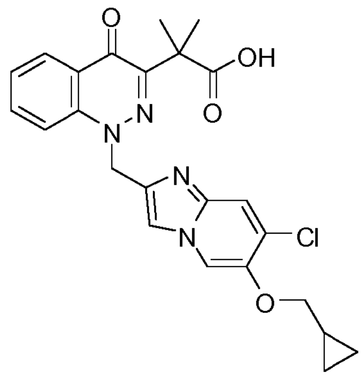 | 2-[1-[[7-chloro-6-(cyclopropylmeth oxy)imidazo [1,2-a]pyridin-2-yl] methyl]-4-oxo-cinnolin-3-yl]-2-methylpropanoic acid | Int.227 | AR | 467 | 468 |
| 89 | 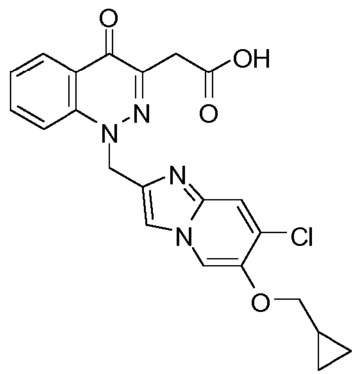 | 2-[1-[[7-chloro-6-(cyclopropylmeth oxy)imidazo [1,2-a]pyridin-2-yl] methyl]-4-oxo-cinnolin-3 - yl]acetic acid | Int.228 | AR | 439 | 439.1 |

(continued)

| Cpd # | Structure | Name | Int | Mtd | MW (calc) | MW (obs) |
|---|---|---|---|---|---|---|
| 90 | 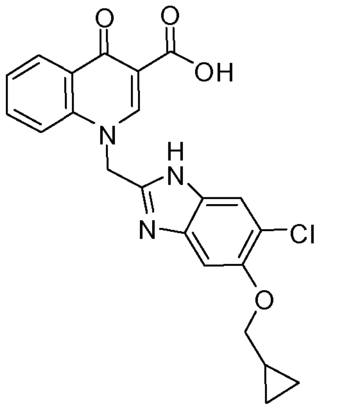 | 1-[[6-chloro-5-(cyclopropylmeth oxy)-1H-benzimidazol-2-yl]methyl]-4-oxo-quinoline-3-carboxylic acid | Int.169 | AR | 424 | 424 |
| 91 | 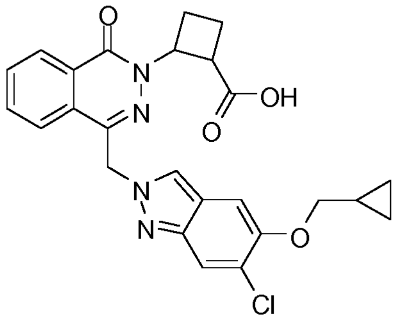 | 2-[4-[[6-chloro-5-(cyclopropylmeth oxy)indazol-2-yl]methyl] -1-oxo-phthalazin- 2-yl]cyclobutanecar boxylic acid | Int.273 | AR | 479 | 479 |
| 92 | 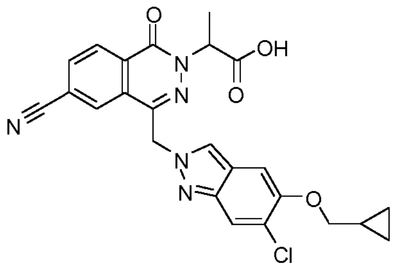 | 2-[4-[[6-chloro-5-(cyclopropylmeth oxy)indazol-2-yl]methyl]-6-cyano-1-oxo-phthalazin- 2-yl]propanoic acid | Int.104 | AR | 478 | 478 |

(continued)

| Cpd # | Structure | Name | Int | Mtd | MW (calc) | MW (obs) |
|---|---|---|---|---|---|---|
| 93 | 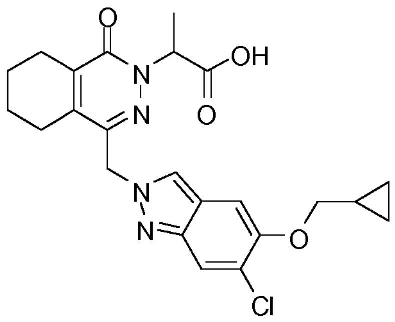 | 2-[4-[[6-chloro-5-(cyclopropylmeth oxy)indazol-2-yl]methyl]-1-oxo-5,6,7,8-tetrahydrophthala zin-2-yl]propanoic acid | Int.269 | AR | 457 | 457 |
| 94 | 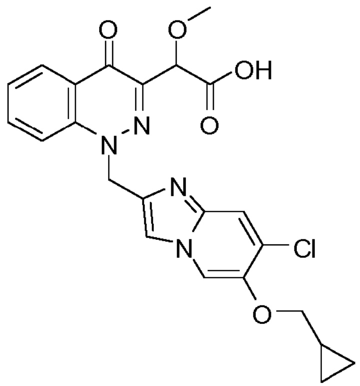 | 2-[1-[[7-chloro-6-(cyclopropylmeth oxy)imidazo [1,2-a]pyridin-2-yl] methyl]-4-oxo-cinnolin-3-yl]-2-methoxyacetic acid | Int.226 | AR | 469 | 469 |
| 95 | 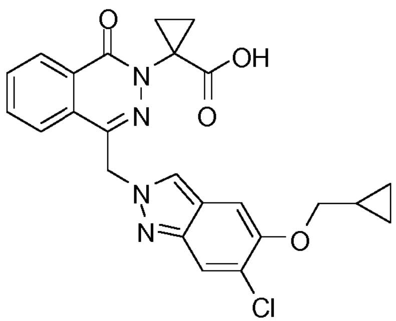 | 1-[4-[[6-chloro-5-(cyclopropylmeth oxy)indazol-2-yl]methyl]-1 -oxo-phthalazin-2-yl]cyclopropanec arboxylic acid | Int.272 | AR | 465 | 465 |

(continued)

| Cpd # | Structure | Name | Int | Mtd | MW (calc) | MW (obs) |
|---|---|---|---|---|---|---|
| 96 | 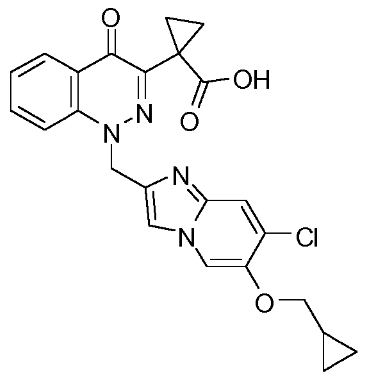 | 1-[1-[[7-chloro-6-(cyclopropylmeth oxy)imidazo [1,2-a]pyridin-2-yl] methyl]-4-oxo-cinnolin-3-yl]cyclopropanec arboxylic acid | Int.221 | AR | 465 | 465 |
| 97 | 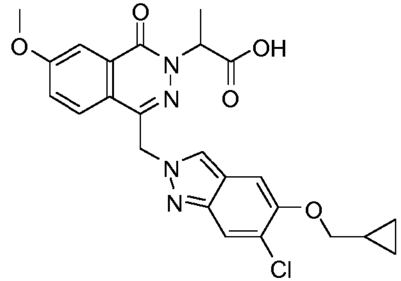 | 2-[4-[[6-chloro-5-(cyclopropylmeth oxy)indazol-2-yl]methyl]-7-methoxy-1-oxo-phthalazin- 2-yl]propanoic acid | Int.270 | AR | 483 | 483 |
| 98 | 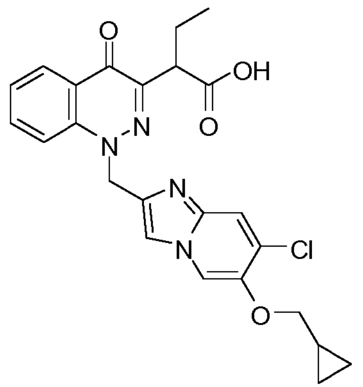 | 2-[1-[[7-chloro-6-(cyclopropylmeth oxy)imidazo [1,2-a]pyridin-2-yl] methyl]-4-oxo-cinnolin-3-yl]butanoic acid | Int.229 | AR | 467 | 467 |

(continued)

| Cpd # | Structure | Name | Int | Mtd | MW (calc) | MW (obs) |
|---|---|---|---|---|---|---|
| 99 | 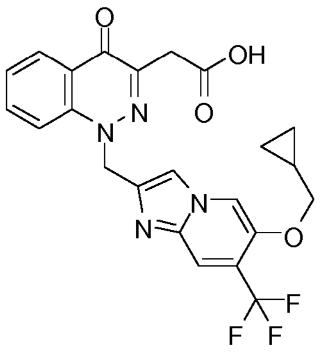 | 2-[1-[[6-(cyclopropylmeth oxy)-7-(trifluoromethyl)i midazo[1,2-a]pyridin-2-yl]methyl]-4-oxo-cinnolin-3 - yl]acetic acid | Int.223 | AR | 472 | 473 |
| 100 | 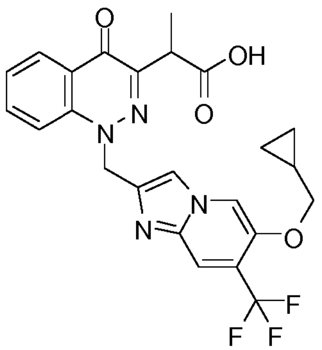 | 2-[1-[[6-(cyclopropylmeth oxy)-7-(trifluoromethyl)i midazo[1,2-a]pyridin-2-yl]methyl]-4-oxo-cinnolin-3-yl]propanoic acid | Int.224 | AR | 486 | 487 |

(continued)

| Cpd # | Structure | Name | Int | Mtd | MW (calc) | MW (obs) |
|---|---|---|---|---|---|---|
| 101 | 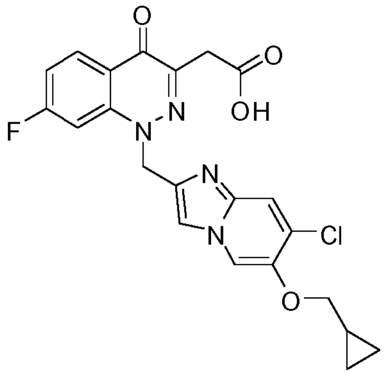 | 2-[1-[[7-chloro-6-(cyclopropylmeth oxy)imidazo [1,2-a]pyridin -2-yl] methyl]-7-fluoro-4-oxo-cinnolin-3-yl]acetic acid | Int.231 | AR | 457 | 457 |
| 102 | 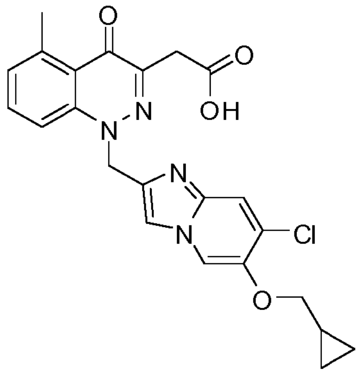 | 2-[1-[[7-chloro-6-(cyclopropylmeth oxy)imidazo [1,2-a]pyridin -2-yl] methyl]-5-methyl-4-oxo-cinnolin-3-yl]acetic acid | Int.23 0 | AR | 453 | 453 |

(continued)

| Cpd # | Structure | Name | Int | Mtd | MW (calc) | MW (obs) |
|---|---|---|---|---|---|---|
| 103 | 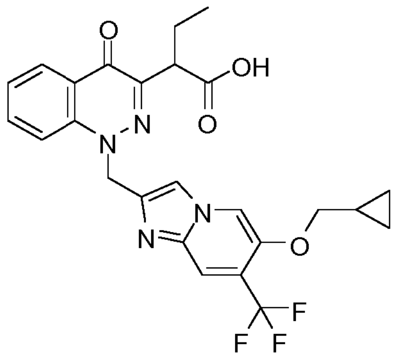 | 2-[1-[[6-(cyclopropylmeth oxy)-7-(trifluoromethyl)i midazo[1,2-a]pyridin-2-yl]methyl]-4-oxo-cinnolin-3-yl]butanoic acid | Int.224 | AR | 500 | 501 |
| 104 | 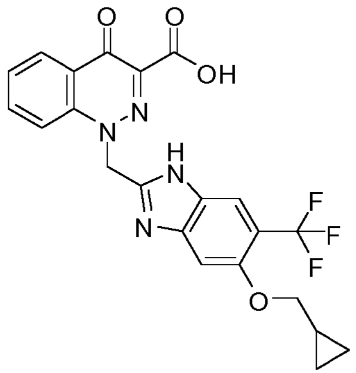 | (1-[[5-(cyclopropylmeth oxy)-6-(trifluoromethyl)-1H-benzimidazol-2-yl] methyl]-4-oxocinnoline-3-carboxylic acid) | Int.167 | AR | 458 | 459 |
| 105 | 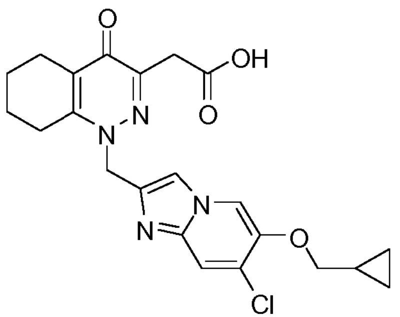 | 2-[1-[[7-chloro-6-(cyclopropylmeth oxy)imidazo [1,2-a]pyridin-2-yl] methyl]-4-oxo-5,6,7,8-tetrahydrocinnoli n-3-yl]acetic acid | Int.225 | AR | 443 | 443 |

(continued)

| Cpd # | Structure | Name | Int | Mtd | MW (calc) | MW (obs) |
|---|---|---|---|---|---|---|
| 106 | 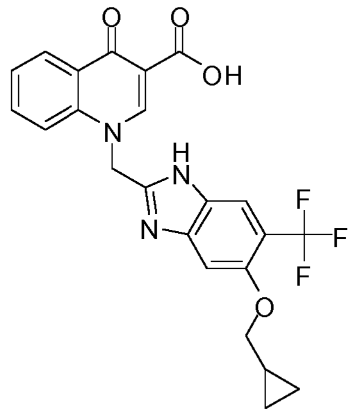 | (1-[[5-(cyclopropylmeth oxy)-6-(trifluoromethyl)-1H-benzimidazol-2-yl] methyl]-4-oxoquinoline-3-carboxylic acid) | Int.168 | AR | 457 | 458 |
| 107 | 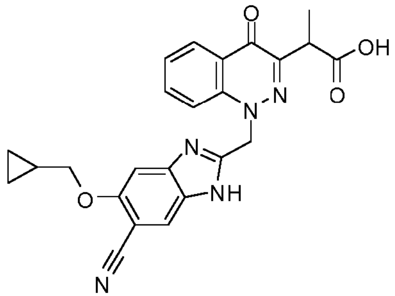 | 2-[1-[[6-cyano-5-(cyclopropylmeth oxy)-1H-benzimidazol-2-yl]methyl]-4-oxo-cinnolin-3-yl]propanoic acid | Int.238 | AR | 443 | 445 |
| 108 | 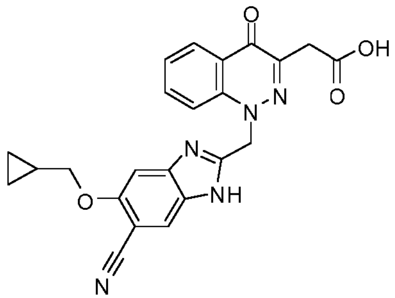 | 2-[1-[[6-cyano-5-(cyclopropylmeth oxy)-1H-benzimidazol-2-yl]methyl]-4-oxo-cinnolin-3 - yl]acetic acid | Int.236 | AR | 429 | 430 |

(continued)

| Cpd # | Structure | Name | Int | Mtd | MW (calc) | MW (obs) |
|---|---|---|---|---|---|---|
| 109 | 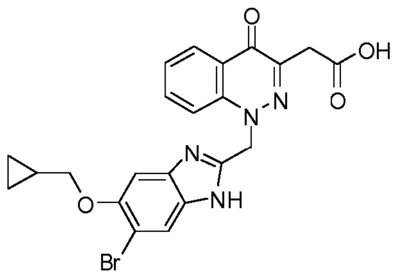 | 2-[1-[[6-bromo-5-(cyclopropylmeth oxy)-1H-benzimidazol-2-yl]methyl]-4-oxo-cinnolin-3- yl]acetic acid | Int.237 | AR | 483 | 484 |
| 110 | 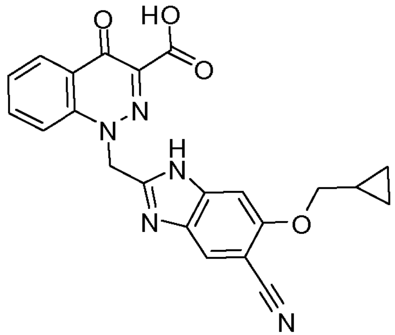 | 1-[[5-cyano-6-(cyclopropylmeth oxy)-1H-benzimidazol-2-yl]methyl]-4-oxo-cinnoline-3-carboxylic acid | Int.218 | AR & AM | 415 | 417 |
| 111 | 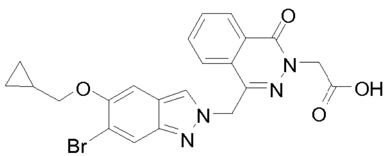 | 2-[4-[[6-bromo-5-(cyclopropylmeth oxy)indazol-2-yl]methyl]-1-oxo-phthalazin-2-yl]acetic acid | Int.276 | AS | 483 | 483 |

(continued)

| Cpd # | Structure | Name | Int | Mtd | MW (calc) | MW (obs) |
|---|---|---|---|---|---|---|
| 112 | 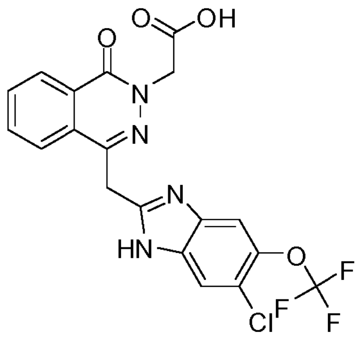 | 2-[4-[[6-chloro-5-(trifluoromethoxy )-1H-benzimidazol-2-yl]methyl]-1-oxo-phthalazin-2-yl]acetic acid | Int.201 | AS | 453 | 453 |
| 113 | 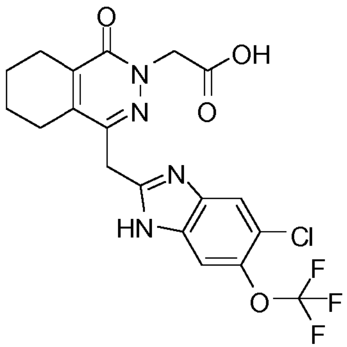 | 2-[4-[[5-chloro-6-(trifluoromethoxy )-1H-benzimidazol-2-yl]methyl]-1-oxo-5,6,7,8-tetrahydrophthala zin-2-yl] acetic acid | Int.191 | AS | 457 | 457 |
| 114 | 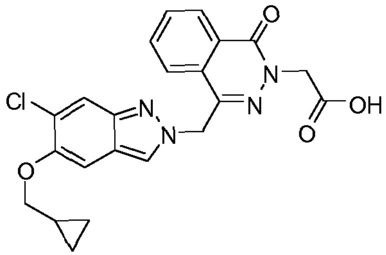 | 2-[4-[[6-chloro-5-(cyclopropylmeth oxy)indazol-2-yl]methyl]-1-oxo-phthalazin-2-yl]acetic acid | Int.277 | AS | 439 | 439 |

(continued)

| Cpd # | Structure | Name | Int | Mtd | MW (calc) | MW (obs) |
|---|---|---|---|---|---|---|
| 115 | 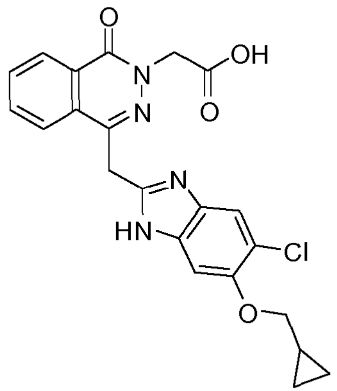 | 2-[4-[[5-chloro-6-(cyclopropylmeth oxy)-1H-benzimidazol-2-yl]methyl]-1-oxo-phthalazin-2-yl]acetic acid | Int.189 | AS | 439 | 439 |
| 116 | 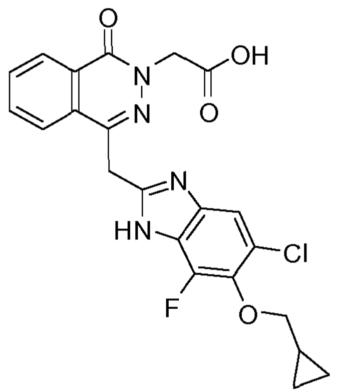 | 2-[4-[[5-chloro-6-(cyclopropylmeth oxy)-7-fluoro-1H-benzimidazol-2-yl] methyl]-1-oxo-phthalazin-2-yl]acetic acid | Int. 190 | AS | 457 | 457 |

(continued)

| Cpd # | Structure | Name | Int | Mtd | MW (calc) | MW (obs) |
|---|---|---|---|---|---|---|
| 117 | 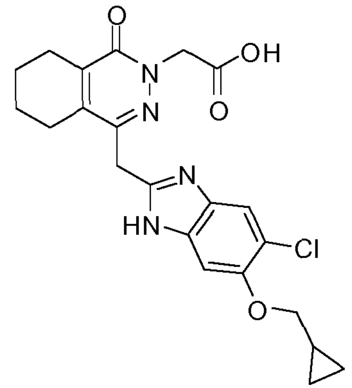 | 2-[4-[[5-chloro-6-(cyclopropylmeth oxy)-1H-benzimidazol-2-yl]methyl]-1-oxo-5,6,7,8-tetrahydrophthala zin-2-yl] acetic acid | Int.188 | AS | 443 | 443 |
| 118 | 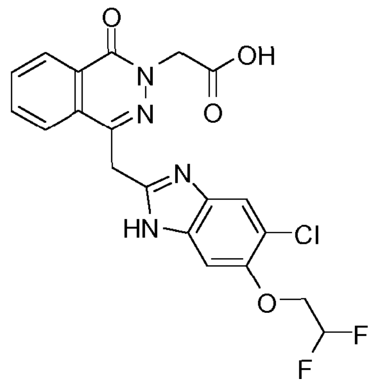 | 2-[4-[[5-chloro-6-(2,2-difluoroethoxy)-1H-benzimidazol-2-yl]methyl]-1-oxo-phthalazin- 2-yl]acetic acid | Int.184 | AS | 449 | 449 |

(continued)

| Cpd # | Structure | Name | Int | Mtd | MW (calc) | MW (obs) |
|---|---|---|---|---|---|---|
| 119 | 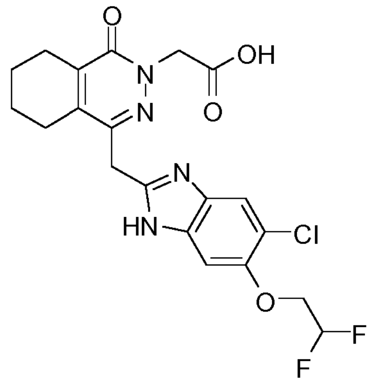 | 2-[4-[[5-chloro-6-(2,2-difluoroethoxy)-1H-benzimidazol-2-yl]methyl]-1-oxo-5,6,7,8-tetrahydrophthala zin-2-yl] acetic acid | Int.183 | AS | 453 | 453 |
| 120 | 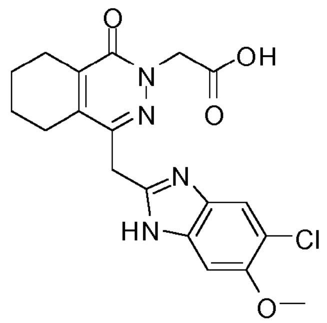 | 2-[4-[(5-chloro-6-methoxy-1H-benzimidazol-2-yl)methyl]-1-oxo-5,6,7,8-tetrahydrophthala zin-2-yl] acetic acid | Int.175 | AS | 403 | 403 |

(continued)

| Cpd # | Structure | Name | Int | Mtd | MW (calc) | MW (obs) |
|---|---|---|---|---|---|---|
| 121 | 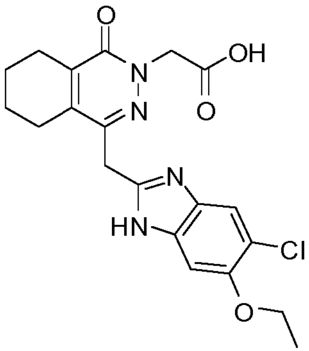 | 2-[4-[(5-chloro-6-ethoxy-1H-benzimidazol-2-yl)methyl]-1-oxo-5,6,7,8-tetrahydrophthala zin-2-yl] acetic acid | Int.215 | AS | 417 | 417 |
| 122 | 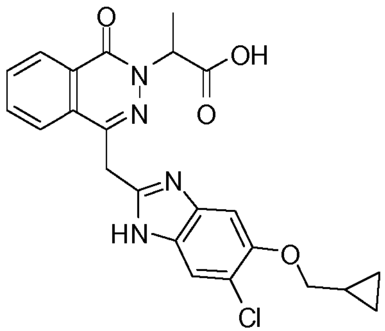 | 2-[4-(6-Chloro-5-cyclopropylmeth oxy -1H-benzoimidazol-2-ylmethyl)-1-oxo -1H-phthalazin-2-yl]-propionic acid | Int.200 | AS | 453 | 453 |

(continued)

| Cpd # | Structure | Name | Int | Mtd | MW (calc) | MW (obs) |
|---|---|---|---|---|---|---|
| 123 | 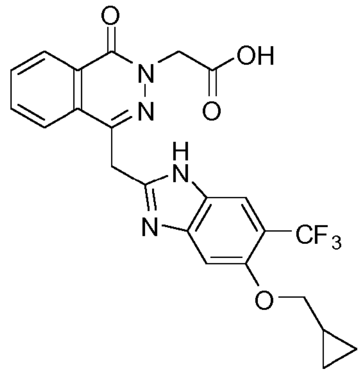 | 2-[4-[[5-(cyclopropylmeth oxy)-6-(trifluoromethyl)-1H-benzimidazol-2-yl]methyl]-1-oxo-phthalazin-2-yl]acetic acid | Int.181 | AS | 472 | 473 |
| 124 | 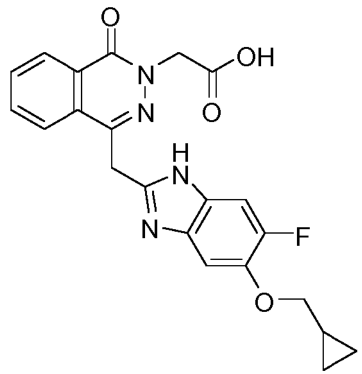 | 2-[4-[[5-(cyclopropylmeth oxy)-6-fluoro-1H-benzimidazol-2-yl]methyl]-1-oxo-phthalazin-2-yl]acetic acid | Int.182 | AS | 422 | 423 |
| 125 | 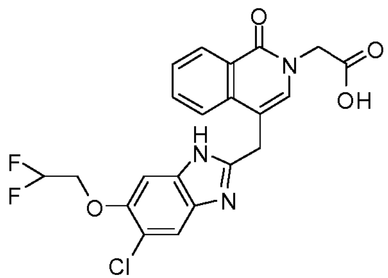 | {4-[5-Chloro-6-(2,2-difluoro-ethoxy)-1H-benzoimidazol-2-ylmethyl]-1-oxo-1H-isoquinolin-2-yl}-acetic acid | Int. 197 | AS | 448 | 448 |

(continued)

| Cpd # | Structure | Name | Int | Mtd | MW (calc) | MW (obs) |
|---|---|---|---|---|---|---|
| 126 | 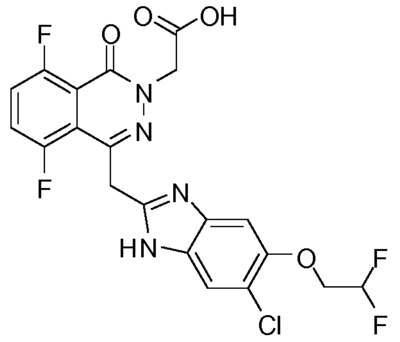 | 2-[4-[[6-chloro-5-(2,2-difluoroethoxy)-1H-benzimidazol-2-yl]methyl]-5,8-difluoro-1-oxo-phthalazin- 2-yl]acetic acid | Int.198 | AS | 485 | 485 |
| 127 | 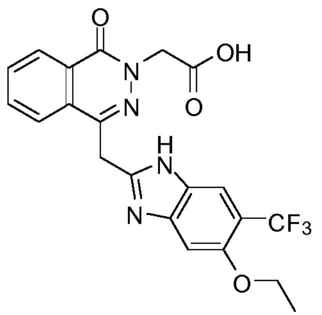 | 2-[4-[[5-ethoxy-6-(trifluoromethyl)-1H-benzimidazol-2-yl]methyl]-1-oxo-phthalazin-2-yl]acetic acid | Int. 196 | AS | 446 | 447 |
| 128 | 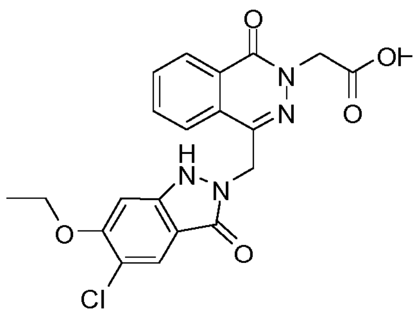 | 2-[4-[(5-chloro-6-ethoxy-3-oxo-1H-indazol-2-yl)methyl]-1-oxo-phthalazin-2-yl]acetic acid | Int.52 | AS | 429 | 429 |

(continued)

| Cpd # | Structure | Name | Int | Mtd | MW (calc) | MW (obs) |
|---|---|---|---|---|---|---|
| 129 | 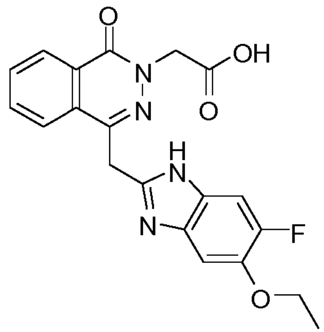 | 2-[4-[(5-ethoxy-6-fluoro-1H-benzimidazol-2-yl)methyl]-1-oxo-phthalazin-2-yl]acetic acid | Int.176 | AS | 396 | 397 |
| 130 | 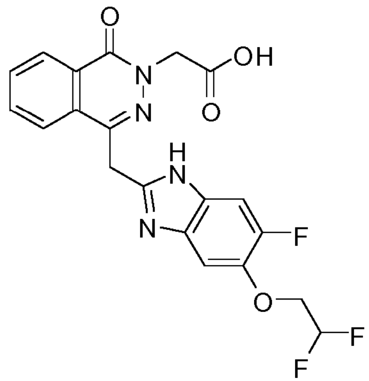 | 2-[4-[[5-(2,2-difluoroethoxy)-6-fluoro-1H-benzimidazol-2-yl]methyl]-1-oxo-phthalazin-2-yl]acetic acid | Int.180 | AS | 432 | 433 |

(continued)

| Cpd # | Structure | Name | Int | Mtd | MW (calc) | MW (obs) |
|---|---|---|---|---|---|---|
| 131 | 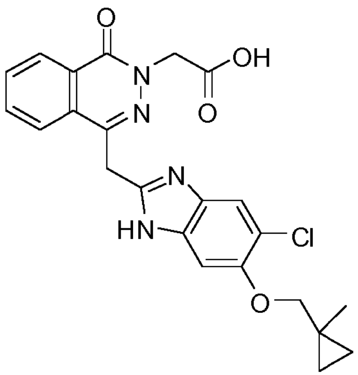 | 2-[4-[[5-chloro-6-[(1-methylcycloprop yl)methoxy]-1H-benzimidazol-2-yl]methyl]-1-oxo-phthalazin-2-yl]acetic acid | Int. 194 | AS | 453 | 453 |
| 132 | 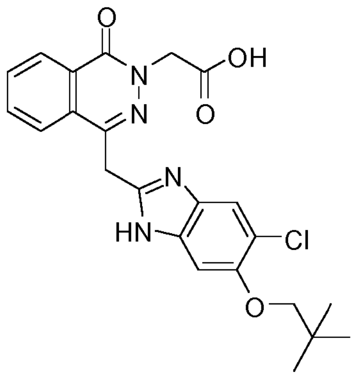 | 2-[4-[[5-chloro-6-(2,2-dimethylpropoxy )-1H-benzimidazol-2-yl]methyl]-1-oxo-phthalazin-2-yl]acetic acid | Int.186 | AS | 455 | 455 |

(continued)

| Cpd # | Structure | Name | Int | Mtd | MW (calc) | MW (obs) |
|---|---|---|---|---|---|---|
| 133 | 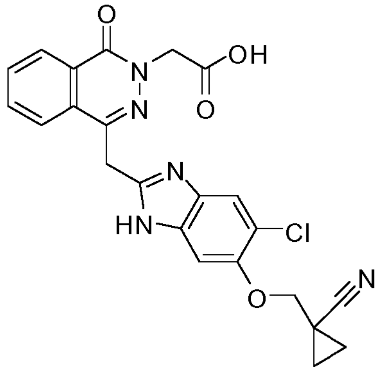 | 2-[4-[[5-chloro-6-[(1-cyanocyclopropyl )methoxy]-1H-benzimidazol-2-yl]methyl]-1-oxo-phthalazin-2-yl]acetic acid | Int.193 | AS | 464 | 464 |
| 134 | 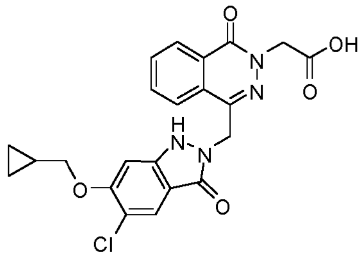 | 2-[4-[[5-chloro-6-(cyclopropylmeth oxy)-3-oxo-1H-indazol-2-yl]methyl]-1-oxo-phthalazin-2-yl]acetic acid | Int.55 | AS | 455 | 455 |
| 135 | 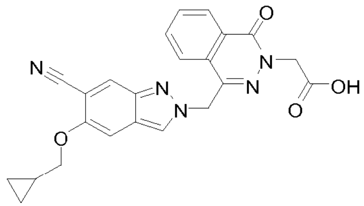 | 2-[4-[[6-cyano-5-(cyclopropylmeth oxy)indazol-2-yl]methyl]-1-oxo-phthalazin-2-yl]acetic acid | Int.110 | AS | 429 | 430 |

(continued)

| Cpd # | Structure | Name | Int | Mtd | MW (calc) | MW (obs) |
|---|---|---|---|---|---|---|
| 136 | 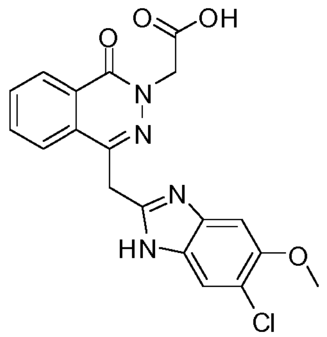 | 2-[4-[(6-chloro-5-methoxy-1H-benzimidazol-2-yl)methyl]-1-oxo-phthalazin-2-yl]acetic acid | Int.178 | AS | 399 | 399 |
| 137 | 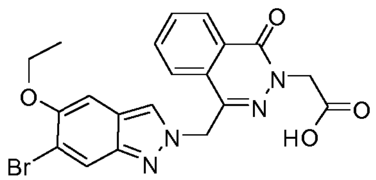 | 2-[4-[(6-bromo-5-ethoxy-indazol-2-yl)methyl]-1-oxo-phthalazin-2-yl] acetic acid | Int.274 | AS | 457 | 457 |
| 138 | 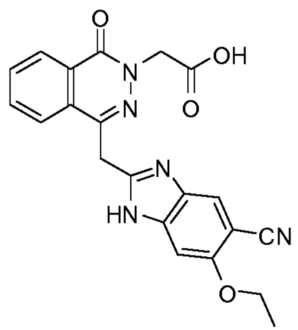 | 2-[4-[(5-cyano-6-ethoxy-1H-benzimidazol-2-yl)methyl]-1-oxo-phthalazin-2-yl]acetic acid | Int.22 | AT | 403 | 404 |

(continued)

| Cpd # | Structure | Name | Int | Mtd | MW (calc) | MW (obs) |
|---|---|---|---|---|---|---|
| 139 | 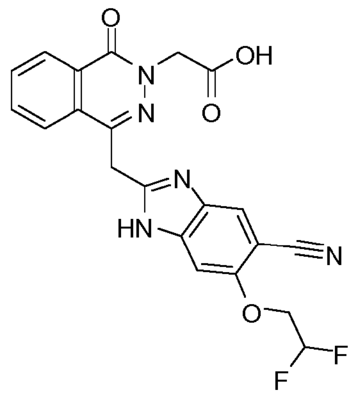 | 2-[4-[[5-cyano-6-(2,2-difluoroethoxy)-1H-benzimidazol-2-yl]methyl]-1-oxo-phthalazin-2-yl] acetic acid | Int.22 | AT | 439 | 440 |
| 140 | 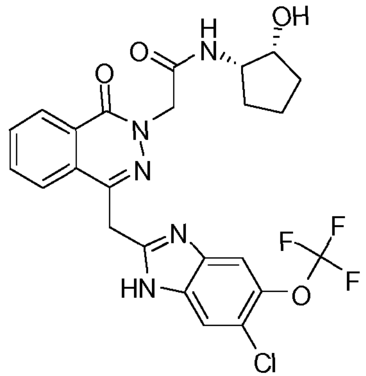 | 2-[4-[[6-chloro-5-(trifluoromethoxy )-1H-benzimidazol-2-yl]methyl]-1-oxo-phthalazin- 2-yl]-N-[(1S,2R)-2-hydroxycyclopen tyl] acetamide | Final compoun d 112 | AU | 536 | 536 |

(continued)

| Cpd # | Structure | Name | Int | Mtd | MW (calc) | MW (obs) |
|---|---|---|---|---|---|---|
| 141 | 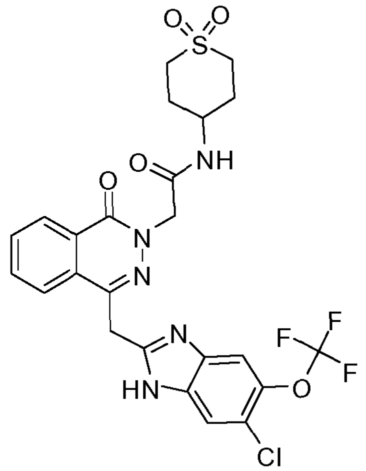 | 2-[4-[[6-chloro-5-(trifluoromethoxy )-1H-benzimidazol-2-yl]methyl]-1-oxo-phthalazin-2-yl]-N-(1,1-dioxothian-4-yl)acetamide | Final compound 112 | AU | 584 | 584 |
| 142 | 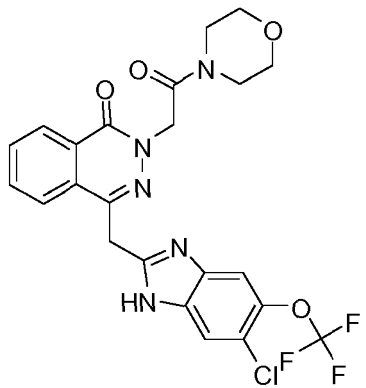 | 4-[[6-chloro-5-(trifluoromethoxy )-1H-benzimidazol-2-yl]methyl]-2-(2-morpholino-2-oxoethyl)phthalazin-1-one | Final compound 112 | AU | 522 | 522 |

(continued)

| Cpd # | Structure | Name | Int | Mtd | MW (calc) | MW (obs) |
|---|---|---|---|---|---|---|
| 143 | 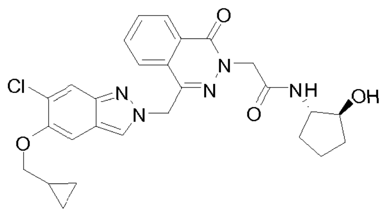 | 2-[4-[[6-chloro-5-(cyclopropylmeth oxy)indazol-2-yl]methyl]-1-oxo-phthalazin-2-yl]-N-[(1S,2S)-2-hydroxycyclopen tyl] acetamide | Final compoun d 114 | AU | 522 | 522 |
| 144 | 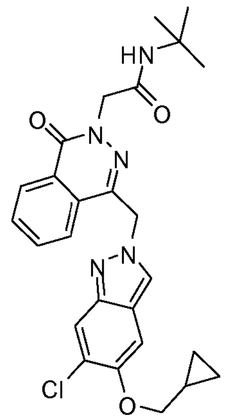 | N-tert-butyl-2-[4-[[6-chloro-5-(cyclopropylmeth oxy)indazol-2-yl] methyl]-1-oxo-phthalazin-2-yl]acetamide | Final compoun d 114 | AU | 494 | 494 |
| 145 | 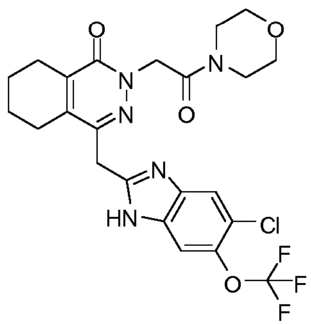 | 4-[[5-chloro-6-(trifluoromethoxy )-1H-benzimidazol-2-yl]methyl]-2-(2-morpholino-2-oxo-ethyl)-5,6,7,8-tetrahydrophthala zin-1-one | Final compoun d 113 | AU | 526 | 526 |

(continued)

| Cpd # | Structure | Name | Int | Mtd | MW (calc) | MW (obs) |
|---|---|---|---|---|---|---|
| 146 | 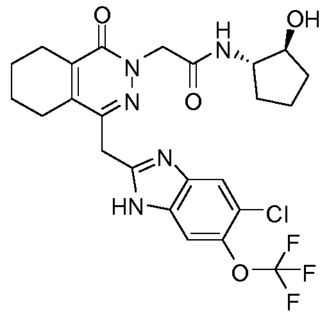 | 2-[4-[[5-chloro-6-(trifluoromethoxy )-1H-benzimidazol-2-yl]methyl]-1-oxo-5,6,7,8-tetrahydrophthala zin-2-yl]-N-[(1S,2S)-2-hydroxycyclopen tyl] acetamide | Final compoun d 113 | AU | 540 | 540 |
| 147 | 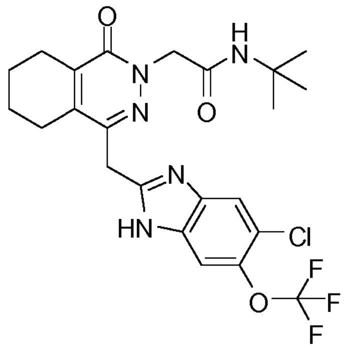 | 2-[4-[[5-chloro-6-(trifluoromethoxy )-1H-benzimidazol-2-yl]methyl]-1-oxo-5,6,7,8-tetrahydrophthala zin-2-yl]-N-[(1S,2S)-2-hydroxycyclopen tyl] acetamide | Final compoun d 113 | AU | 512 | 512 |
| 148 | 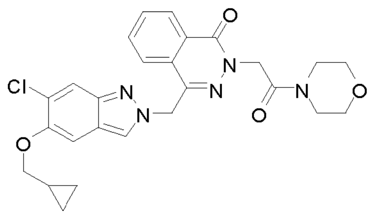 | 4-[[6-chloro-5-(cyclopropylmeth oxy)indazol-2-yl]methyl]-2-(2-morpholino-2-oxoethyl)phthalazin-1-one | Final compoun d 114 | AU | 508 | 508 |

(continued)

| Cpd # | Structure | Name | Int | Mtd | MW (calc) | MW (obs) |
|---|---|---|---|---|---|---|
| 149 | 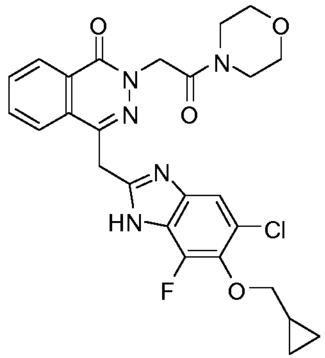 | 4-[[5-chloro-6-(cyclopropylmeth oxy)-7-fluoro-1H-benzimidazol-2-yl] methyl]-2-(2-morpholino-2-oxoethyl)phthalazin-1-one | Final compoun d 116 | AU | 526 | 526 |
| 150 | 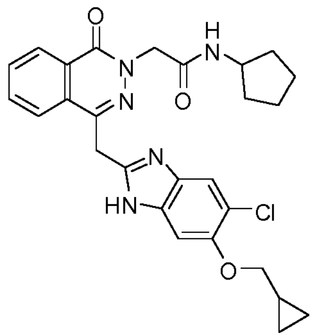 | 2-[4-[[5-chloro-6-(cyclopropylmeth oxy)-1H-benzimidazol-2-yl]methyl]-1-oxo-phthalazin-2-yl]-N-cyclopentyl-acetamide | Final compoun d 115 | AU | 506 | 506 |

(continued)

| Cpd # | Structure | Name | Int | Mtd | MW (calc) | MW (obs) |
|---|---|---|---|---|---|---|
| 151 | 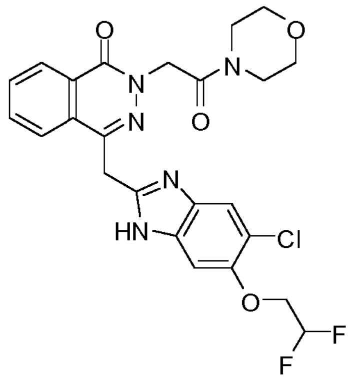 | 4-[[5-chloro-6-(2,2-difluoroethoxy)-1H-benzimidazol-2-yl]methyl]-2-(2-morpholino-2-oxoethyl)phthalazin-1-one | Final compound 118 | AU | 518 | 518 |
| 152 | 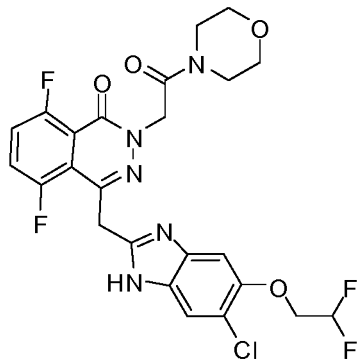 | 4-[[6-chloro-5-(2,2-difluoroethoxy)-1H-benzimidazol-2-yl]methyl]-5,8-difluoro-2-(2-morpholino-2-oxoethyl)phthalazin-1-one | Final compound 126 | AU | 554 | 554 |

(continued)

| Cpd # | Structure | Name | Int | Mtd | MW (calc) | MW (obs) |
|---|---|---|---|---|---|---|
| 153 | 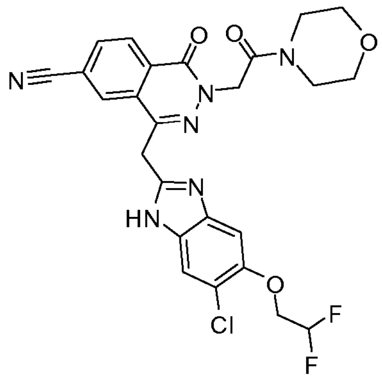 | 4-[[6-chloro-5-(2,2-difluoroethoxy)-1H-benzimidazol-2-yl]methyl]-2-(2-morpholino-2-oxo-ethyl)-1-oxo-phthalazine-6-carbonitrile | Final compound 23 | AU | 543 | 543 |
| 154 | 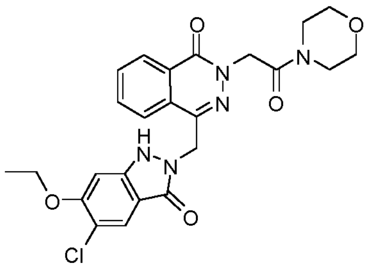 | 4-[(5-chloro-6-ethoxy-3-oxo-1H-indazol-2-yl)methyl]-2-(2-morpholino-2-oxoethyl)phthalazin-1-one | Final compound 128 | AU | 498 | 498 |
| 155 | 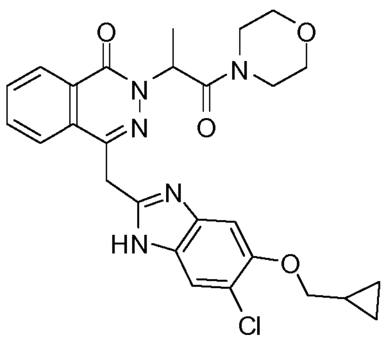 | 4-(6-Chloro-5-cyclopropylmeth oxy-1H-benzoimidazol-2-ylmethyl)-2-(1-methyl-2-morpholin-4-yl-2-oxo-ethyl)-2H-phthalazin-1-one | Final compound 22 | AU | 522 | 522 |

(continued)

| Cpd # | Structure | Name | Int | Mtd | MW (calc) | MW (obs) |
|---|---|---|---|---|---|---|
| 156 | 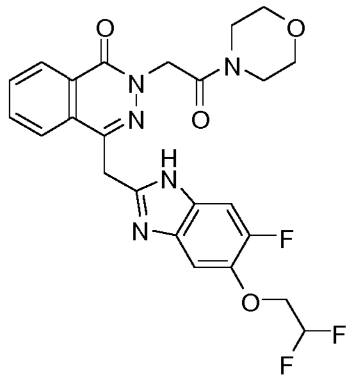 | 4-[[5-(2,2-difluoroethoxy)-6-fluoro-1H-benzimidazol-2-yl]methyl]-2-(2-morpholino-2-oxoethyl)phthalazin-1-one | Final compoun d 130 | AU | 501 | 502 |
| 157 | 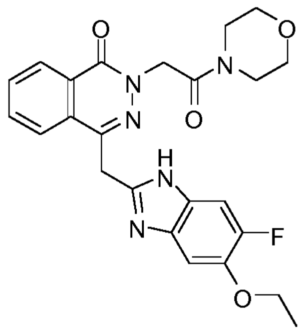 | 4-[(5-ethoxy-6-fluoro-1H-benzimidazol-2-yl)methyl]-2-(2-morpholino-2-oxoethyl)phthalazin-1-one | Final compoun d 129 | AU | 465 | 466 |

(continued)

| Cpd # | Structure | Name | Int | Mtd | MW (calc) | MW (obs) |
|---|---|---|---|---|---|---|
| 158 | 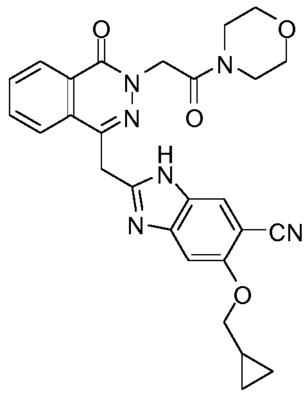 | 6-(cyclopropylmeth oxy)-2-[[3-(2-morpholino-2-oxo-ethyl)-4-oxo-phthalazin-1-yl]methyl]-3H-benzimidazole-5-carbonitrile | Final compoun d 7 | AU | 499 | 499 |
| 159 | 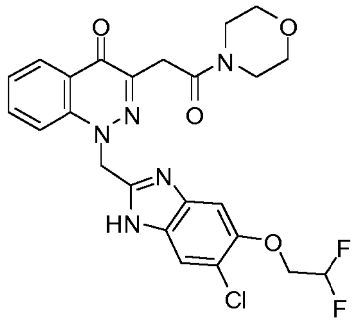 | 1-[6-Chloro-5-(2,2-difluoro-ethoxy)-1H-benzoimidazol-2-ylmethyl]-3-(2-morpholin-4-yl-2-oxo-ethyl)-1H-cinnolin-4-one | Int.78 | AU | 518 | 518 |

(continued)

| Cpd # | Structure | Name | Int | Mtd | MW (calc) | MW (obs) |
|---|---|---|---|---|---|---|
| 160 | 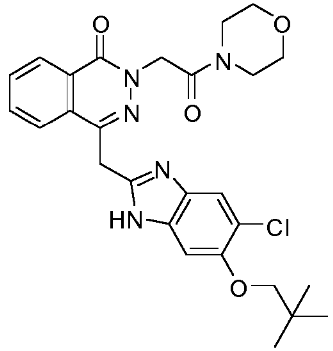 | 4-[[5-chloro-6-(2,2-dimethylpropoxy )-1H-benzimidazol-2-yl]methyl]-2-(2-morpholino-2-oxoethyl)phthalazin-1-one | Final compoun d 132 | AU | 524 | 524 |
| 161 | 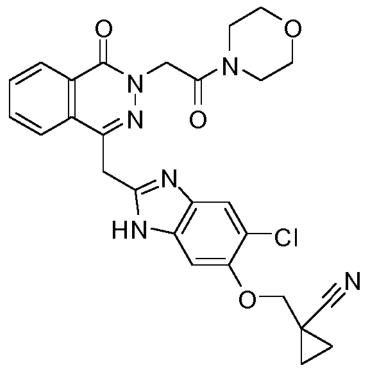 | 1-[[6-chloro-2-[[3-(2-morpholino-2-oxo-ethyl)-4-oxo-phthalazin-1-yl] methyl]-3H-benzimidazol-5-yl] oxymethyl]cyc lopropanecarboni trile | Final compoun d 133 | AU | 533 | 533 |

(continued)

| Cpd # | Structure | Name | Int | Mtd | MW (calc) | MW (obs) |
|---|---|---|---|---|---|---|
| 162 | 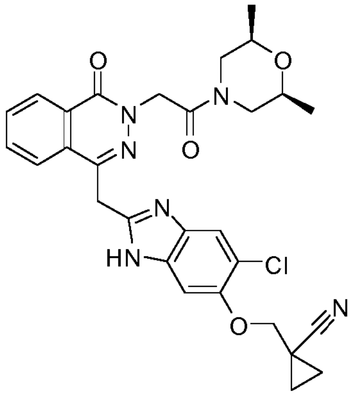 | 1-[[6-chloro-2-[[3-[2-[(2S,6R)-2,6-dimethylmorphol in-4-yl]-2-oxoethyl]-4-oxo-phthalazin-1-yl]methyl]-3H-benzimidazol-5-yl] oxymethyl]cyc lopropanecarboni trile | Final compoun d 133 | AU | 561 | 561 |
| 163 | 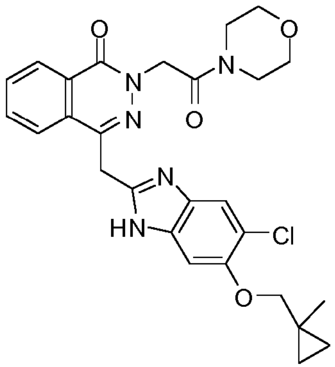 | 4-[[5-chloro-6-[(1-methylcycloprop yl)methoxy]-1H-benzimidazol-2-yl] methyl]-2-(2-morpholino-2-oxoethyl)phthalazin-1-one | Final compoun d 131 | AU | 522 | 522 |

(continued)

| Cpd # | Structure | Name | Int | Mtd | MW (calc) | MW (obs) |
|---|---|---|---|---|---|---|
| 164 | 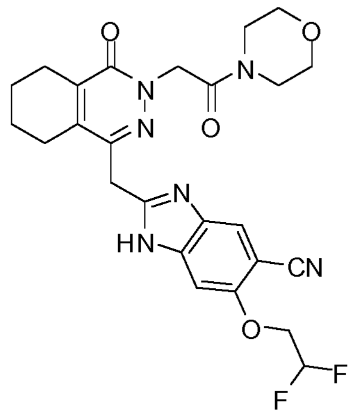 | 6-(2,2-difluoroethoxy)-2-[[3-(2-morpholino-2-oxo-ethyl)-4-oxo-5,6,7,8-tetrahydrophthala zin-1-yl]methyl]-1H-benzimidazole-5-carbonitrile | Int.214 | AU | 513 | 513 |
| 165 | 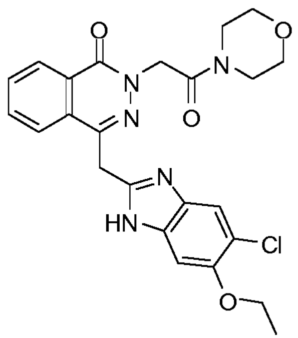 | 4-[(5-chloro-6-ethoxy-1H-benzimidazol-2-yl)methyl]-2-(2-morpholino-2-oxoethyl)phthalazin-1-one | Final compoun d 6 | AU | 482 | 482 |

(continued)

| Cpd # | Structure | Name | Int | Mtd | MW (calc) | MW (obs) |
|---|---|---|---|---|---|---|
| 166 | 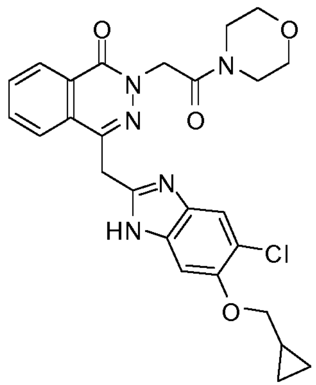 | 4-[[5-chloro-6-(cyclopropylmeth oxy)-1H-benzimidazol-2-yl]methyl]-2-(2-morpholino-2-oxoethyl)phthalazin-1-one | Final compoun d 115 | AU | 508 | 508 |
| 167 | 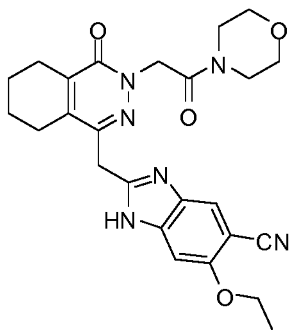 | 6-ethoxy-2-[[3-(2-morpholino-2-oxo-ethyl)-4-oxo-5,6,7,8-tetrahydrophthala zin-1-yl]methyl]-1H-benzimidazole-5-carbonitrile | Int. 163 | AU | 477 | 477 |
| 168 | 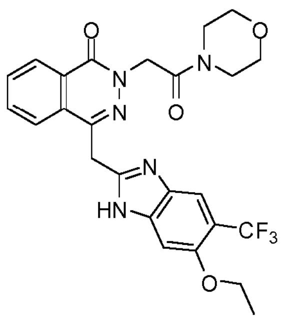 | 4-[[6-ethoxy-5-(trifluoromethyl)-1H-benzimidazol-2-yl]methyl]-2-(2-morpholino-2-oxoethyl)phthalazin-1-one | Final compoun d 127 | AU | 515 | 516 |

(continued)

| Cpd # | Structure | Name | Int | Mtd | MW (calc) | MW (obs) |
|---|---|---|---|---|---|---|
| 169 | 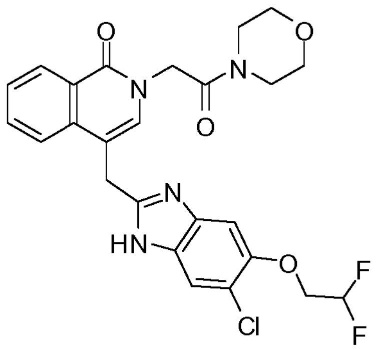 | 4-[6-Chloro-5-(2,2-difluoro-ethoxy)-1H-benzoimidazol-2-ylmethyl]-2-(2-morpholin-4-yl-2-oxo-ethyl)-2H - isoquinolin-1-one | Final compoun d 125 | AU | 517 | 517 |
| 170 | 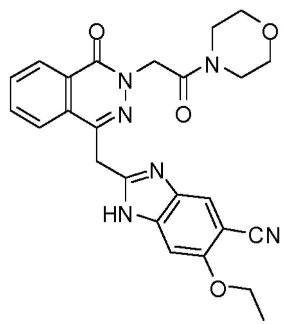 | 6-ethoxy-2-[[3-(2-morpholino-2-oxo-ethyl)-4-oxo-phthalazin-1-yl] methyl]-1H-benzimidazole-5-carbonitrile | Final compoun d 138 | AU | 473 | 473 |

(continued)

| Cpd # | Structure | Name | Int | Mtd | MW (calc) | MW (obs) |
|---|---|---|---|---|---|---|
| 171 | 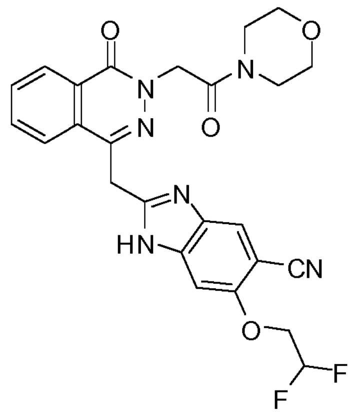 | 6-(2,2-difluoroethoxy)-2-[[3-(2-morpholino-2-oxo-ethyl)-4-oxo-phthalazin-1-yl]methyl]-1H-benzimidazole-5-carbonitrile | Final compound 139 | AU | 508 | 509 |
| 172 | 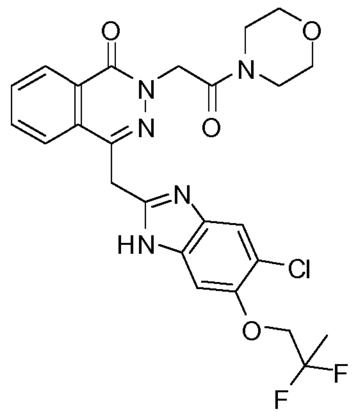 | 4-[[5-chloro-6-(2,2-difluoropropoxy) -1H-benzimidazol-2-yl]methyl]-2-(2-morpholino-2-oxoethyl)phthalazin-1-one | Int.445 | AU | 532 | 532 |

(continued)

| Cpd # | Structure | Name | Int | Mtd | MW (calc) | MW (obs) |
|---|---|---|---|---|---|---|
| 173 | 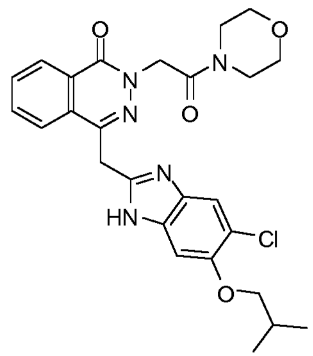 | 4-[(5-chloro-6-isobutoxy-1H-benzimidazol-2-yl)methyl] -2-(2-morpholino-2-oxoethyl)phthalazin-1-one | Int.447 | AU | 510 | 510 |
| 174 | 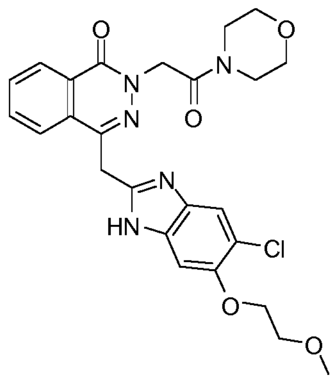 | 4-[[5-chloro-6-(2-methoxyethoxy)-1H-benzimidazol-2-yl]methyl]-2-(2-morpholino-2-oxo-ethyl)phthalazin-1-one | Int.449 | AU | 512 | 512 |

(continued)

| Cpd # | Structure | Name | Int | Mtd | MW (calc) | MW (obs) |
|---|---|---|---|---|---|---|
| 175 | 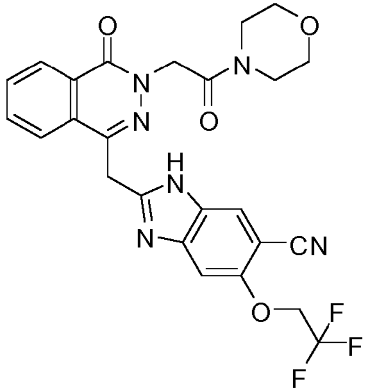 | 2-[[3-(2-morpholino-2-oxo-ethyl)-4-oxo-phthalazin-1-yl]methyl]-6-(2,2,2-trifluoroethoxy)-3H-benzimidazole-5-carbonitrile | Int. 165 | AU | 526 | 527 |
| 176 | 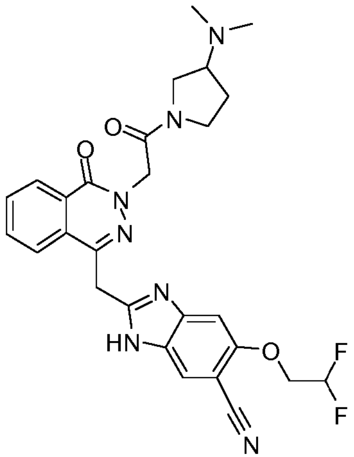 | 6-(2,2-difluoroethoxy)-2-[[3-[2-[3-(dimethylamino) pyrrolidin-1-yl]-2-oxo-ethyl]-4-oxo-phthalazin-1-yl]methyl]-3H-benzimidazole-5-carbonitrile | Final compound 139 | AU | 536 | 536 |

(continued)

| Cpd # | Structure | Name | Int | Mtd | MW (calc) | MW (obs) |
|---|---|---|---|---|---|---|
| 177 | 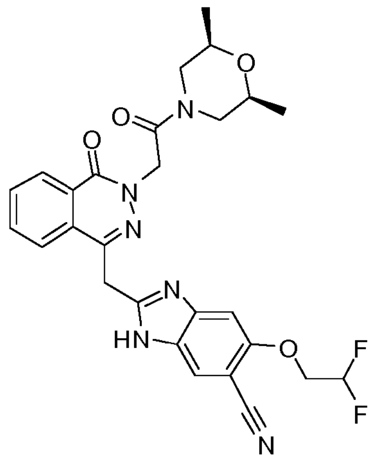 | 6-(2,2-difluoroethoxy)-2-[[3-[2-[(2S,6R)-2,6-dimethylmorphol in-4-yl]-2-oxo-ethyl]-4-oxo-phthalazin-1-yl]methyl]-3H-benzimidazole-5-carbonitrile | Final compoun d 139 | AU | 537 | 537 |
| 178 | 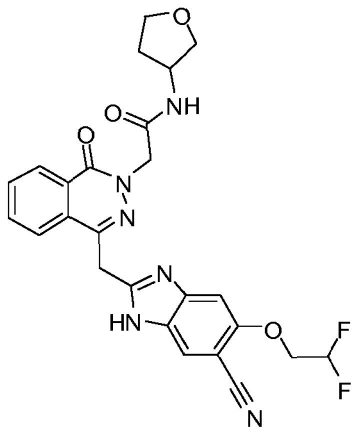 | 2-[4-[[6-cyano-5-(2,2-difluoroethoxy)-1H-benzimidazol-2-yl]methyl]-1-oxo-phthalazin-2-yl]-N-THF-3-yl-acetamide | Final compoun d 139 | AU | 508 | 509 |

(continued)

| Cpd # | Structure | Name | Int | Mtd | MW (calc) | MW (obs) |
|---|---|---|---|---|---|---|
| 179 | 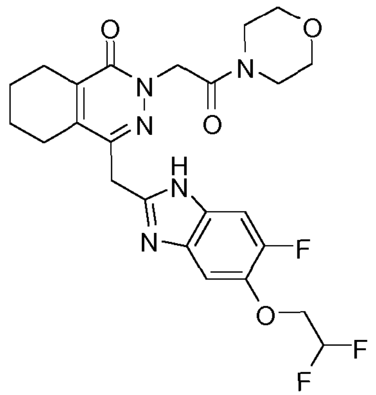 | 4-[[5-(2,2-difluoroethoxy)-6-fluoro-1H-benzimidazol-2-yl]methyl]-2-(2-morpholino-2-oxo-ethyl)-5,6,7,8-tetrahydrophthala zin-1-one | Int.446 | AU | 505 | 506 |
| 180 | 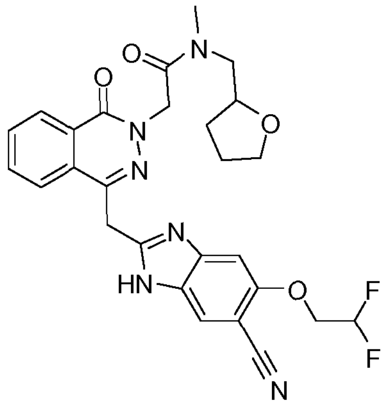 | 2-[4-[[6-cyano-5-(2,2-difluoroethoxy)-1H-benzimidazol-2-yl]methyl]-1-oxo-phthalazin-2-yl]-N-methyl-N-(THF-2-ylmethyl)acetami de | Final compoun d 139 | AU | 537 | 537 |

(continued)

| Cpd # | Structure | Name | Int | Mtd | MW (calc) | MW (obs) |
|---|---|---|---|---|---|---|
| 181 | 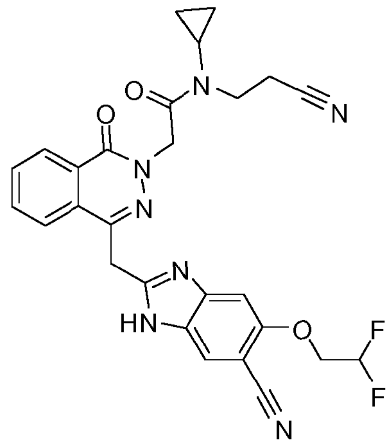 | 2-[4-[[6-cyano-5-(2,2-difluoroethoxy)-1H-benzimidazol-2-yl]methyl]-1-oxo-phthalazin-2-yl]-N-(2-cyanoethyl)-N-cyclopropyl-acetamide | Final compound 139 | AU | 532 | 532 |
| 182 | 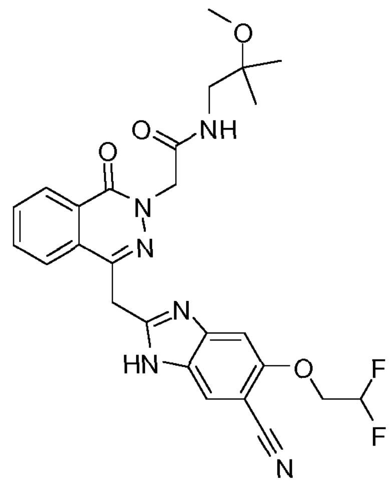 | 2-[4-[[6-cyano-5-(2,2-difluoroethoxy)-1H-benzimidazol-2-yl]methyl]-1-oxo-phthalazin-2-yl]-N-(2-methoxy-2-methylpropyl)acetamide | Final compound 139 | AU | 525 | 525 |

(continued)

| Cpd # | Structure | Name | Int | Mtd | MW (calc) | MW (obs) |
|---|---|---|---|---|---|---|
| 183 | 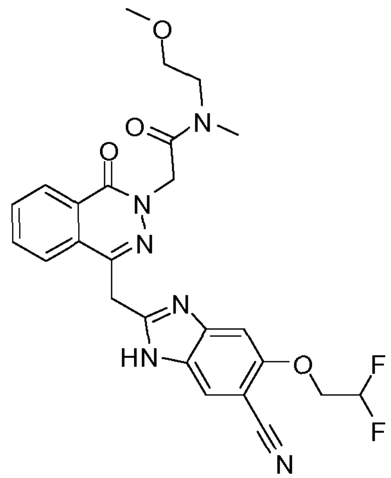 | 2-[4-[[6-cyano-5-(2,2-difluoroethoxy)-1H-benzimidazol-2-yl]methyl]-1-oxo-phthalazin-2-yl]-N-(2-methoxyethyl)-N-methyl-acetamide | Final compoun d 139 | AU | 511 | 511 |
| 184 | 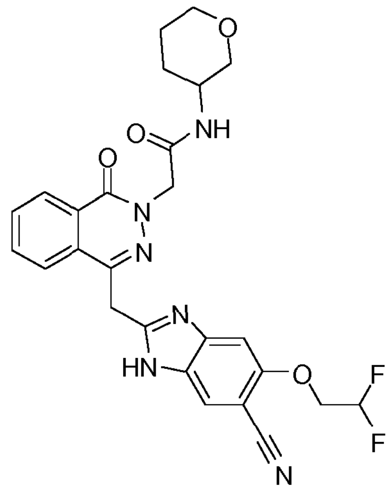 | 2-[4-[[6-cyano-5-(2,2-difluoroethoxy)-1H-benzimidazol-2-yl]methyl]-1-oxo-phthalazin-2-yl]-N-tetrahydropyran-3-yl-acetamide | Final compoun d 139 | AU | 523 | 523 |

(continued)

| Cpd # | Structure | Name | Int | Mtd | MW (calc) | MW (obs) |
|---|---|---|---|---|---|---|
| 185 | 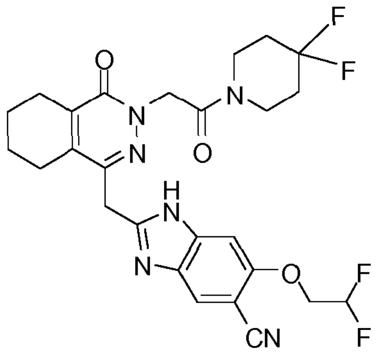 | 6-(2,2-difluoroethoxy)-2-[[3-[2-(4,4-difluoro-1-piperidyl)-2-oxo-ethyl]-4-oxo-5,6,7,8-tetrahydrophthala zin-1-yl]methyl]-1H-benzimidazole-5-carbonitrile | Int. 164 | AU | 547 | 547 |
| 186 | 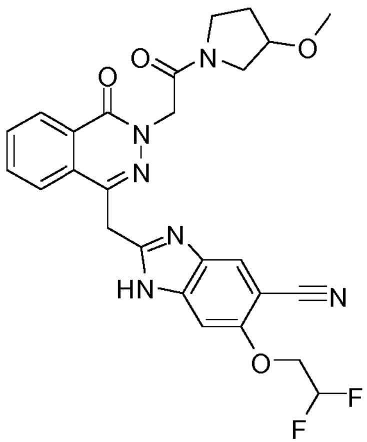 | 6-(2,2-difluoroethoxy)-2-[[3-[2-(3-methoxypyrrolidi n-1-yl)-2-oxo-ethyl]-4-oxo-phthalazin-1-yl]methyl]-1H-benzimidazole-5-carbonitrile | Final compoun d 139 | AU | 523 | 523 |

(continued)

| Cpd # | Structure | Name | Int | Mtd | MW (calc) | MW (obs) |
|---|---|---|---|---|---|---|
| 187 | 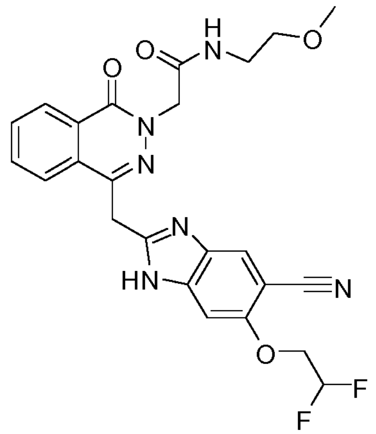 | 2-[4-[[5-cyano-6-(2,2-difluoroethoxy)-1H-benzimidazol-2-yl]methyl]-1-oxo-phthalazin-2-yl]-N-(2-methoxyethyl)ace tamide | Final compoun d 139 | AU | 496 | 497 |
| 188 | 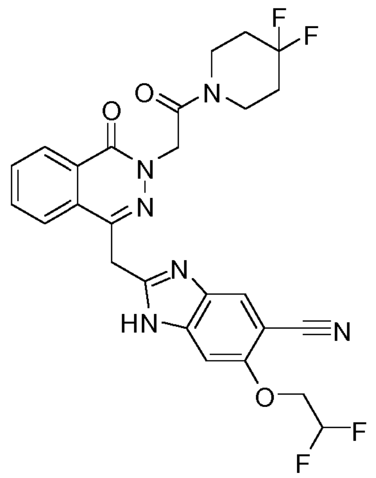 | 6-(2,2-difluoroethoxy)-2-[[3-[2-(4,4-difluoro-1-piperidyl)-2-oxo-ethyl]-4-oxo-phthalazin-1-yl]methyl]-1H-benzimidazole-5-carbonitrile | Final compoun d 139 | AU | 542 | 543 |

(continued)

| Cpd # | Structure | Name | Int | Mtd | MW (calc) | MW (obs) |
|---|---|---|---|---|---|---|
| 189 | 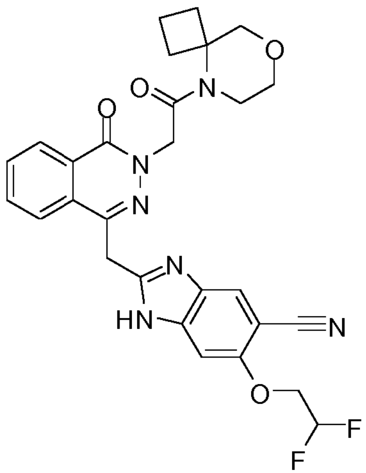 | 6-(2,2-difluoroethoxy)-2-[[3-[2-(6-oxa-9-azaspiro[3.5]non an-9-yl)-2-oxo-ethyl]-4-oxo-phthalazin-1-yl]methyl]-1H-benzimidazole-5-carbonitrile | Final compoun d 139 | AU | 549 | 549 |
| 190 | 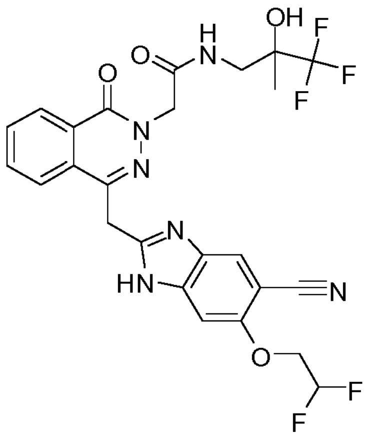 | 2-[4-[[5-cyano-6-(2,2-difluoroethoxy)-1H-benzimidazol-2-yl]methyl]-1-oxo-phthalazin-2-yl]-N-(3,3,3-trifluoro-2-hydroxy-2-methylpropyl) acetamide | Final compoun d 139 | AU | 564 | 565 |

(continued)

| Cpd # | Structure | Name | Int | Mtd | MW (calc) | MW (obs) |
|---|---|---|---|---|---|---|
| 191 | 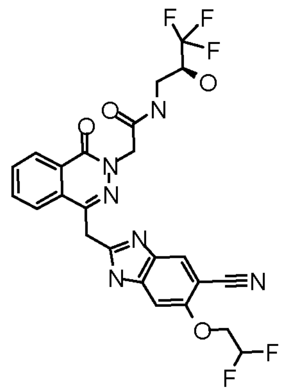 | 2-[4-[[5-cyano-6-(2,2-difluoroethoxy)-1H-benzimidazol-2-yl]methyl]-1-oxo-phthalazin-2-yl]-N-[(2S)-3,3,3-trifluoro-2-hydroxypropyl] acetamide | Final compoun d 139 | AU | 550 | 551 |
| 192 | 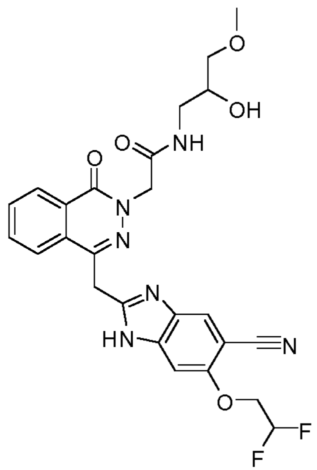 | 2-[4-[[5-cyano-6-(2,2-difluoroethoxy)-1H-benzimidazol-2-yl]methyl]-1-oxo-phthalazin-2-yl]-N-(2-hydroxy-3-methoxypropyl)acetamide | Final compoun d 139 | AU | 526 | 549 |

(continued)

| Cpd # | Structure | Name | Int | Mtd | MW (calc) | MW (obs) |
|---|---|---|---|---|---|---|
| 193 | 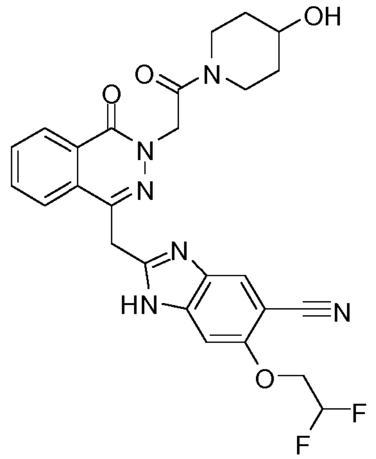 | 6-(2,2-difluoroethoxy)-2-[[3-[2-(4-hydroxy-1-piperidyl)-2-oxo-ethyl]-4-oxo-phthalazin-1-yl]methyl]-1H-benzimidazole-5-carbonitrile | Final compoun d 139 | AU | 523 | 523 |
| 194 | 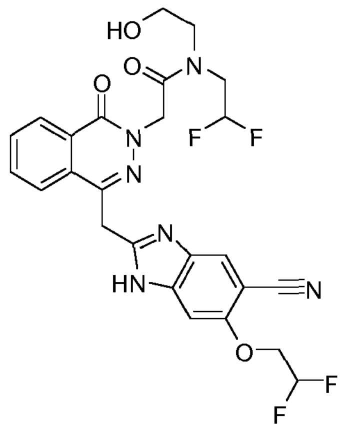 | 2-[4-[[5-cyano-6-(2,2-difluoroethoxy)-1H-benzimidazol-2-yl]methyl]-1-oxo-phthalazin-2-yl]-N-(2,2-difluoroethyl)-N - (2-hydroxyethyl)ace tamide | Final compoun d 139 | AU | 546 | 547 |

(continued)

| Cpd # | Structure | Name | Int | Mtd | MW (calc) | MW (obs) |
|---|---|---|---|---|---|---|
| 195 | 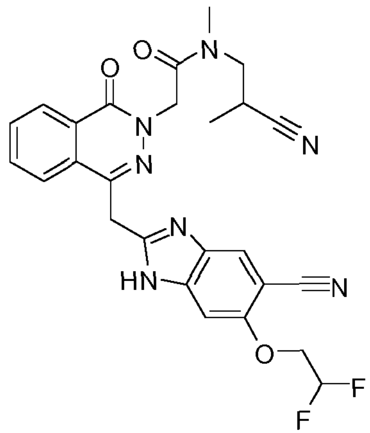 | 2-[4-[[5-cyano-6-(2,2-difluoroethoxy)-1H-benzimidazol-2-yl]methyl]-1-oxo-phthalazin-2-yl]-N-(2-cyanopropyl)-N-methyl-acetamide | Final compoun d 139 | AU | 520 | 520 |
| 196 | 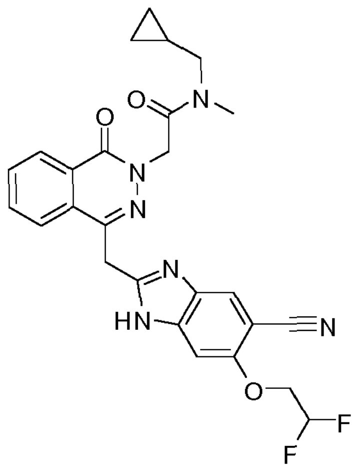 | 2-[4-[[5-cyano-6-(2,2-difluoroethoxy)-1H-benzimidazol-2-yl]methyl]-1-oxo-phthalazin-2-yl]-N-(cyclopropylmeth yl)-N-methyl-acetamide | Final compoun d 139 | AU | 507 | 507 |

(continued)

| Cpd # | Structure | Name | Int | Mtd | MW (calc) | MW (obs) |
|---|---|---|---|---|---|---|
| 197 | 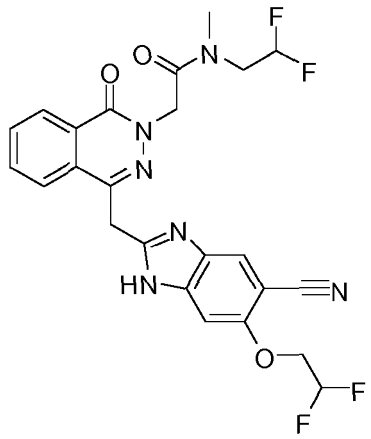 | 2-[4-[[5-cyano-6-(2,2-difluoroethoxy)-1H-benzimidazol-2-yl]methyl]-1-oxo-phthalazin-2-yl]-N-(2,2-difluoroethyl)-N - methyl-acetamide | Final compoun d 139 | AU | 516 | 517 |
| 198 | 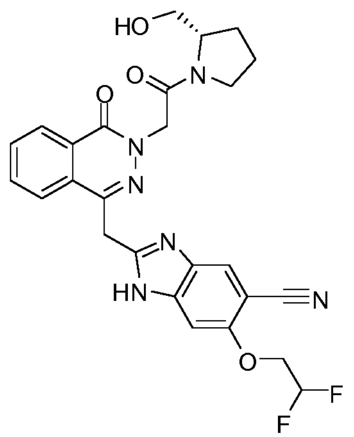 | 6-(2,2-difluoroethoxy)-2-[[3-[2-[(2S)-2-(hydroxymethyl) pyrrolidin-1-yl]-2-oxo-ethyl]-4-oxo-phthalazin-1-yl]methyl]-1H-benzimidazole-5-carbonitrile | Final compoun d 139 | AU | 523 | 523 |

(continued)

| Cpd # | Structure | Name | Int | Mtd | MW (calc) | MW (obs) |
|---|---|---|---|---|---|---|
| 199 | 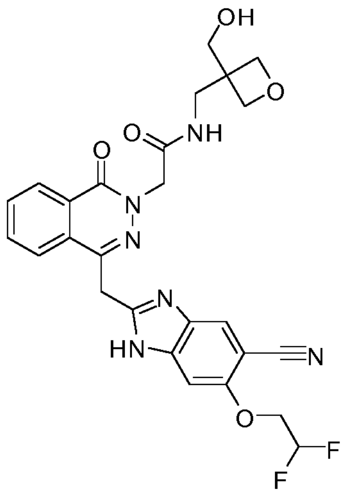 | 2-[4-[[5-cyano-6-(2,2-difluoroethoxy)-1H-benzimidazol-2-yl]methyl]-1-oxo-phthalazin-2-yl]-N-[[3-(hydroxymethyl) oxetan-3-yl]methyl]acetamide | Final compound 139 | AU | 539 | 539 |
| 200 | 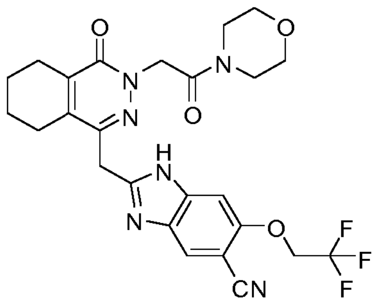 | 2-[[3-(2-morpholino-2-oxo-ethyl)-4-oxo-5,6,7,8-tetrahydrophthala zin-1-yl]methyl]-6-(2,2,2-trifluoroethoxy)-1H-benzimidazole-5-carbonitrile | Int.448 | AU | 531 | 531 |

(continued)

| Cpd # | Structure | Name | Int | Mtd | MW (calc) | MW (obs) |
|---|---|---|---|---|---|---|
| 201 | 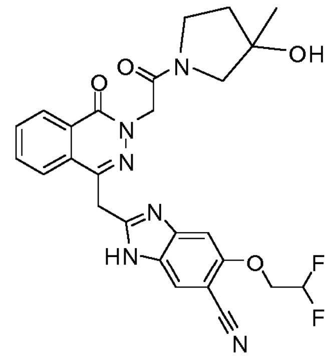 | 6-(2,2-difluoroethoxy)-2-[[3-[2-(3-hydroxy-3-methylpyrrolidin-1-yl)-2-oxo-ethyl]-4-oxo-phthalazin-1-yl]methyl]-3H-benzimidazole-5-carbonitrile | Final compound 139 | AU | 523 | 523 |
| 202 | 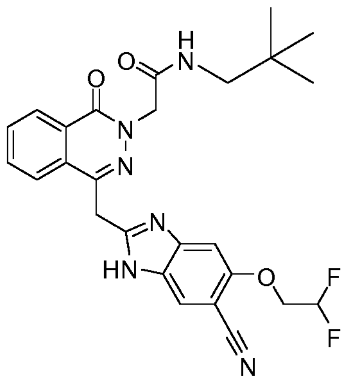 | 2-[4-[[6-cyano-5-(2,2-difluoroethoxy)-1H-benzimidazol-2-yl]methyl]-1-oxo-phthalazin-2-yl]-N-(2,2-dimethylpropyl)a cetamide | Final compound 139 | AU | 509 | 509 |

(continued)

| Cpd # | Structure | Name | Int | Mtd | MW (calc) | MW (obs) |
|---|---|---|---|---|---|---|
| 203 | 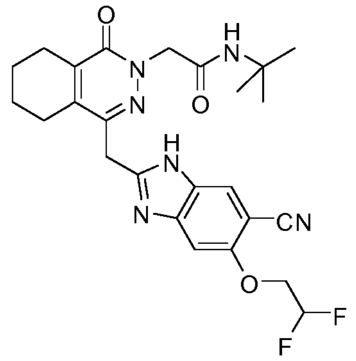 | N-tert-butyl-2-[4-[[6-cyano-5-(2,2-difluoroethoxy)-1H-benzimidazol-2-yl]methyl]-1-oxo-5,6,7,8-tetrahydrophthala zin-2-yl]acetamide | Int. 164 | AU | 499 | 499 |
| 204 | 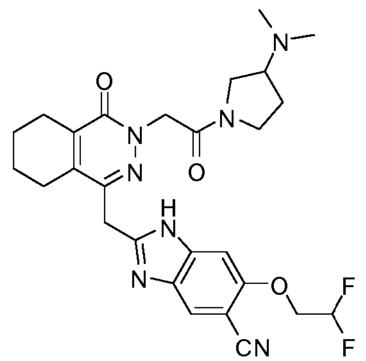 | 6-(2,2-difluoroethoxy)-2-[[3-[2-[3-(dimethylamino) pyrrolidin-1-yl]-2-oxo-ethyl]-4-oxo-5,6,7,8-tetrahydrophthala zin-1-yl]methyl]-1H-benzimidazole-5-carbonitrile | Int. 164 | AU | 540 | 499 |

(continued)

| Cpd # | Structure | Name | Int | Mtd | MW (calc) | MW (obs) |
|---|---|---|---|---|---|---|
| 205 | 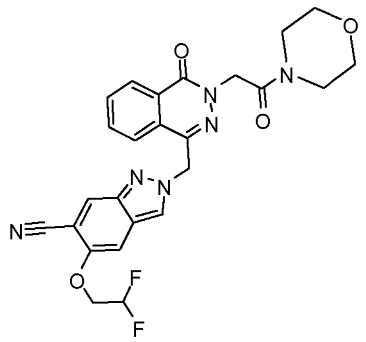 | 5-(2,2-difluoroethoxy)-2-[[3-(2-morpholino-2-oxo-ethyl)-4-oxo-phthalazin-1-yl]methyl]indazol e-6-carbonitrile | Final compoun d 8 | AU | 508 | 509 |
| 206 | 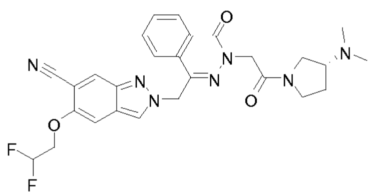 | 5-(2,2-difluoroethoxy)-2-[[3-[2-[(3R)-3-(dimethylamino) pyrrolidin-1-yl]-2-oxo-ethyl]-4-oxo-phthalazin-1-yl]methyl]indazol e-6-carbonitrile | Final compoun d 8 | AU | 536 | 536 |
| 207 | 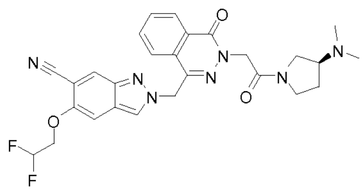 | 5-(2,2-difluoroethoxy)-2-[[3-[2-[(3S)-3-(dimethylamino) pyrrolidin-1-yl]-2-oxo-ethyl]-4-oxo-phthalazin-1-yl]methyl]indazol e-6-carbonitrile | Final compoun d 8 | AU | 536 | 536 |
| 208 | 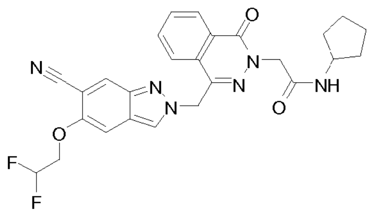 | 2-[4-[[6-cyano-5-(2,2-difluoroethoxy)in dazol-2-yl]methyl]-1-oxo-phthalazin-2-yl]-N-cyclopentyl-acetamide | Final compoun d 8 | AU | 507 | 440 |

(continued)

| Cpd # | Structure | Name | Int | Mtd | MW (calc) | MW (obs) |
|---|---|---|---|---|---|---|
| 209 | 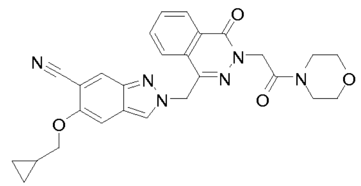 | 5-(cyclopropylmeth oxy)-2-[[3-(2-morpholino-2-oxo-ethyl)-4-oxo-phthalazin-1-yl]methyl]indazol e-6-carbonitrile | Final compoun d 135 | AU | 499 | 499 |
| 210 | 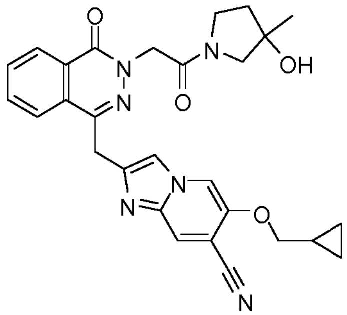 | 6-(cyclopropylmeth oxy)-2-[[3-[2-(3-hydroxy-3-methylpyrrolidin-1-yl)-2-oxo-ethyl]-4-oxo-phthalazin-1-yl]methyl]imidaz o[1,2-a]pyridine-7-carbonitrile | Final compoun d 79 | AU | 513 | 514 |
| 211 | 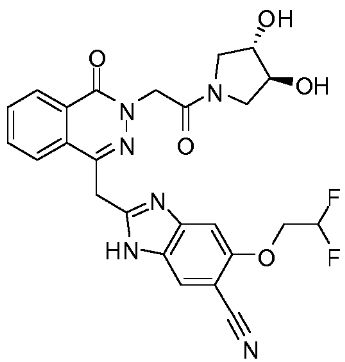 | 6-(2,2-difluoroethoxy)-2-[[3-[2-[(3S,4S)-3,4-dihydroxypyrroli din-1-yl]-2-oxo-ethyl]-4-oxo-phthalazin-1-yl]methyl]-3H-benzimidazole-5-carbonitrile | Final compoun d 139 | AU | 524 | 525 |

(continued)

| Cpd # | Structure | Name | Int | Mtd | MW (calc) | MW (obs) |
|---|---|---|---|---|---|---|
| 212 | 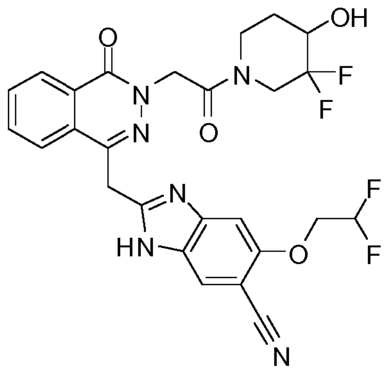 | 6-(2,2-difluoroethoxy)-2-[[3-[2-(3,3-difluoro-4-hydroxy-1-piperidyl)-2-oxo-ethyl]-4-oxo-phthalazin-1-yl]methyl]-3H-benzimidazole-5-carbonitrile | Final compound 139 | AU | 558 | 559 |
| 213 | 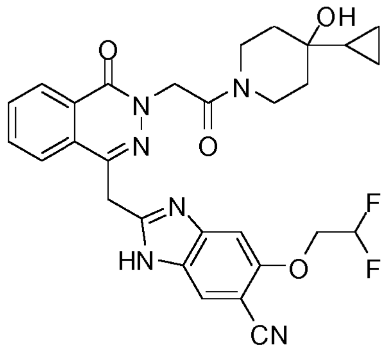 | 2-[[3-[2-(4-cyclopropyl-4-hydroxy-1-piperidyl)-2-oxo-ethyl] -4-oxo-phthalazin-1-yl]methyl]-6-(2,2-difluoroethoxy)-3H-benzimidazole-5-carbonitrile | Final compound 139 | AU | 563 | 563 |
| 214 | 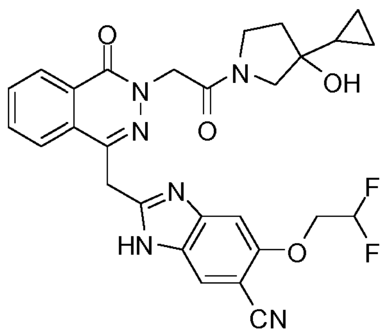 | 2-[[3-[2-(3-cyclopropyl-3-hydroxy-pyrrolidin-1-yl)-2-oxo-ethyl] -4-oxo-phthalazin-1-yl]methyl]-6-(2,2-difluoroethoxy)-3H-benzimidazole-5-carbonitrile | Final compound 139 | AU | 549 | 549 |

(continued)

| Cpd # | Structure | Name | Int | Mtd | MW (calc) | MW (obs) |
|---|---|---|---|---|---|---|
| 215 | 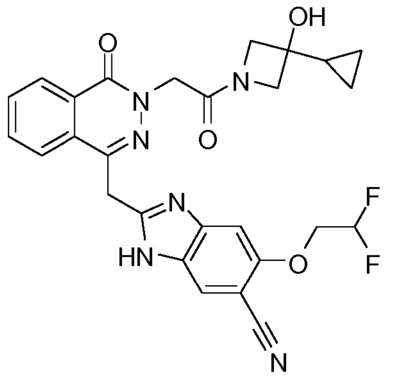 | 2-[[3-[2-(3-cyclopropyl-3-hydroxy-azetidin-1-yl)-2-oxo-ethyl] -4-oxo-phthalazin-1-yl]methyl]-6-(2,2-difluoroethoxy)-3H-benzimidazole-5-carbonitrile | Final compoun d 139 | AU | 535 | 535 |
| 216 | 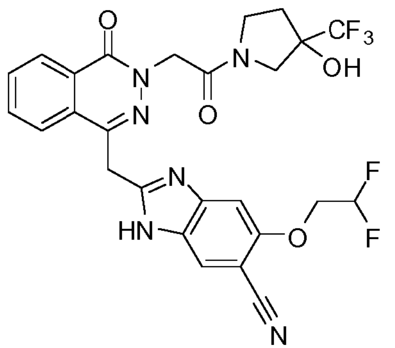 | 6-(2,2-difluoroethoxy)-2-[[3-[2-[3-hydroxy-3-(trifluoromethyl) pyrrolidin-1-yl]-2-oxo-ethyl]-4-oxo-phthalazin-1-yl]methyl]-3H-benzimidazole-5-carbonitrile | Final compoun d 139 | AU | 576 | 577 |
| 217 | 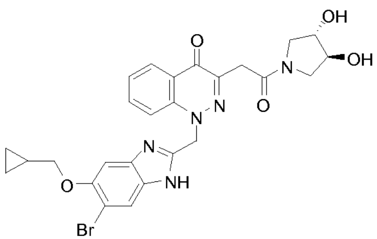 | 1-[[6-bromo-5-(cyclopropylmeth oxy)-1H-benzimidazol-2-yl]methyl]-3-[2-[(3S,4S)-3,4-dihydroxypyrroli din-1-yl]-2-oxo-ethyl] cinnolin-4-one | Final compoun d 109 | AU | 568 | 568 |

(continued)

| Cpd # | Structure | Name | Int | Mtd | MW (calc) | MW (obs) |
|---|---|---|---|---|---|---|
| 218 | 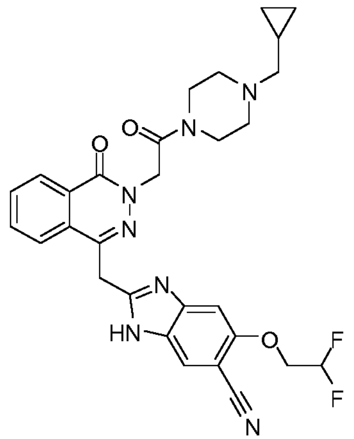 | 2-[[3-[2-[4-(cyclopropylmeth yl)piperazin-1-yl]-2-oxo-ethyl]-4-oxo-phthalazin-1-yl]methyl]-6-(2,2-difluoroethoxy)-3H-benzimidazole-5-carbonitrile | Final compoun d 139 | AV | 562 | 562 |
| 219 | 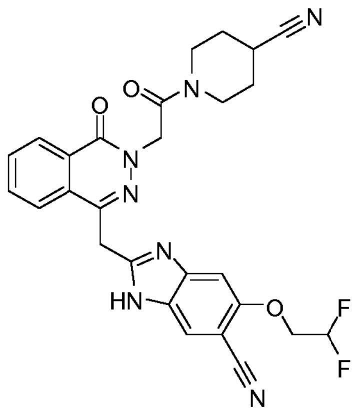 | 2-[[3-[2-(4-cyano-1-piperidyl)-2-oxo-ethyl]-4-oxo-phthalazin-1-yl] methyl]-6-(2,2-difluoroethoxy)-3H-benzimidazole-5-carbonitrile | Final compoun d 139 | AV | 532 | 532 |

(continued)

| Cpd # | Structure | Name | Int | Mtd | MW (calc) | MW (obs) |
|---|---|---|---|---|---|---|
| 220 | 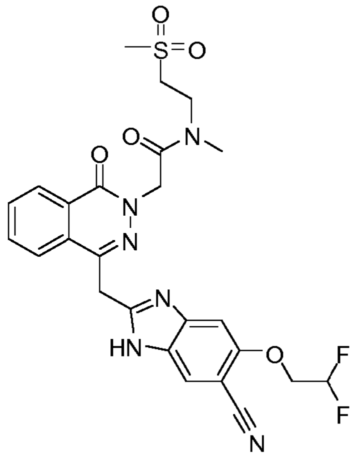 | 2-[4-[[6-cyano-5-(2,2-difluoroethoxy)-1H-benzimidazol-2-yl]methyl]-1-oxo-phthalazin-2-yl]-N-methyl-N-(2-methylsulfonylet hyl)acetamide | Final compoun d 139 | AV | 559 | 559 |
| 221 | 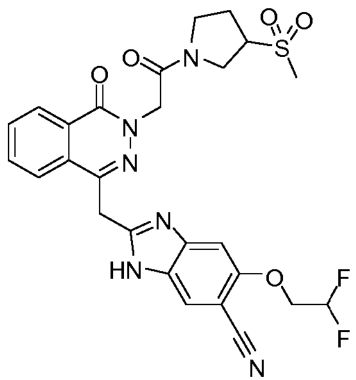 | 6-(2,2-difluoroethoxy)-2-[[3-[2-(3-methylsulfonylpy rrolidin-1-yl)-2-oxo-ethyl]-4-oxo-phthalazin-1-yl]methyl]-3H-benzimidazole-5-carbonitrile | Final compoun d 139 | AV | 571 | 571 |

(continued)

| Cpd # | Structure | Name | Int | Mtd | MW (calc) | MW (obs) |
|---|---|---|---|---|---|---|
| 222 | 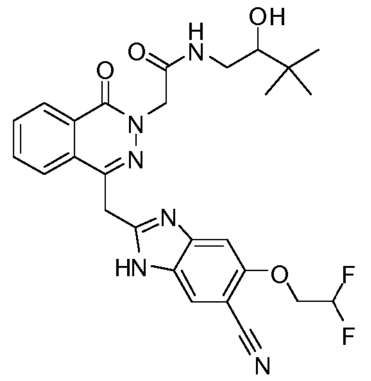 | 2-[4-[[6-cyano-5-(2,2-difluoroethoxy)-1H-benzimidazol-2-yl]methyl]-1-oxo-phthalazin-2-yl]-N-(2-hydroxy-3,3-dimethylbutyl) acetamide | Final compoun d 139 | AV | 539 | 539 |
| 223 | 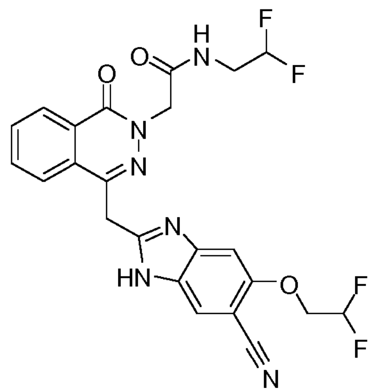 | 2-[4-[[6-cyano-5-(2,2-difluoroethoxy)-1H-benzimidazol-2-yl]methyl]-1-oxo-phthalazin-2-yl]-N-(2,2-difluoroethyl)acet amide | Final compoun d 139 | AV | 502 | 503 |

(continued)

| Cpd # | Structure | Name | Int | Mtd | MW (calc) | MW (obs) |
|---|---|---|---|---|---|---|
| 224 | 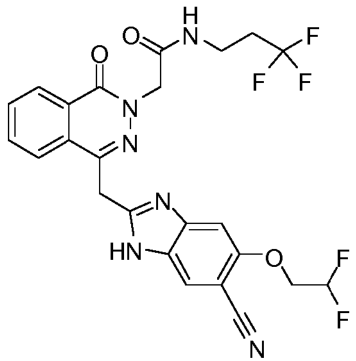 | 2-[4-[[6-cyano-5-(2,2-difluoroethoxy)-1H-benzimidazol-2-yl]methyl]-1-oxo-phthalazin-2-yl]-N-(3,3,3-trifluoropropyl)ac etamide | Final compoun d 139 | AV | 534 | 535 |
| 225 | 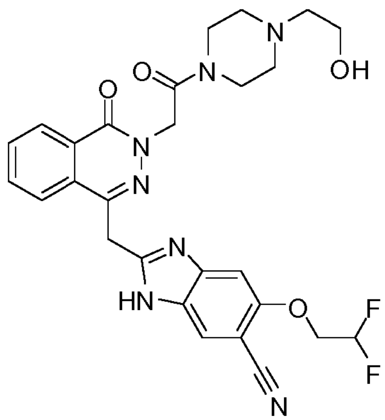 | 6-(2,2-difluoroethoxy)-2-[[3-[2-[4-(2-hydroxyethyl)pip erazin-1-yl]-2-oxo-ethyl]-4-oxo-phthalazin-1-yl]methyl]-3H-benzimidazole-5-carbonitrile | Final compoun d 139 | AV | 552 | 552 |

(continued)

| Cpd # | Structure | Name | Int | Mtd | MW (calc) | MW (obs) |
|---|---|---|---|---|---|---|
| 226 | 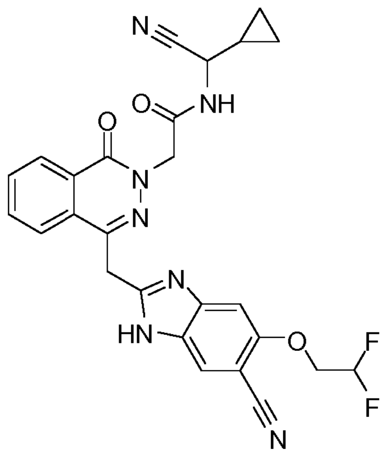 | N-[cyano(cycloprop yl)methyl]-2-[4-[[6-cyano-5-(2,2-difluoroethoxy)-1H-benzimidazol-2-yl]methyl]-1-oxo-phthalazin-2-yl]acetamide | Final compoun d 139 | AV | 517 | 518 |
| 227 | 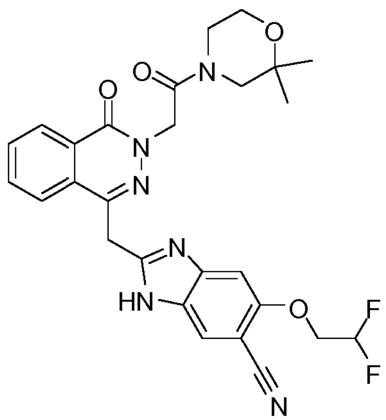 | 6-(2,2-difluoroethoxy)-2-[[3-[2-(2,2-dimethylmorphol in-4-yl)-2-oxo-ethyl]-4-oxo-phthalazin-1-yl]methyl]-3H-benzimidazole-5-carbonitrile | Final compoun d 139 | AV | 537 | 537 |

(continued)

| Cpd # | Structure | Name | Int | Mtd | MW (calc) | MW (obs) |
|---|---|---|---|---|---|---|
| 228 | 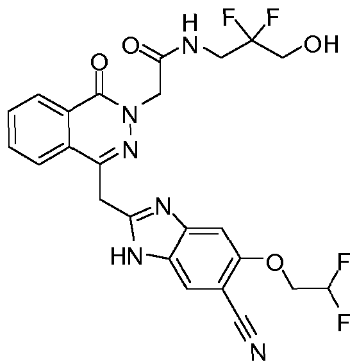 | 2-[4-[[6-cyano-5-(2,2-difluoroethoxy)-1H-benzimidazol-2-yl]methyl]-1-oxo-phthalazin-2-yl]-N-(2,2-difluoro-3-hydroxypropyl)acetamide | Final compoun d 139 | AV | 532 | 533 |
| 229 | 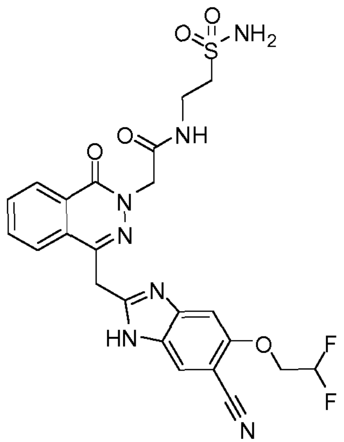 | 2-[4-[[6-cyano-5-(2,2-difluoroethoxy)-1H-benzimidazol-2-yl]methyl]-1-oxo-phthalazin-2-yl]-N-(2-sulfamoylethyl)a cetamide | Final compoun d 139 | AV | 546 | 546 |

(continued)

| Cpd # | Structure | Name | Int | Mtd | MW (calc) | MW (obs) |
|---|---|---|---|---|---|---|
| 230 | 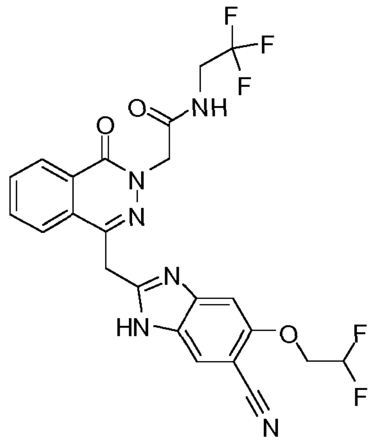 | 2-[4-[[6-cyano-5-(2,2-difluoroethoxy)-1H-benzimidazol-2-yl]methyl]-1-oxo-phthalazin-2-yl]-N-(2,2,2-trifluoroethyl)ace tamide | Final compoun d 139 | AV | 520 | 521 |
| 231 | 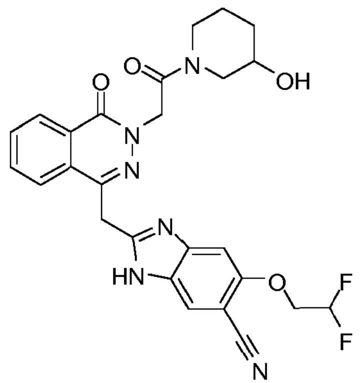 | 6-(2,2-difluoroethoxy)-2-[[3-[2-(3-hydroxy-1-piperidyl)-2-oxo-ethyl]-4-oxo-phthalazin-1-yl]methyl]-3H-benzimidazole-5-carbonitrile | Final compoun d 139 | AV | 523 | 523 |

(continued)

| Cpd # | Structure | Name | Int | Mtd | MW (calc) | MW (obs) |
|---|---|---|---|---|---|---|
| 232 | 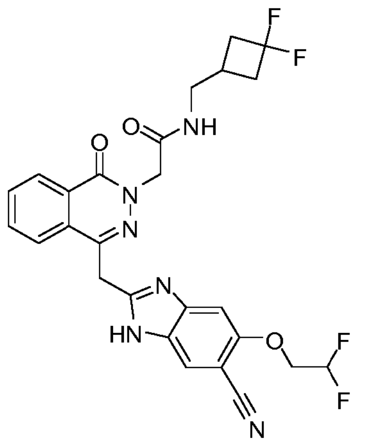 | 2-[4-[[6-cyano-5-(2,2-difluoroethoxy)-1H-benzimidazol-2-yl]methyl]-1-oxo-phthalazin-2-yl]-N-[(3,3-difluorocyclobuty 1)methyl]acetami de | Final compoun d 139 | AV | 542 | 543 |
| 233 | 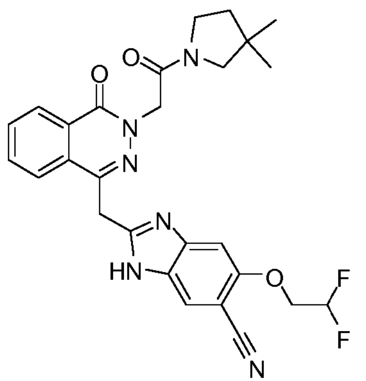 | 6-(2,2-difluoroethoxy)-2-[[3-[2-(3,3-dimethylpyrrolidi n-1-yl)-2-oxo-ethyl]-4-oxo-phthalazin-1-yl]methyl]-3H-benzimidazole-5-carbonitrile | Final compoun d 139 | AV | 521 | 521 |

(continued)

| Cpd # | Structure | Name | Int | Mtd | MW (calc) | MW (obs) |
|---|---|---|---|---|---|---|
| 234 | 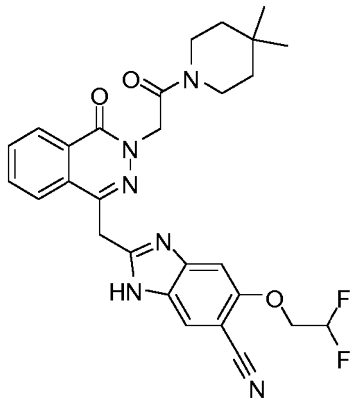 | 6-(2,2-difluoroethoxy)-2-[[3-[2-(4,4-dimethyl-1-piperidyl)-2-oxo-ethyl]-4-oxo-phthalazin-1-yl]methyl]-3H-benzimidazole-5-carbonitrile | Final compound 139 | AV | 535 | 535 |
| 235 | 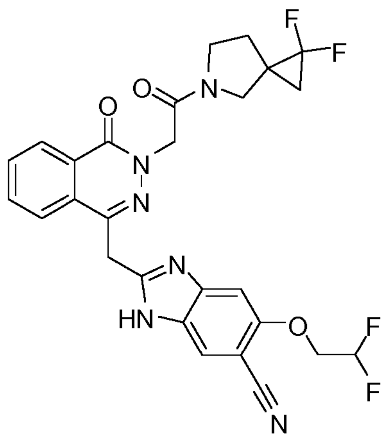 | 2-[[3-[2-(2,2-difluoro-5-azaspiro[2.4]hept an-5-yl)-2-oxo-ethyl]-4-oxo-phthalazin-1-yl]methyl]-6-(2,2-difluoroethoxy)-3H-benzimidazole-5-carbonitrile | Final compound 139 | AV | 555 | 555 |

(continued)

| Cpd # | Structure | Name | Int | Mtd | MW (calc) | MW (obs) |
|---|---|---|---|---|---|---|
| 236 | 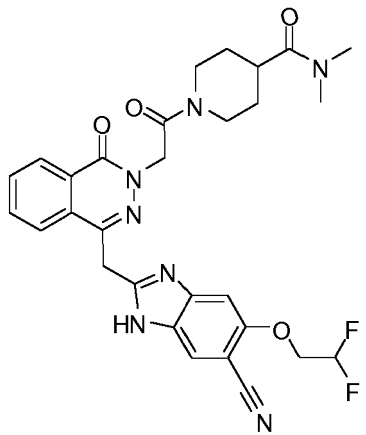 | 1-[2-[4-[[6-cyano-5-(2,2-difluoroethoxy)-1H-benzimidazol-2-yl]methyl]-1-oxo-phthalazin-2-yl]acetyl]-N,N-dimethyl-piperidine-4-carboxamide | Final compound 139 | AV | 578 | 578 |
| 237 | 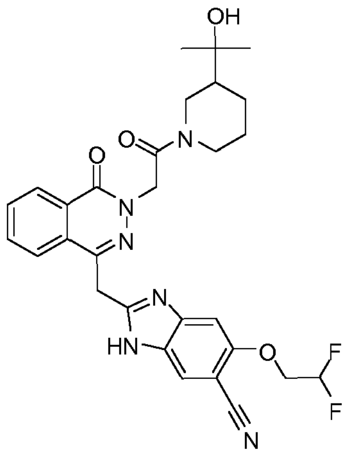 | 6-(2,2-difluoroethoxy)-2-[[3-[2-[3-(1-hydroxy-1-methyl-ethyl)-1-piperidyl]-2-oxo-ethyl]-4-oxo-phthalazin-1-yl]methyl]-3H-benzimidazole-5-carbonitrile | Final compound 139 | AV | 565 | 565 |

(continued)

| Cpd # | Structure | Name | Int | Mtd | MW (calc) | MW (obs) |
|---|---|---|---|---|---|---|
| 238 | 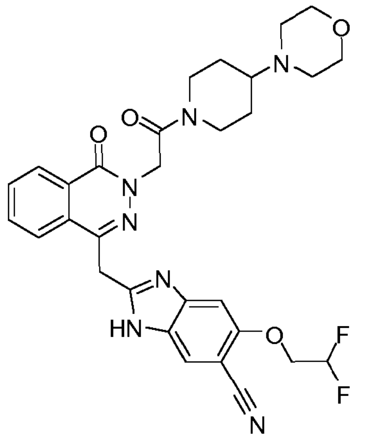 | 6-(2,2-difluoroethoxy)-2-[[3-[2-(4-morpholino-1-piperidyl)-2-oxo-ethyl]-4-oxo-phthalazin-1-yl]methyl]-3H-benzimidazole-5-carbonitrile | Final compoun d 139 | AV | 592 | 593 |
| 239 | 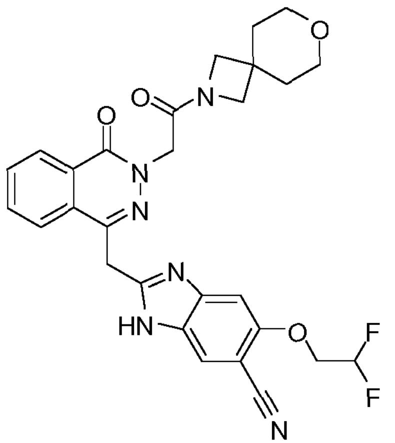 | 6-(2,2-difluoroethoxy)-2-[[3-[2-(7-oxa-2-azaspiro[3.5]non an-2-yl)-2-oxo-ethyl]-4-oxo-phthalazin-1-yl]methyl]-3H-benzimidazole-5-carbonitrile | Final compoun d 139 | AV | 549 | 549 |

(continued)

| Cpd # | Structure | Name | Int | Mtd | MW (calc) | MW (obs) |
|---|---|---|---|---|---|---|
| 240 | 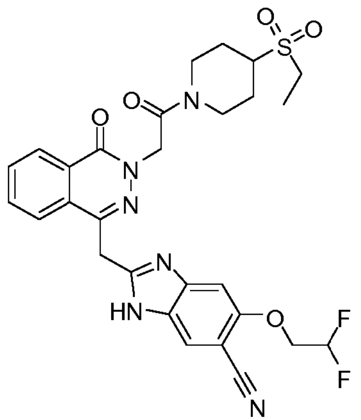 | 6-(2,2-difluoroethoxy)-2-[[3-[2-(4-ethylsulfonyl-1-piperidyl)-2-oxo-ethyl]-4-oxo-phthalazin-1-yl]methyl]-3H-benzimidazole-5-carbonitrile | Final compound 139 | AV | 599 | 599 |
| 241 | 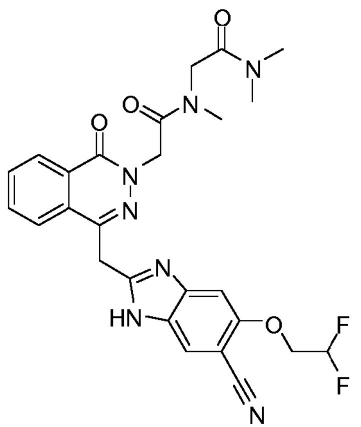 | 2-[4-[[6-cyano-5-(2,2-difluoroethoxy)-1H-benzimidazol-2-yl]methyl]-1-oxo-phthalazin-2-yl]-N-[2-(dimethyl amino)-2-oxo-ethyl]-N-methyl-acetamide | Final compound 139 | AV | 538 | 538 |

(continued)

| Cpd # | Structure | Name | Int | Mtd | MW (calc) | MW (obs) |
|---|---|---|---|---|---|---|
| 242 | 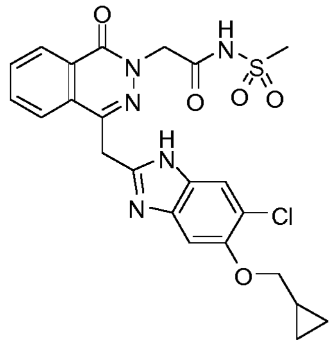 | 2-[4-[[6-chloro-5-(cyclopropylmeth oxy)-1H-benzimidazol-2-yl]methyl]-1-oxo-phthalazin-2-yl]-N-methylsulfonyl-acetamide | Final compound 115 | AW | 516 | 516 |
| 243 | 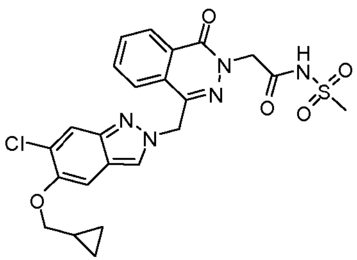 | 2-[4-[[6-chloro-5-(cyclopropylmeth oxy)indazol-2-yl]methyl]-1-oxo-phthalazin-2-yl]-N-methylsulfonyl-acetamide | Final compound 114 | AW | 516 | 516 |
| 244 | 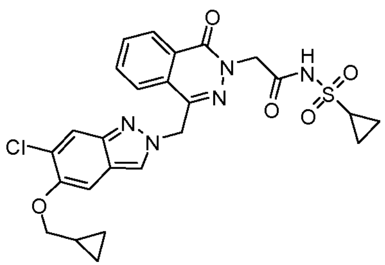 | 2-[4-[[6-chloro-5-(cyclopropylmeth oxy)indazol-2-yl]methyl]-1-oxo-phthalazin-2-yl]-N-cyclopropylsulfo nyl-acetamide | Final compound 114 | AW | 542 | 542 |

(continued)

| Cpd # | Structure | Name | Int | Mtd | MW (calc) | MW (obs) |
|---|---|---|---|---|---|---|
| 245 | 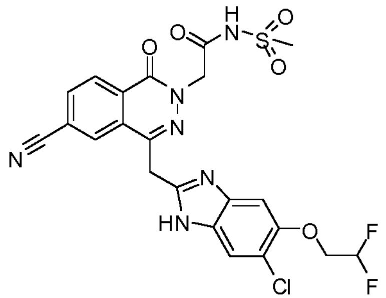 | 2-[4-[[6-chloro-5-(2,2-difluoroethoxy)-1H-benzimidazol-2-yl]methyl]-6-cyano-1-oxo-phthalazin-2-yl]-N-methylsulfonyl-acetamide | Final compoun d 23 | AW | 551 | 551 |
| 246 | 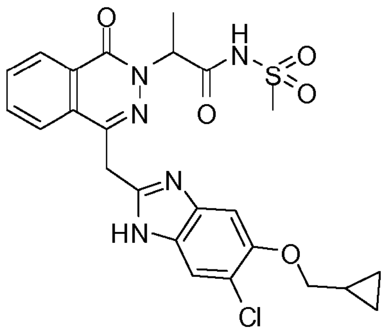 | N-{2-[4-(6-Chloro-5-cyclopropylmeth oxy-1H-benzoimidazol-2-ylmethyl)-1-oxo-1H-phthalazin-2-yl]-propionyl} - methanesulfonam ide | Final compoun d 22 | AW | 530 | 530 |
| 247 | 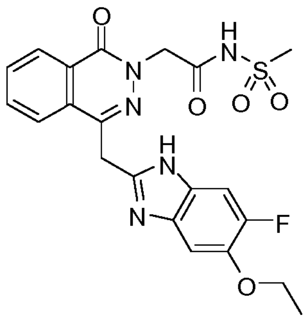 | 2-[4-[(5-ethoxy-6-fluoro-1H-benzimidazol-2-yl)methyl]-1-oxo-phthalazin-2-yl]-N-methylsulfonyl-acetamide | Final compoun d 129 | AW | 473 | 474 |

(continued)

| Cpd # | Structure | Name | Int | Mtd | MW (calc) | MW (obs) |
|---|---|---|---|---|---|---|
| 248 | 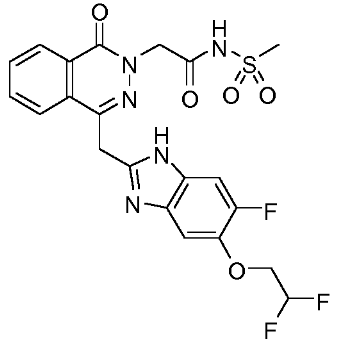 | 2-[4-[[5-(2,2-difluoroethoxy)-6-fluoro-1H-benzimidazol-2-yl]methyl]-1-oxo-phthalazin-2-yl]-N-methylsulfonyl-acetamide | Final compound 130 | AW | 509 | 510 |
| 249 | 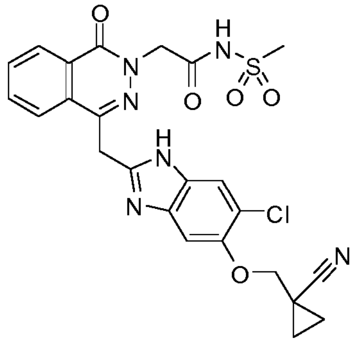 | 2-[4-[[6-chloro-5-[(1-cyanocyclopropyl )methoxy]-1H-benzimidazol-2-yl]methyl]-1-oxo-phthalazin-2-yl]-N-methylsulfonyl-acetamide | Final compound 133 | AW | 541 | 541 |
| 250 | 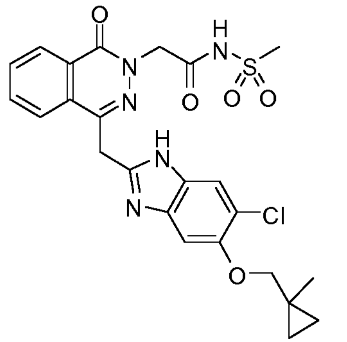 | 2-[4-[[6-chloro-5-[(1-methylcycloprop yl)methoxy]-1H-benzimidazol-2-yl]methyl]-1-oxo-phthalazin-2-yl]-N-methylsulfonyl-acetamide | Final compound 131 | AW | 530 | 530 |

(continued)

| Cpd # | Structure | Name | Int | Mtd | MW (calc) | MW (obs) |
|---|---|---|---|---|---|---|
| 251 | 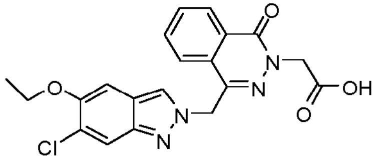 | 2-[4-[(6-chloro-5-ethoxy-indazol-2-yl)methyl]-1-oxo-phthalazin-2-yl] acetic acid | Int.266 | D & AS | 413 | 413 |
| 252 | 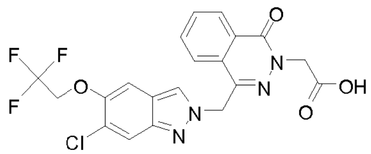 | 2-[4-[[6-chloro-5-(2,2,2-trifluoroethoxy)in dazol-2-yl]methyl]-1-oxo-phthalazin-2-yl]acetic acid | Int.264 | D & AS | 467 | 467 |
| 253 | 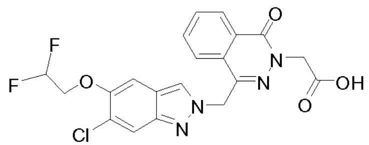 | 2-[4-[[6-chloro-5-(2,2-difluoroethoxy)in dazol-2-yl]methyl]-1-oxo-phthalazin-2-yl]acetic acid | Int.265 | D & AS | 449 | 449 |
| 254 | 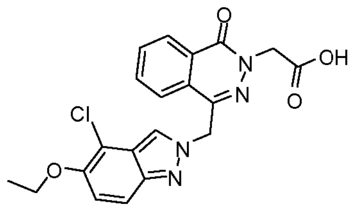 | 2-[4-[(4-chloro-5-ethoxy-indazol-2-yl)methyl]-1-oxo-phthalazin-2-yl] acetic acid | Int.260 | D & AS | 413 | 413 |
| 255 | 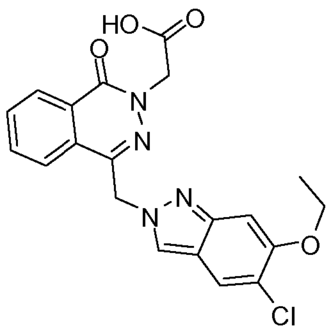 | 2-[4-[(5-chloro-6-ethoxy-indazol-2-yl)methyl]-1-oxo-phthalazin-2-yl] acetic acid | Int.259 | D & AS | 413 | 413 |

(continued)

| Cpd # | Structure | Name | Int | Mtd | MW (calc) | MW (obs) |
|---|---|---|---|---|---|---|
| 256 | 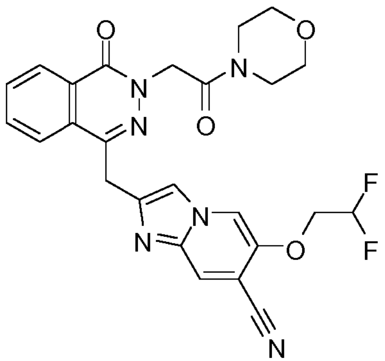 | 6-(2,2-difluoroethoxy)-2-[[3-(2-morpholino-2-oxo-ethyl)-4-oxo-phthalazin-1-yl]methyl]imidaz o[1,2-a]pyridine-7-carbonitrile | Int.287 | J | 508 | 509 |
| 257 | 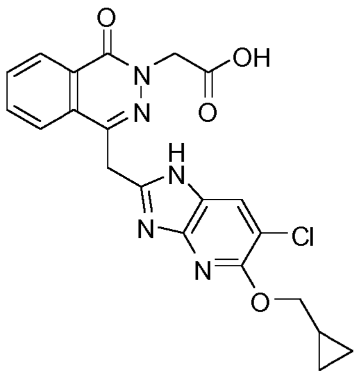 | 2-[4-[[6-chloro-5-(cyclopropylmeth oxy)-1H-imidazo[4,5-b]pyridin-2-yl] methyl]-1-oxo-phthalazin-2-yl]acetic acid | Int.22 | AT | 440 | 440 |

**Table IV. NMR data of illustrative compounds of the invention**

| Cpd# | NMR |
|---|---|
| 1 | $^1$H NMR (400MHz, DMSO-$d_6$, ppm) δ 8.4-8.3 (1H, m), 8.0-7.8 (2H, m), 6.6-6.3 (1H, m), 4.6 (1H, dd), 4.5 (2H, dq), 4.3-4.1 (2H, m), 3.7-3.6 (6H, m), 3.1-2.8 (2H, m), 1.9-1.5 (3H, m) |
| 2 | $^1$H NMR (600 MHz, DMSO-$d_6$, ppm) δ 8.43 (s, 1H), 8.26 (d, 1H), 8.00 (d, 1H), 7.88 (t, 1H), 7.83 (t, 1H), 7.72 (s, 1H), 7.16 (s, 1H), 5.99 (s, 2H), 5.61 (quint, 1H), 3.91 (t, 2H), 3.85 (d, 2H), 3.66 (t, 2H), 1.21 - 1.30 (m, 1H), 0.55 - 0.59 (m, 2H), 0.32 - 0.36 (m, 2H). |
| 3 | $^1$H NMR (400MHz, DMSO-$d_6$, ppm) δ 12.9 (1H, brs), 8.3 (1H, dt), 8.3-8.2 (1H, m), 8.0 (1H, dd), 7.9-7.8 (2H, m), 7.7 (1H, d), 7.2 (1H, d), 6.0-5.9 (2H, m), 5.5 (1H, td), 3.9-3.8 (2H, d), 1.6-1.5 (3H, d), 1.3-1.2 (1H, m), 0.6-0.5 (2H, m), 0.4-0.3 (2H, m) |
| 4 | $^1$H NMR (500 MHz, DMSO-$d_6$) δ/ppm: 12.40 (b.s., 1H), 8.35 (s, 1H), 8.11 (m, 1H), 7.90 (m, 1H), 7.78 (m, 1H), 7.73 (s, 1H), 7.72 (s, 1H), 7.44 (m, 1H), 5.76 (q, 2H), 3.97 (q, 1H), 3.82(d, 2H), 1.42 (d, 3H), 1.27 (m, 1H), 0.59 (m, 2H), 0.33 (m, 2H). |
| 5 | $^1$H NMR (400MHz, DMSO-$d_6$ ppm) δ 14.2 (1H, brs), 12.6 (1H, brs), 8.3 (1H, dd), 8.0 (2H, m), 7.7 (1H, td), 7.6 (1H, brs), 7.2-7.1 (1H, m), 6.1 (2H, s), 3.9 (2H, d), 1.3-1.2 (1H, m), 0.6 (2H, m), 0.3 (2H, m) |
| 6 | $^1$H NMR d (ppm)(DMSO-$d_6$): 8.27 (1 H, dd), 7.95 (1 H, dd), 7.87-7.78 (2 H, m), 7.49 (1 H, d), 7.11 (1 H, broad s), 4.55 (4 H, d), 4.05 (2 H, q), 1.34 (3 H, t). |
| 7 | $^1$H NMR d (ppm)(DMSO-$d_6$): 13.20-12.60 (2 H, m); 8.30 ((1 H, dd), 7.98 (1 H, dd), 7.94-7.85 (3 H, m), 7.32-7.28 (1 H, m), 4.92 (2 H, s), 4.59 (2 H, s), 3.96 (2 H, d), 1.30-1.21 (1H, m), 0.64-0.55 (2 H, m), 0.43-0.36 (2 H, m). |
| 8 | $^1$H NMR (400MHz, DMSO-$d_6$) δ/ppm: 13.3 (br s, 1H), 8.59 (s, 1H), 8.32 (s, 1H), 8.29 - 8.25 (m, 1H), 8.01 - 7.97 (m, 1H), 7.90 - 7.80 (m, 2H), 7.41 (s, 1H), 6.43 (tt, 1H), 6.04 (s, 2H), 4.56 (s, 2H), 4.46 - 4.36 (m, 2H). |
| 9 | $^1$H NMR (500 MHz, DMSO-$d_6$) δ/ppm: $^1$H NMR (500 MHz, DMSO-$d_6$) δ/ppm: 13.11 (bs, 1H), 11.11 (s, 1H), 8.30 (d, 1H), 7.99 (d, 1H), 7.89 (t, 1H), 7.85 (t, 1H), 7.44 (s, 1H), 7.07 (s, 1H), 6.40 (t, 1H), 6.04 (s, 1H), 4.86 (s, 2H), 4.43 (s, 2H), 4.33 (td, 2H). |
| 10 | $^1$H NMR (500 MHz, DMSO-$d_6$) δ/ppm: 12.70 - 13.00 (m, 1H), 8.12 (m, 1H), 7.83 - 8.03 (m, 1H), 7.74 - 7.83 (m, 2H), 7.46 (m, 1H), 7.05 - 7.39 (m, 1H), 5.93 (s, 2H), 5.18 (m, 2H), 3.89 - 4.04 (m, 4H), 3.74 (m, 1H), 3.69(m, 2H), 3.46 (m, 1H), 3.37 (m, 1H), 3.27 (m, 1H), 1.26 (m, 1H), 0.59 (m, 2H), 0.36 (m, 2H). |
| 11 | $^1$H NMR (500 MHz, DMSO-$d_6$) δ/ppm: 11.12 (s, 1H), 8.26-8.31 (m, 1H), 7.95 -7.99 (m, 1H), 7.86-7.91 (m, 1H), 7.82 - 7.86 (m, 1H), 7.44 (s, 1H), 7.07 (s, 1H), 6.41 (tt, 1H), 6.01 (s, 1H), 5.08 (s, 2H), 4.42 (s, 2H), 4.33 (td, 2H), 3.63 - 3.69 (m, 2H), 3.55 - 3.62 (m, 4H), 3.43 -3.49 (m, 2H). |
| 12 | $^1$H NMR (400MHz, DMSO-$d_6$) δ/ppm: 12.48 (d, 1H), 8.48 - 8.44 (m, 1H), 8.37 - 8.37 (m, 1H), 8.31 (s, 1H), 8.27 (dd, 1H), 7.72 (s, 1H), 7.59-7.52 (m, 1H), 7.37-7.24 (d, 1H), 6.55 - 6.22 (m, 1H), 5.05 (s, 2H), 4.61 (s, 2H), 4.45 - 4.27 (m, 2H), 3.67 - 3.61 (m, 2H), 3.62 - 3.53 (m, 4H), 3.46 - 3.41 (m, 2H). |
| 15 | $^1$H NMR (500 MHz, DMSO-$d_6$) δ/ppm: 11.84 (b.s., 1H), 8.33 (s, 1H), 8.13 (m, 1H), 7.95 (m, 1H), 7.81 (m, 1H), 7.78 (s, 1H), 7.71 (s, 1H), 7.46 (m, 1H), 5.77 (m, 2H), 4.11 (q, 1H), 3.82(d, 2H), 3.23 (s, 3H), 1.40 (d, 3H), 1.27 (m, 1H), 0.59 (m, 2H), 0.34 (m, 2H). |
| 16 | $^1$H NMR (600 MHz, DMSO-$d_6$) δ/ppm: 12.49 (b.s., 1H), 11.08 (s, 1H), 8.11 (m, 1H), 7.88 (m, 1H), 7.79 (m, 1H), 7.45 (s, 1H), 7.44 (m, 1H), 6.95 (s, 1H), 6.21 (m, 1H), 5.79 (s, 2H), 3.82(d, 2H), 3.66 (s, 2H), 1.22 (m, 1H), 0.55 (m, 2H), 0.33 (m, 2H). |
| 17 | $^1$H NMR (400MHz, DMSO-$d_6$) δ/ppm: 8.34 (s, 1H), 8.29 (dd, 1H), 8.01 (d, 1H), 7.92-7.88 (m, 1H), 7.88-7.83 (m, 1H), 7.73 (s, 1H), 7.16 (s, 1H), 6.03-5.90 (m, 2H), 5.47 (q, 1H), 3.85 (d, 2H), 1.57 (d, 3H), 1.31-1.21 (m, 1H), 0.60-0.55 (m, 2H), 0.36-0.32 (m, 2H). |
| 18 | $^1$H NMR (400MHz, DMSO-$d_6$) δ/ppm: 12.99 (br s, 1H), 8.34 (d, 1H), 8.29 (dd, 1H), 8.03-8.00 (m, 1H), 7.92-7.89 (m, 1H), 7.88-7.84 (m, 1H), 7.73 (d, 1H), 7.17 (s, 1H), 6.04-5.90 (m, 2H), 5.47 (q, 1H), 3.85 (d, 2H), 1.57 (d, 3H), 1.31-1.21 (m, 1H), 0.60-0.55 (m, 2H), 0.37-0.33 (m, 2H). |

(continued)

| Cpd# | NMR |
|---|---|
| 19 | $^1H$ NMR (400 MHz, DMSO-$d_6$) δ/ppm: 8.35 (s, 1H), 8.11 (d, 1H), 7.90 (d, 1H), 7.81-7.76 (m, 1H), 7.73 (s, 1H), 7.45-7.40 (m, 1H), 5.84-5.70 (m, 1H), 3.97 (q, 1H), 3.81 (d, 2H), 1.42 (d, 3H), 1.30-1.22 (m, 1H), 0.61-0.56 (m, 2H), 0.35-0.31 (m, 2H). |
| 20 | $^1H$ NMR (400 MHz, DMSO-$d_6$) δ/ppm: 8.35 (s, 1H), 8.11 (d, 1H), 7.90 (d, 1H), 7.81-7.75 (m, 1H), 7.73 (s, 1H), 7.46-7.41 (m, 1H), 5.82-5.70 (m, 1H), 3.97 (q, 1H), 3.81 (d, 2H), 1.42 (d, 3H), 1.30-1.21 (m, 1H), 0.61-0.56 (m, 2H), 0.35-0.31 (m, 2H). |
| 21 | $^1H$ NMR (400MHz, DMSO-$d_6$ ppm) δ 8.1 (1H, d), 7.9 (1H, d), 7.8-7.7 (2H, m), 7.5-7.4 (1H, m), 7.2 (1H, m), 6.0-5.9 (2H, m), 4.2-4.1 (1H, m), 4.0 (2H, dt), 1.3-1.2 (1H, m), 0.6-0.5 (2H, m), 0.4-0.3 (2H, m) |
| 22 | $^1H$ NMR (400MHz, DMSO-$d_6$) δ/ppm: 13.3 (br s, 1H), 8.49 (s, 1H), 8.29 - 8.26 (m, 1H), 8.05 - 8.02 (m, 1H), 7.92 - 7.82 (m, 2H), 7.61 (d, 1H), 7.02 (d, 1H), 5.97 (s, 2H), 4.67 (s, 2H), 4.14 (q, 2H), 1.32 (t, 3H). |
| 23 | $^1H$ NMR (400MHz, DMSO-$d_6$) δ/ppm: 12.5 (br s, 1H), 8.67 (s, 1H), 8.43 (d, 1H), 8.27 (dd, 1H), 7.58 (s, 1H), 7.32 (br s, 1H), 6.43 (tt, 1H), 4.83 (s, 2H), 4.63 (s, 2H), 4.43 - 4.32 (m, 2H). |
| 24 | $^1H$ NMR (300 MHz, DMSO-$d_6$) d 12.72 (br. s., 1H), 8.26-8.34 (m, 1H), 7.90-7.99 (m, 1H), 7.86 (dt, J=1.57, 7.05 Hz, 2H), 7.78 (br. s., 1H), 7.68 (br. s., 1H), 5.30-5.44 (m, 1H), 4.62 (s, 2H), 1.83-1.99 (m, 2H), 1.64-1.82 (m, 2H), 1.40-1.60 (m, 4H) |
| 25 | $^1H$ NMR (300 MHz, DMSO-$d_6$) d 12.73 (br. s., 1H), 8.30 (dd, J=2.87, 6.01 Hz, 1H), 7.88-7.96 (m, 1H), 7.80-7.88 (m, 2H), 7.76 (s, 1H), 7.67 (s, 1H), 4.58 (s, 2H), 3.94-4.05 (m, 1H), 0.84-1.03 (m, 4H) |
| 26 | $^1H$ NMR (300 MHz, DMSO-$d_6$) d 12.78 (br. s., 1H), 8.28 (d, J=6.97 Hz, 1H), 7.91 (br. s., 1H), 7.81-7.88 (m, 2H), 7.77 (br. s., 1H), 7.68 (br. s., 1H), 5.36-5.51 (m, 1H), 4.66 (s, 2H), 2.35-2.47 (m, 2H), 2.11-2.30 (m, 2H), 1.60-1.84 (m, 2H) |
| 30 | $^1H$ NMR (400MHz, DMSO-$d_6$) δ/ppm: 8.50 (s, 1H), 8.22 - 8.27 (m, 1H), 7.57 (br s, 1H), 7.45 - 7.41 (m, 2H), 6.73 (br s, 1H), 6.40 (tt, 1H), 5.00 (s, 2H), 4.52 (s, 2H), 4.43 - 4.31 (m, 2H), 3.86 (s, 3H), 3.65 - 3.61 (m, 2H), 3.59 - 3.52 (m, 4H), 3.45 - 3.41 (m, 2H). |
| 31 | $^1H$ NMR (400MHz, DMSO-$d_6$) δ/ppm: 8.22 - 8.18 (m, 1H), 7.98 (d, 1H), 7.65 (d, 1H), 7.57 (br s, 1H), 7.49 - 7.41 (m, 2H), 6.40 (tt, 1H), 5.02 (s, 2H), 4.52 (s, 2H), 4.43 - 4.31 (m, 2H), 3.86 (s, 3H), 3.66 - 3.61 (m, 2H), 3.59 - 3.52 (m, 4H), 3.45 - 3.41 (m, 2H). |
| 32 | $^1H$ NMR (400MHz, DMSO-$d_6$) δ/ppm: 8.33 - 8.19 (m, 1H), 7.95 - 7.91 (m, 1H), 7.89 - 7.81 (m, 2H), 7.56 (s, 1H), 7.29 (br s, 1H), 6.40 (tt, 1H), 4.53 (s, 2H), 4.43 - 4.31 (m, 2H), 4.05 - 3.97 (m, 1H), 1.03 - 0.98 (m, 2H), 0.97 - 0.89 (m, 2H) |
| 33 | $^1H$ NMR (400MHz, DMSO-$d_6$) δ/ppm: 8.08 - 8.04 (m, 1H), 7.84 - 7.80 (m, 1H), 7.58 - 7.50 (m, 2H), 7.47 - 7.43 (m, 1H), 7.29 - 7.26 (m, 1H), 6.40 (tt, 1H), 4.98 (d, 2H), 4.47 (d, 2H), 4.43 - 4.31 (m, 2H), 3.79 - 3.70 (m, 4H), 3.66 - 3.52 (m, 6H), 3.48 - 3.38 (m, 6H), 3.45 - 3.41 (m, 2H). |
| 40 | $^1H$ NMR (400MHz, CDCl3) δ/ppm: 8.75 - 8.71 (m, 1H), 8.60 - 8.49 (m, 1H), 8.14 - 8.00 (m, 2H), 7.74 - 7.70 (m, 1H), 7.42 - 7.32 (m, 1H), 6.35 - 6.03 (m, 1H), 5.24 - 5.13 (m, 4H), 4.35 - 4.25 (m, 2H), 3.87 - 3.67 (m, 8H). |
| 41 | $^1H$ NMR (400MHz, DMSO-$d_6$) δ/ppm: 12.6 (br s, 1H), 8.58 - 8.56 (m, 1H), 8.54 - 8.51 (m, 1H), 8.34 (dd, 1H), 7.56 (s, 1H), 7.30 (br s, 1H), 6.41 (tt, 1H), 5.08 (s, 2H), 4.66 (s, 2H), 4.43 - 4.32 (m, 2H), 3.66 - 3.62 (m, 2H), 3.60 - 3.52 (m, 4H), 3.46 - 3.41 (m, 2H), 3.33 (s, 3H). |
| 42 | $^1H$ NMR (400MHz, DMSO-$d_6$) δ/ppm: 12.6 (br s, 1H), 8.33 - 8.28 (m, 1H), 7.96 - 7.91 (m, 1H), 7.89 - 7.81 (m, 3H), 7.16 (s, 1H), 4.56 (s, 2H), 4.04 - 3.96 (m, 1H), 3.97 - 3.93 (d, 2H), 1.30 - 1.20 (m, 1H), 1.02- 0.94 (m, 2H), 0.94 - 0.88 (m, 2H), 0.62 - 0.55 (m, 2H), 0.39 - 0.33 (m, 2H). |
| 48 | $^1H$ NMR (400MHz, DMSO-$d_6$) δ/ppm: 12.6 (br s, 1H), 8.27 - 8.23 (m, 1H), 7.77 - 7.68 (m, 2H), 7.56 (s, 1H), 7.30 (br s, 1H), 6.41 (tt, 1H), 5.04 (s, 2H), 4.94 (s, 2H), 4.42 - 4.29 (m, 2H), 3.66 - 3.61 (m, 2H), 3.60 - 3.52 (m, 4H), 3.46 - 3.41 (m, 2H), 2.25 - 2.18 (m, 1H), 1.04 - 0.98 (m, 2H), 0.94 - 0.84 (m, 2H). |

(continued)

| Cpd# | NMR |
|---|---|
| 49 | $^1$H NMR (500 MHz, DMSO-$d_6$) δ/ppm: 12.94 (b.s., 1H), 8.14 (dd, 1H), 7.96 (b.s., 1H), 7.8-7.75 (m, 2H), 7.4 (m, 1H), 7.35 (m, 1H), 6.43 (dd, 1H), 5.95 (s, 2H), 4.47 (m, 2H), 3.78 (s, 2H), 3.6-3.5 (m, 6H), 3.4 (m overlapping with $H_2O$, 2H?). |
| 50 | $^1$H NMR (500 MHz, DMSO-$d_6$) δ/ppm: 12.55 (b.s., 1H?), 8.14 (d, 1H), 7.79 (b.s., 2H), 7.5-7.4 (m, 1H), 7.40 (dd, 1H), 7.32 (m, 1H), 6.38 (dd, 1H), 5.88 (s, 2H), 4.35 (dt, 2H), 3.78 (s, 2H), 3.6-3.5 (m, 6H), 3.4 (m overlapping with $H_2O$, 2H?). |
| 51 | $^1$H NMR d (ppm)(DMSO-$d_6$): 8.29 (1 H, dd), 7.98 (1 H, dd), 7.93-7.84 (3 H, m), 7.22 (1 H, broad s), 5.04 (2 H, s), 4.57 (2 H, s), 4.23-4.20 (2 H, m), 3.72-3.69 (2 H, m), 3.66-3.62 (2 H, m), 3.62-3.54 (4 H, m), 3.45-3.42 (2 H, m), 3.34 (3 H, s). |
| 52 | $^1$H NMR d (ppm)(DMSO-$d_6$): 8.30 (1 H, dd), 7.98 (1 H, dd), 7.94-7.85 (3 H, m), 7.34 (1 H, broad s), 5.05 (2 H, s), 4.60 (2 H, s), 4.44 (2 H, t), 3.67-3.55 (6 H, m), 3.46-3.43 (2 H, m), 1.78 (3 H, t). |
| 53 | $^1$H NMR d (ppm)(DMSO-$d_6$): 8.29 (1 H, dd), 7.98 (1 H, dd), 7.96 (1 H, broad s), 7.94-7.85 (2 H, m), 6.39 or 6.54-6.25 (1 H, tt), 5.03 (2 H, s), 4.64 (2 H, s), 4.41 (2 H, dt), 3.63-3.52 (6 H, m), 3.45-3.41 (2 H, m). |
| 55 | $^1$H NMR (400MHz, $CDCl_3$ ppm) δ 11.02 (bs, 1H), 8.34-8.31 (m, 1H), 7.74-7.66 (m, 4H), 7.60 (s, 1H), 5.02-4.97 (m,1H), 4.81-4.76 (m, 1H), 4.61-4.59 (m, 2H), 4.33-4.31 (3, 1H), 3.45-3.40 (m, 3H), 2.44-2.37 (m, 1H), 2.29-2.20 (m, 1H), 2.17-2.06 (m, 1H), 2.00-1.88 (m, 1H). |
| 64 | $^1$H NMR (400MHz, DMSO-$d_6$ ppm) δ 9.6 (1H, dd), 9.3 (1H, m), 8.3 (1H, dd), 8.0 (2H, dd), 8.0-7.9 (2H, m), 7.4 (1H, s), 6.6-6.3 (1H, m), 5.7 (1H, m), 4.8 (2H, s), 4.5 (2H, ddd), 3.4-3.3 (4H, m), 2.4 (1H, ddd), 2.2 (1H, ddd) |
| 65 | $^1$H NMR (400MHz, DMSO-$d_6$ ppm) δ 9.7 (1H, s), 9.4 (1H, dd), 8.3 (1H, dd), 8.1 (1H, dd), 8.1 (1H, t), 8.0-7.9 (2H, m), 7.4 (1H, s), 6.8-6.7 (1H, m), 5.7-5.6 (1H, m), 5.0 (2H, s), 4.3 (2H, dd), 3.7-3.6 (2H, m), 3.7-3.6 (1H, m), 3.6-3.5 (1H, m), 3.3 (3H, s), 3.3-3.2 (2H, m), 2.4-2.2 (2H, m) |
| 66 | $^1$H NMR (400MHz, DMSO-$d_6$ ppm) δ 12,72 (bs, 1H), 8.24 (d, 1H), 7.88 (s, 1H), 7.75-7.68(m, 2H), 7.32 (s, 1H), 6.57-6.28 (m, 1H), 4.69 (s, 2H), 4.45 (td, 2H), 3.62 (d, 2H), 3.55 (dd, 4H), 3.43 (t, 2H), 2.61 (s, 3H). |
| 68 | $^1$H NMR (400MHz, DMSO-$d_6$ ppm) δ 8.3 (1H, t), 8.2 (1H, d), 8.0-7.9 (4H, m), 7.3 (1H, s), 6.4 (1H, td), 5.7 (1H, dd), 4.7 (2H, s), 4.5 (3H, td), 4.3-4.1 (5H, m) |
| 69 | $^1$H NMR (400MHz, DMSO-$d_6$ ppm) δ 8.3 (1H, m), 8.1-7.8 (4H, m), 7.4-7.3 (1H, m), 6.6-6.3 (1H, m), 5.8-5.7 (1H, m), 4.7 (1H, dd), 4.5-4.4 (4H, m), 4.1-4.0 (1H, m), 3.9-3.8 (2H, m), 3.8-3.0 (2H, m) |
| 70 | $^1$H NMR (400MHz, DMSO-$d_6$ ppm) δ 8.3 (1H, m), 8.0-7.9 (4H, m), 7.3 (1H, s), 6.6-6.3 (1H, m), 4.6 (2H, m), 4.6-4.4 (4H, m), 4.4-4.3 (1H, m), 4.1-4.0 (1H, m), 3.9 (2H, m), 3.9-3.6 (2H, m), 2.2-2.1 (1H, m), 2.0-1.7 (3H, m) |
| 71 | $^1$H NMR (400MHz, DMSO-$d_6$ ppm) δ 8.3 (1H, dd), 8.1 (1H, d), 8.0-7.8 (3H, m), 7.3 (1H, s), 6.6-6.3 (1H, m), 5.7-5.6 (1H, m), 4.7 (2H, t), 4.5 (2H, td), 3.5-3.2 (5H, m), 3.1-3.0 (2H, m), 2.4-2.3 (1H, m), 2.1-2.0 (1H, m) |
| 72 | $^1$H NMR (400MHz, DMSO-$d_6$ ppm) δ 8.3 (1H, m), 8.1 (1H, s), 8.0-7.9 (3H, m), 7.4-7.3 (1H, m), 7.3-6.9 (2H, m), 6.6-6.3 (1H, m), 5.7 (1H, ddd), 4.7 (2H, td), 4.5-4.4 (2H, m), 3.7 (2H, m), 3.4 (2H, m), 2.8-2.7 (3H, m), 2.3-2.1 (1H, m) |
| 74 | $^1$H NMR (400MHz, DMSO-$d_6$ ppm) δ 8.3 (1H, m), 8.1-8.0 (1H, m), 8.0-7.9 (3H, m), 7.4-6.9 (3H, m), 6.6-6.3 (1H, m), 5.7-5.6 (1H, m), 4.7-4.5 (2H, m), 4.5-4.4 (2H, m), 2.6 (6H, s) |
| 75 | $^1$H NMR (400MHz, DMSO-$d_6$ ppm) δ 12.9-12.8 (1H, m), 8.3-8.2 (1H, m), 8.1-8.0 (1H, m), 8.0-7.9 (3H, m), 7.3 (1H, s), 6.6-6.2 (1H, m), 5.4-5.3 (1H, m), 4.6-4.5 (2H, brd), 4.5-4.4 (2H, m), 3.9-3.8 (4H, m), 3.7-3.6 (2H, m), 2.2-2.1 (2H, m) |
| 76 | $^1$H NMR (400MHz, DMSO-$d_6$) δ/ppm: 13.3 (br s, 1H), 8.40 (s, 1H), 8.29 - 8.26 (m, 1H), 7.99 - 7.96 (m, 1H), 7.91 - 7.81 (m, 2H), 7.81 - 7.79 (m, 1H), 7.34 (s, 1H), 6.43 (tt, J = 54 Hz, J = 3.8 Hz, 1H), 6.04 - 5.90 (m, 2H), 5.38 (q, 1H), 4.40 - 4.29 (m, 2H), 1.55 (d, 3H). |

(continued)

| Cpd# | NMR |
|---|---|
| 77 | $^1$H NMR (400MHz, DMSO-$d_6$) δ/ppm: 13.1 (br s, 1H), 7.79 - 7.76 (m, 1H), 7.74 - 7.69 (m, 1H), 7.62 - 7.58 (m, 1H), 7.50 (s, 1H), 7.13 (s, 1H), 4.7 (s, 2H), 4.48 (s, 2H), 4.05 (q, 2H), 2.83 (s, 3H), 1.35 (t, 3H) |
| 78 | $^1$H NMR (500 MHz, DMSO-$d_6$) δ/ppm: 12.6 (broad s, 2H), 8.14 (d, 1H), 7.8-7.7 (m, 2H), 7.6 (b.s., 1H), 7.5-7.4 (m, 1H), 7.35-7.3 (m, 1H), 6.26 (dd~t, 1H), 5.92 (s, 2H), 4.37 (m, 2H), 3.64 (s, 2H), 3.17 (d, 1H). |
| 79 | $^1$H NMR (300 MHz, DMSO-$d_6$) δ/ppm 13.05 (br. s, 1H), 8.40 (s, 1H), 8.28 (d, J=7.25 Hz, 1H), 8.19 (s, 1H), 8.03 (d, J=7.25 Hz, 1H), 7.80-7.93 (m, 3H), 4.84 (s, 2H), 4.46 (s, 2H), 3.87 (d, J=7.05 Hz, 2H), 1.23-1.26 (m, 1H), 0.56-0.65 (m, 2H), 0.31-0.39 (m, 2H). |
| 80 | $^1$H NMR (500 MHz, DMSO-$d_6$) δ/ppm: 15.11 (bs, 1H), 13.09 (bs, 1H), 9.33 (s, 1H), 8.41 (d, 1H), 8.00 (s, 1H), 7.88 (s, 1H), 7.87 (s, 1H), 7.63 (septet, 1H), 7.37 (bs, 1H), 6.44 (tt, 1H), 6.13 (s, 2H), 4.47 (td, 2H). |
| 81 | $^1$H NMR (300 MHz, DMSO-$d_6$) δ/ppm 12.90 (br.s, 1H), 8.39 (s, 1H), 8.26-8.31 (m, 1H), 8.19 (s, 1H), 8.00 (d, J=7.56 Hz, 1H), 7.81-7.92 (m, 2H), 7.80 (s, 1H), 5.49 (q, J=7.22 Hz, 1H), 4.37-4.56 (m, 2H), 3.87 (d, J=7.00 Hz, 2H), 1.60 (d, J=7.22 Hz, 3H), 1.21-1.39 (m, 1H), 0.55-0.65 (m, 2H), 0.30-0.39 (m, 2H). |
| 82 | $^1$H NMR (300 MHz, DMSO-$d_6$) δ/ppm 12.83 (br. s, 1H), 8.47 (s, 1H), 8.30 (d, J=7.68 Hz, 1H), 8.07 (d, J=7.68 Hz, 1H), 7.82-7.97 (m, 3H), 7.78 (s, 1H), 4.83 (s, 2H), 4.49 (s, 2H), 3.87 (d, J=7.05 Hz, 2H), 1.23-1.37 (m, 1H), 0.54-0.67 (m, 2H), 0.27-0.43 (m, 2H). |
| 83 | $^1$H NMR (500 MHz, DMSO-$d_6$) δ/ppm: 12.87 (bs, 1H), 8.30 (s, 1H), 8.28 (d, 1H), 8.00 (d, 1H), 7.87 (t, 1H), 7.83 (t, 1H), 7.68 (s, 1H), 7.58 (s, 1H), 5.49 (q, 1H), 4.39 (q, 2H), 3.81 (d, 2H), 1.60 (d, 3H), 1.21 -1.31 (m, 1H), 0.55 - 0.61 (m, 2H), 0.30 - 0.36 (m, 2H). |
| 84 | $^1$H NMR (400 MHz, DMSO-$d_6$) δ/ppm: 15.19 (s, 1H), 9.27 (s, 1H), 8.32 - 8.42 (m, 2H), 8.12 - 8.22 (m, 1H), 7.98 (s, 1H), 7.83 - 7.94 (m, 1H), 7.73 (s, 1H), 7.55 - 7.67 (m, 1H), 5.92 (s, 2H), 3.83 (d, 2H), 1.19 - 1.33 (m, 1H), 0.53 - 0.64 (m, 2H), 0.28 - 0.39 (m, 2H). |
| 85 | $^1$H NMR (400 MHz, DMSO-$d_6$) δ/ppm: 8.33 (s, 1H), 8.26 (d, 1H), 8.16 (d, 1H), 7.90 - 7.96 (m, 1H), 7.89 (s, 1H), 7.71 (s, 1H), 7.63 (t, 1H), 5.95 (s, 2H), 3.82 (d, 2H), 1.20 - 1.33 (m, 1H), 0.55 - 0.62 (m, 2H), 0.30 - 0.36 (m, 2H). |
| 86 | $^1$H NMR (400MHz, DMSO-$d_6$ ppm) δ 8.36-8.33 (m, 2H), 7.85 (dd, 1H), 7.69 (ddd, 3H), 5.46 (q, 1H), 4.38 (dd, 2H), 3.82 (d, 2H), 1.59 (d, 3H), 1.29-1.23 (m, 1H), 0.59 (dt, 2H), 0.35-0.31 (m, 2H). |
| 87 | $^1$H NMR (400MHz, DMSO-$d_6$ ppm) δ 8.3 (1H, brs), 8.27 (1H, brd), 8.0 (1H, brd), 7.9-7.8 (2H, m), 7.7-7.6 (2H, m), 5.7-5.6 (1H, m), 4.4 (2H, s), 3.8 (4H, d), 3.1-3.0 (5H, m), 2.3-2.1 (2H, m), 1.3-1.2 (1H, m), 0.6-0.5 (2H, m), 0.4-0.3 (2H, m) |
| 88 | $^1$H NMR (500 MHz, DMSO-$d_6$) δ/ppm: 8.37 (s, 1H), 12.05 (s, 1H), 8.10 (d, 1H), 7.90 (d, 1H), 7.79 (t, 1H), 7.75 (s, 2H), 7.43 (t, 1H), 5.77 (s, 2H), 3.82 (d, 2H), 1.46 (s, 6H), 1.21 - 1.32 (m, 1H), 0.56 - 0.62 (m, 2H), 0.30 - 0.37 (m, 2H). |
| 89 | $^1$H NMR (500 MHz, DMSO-$d_6$) δ/ppm: 12.42 (b.s., 1H), 8.35 (s, 1H), 8.11 (d, 1H), 7.93 (d, 1H), 7.80 (t, 1H), 7.77 (s, 1H), 7.73 (s, 1H), 7.45 (t, 1H), 5.77 (s, 2H), 3.82 (d, 2H), 3.65 (s, 2H), 1.22 - 1.32 (m, 1H), 0.56 - 0.62 (m, 2H), 0.31 - 0.36 (m, 2H). |
| 90 | $^1$H NMR (500 MHz, DMSO-$d_6$) δ/ppm: 9.34 (s, 1H), 8.38 (d, 1H), 7.96 (s, 1H), 7.79 - 7.88 (m, 1H), 7.62 (s, 1H), 7.58 (s, 1H), 7.22 (s, 1H), 6.03 (s, 2H), 3.87 (d, 2H), 1.19 - 1.29 (m, 1H), 0.52 - 0.60 (m, 2H), 0.28 - 0.39 (m, 2H). |
| 91 | $^1$H NMR (400MHz, DMSO-$d_6$ ppm) δ 12.3 (1H, s), 8.4 (1H, d), 8.28-8.26 (1H, m), 8.0-7.83 (3H, m), 7.7 (1H, s), 7.2 (1H, s), 6.0 (2H, dd), 5.6-5.5 (1H, m),3.9 (2H, d), 3.5-3.4 (1H, m), 2.3-2.15 (2H, m), 2.0-1.9 (2H, m), 1.3-1.2 (1H, m), 0.6-0.5 (2H, m), 0.4-0.3 (2H, m) |
| 92 | $^1$H NMR (400MHz, DMSO-$d_6$ ppm) δ 8.67 (s, 1H), 8.43-8.39 (m, 2H), 8.24 (d, 1H), 7.75 (s, 1H), 7.19 (s, 1H), 6.02 (dd, 2H), 5.35 (q, 1H), 3.86 (d, 2H), 1.51 (d, 3H), 1.3-1.24 (m, 1H), 0.61-0.56 (m, 2H), 0.35 (q, 2H). |

(continued)

| Cpd# | NMR |
|---|---|
| 93 | $^1H$ NMR (400MHz, DMSO-$d_6$ ppm) δ 8.20 (s, 1H), 7.76 (s, 1H), 7.18 (s, 1H), 5.55 (q, 2H), 5.25 (q, 1H), 3.87 (d, 2H), 2.38-2.33 (m, 2H), 1.59-1.56 (m, 6H), 1.46 (d, 3H), 1.28-1.24 (m, 1H), 0.61-0.56 (m, 2H), 0.57 (d, 2H). |
| 94 | $^1H$ NMR (500 MHz, DMSO-$d_6$) δ/ppm: 12.71 (b.s., 1H), 8.36 (s, 1H), 8.14 (m, 1H), 7.94 (m, 1H), 7.82 (m, 1H), 7.74 (s, 1H), 7.72 (s, 1H), 7.49 (t, 1H), 5.78 (q, 2H), 5.04 (s, 1H), 3.82(d, 2H), 3.39 (s, 3H), 1.27 (m, 1H), 0.59 (m, 2H), 0.33 (m, 2H). |
| 96 | $^1H$ NMR (600 MHz, DMSO-$d_6$) δ/ppm: 12.23 (b.s., 1H), 8.33 (s, 1H), 8.11 (m, 1H), 7.89 (m, 1H), 7.77 (m, 1H), 7.73 (s, 1H), 7.72 (s, 1H), 7.43 (t, 1H), 5.72 (s, 2H), 3.81(d, 2H), 1.41 (m, 2H), 1.26 (m, 1H), 1.21 (m, 2H), 0.58 (m, 2H), 0.32 (m, 2H). |
| 97 | $^1H$ NMR (400MHz, DMSO-$d_6$ ppm) 8.29 (s, 1H), 7.93 (d, 1H), 7.73 (s, 1H), 7.65 (d, 1H), 7.46 (dd, 1H), 7.15 (s, 1H), 5.9 (dd, 2H), 5.39 (q, 1H), 3.9 (s, 3H), 3.84 (d, 2H), 1.55 (d, 3H), 1.27-1.23 (m, 1H), 0.57 (ddd, 2H), 0.34 (q, 2H). |
| 98 | $^1H$ NMR (400 MHz, DMSO-$d_6$) δ/ppm: 12.23 (b.s., 1H), 8.36 (s, 1H), 7.90 (m, 1H), 7.78 (m, 1H), 7.74 (s, 1H), 7.44 (t, 1H), 7.71 (s, 1H), 5.77 (q, 2H), 3.80 (d, 2H), 3.80(t, 1H), 1.95 (m, 2H), 1.26 (m, 1H), 0.89 (t, 3H), 0.58 (m, 2H), 0.32 (m, 2H). |
| 99 | $^1H$ NMR (300 MHz, DMSO-$d_6$) δ/ppm 8.45 (s, 1H), 8.11 (d, J=8.02 Hz, 1H), 7.85-7.96 (m, 3H), 7.78 (t, J=7.47 Hz, 1H), 7.44 (t, J=7.47 Hz, 1H), 5.83 (s, 2H), 3.88 (d, J=6.65 Hz, 2H), 3.62 (s, 2H), 1.16-1.31 (m, 1H), 0.49-0.65 (m, 2H), 0.29-0.41 (m, 2H). |
| 100 | $^1H$ NMR (300 MHz, DMSO-$d_6$) δ/ppm 12.27 (br. s, 1H), 8.46 (s, 1H), 8.11 (dd, J=8.22 Hz, J=1.35 Hz, 1H), 7.87-7.95 (m, 3H), 7.75-7.83 (m, 1H), 7.45 (t, J=7.47 Hz, 1H), 5.75-5.92 (m, 2H), 3.98 (q, J=7.23 Hz, 1H), 3.88 (d, J=6.76 Hz, 2H), 1.43 (d, J=7.23 Hz, 3H), 1.19-1.31 (m, 1H), 0.51-0.63 (m, 2H), 0.28-0.37 (m, 2H). |
| 101 | $^1H$ NMR (400MHz, DMSO-$d_6$ ppm) δ 8.6 (1H, s), 8.2 (1H, dd), 8.1 (1H, s), 8.0 (1H, s), 7.9 (1H, dd), 7.4 (1H, td), 5.8 (2H, s), 3.9 (2H, d), 3.6 (2H, s), 1.3-1.28 (1H, m), 0.64-0.59 (2H, m), 0.38-0.34 (2H, m) |
| 102 | $^1H$ NMR (400MHz, DMSO-$d_6$ ppm) δ 8.4 (1H, s), 7.8 (2H, 2d), 7.7-7.6 (2H, m), 7.2 (1H, brd), 4.9 (1H, m), 3.8 (2H, d), 2.8 (3H, d), 1.3-1.2 (1H, m), 1.2-1.1 (3H, d), 0.6-0.5 (2H, m), 0.4-0.3 (2H, m) |
| 103 | $^1H$ NMR (300 MHz, DMSO-$d_6$) δ/ppm 12.26 (s, 1H), 8.47 (s, 1H), 8.13 (dd, J=8.18 Hz, J=1.21 Hz, 1H), 7.85-7.93 (m, 3H), 7.78 (dt, J=8.18 Hz, J=1.21 Hz, 1H), 7.45 (t, J=7.37 Hz, 1H), 5.74-5.93 (m, 2H), 3.88 (d, J=6.89 Hz, 2H), 3.81 (t, J=7.33 Hz, 1H), 1.88-2.05 (m, 2H), 1.19-1.31 (m, 1H), 0.91 (t, J=7.33 Hz, 3H), 0.52-0.63 (m, 2H), 0.28-0.37 (m, 2H). |
| 104 | $^1H$ NMR (500 MHz, DMSO-$d_6$) δ/ppm: 13.18 (b.s., 1H), 8.24 (m, 1H), 7.92 (m, 1H), 7.87 (m, 1H), 7.75 (s, 1H), 7.24 (s, 1H), 6.07 (s, 2H), 3.93(d, 2H), 1.21 (m, 1H), 0.54 (m, 2H), 0.33 (m, 2H). |
| 105 | $^1H$ NMR (400MHz, DMSO-$d_6$ ppm) 12.44 (s, 1H), 8.37 (s, 1H), 7.76 (s, 1H), 7.74 (s, 1H), 5.38 (s, 2H), 3.84 (d, 2H), 3.46 (s, 2H), 2.9 (t, 2H), 2.35 (t, 2H), 1.75-1.68 (m, 2H), 1.62-1.58 (m, 2H), 1.31-1.23 (m, 1H), 0.62-0.58 (m, 2H), 0.37-0.33 (m, 2H). |
| 106 | $^1H$ NMR (400MHz, DMSO-$d_6$) δ/ppm: 9.36 ppm (s, 1H), 8.34 ppm (d, 1H), 8.05-7.98 ppm (m, 1H), 7.79-7.74 (m, 1H), 7.77 ppm (s, 1H), 7.51-7.48 ppm (m, 1H), 7.31 ppm (s, 1H), 5.99 ppm (s, 2H), 3.92 ppm (d, 2H), 1.24-1.14 ppm (m, 1H), 0.53-49 ppm (m, 2H), 0.33-0.29 ppm (m, 2H). |
| 107 | $^1H$ NMR (500 MHz, DMSO-$d_6$) δ/ppm: 12.80 (b.s., 1H), 12.30 (b.s., 1H), 8.14 (m, 1H), 7.93 (b.s., 1H), 7.79 (m, 1H), 7.74 (m, 1H), 7.47 (t, 1H), 7.19 (b.s., 1H), 5.95 (q, 2H), 3.94 (q, 1H), 3.96(d, 2H), 1.39 (d, 3H), 1.26 (m, 1H), 0.59 (m, 2H), 0.36 (m, 2H). |
| 108 | $^1H$ NMR (500 MHz, DMSO-$d_6$) δ/ppm: 12.69 (b.s., 2H), 8.14 (d, 1H), 7.92 (s, 1H), 7.79 (m, 1H), 7.76 (m, 1H), 7.47 (m, 1H), 7.19 (b.s., 1H), 5.95 (s, 2H), 3.95 (d, 2H), 3.64(s, 2H), 1.25 (m, 1H), 0.58 (m, 2H), 0.36 (m, 2H). |

(continued)

| Cpd# | NMR |
|---|---|
| 109 | $^1H$ NMR (500 MHz, DMSO-$d_6$) δ/ppm: 12.49 (b.s., 2H), 8.12 (m, 1H), 7.74 - 7.74 (m, 2H), 7.46 (m, 1H), 7.71 (s, 1H), 7.13 (b.s., 1H), 5.89 (s, 2H), 3.86 (d, 2H), 3.64(s, 2H), 1.23 (m, 1H), 0.55 (m, 2H), 0.33 (m, 2H). |
| 110 | $^1H$ NMR (300 MHz, DMSO-$d_6$) δ/ppm 8.31 (d, J=8.06 Hz, 1H), 7.91-8.02 (m, 3H), 7.65-7.73 (m, 1H), 7.21 (s, 1H), 6.18 (s, 2H), 3.96 (d, J=6.85 Hz, 2H), 1.20-1.30 (m, 1H), 0.53-0.64 (m, 2H), 0.31-0.40 (m, 2H). |
| 111 | $^1H$ NMR (400MHz, DMSO-$d_6$) δ/ppm: 13.1 (br s, 1H), 8.39 (s, 1H), 8.29 - 8.25 (m, 1H), 7.99- 7.95 (m, 1H), 7.90 (s, 1H), 7.88 - 7.79 (m, 2H), 7.13 (s, 1H), 5.93 (s, 2H), 4.66 (s, 2H), 3.94 (d, 2H), 1.29 - 1.20 (m, 1H), 0.59 - 0.53 (m, 2H), 0.38 - 0.33 (m, 2H). |
| 112 | $^1H$ NMR (400MHz, DMSO-$d_6$) δ/ppm: 13.1 (br s, 1H), 10.6 (br s, 1H), 8.32 - 8.27 (m, 1H), 7.98 - 7.95 (m, 1H), 7.89 - 7.82 (m, 2H), 7.75 (s, 1H), 7.65 (s, 1H), 4.7 (s, 2H), 4.66 (s, 2H). |
| 113 | $^1H$ NMR (300 MHz, DMSO-$d_6$) δ/ppm: 12.53-13.25 (m, 2H), 7.55-7.90 (m, 2H), 4.68 (s, 2H), 4.20 (s, 2H), 2.35-2.50 (m, 4H), 1.54-1.74 (m, 4H). |
| 114 | $^1H$ NMR (400MHz, DMSO-$d_6$) δ/ppm: 13.15 (br s, 1H), 8.41 (s, 1H), 8.31 - 8.28 (m, 1H), 8.05 - 8.01 (m, 1H), 7.95 - 7.85 (m, 2H), 7.73 (s, 1H), 7.17 (s, 1H), 5.96 (s, 2H), 4.86 (s, 2H), 3.86 (d, 2H), 1.31 - 1.21 (m, 1H), 0.61 - 0.55 (m, 2H), 0.38 - 0.33 (m, 2H). |
| 115 | $^1H$ NMR d (ppm)(DMSO-$d_6$): 8.26 (1 H, dd), 7.94 (1 H, dd), 7.86-7.78 (2 H, m), 7.49 (1 H, s), 7.09 (1 H, s), 4.54 (4 H, d), 3.84 (2 H,d), 1.28-1.17 (1 H, m), 0.58-0.53 (2 H, m), 0.35-0.31 (2 H, m). |
| 116 | $^1H$ NMR d (ppm)(DMSO-$d_6$): 8.27 (1 H, dd), 7.95 (1 H, dd), 7.87-7.78 (2 H, m), 7.33 (1 H, d), 4.55 (4 H, d), 3.82 (2 H,d), 1.25-1.17 (1 H, m), 0.54-0.49 (2 H, m), 0.26-0.22 (2 H, m). |
| 117 | $^1H$ NMR d (ppm)(DMSO-$d_6$): 8.27 (1 H, dd), 7.93 (1 H, dd), 7.85-7.72 (2 H, m), 7.73 (1 H, s), 7.63 (1 H, m), 4.57 (4 H, d). |
| 118 | $^1H$ NMR d (ppm)(DMSO-$d_6$): 8.27 (1 H, dd), 7.95 (1 H, dd), 7.87-7.78 (2 H, m), 7.54 (1 H, s), 7.26 (1 H, s), 6.55-6.26 or 6.41 (1 H, tt), 4.59 (2 H, s), 4.54 (2 H, s), 4.35 (2 H, dt). |
| 119 | $^1H$ NMR d (ppm)(DMSO-$d_6$): 13.20-12.40 (2 H, br d), 7.59 (1 H, s), 7.32 (1 H, broad s), 7.22-6.96 or 7.09 (1 H, br d), 6.57-6.28 or 6.42 (1 H, tt), 4.69 (2 H, s), 4.38 (2 H, dt), 4.16 (2 H, s), 2-48-2.38 (4 H, m), 1.62 (4 H, m). |
| 120 | $^1H$ NMR d (ppm)(DMSO-$d_6$): 12.96 (1 H, br s), 12.30 (1 H, broad s), 7.56 (1 H, s), 7.19 (1 H, broad s), 7.22-6.96 or 7.09 (1 H, m), 4.70 (2 H, s), 4.15 (2 H, s), 3.84 (3 H, s), 2-48-2.38 (4 H, m), 1.62 (4 H, m). |
| 121 | $^1H$ NMR d (ppm)(DMSO-$d_6$): 7.53 (1 H, s), 7.15(1 H, s), 4.51 (2 H, s), 4.11 (2 H, s), 4.06 (2 H, q), 2.46-2.34 (4 H, m), 1.60 (4 H, m), 1.36 (3 H, t). |
| 122 | $^1H$ NMR (500 MHz, DMSO-$d_6$) δ/ppm: 8.32 (d, 1H), 8.00 (d, 1H), 8.0-7.6 (m, 2H), 7.66 (b.s., 1H), 7.22 (d, 1H), 5.40 (d, 1H), 4.72 (dd, 2H), 3.91 (d, 2H), 1.49 (d, 3H), 1.25 (m, 1H), 0.58 (m, 2H), 0.35 (m, 2H). |
| 123 | $^1H$ NMR d (ppm)(DMSO-$d_6$): 8.30 ((1 H, dd), 7.98 (1 H, dd), 7.93-7.84 (2 H, m), 7.69 (1 H, s), 7.20 (1 H, s), 4.82 (2 H, s), 4.59 (2 H, s), 4.20-4.02 (1H, m), 3.94 (2 H, d), 3.17 (2 H, s), 1.29-1.17 (1H, m), 0.57-0.52 (2 H, m), 0.36-0.32 (2 H, m). |
| 124 | $^1H$ NMR d (ppm)(DMSO-$d_6$): 8.28 ((1 H, dd), 7.97 (1 H, dd), 7.88-7.79 (2 H, m), 7.28 (1 H, d), 7.10 (1 H, d), 4.46 (2 H, s), 4.51 (2 H, s), 3.83 (2 H, d), 1.25-1.18 (1H, m), 0.58-0.53 (2 H, m), 0.33-0.29 (2 H, m). |
| 126 | $^1H$ NMR (400MHz, DMSO-$d_6$) δ/ppm: 12.5 (br s, 1H), 7.77 - 7.68 (m, 2H), 7.54 (s, 1H), 7.35-7.25 (m, 1H), 7.16 - 7.06 (m, 1H), 6.42 (tt, 1H), 4.80 (s, 2H), 4.52 (s, 2H), 4.38 (q, 2H). |
| 127 | $^1H$ NMR d (ppm)(DMSO-$d_6$): 8.29 (1 H, dd), 7.95 (1 H, dd), 7.90-7.82 (2 H, m), 7.69 (1 H, s), 7.21 (1 H, s), 4.75 (2 H, s), 4.58 (2 H, s), 4.10 (2 H, q), 1.32 (3 H, t). |

(continued)

| Cpd# | NMR |
|---|---|
| 128 | $^1$H NMR (400MHz, DMSO-$d_6$) δ/ppm: 8.31 - 8.27 (d, 1H), 8.18 - 8.14 (m, 1H), 7.97 - 7.91 (m, 1H), 7.90 - 7.84 (m, 1H), 7.70 (s, 1H), 6.83 (s, 1H), 6.59 (br s, 1H), 5.23 (s, 2H), 4.83 (s, 2H), 4.10 (q, 2H), 1.35 (t, 3H). |
| 129 | $^1$H NMR d (ppm)(DMSO-$d_6$): 8.33 ((1 H, dd), 8.04 (1 H, dd), 8.00-7.90 (2 H, m), 7.55 (1 H, d), 7.31 (1 H, d), 4.78 (4 H, d), 4.13 (2 H, q), 1.37 (3 H, t). |
| 130 | $^1$H NMR d (ppm)(DMSO-$d_6$): 8.32 ((1 H, dd), 8.03 (1 H, dd), 7.99-7.89 (2 H, m), 7.55 (1 H, d), 7.42 (1 H, d), 6.43 or 6.57-6.28 (1 H, dt), 4.79 (2 H, s), 4.73 (2 H, s), 4.43 (2 H, dt). |
| 131 | $^1$H NMR (400 MHz, DMSO-$d_6$) δ/ppm:12.48 (2 H, br s), 8.30 (1 H, dd), 7.98 (1 H, dd), 7.93-7.85 (2 H, m), 7.55 (1 H, br s), 7.16 (1 H, br s), 4.82 (2 H, s), 4.56 (2 H, s), 4.08 (2 H, br s), 1.91 (3 H, s), 1.39-1.36 (2 H, m), 1.16 (2 H, m). |
| 132 | $^1$H NMR d (ppm)(DMSO-$d_6$): 12.37 (1 H, broad s), 8.30 (1 H, dd), 7.96 (1 H, dd), 7.93-7.84 (2 H, m), 7.51 (1 H, broad s), 7.12 (1 H, broad s),4.83 (2 H, s), 4.55 (2 H, s), 3.67 (2 H, s), 1.91 (2 H, s), 1.03 (9 H, s). |
| 133 | $^1$H NMR d (ppm)(DMSO-$d_6$): 12.47 (1 H, broad s), 8.30 (1 H, dd), 7.98 (1 H, dd), 7.93-7.84 (2 H, m), 7.55 (1 H, broad s), 7.17 (1 H, broad s),4.82 (2 H, s), 4.56 (2 H, s), 4.08 (2 H, broad s), 1.91 (2 H, s), 1.39-1.35 (2 H, m), 1.17-1.14 (2 H, m). |
| 134 | $^1$H NMR (400MHz, DMSO-$d_6$) δ/ppm: 13.15 (br s, 1H), 10.16 (s, 1H), 8.31 - 8.28 (m, 1H), 8.18 - 8.14 (m, 1H), 7.97 - 7.92 (m, 1H), 7.90 - 7.85 (m, 1H), 7.70 (s, 1H), 6.81 (s, 1H), 5.23 (s, 2H), 4.85 (s, 2H), 3.92 (d, 2H), 1.29 (m, 1H), 0.61 - 0.55 (m, 2H), 0.37 - 0.29 (m, 2H). |
| 135 | $^1$H NMR (500 MHz, DMSO-$d_6$) δ/ppm: 13.14 (bs, 1H), 8.52 (s, 1H), 8.27 - 8.32 (m, 1H), 8.25 (s, 1H), 8.00 - 8.05 (m, 1H), 7.85 - 7.96 (m, 2H), 7.26 (s, 1H), 6.05 (s, 2H), 4.85 (s, 2H), 3.91 (d, 2H), 1.20 - 1.33 (m, 1H), 0.55 - 0.64 (m, 2H), 0.31 - 0.42 (m, 2H). |
| 136 | $^1$H NMR (400MHz, DMSO-$d_6$) δ/ppm: 12.82 (br s, 2H), 8.28 - 8.24 (m, 1H), 7.94 - 7.90 (m, 1H), 7.85 - 7.75 (m, 2H), 7.50 (s, 1H), 7.14 (s, 1H), 4.60 (s, 2H), 4.51 (s, 2H), 3.80 (s, 3H). |
| 137 | $^1$H NMR (400MHz, DMSO-$d_6$) δ/ppm: 13.13 (br s, 1H), 8.39 (s, 1H), 8.31 - 8.28 (m, 1H), 8.04 - 8.01 (m, 1H), 7.94 - 7.84 (m, 3H), 7.16 (s, 1H), 5.96 (s, 2H), 4.86 (s, 2H), 4.05 (q, 2H), 1.37 (t, 3H). |
| 138 | $^1$H NMR d (ppm)(DMSO-$d_6$): 13.03 (1 H, broad s), 8.30 (1 H, dd), 7.99 (1 H, dd), 7.93-7.85 (3 H, m), 7.32-7.12 (1 H, broad d), 4.83 (2 H, s), 4.60 (2 H, s), 4.14 (2 H, m), 1.40-1.32 (3 H, m). |
| 139 | $^1$H NMR d (ppm)(DMSO-$d_6$): 12.90 (1 H, broad s), 8.30 (1 H, dd), 7.99-7.85 (4 H, m), 7.33 (1 H, broad s), 6.58-6.28 or 6.43 (1 H, tt), 4.81 (2 H, s), 4.61 (2 H, s), 4.47 (2 H, dt). |
| 140 | $^1$H NMR (400MHz, DMSO-$d_6$) δ/ppm: 8.32 - 8.28 (m, 1H), 8.07 - 8.04 (d, 1H), 8.01 - 7.97 (m, 1H), 7.95 - 7.90 (m, 1H), 7.90 - 7.75 (m, 1H), 7.83 (s, 1H), 7.73 (s, 1H), 4.72 (s, 2H), 4.66 (s, 2H), 3.84 - 3.74 (m, 3H), 1.97 - 1.87 (m, 1H), 1.82 - 1.73 (m, 1H), 1.77 - 1.55 (m, 2H), 1.47 - 1.39 (m, 1H), 1.36 - 1.27 (m, 1H). |
| 141 | $^1$H NMR (400MHz, DMSO-$d_6$) δ/ppm: 12.9 (br s, 1H), 8.32 - 8.25 (m, 2H), 7.99 - 7.97 (m 1H), 7.94 - 7.84 (m, 2H), 7.78 (s, 1H), 7.68 (s, 1H), 4.73 (s, 2H), 4.61 (s, 2H), 4.03 - 3.95 (m, 1H), 3.32 - 3.19 (m, 2H), 3.12 - 3.04 (m, 2H), 2.10 - 2.00 (m, 2H), 1.96 - 1.86 (m, 2H). |
| 142 | $^1$H NMR (400MHz, DMSO-$d_6$) δ/ppm: 12.9 (br s, 1H), 8.36 - 8.33 (m, 1H), 7.99 - 7.95 (m 1H), 7.94 - 7.89 (m, 1H), 7.89 - 7.84 (m, 1H), 7.79 (s, 1H), 7.68 (s, 1H), 5.05 (s, 2H), 4.61 (s, 2H), 3.66 - 3.61 (m, 2H), 3.60 - 3.53 (m, 4H), 3.46 - 3.41 (m, 2H). |
| 143 | $^1$H NMR (400MHz, DMSO-$d_6$) δ/ppm: 8.87 (s, 1H), 8.28 - 8.25 (m, 1H), 8.11 (d, 1H), 8.01 - 7.97 (m, 1H), 7.92 - 7.82 (m, 2H), 7.73 (s, 1H), 7.13 (s, 1H), 5.95 (s, 2H), 4.75 (s, 2H), 3.90 - 3.74 (m, 5H), 1.97 - 1.88 (m, 1H), 1.85 - 1.75 (m, 1H), 1.68 - 1.56 (m, 2H), 1.47 - 1.39 (m, 1H), 1.38 - 1.22 (m, 2H), 0.61 - 0.55 (m, 2H), 0.38 - 0.32 (m, 2H). |
| 144 | $^1$H NMR (400MHz, DMSO-$d_6$) δ/ppm: 8.39 (s, 1H), 8.28 - 8.25 (m, 1H), 8.01 - 7.97 (m, 1H), 7.92 - 7.82 (m, 3H), 7.73 (s, 1H), 7.16 (s, 1H), 5.95 (s, 2H), 4.71 (s, 2H), 3.85 (d, 2H), 1.27 (s, 9H), 1.29 - 1.21 (m, 1H), 0.61 - 0.55 (m, 2H), 0.38 - 0.32 (m, 2H). |

(continued)

| Cpd# | NMR |
|---|---|
| 145 | $^1$H NMR (300 MHz, DMSO-$d_6$) δ/ppm 12.71 (br. s, 1H), 7.78 (s, 1H), 7.69 (s, 1H), 4.90 (s, 2H), 4.19 (s, 2H), 3.36-3.64 (m, 8H), 2.34-2.50 (m, 4H), 1.63 (br. s, 4H). |
| 146 | $^1$H NMR (300 MHz, DMSO-$d_6$) d 12.71 (s, 1H), 12.67 (s, 1H), 7.97 (d, J=6.97 Hz, 2H), 7.84 (s, 1H), 7.74 (s, 1H), 7.72 (s, 1H), 7.62 (d, J=1.05 Hz, 1H), 4.69 (d, J=4.18 Hz, 2H), 4.59 (s, 4H), 4.19 (d, J=1.92 Hz, 4H), 3.68-3.86 (m, 2H), 3.55-3.65 (m, 1H), 3.06-3.17 (m, 1H), 2.35-2.46 (m, 8H), 1.83-1.98 (m, 2H), 1.70-1.81 (m, 2H), 1.59-1.67 (m, 8H), 1.37-1.48 (m, 2H), 1.28-1.35 (m, 2H), 1.18-1.28 (m, 4H) |
| 147 | $^1$H NMR (300 MHz, DMSO-$d_6$) d 12.69 (br. s., 1H), 7.58-7.85 (m, 3H), 4.54 (s, 2H), 4.19 (s, 2H), 2.35-2.46 (m, 4H), 1.62 (br. s., 4H), 1.22 (s, 9H) |
| 149 | $^1$H NMR d (ppm)(DMSO-$d_6$): 8.28 (1 H, dd), 8.00 (1 H, dd), 7.92-7.83 (2H, m), 7.35 (1 H, m), 5.03 (2 H, s), 4.35 (2 H, s), 3.81 (2 H, d), 3.65-3.55 (6 H, m), 3.45-3.42 (2 H, m), 1.27-1.15 (1H, m), 0.54-0.49 (2 H, m), 0.27-0.23 (2 H, m). |
| 150 | $^1$H NMR d (ppm)(DMSO-$d_6$): 8.28 (1 H, dd), 8.12 (1 H, d), 7.97 (1 H, dd), 7.92-7.82 (2 H, m), 7.50 (1 H, m), 7.12 (1 H, broad s), 4.70 (2 H, s), 4.53 (2 H, s), 4.00 (1 H, m), 3.86 (2 H, d), 1.89-1.75 (2 H, m), 1.71-1.56 (2 H, m), 1.56-1.44 (2 H, m), 1.44-1.32 (2 H, m), 1.32-1.18 (2 H, m), 0.59-0.54 (2 H, m), 0.35-0.31 (2 H, m). |
| 151 | $^1$H NMR d (ppm)(DMSO-$d_6$): 8.6 (0.5 H, s), 8.28 (1 H, dd), 7.98 (2 H, dd), 7.92-7.83 (2 H, m), 7.55 (1 H, s), 7.29 1 H, s), 6.40 (1.5 H, dt), 5.05 (2 H, s), 4.54 (1H, s), 4.36 (2 H, dt), 3.66-3.61 (2 H, m), 3.60-3.55 (3H, m), 3.45-3.43 (2 H, m). |
| 152 | $^1$H NMR (400MHz, DMSO-$d_6$) δ/ppm: 8.21 (s, 1H), 7.77 - 7.68 (m, 2H), 7.54 (s, 1H), 7.38-7.25 (m, 1H), 6.42 (tt, 1H), 5.04 (s, 2H), 4.49 (s, 2H), 4.42 - 4.30 (m, 2H), 3.67 - 3.62 (m, 2H), 3.61 - 3.53 (m, 4H), 3.47 - 3.43 (m, 2H). |
| 153 | $^1$H NMR (400MHz, DMSO-$d_6$) δ/ppm: 8.67 (s, 1H), 8.48 (br s, 1H), 8.42 (d, 1H), 8.27 (dd, 1H), 7.58 (s, 1H), 7.32 (br s, 1H), 6.43 (tt, 1H), 5.06 (s, 2H), 4.63 (s, 2H), 4.43 - 4.32 (m, 2H), 3.65 - 3.61 (m, 2H), 3.59 - 3.52 (m, 4H), 3.45 - 3.41 (m, 2H). |
| 154 | $^1$H NMR (400MHz, DMSO-$d_6$) δ/ppm: 8.29 - 8.26 (d, 1H), 8.18 - 8.14 (m, 1H), 7.96 - 7.85 (m, 2H), 7.69 (s, 1H), 6.79 (s, 1H), 6.59 (br s, 1H), 5.21 (s, 2H), 5.08 (s, 2H), 4.10 (q, 2H), 3.68 - 3.63 (m, 2H), 3.61 - 3.56 (m, 4H), 3.47 - 3.43 (m, 2H), 1.35 (t, 3H). |
| 155 | $^1$H NMR (500 MHz, DMSO-$d_6$) δ/ppm: 12.3 (broad s, 1H), 8.32 (dd, 1H), 8.0-7.9 (m, 1H), 7.9-7.8 (m, 2H), 7.50 (s, 1H), 7.15 (dd, 1H), 5.79, (q) + 5.75 (s) (2H), 4.54 (d, 2H), 3.86 (dd, 2H), 3.5-3.4 (m, 8H), 1.61 (m, 1H), 1.45 (d, 3H), 1.25 (m, 2H), 0.94 (m, 1H), 0.58 (m, 2H), 0.36 (m, 2H). |
| 156 | $^1$H NMR (400 MHz, DMSO-$d_6$) δ/ppm:12.42 (1 H, broad s), 8.28 ((1 H, dd), 8.03 (1 H, dd), 7.99-7.89 (2 H, m), 7.55 (1 H, d), 7.42 (1 H, d), 6.43 or 6.57-6.28 (1 H, dt), 4.79 (2 H, s), 4.73 (2 H, s), 4.43 (2 H, dt). |
| 157 | $^1$H NMR (400 MHz, DMSO-$d_6$) δ/ppm:12.33 (1 H, broad s), 8.28 (1 H, dd), 7.99 (1 H, dd), 7.92-7.83 (2 H, m), 7.30 (1 H, d), 7.15 (1 H, broad s), 5.05 (2 H, s), 4.51 (2 H, s), 4.06 (2 H, q), 3.61 (6 H, m), 3.37 (2 H, m), 1.34 (3 H, t). |
| 158 | $^1$H NMR d (ppm)(DMSO-$d_6$): 12.66 (1 H, broad s), 8.29 (1 H, dd), 7.97 (1 H, dd), 7.93-7.85 (2 H, m), 7.13 (1 H, broad s), 4.82 (2 H, s), 5.04 (2 H, m), 4.57 (2 H, m), 3.95 (2 H, m), 3.63-3.52 (6 H, m), 3.43 (2 H, m), 1.25 (1 H, m), 0.58 (2 H, m), 0.36 (2 H, m). |
| 159 | $^1$H NMR (500 MHz, DMSO-$d_6$) δ/ppm: 12.60 (b.s., 1H), 8.13 (d, 1H), 7.79 (dd, 2H), 7.60 (s, 1H), 7.5-7.4 (m, 1H), 7.32 (b.s., 1H), 6.41 (dd, 1H), 5.90 (s, 2H), 4.38 (m, 2H), 3.78 (s. 2H), 3.56 (m, 6H), 3.42 (m, 2H). |
| 160 | $^1$H NMR d (ppm)(DMSO-$d_6$): 12.48 (1 H, broad s), 8.29 (1 H, dd), 7.99 (1 H, dd), 7.93-7.84 (2 H, m), 7.52 (1 H, broad s), 7.15 (1 H, broad s), 5.06 (2 H, s), 4.54 (2 H, s), 3.68-3.54 (8 H, m), 3.46-3.44 (2 H, m), 1.04 (9 H, s). |

(continued)

| Cpd# | NMR |
|---|---|
| 161 | $^1$H NMR d (ppm)(DMSO-$d_6$): 8.38 (1 H, broad s), 8.29 (1 H, dd), 7.99 (1 H, dd), 7.93-7.84 (2 H, m), 7.56 (1 H, s), 7.20 (1 H, broad s), 5.06 (2 H, s), 4.55 (2 H, s), 4.09 (2 H, s), 3.66-3.42 (8 H, m), 1.40-1.36 (2 H, m), 1.18-1.15 (2 H, m). |
| 162 | $^1$H NMR d (ppm)(DMSO-$d_6$): 12.42 (1 H, broad d), 8.29 (1 H, dd), 7.98 (1 H, unresolved dd), 7.92-7.83 (2 H, m), 7.58-7.51 (1 H, broad d), 7.26-7.12 (1 H, broad d), 5.16 (1 H, AB system), 4.93 (1 H, AB system), 4.54 (2 H, s), 4.30 (2 H, d), 4.12-4.04 (2 H, m), 3.88 (1 H, d), 3.61-3.55 (1 H, m), 3.48-3.43 (1 H, m), 2.81-2.73 (1 H, m), 2.33-2.27 (1 H, m), 1.37 (2 H, broad s), 1.20-1.14 (1 H, m), 1.14-1.08 (6 H, m). |
| 163 | $^1$H NMR d (ppm)(DMSO-$d_6$): 8.42 (1 H, broad s), 8.28 (1 H, dd), 7.97 (1 H, dd), 7.92-7.83 (2 H, m), 7.51 (1 H, broad s), 7.11 (1 H, broad s), 5.05 (2 H, s), 4.53 (2 H, s), 3.79 (2 H, s), 3.64-3.54 (6 H, m), 3.45-3.43 (2 H, m), 1.21 (3 H, s), 0.55-0.52 (2 H, m), 0.40-0.37 (2 H, m). |
| 164 | $^1$H NMR (400MHz, DMSO-$d_6$) $\delta$/ppm: 7.93 (s, 1H), 7.35 (s, 1H), 6.45 (tt, 1H), 4.91 (s, 2H), 4.54 - 4.43 (m, 2H), 4.18 (s, 2H), 3.63 - 3.53 (m, 4H), 3.53 - 3.47 (m, 2H), 3.44 - 3.38 (m, 2H), 2.49 - 2.39 (m, 4H), 1.64 br s, 4H). |
| 166 | $^1$H NMR d (ppm)(DMSO-$d_6$): 12.34 (1 H, broad s), 8.28 (1 H, dd), 7.98 (1 H, dd), 7.92-7.83 (1 H, m), 7.51 (1 H, broad s), 7.13 (1 H, broad s), 5.05 (2 H, s), 4.53 (2 H, s), 3.87 (2 H, d), 3.65-3.56 (6 H, m), 3.45-3.43 (2 H, m), 1.24 (1 H, m), 0.59-0.54 (2 H, m), 0.36-0.32 (2 H, m). |
| 167 | $^1$H NMR d (ppm)(DMSO-$d_6$): 12.55 (1 H, broad s), 7.88 (1 H, broad s), 7.17 (1 H, broad s), 4.91 (2 H, s), 4.19-4.13 (4 H, m), 3.61-3.54 (6 H, m), 3.43-3.40 (2 H, m), 2.47-2.41 (4 H, m), 1.64 (4 H, m), 1.38 (3 H, t). |
| 168 | $^1$H NMR d (ppm)(DMSO-$d_6$): 8.27 (1 H, dd), 7.98 (1 H, dd), 7.92-7.84 (2 H, m), 7.69 (1 H, broad s), 7.22 (1 H, broad s), 5.05 (2 H, s), 4.58 (2 H, s), 4.11 (2 H, q), 3.65-3.56 (6 H, m), 3.45-3.43 (2 H, m), 1.33 (3 H, t). |
| 169 | $^1$H NMR (500 MHz, DMSO-$d_6$) $\delta$/ppm: 8.26 (dd, 1H), 7.70 (dd, 2H), 7.57 (b.s., 1H), 7.5-7.4 (m, 1H), 7.35 (b.s., 1H), 7.30 (b.s., 1H), 6.41 (dd, 1H), 4.90 (s, 2H), 4.37 (m, 2H), 4.27 (s, 2H), 3.7-3.6 (m, 5H), 3.45 (m, 2H). |
| 170 | $^1$H NMR d (ppm)(DMSO-$d_6$): 8.29 (1 H, dd), 7.98 (1 H, dd), 7.93-7.84 (3 H, m), 7.18 (1 H, broad s), 5.05 (2 H, s), 4.57 (2 H, s), 4.14 (2 H, q), 3.65-3.55 (6 H, m), 3.45-3.42 (2 H, m), 1.37 (3 H, t). |
| 171 | $^1$H NMR (400MHz, DMSO-$d_6$) $\delta$/ppm: 12.9 (br s, 1H), 8.32 - 8.28 (m, 1H), 8.00 - 7.96 (m, 1H), 7.96 - 7.84 (m, 2H), 7.44 (br s, 1H), 7.26 (br s, 1H), 6.43 (tt, 1H), 5.04 (s, 2H), 4.59 (s, 2H), 4.55 - 4.37 (m, 2H), 3.66 - 3.61 (m, 2H), 3.59 - 3.53 (m, 4H), 3.48 - 3.41 (m, 2H). |
| 172 | $^1$H NMR d (ppm)(DMSO-$d_6$): 12.46 (1 H, broad s), 8.30 (1 H, dd), 7.99 (1 H, dd), 7.93-7.84 (2 H, m), 7.57 (1 H, broad d), 7.30 (1 H, broad d), 5.06 (2 H, s), 4.55 (2 H, s), 4.40-4.28 (2 H, m), 3.65-3.57 (6 H, m), 3.46-3.44 (2 H, m), 1.78 (3 H, t). |
| 173 | $^1$H NMR d (ppm)(DMSO-$d_6$): 8.30 (1 H, dd), 7.99 (1 H, dd), 7.94-7.85 (2 H, m), 7.55 (1 H, s), 7.16 (1 H, s), 5.05 (2 H, s), 4.56 (2 H, s), 3.81 (2 H, d), 3.66-3.56 (6 H, m), 3.47-3.43 (2 H, m), 2.11-2.01 (2 H, m), 1.01 (6 H, d). |
| 174 | $^1$H NMR d (ppm)(DMSO-$d_6$): 12.37 (1 H, broad s), 8.29 (1 H, dd), 7.99 (1 H, unresolved dd), 7.93-7.84 (2 H, m), 7.52 (1 H, large d, rotamers?), 7.20 (1 H, large d, rotamers?), 5.06 (2 H, s), 4.54 (2 H, s), 4.18-4.11 (2 H, m), 3.72-3.64 (8 H, m), 3.47-3.44 (2 H, m), 3.35 (3 H, m). |
| 175 | $^1$H NMR d (ppm)(DMSO-$d_6$): 12.88 (1 H, broad s), 8.30 (1 H, dd), 7.98 (1 H, dd), 7.96-7.85 (3 H, m), 7.41 (1 H, broad s), 5.05 (2 H, s), 4.93 (2 H, m), 4.61 (2 H, s), 3.65-3.56 (6 H, m), 3.46-3.43 (2 H, m). |
| 176 | $^1$H NMR (400MHz, DMSO-$d_6$) $\delta$/ppm: 12.6 (br s, 1H), 8.33 - 8.28 (m, 1H), 8.01 - 7.97 (m, 1H), 7.95 - 7.85 (m, 3H), 7.35 (br s, 1H), 6.44 (tt, 1H), 5.00 - 4.85 (m, 2H), 4.59 (s, 2H), 4.54 - 4.42 (m, 2H), 3.85 - 3.70 (m, 1H), 3.63 - 3.47 (m, 1H), 3.55 - 3.47 (m, 1H), 3.27 - 3.19 (m, 0.5H), 3.04 - 2.97 (m, 0.5H), 2.82 - 2.60 (m, 1H), 2.19 (s, 3H), 2.17 (s, 3H), 2.13 - 1.97 (m, 1H), 1.83 - 1.61 (m, 1H). |

(continued)

| Cpd# | NMR |
|---|---|
| 178 | $^1$H NMR (400MHz, DMSO-$d_6$) δ/ppm: 12.6 (br s, 1H), 8.41 - 8.35 (d, 1H), 8.33 - 8.28 (m, 1H), 8.01 - 7.97 (m, 1H), 7.95 - 7.85 (m, 3H), 7.35 (br s, 1H), 6.44 (tt, 1H), 4.74 (s, 2H), 4.60 (s, 2H), 4.54 - 4.42 (m, 2H), 4.30 - 4.22 (m, 1H), 3.82 - 3.72 (m, 2H), 3.71 - 3.64 (m, 1H), 3.48 (dd, 1H), 2.14 - 2.05 (m, 1H), 1.78 - 1.69 (m, 1H). |
| 181 | $^1$H NMR (400MHz, DMSO-$d_6$) δ/ppm: 12.80 (br s, 1H), 8.33 - 8.28 (m, 1H), 8.01 - 7.95 (m, 2H), 7.94 - 7.85 (m, 2H), 7.43 (s, 0.5H), 7.25 (s, 0.5H), 6.60 - 6.24 (m, 1H), 5.18 (s, 2H), 4.60 (s, 2H), 4.54 - 4.42 (m, 2H), 3.55 (t, 2H), 2.72 (t, 2H), 1.19 - 1.14 (m, 1H), 0.99 - 0.92 (m, 4H). |
| 182 | $^1$H NMR (400MHz, DMSO-$d_6$) δ/ppm: 12.79 (br s, 1H), 8.32 - 8.28 (m, 1H), 8.02 - 7.83 (m, 5H), 7.43 (s, 0.5H), 7.25 (s, 0.5H), 6.60 - 6.24 (m, 1H), 4.80 (s, 2H), 4.59 (s, 2H), 4.54 - 4.38 (m, 2H), 3.14 (d, 2H), 3.10 (s, 3H), 1.06 (s, 6H). |
| 187 | $^1$H NMR (400MHz, DMSO-$d_6$) δ/ppm: 12.79 (br s, 1H), 8.32 - 8.27 (m, 1H), 8.20 - 8.16 (m, 1H), 7.99 - 7.96 (m, 1H), 7.94 - 7.84 (m, 3H), 7.43 (s, 0.5H), 7.25 (s, 0.5H), 6.60 - 6.24 (m, 1H), 4.74 (s, 2H), 4.59 (s, 2H), 4.54 - 4.38 (m, 2H), 3.38 - 3.34 (m, 2H), 3.28 - 3.22 (m, 5H). |
| 194 | $^1$H NMR (400MHz, DMSO-$d_6$) δ/ppm: 12.79 (br s, 1H), 8.32 - 8.25 (m, 1H), 7.99 - 7.96 (m, 1H), 7.94 - 7.84 (m, 3H), 7.33 (s, 1H), 6.43 (tt, 1H), 6.08 (tt, 1H), 5.18 (s, 2H), 5.08 (s, 1H), 4.59 (s, 2H), 4.52 - 4.41 (m, 2H), 3.79 - 3.68 (m, 2H), 3.66 - 3.61 (m, 1H), 3.58 - 3.54 (m, 1H), 3.50 - 3.43 (m, 2H). |
| 197 | $^1$H NMR (400MHz, DMSO-$d_6$) δ/ppm: 12.81 (s, 1H), 8.32 - 8.27 (m, 1H), 7.99 - 7.96 (m, 1H), 7.94 - 7.84 (m, 3H), 7.43 (s, 0.5H), 7.25 (s, 0.5H), 6.60 - 6.24 (m, 1H), 6.10 (tt, 1H), 5.08 (s, 2H), 4.59 (s, 2H), 4.54 - 4.38 (m, 2H), 4.02 - 3.90 (m, 1H), 3.78 - 3.68 (m, 1H), 3.18 (s, 3H). |
| 201 | $^1$H NMR (400MHz, DMSO-$d_6$) δ/ppm: 12.82 (s, 1H), 8.31 - 8.27 (m, 1H), 7.99 - 7.96 (m, 1H), 7.94 - 7.84 (m, 3H), 7.43 (s, 0.5H), 7.25 (s, 0.5H), 6.43 (tt, 1H), 5.00 - 4.81 (m, 3H), 4.61 -4.55 (m, 2H), 4.54 - 4.38 (m, 2H), 3.68 - 3.61 (m, 1H), 3.49 - 3.34 (m, 3H), 1.90 - 1.68 (m, 2H), 1.31 (d, 3H). |
| 203 | $^1$H NMR d (ppm)(DMSO-$d_6$): 12.70 (2 H?, broad s), 7.93 (1 H, broad s), 7.70 (1 H, s), 7.34 (1 H, broad s), 6.44 (2 H, tt), 4.55 (2 H, s), 4.18 (2 H, s), 2.43 (4 H, m), 1.63 (4 H, m), 1.24 (9 H, s). |
| 205 | $^1$H NMR (400MHz, DMSO-$d_6$) δ/ppm: 8.60 (s, 1H), 8.33 (s, 1H), 8.31 - 8.27 (m, 1H), 8.05 - 8.01 (m, 1H), 7.95 - 7.85 (m, 2H), 7.44 (s, 1H), 6.46 (tt, 1H), 6.06 (s, 2H), 5.10 (s, 2H), 4.48 - 4.38 (m, 2H), 3.68 - 3.64 (m, 2H), 3.61 - 3.55 (m, 4H), 3.48 - 3.43 (m, 2H). |
| 206 | $^1$H NMR (500 MHz, DMSO-$d_6$) δ/ppm: 8.56 (s, 1H), 8.32 (s, 1H), 8.26 - 8.30 (m, 1H), 8.00 - 8.05 (m, 1H), 7.89 - 7.95 (m, 1H), 7.84 - 7.89 (m, 1H), 7.43 (s, 1H), 6.46 (tt, 1H), 6.05 (s, 2H), 4.88 - 5.03 (m, 2H), 4.42 (td, 2H), 3.80 - 3.88 (m, 1H), 3.70 - 3.77 (m, 1H), 3.57 - 3.63 (m, 1H), 3.47 - 3.56 (m, 1H), 3.18 - 3.30 (m, 2H), 2.97 - 3.03 (m, 2H), 2.72 - 2.79 (m, 1H), 2.58 - 2.68 (m, 1H), 2.19 (s, 6H), 2.16 (s, 6H), 2.07 - 2.16 (m, 1H), 1.97 - 2.06 (m, 1H), 1.73 - 1.85 (m, 1H), 1.59 - 1.71 (m, 1H). |
| 207 | $^1$H NMR (500 MHz, DMSO-$d_6$) δ/ppm: 8.56 (s, 1H), 8.31 (s, 1H), 8.31 (s, 1H), 8.28 (d, 1H), 8.00 - 8.05 (m, 1H), 7.89 - 7.94 (m, 1H), 7.84 - 7.89 (m, 1H), 7.43 (s, 1H), 6.46 (tt, 1H), 6.05 (s, 2H), 4.89 - 5.03 (m, 2H), 4.42 (td, 2H), 3.81 - 3.90 (m, 1H), 3.71 - 3.80 (m, 1H), 3.59 - 3.67 (m, 1H), 3.48 - 3.58 (m, 2H),3.18 - 3.28 (m, 2H), 3.00 - 3.12 (m, 1H), 2.70 - 2.94 (m, 2H), 2.25 (s, 6H), 2.10 - 2.19 (m, 1H), 2.00 - 2.09 (m, 1H), 1.78 - 1.90 (m, 1H),1.62 - 1.75 (m, 1H). |
| 208 | $^1$H NMR (500 MHz, DMSO-$d_6$) δ/ppm: 8.56 (s, 1H), 8.31 (s, 1H), 8.27 - 8.30 (m, 1H), 8.12 (d, 1H), 8.00 - 8.04 (m, 1H), 7.88 - 7.93 (m, 1H), 7.83 - 7.88 (m, 1H), 7.42 (s, 1H), 6.45 (tt, 1H), 6.05 (s, 2H), 4.72 (s, 2H), 4.42 (td, 2H), 4.00 (sext, 1H), 1.76 - 1.85 (m, 2H), 1.59 - 1.69 (m, 2H), 1.45 - 1.56 (m, 2H), 1.34 - 1.43 (m, 2H). |
| 209 | $^1$H NMR (500 MHz, DMSO-$d_6$) δ/ppm: 8.49 (s, 1H), 8.26 - 8.30 (m, 1H), 8.25 (s, 1H), 7.99 - 8.04 (m, 1H), 7.88 - 7.93 (m, 1H), 7.83 - 7.88 (m, 1H), 7.26 (s, 1H), 6.03 (s, 2H), 5.09 (s, 2H), 3.91 (d, 2H), 3.63 - 3.69 (m, 2H), 3.53 - 3.62 (m, 4H), 3.41 - 3.49 (m, 2H), 1.24 - 1.33 (m, 1H), 0.55 - 0.63 (m, 2H), 0.32 - 0.40 (m, 2H). |
| 212 | $^1$H NMR (400MHz, DMSO-$d_6$ ppm) δ 8.3 (1H, d), 8.0 (1H, m), 7.9-7.8 (3H, m), 7.3 (1H, t), 6.6-6.3 (1H, m), 5.9-5.7 (2H, m), 5.1 (2H, dt), 4.5 (4H, ddd), 4.1-3.6 (5H, m), 1.9-1.5 (2H, m) |

(continued)

| Cpd# | NMR |
|---|---|
| 215 | $^1H$ NMR (400MHz, DMSO-$d_6$ ppm) δ 8.3 (1H, dd), 8.0-7.8 (3H, m), 7.4-7.3 (1H, m), 6.6-6.3 (1H, m), 5.7 (1H, d), 4.8 (2H, d), 4.6-4.4 (3H, m), 4.5-4.4 (2H, m), 4.0-3.9 (2H, m), 3.7-3.6 (3H, m), 1.2-1.1 (1H, m), 0.4-0.3 (4H, m) |
| 217 | $^1H$ NMR (500 MHz, DMSO-$d_6$) δ/ppm: 12.47 (b.s., 1H), 8.11 (m, 1H), 7.78 (m, 1H), 7.70 (m, 2H), 7.44 (m, 1H), 7.15 (m, 1H), 5.87 (s, 2H), 5.17 (m, 2H), 4.00 (m, 1H), 3.91 (m, 1H), 3.87 (m, 2H), 3.74 (m, 1H), 3.68(m, 2H), 3.46 (m, 1H), 3.37 (m, 1H), 3.26 (m, 1H), 1.23 (m, 1H), 0.55 (m, 2H), 0.34 (m, 2H). |
| 242 | $^1H$ NMR d (ppm)(DMSO-$d_6$): 8.29 ((1 H, dd), 7.99 (1 H, dd), 7.94-7.85 (2 H, m), 7.52 (1 H, d), 7.13 (1 H, d), 4.90 (2 H, s), 4.55 (2 H, s), 3.87 (2 H, d), 1.30-1.19 (4H, m), 0.95-0.79 (1 H, m), 0.59-0.52 (2 H, m), 0.36-0.32 (2 H, m). |
| 243 | $^1H$ NMR (400MHz, DMSO-$d_6$) δ/ppm: 12.22 (br s, 1H), 8.38 (s, 1H), 8.29 - 8.27 (m, 1H), 8.05 - 8.01 (m, 1H), 7.95 - 7.85 (m, 2H), 7.73 (s, 1H), 7.17 (s, 1H), 5.96 (s, 2H), 4.93 (s, 2H), 3.86 (d, 2H), 3.26 (s, 3H), 1.31 - 1.21 (m, 1H), 0.61 - 0.55 (m, 2H), 0.37 - 0.33 (m, 2H). |
| 244 | $^1H$ NMR (400MHz, DMSO-$d_6$) δ/ppm: 12.15 (br s, 1H), 8.38 (s, 1H), 8.31 - 8.27 (m, 1H), 8.05 - 8.01 (m, 1H), 7.94 - 7.84 (m, 2H), 7.73 (s, 1H), 7.17 (s, 1H), 5.96 (s, 2H), 4.94 (s, 2H), 3.86 (d, 2H), 2.98 - 2.91 (m, 1H), 1.31 - 1.21 (m, 1H), 1.13 - 1.08 (m, 4H), 0.61 - 0.55 (m, 2H), 0.37 - 0.33 (m, 2H). |
| 245 | $^1H$ NMR (400MHz, DMSO-$d_6$) δ/ppm: 8.64 (s, 1H), 8.44 - 8.41 (m, 1H), 8.36 - 8.34 (s, 1H), 8.21 (dd, 1H), 7.58 (s, 1H), 7.33 (s, 1H), 6.43 (tt, 1H), 4.62 (s, 2H), 4.57 (s, 2H), 4.41 - 4.32 (m, 2H), 2.72 (s, 3H). |
| 246 | $^1H$ NMR (500 MHz, DMSO-$d_6$) δ/ppm: 8.32 (dd, 1H), 8.13, (d, 1H), 8.0-7.9 (m, 1H), 7.9-7.85 (m, 1H), 7.56 (s, 1H), 7.19 (s, 1H), 7.16 (s, 1H), 7.07 (s, 1H), 6.94 (s, 1H), 5.41 (q, 1H), 4.64+4.54 (d+d, 2H), 3.89 (d, 2H), 1.50 (d, 3H), 1.25 (m, 2H), 0.58 (m, 2H), 0.36 (m, 2H). |
| 247 | $^1H$ NMR d (ppm)(DMSO-$d_6$): 8.28 (1 H, dd), 8.19 (1 H, s), 7.98 (2 H, m), 7.95-7.79 (1 H, m), 7.30 (1 H, d), 7.18 (1 H, m), 4.59 (2 H, s), 4.51 (2 H, s), 4.05 (2 H, q), 3.70 (0.3 H, s), 3.17 (0.7 H, s), 2.75 (3 H, s) 1.33 (3 H, s). |
| 248 | $^1H$ NMR d (ppm)(DMSO-$d_6$): 8.29 (1 H, dd), 8.00 (1 H, dd), 7.93-7.84 (2 H, m), 7.35 (1 H, d), 7.29 (1 H, d), 6.40 or 6.54-6.25 (1 H, dt), 4.87 (2 H, s), 4.55 (2 H, s), 4.35 (2 H, dt), 3.20 (3 H, s). |
| 249 | $^1H$ NMR d (ppm)(DMSO-$d_6$): 8.29 (1 H, dd), 7.98 (1 H, dd), 7.91-7.83 (2 H, m), 7.55 (1 H, s), 7.19 (1 H, s), 4.78 (2 H, s), 4.55 (2 H, s), 4.05 (2 H, s), 3.06 (3 H, s), 1.38-1.35 (2 H, m), 1.19-1.14 (3 H, m). |
| 250 | $^1H$ NMR d (ppm)(DMSO-$d_6$): 8.29 (1 H, dd), 7.98 (1 H, dd), 7.92-7.83 (2 H, m), 7.51 (1 H, s), 7.11 (1 H, s), 4.81 (2 H, s), 4.54 (2 H, s), 3.79 (2 H, s), 3.11 (3 H, s), 1.21 (3 H, s), 0.55-0.52 (2 H, m), 0.40-0.37 (2 H, m). |
| 251 | $^1H$ NMR (400MHz, DMSO-$d_6$) δ/ppm: 13.1 (br s, 1H), 8.39 (s, 1H), 8.28 - 8.25 (m, 1H), 7.99- 7.95 (m, 1H), 7.88 - 7.78 (m, 2H), 7.75 (s, 1H), 7.13 (s, 1H), 5.93 (s, 2H), 4.57 (s, 2H), 4.04 (q, 2H), 1.36 (t, 3H). |
| 252 | $^1H$ NMR (400MHz, DMSO-$d_6$) δ/ppm: 13.1 (br s, 1H), 8.51 (s, 1H), 8.28 - 8.25 (m, 1H), 7.98- 7.95 (m, 1H), 7.88 - 7.78 (m, 3H), 7.40 (s, 1H), 5.95 (s, 2H), 4.80 (q, 2H), 4.52 (s, 2H). |
| 253 | $^1H$ NMR (400MHz, DMSO-$d_6$) δ/ppm: 13.1 (br s, 1H), 8.44 (s, 1H), 8.28 - 8.25 (m, 1H), 7.98- 7.95 (m, 1H), 7.88 - 7.78 (m, 2H), 7.77 (s, 1H), 7.40 (s, 1H), 6.58 - 6.28 (m, 1H), 5.95 (s, 2H), 4.61 (s, 2H), 4.35 (dt, 2H) |
| 255 | $^1H$ NMR (400MHz, DMSO-$d_6$) δ/ppm: 13.3 (br s, 1H), 8.39 (s, 1H), 8.29 - 8.26 (m, 1H), 8.03 - 7.99 (m, 1H), 7.90 - 7.80 (m, 2H), 7.79 (s, 1H), 7.08 (s, 1H), 5.91 (s, 2H), 4.68 (s, 2H), 4.07 (q, 2H), 1.35 (t, 3H). |
| 256 | $^1H$ NMR (300 MHz, DMSO-$d_6$) δ/ppm 8.58 (s, 1H), 8.27 (d, J=7.77 Hz, 1H), 8.26 (s, 1H), 8.02 (d, J=7.77 Hz, 1H), 7.79-7.93 (m, 3H), 6.46 (tt, J=54.36 Hz, J=3.06 Hz, 1H), 5.07 (s, 2H), 4.47 (s, 2H), J=3.06 Hz, 2H), 3.49 (dt, J=14.51 Hz), 3.40-3.72 (m, 8H). |

(continued)

| Cpd# | NMR |
|---|---|
| 257 | $^1H$ NMR d (ppm)(DMSO-$d_6$): 10.21 (1 H, broad s), 9.45 (1 H, broad s), 8.29 (1 H, dd), 8.05 and 8.005 (1 H, m), 8.01-7.87(3 H, m), 4.76 (1 H, d), 4.54 (1 H, s), 4.17-4.06 (3 H, m), 2.00 (1 H, s), 1.32-1.22 (1 H, m), 0.57-0.52 (2 H, m), 0.36-0.31 (2 H, m). |

## BIOLOGICAL EXAMPLES

### Example 2. In vitro assays

#### *2.1. $Ca^{2+}$ assay*

Triggering of S1PR2 by administration of Sphingosine-1-phosphate leads to a transient increase in intracellular $Ca^{2+}$. The $Ca^{2+}$ flux assays are measuring the release of $Ca^{2+}$ intracellularly by use of a $Ca^{2+}$ sensitive fluorescent dye. The assay is firstly run in agonist mode (incubation of compounds alone) to ensure that the $Ca^{2+}$ released measured is not caused by the test compound having an agonistic effect. Then the assay is continued in antagonist mode (Sphingosine-1-Phosphate added to incubated medium containing the test compounds).

*2.1.1. S1PR2 agonist assay*

CHO cells stably overexpressing human GPCR sphingosine 1-phosphate receptor 2 (CHO-S1PR2 Perkin Elmer; ES-594-A) are seeded from a frozen stock in 384 wells sterile microplates (50 μL; 7,500 cells/well) and are incubated overnight at 37°C and 5% $CO_2$. The next day cells are washed twice with starvation medium (F-12 Ham's medium containing 0.1% BSA (Fatty acid free: FAF)) and left in 25μL starvation medium for 1 h at 37°C, 5% $CO_2$. After this starvation cells are incubated with 25μL buffer containing the $Ca^{2+}$-sensitive fluorescent dye (0.5mg Fluo8 + 125mg Allura Red in 100 mL of 1% DMSO, in HBSS+20 mM Hepes + 5mM probenecid). The cells are incubated for an additional 1 h after which 10 μL compounds, diluted in HBSS buffer with 20mM Hepes and 0.1% BSA (FAF), are added to the cells and intracellular $Ca^{2+}$ changes are immediately measured by reading fluorescence during 3 min (FDSS/μCELL reader). The ratio of the maximal fluorescence over the background fluorescence before compound injection is used to determine compound response.

*2.1.2. S1PR2 antagonist assay*

After readout of the agonist activity, the plates are incubated for 15 min at 37°C and 5% $CO_2$. Then, cells are stimulated with 10 μL Sphingosine-1-Phosphate (S1P) (Avanti Polar lipids-860492P) at its EC80 concentration. Intracellular $Ca^{2+}$ changes are immediately measured by reading fluorescence during 3 min (FDSS/μCELL reader). The ratio of the maximal fluorescence over the background fluorescence before compound injection is used to determine compound response.

For $EC_{50}$ determination, a 10 point dilution series of compounds starting from 23.3μM and 20μM highest final concentration for agonist and antagonist respectively, 1/3 dilution was performed.

The obtained ratio's for agonist and antagonist readout were normalized versus vehicle and EC100 of S1P as controls for agonist mode and versus vehicle and $EC_{80}$ of S1P for antagonist mode. From these normalized data $EC_{50}$ of the compounds are derived.

**Table V. S1P2 antagonist $EC_{50}$ of illustrative compounds of the invention**

| Cpd# | S1P2 $EC_{50}$ (nM) | Cpd# | S1P2 $EC_{50}$ (nM) | Cpd# | S1P2 $EC_{50}$ (nM) |
|---|---|---|---|---|---|
| 1 | 6610 | 31 | 9.11 | 64 | 136.7 |
| 2 | 81.63 | 32 | 19.33 | 65 | 2079 |
| 3 | 8.98 | 33 | 86.51 | 66 | 18.86 |
| 4 | 7.39 | 34 | 4337 | 67 | 37.4 |
| 5 | 3.48 | 35 | 6640 | 68 | 547 |
| 6 | 18.44 | 36 | 19900 | 69 | 238.4 |
| 7 | 8.26 | 38 | 1278 | 70 | 836.3 |
| 8 | 10699 | 39 | 19900 | 71 | 356.1 |
| 9 | 39.53 | 40 | 86.43 | 72 | 6610 |
| 10 | 4.55 | 41 | 1045 | 73 | 1327 |
| 11 | 80.04 | 42 | 19.66 | 74 | 2656 |
| 12 | 276.1 | 43 | 6640 | 75 | 3855 |
| 13 | 120.1 | 44 | 2089 | 76 | 161.4 |
| 14 | 615.3 | 47 | 2239 | 77 | 29.84 |
| 15 | 5.57 | 48 | 98.4 | 78 | 6.16 |
| 16 | 4.58 | 49 | 32.84 | 79 | 82.67 |
| 17 | 3.45 | 50 | 195.4 | 80 | 42.26 |
| 18 | 301.4 | 51 | 77.28 | 81 | 22.62 |
| 19 | 3.22 | 52 | 11.63 | 82 | 9.6 |
| 20 | 29.44 | 53 | 2365 | 83 | 12.69 |
| 21 | 5.4 | 54 | 23.76 | 84 | 41.94 |
| 22 | 19900 | 55 | 1464 | 85 | 37.36 |
| 23 | 14.59 | 56 | 838.9 | 86 | 6.33 |
| 24 | 6640 | 57 | 163.3 | 87 | 148.6 |
| 25 | 217.7 | 58 | 890.4 | 88 | 7.83 |
| 26 | 888.7 | 59 | 94.36 | 89 | 205.1 |
| 27 | 868 | 61 | 677.4 | 90 | 5.29 |
| 28 | 1491 | 62 | 968.3 | 91 | 22.19 |
| 30 | 9.47 | 63 | 4157 | 92 | 8.99 |

| Cpd# | S1P2 $EC_{50}$ (nM) |
|---|---|
| 93 | 8.99 |
| 94 | 23.43 |
| 95 | 19.41 |
| 96 | 16.52 |
| 97 | 53.38 |
| 98 | 5.61 |
| 99 | 3.56 |
| 100 | 3.45 |
| 101 | 3.48 |
| 102 | 3.02 |
| 103 | 4.59 |
| 104 | 3.02 |
| 105 | 3.02 |
| 106 | 3.45 |
| 107 | 3.49 |
| 108 | 2.94 |
| 109 | 2.32 |
| 110 | 2.17 |
| 111 | 15.21 |
| 112 | 34.08 |
| 113 | 23.73 |
| 114 | 11.29 |
| 115 | 11.9 |
| 116 | 25.18 |
| 117 | 8.18 |
| 118 | 12.96 |
| 119 | 6.58 |
| 120 | 57.04 |
| 121 | 6.23 |
| 122 | 13.62 |
| 123 | 6.78 |
| 124 | 18.5 |
| 125 | 24.35 |
| 126 | 19.13 |
| 127 | 8.27 |

| Cpd# | S1P2 $EC_{50}$ (nM) |
|---|---|
| 128 | 28.29 |
| 129 | 79.3 |
| 130 | 63.82 |
| 131 | 13.21 |
| 132 | 26.42 |
| 133 | 14.81 |
| 134 | 10 |
| 135 | 37.46 |
| 136 | 60.63 |
| 137 | 20.99 |
| 138 | 45 |
| 139 | 118.7 |
| 140 | 12.95 |
| 141 | 21.07 |
| 142 | 30.11 |
| 143 | 13.78 |
| 144 | 191.1 |
| 145 | 42.64 |
| 146 | 15.27 |
| 147 | 1375 |
| 148 | 41.55 |
| 149 | 30.39 |
| 150 | 13.72 |
| 151 | 13.64 |
| 152 | 19.05 |
| 153 | 28.53 |
| 154 | 27.27 |
| 155 | 14.75 |
| 156 | 59.79 |
| 157 | 105.7 |
| 158 | 3.98 |
| 159 | 9.25 |
| 160 | 47.93 |
| 161 | 15.59 |
| 162 | 9.07 |

| Cpd# | S1P2 $EC_{50}$ (nM) |
|---|---|
| 163 | 11.56 |
| 164 | 31.91 |
| 165 | 11.5 |
| 166 | 9.34 |
| 167 | 13.23 |
| 168 | 11.94 |
| 169 | 19.86 |
| 170 | 42.5 |
| 171 | 29.96 |
| 172 | 7.57 |
| 173 | 16.04 |
| 174 | 34.89 |
| 175 | 11.42 |
| 176 | 488.9 |
| 177 | 57.15 |
| 178 | 118.3 |
| 179 | 107.1 |
| 180 | 21.08 |
| 181 | 95.83 |
| 182 | 17.28 |
| 183 | 76.04 |
| 184 | 25.25 |
| 185 | 30.43 |
| 186 | 25.04 |
| 187 | 50.16 |
| 188 | 63.35 |
| 189 | 143.3 |
| 190 | 15.02 |
| 191 | 11.76 |
| 192 | 40.95 |
| 193 | 36.52 |
| 194 | 41.86 |
| 195 | 31.88 |
| 196 | 15.68 |
| 197 | 32.53 |

| Cpd# | S1P2 $EC_{50}$ (nM) | Cpd# | S1P2 $EC_{50}$ (nM) | Cpd# | S1P2 $EC_{50}$ (nM) |
|---|---|---|---|---|---|
| 198 | 416.3 | 218 | 1406 | 238 | 6610 |
| 199 | 54.6 | 219 | 32.43 | 239 | 781.4 |
| 200 | 8.77 | 220 | 52.64 | 240 | 344.6 |
| 201 | 44.8 | 221 | 45.84 | 241 | 47.19 |
| 202 | 6.86 | 222 | 26.11 | 242 | 19.16 |
| 203 | 147.7 | 223 | 68.75 | 243 | 9 |
| 204 | 117.3 | 224 | 19.55 | 244 | 8.29 |
| 205 | 13205 | 225 | 388.3 | 245 | 14.32 |
| 206 | 19800 | 226 | 66.2 | 246 | 13.65 |
| 207 | 19800 | 227 | 14.54 | 247 | 87.92 |
| 208 | 10769 | 228 | 21.12 | 248 | 89.59 |
| 209 | 194.5 | 229 | 119.9 | 249 | 10.23 |
| 210 | 102.2 | 230 | 407.6 | 250 | 9.39 |
| 211 | 39.74 | 231 | 77.87 | 251 | 178.9 |
| 212 | 19.21 | 232 | 16.27 | 252 | 62.56 |
| 213 | 41.36 | 233 | 16.83 | 253 | 184.1 |
| 214 | 34.59 | 234 | 13.03 | 254 | 19900 |
| 215 | 120.1 | 235 | 18.21 | 255 | 6640 |
| 216 | 12.97 | 236 | 957.6 | 256 | 11593 |
| 217 | 2.23 | 237 | 55.76 | 257 | 24.31 |

*2.1.3. S1PR1 and S1PR5 Selectivity assays*

*2.1.3.1. Overview*

To evaluate the selectivity of the compounds for S1PR2 over S1PR1 and S1PR5, a similar assay set up as described above for the S1PR2 calcium flux assays was performed: CHO cells stably overexpressing human GPCR sphingosine 1-phosphate receptor 1 and Gq protein (Euroscreen, FAST-0197A) or overexpressing human GPCR sphingosine 1-phosphate receptor 5 and Gq protein were used (Perkin Elmer, ES-593-A). In case of the S1PR5 assay triggering was done with an S1PR5 specific agonist instead of with S1P (Hobson et al., 2015).

*2.1.3.2. Protocol*

50 µL of Chinese hamster ovary (CHO) cells stably overexpressing human S1PR1 or S1PR5 are seeded in 384 well sterile microplates (7,500 cells/well or 10,000 cells/well respectively) and are incubated overnight at 37°C under 5% CO2. Cells are subsequently washed twice with 25 µL/well of starvation medium (F-12 Ham's medium containing 0.1% fatty acid free bovine serum albumin (FAF BSA)). After one h starvation at 37°C, 5% CO2, cells are loaded with 25 µL of Fluo 8 dye diluted in Hank's balanced salt solution (HBSS) with 20 mM Hepes complemented with 5 mM of probenecid following the recommendations of the manufacturer (one h at 37°C, 5% CO2). Dilution series of compounds are prepared in HBSS buffer with 20 mM Hepes and 0.1% BSA (FAF). 10 µL of diluted compound is added to the cells

using the FDSS/μCELL and intracellular calcium changes are measured immediately by recording fluorescence during 3 min (agonist readout). The ratio of maximal signal over background before compound injection is used to determine compound response.

After an incubation of 15 min at 37°C, following the agonist readout, the plates are transferred to the FDSS/μCELL reader. Cells are stimulated with 10 μL of EC80 concentration of S1P (6 nM for S1P1 and 20 nM for S1P5) and intracellular calcium changes are measured immediately by recording fluorescence during three min (antagonist readout). The ratio of maximal signal over background before S1P injection was used to determine the inhibitory effect of the compound.

*2.1.3.3. Calculations & statistics*

Raw data obtained in each experiment (ratio of maximal signal over background for calcium mobilization assays) were normalized in percentage of activation (agonist mode) or inhibition (antagonist mode) using positive and negative experiment controls. Each individual plate quality was assessed by evaluation of Z' factor calculated with raw data using the following equation

$$Z' = 1-(3* \sigma^2p+3*\sigma^2n )|\mu_p-\mu_n|$$

Compound $EC_{50}$ were estimated based on percentages of activation/inhibition with Galapagos designed application using the four parameters fitting equation below:

$Y = bottom + (top - bottom)/(1+(10^\wedge(LogIC_{50}-X)*Hillslope))$ where X is the logarithm of the concentration of the compound

*2.1.3.4. Results*

Following the protocol above, the following activities are measured

**Table VI. S1P1 antagonist $EC_{50}$ of illustrative compounds of the invention**

| Cpd # | S1P1 $EC_{50}$ (nM) | Cpd # | S1P1 $EC_{50}$ (nM) | Cpd # | S1P1 $EC_{50}$ (nM) |
|---|---|---|---|---|---|
| 1 | 19800 | 19 | 19850 | 59 | 19800 |
| 3 | 6610 | 20 | 19800 | 64 | 19800 |
| 4 | 19800 | 21 | 6610 | 65 | 19800 |
| 7 | 19800 | 23 | 19800 | 67 | 19800 |
| 8 | 19800 | 25 | 4712 | 68 | 19800 |
| 9 | 6610 | 32 | 19800 | 69 | 19800 |
| 10 | 19800 | 42 | 19800 | 70 | 19800 |
| 11 | 19800 | 49 | 19800 | 71 | 19800 |
| 12 | 19800 | 50 | 19800 | 72 | 19800 |
| 13 | 19800 | 51 | 19800 | 76 | 19800 |
| 15 | 19800 | 52 | 19800 | 77 | 6610 |
| 17 | 8808 | 56 | 19800 | 79 | 19800 |
| 18 | 19800 | 57 | 19800 | 80 | 19800 |

| Cpd # | S1P1 $EC_{50}$ (nM) |
|---|---|
| 81 | 19800 |
| 82 | 19800 |
| 83 | 19800 |
| 84 | 19800 |
| 85 | 19800 |
| 86 | 6610 |
| 87 | 19800 |
| 88 | 19800 |
| 89 | 19800 |
| 90 | 4577 |
| 91 | 19800 |
| 92 | 13205 |
| 93 | 19800 |
| 94 | 19800 |
| 95 | 19800 |
| 96 | 19800 |
| 97 | 19800 |
| 98 | 19800 |
| 99 | 6610 |
| 100 | 6610 |
| 101 | 19800 |
| 102 | 19800 |
| 106 | 829.6 |
| 107 | 2152 |
| 108 | 3082 |
| 111 | 6640 |
| 112 | 19900 |
| 113 | 19900 |
| 114 | 19900 |
| 115 | 3624 |
| 116 | 19800 |
| 117 | 1535 |

| Cpd # | S1P1 $EC_{50}$ (nM) |
|---|---|
| 118 | 19800 |
| 119 | 19800 |
| 120 | 19800 |
| 122 | 3724 |
| 123 | 1843 |
| 124 | 13205 |
| 125 | 19800 |
| 126 | 19800 |
| 127 | 3498 |
| 128 | 19800 |
| 134 | 12020 |
| 135 | 19800 |
| 136 | 19900 |
| 137 | 19900 |
| 140 | 19900 |
| 141 | 19900 |
| 142 | 19900 |
| 143 | 6640 |
| 144 | 19800 |
| 145 | 19900 |
| 146 | 19900 |
| 148 | 19900 |
| 149 | 19800 |
| 150 | 6610 |
| 152 | 19800 |
| 153 | 19800 |
| 154 | 19800 |
| 156 | 19800 |
| 159 | 19800 |
| 161 | 19800 |
| 164 | 19800 |
| 167 | 19800 |

| Cpd # | S1P1 $EC_{50}$ (nM) |
|---|---|
| 168 | 19800 |
| 169 | 19800 |
| 170 | 19800 |
| 171 | 19800 |
| 172 | 19800 |
| 174 | 19800 |
| 175 | 19800 |
| 176 | 19800 |
| 177 | 19800 |
| 178 | 19800 |
| 179 | 19800 |
| 198 | 19800 |
| 201 | 13205 |
| 204 | 19800 |
| 208 | 19800 |
| 209 | 19800 |
| 211 | 19800 |
| 212 | 19800 |
| 213 | 19800 |
| 214 | 19800 |
| 216 | 19800 |
| 217 | 697.6 |
| 218 | 19800 |
| 227 | 19800 |
| 234 | 19800 |
| 242 | 559.6 |
| 243 | 19800 |
| 244 | 19800 |
| 251 | 19900 |
| 252 | 19900 |
| 253 | 19900 |
| 257 | 6610 |

**Table VII. S1P5 antagonist $EC_{50}$ of illustrative compounds of the invention**

| Cpd # | S1P5 $EC_{50}$ (nM) | Cpd # | S1P5 $EC_{50}$ (nM) | Cpd # | S1P5 $EC_{50}$ (nM) |
|---|---|---|---|---|---|
| 1 | 19800 | 15 | 19800 | 51 | 19800 |
| 3 | 19800 | 17 | 19800 | 52 | 19800 |
| 4 | 19800 | 18 | 19800 | 56 | 19800 |
| 5 | 1617 | 19 | 19850 | 57 | 19800 |
| 6 | 19900 | 20 | 19800 | 59 | 19800 |
| 7 | 19800 | 21 | 19800 | 64 | 19800 |
| 8 | 19800 | 23 | 19800 | 65 | 19800 |
| 9 | 6610 | 25 | 4792 | 67 | 19800 |
| 10 | 19800 | 32 | 6610 | 68 | 19800 |
| 11 | 19800 | 42 | 19800 | 69 | 19800 |
| 12 | 19800 | 49 | 19800 | 70 | 19800 |
| 13 | 19800 | 50 | 19800 | 71 | 19800 |

| Cpd # | S1P5 $EC_{50}$ (nM) |
|---|---|
| 76 | 19800 |
| 77 | 6610 |
| 79 | 19800 |
| 80 | 19800 |
| 81 | 19800 |
| 82 | 4383 |
| 83 | 19800 |
| 84 | 19800 |
| 85 | 19800 |
| 86 | 19800 |
| 87 | 19800 |
| 88 | 19800 |
| 89 | 6610 |
| 90 | 6610 |
| 92 | 19800 |
| 93 | 19800 |
| 94 | 19800 |
| 95 | 19800 |
| 96 | 19800 |
| 97 | 19800 |
| 98 | 19800 |
| 99 | 6610 |
| 100 | 19800 |
| 101 | 19800 |
| 102 | 19800 |
| 106 | 19800 |
| 107 | 19800 |
| 108 | 19800 |
| 110 | 2241 |
| 111 | 10000 |
| 112 | 19900 |
| 113 | 19900 |
| 114 | 627.3 |
| 115 | 19900 |

| Cpd # | S1P5 $EC_{50}$ (nM) |
|---|---|
| 116 | 19850 |
| 117 | 13205 |
| 118 | 19800 |
| 119 | 19800 |
| 120 | 19800 |
| 122 | 19800 |
| 123 | 19800 |
| 124 | 19800 |
| 125 | 19800 |
| 126 | 19800 |
| 127 | 19800 |
| 128 | 19800 |
| 134 | 19800 |
| 135 | 19800 |
| 136 | 19900 |
| 137 | 19900 |
| 140 | 19900 |
| 141 | 19900 |
| 142 | 19900 |
| 143 | 19900 |
| 144 | 19800 |
| 145 | 19900 |
| 146 | 6640 |
| 148 | 19900 |
| 149 | 19800 |
| 150 | 19800 |
| 152 | 19800 |
| 153 | 19800 |
| 154 | 19800 |
| 156 | 19800 |
| 159 | 19800 |
| 161 | 19800 |
| 164 | 19800 |
| 167 | 19800 |

| Cpd # | S1P5 $EC_{50}$ (nM) |
|---|---|
| 168 | 19800 |
| 169 | 19800 |
| 170 | 19800 |
| 171 | 19800 |
| 172 | 19800 |
| 174 | 19800 |
| 175 | 19800 |
| 176 | 19800 |
| 177 | 19800 |
| 178 | 19800 |
| 179 | 19800 |
| 198 | 19800 |
| 201 | 19800 |
| 204 | 19800 |
| 208 | 19800 |
| 209 | 19800 |
| 211 | 19800 |
| 212 | 19800 |
| 213 | 19800 |
| 214 | 19800 |
| 216 | 19800 |
| 217 | 19800 |
| 218 | 19800 |
| 227 | 19800 |
| 234 | 19800 |
| 242 | 19800 |
| 243 | 19800 |
| 244 | 19800 |
| 251 | 19900 |
| 252 | 19900 |
| 253 | 19900 |
| 257 | 19800 |

### *2.2. S1PR2 Binding assay*

The following assay can be used for determination of S1PR2 binding. The binding assay measures the potential to compete with radioactively labeled S1P for binding to the receptor.

The assay is performed in a 96 well plate where the following reagents are added. First 50 μL compound is added into the assay plate, followed by addition of 100 μL of a mixture consisting of membrane and Scintillation proximity Assay (SpA) beads [mixture consists of 20 μg/well membranes derived from stable cell line over expressing S1PR2, 0.5 mg/well Polyvinyltoluene-Wheat Germ-Agglutinin (PVT-WGA) beads (Perkin Elmer, RPNQ0001)]. All components are diluted in assay buffer containing 20mM Tris pH 7.5; 10mM $MgCl_2$; 100mM NaCl; 0.4% BSA FAF; 1 mM $Na_3VO_4$) and incubated for 15 min until addition to the assay plate. Subsequently, 50 ul of radioactively labeled S1P is added to the wells (Sphingosine, D-erythro-[3-3H] 1-phosphate; ARC; ART0778). After an incubation for 2h at room temperature, plates are centrifuged at 2000 rpm during 20 min. Plates are read on a Topcount reader (Perkin Elmer) immediately after centrifugation (readout time, 1 min/well).

*2.3. Cell based assay: GTp-γS binding assay*

The following assay can be used for determination of S1PR2 activation. The [$^{35}S$] GTPγS assay measures the level of G protein activation following agonist occupation of a GPCR, by determining the binding of the non-hydrolysable analog [$^{35}S$] GTPγS to Gα subunits.

The assay is performed in a 96 well plate where the following reagents are added. First 50 μL compound is added into the assay plate, followed by addition of 20 μL S1P at EC80 concentration (concentration which gives 80% of the activity of S1PR2). Then, 30 μL of a mixture consisting of membranes-GTPyS-SpA beads is added [mixture consists of 2.5 μg/well membranes derived from stable cell line over expressing S1PR2 (membranes are pre-incubated with 1 μM GDP for 15 min at 4°C), 0.1 nM [35S]GTPγS (Perkin Elmer, NEG030) and 0.5 mg/well PVT-WGA SpA beads (Perkin Elmer, RPNQ0001)]. All components are diluted in assay buffer containing 20mM Tris pH 7.5; 10mM $MgCl_2$; 100mM NaCl; 0.1% BSA FAF; 50ug/mL saponin. After an incubation for 4h at room temperature, plates are centrifuged at 2000 rpm during 20 min. Plates are read on a Topcount reader (Perkin Elmer) immediately after centrifugation (readout time, 1 min/well).

**Table VIII. S1PR2 binding $EC_{50}$ for illustrative compounds of the invention**

| Cpd # | S1P2 $EC_{50}$ (nM) | Cpd # | S1P2 $EC_{50}$ (nM) | Cpd # | S1P2 $EC_{50}$ (nM) |
|---|---|---|---|---|---|
| 1 | 3927 | 24 | 8447 | 55 | 4570 |
| 2 | 75.68 | 25 | 565.2 | 56 | 722.4 |
| 3 | 38.18 | 27 | 3905 | 57 | 456.9 |
| 4 | 32.21 | 28 | 33300 | 58 | 1102 |
| 5 | 22.02 | 30 | 93.33 | 59 | 41.99 |
| 6 | 134.5 | 31 | 93.19 | 61 | 581.3 |
| 7 | 50.35 | 32 | 244.7 | 62 | 453.4 |
| 8 | 2492 | 33 | 377.9 | 63 | 1268 |
| 9 | 212.4 | 34 | 5186 | 64 | 130.7 |
| 10 | 11.56 | 35 | 5405 | 65 | 1034 |
| 11 | 323.9 | 36 | 100000 | 66 | 57.09 |
| 12 | 582.2 | 38 | 1209 | 67 | 64.88 |
| 13 | 220.7 | 39 | 13430 | 68 | 274.9 |
| 14 | 4948 | 40 | 260.6 | 69 | 271.5 |
| 15 | 377.7 | 41 | 1307 | 70 | 2099 |
| 16 | 29.91 | 42 | 20.63 | 71 | 356.7 |
| 17 | 15.29 | 47 | 4525 | 72 | 3090 |
| 18 | 1225 | 49 | 92.72 | 73 | 477.9 |
| 19 | 17.9 | 50 | 267.3 | 74 | 5328 |
| 20 | 276.8 | 51 | 365.6 | 75 | 2922 |
| 21 | 30.36 | 52 | 104.5 | 76 | 317.5 |
| 23 | 305.5 | 54 | 93.53 | 77 | 241.1 |

| Cpd # | S1P2 $EC_{50}$ (nM) |
|---|---|
| 78 | 55.87 |
| 79 | 220.8 |
| 80 | 619.4 |
| 81 | 120.3 |
| 82 | 92.85 |
| 83 | 53.24 |
| 84 | 459 |
| 85 | 618.6 |
| 86 | 28.66 |
| 87 | 178.5 |
| 88 | 105.6 |
| 89 | 33.18 |
| 90 | 13.34 |
| 91 | 119.8 |
| 92 | 32.13 |
| 93 | 22.39 |
| 94 | 64.13 |
| 95 | 208.3 |
| 96 | 95.59 |
| 97 | 432.3 |
| 98 | 45.03 |
| 99 | 44.89 |
| 100 | 51.14 |
| 101 | 33.2 |
| 102 | 30 |
| 103 | 222.9 |
| 104 | 22.4 |
| 105 | 35.31 |
| 106 | 14.07 |
| 107 | 52.93 |
| 108 | 9.21 |
| 109 | 3.84 |
| 110 | 11.24 |
| 111 | 49.03 |
| 112 | 341.8 |
| 113 | 153.8 |
| 114 | 62.22 |
| 115 | 25.36 |
| 116 | 183.4 |
| 117 | 33.19 |
| 118 | 15.2 |
| 119 | 23.26 |
| 120 | 391.7 |
| 121 | 84.81 |
| 122 | 341.8 |
| 123 | 67.83 |
| 124 | 180.1 |
| 125 | 200.1 |
| 126 | 93.55 |
| 127 | 479.5 |
| 128 | 1968 |
| 129 | 1393 |
| 130 | 2499 |

| Cpd # | S1P2 $EC_{50}$ (nM) |
|---|---|
| 131 | 196.6 |
| 132 | 649.1 |
| 133 | 262.5 |
| 134 | 745 |
| 135 | 177.6 |
| 136 | 241 |
| 137 | 59.44 |
| 139 | 158.7 |
| 140 | 29.8 |
| 141 | 70.63 |
| 142 | 103.3 |
| 143 | 15.2 |
| 144 | 225.8 |
| 145 | 80.21 |
| 146 | 23.03 |
| 147 | 1089 |
| 148 | 32.07 |
| 149 | 142.2 |
| 150 | 15.61 |
| 151 | 34.68 |
| 152 | 64.04 |
| 153 | 245.5 |
| 154 | 997.4 |
| 155 | 229 |
| 156 | 647.3 |
| 157 | 617.9 |
| 158 | 18.63 |
| 159 | 30.72 |
| 161 | 154 |
| 162 | 75.13 |
| 163 | 108.7 |
| 164 | 129.1 |
| 165 | 22.06 |
| 166 | 22.38 |
| 167 | 191.2 |
| 168 | 86.43 |
| 169 | 375.4 |
| 170 | 112.4 |
| 171 | 88.54 |
| 172 | 15.82 |
| 174 | 56.84 |
| 175 | 78.93 |
| 176 | 386.3 |
| 177 | 124.1 |
| 178 | 187.8 |
| 179 | 207.4 |
| 180 | 93.94 |
| 181 | 297 |
| 182 | 53.37 |
| 183 | 307 |
| 184 | 94.34 |
| 185 | 166 |
| 186 | 140.7 |

| Cpd # | S1P2 $EC_{50}$ (nM) |
|---|---|
| 187 | 164.7 |
| 188 | 379.8 |
| 189 | 558.2 |
| 190 | 63.59 |
| 191 | 66.41 |
| 192 | 189.8 |
| 193 | 346 |
| 194 | 267.7 |
| 195 | 196.6 |
| 196 | 83.07 |
| 197 | 80.3 |
| 198 | 485.9 |
| 199 | 173 |
| 200 | 93.73 |
| 201 | 137.7 |
| 202 | 48.05 |
| 203 | 343 |
| 204 | 295.6 |
| 205 | 7682 |
| 206 | 3622 |
| 207 | 21895 |
| 208 | 1508 |
| 209 | 235.4 |
| 210 | 232.8 |
| 211 | 142.2 |
| 212 | 65.59 |
| 213 | 158.7 |
| 214 | 123.9 |
| 215 | 201.8 |
| 216 | 44.69 |
| 217 | 2.58 |
| 218 | 526.5 |
| 219 | 265 |
| 220 | 320.2 |
| 221 | 364.7 |
| 222 | 137.4 |
| 223 | 329.7 |
| 224 | 232.5 |
| 225 | 346.2 |
| 226 | 552.5 |
| 227 | 82.07 |
| 228 | 52.52 |
| 229 | 502.8 |
| 230 | 1948 |
| 231 | 453.2 |
| 232 | 121.7 |
| 233 | 209.7 |
| 234 | 269.6 |
| 235 | 116.8 |
| 236 | 2091 |
| 237 | 650.2 |
| 238 | 5238 |
| 239 | 1737 |

| Cpd # | S1P2 EC50 (nM) |
|---|---|
| 240 | 1300 |
| 241 | 371.5 |
| 242 | 299 |
| 243 | 399.5 |
| 244 | 136 |

| Cpd # | S1P2 EC50 (nM) |
|---|---|
| 245 | 609.4 |
| 246 | 775 |
| 247 | 2474 |
| 251 | 113 |
| 252 | 239 |

| Cpd # | S1P2 EC50 (nM) |
|---|---|
| 253 | 182.3 |
| 256 | 5113 |
| 257 | 231.1 |

*2.4. IL-8 production*

S1P is able to induce cytokines such as IL-8 in a process that is S1PR2 dependent (O'Sullivan et al, 2014; Bruennert et al, 2015). This assay is designed to test inhibitory activity of compounds on S1P induced IL-8 on HFL-1 cells, a human fetal lung fibroblast cell line.

*2.4.1. IL8*

Human Fetal Lung cells (HFL-1) are seeded in 96 well plates in growth medium (F12K + 10% heat inactivated FBS + 1% Pen/strep). After overnight incubation at 37°C, 5% $CO_2$ cells are refreshed with starvation medium without HSA (F12K + 1% FBS + 1% Pen/strep). On day three, compounds are added (10 point serial dilution, 30μM highest concentration, 1/3 dilution, 0.3% DMSO final) and plates are incubated for one h at 37°C, 5% $CO_2$. Subsequently S1P at 1μM final concentration is added and plates are incubated for 16 to 24 h at 37°C, 5% $CO_2$ after which the supernatant was collected. IL-8 levels in the supernatant are determined with the IL-8 ELISA of R&D systems.

**Table IX. IL8 production assay for illustrative compounds of the invention**

| Cpd# | IL8 EC50 (nM) |
|---|---|
| 1 | 3320 |
| 2 | 38.83 |
| 3 | 3.99 |
| 4 | 1.64 |
| 5 | 1.52 |
| 6 | 12.44 |
| 7 | 3.67 |
| 8 | 609.8 |
| 9 | 36.99 |
| 10 | 0.567 |
| 11 | 59.61 |
| 12 | 143 |
| 13 | 105.1 |
| 14 | 1446 |
| 15 | 24.2 |
| 16 | 8.49 |
| 17 | 3.02 |
| 18 | 210.1 |
| 19 | 1.29 |
| 20 | 133.5 |
| 21 | 2.52 |

| Cpd# | IL8 EC50 (nM) |
|---|---|
| 23 | 58.06 |
| 25 | 836.1 |
| 27 | 1110 |
| 30 | 13.71 |
| 31 | 10.98 |
| 32 | 95.74 |
| 33 | 15.64 |
| 40 | 145.2 |
| 41 | 685.2 |
| 42 | 30.94 |
| 47 | 1359 |
| 49 | 31.68 |
| 50 | 246.5 |
| 51 | 177.1 |
| 52 | 46.72 |
| 54 | 32.2 |
| 56 | 3330 |
| 57 | 551.7 |
| 59 | 98.84 |
| 61 | 591.2 |
| 62 | 349.3 |

| Cpd# | IL8 EC50 (nM) |
|---|---|
| 64 | 25.64 |
| 65 | 108.2 |
| 67 | 9.07 |
| 68 | 76.72 |
| 69 | 109.1 |
| 70 | 251.1 |
| 71 | 156.5 |
| 72 | 5175 |
| 73 | 364.9 |
| 74 | 822.6 |
| 75 | 938.2 |
| 76 | 984.5 |
| 77 | 1.63 |
| 78 | 4.65 |
| 79 | 57.67 |
| 80 | 46.8 |
| 81 | 20.03 |
| 82 | 9.96 |
| 83 | 4.69 |
| 84 | 198.4 |
| 85 | 78.9 |

| Cpd# | IL8 $EC_{50}$ (nM) |
|---|---|
| 86 | 0.911 |
| 87 | 282.9 |
| 88 | 29.75 |
| 89 | 5.7 |
| 90 | 1.11 |
| 91 | 60.66 |
| 92 | 4.41 |
| 93 | 3.04 |
| 94 | 104.9 |
| 95 | 53.37 |
| 96 | 30.02 |
| 97 | 60.52 |
| 98 | 5.69 |
| 100 | 4.97 |
| 101 | 6.74 |
| 103 | 18.89 |
| 104 | 4.55 |
| 105 | 10.63 |
| 106 | 1.86 |
| 107 | 0.986 |
| 108 | 1.54 |
| 109 | 0.137 |
| 110 | 2.37 |
| 111 | 42.17 |
| 112 | 541.2 |
| 113 | 418 |
| 114 | 32.41 |
| 115 | 5.73 |
| 116 | 77.78 |
| 117 | 0.96 |
| 118 | 25.5 |
| 119 | 11.29 |
| 120 | 96.69 |
| 121 | 6.35 |
| 122 | 2.29 |
| 123 | 1.76 |
| 124 | 66.6 |
| 125 | 66.14 |
| 126 | 5.97 |
| 127 | 13.59 |
| 128 | 30.82 |
| 131 | 11.99 |
| 132 | 97.35 |
| 133 | 33.29 |
| 134 | 10.06 |
| 135 | 24.27 |
| 136 | 25.98 |

| Cpd# | IL8 $EC_{50}$ (nM) |
|---|---|
| 137 | 104.9 |
| 139 | 5.39 |
| 140 | 114.5 |
| 141 | 130.1 |
| 142 | 191 |
| 143 | 21.59 |
| 144 | 510.6 |
| 145 | 89.27 |
| 146 | 29.47 |
| 148 | 68.93 |
| 149 | 211.4 |
| 150 | 6.14 |
| 151 | 7.11 |
| 152 | 6.65 |
| 153 | 27.38 |
| 154 | 80.98 |
| 155 | 49.48 |
| 156 | 593.1 |
| 157 | 779.5 |
| 158 | 1.5 |
| 159 | 1.02 |
| 160 | 132.7 |
| 161 | 47.35 |
| 162 | 4.44 |
| 163 | 12.64 |
| 164 | 42.65 |
| 165 | 4.65 |
| 166 | 1.41 |
| 167 | 6.61 |
| 168 | 4.57 |
| 169 | 48.69 |
| 170 | 10.55 |
| 171 | 23.08 |
| 172 | 29.15 |
| 173 | 2.4 |
| 174 | 20.1 |
| 175 | 10.95 |
| 176 | 116.2 |
| 177 | 18.61 |
| 178 | 159.7 |
| 179 | 352.1 |
| 180 | 28.29 |
| 181 | 544.3 |
| 182 | 9.66 |
| 183 | 196.1 |
| 184 | 23.77 |
| 185 | 93.65 |

| Cpd# | IL8 $EC_{50}$ (nM) |
|---|---|
| 186 | 34.78 |
| 187 | 74.5 |
| 188 | 258.4 |
| 190 | 20.14 |
| 191 | 36.65 |
| 192 | 156 |
| 193 | 49.67 |
| 194 | 186.3 |
| 195 | 27.76 |
| 196 | 27.16 |
| 197 | 139.4 |
| 198 | 74.79 |
| 199 | 80.29 |
| 200 | 10.57 |
| 201 | 63.77 |
| 202 | 13.37 |
| 203 | 1018 |
| 204 | 119.9 |
| 205 | 2641 |
| 206 | 2990 |
| 207 | 29900 |
| 208 | 9950 |
| 209 | 122.8 |
| 210 | 356 |
| 211 | 46.52 |
| 212 | 10.36 |
| 213 | 70.72 |
| 214 | 7.04 |
| 215 | 199.8 |
| 216 | 12.19 |
| 217 | 0.04 |
| 218 | 151.4 |
| 219 | 177.3 |
| 220 | 102.3 |
| 221 | 127.3 |
| 227 | 16.87 |
| 233 | 28.84 |
| 234 | 34.22 |
| 235 | 49.22 |
| 242 | 77.32 |
| 243 | 764.2 |
| 244 | 21.28 |
| 251 | 111 |
| 252 | 144.8 |
| 253 | 289.6 |
| 256 | 2659 |
| 257 | 53.92 |

*2.4.2. IL8 assay in the presence of human serum albumin*

To evaluate the influence of plasma protein binding of S1P as well as compound, the S1P induced IL-8 levels were evaluated in presence of 2% Human Serum Albumin (HSA, equivalent to 40% human serum). By adding 2% HSA,

which affects both the activity of the compounds as well as the S1P potency, the physiological condition are reproduced and the shift in potency expected under in vivo serum conditions can be measured.

Human Fetal Lung cells (HFL-1) are seeded in 96 well plates in growth medium (F12K + 10% heat inactivated FBS + 1% Pen/strep). After overnight incubation at 37°C, 5% $CO_2$ cells are refreshed with starvation medium with HSA (F12K + 1% FBS + 1% Pen/strep+1.95% HSA). On day three, compounds are added (10 point serial dilution, 30μM highest concentration, 1/3 dilution, 0.3% DMSO final) and plates are incubated for 1 h at 37°C, 5% $CO_2$. Subsequently S1P at 5 μM final concentration is added and plates are incubated for 16 to 24 h at 37°C, 5% $CO_2$ after which the supernatant was collected. IL-8 levels in the supernatant are determined with the IL-8 ELISA of R&D systems.

**Table X. IL8 production assay with HSA for illustrative compounds of the invention**

| Cpd # | HSA IL8 $EC_{50}$ (nM) | Cpd # | HSA IL8 $EC_{50}$ (nM) | Cpd # | HSA IL8 $EC_{50}$ (nM) |
|---|---|---|---|---|---|
| 1 | 15580 | 47 | 10000 | 85 | 2279 |
| 2 | 165.6 | 49 | 47.07 | 86 | 97.06 |
| 3 | 217.3 | 50 | 365.4 | 87 | 324.4 |
| 4 | 19.23 | 51 | 290.6 | 88 | 410.4 |
| 5 | 17.51 | 52 | 50.01 | 89 | 64.49 |
| 6 | 57.19 | 54 | 41.12 | 90 | 29.15 |
| 7 | 11.27 | 56 | 2928 | 91 | 5706 |
| 8 | 9950 | 57 | 501.3 | 92 | 544.5 |
| 9 | 850.2 | 59 | 180.5 | 93 | 22.81 |
| 10 | 3.17 | 61 | 1120 | 94 | 267.3 |
| 11 | 693.5 | 62 | 6430 | 95 | 1826 |
| 12 | 1095 | 64 | 33.47 | 96 | 424.7 |
| 13 | 251.3 | 65 | 91.11 | 97 | 5699 |
| 14 | 10000 | 67 | 19.18 | 98 | 26.9 |
| 15 | 342.9 | 68 | 123.1 | 99 | 322.4 |
| 16 | 51.36 | 69 | 96.28 | 100 | 63.53 |
| 17 | 210.8 | 70 | 443.1 | 101 | 154.5 |
| 18 | 22512 | 71 | 134.2 | 102 | 18.01 |
| 19 | 18.24 | 72 | 1949 | 103 | 108.1 |
| 20 | 3325 | 73 | 562.9 | 104 | 26.98 |
| 21 | 52.11 | 74 | 778.9 | 105 | 109.6 |
| 23 | 293.2 | 75 | 1302 | 106 | 98.93 |
| 25 | 10000 | 76 | 3431 | 107 | 7.56 |
| 27 | 10000 | 77 | 27.42 | 108 | 5.89 |
| 30 | 74.45 | 78 | 41.47 | 109 | 2.03 |
| 31 | 72.59 | 79 | 503.2 | 110 | 15.25 |
| 32 | 2963 | 80 | 122.4 | 111 | 1080 |
| 33 | 129.2 | 81 | 151 | 112 | 1638 |
| 40 | 291.9 | 82 | 390.5 | 113 | 917.2 |
| 41 | 2783 | 83 | 125 | 114 | 1665 |
| 42 | 442.7 | 84 | 2457 | 115 | 51.83 |

| Cpd # | HSA IL8 $EC_{50}$ (nM) |
|---|---|
| 116 | 565.7 |
| 117 | 8.83 |
| 118 | 95.18 |
| 119 | 188.7 |
| 120 | 822.3 |
| 121 | 50.82 |
| 122 | 13.35 |
| 123 | 25.91 |
| 124 | 446.4 |
| 125 | 1682 |
| 126 | 113.3 |
| 127 | 61.66 |
| 128 | 700.8 |
| 131 | 267 |
| 132 | 800 |
| 133 | 267 |
| 134 | 163.1 |
| 135 | 3462 |
| 137 | 3178 |
| 139 | 28.02 |
| 140 | 1110 |
| 142 | 952.5 |
| 143 | 179.3 |
| 144 | 3404 |
| 145 | 330.5 |
| 146 | 280.2 |
| 148 | 785.4 |
| 149 | 381 |
| 150 | 41.36 |
| 151 | 43.7 |
| 152 | 201.2 |
| 153 | 253.6 |
| 154 | 646.1 |
| 155 | 229.8 |
| 156 | 868.4 |
| 157 | 1221 |
| 158 | 10.98 |

| Cpd # | HSA IL8 $EC_{50}$ (nM) |
|---|---|
| 159 | 13.96 |
| 160 | 1054 |
| 161 | 107.8 |
| 162 | 8.27 |
| 163 | 106.6 |
| 164 | 64.91 |
| 165 | 79.91 |
| 166 | 3.3 |
| 167 | 18.42 |
| 168 | 28.11 |
| 169 | 399.5 |
| 170 | 136.9 |
| 171 | 44.9 |
| 172 | 33.4 |
| 173 | 3.47 |
| 174 | 58.94 |
| 175 | 61.64 |
| 176 | 618.3 |
| 177 | 120.4 |
| 178 | 503.8 |
| 179 | 352.6 |
| 180 | 67.04 |
| 181 | 906.1 |
| 182 | 54.69 |
| 183 | 430 |
| 184 | 82.26 |
| 185 | 301.5 |
| 186 | 80.86 |
| 187 | 80.41 |
| 188 | 662.6 |
| 190 | 37.98 |
| 191 | 99.92 |
| 192 | 666 |
| 193 | 183.3 |
| 194 | 453.2 |
| 195 | 171.4 |
| 196 | 445.1 |

| Cpd # | HSA IL8 $EC_{50}$ (nM) |
|---|---|
| 197 | 634.5 |
| 198 | 210.2 |
| 199 | 276.1 |
| 200 | 102.6 |
| 201 | 151.9 |
| 202 | 479.3 |
| 203 | 1936 |
| 204 | 546.4 |
| 205 | 9067 |
| 206 | 2634 |
| 207 | 3028 |
| 208 | 3320 |
| 209 | 381.6 |
| 210 | 187.5 |
| 211 | 159.4 |
| 212 | 64.03 |
| 213 | 177.7 |
| 214 | 33.41 |
| 215 | 112.1 |
| 216 | 16.03 |
| 217 | 1.36 |
| 218 | 15213 |
| 219 | 606.8 |
| 220 | 325.4 |
| 221 | 386.6 |
| 227 | 84.24 |
| 233 | 562.3 |
| 234 | 378 |
| 235 | 159.3 |
| 242 | 110.6 |
| 243 | 2579 |
| 244 | 807.8 |
| 251 | 3456 |
| 252 | 16503 |
| 253 | 4129 |
| 256 | 2352 |
| 257 | 216.3 |

***2.5. In vivo assays***

*2.5.1. Bleomycin induced pulmonary fibrosis in mice*

*2.5.1.1. Prophylactic bleomycin induced pulmonary fibrosis 14-day mice model*

The aim of the study is to test the efficacy of a test compound at three different doses in a 14-day model of bleomycin induced pulmonary fibrosis in mice.

*2.5.1.1.1 Animals*

This study is carried out on C527BL/6N male mice, supplied by Charles River, Italy, which are acclimatized for

at least 5 days in an environment maintained at 22°C, at 55% relative humidity, with 15-20 air changes per h under light cycles of 12 h. Mice pelleted food and water are provided ad libitum.

At least one day prior to start of experiment, all animals are allocated randomly into groups as indicated in the table below.

All animal related research is conducted in accordance with 2010/63/EU and National legislation regulating the use of laboratory animals in scientific research and for other purposes (Official Gazette 55/13).

**Table XI. Study groups**

| Groups | Purpose | n | Dose | Treatment schedule Days (Frequency) | Route | Vehicle |
|---|---|---|---|---|---|---|
| **1 PBS+Vehicle** | control | 15 | - | D0-D14 (BID) | NA | NA |
| **2 BLM+Vehicle** | control | 15 | - | D0-D14 (BID) | PO | PEG/MC |
| 3 **BLM+ Pirfenidone** | control | 15 | 50 mg/kg | D0-D14 (BID) | PO | 0.1% Natrosol |
| **4 BLM+ test compound** | Active | 15 | 1 mg/kg | D0-D14 (BID) | PO | PEG400/MC 0.5% 20/80 (v/v) |
| **5 BLM+ test compound** | Active | 15 | 3 mg/kg | D0-D14 (BID) | PO | PEG400/MC 0.5% 20/80 (v/v) |
| **6 BLM+ test compound** | Active | 15 | 10 mg/kg | D0-D14 (BID) | PO | PEG400/MC 0.5% 20/80 (v/v) |
| **7 BLM+ test compound satellite for PK** | Active | 10 | 10 mg/kg | D0-D7 (BID) | PO | PEG400/MC 0.5% 20/80 (v/v) |

*2.5.1.1.2 Materials*

The solvent for the test solutions is prepared by adding 0.5 g of hydroxyethylcellulose (Natrosol) into 500 mL Aqua distillate (0.1%) under continuous stirring without heating for 5 h on a magnetic stirrer.

Anesthetic solution is prepared by adding 1 mL of Narketan (Narketan 10, Vetoquinol, Bern, Switzerland, 03605877535982) and 0.5 mL of Rompun (Rompun, 2%: Bayer, Leverkusen, Germany) into 9 mL saline. The resulting solution is administered at 10 mL/kg.

To prepare a solution for intranasal (i.n.) challenge, 0.8 mg/mL stock solutions of bleomycin (Bleomycin sulphate, Enzo Life Sciences, Inc., USA; CAS No. 9041-93-4; Cat. No. BML-AP302-0010) are thawed and diluted in 330 μL of saline.

Prior to i.n administration, mice are anesthetized i.p. with the anesthetic solution described above.

Fresh pirfenidone formulation is prepared daily in 0.1% Natrosol formulations to a final concentration of 5 mg/mL. Before dosing, animals are weighed and the Pirfenidone amount administered is adjusted accordingly to individual weights corresponding to 10 mL/kg body weight, twice daily p.o., with 7.5 h interval between two administrations.

Finally, test compound solutions are prepared by dissolving the suitable amount of said test compound in PEG 400 (20% of the final volume) then MC 0.5% (80% of the final volume) to reach final concentrations of 1 mg/mL, 0.3 mg/mL and 0.1 mg/mL, thus yielding compound for a doses of 10 mg/kg, 3 mg/kg and 1 mg/kg. Prior to dosing, animals are weighed and the amount administered adjusted accordingly to individual weights.

The application volume of the test doses corresponds to 10 mL/kg body weight, and is the test compounds are administered p.o. twice daily, with 7.5 h interval between two administrations.

*2.5.1.1.3 Study*

Animals are examined clinically twice daily. List of clinical signs and parameters are indicated in human endpoints table. Animals are weighed daily starting from D0.

On day 14, two h post dosing with vehicle, pirfenidone or test compound, mice are sacrificed by anesthetic overdose.

The lungs are excised and weighed individually. For all groups: the whole superior right lung lobe is placed into

a Precellys tube containing silica beads and immediately snap frozen in liquid nitrogen and subjected to gene expression analysis.

All remaining lungs are placed into marked bottles containing 10% buffered formalin for further histopathological evaluation.

*2.5.1.2. Therapeutic bleomycin induced pulmonary fibrosis 21-day mice model*

The aim of the study is to test the efficacy of a test compound at three different doses in a 21-day model of bleomycin induced pulmonary fibrosis in mice.

*2.5.1.2.1 Animals*

This study is carried out on C57BL/6N male mice, supplied by Charles River, Italy, which are acclimatized for at least 5 days in an environment maintained at 22°C, at 55% relative humidity, with 15-20 air changes per h under light cycles of 12 h. Mice pelleted food and water are provided ad libitum.

At least one day prior to start of experiment, all animals are allocated randomly into groups as indicated in the table below.

All animal related research is conducted in accordance with 2010/63/EU and National legislation regulating the use of laboratory animals in scientific research and for other purposes (Official Gazette 55/13).

**Table XII. Study groups**

| Groups | Purpose | n | Dose | Treatment schedule Days (Frequency) | Route | Vehicle |
|---|---|---|---|---|---|---|
| **1 PBS+Vehicle** | control | 15 | - | D7-D21 (BID) | NA | NA |
| **2 BLM+Vehicle** | control | 15 | - | D7-D21 (BID) | PO | PEG/MC |
| **3 BLM+ Nintedanib** | control | 15 | 60 mg/kg | D7-D21 (QD) | PO | 0.1% Natrosol |
| **4 BLM+ test compound** | Active | 15 | 1 mg/kg | D7-D21 (BID) | PO | PEG400/MC 0.5% 20/80 (v/v) |
| **5 BLM+ test compound** | Active | 15 | 3 mg/kg | D7-D21 (BID) | PO | PEG400/MC 0.5% 20/80 (v/v) |
| **6 BLM+ test compound** | Active | 15 | 10 mg/kg | D7-D21 (BID) | PO | PEG400/MC 0.5% 20/80 (v/v) |
| **7 BLM+ test compound satellite for PK** | Active | 10 | 10 mg/kg | D0-D7 (BID) | PO | PEG400/MC 0.5% 20/80 (v/v) |

*2.5.1.2.2 Materials*

The solvent for the test solutions is prepared by adding 0.5 g of hydroxyethylcellulose (Natrosol) into 500 mL Aqua distillate (0.1%) under continuous stirring without heating for 5 h on a magnetic stirrer.

Anesthetic solution is prepared by adding 1 mL of Narketan (Narketan 10, Vetoquinol, Bern, Switzerland, 03605877535982) and 0.5 mL of Rompun (Rompun, 2%: Bayer, Leverkusen, Germany) into 9 mL saline. The resulting solution is administered at 10 mL/kg.

To prepare a solution for intranasal (i.n.) challenge, 0.8 mg/mL stock solutions of bleomycin (Bleomycin sulphate, Enzo Life Sciences, Inc., USA; CAS No. 9041-93-4; Cat. No. BML-AP302-0010) are thawed and diluted in 330 μL of saline.

Prior to i.n administration, mice are anesthetized i.p. with the anesthetic solution described above.

Fresh Nintedanib formulation is prepared daily in 0.1% Natrosol formulations to a final concentration of 5 mg/mL. Before dosing, animals are weighed and the Nintedanib amount administered is adjusted accordingly to individual weights corresponding to 10 mL/kg body weight, once daily p.o.,

Finally, test compound solutions are prepared by dissolving the suitable amount of said test compound in PEG

400 (20% of the final volume) then MC 0.5% (80% of the final volume) to reach final concentrations of 1 mg/mL, 0.3 mg/mL and 0.1 mg/mL, thus yielding compound for a doses of 10 mg/kg, 3 mg/kg and 1 mg/kg. Prior to dosing, animals are weighed and the amount administered adjusted accordingly to individual weights.

The application volume of the test doses corresponds to 10 mL/kg body weight, and is the test compounds are administered p.o. twice daily, with 7.5 h interval between two administrations.

*2.5.1.2.3 Study*

Animals are examined clinically twice daily. List of clinical signs and parameters are indicated in human endpoints table. Animals are weighed daily starting from D0.

On day 21, 2 h post dosing with vehicle, Nintedanib or test compound, mice are sacrificed by anesthetic overdose.

The lungs are excised and weighed individually. For all groups: the whole superior right lung lobe is placed into a Precellys tube containing silica beads and immediately snap frozen in liquid nitrogen and subjected to gene expression analysis.

All remaining lungs are placed into marked bottles containing 10% buffered formalin for further histopathological evaluation.

*2.5.1.3. Sample analysis, data processing and statistical evaluation*

Body weight data and lung weight data are processed using MS Excel. Statistical analysis and graphical presentation are performed using GraphPad Prism software (version 5.04).

One-way ANOVA or Mann-Whitney test are employed for lung weights.

Two-way ANOVA are employed for body weight changes.

Differences between groups will be considered statistically significant when $p<0.05$.

For histopathological evaluation, whole lungs (except sampled superior right lung) are embedded in paraffin and stained with Mallory's trichrome.

Pulmonary histological changes are assessed using Matsuse modification of Ashcroft score (Ashcroft et al., 1988; Matsuse et al., 1999). Statistical analysis and graphical presentation is performed using GraphPad Prism software (version 5.04). Mann-Whitney test is employed.

Differences between groups will be considered statistically significant when $p<0.05$.

| | **Ashcroft Score** |
|---|---|
| 1 | Normal lungs (no fibrosis) |
| 2 | Minimal fibrotic thickening of alveolar or bronchial walls (network of fine collagen fibrils) |
| 3 | Moderate fibrotic thickening of walls without obvious damage to lung architecture |
| 4 | Fibrosis with damage of pulmonary structure (coarse fibrous bands or small fibrous masses, intra-alveolar collagen fibrils) |
| 5 | Large fibrous area with severe distortion of lung structure |

For example, when tested in these therapeutic and prophylactic models, a statistically significant Ashcroft score difference was obtained at 10 mg/kg BID with representative compounds 17 and 115.

*2.5.1.4. PK analysis - Group 7*

*2.5.1.4.1 Protocol*

Animals in group 7 (n=10) are included for PK study only and are not be subjected to clinical sign scoring.

These animals are induced with the disease at the start of treatment at day 0 and are sequentially sacrificed on day 7 at 1 h, 3 h, 6 h, 8 h, 24 h after the first administration of test compound.

A blood sample (50 μL) is collected from the tail vein into Li-heparin anticoagulant tubes for each time point and kept on ice until separation. Within maximum 30 min after collection, blood samples are centrifuged at 2000 g for 10 min at 4°C and the resulting plasma samples are aliquoted into polypropylene tubes (1x 25 μL). The samples are stored frozen at -20°C until analysis.

The lung tissue is collected at sacrifice after blood sampling for each animal, then weighed and placed into polypropylene tubes prior to freezing. The samples are stored frozen at -80°C until analysis.

*2.5.1.4.2 Plasma concentration and pharmacokinetic analysis*

Plasma and lung concentrations are measured via LC-MS/MS. Samples are prepared for LC-MS/MS analysis via protein precipitation. The plasma concentrations measured below the lower limit of quantification (LLOQ) are reported as below the limit of quantification (BLQ).

The test compound concentrations in plasma are expressed in ng/mL.

Mean plasma concentrations are calculated. For mean calculation, the concentrations below the LLOQ are set to zero. Therefore, mean values may be BLQ. Standard deviation (SD), standard error of the mean (SE) and coefficient of variation (CV, %) are tabulated when at least three plasma concentration values are above the LLOQ.

Non-compartmental analysis on individual plasma concentrations is performed using PhoenixTM WinNonlin® 6.3 (Pharsight Corporation) to determine at least, the following pharmacokinetic parameters:

- Maximum plasma concentration, Cmax (μg/mL) with the corresponding time, tmax (h),
- Area under the plasma concentration versus time curve up to the last quantifiable concentration $AUC_{0-t}$ or up to 24 h $AUC_{0-24h}$ (μg.h/mL) (if compound is quantifiable up to 24h postdose), and/or up to infinity $AUC_{0-\infty}$, (μg.h/mL) is calculated according to the linear up/log down trapezoidal rule. Partial AUC may be calculated if deemed necessary. Concentrations below the limit of quantification (BLQ) are set to zero. No AUC is calculated if there are less than three quantifiable time points. AUCO-∞ is considered if %AUCextra < 20%,
- Apparent terminal elimination half-life, t1/2 (h) is only reported if three or more time points, excluding tmax is used for linear regression, and if the adjusted $R^2$ > 0.80.
- Normalized AUC and Cmax dose.
- Mean pharmacokinetic parameters are calculated. Standard deviation (SD) and coefficient of variation (CV, %) are tabulated if at least three values are available.

***2.6. Radiation induced fibrosis mice model***

*2.6.1. Study overview*

Pneumonitis and lung fibrosis are the major radiation-induced complications following thoracic radiotherapy, which is one of the major treatment of lung and breast cancers, lymphomas and hematopoietic transplant conditioning.

The objective of this model is to evaluate the effect of a compound of the invention in lung fibrosis induced by radiation in mice. (Bickelhaupt et al., 2017)

*2.6.2. Animals*

7 weeks old (18/22 gr) female C57BL/6J mice from Charles River (France, batch number S1672) are maintained on 12 h light/dark cycle at 22°C with *ad libidum* access to tap water and food.

*2.6.3. Materials*

The test compounds are dissolved/suspended in appropriate vehicle prior to using and the kept light-free, under agitation at room temperature.

An aliquot of the formulation (approx. 200 μL) is frozen at T0 (day of preparation) and all the formulations are checked (daily) for any change in aspect.

The dose volume administered is 10 mL/kg and the volume is adapted following mean (body weight (BW) of the group as follows: 200μL if mean BW <22.5g, 250μL if mean BW ≥22.5g; 300μL if mean BW >27.5g.

*2.6.4. In vivo experimental procedure*

On day 1 of week 1, the animals are exposed at the thorax to a 17 Gray irradiation dose, under isoflurane anesthesia.

At the beginning of week 18 post irradiation (Day 1), animals are randomized into 6 study groups (15 subjects per group) 1) sham (vehicle: methylcellulose (MC) 0.5%), 2) diseased (vehicle: methylcellulose (MC) 0.5%), 3) positive control (nintedanib dosed 60 mg/kg in 0.1% Natrosol), and 4) 3 groups test compound (1.2/3.6/12 mg/kg in 0.1% Natrosol (hydroxyethylcellulose)), and dosed p.o. q.d until Day 23 (week 21).

Body weight are recorded once a week, and on Day 23, lung function measurement under anesthesia is realized by Flexivent (Devos et al., 2017) for all groups (6 successful measurement per group) before sacrifice.

### *2.7. Murine sclerodermatous chronic graft-versus-host disease (cGvHD)*

*2.7.1. Study overview*

This inflammation driven fibrosis model reproduces the rapidly progressing diffuse cutaneous systemic scleroderma (SSc) observed in patients, and is used to evaluate the effect of the compounds of the invention on the pathology. (Chen et al., 2017)

In this model, fibrosis is induced in BALB/c (H-2d) mice by allogeneic transplantation of bone marrow cells and splenocytes from B10.D2 (H-2d) donor mice (minor HLA mismatch).

*2.7.2. Animals*

BALB/c (H-2d) mice were purchased from Janvier (Le Genest St. Isle, France).

B10.D2 (H-2d) mice were purchased from Jackson Laboratory (Bar Harbor, ME).

All mice are maintained in specific pathogen-free conditions with sterile pellet food and water and a normal day-night cycle.

*2.7.3. Study protocol*

Transplantation of tibial and femoral bone marrow cells and splenocytes is performed as follows: 8-weeks old mice (BALB/c (H-2d)) receives total body irradiation with 700 cGy. Six h after irradiation, all BALB/c (H-2d) recipients receive bone marrow from either BALB/c (H-2d) in a syngeneic or B10.D2 (H-2d) in an allogeneic transplantation manner. For transplantation, 5 x $10^6$ splenocytes and 1 x $10^6$ bone marrow cells from donor mice are resuspended in 0.2 mL of PBS and injected via tail veins.

Treatment is started 21 days after bone marrow transplantation and thus several days after the first clinically detectable manifestations of cGvHD in allogeneically transplanted mice.

The following study groups are made:

- Syngeneically transplanted, placebo-treated control group Syngeneic bone marrow and splenocyte transplantation (BALB/c (H-2d) → BALB/c (H-2d)). Application of the vehicle from day 21 to day 56 post-transplantation.
- Placebo-treated fibrosis group Allogeneic bone marrow and splenocyte transplantation (B10.D2 (H-2d) → BALB/c (H-2d)). Application of the vehicle from day 21 to day 56 post-transplantation.
- Control group to assess pretreatment change induced by allogeneic transplantation Allogeneic bone marrow and splenocyte transplantation (B10.D2 (H-2d) → BALB/c (H-2d)). Sacrifice at day 21, before treatment is initiated in the control groups.
- Treatment group 1 Allogeneic bone marrow and splenocyte transplantation (B10.D2 (H-2d) → BALB/c (H-2d)). Application of low doses of test compound (10 mg/kg/bid p.o.) from day 21 to day 56 post transplantation.
- Treatment group 2 Allogeneic bone marrow and splenocyte transplantation (B10.D2 (H-2d) → BALB/c (H-2d)). Application of high doses of test compound (30 mg/kg/ bid p.o.) from day 21 to day 56 post transplantation.
- Positive control group: Allogeneic bone marrow and splenocyte transplantation (B10.D2 (H-2d) → BALB/c (H-2d)). Application of 60 mg/kg qd nintedanib from day 21 to day 56 post transplantation.

*2.7.4. Histological evaluation of skin fibrosis*

Skin samples are fixed in 4 % formalin for 6 h and embedded in paraffin. 5 μm sections are cut and stained with hematoxylin and eosin, with Trichrome or with Sirius Red.

The dermal thickness is measured at 100-fold magnification by measuring the distance between the epidermal-dermal junction and the dermal-subcutaneous fat junction at four sites oer mouse. As for other readouts, the analyses are performed in a blinded manner.

2.7.5. *Detection of myofibroblasts*

Myofibroblasts are characterized by the expression of α-smooth muscle actin (αSMA). Fibroblasts positive for αSMA are detected by incubation with monoclonal anti-aSMA antibodies (clone 1A4, Sigma-Aldrich, Steinheim, Germany). The expression is visualized with horseradish peroxidase labeled secondary antibodies and 3,3-diaminobenzidine tetrahydrochloride (DAB) (Sigma-Aldrich). Monoclonal mouse IgG antibodies (Calbiochem, San Diego, CA, USA) are used for controls.

*2.7.6. Hydroxyproline assay*

The amount of collagen protein in skin samples is determined via hydroxyproline assay. After digestion of punch biopsies (Ø 3 mm) in 6 M HCl for three h at 120 °C, the pH of the samples is adjusted to 6 with 6 M sodium hydroxide (NaOH). Afterwards, 0.06 M chloramine T is added to each sample and incubated for 20 min at room temperature. Next, 3.15 M perchloric acid and 20 % p-dimethylaminobenzaldehyde are added and samples are incubated for additional 20 min at 60 °C. The absorbance is determined at 557 nm with a Spectra MAX 190 microplate spectrophotometer.

*2.7.7. Clinical score of cutaneous cGvHD*

Recipient mice are clinically monitored once daily from the day of transplantation to the indicated days after transplantation to determine the incidence and severity of cutaneous cGvHD as well as mobility, diarrhea and weight loss. The following scoring system for cutaneous cGvHD is used: healthy appearance = 0; skin lesions with alopecia < 1 $cm^2$ in area = 1; skin lesions with alopecia 1 - 2 $cm^2$ in area = 2; skin lesions with alopecia > 2 $cm^2$ in area = 3. Incidence is expressed as the percentage of mice that showed clinical manifestations.

*2.7.8. Statistics*

All data are presented as mean ± SD, and differences between the groups are tested for their statistical significance by paired student t-tests for related samples and Mann-Whitney U non-parametric test for non-related samples. P-values less than 0.05 are considered significant. P-values are expressed as follows: 0.05 > p > 0.01 as *; 0.01 > p > 0.001 as **.; p < 0.001 as ***.

**Example 3. hADME**

***3.1. Aqueous Solubility***

Starting from a 10 mM stock in DMSO, a serial dilution of the compound is prepared in DMSO. The dilution series is transferred to a 96 NUNC Maxisorb plate F-bottom and 0.1 M phosphate buffer pH 7.4 or 0.1 M citrate buffer pH 3.0 at room temperature is added.

The final concentrations range from 18.75 to 300 μM in 5 equal dilution steps. The final DMSO concentration does not exceed 3%.

200 μM Pyrene is added to the corner points of each 96-well plate and serves as a reference point for calibration of Z-axis on the microscope.

The assay plates are sealed and incubated for 1 h at 37°C while shaking at 230 rpm. The plates are then scanned under a white light microscope, yielding individual pictures of the precipitate per concentration. The first concentration at which the compound appears completely dissolved is the concentration reported, however the true concentration lies somewhere between this concentration and one dilution step higher.

Solubility values are reported in μM and in μg/mL.

***3.2. Thermodynamic solubility***

Thermodynamic solubility of a compound is determined in water, phosphate or citrate buffer with pH of choice or biologically relevant gastrointestinal media (FaSSIF, FeSSIF, SGF). Dry matter of the compound is added to the medium of choice and incubated for 24 h at room temperature. The concentration of compound in the supernatant is analyzed by LC/MS-MS and the signal is plotted against the linear standard curve of that compound.

2.5-3 mg dry matter of test compound is dissolved in water, phosphate or citrate buffer with pH of choice or biologically relevant gastrointestinal media (FaSSIF, FeSSIF, SGF) in a glass vial. After addition of a magnetic stirrer, the samples are stirred for 24 h at room temperature. The vials are then centrifuged shortly and the supernatant is filtered. Each sample is diluted by a factor of 100 and a 10 in DMSO. A final 100 fold dilution in 70/30 water/acetonitrile is used for LCMS-MS analysis.

A standard curve is made starting from a 10 mM stock in DMSO, freshly prepared from dry matter. From this 10 mM DMSO stock solution, intermediate working solutions of 200, 50 and 10 μg/mL in DMSO are made and used to prepare 40, 20, 10, 5, 1, 0.2, 0.1 and 0.04 μg/mL solutions in DMSO. Two quality control samples are made: one of 15 μg/mL and one of 0.5 μg/mL in DMSO, also starting from the DMSO working stock solutions.

The standard curve and quality controls are diluted by a factor of 100 in 70/30 water/acetonitrile and analyzed on LC/MS-MS. The peak areas of the standard curve are plotted in a graph and a linear or polynomial of the second order equation is used to calculate the unknown concentrations of the test compound.

Solubility values are reported in μM or μg/mL.

### *3.3. Liver microsomal stability*

A 10 mM stock solution of compound in DMSO is 1,668 fold diluted in a 105 mM phosphate buffer pH 7.4. Of this compound dilution, 50 μL is transferred in two 96-well plates: one for time point 0 min (T0 plate) and one for time point 30 min (T30 plate) and pre-warmed at 37°C.

In the time zero reference sample (T0 plate), 100 μL MeOH (1:1) is added to the wells. In each assay plate (T0 and T30 min), 50 μL of liver microsomal mix is then added.

Final reaction concentrations are: 3 μM compound, 0.5 mg/mL liver microsomes, 0.4 U/mL GDPDH, 3.3 mM $MgCl_2$, 3.3 mM glucose-6-phosphate and 1.3 mM NADP+.

The T30 plate is incubated at 37°C, 300 rpm and after 30 min of incubation the reaction is stopped with MeOH (1:1). The samples are mixed, centrifuged and the supernatant is harvested for analysis on LC-MS/MS (API2000 from Applied Biosystems).

The samples are analyzed on LC-MS/MS with a flow rate of 0.5mL/min. Solvent A is 0.1% Formic Acid in water and solvent B is 0.1% Formic Acid in methanol. The sample is run under positive ion spray on a Pursuit 5 C18 2.0mm column (Varian). The solvent gradient has a total run time of 1.4 min and ranges from 10% B to 100% B. Peak area from the parent compound at time 0 is considered to be 100% remaining. The percentage remaining after 30 min incubation is calculated from time 0. The solubility of the compound in the final test concentration in buffer is inspected by microscope and results are also reported.

### *3.4. Hepatocyte stability.*

Test compounds (1 μM initial concentration, n=2) are incubated in Williams' Medium E, containing 4 mM L-glutamine and 2 mM magnesium sulphate, with pooled cryopreserved hepatocytes (Celsis International) in suspension at cell densities of 0.25-0.5 million viable cells/mL. The incubations are performed at 37°C in a shaking water bath with 100 μL samples taken from the incubation at 0, 10, 20, 45 and 90 min, and reactions terminated by addition of 100 μL of acetonitrile containing carbamazepine as analytical internal standard. Samples are centrifuged and the supernatant fractions analysed by LC-MS/MS. The instrument responses (i.e. peak heights) are referenced to the zero time-point samples (as 100%) in order to determine the percentage of compound remaining. Ln plots of the % remaining for each compound are used to determine the half-life for the hepatocyte incubations. Half-life values are calculated from the relationship: T1/2 (min) = -0.693/λ, where λ is the slope of the Ln concentration vs time curve. Standard compounds testosterone, midazolam, and 4-methylumbelliferone are included in the assay design.

### *3.5. Plasma Protein Binding (Equilibrium Dialysis)*

A 10 mM stock solution of the compound in DMSO is diluted with a factor 10 in DMSO. This solution is further diluted in freshly thawed human, rat, mouse or dog plasma (BioReclamation INC) with a final concentration of 5 μM and final DMSO concentration of 0.5%.

A Pierce Red Device plate with inserts (ThermoScientific) is prepared and filled with 450 μL PBS in the buffer chamber and 300 μL of the spiked plasma in the plasma chamber. The plate is incubated for 4 h at 37°C while shaking at 100 rpm. After incubation, 120 μL of both chambers is transferred to 480 μL methanol in a 96-well round bottom, PP deep-well plates (Nunc) and sealed with an aluminum foil lid. The samples are mixed and immediately centrifuged 30 min at 1400 RCF at 4°C and the supernatant is transferred to a 96 v-bottom PP plate (Greiner, 651201) for analysis on LC-MS/MS (API2000 from Applied Biosystems).

The samples are analyzed on LC-MS/MS with a flow rate of 0.5 mL/min. Solvent A is 0.1% formic acid in water and solvent B is 0.1% formic acid in methanol. The sample is run under positive ion spray on a Pursuit 5 C18 2.0mm column (Varian). The solvent gradient has a total run time of 1.4 min and ranges from 10% B to 100% B.

Peak area from the compound in the buffer chamber and the plasma chamber are considered to be 100% compound. The percentage bound to plasma is derived from these results and is reported as percentage bound to plasma.

The solubility of the compound in the final test concentration in PBS is inspected by microscope to indicate whether precipitation is observed or not.

### *3.6. Caco-2 Permeability*

Bi-directional Caco-2 assays are performed as described below. Caco-2 cells are obtained from European Collection of Cell Cultures (ECACC, cat 86010202) and used after a 21 day cell culture in 24-well Transwell plates (Corning, cell growth area: 0.33 $cm^2$, membrane pore size: 0.4 μM, membrane diameter: 6.5 mm).

2x$10^5$ cells/well are seeded in plating medium consisting of DMEM + GlutaMAX™-I + 1% NEAA + 10% FBS (FetalClone II) + 1% Pen/Strep. The medium is changed every 2 - 3 days.

Test and reference compounds (propranolol and rhodamine 123 or vinblastine, all purchased from Sigma) are prepared in Hanks' Balanced Salt Solution containing 25 mM HEPES (pH 7.4) and added to either the apical (125 μL) or basolateral (600 μL) chambers of the Transwell plate assembly at a concentration of 10 μM with a final DMSO concentration of 0.25%.

50 μM Lucifer Yellow (Sigma) is added to the donor buffer in all wells to assess integrity of the cell layers by monitoring Lucifer Yellow permeation. As Lucifer Yellow (LY) cannot freely permeate lipophilic barriers, a high degree of LY transport indicates poor integrity of the cell layer.

After a 1 h incubation at 37°C while shaking at an orbital shaker at 150 rpm, 70 μL aliquots are taken from both apical (A) and basal (B) chambers and added to 100 μL 50:50 MeCN:water solution containing analytical internal standard (0.5 μM carbamazepine) in a 96-well plate.

Lucifer yellow is measured with a Spectramax Gemini XS (Ex 426nm and Em 538nm) in a clean 96-well plate containing 150 μL of liquid from basolateral and apical side.

Concentrations of compound in the samples are measured by high performance liquid-chromatography/mass spectroscopy (LC-MS/MS).

Apparent permeability (Papp) values are calculated from the relationship:

$$\text{Papp} = [\text{compound}]\text{acceptor final} \times \text{Vacceptor} / ([\text{compound}]\text{donor initial} \times \text{Vdonor}) / \text{Tinc} \times \text{Vdonor} / \text{surface area} \times 60 \times 10^{-6}\ \text{cm/s}$$

- V = chamber volume
- Tinc = incubation time.
- Surface area = 0.33 $cm^2$
- The Efflux ratios, as an indication of active efflux from the apical cell surface, are calculated using the ratio of Papp B>A/ Papp A>B.

The following assay acceptance criteria are used:

- Propranolol: Papp (A>B) value ≥ 20(×$10^{-6}$ cm/s)
- Rhodamine 123 or Vinblastine: Papp (A>B) value < 5 (×$10^{-6}$ cm/s) with Efflux ratio ≥5.
- Lucifer yellow permeability: ≤100 nm/s

### *3.7. Pharmacokinetic study*

*3.7.1. Single dose pharmacokinetic study in rats*

Compounds are formulated in PEG200/physiological saline mixtures for the intravenous route and in PEG400/0.5% methylcellulose (10/90 v/v) for the oral route. Test compounds are orally dosed as a single esophageal gavage at 5-10 mg/kg and intravenously dosed as a bolus via the caudal vein at 1 mg/kg to male Sprague-Dawley rats. Each group consists of 3 rats. Blood samples are collected either via the jugular vein using cannulated rats or at the retro-orbital sinus with lithium heparin as anti-coagulant at the time points in the following range: 0.05 to 8 h (intravenous route), and 0.25 to 6 or 24 h (oral route). Whole blood samples are centrifuged at 5000 rpm for 10 min and the resulting plasma samples are stored at -20°C pending analysis.

*3.7.2. Multiple dose pharmacokinetic study in rats*

Compounds are formulated in PEG400/0.5% methylcellulose (10/90 v/v) for the oral route. Test compounds are orally dosed as an esophageal daily gavage at 30 or 300 mg/kg to male Sprague-Dawley rats for 14 days. Each group consists of 3 rats. Blood samples are collected via the tail vein with lithium heparin as anti-coagulant at the following time points on day 1, 7 and 14: 0.25, 1, 4, 8 and 24 h. In addition, on day 2 blood samples are taken at 0.25, 1 and 4 h and at day 4 and 11 at 0.25 h. Whole blood samples are centrifuged at 5000 rpm for 10 min and the resulting plasma samples are stored at -20°C pending analysis.

*3.7.3. Quantification of compound levels in plasma*

Plasma concentrations of each test compound are determined by an LC-MS/MS method in which the mass

spectrometer is operated in positive or negative electrospray mode.

*3.7.4. Determination of pharmacokinetic parameters*

Pharmacokinetic parameters are calculated using Winnonlin® (Pharsight®, US)

## FINAL REMARKS

It will be appreciated by those skilled in the art that the foregoing descriptions are exemplary and explanatory in nature, and intended to illustrate the invention and its preferred embodiments. The invention is intended to be defined not by the above description, but by the following claims.

It should be understood that factors such as the differential cell penetration capacity of the various compounds can contribute to discrepancies between the activity of the compounds in the *in vitro* biochemical and cellular assays.

At least some of the chemical names of compound of the invention as given and set forth in this application, may have been generated on an automated basis by use of a commercially available chemical naming software program, and have not been independently verified. Representative programs performing this function include the Lexichem naming tool sold by Open Eye Software, Inc. and the Autonom Software tool sold by MDL, Inc. In the instance where the indicated chemical name and the depicted structure differ, the depicted structure will control.

## REFERENCES

Adada, M., Canals, D., Hannun, Y.A., Obeid, L.M., 2013. Sphingosine-1-phosphate receptor 2. FEBS J. 280, 6354-6366. https://doi.org/10.1111/febs.12446

Ashcroft, T., Simpson, J.M., Timbrell, V., 1988. Simple method of estimating severity of pulmonary fibrosis on a numerical scale. J. Clin. Pathol. 41, 467-470.

Bickelhaupt, S., Erbel, C., Timke, C., Wirkner, U., Dadrich, M., Flechsig, P., Tietz, A., Pföhler, J., Gross, W., Peschke, P., Hoeltgen, L., Katus, H.A., Gröne, H.-J., Nicolay, N.H., Saffrich, R., Debus, J., Sternlicht, M.D., Seeley, T.W., Lipson, K.E., Huber, P.E., 2017. Effects of CTGF Blockade on Attenuation and Reversal of Radiation-Induced Pulmonary Fibrosis. JNCI J. Natl. Cancer Inst. 109. https://doi.org/10.1093/jnci/djw339

Blankenbach, K.V., Schwalm, S., Pfeilschifter, J., Meyer Zu Heringdorf, D., 2016. Sphingosine-1-Phosphate Receptor-2 Antagonists: Therapeutic Potential and Potential Risks. Front. Pharmacol. 7, 167. https://doi.org/10.3389/fphar.2016.00167

Brunnemer, E., Wälscher, J., Tenenbaum, S., Hausmanns, J., Schulze, K., Seiter, M., Heussel, C.P., Warth, A., Herth, F.J.F., Kreuter, M., 2018. Real-World Experience with Nintedanib in Patients with Idiopathic Pulmonary Fibrosis. Respiration 95, 301-309. https://doi.org/10.1159/000485933

Chen, C.-W., Beyer, C., Liu, J., Maier, C., Li, C., Trinh-Minh, T., Xu, X., Cole, S.H., Hsieh, M.H., Ng, N., Althage, A., Meeusen, S., Pan, S., Svensson, E.C., Seidel, H.M., Schett, G., Gergely, P., Harris, J.L., Distler, J.H.W., 2017. Pharmacological inhibition of porcupine induces regression of experimental skin fibrosis by targeting Wnt signalling. Ann. Rheum. Dis. 76, 773-778. https://doi.org/10.1136/annrheumdis-2016-210294

Devos, F.C., Maaske, A., Robichaud, A., Pollaris, L., Seys, S., Lopez, C.A., Verbeken, E., Tenbusch, M., Lories, R., Nemery, B., Hoet, P.H., Vanoirbeek, J.A., 2017. Forced expiration measurements in mouse models of obstructive and restrictive lung diseases. Respir. Res. 18, 123. https://doi.org/10.1186/s12931-017-0610-1

Hobson, A.D., Harris, C.M., van der Kam, E.L., Turner, S.C., Abibi, A., Aguirre, A.L., Bousquet, P., Kebede, T., Konopacki, D.B., Gintant, G., Kim, Y., Larson, K., Maull, J.W., Moore, N.S., Shi, D., Shrestha, A., Tang, X., Zhang, P., Sarris, K.K., 2015. Discovery of A-971432, An Orally Bioavailable Selective Sphingosine-1-Phosphate Receptor 5 (S1P5) Agonist for the Potential Treatment of Neurodegenerative Disorders. J. Med. Chem. 58, 9154-9170. https://doi.org/10.1021/acs.jmedchem.5b00928

Kitada, Y., Kajita, K., Taguchi, K., Mori, I., Yamauchi, M., Ikeda, T., Kawashima, M., Asano, M., Kajita, T., Ishizuka, T., Banno, Y., Kojima, I., Chun, J., Kamata, S., Ishii, I., Morita, H., 2016. Blockade of Sphingosine 1-Phosphate Receptor 2 Signaling Attenuates High-Fat Diet-Induced Adipocyte Hypertrophy and Systemic Glucose Intolerance in Mice. Endocrinology 157, 1839-1851. https://doi.org/10.1210/en.2015-1768

Lancaster, L.H., Andrade, J.A. de, Zibrak, J.D., Padilla, M.L., Albera, C., Nathan, S.D., Wijsenbeek, M.S., Stauffer, J.L., Kirchgaessler, K.-U., Costabel, U., 2017. Pirfenidone safety and adverse event management in idiopathic pulmonary fibrosis. Eur. Respir. Rev. 26, 170057. https://doi.org/10.1183/16000617.0057-2017

Matsuse, T., Teramoto, S., Katayama, H., Sudo, E., Ekimoto, H., Mitsuhashi, H., Uejima, Y., Fukuchi, Y., Ouchi, Y., 1999. ICAM-1 mediates lung leukocyte recruitment but not pulmonary fibrosis in a murine model of bleomycin-induced lung injury. Eur. Respir. J. 13, 71-77.
Milstien, S., Spiegel, S., 2006. Targeting sphingosine-1-phosphate: A novel avenue for cancer therapeutics. Cancer Cell 9, 148-150. https://doi.org/10.1016/j.ccr.2006.02.025
Nanthakumar, C.B., Hatley, R.J.D., Lemma, S., Gauldie, J., Marshall, R.P., Macdonald, S.J.F., 2015. Dissecting fibrosis: therapeutic insights from the small-molecule toolbox. Nat. Rev. Drug Discov. 14, 693-720. https://doi.org/10.1038/nrd4592
Richeldi, L., du Bois, R.M., Raghu, G., Azuma, A., Brown, K.K., Costabel, U., Cottin, V., Flaherty, K.R., Hansell, D.M., Inoue, Y., Kim, D.S., Kolb, M., Nicholson, A.G., Noble, P.W., Selman, M., Taniguchi, H., Brun, M., Le Maulf, F., Girard, M., Stowasser, S., Schlenker-Herceg, R., Disse, B., Collard, H.R., INPULSIS Trial Investigators, 2014. Efficacy and safety of nintedanib in idiopathic pulmonary fibrosis. N. Engl. J. Med. 370, 2071-2082. https://doi.org/10.1056/NEJMoa1402584
Sobel, K., Menyhart, K., Killer, N., Renault, B., Bauer, Y., Studer, R., Steiner, B., Bolli, M.H., Nayler, O., Gatfield, J., 2013. Sphingosine 1-Phosphate (SIP) Receptor Agonists Mediate Pro-fibrotic Responses in Normal Human Lung Fibroblasts via S1P2 and S1P3 Receptors and Smad-independent Signaling. J. Biol. Chem. 288, 14839-14851. https://doi.org/10.1074/jbc.M112.426726
Takabe, K., Paugh, S.W., Milstien, S., Spiegel, S., 2008. "Inside-Out" Signaling of Sphingosine-1-Phosphate: Therapeutic Targets. Pharmacol. Rev. 60, 181-195. https://doi.org/10.1124/pr.107.07113
Yuan, S.Y., Rigor, R.R., 2010. Regulation of Endothelial Barrier Function, Integrated Systems Physiology: From Molecule to Function to Disease. Morgan & Claypool Life Sciences, San Rafael (CA).

## Claims

**1.** A compound, or a pharmaceutically acceptable salt, or a solvate or the pharmaceutically acceptable salt of a solvate thereof, according to Formula I:

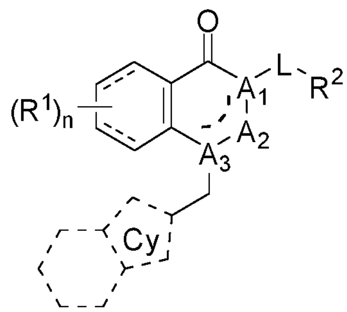

I

wherein

each $A_1$, $A_2$ and $A_3$ is independently selected from C and N provided that $A_1$, $A_2$ and $A_3$ are not simultaneously C or N;
each $R^1$ is independently selected from

- $C_{1-4}$ alkyl,
- $C_{1-4}$ alkoxy,
- $C_{3-6}$ cycloalkyl,
- 4-7 membered monocyclic heteroaryl comprising 1, 2, or 3 hteroatoms independently selected from N, O, or S, optionally substituted with one or two =O groups,
- $-S(O)_2C_{1-4}$ alkyl,
- -CN,
- $-C(=O)NH_2$, and
- halo;

the subscript n is 0, 1 or 2;
Cy is a 5-membered ring linked 9-membered fused 5-6 bicyclic heteroaryl, comprising 1, 2 or 3 N atoms, which heteroaryl is substituted with one $R^3$ group and one $R^{4a}$ group, and optionally further substituted with one halo,

or OH;
$R^3$ is $C_{1-6}$ alkoxy optionally substituted with one or more independently selected from

- halo,
- $C_{1-4}$ alkoxy, or
- $C_{3-7}$ cycloalkyl optionally substituted with one $C_{1-4}$ alkyl, halo, or -CN;

$R^{4a}$ is

- $C_{1-4}$ alkyl optionally substituted with one or more halo,
- halo, or
- -CN;

L is absent or is $-CR^{5a}R^{5b}-$;
$R^2$ is

- -C(=O)OH,
- $-C(=O)NR^{6a}R^{6b}$,
- $-C(O)NHS(O)_2-C_{1-4}$ alkyl,
- $-C(O)NHS(O)_2-C_{3-7}$ cycloalkyl,
- $-Cy_1$, or
- $-C(=O)Cy_2$;

each $R^{5a}$ and $R^{5b}$ is independently selected from

- H,
- $C_{1-4}$ alkyl optionally substituted with one, two or three halo or $-NR^{8a}R^{8b}$, and
- $C_{1-4}$ alkoxy;

each $R^{6a}$ and $R^{6b}$ is independently selected from

- H,
- $C_{1-6}$ alkyl optionally substituted from one more independently selected
  - ◦ OH,
  - ◦ -CN,
  - ◦ halo,
  - ◦ $C_{1-4}$ alkoxy,
  - ◦ monocyclic 4-6 membered heterocycloalkyl comprising one or two heteroatoms selected from N, O, and S, optionally substituted with one $-CH_2-OH$
  - ◦ $-S(O)_2C_{1-4}$ alkyl,
  - ◦ $-S(O)_2NH_2$,
  - ◦ $-C(O)NR^{9a}R^{9b}$, or
  - ◦ $C_{3-7}$ cycloalkyl optionally substituted with OH, or halo;
- $C_{1-4}$ alkoxy,
- $C_{3-7}$ cycloalkyl optionally substituted with one or more OH, and
- monocyclic 4-6 membered heterocycloalkyl comprising one or two heteroatoms selected from N, O, and S, optionally substited with one or two oxo;

$Cy_1$ is

- $C_{3-7}$ monocyclic cycloalkyl, optionally substituted with one -C(=O)OH,
- monocyclic 4-6 membered heterocycloalkyl comprising one or two heteroatoms selected from N, O, and S, optionally substituted with one or two
  - ◦ $C_{1-4}$ alkyl optionally substituted with one -C(=O)OH,

$Cy_2$ is

- N-linked monocyclic 4-7 membered heterocycloalkyl comprising at least one N atom, and optionally one or two heteroatoms selected from N, O, and S, optionally substituted with one or more

    ◦ OH,
    ◦ Oxo,
    ◦ -CN,
    ◦ halo,
    ◦ $C_{1-4}$ alkoxy,
    ◦ $C_{1-4}$ alkyl optionally substituted with one or more independently selected

        ▪ halo,
        ▪ OH,

    ◦ $C_{3-7}$ cycloalkyl,
    ◦ $-S(O)_2C_{1-4}$ alkyl, or
    ◦ $-NR^{7a}R^{7b}$; or

- N-linked spirocyclic 7-9 membered heterocycloalkyl comprising at least one N atom, and optionally one or two heteroatoms selected from N, O, and S, optionally substituted with one or more halo;

each $R^{7a}$, $R^{7b}$, $R^{8a}$, $R^{8b}$, $R^{9a}$ and $R^{9b}$ is independently selected from H, and $C_{1-4}$ alkyl;
provided that

- when L is absent, $A_1$ and $A_2$ are C, $A_3$ is N, $R^3$ is unsubstituted $C_{1-6}$ alkoxy or $C_{1-6}$ alkoxy substituted with halogen, then $R^2$ is not COOH; and
- when $A_1$ and $A_2$ are N, $A_3$ is C, and $R^{4a}$ is $-CH_3$ then $R^3$ is not unsubstituted $C_{1-4}$ alkoxy.

2. The compound or pharmaceutically acceptable salt thereof according to claim 1, wherein the subscript n is 0.

3. The compound or pharmaceutically acceptable salt thereof according to claim 1 or 2, wherein wherein Cy is selected from $Cy_A$, $Cy_B$, $Cy_C$, and $Cy_D$:

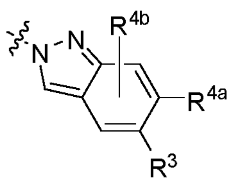
$Cy_A$,

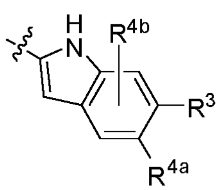
$Cy_B$,

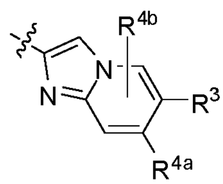
$Cy_C$,

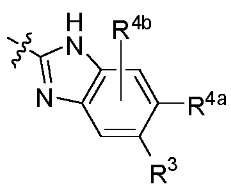
$Cy_D$.

4. The compound or pharmaceutically acceptable salt thereof according to claim 3, wherein $R^3$ is $-OCH_3$, $-OCH_2CH_3$, $-OCF_3$, $-OCH_2CF_3$, $-OCH_2CHF_2$, $-OCH_2CH_2OCH_3$,

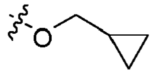,
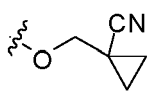, or
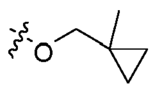.

5. The compound or pharmaceutically acceptable salt thereof according to claim 3 or 4, wherein $R^{4b}$ is H, F, Cl or OH.

6. The compound or pharmaceutically acceptable salt thereof according to claim 3 or 4, wherein $R^{4a}$ is F, Cl, -CN, or $-CF_3$.

7. The compound or pharmaceutically acceptable salt thereof according to claim 1, wherein the compound is according to Formula Va, Vb, Vc, VIa, VIb, or VIc:

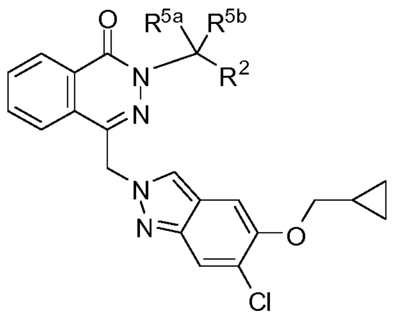
Va,
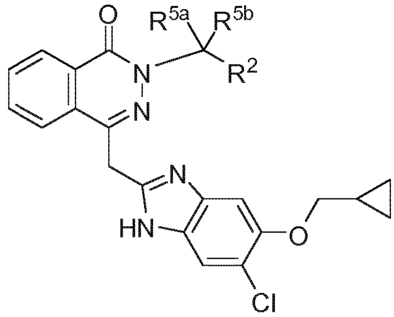
Vb,
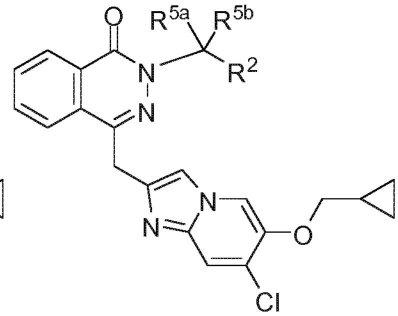
Vc

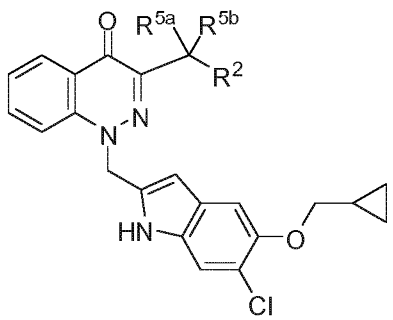
VIa,
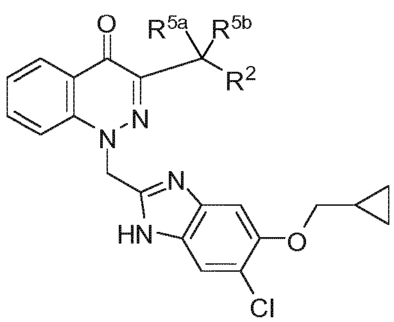
VIb, or
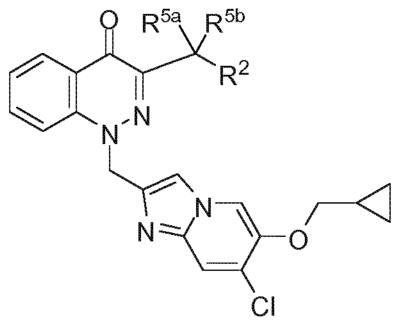
Vic.

8. The compound or pharmaceutically acceptable salt thereof according to claim 3 or 4, wherein wherein each $R^{5a}$ and $R^{5b}$ is independently selected from H, $-OCH_3$, $-OCH_2CH_3$, $-CH_3$, $-CH_2CH_3$, $-CH_3$, $-CH_2CHF_2$, $-CH_2CF_3$, and $-CH_2CH_2-N(CH_3)_2$.

9. The compound or pharmaceutically acceptable salt thereof according to claim 1, wherein the compound is according to Formula VIIa, VIIb, or VIIc:

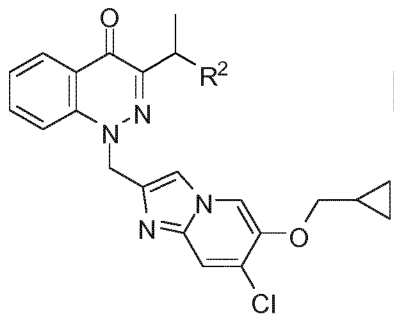
VIIa,
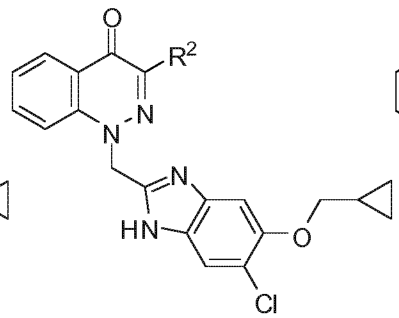
VIIb, or
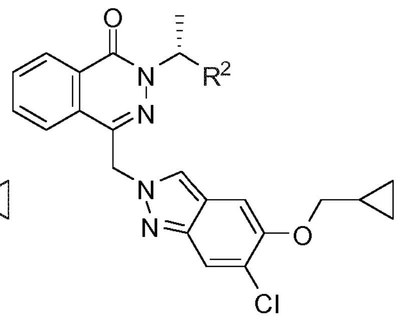
VIIc

10. The compound or pharmaceutically acceptable salt thereof according to anyone of claims 1-9, wherein $R^2$ is $-C(=O)OH$, $-C(O)NHS(O)_2-CH_3$, $-C(O)NHS(O)_2-C_{3-7}$ cycloalkyl,

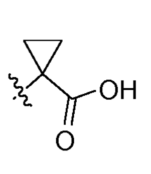
or
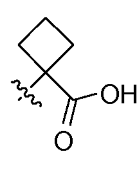
.

11. The compound or pharmaceutically acceptable salt thereof according to claim 1 selected from:
6-(2,2-difluoroethoxy)-2-[[4-oxo-3-(pyrrolidin-2-ylmethyl)phthalazin-1-yl]methyl]-3H-benzimidazole-5-carbonitrile, 2-(azetidin-3-yl)-4-[[6-chloro-5-(cyclopropylmethoxy)indazol-2-yl]methyl]phthalazin-1-one, 2-[4-[[6-chloro-5-(cyclopropylmethoxy)indazol-2-yl]methyl]-1-oxo-phthalazin-2-yl]propanoic acid, 2-[1-[[7-chloro-6-(cyclopropylmethoxy)imidazo[1,2-a]pyridin-2-yl]methyl]-4-oxo-cinnolin-3-yl]propanoic acid, 1-[[6-chloro-5-(cyclopropylmethoxy)-1H-benzimidazol-2-yl]methyl]-4-oxo-cinnoline-3-carboxylic acid, 2-[4-[(5-chloro-6-ethoxy-1H-benzimidazol-2-yl)methyl]-1-oxo-phthalazin-2-yl]acetic acid, 2-[4-[[6-cyano-5-(cyclopropylmethoxy)-1H-benzimidazol-2-yl]methyl]-1-oxo-phthalazin-2-yl]acetic acid, 2-[4-[[6-cyano-5-(2,2-difluoroethoxy)indazol-2-yl]methyl]-1-oxo-phthalazin-2-yl]acetic acid, 2-[4-[[5-chloro-6-(2,2-difluoroethoxy)-1H-indol-2-yl]methyl]-1-oxo-phthalazin-2-yl]acetic acid, 6-(cyclopropylmethoxy)-2-[[3-[2-[(3S,4S)-3,4-dihydroxypyrrolidin-1-yl]-2-oxo-ethyl]-4-oxo-cinnolin-1-yl]methyl]-3H-benzimidazole-5-carbonitrile, 4-[[5-chloro-6-(2,2-difluoroethoxy)-1H-indol-2-yl]methyl]-2-(2-morpholino-2-oxoethyl)phthalazin-1-one, 4-[[6-chloro-5-(2,2-difluoroethoxy)-1H-benzimidazol-2-yl]methyl]-2-(2-morpholino-2-oxoethyl)-1-oxo-phthalazine-6-carboxamide, 6-(2,2-difluoroethoxy)-2-[[3-[2-[(3S)-3-hydroxypyrrolidin-1-yl]-2-oxoethyl]-4-oxo-phthalazin-1-yl]methyl]-3H-benzimidazole-5-carbonitrile, 2-[(1-acetylpyrrolidin-2-yl)methyl]-4-[[5-chloro-6-(trifluoromethoxy)-1H-benzimidazol-2-yl]methyl]phthalazin-1-one, 2-[1-[[7-chloro-6-(cyclopropylmethoxy)imidazo[1,2-a]pyridin-2-yl]methyl]-4-oxo-cinnolin-3-yl]-N-methylsulfonyl-propanamide, 2-[1-[[5-chloro-6-(cyclopropylmethoxy)-1H-indol-2-yl]methyl]-4-oxo-cinnolin-3-yl]acetic acid, ((2R)-2-[4-[[6-chloro-5-(cyclopropylmethoxy)indazol-2-yl]methyl]-1-oxo-phthalazin-2-yl]propanoic acid), (2S)-2-[4-[[6-chloro-5-(cyclopropylmethoxy)indazol-2-yl]methyl]-1-oxo-phthalazin-2-yl]propanoic acid, (2S)-2-[1-[[7-chloro-6-(cyclopropylmethoxy)imidazo[1,2-a]pyridin-2-yl]methyl]-4-oxocinnolin-3-yl]propanoic acid, (2R)-2-[1-[[7-chloro-6-(cyclopropylmethoxy)imidazo[1,2-a]pyridin-2-yl]methyl]-4-oxocinnolin-3-yl]propanoic acid, (2-[1-[[5-cyano-6-(cyclopropylmethoxy)-1H-benzimidazol-2-yl]methyl]-4-oxocinnolin-3-yl]-3-(dimethylamino)propanoic acid), 2-[4-[(7-chloro-6-ethoxy-indazol-2-yl)methyl]-1-oxo-phthalazin-2-yl]acetic acid, 2-[4-[[5-chloro-6-(2,2-difluoroethoxy)-1H-benzimidazol-2-yl]methyl]-6-cyano-1-oxo-phthalazin-2-yl]acetic acid, 4-[[5-chloro-6-(trifluoromethoxy)-1H-benzimidazol-2-yl]methyl]-2-cyclopentyl-phthalazin-1-one, 4-[[5-chloro-6-(trifluoromethoxy)-1H-benzimidazol-2-yl]methyl]-2-cyclopropyl-phthalazin-1-one, 4-[[5-chloro-6-(trifluoromethoxy)-1H-benzimidazol-2-yl]methyl]-2-cyclobutyl-phthalazin-1-one, 4-[[6-chloro-5-(trifluoromethoxy)-1H-benzimidazol-2-yl]methyl]-2-(cyclopropylmethyl)phthalazin-1-one, 4-[[6-chloro-5-(trifluoromethoxy)-1H-benzimidazol-2-yl]methyl]-2-(cyclobutylmethyl)phthalazin-1-one, 4-[[5-chloro-6-(2,2-difluoroethoxy)-1H-benzimidazol-2-yl]methyl]-6-methoxy-2-(2-morpholino-2-oxoethyl)phthalazin-1-one, 4-[[5-chloro-6-(2,2-difluoroethoxy)-1H-benzimidazol-2-yl]methyl]-7-methoxy-2-(2-morpholino-2-oxoethyl)phthalazin-1-one, 4-[[5-chloro-6-(2,2-difluoroethoxy)-1H-benzimidazol-2-yl]methyl]-2-cyclopropyl-phthalazin-1-one, 4-[[5-chloro-6-(2,2-difluoroethoxy)-1H-benzimidazol-2-yl]methyl]-7-morpholino-2-(2-morpholino-2-oxo-ethyl)phthalazin-1-one, 4-[[6-chloro-5-(trifluoromethoxy)-1H-benzimidazol-2-yl]methyl]-2-(THF-2-ylmethyl)phthalazin-1-one, 4-[[6-chloro-5-(trifluoromethoxy)-1H-benzimidazol-2-yl]methyl]-2-(tetrahydropyran-2-ylmethyl)phthalazin-1-one, 4-[[6-chloro-5-(trifluoromethoxy)-1H-benzimidazol-2-yl]methyl]-2-[(3-methyloxetan-3-yl)methyl]phthalazin-1-one, 4-[[6-chloro-5-(trifluoromethoxy)-1H-benzimidazol-2-yl]methyl]-2-[(2,2-dimethyl-1,3-dioxolan-4-yl)methyl]phthalazin-1-one, 4-[[6-chloro-5-(trifluoromethoxy)-1H-benzimidazol-2-yl]methyl]-2-[(1-methyl-3-piperidyl)methyl]phthalazin-1-one, 1-[[5-chloro-6-(2,2-difluoroethoxy)-1H-benzimidazol-2-yl]methyl]-3-(2-morpholino-2-oxo-ethyl)-4-oxo-phthalazine-6-carbonitrile, 4-[[5-chloro-6-(2,2-difluoroethoxy)-1H-benzimidazol-2-yl]methyl]-6-methylsulfonyl-2-(2-morpholino-2-oxoethyl)phthalazin-1-one, 6-(cyclopropylmethoxy)-2-[(3-cyclopropyl-4-oxo-phthalazin-1-yl)methyl]-1H-benzimidazole-5-carbonitrile, 4-[[6-chloro-5-(trifluoromethoxy)-1H-benzimidazol-2-yl]methyl]-2-[(2,2-difluorocyclopropyl)methyl]phthalazin-1-one, 4-[[6-chloro-5-(trifluoromethoxy)-1H-benzimidazol-2-yl]methyl]-2-(3,3-dimethyl-2-oxobutyl)phthalazin-1-one, 1-[[4-[[5-chloro-6-(trifluoromethoxy)-1H-benzimidazol-2-yl]methyl]-1-oxo-phthalazin-2-yl]methyl]cyclopropanecarboxylic acid, 4-[[6-chloro-5-(2,2-difluoroethoxy)-1H-benzimidazol-2-yl]methyl]-5-cyclopropyl-2-(2-morpholino-2-oxo-ethyl)phthalazin-1-one, 6-(2,2-Difluoro-ethoxy)-2-[3-(2-morpholin-4-yl-2-oxo-ethyl)-4-oxo-4H-cinnolin-1-ylmethyl]-3H-enzoimidazole-5 -carbonitrile, 1-[5-(2,2-Difluoro-ethoxy)-6-fluoro, -1H-benzoimidazol-2-ylmethyl]-3-(2-morpholin-4-yl-2-oxo-ethyl)-1H-cinnolin-4-one, 6-(2-methoxyethoxy)-2-[[3-(2-morpholino-2-oxo-ethyl)-4-oxo-phthalazin-1-yl]methyl]-3H-benzimidazole-5 -carbonitrile, 6-(2,2-difluoropropoxy)-2-[[3-(2-morpholino-2-oxo-ethyl)-4-oxo-phthalazin-1-yl]methyl]-3H-benzimidazole-5 -carbonitrile, 7-chloro-6-(2,2-difluoroethoxy)-2-[[3-(2-morpholino-2-oxo-ethyl)-4-oxo-phthalazin-1-yl]methyl]-3H-benzimidazole-5 -carbonitrile, 2-[[8-cyclopropyl-3-(2-morpholino-2-oxo-ethyl)-4-oxo-phthalazin-1-yl]methyl]-6-(2,2-difluoroethoxy)-3H-benzimidazole-5-carbonitrile, 4-[[6-chloro-5-(trifluoromethoxy)-1H-benzimidazol-2-yl]methyl]-2-(pyrrolidin-2-ylmethyl)phthalazin-1-one, 4-[[6-chloro-5-(trifluoromethoxy)-1H-benzimidazol-2-yl]methyl]-2-(4-piperidylmethyl)phthalazin-1-one, 4-[[6-chloro-5-(trifluoromethoxy)-1H-benzimidazol-2-yl]methyl]-2-(pyrrolidin-3-ylmethyl)phthalazin-1-one, 4-[[6-chloro-5-(trifluoromethoxy)-1H-benzimidazol-2-yl]methyl]-2-(3-piperidylmethyl)phthalazin-1-one, 4-[[6-chloro-5-(trifluoromethoxy)-1H-benzimidazol-2-yl]methyl] -2-pyrrolidin-3-yl-phthalazin-1 -one, 2-(azetidin-3-ylmethyl)-4-[[6-chloro-5-(trifluoromethoxy)-1H-benzimidazol-2-yl]methyl]phthalazin-1-one, 4-[[6-chloro-5-(trifluoromethoxy)-1H-benzimidazol-2-yl]methyl]-2-(4-piperidyl)phthalazin-1-one, 4-[[5-chloro-6-(trifluoromethoxy)-1H-benzimidazol-2-yl]methyl]-2-(morpholin-2-ylmethyl)phthalazin-1-one, 6-(2,2-difluoroethoxy)-2-[(4-oxo-3-pyrrolidin-3-yl-phthalazin-1-yl)methyl]-3H-benzimidazole-5-carbonitrile, 6-(2-methoxyethoxy)-2-[(4-oxo-3-pyrrolidin-3-yl-phthalazin-1-yl)methyl]-3H-benzimidazole-5-carbonitrile, 6-(2,2-difluoroethoxy)-2-[[8-methyl-3-(2-morpholino-2-oxo-ethyl)-4-oxo-phthalazin-1-yl]methyl]-3H-benzimidazole-5 -carbonitrile, 4-[[6-chloro-5-(2-methoxyethoxy)-1H-benzimidazol-2-yl]methyl] -2-pyrrolidin-3 -yl-phthalazin-1 -one, 2-[[3-(azetidin-3-yl)-4-oxo-phthalazin-1-yl]methyl]-6-(2,2-difluoroethoxy)-3H-benzimidazole-5-carbonitrile, 2-[3-[4-[[6-cyano-5-(2,2-difluoroethoxy)-1H-benzimidazol-2-yl]methyl]-1-oxo-phthalazin-2-yl]azetidin-1-yl] acetic acid, 2-[2-[[4-[[6-cyano-5-(2,2-difluoroethoxy)-1H-benzimidazol-2-yl]methyl]-1-oxo-phthalazin-2-yl]methyl]pyrrolidin-1-yl]acetic acid, 2-[3-[4-[[6-cyano-5-(2,2-difluoroethoxy)-1H-benzimidazol-2-yl]methyl]-1-oxo-phthalazin-2-yl]pyrrolidin-1-yl]acetic acid, 6-(2,2-difluoroethoxy)-2-[[3-(1-methylpyrrolidin-3-yl)-4-oxo-phthalazin-1-yl]methyl]-3H-benzimidazole-5-carbonitrile, 2-[3-[4-[[7-cyano-6-(cyclopropylmethoxy)imidazo[1,2-a]pyridin-2-yl]methyl]-1-oxo-phthalazin-2-yl]azetidin-1-yl] acetic acid, 2-[4-[[6-cyano-5-(2,2-difluoroethoxy)-1H-benzimidazol-2-yl]methyl]-1-oxo-phthalazin-2-yl]-3-(dimethylamino)propanoic acid, 3-(azetidin-1-yl)-2-[4-[[6-cyano-5-(2,2-difluoroethoxy)-1H-benzimidazol-2-yl]methyl] -1 -oxo-phthalazin-2-yl]propanoic acid, 2-[4-[[6-chloro-5-(2,2-difluoroethoxy)indazol-2-yl]methyl]-1-oxo-phthalazin-2-yl]propanoic acid, 2-[4-[(6-chloro-5-ethoxy-1H-benzimidazol-2-yl)methyl]-8-methyl-1-oxo-phthalazin-2-yl]acetic acid, {1-[6-Chloro-5-(2,2-difluoro-ethoxy)-1H-benzoimidazol-2-ylmethyl]-4-oxo-1,4-dihydro-cinnolin-3-yl}-acetic acid, 2-[4-[[7-cyano-6-(cyclopropylmethoxy)imidazo[1,2-a]pyridin-2-yl]methyl]-1-oxo-phthalazin-2-yl]acetic acid, 1-[[6-cyano-5-(2,2-difluoroethoxy)-1H-benzimidazol-2-yl]methyl]-4-oxo-quinoline-3-carboxylic acid, 2-[4-[[7-cyano-6-(cyclopropylmethoxy)imidazo[1,2-a]pyridin-2-yl]methyl]-1-oxo-phthalazin-2-yl]propanoic acid, 2-[4-[[7-chloro-6-(cyclopropylmethoxy)imidazo[1,2-a]pyridin-2-yl]methyl]-1-oxo-phthalazin-2-yl]acetic acid, 2-[4-[[7-chloro-6-(cyclopropylmethoxy)imidazo[1,2-a]pyridin-2-yl]methyl]-1-oxo-phthalazin-2-yl]propanoic acid, 1-[[7-chloro-6-(cyclopropylmethoxy)imidazo[1,2-a]pyridin-2-yl]methyl]-4-oxo-quinoline-3-carboxylic acid, 1-[[7-chloro-6-(cyclopropylmethoxy)imidazo[1,2-a]pyridin-2-yl]methyl]-4-oxo-cinnoline-3-carboxylic acid, 2-[4-[[7-chloro-6-(cyclopropylmethoxy)imidazo[1,2-a]pyridi-2-yl]methyl]-6-fluoro-1-oxo-phthalazin-2-yl]propanoic acid, 2-[3-[4-[[7-chloro-6-(cyclopropylmethoxy)imidazo[1,2-a]pyridin-2-yl]methyl]-1-oxo-phthalazin-2-yl]pyrrolidin-1-yl]acetic acid, 2-[l-[[7-chloro-6-(cyclopropylmethoxy)imidazo[1,2-a]pyridin-2-yl]methyl]-4-oxo-cinnolin-3-yl]-2-methyl-propanoic acid, 2-[1-[[7-chloro-6-(cyclopropylmethoxy)imidazo[1,2-a]pyridin-2-yl]methyl]-4-oxo-cinnolin-3-yl]acetic acid, 1-[[6-chloro-5-(cyclopropylmethoxy)-1H-benzimidazol-2-yl]methyl]-4-oxo-quinoline-3-carboxylic acid, 2-[4-[[6-chloro-5-(cyclopropylmethoxy)indazol-2-yl]methyl]-1-oxo-phthalazin-2-yl]cyclobutanecarboxylic acid, 2-[4-[[6-chloro-5-(cyclopropylmethoxy)indazol-2-yl]methyl]-6-cyano-1-oxo-phthalazin-2-yl]propanoic acid, 2-[4-[[6-chloro-5-(cyclopropylmethoxy)indazol-2-yl]methyl]-1-oxo-5,6,7,8-tetrahydrophthalazin-2-yl]propanoic acid, 2-[1-[[7-chloro-6-(cyclopropylmethoxy)imidazo[1,2-a]pyridin-2-yl]methyl]-4-oxo-cinnolin-3-yl]-2-methoxy-acetic acid, 1-[4-[[6-chloro-5-(cyclopropylmethoxy)indazol-2-yl]methyl]-1-oxo-phthalazin-2-yl]cyclopropanecarboxylic acid, 1-[1-[[7-chloro-6-(cyclopropylmethoxy)imidazo[1,2-a]pyridin-2-yl]methyl]-4-oxo-cinnolin-3-yl]cyclopropanecarboxylic acid, 2-[4-[[6-chloro-5-(cyclopropylmethoxy)indazol-2-yl]methyl]-7-methoxy-1-oxo-phthalazin-2-yl]propanoic acid, 2-[1-[[7-chloro-6-(cyclopropylmethoxy)imidazo[1,2-a]pyridin-2-yl]methyl]-4-oxo-cinnolin-3-yl]butanoic acid, 2-[1-[[6-(cyclopropylmethoxy)-7-(trifluoromethyl)imidazo[1,2-a]pyridin-2-yl]methyl]-4-oxo-cinnolin-3-yl]acetic acid, 2-[1-[[6-(cyclopropylmethoxy)-7-(trifluoromethyl)imidazo[1,2-a]pyridin-2-yl]methyl]-4-oxo-cinnolin-3-yl]propanoic acid, 2-[1-[[7-chloro-6-(cyclopropylmethoxy)imidazo[1,2-a]pyridin-2-yl]methyl]-7-fluoro-4-oxo-cinnolin-3-yl]acetic acid, 2-[1-[[7-chloro-6-(cyclopropylmethoxy)imidazo[1,2-a]pyridin-2-yl]methyl]-5-methyl-4-oxo-cinnolin-3-yl]acetic acid, 2-[1-[[6-(cyclopropylmethoxy)-7-(trifluoromethyl)imidazo[1,2-a]pyridin-2-yl]methyl]-4-oxo-cinnolin-3-yl]butanoic acid, (1-[[5-(cyclopropylmethoxy)-6-(trifluoromethyl)-1H-benzimidazol-2-yl]methyl]-4-oxocinnoline-3-carboxylic acid), 2-[1-[[7-chloro-6-(cyclopropylmethoxy)imidazo[1,2-a]pyridin-2-yl]methyl]-4-oxo-5,6,7,8-tetrahydrocinnolin-3-yl] acetic acid, (1-[[5-(cyclopropylmethoxy)-6-(trifluoromethyl)-1H-benzimidazol-2-yl]methyl]-4-oxoquinoline-3-carboxylic acid), 2-[1-[[6-cyano-5-(cyclopropylmethoxy)-1H-benzimidazol-2-yl]methyl]-4-oxo-cinnolin-3-yl]propanoic acid, 2-[1-[[6-cyano-5-(cyclopropylmethoxy)-1H-benzimidazol-2-yl]methyl]-4-oxo-cinnolin-3-yl] acetic acid, 2-[1-[[6-bromo-5-(cyclopropylmethoxy)-1H-benzimidazol-2-yl]methyl]-4-oxo-cinnolin-3-yl]acetic acid, 1-[[5-cyano-6-(cyclopropylmethoxy)-1H-benzimidazol-2-yl]methyl]-4-oxo-cinnoline-3-carboxylic acid, 2-[4-[[6-bromo-5-(cyclopropylmethoxy)indazol-2-yl]methyl]-1-oxo-phthalazin-2-yl]acetic acid, 2-[4-[[6-chloro-5-(trifluoromethoxy)-1H-benzimidazol-2-yl]methyl]-1-oxo-phthalazin-2-yl]acetic acid, 2-[4-[[5-chloro-6-(trifluoromethoxy)-1H-benzimidazol-2-yl]methyl]-1-oxo-5,6,7,8-tetrahydrophthalazin-2-yl]acetic acid, 2-[4-[[6-chloro-5-(cyclopropylmethoxy)indazol-2-yl]methyl]-1-oxo-phthalazin-2-yl]acetic acid, 2-[4-[[5-chloro-6-(cyclopropylmethoxy)-1H-benzimidazol-2-yl]methyl]-1-oxo-phthalazin-2-yl]acetic acid, 2-[4-[[5-chloro-6-(cyclopropylmethoxy)-7-fluoro-1H-benzimidazol-2-yl]methyl]-1-oxo-phthalazin-2-yl]acetic acid, 2-[4-[[5-chloro-6-(cyclopropylmethoxy)-1H-benzimidazol-2-yl]methyl]-1-oxo-5,6,7,8-tetrahydrophthalazin-2-yl] acetic acid, 2-[4-[[5-chloro-6-(2,2-difluoroethoxy)-1H-benzimidazol-2-yl]methyl]-1-oxo-phthalazin-2-yl]acetic acid, 2-[4-[[5-chloro-6-(2,2-difluoroethoxy)-1H-benzimidazol-2-yl]methyl]-1-oxo-5,6,7,8-tetrahydrophthalazin-2-yl]acetic acid, 2-[4-[(5-chloro-6-methoxy-1H-benzimidazol-2-yl)methyl]-1-oxo-5,6,7,8-tetrahydrophthalazin-2-yl]acetic acid, 2-[4-[(5-chloro-6-ethoxy-1H-benzimidazol-2-yl)methyl]-1-oxo-5,6,7,8-tetrahydrophthalazin-2-yl]acetic acid, 2-[4-(6-Chloro-5-cyclopropylmethoxy-1H-benzoimidazol-2-ylmethyl)-1 -oxo-1H-phthalazin-2-yl]-propionic acid, 2-[4-[[5-(cyclopropylmethoxy)-6-(trifluoromethyl)-1H-benzimidazol-2-yl]methyl]-1-oxo-phthalazin-2-yl]acetic acid, 2-[4-[[5-(cyclopropylmethoxy)-6-fluoro-1H-benzimidazol-2-yl]methyl]-1-oxo-phthalazin-2-yl]acetic acid, {4-[5-Chloro-6-(2,2-difluoro-ethoxy)-1H-benzoimidazol-2-ylmethyl]-1-oxo-1H-isoquinolin-2-yl}-acetic acid, 2-[4-[[6-chloro-5-(2,2-difluoroethoxy)-1H-benzimidazol-2-yl]methyl]-5,8-difluoro-1-oxo-phthalazin-2-yl]acetic acid, 2-[4-[[5-ethoxy-6-(trifluoromethyl)-1H-benzimidazol-2-yl]methyl]-1-oxo-phthalazin-2-yl]acetic acid, 2-[4-[(5-chloro-6-ethoxy-3-oxo-1H-indazol-2-yl)methyl]-1-oxo-phthalazin-2-yl] acetic acid, 2-[4-[(5-ethoxy-6-fluoro-1H-benzimidazol-2-yl)methyl]-1-oxo-phthalazin-2-yl]acetic acid, 2-[4-[[5-(2,2-difluoroethoxy)-6-fluoro-1H-benzimidazol-2-yl]methyl]-1-oxo-phthalazin-2-yl]acetic acid, 2-[4-[[5-chloro-6-[(1-methylcyclopropyl)methoxy]-1H-benzimidazol-2-yl]methyl]-1-oxo-phthalazin-2-yl]acetic acid, 2-[4-[[5-chloro-6-(2,2-dimcthylpropoxy)-1H-benzimidazol-2-yl]methyl]-1-oxo-phthalazin-2-yl] acetic acid, 2-[4-[[5-chloro-6-[(1-cyanocyclopropyl)methoxy]-1H-benzimidazol-2-yl]methyl]-1-oxo-phthalazin-2-yl]acetic acid, 2-[4-[[5-chloro-6-(cyclopropylmethoxy)-3-oxo-1H-indazol-2-yl]methyl]-1-oxo-phthalazin-2-yl]acetic acid, 2-[4-[[6-cyano-5-(cyclopropylmethoxy)indazol-2-yl]methyl]-1-oxo-phthalazin-2-yl]acetic acid, 2-[4-[(6-chloro-5-methoxy-1H-benzimidazol-2-yl)methyl]-1-oxo-phthalazin-2-yl]acetic acid, 2-[4-[(6-bromo-5-ethoxy-indazol-2-yl)methyl]-1-oxo-phthalazin-2-yl]acetic acid, 2-[4-[(5-cyano-6-ethoxy-1H-benzimidazol-2-yl)methyl]-1-oxo-phthalazin-2-yl]acetic acid, 2-[4-[[5-cyano-6-(2,2-difluoroethoxy)-1H-benzimidazol-2-yl]methyl]-1-oxo-phthalazin-2-yl]acetic acid, 2-[4-[[6-chloro-5-(triauoromethoxy)-1H-benzimidazol-2-yl]methyl]-1-oxo-phthalazin-2-yl]-N-[(1S,2R)-2-hydroxycyclopentyl] acetamide, 2-[4-[[6-chloro-5-(trifluoromethoxy)-1H-benzimidazol-2-yl]methyl]-1-oxo-phthalazin-2-yl]-N-(1,1-dioxothian-4-yl)acetamide, 4-[[6-chloro-5-(trifluoromethoxy)-1H-benzimidazol-2-yl]methyl]-2-(2-morpholino-2-oxoethyl)phthalazin-1-one, 2-[4-[[6-chloro-5-(cyclopropylmethoxy)indazol-2-yl]methyl]-1-oxo-phthalazin-2-yl]-N-[(1S,2S)-2-hydroxycyclopentyl] acetamide, N-tert-butyl-2- [4-[[6-chloro-5-(cyclopropylmethoxy)indazol-2-yl]methyl]-1-oxo-phthalazin-2-yl]acetamide, 4-[[5-chloro-6-(trifluoromethoxy)-1H-benzimidazol-2-yl]methyl]-2-(2-morpholino-2-oxo-ethyl)-5,6,7,8-tetrahydrophthalazin-1-one, 2-[4-[[5-chloro-6-(trifluoromethoxy)-1H-benzimidazol-2-yl]methyl]-1-oxo-5,6,7,8-tetrahydrophthalazin-2-yl]-N-[(1S,2S)-2-hydroxycyclopentyl]acetamide, 2-[4-[[5-chloro-6-(trifluoromethoxy)-1H-benzimidazol-2-yl]methyl]-1-oxo-5,6,7,8-tetrahydrophthalazin-2-yl]-N-[(1S,2S)-2-hydroxycyclopentyl]acetamide, 4-[[6-chloro-5-(cyclopropylmethoxy)indazol-2-yl]methyl]-2-(2-morpholino-2-oxo-ethyl)phthalazin-1-one, 4-[[5-chloro-6-(cyclopropylmethoxy)-7-fluoro-1H-benzimidazol-2-yl]methyl]-2-(2-morpholino-2-oxoethyl)phthalazin-1-one, 2-[4-[[5-chloro-6-(cyclopropylmethoxy)-1H-benzimidazol-2-yl]methyl]-1-oxo-phthalazin-2-yl]-N-cyclopentyl-acetamide, 4-[[5-chloro-6-(2,2-difluoroethoxy)-1H-benzimidazol-2-yl]methyl]-2-(2-morpholino-2-oxoethyl)phthalazin-1-one, 4-[[6-chloro-5-(2,2-difluoroethoxy)-1H-benzimidazol-2-yl]methyl]-5,8-difluoro-2-(2-morpholino-2-oxoethyl)phthalazin-1-one, 4-[[6-chloro-5-(2,2-difluoroethoxy)-1H-benzimidazol-2-yl]methyl]-2-(2-morpholino-2-oxo-ethyl)-1-oxo-phthalazine-6-carbonitrile, 4-[(5-chloro-6-ethoxy-3-oxo-1H-indazol-2-yl)methyl]-2-(2-morpholino-2-oxo-ethyl)phthalazin-1-one, 4-(6-Chloro-5-cyclopropylmethoxy-1H-benzoimidazol-2-ylmethyl)-2-(1-methyl-2-morpholin-4-yl-2-oxoethyl)-2H-phthalazin-1-one, 4-[[5-(2,2-difluoroethoxy)-6-fluoro-1H-benzimidazol-2-yl]methyl]-2-(2-morpholino-2-oxoethyl)phthalazin-1-one, 4-[(5-ethoxy-6-fluoro-1H-benzimidazol-2-yl)methyl]-2-(2-morpholino-2-oxo-ethyl)phthalazin-1-one, 6-(cyclopropylmethoxy)-2-[[3-(2-morpholino-2-oxo-ethyl)-4-oxo-phthalazin-1-yl]methyl]-3H-benzimidazole-5 -carbonitrile, 1-[6-Chloro-5-(2,2-difluoro-ethoxy)-1H-benzoimidazol-2-ylmethyl]-3-(2-morpholin-4-yl-2-oxo-ethyl)-1H-cinnolin-4-one, 4-[[5-chloro-6-(2,2-dimethylpropoxy)-1H-benzimidazol-2-yl]methyl]-2-(2-morpholino-2-oxoethyl)phthalazin-1-one, 1-[[6-chloro-2-[[3-(2-morpholino-2-oxo-ethyl)-4-oxo-phthalazin-1-yl]methyl]-3H-benzimidazol-5-yl]oxymethyl]cyclopropanecarbonitrile, 1-[[6-chloro-2-[[3-[2-[(2S,6R)-2,6-dimethylmorpholin-4-yl]-2-oxo-ethyl]-4-oxo-phthalazin-1-yl]methyl]-3H-benzimidazol-5-yl]oxymethyl]cyclopropanecarbonitrile, 4-[[5-chloro-6-[(1-methylcyclopropyl)methoxy]-1H-benzimidazol-2-yl]methyl]-2-(2-morpholino-2-oxoethyl)phthalazin-1-one, 6-(2,2-difluoroethoxy)-2-[[3-(2-morpholino-2-oxo-ethyl)-4-oxo-5,6,7,8-tetrahydrophthalazin-1-yl]methyl]-1H-benzimidazole-5 -carbonitrile, 4-[(5-chloro-6-ethoxy-1H-benzimidazol-2-yl)methyl]-2-(2-morpholino-2-oxo-ethyl)phthalazin-1-one, 4-[[5-chloro-6-(cyclopropylmethoxy)-1H-benzimidazol-2-yl]methyl]-2-(2-morpholino-2-oxoethyl)phthalazin-1-one, 6-ethoxy-2-[[3-(2-morpholino-2-oxo-ethyl)-4-oxo-5,6,7,8-tetrahydrophthalazin-1-yl]methyl]-1H-benzimidazole-5 -carbonitrile, 4-[[6-ethoxy-5-(trifluoromethyl)-1H-benzimidazol-2-yl]methyl]-2-(2-morpholino-2-oxoethyl)phthalazin-1-one, 4-[6-Chloro-5-(2,2-difluoro-ethoxy)-1H-benzoimidazol-2-ylmethyl]-2-(2-morpholin-4-yl-2-oxo-ethyl)-2H-isoquinolin-1-one, 6-ethoxy-2-[[3-(2-morpholino-2-oxo-ethyl)-4-oxo-phthalazin-1-yl]methyl]-1H-benzimidazole-5-carbonitrile, 6-(2,2-difluoroethoxy)-2-[[3-(2-morpholino-2-oxo-ethyl)-4-oxo-phthalazin-1-yl]methyl]-1H-benzimidazole-5 -carbonitrile, 4-[[5-chloro-6-(2,2-difluoropropoxy)-1H-benzimidazol-2-yl]methyl]-2-(2-morpholino-2-oxoethyl)phthalazin-1-one, 4-[(5-chloro-6-isobutoxy-1H-benzimidazol-2-yl)methyl]-2-(2-morpholino-2-oxo-ethyl)phthalazin-1-one, 4-[[5-chloro-6-(2-methoxyethoxy)-1H-benzimidazol-2-yl]methyl]-2-(2-morpholino-2-oxoethyl)phthalazin-1-one, 2-[[3-(2-morpholino-2-oxo-ethyl)-4-oxo-phthalazin-1-yl]methyl]-6-(2,2,2-trifluoroethoxy)-3H-benzimidazole-5 -carbonitrile, 6-(2,2-difluoroethoxy)-2-[[3-[2-[3-(dimethylamino)pyrrolidin-1-yl]-2-oxo-ethyl]-4-oxo-phthalazin-1-yl]methyl]-3H-benzimidazole-5-carbonitrile, 6-(2,2-difluoroethoxy)-2-[[3-[2-[(2S,6R)-2,6-dimethylmorpholin-4-yl]-2-oxo-ethyl]-4-oxo-phthalazin-1-yl]methyl]-3H-benzimidazole-5-carbonitrile, 2-[4-[[6-cyano-5-(2,2-difluoroethoxy)-1H-benzimidazol-2-yl]methyl]-1-oxo-phthalazin-2-yl]-N-THF-3-yl-acetamide, 4-[[5-(2,2-difluoroethoxy)-6-fluoro-1H-benzimidazol-2-yl]methyl]-2-(2-morpholino-2-oxo-ethyl)-5,6,7,8-tetrahydrophthalazin-1-one, 2-[4-[[6-cyano-5-(2,2-difluoroethoxy)-1H-benzimidazol-2-yl]methyl]-1-oxo-phthalazin-2-yl]-N-methyl-N-(THF-2-ylmethyl)acetamide, 2-[4-[[6-cyano-5-(2,2-difluoroethoxy)-1H-benzimidazol-2-yl]methyl]-1-oxo-phthalazin-2-yl]-N-(2-cyanoethyl)-N-cyclopropyl-acetamide, 2-[4-[[6-cyano-5-(2,2-difluoroethoxy)-1H-benzimidazol-2-yl]methyl]-1-oxo-phthalazin-2-yl]-N-(2-methoxy-2-methyl-propyl)acetamide, 2-[4-[[6-cyano-5-(2,2-difluoroethoxy)-1H-benzimidazol-2-yl]methyl]-1-oxo-phthalazin-2-yl]-N-(2-methoxyethyl)-N-methyl-acetamide, 2-[4-[[6-cyano-5-(2,2-difluoroethoxy)-1H-benzimidazol-2-yl]methyl]-1-oxo-phthalazin-2-yl]-N-tetrahydropyran-3 -yl-acetamide, 6-(2,2-difluoroethoxy)-2-[[3-[2-(4,4-difluoro-1-piperidyl)-2-oxo-ethyl]-4-oxo-5,6,7,8-tetrahydrophthalazin-1-yl]methyl]-1H-benzimidazole-5 -carbonitrile, 6-(2,2-difluoroethoxy)-2-[[3-[2-(3-methoxypyrrolidin-1-yl)-2-oxo-ethyl]-4-oxo-phthalazin-1-yl]methyl]-1H-benzimidazole-5-carbonitrile, 2-[4-[[5-cyano-6-(2,2-difluoroethoxy)-1H-benzimidazol-2-yl]methyl]-1-oxo-phthalazin-2-yl]-N-(2-methoxyethyl)acetamide, 6-(2,2-difluoroethoxy)-2-[[3-[2-(4,4-difluoro-1-piperidyl)-2-oxo-ethyl]-4-oxo-phthalazin-1-yl]methyl]-1H-benzimidazole-5-carbonitrile, 6-(2,2-difluoroethoxy)-2-[[3-[2-(6-oxa-9-aza-spiro[3.5]nonan-9-yl)-2-oxo-ethyl]-4-oxo-phthalazin-1-yl]methyl]-1H-benzimidazole-5 -carbonitrile, 2-[4-[[5-cyano-6-(2,2-difluoroethoxy)-1H-benzimidazol-2-yl]methyl]-1-oxo-phthalazin-2-yl]-N-(3,3,3-trifluoro-2-hydroxy-2-methyl-propyl)acetamide, 2-[4-[[5-cyano-6-(2,2-difluoroethoxy)-1H-benzimidazol-2-yl]methyl]-1-oxo-phthalazin-2-yl]-N-[(2S)-3,3,3 -trifluoro-2-hydroxy-propyl] acetamide, 2-[4-[[5-cyano-6-(2,2-difluoroethoxy)-1H-benzimidazol-2-yl]methyl]-1-oxo-phthalazin-2-yl]-N-(2-hydroxy-3-methoxy-propyl)acetamide, 6-(2,2-difluoroethoxy)-2-[[3-[2-(4-hydroxy-1-piperidyl)-2-oxo-ethyl]-4-oxo-phthalazin-1-yl]methyl]-1H-benzimidazole-5 -carbonitrile, 2-[4-[[5-cyano-6-(2,2-difluoroethoxy)-1H-benzimidazol-2-yl]methyl]-1-oxo-phthalazin-2-yl]-N-(2,2-difluoroethyl)-N-(2-hydroxyethyl)acetamide, 2-[4-[[5-cyano-6-(2,2-difluoroethoxy)-1H-benzimidazol-2-yl]methyl]-1-oxo-phthalazin-2-yl]-N-(2-cyanopropyl)-N-methyl-acetamide, 2-[4-[[5-cyano-6-(2,2-difluoroethoxy)-1H-benzimidazol-2-yl]methyl]-1-oxo-phthalazin-2-yl]-N-(cyclopropylmethyl)-N-methyl-acetamide, 2-[4-[[5-cyano-6-(2,2-difluoroethoxy)-1H-benzimidazol-2-yl]methyl]-1-oxo-phthalazin-2-yl]-N-(2,2-difluoroethyl)-N-methyl-acetamide, 6-(2,2-difluoroethoxy)-2-[[3-[2-[(2S)-2-(hydroxymethyl)pyrrolidin-1-yl]-2-oxo-ethyl]-4-oxo-phthalazin-1-yl]methyl]-1H-benzimidazole-5-carbonitrile, 2-[4-[[5-cyano-6-(2,2-difluoroethoxy)-1H-benzimidazol-2-yl]methyl]-1-oxo-phthalazin-2-yl]-N-[[3-(hydroxymethyl)oxetan-3-yl]methyl]acetamide, 2-[[3-(2-morpholino-2-oxo-ethyl)-4-oxo-5,6,7,8-tetrahydrophthalazin-1-yl]methyl]-6-(2,2,2-trifluoroethoxy)-1H-benzimidazole-5-carbonitrile, 6-(2,2-difluoroethoxy)-2-[[3-[2-(3-hydroxy-3-methyl-pyrrolidin-1-yl)-2-oxo-ethyl]-4-oxo-phthalazin-1-yl]methyl]-3H-benzimidazole-5-carbonitrile, 2-[4-[[6-cyano-5-(2,2-difluoroethoxy)-1H-benzimidazol-2-yl]methyl]-1-oxo-phthalazin-2-yl]-N-(2,2-dimethylpropyl)acetamide, N-tert-butyl-2-[4-[[6-cyano-5-(2,2-difluoroethoxy)-1H-benzimidazol-2-yl]methyl]-1-oxo-5,6,7,8-tetrahydrophthalazin-2-yl] acetamide, 6-(2,2-diauoroethoxy)-2-[[3-[2-[3-(dimethylamino)pyrrolidin-1-yl]-2-oxo-ethyl]-4-oxo-5,6,7,8-tetrahydrophthalazin-1-yl]methyl]-1H-benzimidazole-5 -carbonitrile, 5-(2,2-difluoroethoxy)-2-[[3-(2-morpholino-2-oxo-ethyl)-4-oxo-phthalazin-1-yl]methyl]indazole-6-carbonitrile, 5-(2,2-difluoroethoxy)-2-[[3-[2-[(3R)-3-(dimethylamino)pyrrolidin-1-yl]-2-oxo-ethyl]-4-oxo-phthalazin-1-yl]methyl]indazole-6-carbonitrile, 5-(2,2-difluoroethoxy)-2-[[3-[2-[(3S)-3-(dimethylamino)pyrrolidin-1-yl]-2-oxo-ethyl]-4-oxo-phthalazin-1-yl]methyl]indazole-6-carbonitrile, 2-[4-[[6-cyano-5-(2,2-difluoroethoxy)indazol-2-yl]methyl]-1-oxo-phthalazin-2-yl]-N-cyclopentyl-acetamide, 5-(cyclopropylmethoxy)-2-[[3-(2-morpholino-2-oxo-ethyl)-4-oxo-phthalazin-1-yl]methyl]indazole-6-carbonitrile, 6-(cyclopropylmethoxy)-2-[[3-[2-(3-hydroxy-3-methyl-pyirrolidin-1-yl)-2-oxo-ethyl]-4-oxo-phthalazin-1-yl]methyl]imidazo[1,2-a]pyridine-7-carbonitrile, 6-(2,2-difluoroethoxy)-2-[[3-[2-[(3S,4S)-3,4-dihydroxypyrrolidin-1-yl]-2-oxo-ethyl]-4-oxo-phthalazin-1-yl]methyl]-3H-benzimidazole-5-carbonitrile, 6-(2,2-difluoroethoxy)-2-[[3-[2-(3,3-difluoro-4-hydroxy-1-piperidyl)-2-oxo-ethyl]-4-oxo-phthalazin-1-yl]methyl]-3H-benzimidazole-5-carbonitrile, 2-[[3-[2-(4-cyclopropyl-4-hydroxy-1-piperidyl)-2-oxo-ethyl]-4-oxo-phthalazin-1-yl]methyl]-6-(2,2-difluoroethoxy)-3H-benzimidazole-5-carbonitrile, 2-[[3-[2-(3-cyclopropyl-3-hydroxy-pyrrolidin-1-yl)-2-oxo-ethyl], -4-oxo-phthalazin-1-yl]methyl]-6-(2,2-difluoroethoxy)-3H-benzimidazole-5-carbonitrile, 2-[[3-[2-(3-cyclopropyl-3-hydroxy-azetidin-1-yl)-2-oxo-ethyl]-4-oxo-phthalazin-1-yl]methyl]-6-(2,2-difluoroethoxy)-3H-benzimidazole-5-carbonitrile, 6-(2,2-difluoroethoxy)-2-[[3-[2-[3-hydroxy-3-(trifluoromethyl)pyrrolidin-1-yl]-2-oxo-ethyl]-4-oxo-phthalazin-1-yl]methyl]-3H-benzimidazole-5-carbonitrile, 1-[[6-bromo-5-(cyclopropylmethoxy)-1H-benzimidazol-2-yl]methyl]-3-[2-[(3S,4S)-3,4-dihydroxypyrrolidin-1-yl]-2-oxo-ethyl]cinnolin-4-one, 2-[[3-[2-[4-(cyclopropylmethyl)piperazin-1-yl]-2-oxo-ethyl]-4-oxo-phthalazin-1-yl]methyl]-6-(2,2-difluoroethoxy)-3H-benzimidazole-5-carbonitrile, 2-[[3-[2-(4-cyano-1-piperidyl)-2-oxo-ethyl]-4-oxo-phthalazin-1-yl]methyl]-6-(2,2-difluoroethoxy)-3H-benzimidazole-5 -carbonitrile, 2-[4-[[6-cyano-5-(2,2-difluoroethoxy)-1H-benzimidazol-2-yl]methyl]-1-oxo-phthalazin-2-yl]-N-methyl-N-(2-methylsulfonylethyl)acetamide, 6-(2,2-difluoroethoxy)-2-[[3-[2-(3-methylsulfonylpyrrolidin-1-yl)-2-oxo-ethyl]-4-oxo-phthalazin-1-yl]methyl]-3H-benzimidazole-5-carbonitrile, 2-[4-[[6-cyano-5-(2,2-difluoroethoxy)-1H-benzimidazol-2-yl]methyl]-1-oxo-phthalazin-2-yl]-N-(2-hydroxy-3,3 -dimethyl-butyl)acetamide, 2-[4-[[6-cyano-5-(2,2-difluoroethoxy)-1H-benzimidazol-2-yl]methyl]-1-oxo-phthalazin-2-yl]-N-(2,2-difluoroethyl)acetamide, 2-[4-[[6-cyano-5-(2,2-diauoroethoxy)-1H-benzimidazol-2-yl]methyl]-1-oxo-phthalazin-2-yl]-N-(3,3,3-trifluoropropyl)acetamide, 6-(2,2-difluoroethoxy)-2-[[3-[2-[4-(2-hydroxyethyl)piperazin-1-yl]-2-oxo-ethyl]-4-oxo-phthalazin-1-yl]methyl]-3H-benzimidazole-5-carbonitrile, N-[cyano(cyclopropyl)methyl]-2-[4-[[6-cyano-5-(2,2-difluoroethoxy)-1H-benzimidazol-2-yl]methyl]-1-oxo-phthalazin-2-yl] acetamide, 6-(2,2-difluoroethoxy)-2-[[3-[2-(2,2-dimethylmorpholin-4-yl)-2-oxo-ethyl]-4-oxo-phthalazin-1-yl]methyl]-3H-benzimidazole-5-carbonitrile, 2-[4-[[6-cyano-5-(2,2-difluoroethoxy)-1H-benzimidazol-2-yl]methyl]-1-oxo-phthalazin-2-yl]-N-(2,2-difluoro-3 -hydroxy-propyl)acetamide, 2-[4-[[6-cyano-5-(2,2-difluoroethoxy)-1H-benzimidazol-2-yl]methyl]-1-oxo-phthalazin-2-yl]-N-(2-sulfamoylethyl)acetamide, 2-[4-[[6-cyano-5-(2,2-difluoroethoxy)-1H-benzimidazol-2-yl]methyl]-1-oxo-phthalazin-2-yl]-N-(2,2,2-trifluoroethyl)acetamide, 6-(2,2-difluoroethoxy)-2-[[3-[2-(3-hydroxy-1-piperidyl)-2-oxo-ethyl]-4-oxo-phthalazin-1-yl]methyl]-3H-benzimidazole-5 -carbonitrile, 2-[4-[[6-cyano-5-(2,2-difluoroethoxy)-1H-benzimidazol-2-yl]methyl]-1-oxo-phthalazin-2-yl]-N-[(3,3-difluorocyclobutyl)methyl]acetamide, 6-(2,2-difluoroethoxy)-2-[[3-[2-(3,3-dimethylpyrrolidin-1-yl)-2-oxo-ethyl]-4-oxo-phthalazin-1-yl]methyl]-3H-benzimidazole-5-carbonitrile, 6-(2,2-difluoroethoxy)-2-[[3-[2-(4,4-dimethyl-1-piperidyl)-2-oxo-ethyl]-4-oxo-phthalazin-1-yl]methyl]-3H-benzimidazole-5-carbonitrile, 2-[[3-[2-(2,2-difluoro-5-aza-spiro[2.4]heptan-5-yl)-2-oxo-ethyl]-4-oxo-phthalazin-1-yl]methyl]-6-(2,2-difluoroethoxy)-3H-benzimidazole-5-carbonitrile, 1-[2-[4-[[6-cyano-5-(2,2-difluoroethoxy)-1H-benzimidazol-2-yl]methyl]-1-oxo-phthalazin-2-yl]acetyl]-N,N-dimethyl-piperidine-4-carboxamide, 6-(2,2-difluoroethoxy)-2-[[3-[2-[3-(1-hydroxy-1-methyl-ethyl)-1-piperidyl]-2-oxo-ethyl]-4-oxo-phthalazin-1-yl]methyl]-3H-benzimidazole-5-carbonitrile, 6-(2,2-difluoroethoxy)-2-[[3-[2-(4-morpholino-1-piperidyl)-2-oxo-ethyl]-4-oxo-phthalazin-1-yl]methyl]-3H-benzimidazole-5-carbonitrile, 6-(2,2-difluoroethoxy)-2-[[3-[2-(7-oxa-2-azaspiro[3.5]nonan-2-yl)-2-oxo-ethyl]-4-oxo-phthalazin-1-yl]methyl]-3H-benzimidazole-5-carbonitrile, 6-(2,2-difluoroethoxy)-2-[[3-[2-(4-ethylsulfonyl-1-piperidyl)-2-oxo-ethyl]-4-oxo-phthalazin-1-yl]methyl]-3H-benzimidazole-5-carbonitrile, 2-[4-[[6-cyano-5-(2,2-difluoroethoxy)-1H-benzimidazol-2-yl]methyl]-1-oxo-phthalazin-2-yl]-N-[2-(dimethylamino)-2-oxo-ethyl]-N-methyl-acetamide, 2-[4-[[6-chloro-5-(cyclopropylmethoxy)-1H-benzimidazol-2-yl]methyl]-1-oxo-phthalazin-2-yl]-N-methylsulfonyl-acetamide, 2-[4-[[6-chloro-5-(cyclopropylmethoxy)indazol-2-yl]methyl]-1-oxo-phthalazin-2-yl]-N-methylsulfonyl-acetamide, 2-[4-[[6-chloro-5-(cyclopropylmethoxy)indazol-2-yl]methyl]-1-oxo-phthalazin-2-yl]-N-cyclopropylsulfonyl-acetamide, 2-[4-[[6-chloro-5-(2,2-difluoroethoxy)-1H-benzimidazol-2-yl]methyl]-6-cyano-1-oxo-phthalazin-2-yl]-N-methylsulfonyl-acetamide, N-{2-[4-(6-Chloro-5-cyclopropylmethoxy-1H-benzoimidazol-2-ylmethyl)-1-oxo-1H-phthalazin-2-yl]-propionyl}-methanesulfonamide, 2-[4-[(5-ethoxy-6-fluoro-1H-benzimidazol-2-yl)methyl]-1-oxo-phthalazin-2-yl]-N-methylsulfonylacetamide, 2-[4-[[5-(2,2-difluoroethoxy)-6-fluoro-1H-benzimidazol-2-yl]methyl]-1-oxo-phthalazin-2-yl]-N-methylsulfonyl-acetamide, 2-[4-[[6-chloro-5-[(1-cyanocyclopropyl)methoxy]-1H-benzimidazol-2-yl]methyl]-1-oxo-phthalazin-2-yl]-N-methylsulfonyl-acetamide, 2-[4-[[6-chloro-5-[(1-methylcyclopropyl)methoxy]-1H-benzimidazol-2-yl]methyl]-1-oxo-phthalazin-2-yl]-N-methylsulfonyl-acetamide, 2-[4-[(6-chloro-5-ethoxy-indazol-2-yl)methyl]-1-oxo-phthalazin-2-yl]acetic acid, 2-[4-[[6-chloro-5-(2,2,2-trifluoroethoxy)indazol-2-yl]methyl]-1-oxo-phthalazin-2-yl]acetic acid, 2-[4-[[6-chloro-5-(2,2-difluoroethoxy)indazol-2-yl]methyl]-1-oxo-phthalazin-2-yl]acetic acid, 2-[4-[(4-chloro-5-ethoxy-indazol-2-yl)methyl]-1-oxo-phthalazin-2-yl]acetic acid, 2-[4-[(5-chloro-6-ethoxy-indazol-2-yl)methyl]-1-oxo-phthalazin-2-yl]acetic acid, 6-(2,2-difluoroethoxy)-2-[[3-(2-morpholino-2-oxo-ethyl)-4-oxo-phthalazin-1-yl]methyl]imidazo[1,2-a]pyridine-7-carbonitrile, and 2-[4-[[6-chloro-5-(cyclopropylmethoxy)-1H-imidazo[4,5-b]pyridin-2-yl]methyl]-1-oxo-phthalazin-2-yl]acetic acid.

**12.** A pharmaceutical composition comprising a compound or a pharmaceutically acceptable salt thereof according to any one of claims 1-11, and a pharmaceutically acceptable carrier.

**13.** A pharmaceutical composition according to claim 12 comprising a further therapeutic agent.

**14.** A compound or a pharmaceutically acceptable salt thereof, according to any one of claims 1-11, or a pharmaceutical composition according to claim 12 or 13 for use in medicine.

**15.** A compound or a pharmaceutically acceptable salt thereof, according to any one of claims 1-11, or a pharmaceutical composition according to claim 12 or 13 for use in the prophylaxis and/or treatment of fibrotic diseases, inflammatory diseases, respiratory diseases, autoimmune diseases, metabolic diseases, cardiovascular diseases, and/or proliferative diseases.

**16.** A pharmaceutical composition according to claim 13, wherein the further therapeutic agent is an agent for the prophylaxis and/or treatment of fibrotic diseases, inflammatory diseases, respiratory diseases, autoimmune diseases, metabolic diseases, cardiovascular diseases, and/or proliferative diseases.

## Patentansprüche

**1.** Verbindung oder pharmazeutisch annehmbares Salz oder Solvat oder das pharmazeutisch annehmbare Salz eines Solvats davon, nach der Formel I:

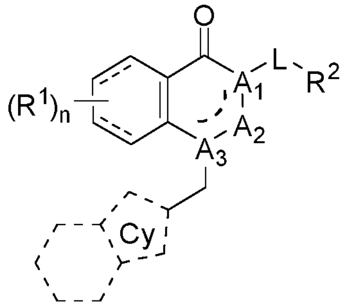

I

wobei

jedes $A_1$, $A_2$ und $A_3$ unabhängig aus C und N ausgewählt ist, vorausgesetzt, dass $A_1$, $A_2$ und $A_3$ nicht gleichzeitig C oder N sind;
jedes $R_1$ unabhängig ausgewählt ist aus

- $C_{1-4}$-Alkyl,
- $C_{1-4}$-Alkoxy,
- $C_{3-6}$-Cycloalkyl,
- 4-7-gliedrigem monocyclischem Heteroaryl, umfassend 1, 2 oder 3 Heteroatome, die unabhängig ausgewählt sind aus N, O oder S, gegebenenfalls substituiert mit einer oder zwei =O-Gruppen,
- $-S(O)_2C_{1-4}$-Alkyl,
- -CN,
- $-C(=O)NH_2$, und
- Halo;

der tiefgestellte Index n 0, 1 oder 2 ist;
Cy ein 5-gliedriges ringverknüpftes 9-gliedriges kondensiertes 5-6-bicyclisches Heteroaryl ist, das 1, 2 oder 3 N-Atome umfasst, wobei das Heteroaryl mit einer $R^3$-Gruppe und einer $R^{4a}$-Gruppe substituiert ist und gegebenenfalls ferner mit einem Halo oder OH substituiert ist;
$R^3$ $C_{1-6}$-Alkoxy ist, gegebenenfalls substituiert mit einem oder mehreren unabhängig ausgewählten

- Halo,
- $C_{1-4}$-Alkoxy, oder
- $C_{3-7}$-Cycloalkyl, gegebenenfalls substituiert mit einem $C_{1-4}$-Alkyl, Halo oder -CN;

$R^{4a}$

- $C_{1-4}$-Alkyl, gegebenenfalls substituiert mit einem oder mehreren Halo,
- Halo, oder
- -CN ist;

L abwesend ist oder $-CR^{5a}R^{5b}-$ ist;
$R^2$

- -C(=O)OH,
- $-C(=O)NR^{6a}R^{6b}$,
- $-C(O)NHS(O)_2-C_{1-4}$-Alkyl,
- $-C(O)NHS(O)_2-C_{3-7}$-Cycloalkyl,
- $-Cy_1$, oder
- $-C(=O)Cy_2$ ist;

jedes $R^{5a}$ und $R^{5b}$ unabhängig ausgewählt ist aus

- H,

- $C_{1-4}$-Alkyl, gegebenenfalls substituiert mit einem, zwei oder drei Halo oder -$NR^{8a}R^{8b}$, und
- $C_{1-4}$-Alkoxy;

jedes $R^{6a}$ und $R^{6b}$ unabhängig ausgewählt ist aus

- H,
- $C_{1-6}$-Alkyl, gegebenenfalls substituiert von einem weiteren unabhängig ausgewählten
  - OH,
  - -CN,
  - Halo,
  - $C_{1-4}$-Alkoxy,
  - monocyclischen 4-6-gliedrigen Heterocycloalkyl, umfassend ein oder zwei Heteroatome, die ausgewählt sind aus N, O und S, gegebenenfalls substituiert mit einem -$CH_2$-OH
  - -$S(O)_2C_{1-4}$-Alkyl,
  - -$S(O)_2NH_2$,
  - -C(O)$NR^{9a}R^{9b}$, oder
  - $C_{3-7}$-Cycloalkyl, gegebenenfalls substituiert mit OH, oder Halo;
- $C_{1-4}$-Alkoxy,
- $C_{3-7}$-Cycloalkyl, gegebenenfalls substituiert mit einem oder mehreren OH, und
- monocyclischem 4-6-gliedrigem Heterocycloalkyl, umfassend ein oder zwei Heteroatome, die ausgewählt sind aus N, O und S, gegebenenfalls substituiert mit einem oder zwei Oxo;

$Cy_1$

- $C_{3-7}$ monocyclisches Cycloalkyl, gegebenenfalls substituiert mit einem -C(=O)OH,
- monocyclisches 4-6-gliedriges Heterocycloalkyl ist, umfassend ein oder zwei Heteroatome, die ausgewählt sind aus N, O und S, gegebenenfalls substituiert mit einem oder zwei
o $C_{1-4}$-Alkyl, gegebenenfalls substituiert mit einem -C(=O)OH,

$Cy_2$

- N-verknüpftes monocyclisches 4-7-gliedriges Heterocycloalkyl ist, umfassend mindestens ein N-Atom, und gegebenenfalls ein oder zwei Heteroatome, die ausgewählt sind aus N, O und S, gegebenenfalls substituiert mit einem oder mehreren
  - OH,
  - Oxo,
  - -CN,
  - Halo,
  - $C_{1-4}$-Alkoxy,
  - $C_{1-4}$-Alkyl, gegebenenfalls substituiert mit einem oder mehreren unabhängig ausgewählten
    - Halo,
    - OH,
  - $C_{3-7}$-Cycloalkyl,
  - -$S(O)_2C_{1-4}$-Alkyl, oder
  - -$NR^{7a}R^{7b}$; oder
- N-verknüpftes spirocyclisches 7-9-gliedriges Heterocycloalkyl ist, umfassend mindestens ein N-Atom und gegebenenfalls ein oder zwei Heteroatome, die ausgewählt sind aus N, O und S, gegebenenfalls substituiert mit einem oder mehreren Halo;

jedes $R^{7a}$, $R^{7b}$, $R^{8a}$, $R^{8b}$, $R^{9a}$ und $R^{9b}$ unabhängig ausgewählt ist aus H und $C_{1-4}$-Alkyl;
mit der Maßgabe, dass

- wenn L abwesend ist, $A_1$ und $A_2$ C sind, $A_3$ N ist, $R^3$ unsubstituiertes $C_{1-6}$-Alkoxy oder mit Halogen substituiertes $C_{1-6}$-Alkoxy ist, dann $R^2$ nicht COOH ist; und
- wenn $A_1$ und $A_2$ N sind, $A_3$ C ist, und $R^{4a}$ $-CH_3$ ist, dann $R^3$ nicht unsubstituiertes $C_{1-4}$-Alkoxy ist.

**2.** Verbindung oder pharmazeutisch annehmbares Salz davon nach Anspruch 1, wobei der tiefgestellte Index n 0 ist.

**3.** Verbindung oder pharmazeutisch annehmbares Salz davon nach Anspruch 1 oder 2, wobei wobei Cy ausgewählt ist aus $Cy_A$, $Cy_B$, $Cy_c$ und $Cy_D$:

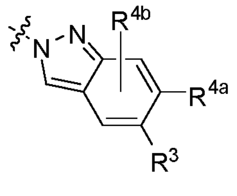
$Cy_A$,
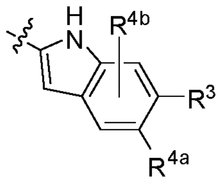
$Cy_B$,
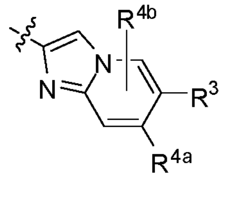
$Cy_C$,
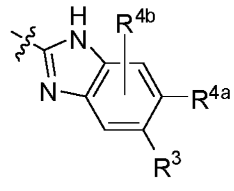
$Cy_D$.

**4.** Verbindung oder pharmazeutisch annehmbares Salz davon nach Anspruch 3, wobei $R^3$ $-OCH_3$, $-OCH_2CH_3$, $-OCF_3$, $-OCH_2CF_3$, $-OCH_2CHF_2$, $-OCH_2CH_2OCH_3$,

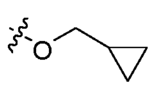
,
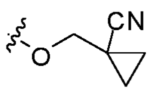
, oder
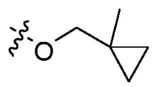

ist.

**5.** Verbindung oder pharmazeutisch annehmbares Salz davon nach Anspruch 3 oder 4, wobei $R^{4b}$ H, F, Cl oder OH ist.

**6.** Verbindung oder pharmazeutisch annehmbares Salz davon nach Anspruch 3 oder 4, wobei $R^{4a}$ F, Cl, -CN oder $-CF_3$ ist.

**7.** Verbindung oder pharmazeutisch annehmbares Salz davon nach Anspruch 1, wobei die Verbindung der Formel Va, Vb, Vc, VIa, VIb oder VIc entspricht:

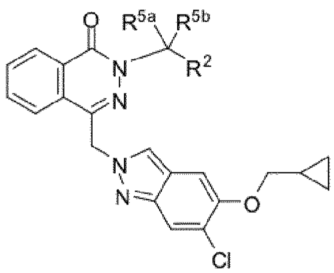
Va,
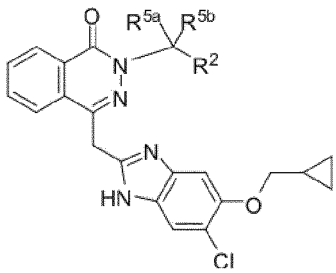
Vb,
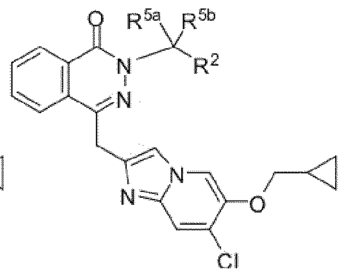
Vc

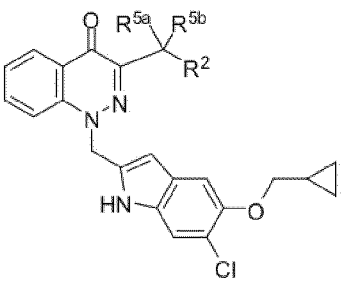 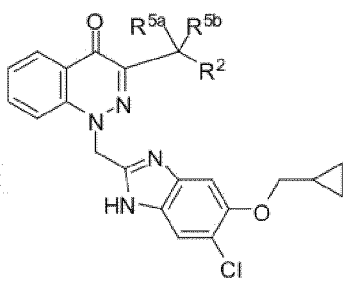 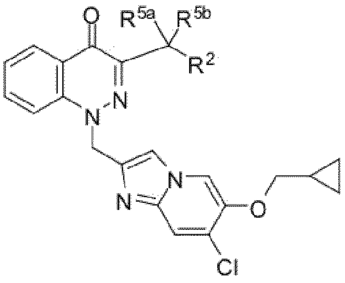

VIa, VIb, oder VIc.

8. Verbindung oder pharmazeutisch annehmbares Salz davon nach Anspruch 3 oder 4, wobei wobei jedes $R^{5a}$ und $R^{5b}$ unabhängig ausgewählt ist aus H, $-OCH_3$, $-OCH_2CH_3$, $-CH_3$, $-CH_2CH_3$, $-CH_3$, $CH_2CHF_2$, $-CH_2CF_3$ und $-CH_2CH_2-N(CH_3)_2$.

9. Verbindung oder pharmazeutisch annehmbares Salz davon nach Anspruch 1, wobei die Verbindung der Formel VIIa, VIIb oder VIIc entspricht:

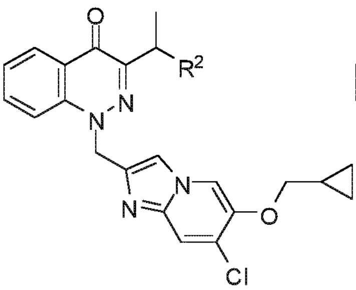 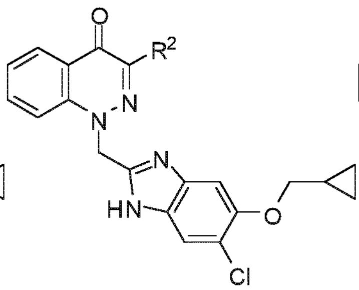 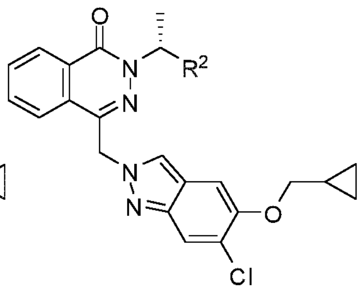

VIIa, VIIb, oder VIIc

10. Verbindung oder pharmazeutisch annehmbares Salz davon nach einem der Ansprüche 1-9, wobei $R^2$ -C(=O)OH, $-C(O)NHS(O)_2-CH_3$, $-C(O)NHS(O)_2-C_{3-7}$-Cycloalkyl,

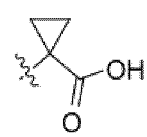 oder 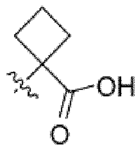 ist.

11. Verbindung oder pharmazeutisch annehmbares Salz davon, wobei die Verbindung nach Anspruch 1 ausgewählt ist aus:

6-(2,2-Difluorethoxy)-2-[[4-oxo-3-(pyrrolidin-2-ylmethyl)phthalazin-1-yl]methyl]-3H-benzimidazol-5-carbonitril,
2-(Azetidin-3-yl)-4-[[6-Chlor-5-(cyclopropylmethoxy)indazol-2-yl]methyl]phthalazin-1-on,
2-[4-[[6-Chlor-5-(cyclopropylmethoxy)indazol-2-yl]methyl]-1-oxo-phthalazin-2-yl]propansäure,
2-[1-[[7-Chlor-6-(cyclopropylmethoxy)imidazo[1,2-a]pyridin-2-yl]methyl]-4-oxocinnolin-3-yl]propansäure,
1-[[6-Chlor-5-(cyclopropylmethoxy)-1H-benzimidazol-2-yl]methyl]-4-oxo-cinnolin-3-carbonsäure,
2-[4-[(5-Chlor-6-ethoxy-1H-benzimidazol-2-yl)methyl]-1-oxo-phthalazin-2-yl]essigsäure,
2-[4-[[6-Cyano-5-(cyclopropylmethoxy)-1H-benzimidazol-2-yl]methyl]-1-oxo-phthalazin-2-yl]essigsäure,
2-[4-[[6-Cyano-5-(2,2-difluorethoxy)indazol-2-yl]methyl]-1-oxo-phthalazin-2-yl]essigsäure,
2-[4-[[5-Chlor-6-(2,2-difluorethoxy)-1H-indol-2-yl]methyl]-1-oxo-phthalazin-2-yl]essigsäure,
6-(Cyclopropylmethoxy)-2-[[3-[2-[(3S,4S)-3,4-dihydroxypyrrolidin-1-yl]-2-oxo-ethyl]-4-oxo-cinnolin-1-yl]methyl]-3H-benzimidazol-5-carbonitril,
4-[[5-Chlor-6-(2,2-difluorethoxy)-1H-indol-2-yl]methyl]-2-(2-morpholino-2-oxo-ethyl)phthalazin-1-on,

4-[[6-Chlor-5-(2,2-difluorethoxy)-1H-benzimidazol-2-yl]methyl]-2-(2-morpholino-2-oxo-ethyl)-1-oxo-phthalazin-6-carboxamid,
6-(2,2-Difluorethoxy)-2-[[3-[2-[(3S)-3-hydroxypyrrolidin-1-yl]-2-oxo-ethyl]-4-oxo-phthalazin-1-yl]methyl]-3H-benzimidazol-5-carbonitril,
2-[(1-Acetylpyrrolidin-2-yl)methyl]-4-[[5-Chlor-6-(trifluormethoxy)-1H-benzimidazol-2-yl]methyl]phthalazin-1-on,
2-[1-[[7-Chlor-6-(cyclopropylmethoxy)imidazo[1,2-a]pyridin-2-yl]methyl]-4-oxo-cinnolin-3-yl]-N-methylsulfonylpropanamid,
2-[1-[[5-Chlor-6-(cyclopropylmethoxy)-1H-indol-2-yl]methyl]-4-oxo-cinnolin-3-yl]essigsäure,
((2R)-2-[4-[[6-Chlor-5-(cyclopropylmethoxy)indazol-2-yl]methyl]-1-oxo-phthalazin-2-yl]propansäure),
(2S)-2-[4-[[6-Chlor-5-(cyclopropylmethoxy)indazol-2-yl]methyl]-1-oxo-phthalazin-2-yl]propansäure,
(2S)-2-[1-[[7-Chlor-6-(cyclopropylmethoxy)imidazo[1,2-a]pyridin-2-yl]methyl]-4-oxocinnolin-3-yl]propansäure,
(2R)-2-[1-[[7-Chlor-6-(cyclopropylmethoxy)imidazo[1,2-a]pyridin-2-yl]methyl]-4-oxocinnolin-3-yl]propansäure,
(2-[1-[[5-Cyano-6-(cyclopropylmethoxy)-1H-benzimidazol-2-yl]methyl]-4-oxocinnolin-3-yl]-3-(dimethylamino)propansäure),
2-[4-[(7-Chlor-6-ethoxy-indazol-2-yl)methyl]-1-oxo-phthalazin-2-yl]essigsäure, 2-[4-[[5-Chlor-6-(2,2-difluorethoxy)-1H-benzimidazol-2-yl]methyl]-6-cyano-1-oxo-phthalazin-2-yl]essigsäure,
4-[[5-Chlor-6-(trifluormethoxy)-1H-benzimidazol-2-yl]methyl]-2-cyclopentyl-phthalazin-1-on,
4-[[5-Chlor-6-(trifluormethoxy)-1H-benzimidazol-2-yl]methyl]-2-cyclopropyl-phthalazin-1-on,
4-[[5-Chlor-6-(trifluormethoxy)-1H-benzimidazol-2-yl]methyl]-2-cyclobutyl-phthalazin-1-on,
4-[[6-Chlor-5-(trifluormethoxy)-1H-benzimidazol-2-yl]methyl]-2-(cyclopropylmethyl)phthalazin-1-on,
4-[[6-Chlor-5-(trifluormethoxy)-1H-benzimidazol-2-yl]methyl]-2-(cyclobutylmethyl)phthalazin-1-on,
4-[[5-Chlor-6-(2,2-difluorethoxy)-1H-benzimidazol-2-yl]methyl]-6-methoxy-2-(2-morpholino-2-oxo-ethyl)phthalazin-1-on,
4-[[5-Chlor-6-(2,2-difluorethoxy)-1H-benzimidazol-2-yl]methyl]-7-methoxy-2-(2-morpholino-2-oxo-ethyl)phthalazin-1-on,
4-[[5-Chlor-6-(2,2-difluorethoxy)-1H-benzimidazol-2-yl]methyl]-2-cyclopropyl-phthalazin-1-on,
4-[[5-Chlor-6-(2,2-difluorethoxy)-1H-benzimidazol-2-yl]methyl]-7-morpholino-2-(2-morpholino-2-oxo-ethyl)phthalazin-1-on,
4-[[6-Chlor-5-(trifluormethoxy)-1H-benzimidazol-2-yl]methyl]-2-(THF-2-ylmethyl)phthalazin-1-on,
4-[[6-Chlor-5-(trifluormethoxy)-1H-benzimidazol-2-yl]methyl]-2-(tetrahydropyran-2-ylmethyl)phthalazin-1-on,
4-[[6-Chlor-5-(trifluormethoxy)-1H-benzimidazol-2-yl]methyl]-2-[(3-methyloxetan-3-yl)methyl]phthalazin-1-on,
4-[[6-Chlor-5-(trifluormethoxy)-1H-benzimidazol-2-yl]methyl]-2-[(2,2-dimethyl-1,3-dioxolan-4-yl)methyl]phthalazin-1-on,
4-[[6-Chlor-5-(trifluormethoxy)-1H-benzimidazol-2-yl]methyl]-2-[(1-Methyl-3-piperidyl)methyl]phthalazin-1-on,
1-[[5-Chlor-6-(2,2-difluorethoxy)-1H-benzimidazol-2-yl]methyl]-3-(2-morpholino-2-oxo-ethyl)-4-oxo-phthalazin-6-carbonitril,
4-[[5-Chlor-6-(2,2-difluorethoxy)-1H-benzimidazol-2-yl]methyl]-6-methylsulfonyl-2-(2-morpholino-2-oxo-ethyl)phthalazin-1-on,
6-(Cyclopropylmethoxy)-2-[(3-Cyclopropyl-4-oxo-phthalazin-1-yl)methyl]-1H-benzimidazol-5-carbonitril,
4-[[6-Chlor-5-(trifluormethoxy)-1H-benzimidazol-2-yl]methyl]-2-[(2,2-difluorcyclopropyl)methyl]phthalazin-1-on,
4-[[6-Chlor-5-(trifluormethoxy)-1H-benzimidazol-2-yl]methyl]-2-(3,3-dimethyl-2-oxo-butyl)phthalazin-1-on,
1-[[4-[[5-Chlor-6-(trifluormethoxy)-1H-benzimidazol-2-yl]methyl]-1-oxo-phthalazin-2-yl]methyl]cyclopropancarbonsäure,
4-[[6-Chlor-5-(2,2-difluorethoxy)-1H-benzimidazol-2-yl]methyl]-5-cyclopropyl-2-(2-morpholino-2-oxo-ethyl)phthalazin-1-on,
6-(2,2-Difluorethoxy)-2-[3-(2-morpholin-4-yl-2-oxo-ethyl)-4-oxo-4H-cinnolin-1-ylmethyl]-3H-enzoimidazol-5-carbonitril,
1-[5-(2,2-Difluorethoxy)-6-fluor,
-1H-benzimidazol-2-ylmethyl]-3-(2-morpholin-4-yl-2-oxo-ethyl)-1H-cinnolin-4-on,
6-(2-Methoxyethoxy)-2-[[3-(2-morpholino-2-oxo-ethyl)-4-oxo-phthalazin-1-yl]methyl]-3H-benzimidazol-5-carbonitril,
6-(2,2-Difluorpropoxy)-2-[[3-(2-morpholino-2-oxo-ethyl)-4-oxo-phthalazin-1-yl]methyl]-3H-benzimidazol-5-carbonitril,
7-Chlor-6-(2,2-difluorethoxy)-2-[[3-(2-morpholino-2-oxo-ethyl)-4-oxo-phthalazin-1-yl]methyl]-3H-benzimidazol-5-carbonitril,
2-[[8-Cyclopropyl-3-(2-morpholino-2-oxo-ethyl)-4-oxo-phthalazin-1-yl]methyl]-6-(2,2-difluorethoxy)-3H-benzimidazol-5-carbonitril,
4-[[6-Chlor-5-(trifluormethoxy)-1H-benzimidazol-2-yl]methyl]-2-(pyrrolidin-2-ylmethyl)phthalazin-1-on,
4-[[6-Chlor-5-(trifluormethoxy)-1H-benzimidazol-2-yl]methyl]-2-(4-piperidylmethyl)phthalazin-1-on,
4-[[6-Chlor-5-(trifluormethoxy)-1H-benzimidazol-2-yl]methyl]-2-(pyrrolidin-3-ylmethyl)phthalazin-1-on,
4-[[6-Chlor-5-(trifluormethoxy)-1H-benzimidazol-2-yl]methyl]-2-(3-piperidylmethyl)phthalazin-1-on,
4-[[6-Chlor-5-(trifluormethoxy)-1H-benzimidazol-2-yl]methyl]-2-pyrrolidin-3-yl-phthalazin-1-on,
2-(Azetidin-3-ylmethyl)-4-[[6-chlor-5-(trifluormethoxy)-1H-benzimidazol-2-yl]methyl]phthalazin-1-on,
4-[[6-Chlor-5-(trifluormethoxy)-1H-benzimidazol-2-yl]methyl]-2-(4-piperidyl)phthalazin-1-on,
4-[[5-Chlor-6-(trifluormethoxy)-1H-benzimidazol-2-yl]methyl]-2-(morpholin-2-ylmethyl)-phthalazin-1-on,
6-(2,2-Difluorethoxy)-2-[(4-oxo-3-pyrrolidin-3-yl-phthalazin-1-yl)methyl]-3H-benzimidazol-5-carbonitril,
6-(2-Methoxyethoxy)-2-[(4-Oxo-3-pyrrolidin-3-yl-phthalazin-1-yl)methyl]-3H-benzimidazol-5-carbonitril,
6-(2,2-Difluorethoxy)-2-[[8-methyl-3-(2-morpholino-2-oxo-ethyl)-4-oxo-phthalazin-1-yl]methyl]-3H-benzimidazol-5-carbonitril,
4-[[6-Chlor-5-(2-methoxyethoxy)-1H-benzimidazol-2-yl]methyl]-2-pyrrolidin-3-yl-phthalazin-1-one,
2-[[3-(Azetidin-3-yl)-4-oxo-phthalazin-1-yl]methyl]-6-(2,2-difluorethoxy)-3H-benzimidazol-5-carbonitril,
2-[3-[4-[[6-Cyano-5-(2,2-difluorethoxy)-1H-benzimidazol-2-yl]methyl]-1-oxo-phthalazin-2-yl]azetidin-1-yl]essigsäure,
2-[2-[[4-[[6-Cyano-5-(2,2-difluorethoxy)-1H-benzimidazol-2-yl]methyl]-1-oxo-phthalazin-2-yl]methyl]pyrrolidin-1-yl]essigsäure,
2-[3-[4-[[6-Cyano-5-(2,2-difluorethoxy)-1H-benzimidazol-2-yl]methyl]-1-oxo-phthalazin-2-yl]pyrrolidin-1-yl]essigsäure,
6-(2,2-Difluorethoxy)-2-[[3-(1-methylpyrrolidin-3-yl)-4-oxo-phthalazin-1-yl]methyl]-3H-benzimidazol-5-carbonitril,
2-[3-[4-[[7-Cyano-6-(cyclopropylmethoxy)imidazo[1,2-a]pyridin-2-yl]methyl]-1-oxo-phthalazin-2-yl]azetidin-1-yl]essigsäure,
2-[4-[[6-Cyano-5-(2,2-difluorethoxy)-1H-benzimidazol-2-yl]methyl]-1-oxo-phthalazin-2-yl]-3-(dimethylamino)propansäure,
3-(Azetidin-1-yl)-2-[4-[[6-cyano-5-(2,2-difluorethoxy)-1H-benzimidazol-2-yl]methyl]-1-oxo-phthalazin-2-yl]propionsäure,
2-[4-[[6-Chlor-5-(2,2-difluorethoxy)indazol-2-yl]methyl]-1-oxo-phthalazin-2-yl]propionsäure,
2-[4-[(6-Chlor-5-ethoxy-1H-benzimidazol-2-yl)methyl]-8-methyl-1-oxo-phthalazin-2-yl]essigsäure,
{1-[6-Chlor-5-(2,2-difluorethoxy)-1H-benzi midazol-2-ylmethyl]-4-oxo-1,4-dihydrocinnolin-3-yl}-essigsäure,
2-[4-[[7-Cyano-6-(cyclopropylmethoxy)imidazo[1,2-a]pyridin-2-yl]methyl]-1-oxo-phthalazin-2-yl]essigsäure,
1-[[6-Cyano-5-(2,2-difluorethoxy)-1H-benzimidazol-2-yl]methyl]-4-oxo-chinolin-3-carbonsäure,
2-[4-[[7-Cyano-6-(cyclopropylmethoxy)imidazo[1,2-a]pyridin-2-yl]methyl]-1-oxo-phthalazin-2-yl]propansäure,
2-[4-[[7-Chlor-6-(cyclopropylmethoxy)imidazo[1,2-a]pyridin-2-yl]methyl]-1-oxo-phthalazin-2-yl]essigsäure,
2-[4-[[7-Chlor-6-(cyclopropylmethoxy)imidazo[1,2-a]pyridin-2-yl]methyl]-1-oxo-phthalazin-2-yl]propansäure,
1-[[7-Chlor-6-(cyclopropylmethoxy)imidazo[1,2-a]pyridin-2-yl]methyl]-4-oxo-chinolin-3-carbonsäure,
1-[[7-Chlor-6-(cyclopropylmethoxy)imidazo[1,2-a]pyridin-2-yl]methyl]-4-oxo-cinnolin-3-carbonsäure,
2-[4-[[7-Chlor-6-(cyclopropylmethoxy)imidazo[1,2-a]pyridin-2-yl]methyl]-6-fluor-1-oxo-phthalazin-2-yl]propansäure,
2-[3-[4-[[7-Chlor-6-(cyclopropylmethoxy)imidazo[1,2-a]pyridin-2-yl]methyl]-1-oxo-phthalazin-2-yl]pyrrolidin-1-yl]essigsäure,
2-[1-[[7-Chlor-6-(cyclopropylmethoxy)imidazo[1,2-a]pyridin-2-yl]methyl]-4-oxo-cinnolin-3-yl]-2-methylpropionsäure,
2-[1-[[7-Chlor-6-(cyclopropylmethoxy)imidazo[1,2-a]pyridin-2-yl]methyl]-4-oxo-cinnolin-3-yl]essigsäure,
1-[[6-Chlor-5-(cyclopropylmethoxy)-1H-benzimidazol-2-yl]methyl]-4-oxo-chinolin-3-carbonsäure,
2-[4-[[6-Chlor-5-(cyclopropylmethoxy)indazol-2-yl]methyl]-1-oxo-phthalazin-2-yl]cyclobutancarbonsäure,
2-[4-[[6-Chlor-5-(cyclopropylmethoxy)indazol-2-yl]methyl]-6-cyano-1-oxo-phthalazin-2-yl]propansäure,
2-[4-[[6-Chlor-5-(cyclopropylmethoxy)indazol-2-yl]methyl]-1-oxo-5,6,7,8-tetrahydrophthalazin-2-yl]propansäure,
2-[1-[[7-Chlor-6-(cyclopropylmethoxy)imidazo[1,2-a]pyridin-2-yl]methyl]-4-oxo-cinnolin-3-yl]-2-methoxyessigsäure,
1-[4-[[6-Chlor-5-(cyclopropylmethoxy)indazol-2-yl]methyl]-1-oxo-phthalazin-2-yl]cyclopropancarbonsäure,
1-[1-[[7-Chlor-6-(cyclopropylmethoxy)imidazo[1,2-a]pyridin-2-yl]methyl]-4-oxo-cinnolin-3-yl]cyclopropancarbonsäure,
2-[4-[[6-Chlor-5-(cyclopropylmethoxy)indazol-2-yl]methyl]-7-methoxy-1-oxo-phthalazin-2-yl]propansäure,
2-[1-[[7-Chlor-6-(cyclopropylmethoxy)imidazo[1,2-a]pyridin-2-yl]methyl]-4-oxo-cinnolin-3-yl]butansäure,

2-[1-[[6-(Cyclopropylmethoxy)-7-(trifluormethyl)imidazo[1,2-a]pyridin-2-yl]methyl]-4-oxo-cinnolin-3-yl]essigsäure,
2-[1-[[6-(Cyclopropylmethoxy)-7-(trifluormethyl)imidazo[1,2-a]pyridin-2-yl]methyl]-4-oxo-cinnolin-3-yl]propansäure,
2-[1-[[7-Chlor-6-(cyclopropylmethoxy)imidazo[1,2-a]pyridin-2-yl]methyl]-7-fluor-4-oxo-cinnolin-3-yl]essigsäure,
2-[1-[[7-Chlor-6-(cyclopropylmethoxy)imidazo[1,2-a]pyridin-2-yl]methyl]-5-methyl-4-oxo-cinnolin-3-yl]essigsäure,
2-[1-[[6-(Cyclopropylmethoxy)-7-(trifluormethyl)imidazo[1,2-a]pyridin-2-yl]methyl]-4-oxo-cinnolin-3-yl]butansäure,
(1-[[5-(Cyelopropylmethoxy)-6-(trifluormethyl)-1H-benzimidazol-2-yl]methyl]-4-oxocinnolin-3-carbonsäure),
2-[1-[[7-Chlor-6-(cyclopropylmethoxy)imidazo[1,2-a]pyridin-2-yl]methyl]-4-oxo-5,6,7,8-tetrahydrocinnolin-3-yl]essigsäure,
(1-[[5-(Cyelopropylmethoxy)-6-(trifluormethyl)-1H-benzimidazol-2-yl]methyl]-4-oxochinolin-3-carbonsäure),
2-[1-[[6-Cyano-5-(cyclopropylmethoxy)-1H-benzimidazol-2-yl]methyl]-4-oxo-cinnolin-3-yl]propansäure,
2-[1-[[6-Cyano-5-(cyclopropylmethoxy)-1H-benzimidazol-2-yl]methyl]-4-oxo-cinnolin-3-yl]essigsäure,
2-[1-[[6-Brom-5-(cyclopropylmethoxy)-1H-benzimidazol-2-yl]methyl]-4-oxo-cinnolin-3-yl]essigsäure,
1-[[5-Cyano-6-(cyclopropylmethoxy)-1H-benzimidazol-2-yl]methyl]-4-oxo-cinnolin-3-carbonsäure,
2-[4-[[6-Brom-5-(cyclopropylmethoxy)indazol-2-yl]methyl]-1-oxo-phthalazin-2-yl]essigsäure,
2-[4-[[6-Chlor-5-(trifluormethoxy)-1H-benzimidazol-2-yl]methyl]-1-oxo-phthalazin-2-yl]essigsäure,
2-[4-[[5-Chlor-6-(trifluormethoxy)-1H-benzimidazol-2-yl]methyl]-1-oxo-5,6,7,8-tetrahydrophthalazin-2-yl]essigsäure,
2-[4-[[6-Chlor-5-(cyclopropylmethoxy)indazol-2-yl]methyl]-1-oxo-phthalazin-2-yl]essigsäure,
2-[4-[[5-Chlor-6-(cyclopropylmethoxy)-1H-benzimidazol-2-yl]methyl]-1-oxo-phthalazin-2-yl]essigsäure,
2-[4-[[5-Chlor-6-(cyclopropylmethoxy)-7-fluor-1H-benzimidazol-2-yl]methyl]-1-oxo-phthalazin-2-yl]essigsäure,
2-[4-[[5-Chlor-6-(cyclopropylmethoxy)-1H-benzimidazol-2-yl]methyl]-1-oxo-5,6,7,8-tetrahydrophthalazin-2-yl]essigsäure,
2-[4-[[5-Chlor-6-(2,2-difluorethoxy)-1H-benzimidazol-2-yl]methyl]-1-oxo-phthalazin-2-yl]essigsäure,
2-[4-[[5-Chlor-6-(2,2-difluorethoxy)-1H-benzimidazol-2-yl]methyl]-1-oxo-5,6,7,8-tetrahydrophthalazin-2-yl]essigsäure,
2-[4-[(5-Chlor-6-methoxy-1H-benzimidazol-2-yl)methyl]-1-oxo-5,6,7,8-tetrahydrophthalazin-2-yl]essigsäure,
2-[4-[(5-Chlor-6-ethoxy-1H-benzimidazol-2-yl)methyl]-1-oxo-5,6,7,8-tetrahydrophthalazin-2-yl]essigsäure,
2-[4-(6-Chlor-5-cyclopropylmethoxy-1H-benzimidazol-2-ylmethyl)-1-oxo-1H-phthalazin-2-yl]-propionsäure,
2-[4-[[5-(Cyclopropylmethoxy)-6-(trifluormethyl)-1H-benzimidazol-2-yl]methyl]-1-oxo-phthalazin-2-yl]essigsäure,
2-[4-[[5-(Cyclopropylmethoxy)-6-fluor-1H-benzimidazol-2-yl]methyl]-1-oxo-phthalazin-2-yl]essigsäure,
{4-[5-Chlor-6-(2,2-difluorethoxy)-1H-benzimidazol-2-ylmethyl]-1-oxo-1H-isochinolin-2-yl}-essigsäure,
2-[4-[[6-Chlor-5-(2,2-difluorethoxy)-1H-benzimidazol-2-yl]methyl]-5,8-difluor-1-oxo-phthalazin-2-yl]essigsäure,
2-[4-[[5-Ethoxy-6-(trifluormethyl)-1H-benzimidazol-2-yl]methyl]-1-oxo-phthalazin-2-yl]essigsäure,
2-[4-[(5-Chlor-6-ethoxy-3-oxo-1H-indazol-2-yl)methyl]-1-oxo-phthalazin-2-yl]essigsäure,
2-[4-[(5-Ethoxy-6-fluor-1H-benzimidazol-2-yl)methyl]-1-oxo-phthalazin-2-yl]essigsäure,
2-[4-[[5-(2,2-Difluorethoxy)-6-fluor-1H-benzimidazol-2-yl]methyl]-1-oxo-phthalazin-2-yl]essigsäure,
2-[4-[[5-Chlor-6-[(1-methylcyclopropyl)methoxy]-1H-benzimidazol-2-yl]methyl]-1-oxo-phthalazin-2-yl]essigsäure,
2-[4-[[5-Chlor-6-(2,2-dimethylpropoxy)-1H-benzimidazol-2-yl]methyl]-1-oxo-phthalazin-2-yl]essigsäure,
2-[4-[[5-Chlor-6-[(1-cyanocyclopropyl)methoxy]-1H-benzimidazol-2-yl]methyl]-1-oxo-phthalazin-2-yl]essigsäure,
2-[4-[[5-Chlor-6-(cyclopropylmethoxy)-3-oxo-1H-indazol-2-yl]methyl]-1-oxo-phthalazin-2-yl]essigsäure,
2-[4-[[6-Cyano-5-(cyclopropylmethoxy)indazol-2-yl]methyl]-1-oxo-phthalazin-2-yl]essigsäure,
2-[4-[(6-Chlor-5-methoxy-1H-benzimidazol-2-yl)methyl]-1-oxo-phthalazin-2-yl]essigsäure,
2-[4-[(6-Brom-5-ethoxyindazol-2-yl)methyl]-1-oxo-phthalazin-2-yl]essigsäure,
2-[4-[(5-Cyano-6-ethoxy-1H-benzimidazol-2-yl)methyl]-1-oxo-phthalazin-2-yl]essigsäure,
2-[4-[[5-Cyano-6-(2,2-difluorethoxy)-1H-benzimidazol-2-yl]methyl]-1-oxo-phthalazin-2-yl]essigsäure,
2-[4-[[6-Chlor-5-(trifluormethoxy)-1H-benzimidazol-2-yl]methyl]-1-oxo-phthalazin-2-yl]-N-[(1S,2R)-2-hydroxycyclopentyl]acetamid,
2-[4-[[6-Chlor-5-(trifluormethoxy)-1H-benzimidazol-2-yl]methyl]-1-oxo-phthalazin-2-yl]-N-(1,1-dioxothian-4-yl)acetamid,
4-[[6-Chlor-5-(trifluormethoxy)-1H-benzimidazol-2-yl]methyl]-2-(2-morpholino-2-oxo-ethyl)phthalazin-1-on,

2-[4-[[6-Chlor-5-(cyclopropylmethoxy)indazol-2-yl]methyl]-1-oxo-phthalazin-2-yl]-N-[(1S,2S)-2-hydroxycyclopentyl]acetamid,
N-tert-butyl-2-[4-[[6-Chlor-5-(cyclopropylmethoxy)indazol-2-yl]methyl]-1-oxo-phthalazin-2-yl]acetamid,
4-[[5-Chlor-6-(trifluormethoxy)-1H-benzimidazol-2-yl]methyl]-2-(2-morpholino-2-oxo-ethyl)-5,6,7,8-tetrahydrophthalazin-1-on,
2-[4-[[5-Chlor-6-(trifluormethoxy)-1H-benzimidazol-2-yl]methyl]-1-oxo-5,6,7,8-tetrahydrophthalazin-2-yl]-N-[(1S,2S)-2-hydroxycyclopentyl]acetamid,
2-[4-[[5-Chlor-6-(trifluormethoxy)-1H-benzimidazol-2-yl]methyl]-1-oxo-5,6,7,8-tetrahydrophthalazin-2-yl]-N-[(1S,2S)-2-hydroxycyclopentyl]-acetamid,
4-[[6-Chlor-5-(cyclopropylmethoxy)indazol-2-yl]methyl]-2-(2-morpholino-2-oxo-ethyl)phthalazin-1-on,
4-[[5-Chlor-6-(cyclopropylmethoxy)-7-fluor-1H-benzimidazol-2-yl]methyl]-2-(2-morpholino-2-oxo-ethyl)phthalazin-1-on,
2-[4-[[5-Chlor-6-(cyclopropylmethoxy)-1H-benzimidazol-2-yl]methyl]-1-oxo-phthalazin-2-yl]-N-cyclopentylacetamid,
4-[[5-Chlor-6-(2,2-difluorethoxy)-1H-benzimidazol-2-yl]methyl]-2-(2-morpholino-2-oxo-ethyl)phthalazin-1-one,
4-[[6-Chlor-5-(2,2-difluorethoxy)-1H-benzimidazol-2-yl]methyl]-5,8-difluor-2-(2-morpholino-2-oxo-ethyl)phthalazin-1-on,
4-[[6-Chlor-5-(2,2-difluorethoxy)-1H-benzimidazol-2-yl]methyl]-2-(2-morpholino-2-oxo-ethyl)-1-oxo-phthalazin-6-carbonitril,
4-[(5-Chlor-6-ethoxy-3-oxo-1H-indazol-2-yl)methyl]-2-(2-morpholino-2-oxo-ethyl)phthalazin-1-on,
4-(6-Chlor-5-cyclopropylmethoxy-1H-benzimidazol-2-ylmethyl)-2-(1-methyl-2-morpholin-4-yl-2-oxo-ethyl)-2H-phthalazin-1-on,
4-[[5-(2,2-Difluorethoxy)-6-fluor-1H-benzimidazol-2-yl]methyl]-2-(2-morpholino-2-oxo-ethyl)phthalazin-1-on,
4-[(5-Ethoxy-6-fluor-1H-benzimidazol-2-yl)methyl]-2-(2-morpholino-2-oxo-ethyl)phthalazin-1-on,
6-(Cyclopropylmethoxy)-2-[[3-(2-morpholino-2-oxo-ethyl)-4-oxo-phthalazin-1-yl]methyl]-3H-benzimidazol-5-carbonitril,
1-[6-Chlor-5-(2,2-difluorethoxy)-1H-benzimidazol-2-ylmethyl]-3-(2-morpholin-4-yl-2-oxo-ethyl)-1H-cinnolin-4-on,
4-[[5-Chlor-6-(2,2-dimethylpropoxy)-1H-benzimidazol-2-yl]methyl]-2-(2-morpholino-2-oxo-ethyl)phthalazin-1-on,
1-[[6-Chlor-2-[[3-(2-morpholino-2-oxo-ethyl)-4-oxo-phthalazin-1-yl]methyl]-3H-benzimidazol-5-yl]oxymethyl]cyclopropancarbonitril,
1-[[6-Chlor-2-[[3-[2-[(2S,6R)-2,6-dimethylmorpholin-4-yl]-2-oxo-ethyl]-4-oxo-phthalazin-1-yl]methyl]-3H-benzimidazol-5-yl]oxymethyl]cyclopropancarbonitril,
4-[[5-Chlor-6-[(1-methylcyclopropyl)methoxy]-1H-benzimidazol-2-yl]methyl]-2-(2-morpholino-2-oxo-ethyl)phthalazin-1-on,
6-(2,2-Difluorethoxy)-2-[[3-(2-morpholino-2-oxo-ethyl)-4-oxo-5,6,7,8-tetrahydrophthalazin-1-yl]methyl]-1H-benzimidazol-5-carbonitril,
4-[(5-Chlor-6-ethoxy-1H-benzimidazol-2-yl)methyl]-2-(2-morpholino-2-oxo-ethyl)phthalazin-1-on,
4-[[5-Chlor-6-(cyclopropylmethoxy)-1H-benzimidazol-2-yl]methyl]-2-(2-morpholino-2-oxo-ethyl)phthalazin-1-on,
6-Ethoxy-2-[[3-(2-morpholino-2-oxo-ethyl)-4-oxo-5,6,7,8-tetrahydrophthalazin-1-yl]methyl]-1H-benzimidazol-5-carbonitril,
4-[[6-Ethoxy-5-(trifluormethyl)-1H-benzimidazol-2-yl]methyl]-2-(2-morpholino-2-oxo-ethyl)phthalazin-1-on,
4-[6-Chlor-5-(2,2-difluorethoxy)-1H-benzimidazol-2-ylmethyl]-2-(2-morpholin-4-yl-2-oxo-ethyl)-2H-isochinolin-1-on,
6-Ethoxy-2-[[3-(2-morpholino-2-oxo-ethyl)-4-oxo-phthalazin-1-yl]methyl]-1H-benzimidazol-5-carbonitril,
6-(2,2-Difluorethoxy)-2-[[3-(2-morpholino-2-oxo-ethyl)-4-oxo-phthalazin-1-yl]methyl]-1H-benzimidazol-5-carbonitril,
4-[[5-Chlor-6-(2,2-difluorpropoxy)-1H-benzimidazol-2-yl]methyl]-2-(2-morpholino-2-oxo-ethyl)phthalazin-1-on,
4-[(5-Chlor-6-isobutoxy-1H-benzimidazol-2-yl)methyl]-2-(2-morpholino-2-oxo-ethyl)phthalazin-1-on,
4-[[5-Chlor-6-(2-methoxyethoxy)-1H-benzimidazol-2-yl]methyl]-2-(2-morpholino-2-oxo-ethyl)phthalazin-1-on,
2-[[3-(2-Morpholino-2-oxo-ethyl)-4-oxo-phthalazin-1-yl]methyl]-6-(2,2,2-Trifluorethoxy)-3H-benzimidazol-5-carbonitril,
6-(2,2-Difluorethoxy)-2-[[3-[2-[3-(dimethylamino)pyrrolidin-1-yl]-2-oxo-ethyl]-4-oxo-phthalazin-1-yl]methyl]-3H-benzimidazol-5-carbonitril,
6-(2,2-Difluorethoxy)-2-[[3-[2-[(2S,6R)-2,6-dimethylmorpholin-4-yl]-2-oxo-ethyl]-4-oxo-phthalazin-1-yl]methyl]-3H-benzimidazol-5-carbonitril,

2-[4-[[6-Cyano-5-(2,2-difluorethoxy)-1H-benzimidazol-2-yl]methyl]-1-oxo-phthalazin-2-yl]-N-THF-3-yl-acetamid,
4-[[5-(2,2-Difluorethoxy)-6-fluor-1H-benzimidazol-2-yl]methyl]-2-(2-morpholino-2-oxo-ethyl)-5,6,7,8-tetrahydrophthalazin-1-on,
2-[4-[[6-Cyano-5-(2,2-difluorethoxy)-1H-benzimidazol-2-yl]methyl]-1-oxo-phthalazin-2-yl]-N-methyl-N-(THF-2-ylmethyl)acetamid,
2-[4-[[6-Cyano-5-(2,2-difluorethoxy)-1H-benzimidazol-2-yl]methyl]-1-oxo-phthalazin-2-yl]-N-(2-cyanoethyl)-N-cyclopropylacetamid,
2-[4-[[6-Cyano-5-(2,2-difluorethoxy)-1H-benzimidazol-2-yl]methyl]-1-oxo-phthalazin-2-yl]-N-(2-methoxy-2-methylpropyl)acetamid
2-[4-[[6-Cyano-5-(2,2-difluorethoxy)-1H-benzimidazol-2-yl]methyl]-1-oxo-phthalazin-2-yl]-N-(2-methoxyethyl)-N-methylacetamid,
2-[4-[[6-Cyano-5-(2,2-difluorethoxy)-1H-benzimidazol-2-yl]methyl]-1-oxo-phthalazin-2-yl]-N-tetrahydropyran-3-yl-acetamid,
6-(2,2-Difluorethoxy)-2-[[3-[2-(4,4-difluor-1-piperidyl)-2-oxo-ethyl]-4-oxo-5,6,7,8-tetrahydrophthalazin-1-yl]methyl]-1H-benzimidazol-5-carbonitril,
6-(2,2-Difluorethoxy)-2-[[3-[2-(3-methoxypyrrolidin-1-yl)-2-oxo-ethyl]-4-oxo-phthalazin-1-yl]methyl]-1H-benzimidazol-5-carbonitril,
2-[4-[[5-Cyano-6-(2,2-difluorethoxy)-1H-benzimidazol-2-yl]methyl]-1-oxo-phthalazin-2-yl]-N-(2-methoxyethyl)acetamid,
6-(2,2-Difluorethoxy)-2-[[3-[2-(4,4-difluor-1-piperidyl)-2-oxo-ethyl]-4-oxo-phthalazin-1-yl]methyl]-1H-benzimidazol-5-carbonitril,
6-(2,2-Difluorethoxy)-2-[[3-[2-(6-oxa-9-azaspiro[3.5]nonan-9-yl)-2-oxo-ethyl]-4-oxo-phthalazin-1-yl]methyl]-1H-benzimidazol-5-carbonitril,
2-[4-[[5-Cyano-6-(2,2-difluorethoxy)-1H-benzimidazol-2-yl]methyl]-1-oxo-phthalazin-2-yl]-N-(3,3,3-trifluor-2-hydroxy-2-methylpropyl)acetamid,
2-[4-[[5-Cyano-6-(2,2-difluorethoxy)-1H-benzimidazol-2-yl]methyl]-1-oxo-phthalazin-2-yl]-N-[(2S)-3,3,3-trifluor-2-hydroxy-propyl]acetamid,
2-[4-[[5-Cyano-6-(2,2-difluorethoxy)-1H-benzimidazol-2-yl]methyl]-1-oxo-phthalazin-2-yl]-N-(2-hydroxy-3-methoxypropyl)acetamid,
6-(2,2-Difluorethoxy)-2-[[3-[2-(4-hydroxy-1-piperidyl)-2-oxo-ethyl]-4-oxo-phthalazin-1-yl]methyl]-1H-benzimidazol-5-carbonitril,
2-[4-[[5-Cyano-6-(2,2-difluorethoxy)-1H-benzimidazol-2-yl]methyl]-1-oxo-phthalazin-2-yl]-N-(2,2-difluorethyl)-N-(2-hydroxyethyl)acetamid,
2-[4-[[5-Cyano-6-(2,2-difluorethoxy)-1H-benzimidazol-2-yl]methyl]-1-oxo-phthalazin-2-yl]-N-(2-cyanopropyl)-N-methylacetamid,
2-[4-[[5-Cyano-6-(2,2-difluorethoxy)-1H-benzimidazol-2-yl]methyl]-1-oxo-phthalazin-2-yl]-N-(cyclopropylmethyl)-N-methylacetamid,
2-[4-[[5-Cyano-6-(2,2-difluorethoxy)-1H-benzimidazol-2-yl]methyl]-1-oxo-phthalazin-2-yl]-N-(2,2-difluoreethyl)-N-methylacetamid,
6-(2,2-Difluorethoxy)-2-[[3-[2-[(2S)-2-(hydroxymethyl)pyrrolidin-1-yl]-2-oxo-ethyl]-4-oxo-phthalazin-1-yl]methyl]-1H-benzimidazol-5-carbonitril,
2-[4-[[5-Cyano-6-(2,2-difluorethoxy)-1H-benzimidazol-2-yl]methyl]-1-oxo-phthalazin-2-yl]-N-[[3-(hydroxymethyl)oxetan-3-yl]methyl]acetamid,
2-[[3-(2-Morpholino-2-oxo-ethyl)-4-oxo-5,6,7,8-tetrahydrophthalazin-1-yl]methyl]-6-(2,2,2-trifluorethoxy)-1H-benzimidazol-5-carbonitril,
6-(2,2-Difluorethoxy)-2-[[3-[2-(3-hydroxy-3-methylpyrrolidin-1-yl)-2-oxo-ethyl]-4-oxo-phthalazin-1-yl]methyl]-3H-benzimidazol-5-carbonitril,
2-[4-[[6-Cyano-5-(2,2-difluorethoxy)-1H-benzimidazol-2-yl]methyl]-1-oxo-phthalazin-2-yl]-N-(2,2-dimethylpropyl)acetamid,
N-tert-butyl-2-[4-[[6-Cyano-5-(2,2-difluorethoxy)-1H-benzimidazol-2-yl]methyl]-1-oxo-5,6,7,8-tetrahydrophthalazin-2-yl]acetamid,
6-(2,2-Difluorethoxy)-2-[[3-[2-[3-(dimethylamino)pyrrolidin-1-yl]-2-oxo-ethyl]-4-oxo-5,6,7,8-tetrahydrophthalazin-1-yl]methyl]-1H-benzimidazol-5-carbonitril,
5-(2,2-Difluorethoxy)-2-[[3-(2-morpholino-2-oxo-ethyl)-4-oxo-phthalazin-1-yl]methyl]indazol-6-carbonitril,
5-(2,2-Difluorethoxy)-2-[[3-[2-[(3R)-3-(dimethylamino)pyrrolidin-1-yl]-2-oxo-ethyl]-4-oxo-phthalazin-1-yl]methyl]indazol-6-carbonitril,
5-(2,2-Difluorethoxy)-2-[[3-[2-[(3S)-3-(dimethylamino)pyrrolidin-1-yl]-2-oxo-ethyl]-4-oxo-phthalazin-1-yl]methyl]indazol-6-carbonitril,
2-[4-[[6-Cyano-5-(2,2-difluorethoxy)indazol-2-yl]methyl]-1-oxo-phthalazin-2-yl]-N-cyclopentylacetamid,
5-(Cyclopropylmethoxy)-2-[[3-(2-morpholino-2-oxo-ethyl)-4-oxo-phthalazin-1-yl]methyl]indazol-6-carbonitril,
6-(Cyclopropylmethoxy)-2-[[3-[2-(3-hydroxy-3-methyl-pyrrolidin-1-yl)-2-oxo-ethyl]-4-oxo-phthalazin-1-yl]methyl]imidazo[1,2-a]pyridin-7-carbonitril,
6-(2,2-Difluorethoxy)-2-[[3-[2-[(3S,4S)-3,4-dihydroxypyrrolidin-1-yl]-2-oxo-ethyl]-4-oxo-phthalazin-1-yl]methyl]-3H-benzimidazol-5-carbonitril,
6-(2,2-Difluorethoxy)-2-[[3-[2-(3,3-difluor-4-hydroxy-1-piperidyl)-2-oxo-ethyl]-4-oxo-phthalazin-1-yl]methyl]-3H-benzimidazol-5-carbonitril,
2-[[3-[2-(4-Cyclopropyl-4-hydroxy-1-piperidyl)-2-oxo-ethyl]-4-oxo-phthalazin-1-yl]methyl]-6-(2,2-difluorethoxy)-3H-benzimidazol-5-carbonitril,
2-[[3-[2-(3-Cyclopropyl-3-hydroxy-pyrrolidin-1-yl)-2-oxo-ethyl],
-4-oxo-phthalazin-1-yl]methyl]-6-(2,2-difluorethoxy)-3H-benzimidazol-5-carbonitril,
2-[[3-[2-(3-Cyclopropyl-3-hydroxy-azetidin-1-yl)-2-oxo-ethyl]-4-oxo-phthalazin-1-yl]methyl]-6-(2,2-difluorethoxy)-3H-benzimidazol-5-carbonitril,
6-(2,2-Difluorethoxy)-2-[[3-[2-[3-hydroxy-3-(trifluormethyl)pyrrolidin-1-yl]-2-oxo-ethyl]-4-oxo-phthalazin-1-yl]methyl]-3H-benzimidazol-5-carbonitril,
1-[[6-Brom-5-(cyclopropylmethoxy)-1H-benzimidazol-2-yl]methyl]-3-[2-[(3S,4S)-3,4-dihydroxypyrrolidin-1-yl]-2-oxo-ethyl]cinnolin-4-on,
2-[[3-[2-[4-(Cyclopropylmethyl)piperazin-1-yl]-2-oxo-ethyl]-4-oxo-phthalazin-1-yl]methyl]-6-(2,2-difluorethoxy)-3H-benzimidazol-5-carbonitril,
2-[[3-[2-(4-Cyano-1-piperidyl)-2-oxo-ethyl]-4-oxo-phthalazin-1-yl]methyl]-6-(2,2-difluorethoxy)-3H-benzimidazol-5-carbonitril,
2-[4-[[6-Cyano-5-(2,2-difluorethoxy)-1H-benzimidazol-2-yl]methyl]-1-oxo-phthalazin-2-yl]-N-methyl-N-(2-methylsulfonylethyl)acetamid,
6-(2,2-Difluorethoxy)-2-[[3-[2-(3-methylsulfonylpyrrolidin-1-yl)-2-oxo-ethyl]-4-oxo-phthalazin-1-yl]methyl]-3H-benzimidazol-5-carbonitril,
2-[4-[[6-Cyano-5-(2,2-difluorethoxy)-1H-benzimidazol-2-yl]methyl]-1-oxo-phthalazin-2-yl]-N-(2-hydroxy-3,3-dimethyl-butyl)acetamid,
2-[4-[[6-Cyano-5-(2,2-difluorethoxy)-1H-benzimidazol-2-yl]methyl]-1-oxo-phthalazin-2-yl]-N-(2,2-difluorethyl)acetamid,
2-[4-[[6-Cyano-5-(2,2-difluorethoxy)-1H-benzimidazol-2-yl]methyl]-1-oxo-phthalazin-2-yl]-N-(3,3,3-trifluorpropyl)acetamid,
6-(2,2-Difluorethoxy)-2-[[3-[2-[4-(2-hydroxyethyl)piperazin-1-yl]-2-oxo-ethyl-4-oxo-phthalazin-1-yl]methyl]-3H-benzimidazol-5-carbonitril,
N-[Cyano(cyclopropyl)methyl]-2-[4-[[6-Cyano-5-(2,2-difluorethoxy)-1H-benzimidazol-2-yl]methyl]-1-oxo-phthalazin-2-yl]acetamid,
6-(2,2-Difluorethoxy)-2-[[3-[2-(2,2-dimethylmorpholin-4-yl)-2-oxo-ethyl]-4-oxo-phthalazin-1-yl]methyl]-3H-benzimidazol-5-carbonitril,
2-[4-[[6-Cyano-5-(2,2-difluorethoxy)-1H-benzimidazol-2-yl]methyl]-1-oxo-phthalazin-2-yl]-N-(2,2-difluor-3-hydroxypropyl)acetamid,
2-[4-[[6-Cyano-5-(2,2-difluorethoxy)-1H-benzimidazol-2-yl]methyl]-1-oxo-phthalazin-2-yl]-N-(2-sulfamoylethyl)acetamid,
2-[4-[[6-Cyano-5-(2,2-difluorethoxy)-1H-benzimidazol-2-yl]methyl]-1-oxo-phthalazin-2-yl]-N-(2,2,2-trifluorethyl)acetamid,
6-(2,2-Difluorethoxy)-2-[[3-[2-(3-hydroxy-1-piperidyl)-2-oxo-ethyl]-4-oxo-phthalazin-1-yl]methyl]-3H-benzimidazol-5-carbonitril,
2-[4-[[6-Cyano-5-(2,2-difluorethoxy)-1H-benzimidazol-2-yl]methyl]-1-oxo-phthalazin-2-yl]-N-[(3,3-Difluorcyclobutyl)methyl]acetamid,
6-(2,2-Difluorethoxy)-2-[[3-[2-(3,3-dimethylpyrrolidin-1-yl)-2-oxo-ethyl]-4-oxo-phthalazin-1-yl]methyl]-3H-benzimidazol-5-carbonitril,
6-(2,2-Difluorethoxy)-2-[[3-[2-(4,4-dimethyl-1-piperidyl)-2-oxo-ethyl]-4-oxo-phthalazin-1-yl]methyl]-3H-benzimidazol-5-carbonitril,
2-[[3-[2-(2,2-Difluor-5-azaspiro[2.4]heptan-5-yl)-2-oxo-ethyl]-4-oxo-phthalazin-1-yl]methyl]-6-(2,2-difluorethoxy)-3H-benzimidazol-5-carbonitril,
1-[2-[4-[[6-Cyano-5-(2,2-difluorethoxy)-1H-benzimidazol-2-yl]methyl]-1-oxo-phthalazin-2-yl]acetyl]-N,N-dimethylpiperidin-4-carboxamid,
6-(2,2-Difluorethoxy)-2-[[3-[2-[3-(1-hydroxy-1-methyl-ethyl)-1-piperidyl]-2-oxo-ethyl]-4-oxo-phthalazin-1-yl]methyl]-3H-benzimidazol-5-carbonitril,
6-(2,2-Difluorethoxy)-2-[[3-[2-(4-morpholino-1-piperidyl)-2-oxo-ethyl]-4-oxo-phthalazin-1-yl]methyl]-3H-benzimidazol-5-carbonitril,
6-(2,2-Difluorethoxy)-2-[[3-[2-(7-oxa-2-azaspiro[3.5]nonan-2-yl)-2-oxo-ethyl]-4-oxo-phthalazin-1-yl]methyl]-3H-benzimidazol-5-carbonitril,
6-(2,2-Difluorethoxy)-2-[[3-[2-(4-ethylsulfonyl-1-piperidyl)-2-oxo-ethyl]-4-oxo-phthalazin-1-yl]methyl]-3H-benzimidazol-5-carbonitril,
2-[4-[[6-Cyano-5-(2,2-difluorethoxy)-1H-benzimidazol-2-yl]methyl]-1-oxo-phthalazin-2-yl]-N-[2-(dimethylamino)-2-oxo-ethyl]-N-methylacetamid,
2-[4-[[6-Chlor-5-(cyclopropylmethoxy)-1H-benzimidazol-2-yl]methyl]-1-oxo-phthalazin-2-yl]-N-methylsulfonylacetamid,
2-[4-[[6-Chlor-5-(cyclopropylmethoxy)indazol-2-yl]methyl]-1-oxo-phthalazin-2-yl]-N-methylsulfonylacetamid,
2-[4-[[6-Chlor-5-(cyclopropylmethoxy)indazol-2-yl]methyl]-1-oxo-phthalazin-2-yl]-N-cyclopropylsulfonylacetamid,
2-[4-[[6-Chlor-5-(2,2-difluorethoxy)-1H-benzimidazol-2-yl]methyl]-6-cyano-1-oxo-phthalazin-2-yl]-N-methylsulfonylacetamid,
N-{2-[4-(6-Chlor-5-cyclopropylmethoxy-1H-benzimidazol-2-ylmethyl)-1-oxo-1H-phthalazin-2-yl]-propionyl}-methansulfonamid,
2-[4-[(5-Ethoxy-6-fluor-1H-benzimidazol-2-yl)methyl]-1-oxo-phthalazin-2-yl]-N-methylsulfonylacetamid,
2-[4-[[5-(2,2-Difluorethoxy)-6-fluor-1H-benzimidazol-2-yl]methyl]-1-oxo-phthalazin-2-yl]-N-methylsulfonylacetamid,
2-[4-[[6-Chlor-5-[(1-cyanocyclopropyl)methoxy]-1H-benzimidazol-2-yl]methyl]-1-oxo-phthalazin-2-yl]-N-methylsulfonylacetamid,
2-[4-[[6-Chlor-5-[(1-methylcyclopropyl)methoxy]-1H-benzimidazol-2-yl]methyl]-1-oxo-phthalazin-2-yl]-N-methylsulfonylacetamid,
2-[4-[(6-Chlor-5-ethoxyindazol-2-yl)methyl]-1-oxo-phthalazin-2-yl]essigsäure,
2-[4-[[6-Chlor-5-(2,2,2-trifluorethoxy)indazol-2-yl]methyl]-1-oxo-phthalazin-2-yl]essigsäure,
2-[4-[[6-Chlor-5-(2,2-difluorethoxy)indazol-2-yl]methyl]-1-oxo-phthalazin-2-yl]essigsäure,
2-[4-[(4-Chlor-5-ethoxyindazol-2-yl)methyl]-1-oxo-phthalazin-2-yl]essigsäure,
2-[4-[(5-Chlor-6-ethoxyindazol-2-yl)methyl]-1-oxo-phthalazin-2-yl]essigsäure,
6-(2,2-Difluorethoxy)-2-[[3-(2-morpholino-2-oxo-ethyl)-4-oxo-phthalazin-1-yl]methyl]imidazo[1,2-a]pyridin-7-carbonitril und
2-[4-[[6-Chlor-5-(cyclopropylmethoxy)-1H-imidazo[4,5-b]pyridin-2-yl]methyl]-1-oxo-phthalazin-2-yl]essigsäure.

**12.** Pharmazeutische Zusammensetzung, umfassend eine Verbindung oder ein pharmazeutisch annehmbares Salz davon nach einem der Ansprüche 1-11 und einen pharmazeutisch annehmbaren Träger.

**13.** Pharmazeutische Zusammensetzung nach Anspruch 12, umfassend ein weiteres therapeutisches Mittel.

**14.** Verbindung oder pharmazeutisch annehmbares Salz davon nach einem der Ansprüche 1-11 oder pharmazeutische Zusammensetzung nach Anspruch 12 oder 13 zur Verwendung in der Medizin.

**15.** Verbindung oder ein pharmazeutisch annehmbares Salz davon nach einem der Ansprüche 1-11 oder pharmazeutische Zusammensetzung nach Anspruch 12 oder 13 zur Verwendung bei der Vorbeugung und/oder Behandlung von fibrotischen Erkrankungen, entzündlichen Erkrankungen, Atemwegserkrankungen, Autoimmunerkrankungen, Stoffwechselerkrankungen, kardiovaskulären Erkrankungen und/oder proliferativen Erkrankungen.

**16.** Pharmazeutische Zusammensetzung nach Anspruch 13, wobei das weitere therapeutische Mittel ein Mittel zur Vorbeugung und/oder Behandlung von fibrotischen Erkrankungen, entzündlichen Erkrankungen, Atemwegserkrankungen, Autoimmunerkrankungen, Stoffwechselerkrankungen, kardiovaskulären Erkrankungen und/oder proliferativen Erkrankungen ist.

## Revendications

**1.** Composé, ou sel pharmaceutiquement acceptable, ou solvate ou sel pharmaceutiquement acceptable d'un solvate de celui-ci, selon la formule I :

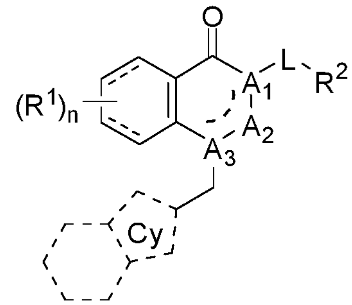

I

dans lequel

chaque $A_1$, $A_2$ et $A_3$ est choisi indépendamment parmi C et N à condition que $A_1$, $A_2$ et $A_3$ ne représentent pas simultanément C ou N ;
chaque $R^1$ est choisi indépendamment parmi

- alkyle en $C_{1-4}$,
- alcoxy en $C_{1-4}$,
- cycloalkyle en $C_{3-6}$,
- hétéroaryle monocyclique de 4 à 7 chaînons comprenant 1, 2 ou 3 hétéroatomes choisis indépendamment parmi N, O ou S, facultativement substitué par un ou deux groupes =O,
- $-S(O)_2C_{1-4}$ alkyle,
- -CN,
- $-C(=O)NH_2$, et
- halo ;

l'indice n vaut 0, 1 ou 2 ;
Cy représente un hétéroaryle 5-6 bicyclique fusionné à 9 chaînons relié à un cycle à 5 chaînons, comprenant 1, 2 ou 3 atomes N, lequel hétéroaryle est substitué par un groupe $R^3$ et un groupe $R^{4a}$, et facultativement substitué en outre par un halo ou OH ;
$R^3$ représente un alcoxy en $C_{1-6}$ facultativement substitué par un ou plusieurs éléments choisis indépendamment parmi

- halo,
- alcoxy en $C_{1-4}$ ou
- cycloalkyle en $C_{3-7}$ facultativement substitué par un alkyle en $C_{1-4}$, halo ou - CN;

$R^{4a}$ représente

- alkyle en $C_{1-4}$ facultativement substitué par un ou plusieurs halo,
- halo ou
- -CN ;

L est absent ou représente $-CR^{5a}R^{5b}-$ ;
$R^2$ représente

- -C(=O)OH,
- $-C(=O)NR^{6a}R^{6b}$,
- $-C(O)NHS(O)_2-C_{1-4}$ alkyle,
- $-C(O)NHS(O)_2-C_{3-7}$ cycloalkyle,
- $-Cy_1$ ou
- $-C(=O)Cy_2$;

chaque $R^{5a}$ et $R^{5b}$ est choisi indépendamment parmi

- H,
- alkyle en $C_{1-4}$ facultativement substitué par un, deux ou trois halo ou $-NR^{8a}R^{8b}$, et
- alcoxy en $C_{1-4}$ ;

chaque $R^{6a}$ et $R^{6b}$ est choisi indépendamment parmi

- H,
- alkyle en $C_{1-6}$ facultativement substitué parmi l'un plusieurs des éléments choisis indépendamment parmi
    - ◦ OH,
    - ◦ -CN,
    - ◦ halo,
    - ◦ alcoxy en $C_{1-4}$,
    - ◦ hétérocycloalkyle monocyclique de 4 à 6 chaînons comprenant un ou deux hétéroatomes choisis parmi N, O et S, facultativement substitué par un $-CH_2-OH$,
    - ◦ $-S(O)_2C_{1-4}$ alkyle,
    - ◦ $-S(O)_2NH_2$,
    - ◦ $-C(O)NR^{9a}R^{9b}$ ou
    - ◦ cycloalkyle en $C_{3-7}$ facultativement substitué par OH ou halo ;

        - alcoxy en $C_{1-4}$,
        - cycloalkyle en $C_{3-7}$ facultativement substitué par un ou plusieurs OH, et
        - hétérocycloalkyle monocyclique de 4 à 6 chaînons comprenant un ou deux hétéroatomes choisis parmi N, O et S, facultativement substitué par un ou deux oxo ;

$Cy_1$ représente

- cycloalkyle monocyclique en $C_{3-7}$, facultativement substitué par un - C(=O)OH,
- hétérocycloalkyle monocyclique de 4 à 6 chaînons comprenant un ou deux hétéroatomes choisis parmi N, O et S, facultativement substitué par un ou deux
- ◦ alkyle en $C_{1-4}$ facultativement substitué par un -C(=O)OH,

$Cy_2$ représente

- hétérocycloalkyle monocyclique lié à N de 4 à 7 chaînons comprenant au moins un atome N, et facultativement un ou deux hétéroatomes choisis parmi N, O et S, facultativement substitué par un ou plusieurs
    - ◦ OH,
    - ◦ Oxo,
    - ◦ -CN,
    - ◦ halo,
    - ◦ alcoxy en $C_{1-4}$,
    - ◦ alkyle en $C_{1-4}$ facultativement substitué par un ou plusieurs éléments choisis indépendamment parmi
        - ▪ halo,
        - ▪ OH,
    - ◦ cycloalkyle en $C_{3-7}$,
    - ◦ $-S(O)_2C_{1-4}$ alkyle ou
    - ◦ $-NR^{7a}R^{7b}$; ou

        - hétérocycloalkyle spirocyclique lié à N de 7 à 9 chaînons comprenant au moins un atome N, et facultativement un ou deux hétéroatomes choisis parmi N, O et S, facultativement substitué par un ou plusieurs halo ;

chaque $R^{7a}$, $R^{7b}$, $R^{8a}$, $R^{8b}$, $R^{9a}$ et $R^{9b}$ est choisi indépendamment parmi H et alkyle en $C_{1-4}$ ;
à condition que

- si L est absent, $A_1$ et $A_2$ représentent C, $A_3$ représente N, et $R^2$ représente COOH, $R^3$ représente un alcoxy en $C_{1-6}$ non substitué ou un alcoxy en $C_{1-6}$ substitué par un halogène, donc n'est pas COOH ; et
- si $A_1$ et $A_2$ représentent N, $A_3$ représente C, et $R^{4a}$ représente $-CH_3$ alors $R^3$ ne représente pas un alcoxy en $C_{1-4}$ non substitué.

**2.** Composé ou sel pharmaceutiquement acceptable de celui-ci selon la revendication 1, dans lequel l'indice n vaut 0.

**3.** Composé ou sel pharmaceutiquement acceptable de celui-ci selon la revendication 1 ou 2, dans lequel Cy est choisi parmi $Cy_A$, $Cy_B$, $Cy_C$ et $Cy_D$ :

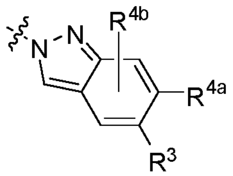
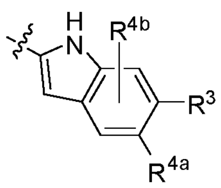
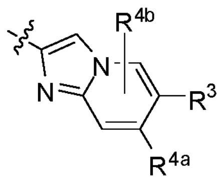
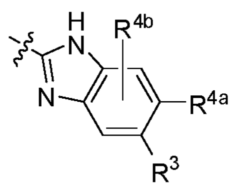

$Cy_A$, $Cy_B$, $Cy_C$, $Cy_D$.

**4.** Composé ou sel pharmaceutiquement acceptable de celui-ci selon la revendication 3, dans lequel $R^3$ représente $-OCH_3$, $-OCH_2CH_3$, $-OCF_3$, $-OCH_2CF_3$, $-OCH_2CHF_2$, $-OCH_2CH_2OCH_3$,

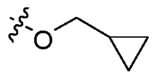 , 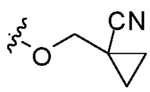 ou 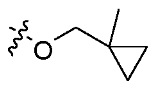 .

**5.** Composé ou sel pharmaceutiquement acceptable de celui-ci selon la revendication 3 ou 4, dans lequel $R^{4b}$ représente H, F, Cl ou OH.

**6.** Composé ou sel pharmaceutiquement acceptable de celui-ci selon la revendication 3 ou 4, dans lequel $R^{4a}$ représente F, Cl, -CN ou $-CF_3$.

**7.** Composé ou sel pharmaceutiquement acceptable de celui-ci selon la revendication 1, dans lequel le composé se présente selon la formule Va, Vb, Vc, VIa, VIb ou Vic :

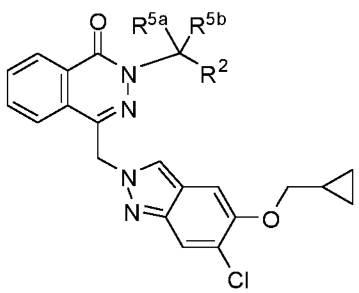

Va,

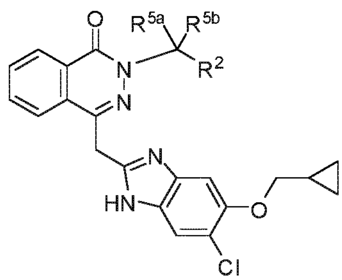

Vb,

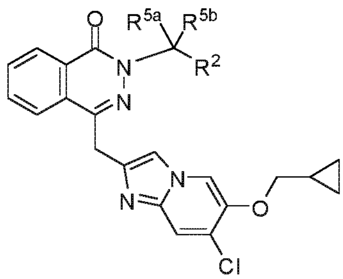

Vc

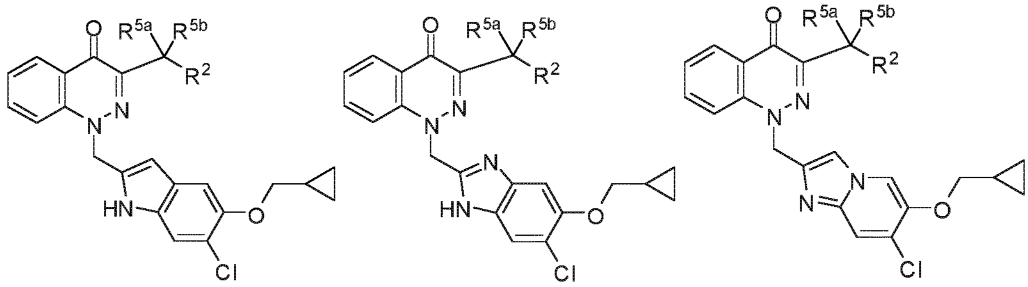

VIa, VIb ou Vic.

8. Composé ou sel pharmaceutiquement acceptable de celui-ci selon la revendication 3 ou 4, dans lequel chaque $R^{5a}$ et $R^{5b}$ est choisi indépendamment parmi H, $-OCH_3$,$-OCH_2CH_3$, $-CH_3$, $-CH_2CH_3$, $-CH_3$, $-CH_2CHF_2$, $-CH_2CF_3$ et $-CH_2CH_2-N(CH_3)_2$.

9. Composé ou sel pharmaceutiquement acceptable de celui-ci selon la revendication 1, dans lequel le composé se présente selon la formule VIIa, VIIb ou VIIc :

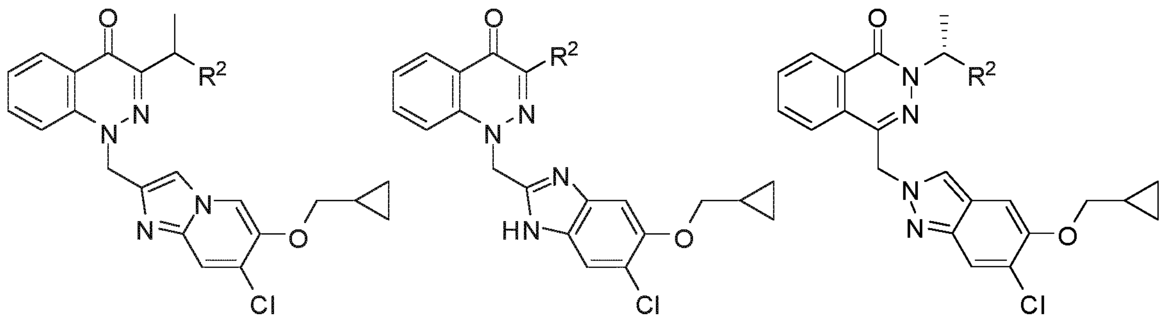

VIIa, VIIb ou VIIc

10. Composé ou sel pharmaceutiquement acceptable de celui-ci selon l'une quelconque des revendications 1-9, dans lequel $R^2$ représente $-C(=O)OH$, $-C(O)NHS(O)_2-CH_3$, $-C(O)NHS(O)_2-C_{3-7}$ cycloalkyle,

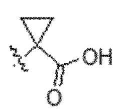

ou

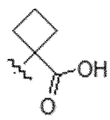

11. Composé ou sel pharmaceutiquement acceptable de celui-ci, dans lequel le composé selon la revendication 1 est choisi parmi :

6-(2,2-difluoroéthoxy)-2-[[4-oxo-3-(pyrrolidin-2-ylméthyl)phthalazin-1-yl]méthyl]-3H-benzimidazole-5-carbonitrile,
2-(azétidin-3-yl)-4-[[6-chloro-5-(cyclopropylméthoxy)indazol-2-yl]méthyl]phthalazin-1-one,
acide 2-[4-[[6-chloro-5-(cyclopropylméthoxy)indazol-2-yl]méthyl]-1-oxo-phthalazin-2-yl]propanoïque,
acide 2-[1-[[7-chloro-6-(cyclopropylméthoxy)imidazo[1,2-a]pyridin-2-yl]méthyl]-4-oxo-cinnolin-3-yl]propanoïque,
acide 1-[[6-chloro-5-(cyclopropylméthoxy)-1H-benzimidazol-2-yl]méthyl]-4-oxo-cinnoline-3-carboxylique,
acide 2-[4-[(5-chloro-6-éthoxy-1H-benzimidazol-2-yl)méthyl]-1-oxo-phthalazin-2-yl]acétique,
acide 2-[4-[[6-cyano-5-(cyclopropylméthoxy)-1H-benzimidazol-2-yl]méthyl]-1-oxo-phthalazin-2-yl]acétique,
acide 2-[4-[[6-cyano-5-(2,2-difluoroéthoxy)indazol-2-yl]méthyl]-1-oxo-phthalazin-2-yl]acétique,
acide 2-[4-[[5-chloro-6-(2,2-difluoroéthoxy)-1H-indol-2-yl]méthyl]-1-oxo-phthalazin-2-yl] acétique,
6-(cyclopropylméthoxy)-2-[[3-[2-[(3S,4S)-3,4-dihydroxypyrrolidin-1-yl]-2-oxo-éthyl]-4-oxo-cinnolin-1-yl]méthyl]-3H-benzimidazole-5-carbonitrile,
4-[[5-chloro-6-(2,2-difluoroéthoxy)-1H-indol-2-yl]méthyl]-2-(2-morpholino-2-oxo-éthyl)phthalazin-1-one,

4-[[6-chloro-5-(2,2-difluoroéthoxy)-1H-benzimidazol-2-yl]méthyl]-2-(2-morpholino-2-oxo-éthyl)-1-oxo-phthalazine-6-carboxamide,
6-(2,2-difluoroéthoxy)-2-[[3-[2-[(3S)-3-hydroxypyrrolidin-1-yl]-2-oxo-éthyl]-4-oxo-phthalazin-1-yl]méthyl]-3H-benzimidazole-5-carbonitrile,
2-[(l-acétylpyrrolidin-2-yl)méthyl]-4-[[5-chloro-6-(trifluorométhoxy)-1H-benzimidazol-2-yl]méthyl]phthalazin-1-one,
2-[1-[[7-chloro-6-(cyclopropylméthoxy)imidazo[1,2-a]pyridin-2-yl]méthyl]-4-oxo-cinnolin-3-yl]-N-méthylsulfonyl-propanamide,
acide 2-[1-[[5-chloro-6-(cyclopropylméthoxy)-1H-indol-2-yl]méthyl]-4-oxo-cinnolin-3-yl]acétique,
(acide (2R)-2-[4-[[6-chloro-5-(cyclopropylméthoxy)indazol-2-yl]méthyl]-1-oxo-phthalazin-2-yl]propanoïque),
acide (2S)-2-[4-[[6-chloro-5-(cyclopropylméthoxy)indazol-2-yl]méthyl]-1-oxo-phthalazin-2-yl]propanoïque,
acide (2S)-2-[1-[[7-chloro-6-(cyclopropylméthoxy)imidazo[1,2-a]pyridin-2-yl]méthyl]-4-oxocinnolin-3-yl]propanoïque,
acide (2R)-2-[1-[[7-chloro-6-(cyclopropylméthoxy)imidazo[1,2-a]pyridin-2-yl]méthyl]-4-oxocinnolin-3-yl]propanoïque,
(acide 2-[1-[[5-cyano-6-(cyclopropylméthoxy)-1H-benzimidazol-2-yl]méthyl]-4-oxocinnolin-3-yl]-3-(diméthylamino)propanoïque),
acide 2-[4-[(7-chloro-6-éthoxy-indazol-2-yl)méthyl]-1-oxo-phthalazin-2-yl]acétique,
acide 2-[4-[[5-chloro-6-(2,2-difluoroéthoxy)-1H-benzimidazol-2-yl]méthyl]-6-cyano-1-oxo-phthalazin-2-yl]acétique,
4-[[5-chloro-6-(trifluorométhoxy)-1H-benzimidazol-2-yl]méthyl]-2-cyclopentyl-phthalazin-1-one,
4-[[5-chloro-6-(trifluorométhoxy)-1H-benzimidazol-2-yl]méthyl]-2-cyclopropyl-phthalazin-1-one,
4-[[5-chloro-6-(trifluorométhoxy)-1H-benzimidazol-2-yl]méthyl]-2-cyclobutyl-phthalazin-1-one,
4-[[6-chloro-5-(trifluorométhoxy)-1H-benzimidazol-2-yl]méthyl]-2-(cyclopropylméthyl)phthalazin-1-one,
4-[[6-chloro-5-(trifluorométhoxy)-1H-benzimidazol-2-yl]méthyl]-2-(cyclobutylméthyl)phthalazin-1-one,
4-[[5-chloro-6-(2,2-difluoroéthoxy)-1H-benzimidazol-2-yl]méthyl]-6-méthoxy-2-(2-morpholino-2-oxo-éthyl)phthalazin-1-one,
4-[[5-chloro-6-(2,2-difluoroéthoxy)-1H-benzimidazol-2-yl]méthyl]-7-méthoxy-2-(2-morpholino-2-oxo-ethyl)phthalazin-1-one,
4-[[5-chloro-6-(2,2-difluoroéthoxy)-1H-benzimidazol-2-yl]méthyl]-2-cyclopropyl-phthalazin-1-one,
4-[[5-chloro-6-(2,2-difluoroéthoxy)-1H-benzimidazol-2-yl]méthyl]-7-morpholino-2-(2-morpholino-2-oxo-éthyl)phthalazin-1-one,
4-[[6-chloro-5-(trifluorométhoxy)-1H-benzimidazol-2-yl]méthyl]-2-(THF-2-ylméthyl)phthalazin-1-one,
4-[[6-chloro-5-(trifluorométhoxy)-1H-benzimidazol-2-yl]méthyl]-2-(tétrahydropyran-2-ylméthyl)phthalazin-1-one,
4-[[6-chloro-5-(trifluorométhoxy)-1H-benzimidazol-2-yl]méthyl]-2-[(3-méthyloxétan-3-yl)méthyl]phthalazin-1-one,
4-[[6-chloro-5-(trifluorométhoxy)-1H-benzimidazol-2-yl]méthyl]-2-[(2,2-diméthyl-1,3-dioxolan-4-yl)méthyl]phthalazin-1-one,
4-[[6-chloro-5-(trifluorométhoxy)-1H-benzimidazol-2-yl]méthyl]-2-[(1-méthyl-3-piperidyl)méthyl]phthalazin-1-one,
1-[[5-chloro-6-(2,2-difluoroéthoxy)-1H-benzimidazol-2-yl]méthyl]-3-(2-morpholino-2-oxo-éthyl)-4-oxo-phthalazine-6-carbonitrile,
4-[[5-chloro-6-(2,2-difluoroéthoxy)-1H-benzimidazol-2-yl]méthyl]-6-méthylsulfonyl-2-(2-morpholino-2-oxo-éthyl)phthalazin-1-one,
6-(cyclopropylméthoxy)-2-[(3-cyclopropyl-4-oxo-phthalazin-1-yl)méthyl]-1H-benzimidazole-5-carbonitrile,
4-[[6-chloro-5-(trifluorométhoxy)-1H-benzimidazol-2-yl]méthyl]-2-[(2,2-difluorocyclopropyl)méthyl]phthalazin-1-one,
4-[[6-chloro-5-(trifluorométhoxy)-1H-benzimidazol-2-yl]méthyl]-2-(3,3-diméthyl-2-oxo-butyl)phthalazin-1-one,
acide 1-[[4-[[5-chloro-6-(trifluorométhoxy)-1H-benzimidazol-2-yl]méthyl]-1-oxo-phthalazin-2-yl]méthyl]cyclopropanecarboxylique,
4-[[6-chloro-5-(2,2-difluoroéthoxy)-1H-benzimidazol-2-yl]méthyl]-5-cyclopropyl-2-(2-morpholino-2-oxo-éthyl)phthalazin-1-one,
6-(2,2-Difluoro-éthoxy)-2-[3-(2-morpholin-4-yl-2-oxo-éthyl)-4-oxo-4H-cinnolin-1-ylméthyl]-3H-enzoimidazole-5-carbonitrile,
1-[5-(2,2-Difluoro-éthoxy)-6-fluoro,
-1H-benzoimidazol-2-ylméthyl]-3-(2-morpholin-4-yl-2-oxo-éthyl)-1H-cinnolin-4-one,
6-(2-méthoxyéthoxy)-2-[[3-(2-morpholino-2-oxo-éthyl)-4-oxo-phthalazin-1-yl]méthyl]-3H-benzimidazole-5-carbonitrile,
6-(2,2-difluoropropoxy)-2-[[3-(2-morpholino-2-oxo-éthyl)-4-oxo-phthalazin-1-yl]méthyl]-3H-benzimidazole-5-carbonitrile,
7-chloro-6-(2,2-difluoroéthoxy)-2-[[3-(2-morpholino-2-oxo-éthyl)-4-oxo-phthalazin-1-yl]méthyl]-3H-benzimidazole-5-carbonitrile,
2-[[8-cyclopropyl-3-(2-morpholino-2-oxo-éthyl)-4-oxo-phthalazin-1-yl]méthyl]-6-(2,2-difluoroéthoxy)-3H-benzimidazole-5-carbonitrile,
4-[[6-chloro-5-(trifluorométhoxy)-1H-benzimidazol-2-yl]méthyl]-2-(pyrrolidin-2-ylméthyl)phthalazin-1-one,
4-[[6-chloro-5-(trifluorométhoxy)-1H-benzimidazol-2-yl]méthyl]-2-(4-piperidylméthyl)phthalazin-1-one,
4-[[6-chloro-5-(trifluorométhoxy)-1H-benzimidazol-2-yl]méthyl]-2-(pyrrolidin-3-ylméthyl)phthalazin-1-one,
4-[[6-chloro-5-(trifluorométhoxy)-1H-benzimidazol-2-yl]méthyl]-2-(3-pipéridylméthyl)phthalazin-1-one,
4-[[6-chloro-5-(trifluorométhoxy)-1H-benzimidazol-2-yl]méthyl]-2-pyrrolidin-3-yl-phthalazin-1-one,
2-(azétidin-3-ylméthyl)-4-[[6-chloro-5-(trifluorométhoxy)-1H-benzimidazol-2-yl]méthyl]phthalazin-1-one,
4-[[6-chloro-5-(trifluorométhoxy)-1H-benzimidazol-2-yl]méthyl]-2-(4-pipéridyl)phthalazin-1-one,
4-[[5-chloro-6-(trifluorométhoxy)-1H-benzimidazol-2-yl]méthyl]-2-(morpholin-2-ylméthyl)phthalazin-1-one,
6-(2,2-difluoroéthoxy)-2-[(4-oxo-3-pyrrolidin-3-yl-phthalazin-1-yl)méthyl]-3H-benzimidazole-5-carbonitrile,
6-(2-méthoxyéthoxy)-2-[(4-oxo-3-pyrrolidin-3-yl-phthalazin-1-yl)méthyl]-3H-benzimidazole-5-carbonitrile,
6-(2,2-difluoroéthoxy)-2-[[8-méthyl-3-(2-morpholino-2-oxo-éthyl)-4-oxo-phthalazin-1-yl]méthyl]-3H-benzimidazole-5-carbonitrile,
4-[[6-chloro-5-(2-méthoxyéthoxy)-1H-benzimidazol-2-yl]méthyl]-2-pyrrolidin-3-yl-phthalazin-1-one,
2-[[3-(azétidin-3-yl)-4-oxo-phthalazin-1-yl]méthyl]-6-(2,2-difluoroéthoxy)-3H-benzimidazole-5-carbonitrile,
acide 2-[3-[4-[[6-cyano-5-(2,2-difluoroéthoxy)-1H-benzimidazol-2-yl]méthyl]-1-oxo-phthalazin-2-yl]azétidin-1-yl]acétique,
acide 2-[2-[[4-[[6-cyano-5-(2,2-difluoroéthoxy)-1H-benzimidazol-2-yl]méthyl]-1-oxo-phthalazin-2-yl]méthyl]pyrrolidin-1-yl]acétique,
acide 2-[3-[4-[[6-cyano-5-(2,2-difluoroéthoxy)-1H-benzimidazol-2-yl]méthyl]-1-oxo-phthalazin-2-yl]pyrrolidin-1-yl] acétique,
6-(2,2-difluoroéthoxy)-2-[[3-(1-méthylpyrrolidin-3-yl)-4-oxo-phthalazin-1-yl]méthyl]-3H-benzimidazole-5-carbonitrile,
acide 2-[3-[4-[[7-cyano-6-(cyclopropylméthoxy)imidazo[1,2-a]pyridin-2-yl]méthyl]-1-oxo-phthalazin-2-yl]azétidin-1-yl]acétique,
acide 2-[4-[[6-cyano-5-(2,2-difluoroéthoxy)-1H-benzimidazol-2-yl]méthyl]-1-oxo-phthalazin-2-yl]-3-(diméthylamino)propanoïque,
acide 3-(azétidin-1-yl)-2-[4-[[6-cyano-5-(2,2-difluoroéthoxy)-1H-benzimidazol-2-yl]méthyl]-1-oxo-phthalazin-2-yl]propanoïque,
acide 2-[4-[[6-chloro-5-(2,2-difluoroéthoxy)indazol-2-yl]méthyl]-1-oxo-phthalazin-2-yl]propanoïque,
acide 2-[4-[(6-chloro-5-éthoxy-1H-benzimidazol-2-yl)méthyl]-8-méthyl-1-oxo-phthalazin-2-yl]acétique,
acide {1-[6-Chloro-5-(2,2-difluoro-éthoxy)-1H-benzoimidazol-2-ylméthyl]-4-oxo-1,4-dihydro-cinnolin-3-yl}-acétique,
acide 2-[4-[[7-cyano-6-(cyclopropylméthoxy)imidazo[1,2-a]pyridin-2-yl]méthyl]-1-oxo-phthalazin-2-yl]acétique,
acide 1-[[6-cyano-5-(2,2-difluoroéthoxy)-1H-benzimidazol-2-yl]méthyl]-4-oxo-quinoline-3-carboxylique,
acide 2-[4-[[7-cyano-6-(cyclopropylméthoxy)imidazo[1,2-a]pyridin-2-yl]méthyl]-1-oxo-phthalazin-2-yl]propanoïque,
acide 2-[4-[[7-chloro-6-(cyclopropylméthoxy)imidazo[1,2-a]pyridin-2-yl]méthyl]-1-oxo-phthalazin-2-yl] acétique,
acide 2-[4-[[7-chloro-6-(cyclopropylméthoxy)imidazo[1,2-a]pyridin-2-yl]méthyl]-1-oxo-phthalazin-2-yl]propanoïque,
acide 1-[[7-chloro-6-(cyclopropylméthoxy)imidazo[1,2-a]pyridin-2-yl]méthyl]-4-oxo-quinoline-3-carboxylique,
acide 1-[[7-chloro-6-(cyclopropylméthoxy)imidazo[1,2-a]pyridin-2-yl]méthyl]-4-oxo-cinnoline-3-carboxylique,
acide 2-[4-[[7-chloro-6-(cyclopropylméthoxy)imidazo[1,2-a]pyridin-2-yl]méthyl]-6-fluoro-1-oxo-phthalazin-2-yl]propanoïque,
acide 2-[3-[4-[[7-chloro-6-(cyclopropylméthoxy)imidazo[1,2-a]pyridin-2-yl]méthyl]-1-oxo-phthalazin-2-yl]pyrrolidin-1-yl]acétique,
acide 2-[1-[[7-chloro-6-(cyclopropylméthoxy)imidazo[1,2-a]pyridin-2-yl]méthyl]-4-oxo-cinnolin-3-yl]-2-méthylpropanoïque,
acide 2-[1-[[7-chloro-6-(cyclopropylméthoxy)imidazo[1,2-a]pyridin-2-yl]méthyl]-4-oxo-cinnolin-3-yl]acétique,
acide 1-[[6-chloro-5-(cyclopropylméthoxy)-1H-benzimidazol-2-yl]méthyl]-4-oxo-quinoline-3-carboxylique,
acide 2-[4-[[6-chloro-5-(cyclopropylméthoxy)indazol-2-yl]méthyl]-1-oxo-phthalazin-2-yl]cyclobutanecarboxylique,

acide 2-[4-[[6-chloro-5-(cyclopropylméthoxy)indazol-2-yl]méthyl]-6-cyano-1-oxo-phthalazin-2-yl]propanoïque,
acide 2-[4-[[6-chloro-5-(cyclopropylméthoxy)indazol-2-yl]méthyl]-1-oxo-5,6,7,8-tétrahydrophthalazin-2-yl]propanoïque,
acide 2-[1-[[7-chloro-6-(cyclopropylméthoxy)imidazo[1,2-a]pyridin-2-yl]méthyl]-4-oxo-cinnolin-3-yl]-2-méthoxy-acétique,
acide 1-[4-[[6-chloro-5-(cyclopropylméthoxy)indazol-2-yl]méthyl]-1-oxo-phthalazin-2-yl]cyclopropanecarboxylique,
acide 1-[1-[[7-chloro-6-(cyclopropylméthoxy)imidazo[1,2-a]pyridin-2-yl]méthyl]-4-oxo-cinnolin-3-yl]cyclopropanecarboxylique,
acide 2-[4-[[6-chloro-5-(cyclopropylméthoxy)indazol-2-yl]méthyl]-7-méthoxy-1-oxo-phthalazin-2-yl]propanoïque,
acide 2-[1-[[7-chloro-6-(cyclopropylméthoxy)imidazo[1,2-a]pyridin-2-yl]méthyl]-4-oxo-cinnolin-3-yl]butanoïque,
acide 2-[1-[[6-(cyclopropylméthoxy)-7-(trifluorométhyl)imidazo[1,2-a]pyridin-2-yl]méthyl]-4-oxo-cinnolin-3-yl]acétique,
acide 2-[1-[[6-(cyclopropylméthoxy)-7-(trifluorométhyl)imidazo[1,2-a]pyridin-2-yl]méthyl]-4-oxo-cinnolin-3-yl]propanoïque,
acide 2-[1-[[7-chloro-6-(cyclopropylméthoxy)imidazo[1,2-a]pyridin-2-yl]méthyl]-7-fluoro-4-oxo-cinnolin-3-yl]acétique,
acide 2-[1-[[7-chloro-6-(cyclopropylméthoxy)imidazo[1,2-a]pyridin-2-yl]méthyl]-5-méthyl-4-oxo-cinnolin-3-yl]acétique,
acide 2-[1-[[6-(cyclopropylméthoxy)-7-(trifluorométhyl)imidazo[1,2-a]pyridin-2-yl]méthyl]-4-oxo-cinnolin-3-yl]butanoïque,
(acide 1-[[5-(cyclopropylméthoxy)-6-(trifluorométhyl)-1H-benzimidazol-2-yl]méthyl]-4-oxocinnoline-3-carboxylique),
acide 2-[1-[[7-chloro-6-(cyclopropylméthoxy)imidazo[1,2-a]pyridin-2-yl]méthyl]-4-oxo-5,6,7,8-tétrahydrocinnolin-3-yl]acétique,
(acide 1-[[5-(cyclopropylméthoxy)-6-(trifluorométhyl)-1H-benzimidazol-2-yl]méthyl]-4-oxoquinoline-3-carboxylique),
acide 2-[1-[[6-cyano-5-(cyclopropylméthoxy)-1H-benzimidazol-2-yl]méthyl]-4-oxo-cinnolin-3-yl]propanoïque,
acide 2-[1-[[6-cyano-5-(cyclopropylméthoxy)-1H-benzimidazol-2-yl]méthyl]-4-oxo-cinnolin-3-yl]acétique,
acide 2-[1-[[6-bromo-5-(cyclopropylméthoxy)-1H-benzimidazol-2-yl]méthyl]-4-oxo-cinnolin-3-yl]acétique,
acide 1-[[5-cyano-6-(cyclopropylméthoxy)-1H-benzimidazol-2-yl]méthyl]-4-oxo-cinnoline-3-carboxylique,
acide 2-[4-[[6-bromo-5-(cyclopropylméthoxy)indazol-2-yl]méthyl]-1-oxo-phthalazin-2-yl]acétique,
acide 2-[4-[[6-chloro-5-(trifluorométhoxy)-1H-benzimidazol-2-yl]méthyl]-1-oxo-phthalazin-2-yl]acétique,
acide 2-[4-[[5-chloro-6-(trifluorométhoxy)-1H-benzimidazol-2-yl]méthyl]-1-oxo-5,6,7,8-tétrahydrophthalazin-2-yl]acétique,
acide 2-[4-[[6-chloro-5-(cyclopropylméthoxy)indazol-2-yl]méthyl]-1-oxo-phthalazin-2-yl]acétique,
acide 2-[4-[[5-chloro-6-(cyclopropylméthoxy)-1H-benzimidazol-2-yl]méthyl]-1-oxo-phthalazin-2-yl]acétique,
acide 2-[4-[[5-chloro-6-(cyclopropylméthoxy)-7-fluoro-1H-benzimidazol-2-yl]méthyl]-1-oxo-phthalazin-2-yl]acétique,
acide 2-[4-[[5-chloro-6-(cyclopropylméthoxy)-1H-benzimidazol-2-yl]méthyl]-1-oxo-5,6,7,8-tétrahydrophthalazin-2-yl]acétique,
acide 2-[4-[[5-chloro-6-(2,2-difluoroéthoxy)-1H-benzimidazol-2-yl]méthyl]-1-oxo-phthalazin-2-yl]acétique,
acide 2-[4-[[5-chloro-6-(2,2-difluoroéthoxy)-1H-benzimidazol-2-yl]méthyl]-1-oxo-5,6,7,8-tétrahydrophthalazin-2-yl]acétique,
acide 2-[4-[(5-chloro-6-méthoxy-1H-benzimidazol-2-yl)méthyl]-1-oxo-5,6,7,8-tétrahydrophthalazin-2-yl]acétique,
acide 2-[4-[(5-chloro-6-éthoxy-1H-benzimidazol-2-yl)méthyl]-1-oxo-5,6,7,8-tétrahydrophthalazin-2-yl]acétique,
acide 2-[4-(6-Chloro-5-cyclopropylméthoxy-1H-benzoimidazol-2-ylméthyl)-1-oxo-1H-phthalazin-2-yl]-propionique,
acide 2-[4-[[5-(cyclopropylméthoxy)-6-(trifluorométhyl)-1H-benzimidazol-2-yl]méthyl]-1-oxo-phthalazin-2-yl]acétique,
acide 2-[4-[[5-(cyclopropylméthoxy)-6-fluoro-1H-benzimidazol-2-yl]méthyl]-1-oxo-phthalazin-2-yl]acétique,
acide {4-[5-Chloro-6-(2,2-difluoro-éthoxy)-1H-benzoimidazol-2-ylméthyl]-1-oxo-1H-isoquinolin-2-yl}-acétique,
acide 2-[4-[[6-chloro-5-(2,2-difluoroéthoxy)-1H-benzimidazol-2-yl]méthyl]-5,8-difluoro-1-oxo-phthalazin-2-yl]acétique,
acide 2-[4-[[5-éthoxy-6-(trifluorométhyl)-1H-benzimidazol-2-yl]méthyl]-1-oxo-phthalazin-2-yl]acétique,
acide 2-[4-[(5-chloro-6-éthoxy-3-oxo-1H-indazol-2-yl)méthyl]-1-oxo-phthalazin-2-yl]acétique,

acide 2-[4-[(5-éthoxy-6-fluoro-1H-benzimidazol-2-yl)méthyl]-1-oxo-phthalazin-2-yl]acétique,
acide 2-[4-[[5-(2,2-difluoroéthoxy)-6-fluoro-1H-benzimidazol-2-yl]méthyl]-1-oxo-phthalazin-2-yl]acétique,
acide 2-[4-[[5-chloro-6-[(1-méthylcyclopropyl)méthoxy]-1H-benzimidazol-2-yl]méthyl]-1-oxo-phthalazin-2-yl]acétique,
acide 2-[4-[[5-chloro-6-(2,2-diméthylpropoxy)-1H-benzimidazol-2-yl]méthyl]-1-oxo-phthalazin-2-yl]acétique,
acide 2-[4-[[5-chloro-6-[(1-cyanocyclopropyl)méthoxy]-1H-benzimidazol-2-yl]méthyl]-1-oxo-phthalazin-2-yl]acétique,
acide 2-[4-[[5-chloro-6-(cyclopropylméthoxy)-3-oxo-1H-indazol-2-yl]méthyl]-1-oxo-phthalazin-2-yl]acétique,
acide 2-[4-[[6-cyano-5-(cyclopropylméthoxy)indazol-2-yl]méthyl]-1-oxo-phthalazin-2-yl]acétique,
acide 2-[4-[(6-chloro-5-méthoxy-1H-benzimidazol-2-yl)méthyl]-1-oxo-phthalazin-2-yl]acétique,
acide 2-[4-[(6-bromo-5-éthoxy-indazol-2-yl)méthyl]-1-oxo-phthalazin-2-yl]acétique,
acide 2-[4-[(5-cyano-6-éthoxy-1H-benzimidazol-2-yl)méthyl]-1-oxo-phthalazin-2-yl]acétique,
acide 2-[4-[[5-cyano-6-(2,2-difluoroéthoxy)-1H-benzimidazol-2-yl]méthyl]-1-oxo-phthalazin-2-yl]acétique,
2-[4-[[6-chloro-5-(trifluorométhoxy)-1H-benzimidazol-2-yl]méthyl]-1-oxo-phthalazin-2-yl]-N-[(1S,2R)-2-hydroxycyclopentyl]acétamide,
2-[4-[[6-chloro-5-(trifluorométhoxy)-1H-benzimidazol-2-yl]méthyl]-1-oxo-phthalazin-2-yl]-N-(1,1-dioxothian-4-yl)acétamide,
4-[[6-chloro-5-(trifluorométhoxy)-1H-benzimidazol-2-yl]méthyl]-2-(2-morpholino-2-oxo-éthyl)phthalazin-1-one,
2-[4-[[6-chloro-5-(cyclopropylméthoxy)indazol-2-yl]méthyl]-1-oxo-phthalazin-2-yl]-N-[(1S,2S)-2-hydroxycyclopentyl]acétamide,
N-tert-butyl-2-[4-[[6-chloro-5-(cyclopropylméthoxy)indazol-2-yl]méthyl]-1-oxo-phthalazin-2-yl]acétamide,
4-[[5-chloro-6-(trifluorométhoxy)-1H-benzimidazol-2-yl]méthyl]-2-(2-morpholino-2-oxo-éthyl)-5,6,7,8-tétrahydrophthalazin-1-one,
2-[4-[[5-chloro-6-(trifluorométhoxy)-1H-benzimidazol-2-yl]méthyl]-1-oxo-5,6,7,8-tétrahydrophthalazin-2-yl]-N-[(1S,2S)-2-hydroxycyclopentyl]acétamide,
2-[4-[[5-chloro-6-(trifluorométhoxy)-1H-benzimidazol-2-yl]méthyl]-1-oxo-5,6,7,8-tétrahydrophthalazin-2-yl]-N-[(1S,2S)-2-hydroxycyclopentyl]acétamide,
4-[[6-chloro-5-(cyclopropylméthoxy)indazol-2-yl]méthyl]-2-(2-morpholino-2-oxo-éthyl)phthalazin-1-one,
4-[[5-chloro-6-(cyclopropylméthoxy)-7-fluoro-1H-benzimidazol-2-yl]méthyl]-2-(2-morpholino-2-oxo-éthyl)phthalazin-1-one,
2-[4-[[5-chloro-6-(cyclopropylméthoxy)-1H-benzimidazol-2-yl]méthyl]-1-oxo-phthalazin-2-yl]-N-cyclopentyl-acétamide,
4-[[5-chloro-6-(2,2-difluoroéthoxy)-1H-benzimidazol-2-yl]méthyl]-2-(2-morpholino-2-oxo-éthyl)phthalazin-1-one,
4-[[6-chloro-5-(2,2-difluoroéthoxy)-1H-benzimidazol-2-yl]méthyl]-5,8-difluoro-2-(2-morpholino-2-oxo-éthyl)phthalazin-1-one,
4-[[6-chloro-5-(2,2-difluoroéthoxy)-1H-benzimidazol-2-yl]méthyl]-2-(2-morpholino-2-oxo-éthyl)-1-oxo-phthalazine-6-carbonitrile,
4-[(5-chloro-6-éthoxy-3-oxo-1H-indazol-2-yl)méthyl]-2-(2-morpholino-2-oxo-éthyl)phthalazin-1-one,
4-(6-Chloro-5-cyclopropylméthoxy-1H-benzoimidazol-2-ylméthyl)-2-(1-méthyl-2-morpholin-4-yl-2-oxo-éthyl)-2H-phthalazin-1-one,
4-[[5-(2,2-difluoroéthoxy)-6-fluoro-1H-benzimidazol-2-yl]méthyl]-2-(2-morpholino-2-oxo-éthyl)phthalazin-1-one,
4-[(5-éthoxy-6-fluoro-1H-benzimidazol-2-yl)méthyl]-2-(2-morpholino-2-oxo-éthyl)phthalazin-1-one,
6-(cyclopropylméthoxy)-2-[[3-(2-morpholino-2-oxo-éthyl)-4-oxo-phthalazin-1-yl]méthyl]-3H-benzimidazole-5-carbonitrile,
1-[6-Chloro-5-(2,2-difluoro-éthoxy)-1H-benzoimidazol-2-ylméthyl]-3-(2-morpholin-4-yl-2-oxo-éthyl)-1H-cinnolin-4-one,
4-[[5-chloro-6-(2,2-diméthylpropoxy)-1H-benzimidazol-2-yl]méthyl]-2-(2-morpholino-2-oxo-éthyl)phthalazin-1-one,
1-[[6-chloro-2-[[3-(2-morpholino-2-oxo-éthyl)-4-oxo-phthalazin-1-yl]méthyl]-3H-benzimidazol-5-yl]oxyméthyl]cyclopropanecarbonitrile,
1-[[6-chloro-2-[[3-[2-[(2S,6R)-2,6-diméthylmorpholin-4-yl]-2-oxo-éthyl]-4-oxo-phthalazin-1-yl]méthyl]-3H-benzimidazol-5-yl]oxyméthyl]cyclopropanecarbonitrile,
4-[[5-chloro-6-[(1-méthylcyclopropyl)méthoxy]-1H-benzimidazol-2-yl]méthyl]-2-(2-morpholino-2-oxo-éthyl)phthalazin-1-one,
6-(2,2-difluoroéthoxy)-2-[[3-(2-morpholino-2-oxo-éthyl)-4-oxo-5,6,7,8-tétrahydrophthalazin-1-yl]méthyl]-1H-benzimidazol-5-carbonitrile,

4-[(5-chloro-6-éthoxy-1H-benzimidazol-2-yl)méthyl]-2-(2-morpholino-2-oxo-éthyl)phthalazin-1-one,
4-[[5-chloro-6-(cyclopropylméthoxy)-1H-benzimidazol-2-yl]méthyl]-2-(2-morpholino-2-oxo-éthyl)phthalazin-1-one,
6-éthoxy-2-[[3-(2-morpholino-2-oxo-éthyl)-4-oxo-5,6,7,8-tétrahydrophthalazin-1-yl]méthyl]-1H-benzimidazol-5-carbonitrile,
4-[[6-éthoxy-5-(trifluorométhyl)-1H-benzimidazol-2-yl]méthyl]-2-(2-morpholino-2-oxo-éthyl)phthalazin-1-one,
4-[6-Chloro-5-(2,2-difluoro-éthoxy)-1H-benzimidazol-2-ylméthyl]-2-(2-morpholin-4-yl-2-oxo-éthyl)-2H-isoquinolin-1-one,
6-éthoxy-2-[[3-(2-morpholino-2-oxo-éthyl)-4-oxo-phthalazin-1-yl]méthyl]-1H-benzimidazol-5-carbonitrile,
6-(2,2-difluoroéthoxy)-2-[[3-(2-morpholino-2-oxo-éthyl)-4-oxo-phthalazin-1-yl]méthyl]-1H-benzimidazol-5-carbonitrile,
4-[[5-chloro-6-(2,2-difluoropropoxy)-1H-benzimidazol-2-yl]méthyl]-2-(2-morpholino-2-oxo-éthyl)phthalazin-1-one,
4-[(5-chloro-6-isobutoxy-1H-benzimidazol-2-yl)méthyl]-2-(2-morpholino-2-oxo-éthyl)phthalazin-1-one,
4-[[5-chloro-6-(2-méthoxyéthoxy)-1H-benzimidazol-2-yl]méthyl]-2-(2-morpholino-2-oxo-éthyl)phthalazin-1-one,
2-[[3-(2-morpholino-2-oxo-éthyl)-4-oxo-phthalazin-1-yl]méthyl]-6-(2,2,2-trifluoroéthoxy)-3H-benzimidazol-5-carbonitrile,
6-(2,2-difluoroéthoxy)-2-[[3-[2-[3-(diméthylamino)pyrrolidin-1-yl]-2-oxo-éthyl]-4-oxo-phthalazin-1-yl]méthyl]-3H-benzimidazol-5-carbonitrile,
6-(2,2-difluoroéthoxy)-2-[[3-[2-[(2S,6R)-2,6-diméthylmorpholin-4-yl]-2-oxo-éthyl]-4-oxo-phthalazin-1-yl]méthyl]-3H-benzimidazole-5-carbonitrile,
2-[4-[[6-cyano-5-(2,2-difluoroéthoxy)-1H-benzimidazol-2-yl]méthyl]-1-oxo-phthalazin-2-yl]-N-THF-3-yl-acétamide,
4-[[5-(2,2-difluoroéthoxy)-6-fluoro-1H-benzimidazol-2-yl]méthyl]-2-(2-morpholino-2-oxo-éthyl)-5,6,7,8-tétrahydrophthalazin-1-one,
2-[4-[[6-cyano-5-(2,2-difluoroéthoxy)-1H-benzimidazol-2-yl]méthyl]-1-oxo-phthalazin-2-yl]-N-méthyl-N-(THF-2-ylméthyl)acétamide,
2-[4-[[6-cyano-5-(2,2-difluoroéthoxy)-1H-benzimidazol-2-yl]méthyl]-1-oxo-phthalazin-2-yl]-N-(2-cyanoéthyl)-N-cyclopropyl-acétamide,
2-[4-[[6-cyano-5-(2,2-difluoroéthoxy)-1H-benzimidazol-2-yl]méthyl]-1-oxo-phthalazin-2-yl]-N-(2-méthoxy-2-méthyl-propyl)acétamide,
2-[4-[[6-cyano-5-(2,2-difluoroéthoxy)-1H-benzimidazol-2-yl]méthyl]-1-oxo-phthalazin-2-yl]-N-(2-méthoxyéthyl)-N-méthyl-acétamide,
2-[4-[[6-cyano-5-(2,2-difluoroéthoxy)-1H-benzimidazol-2-yl]méthyl]-1-oxo-phthalazin-2-yl]-N-tétrahydropyran-3-yl-acétamide,
6-(2,2-difluoroéthoxy)-2-[[3-[2-(4,4-difluoro-1-pipéridyl)-2-oxo-éthyl]-4-oxo-5,6,7,8-tétrahydrophthalazin-1-yl]méthyl]-1H-benzimidazole-5-carbonitrile,
6-(2,2-difluoroéthoxy)-2-[[3-[2-(3-méthoxypyrrolidin-1-yl)-2-oxo-éthyl]-4-oxo-phthalazin-1-yl]méthyl]-1H-benzimidazol-5-carbonitrile,
2-[4-[[5-cyano-6-(2,2-difluoroéthoxy)-1H-benzimidazol-2-yl]méthyl]-1-oxo-phthalazin-2-yl]-N-(2-méthoxyéthyl)acétamide,
6-(2,2-difluoroéthoxy)-2-[[3-[2-(4,4-difluoro-1-pipéridyl)-2-oxo-éthyl]-4-oxo-phthalazin-1-yl]méthyl]-1H-benzimidazol-5-carbonitrile,
6-(2,2-difluoroéthoxy)-2-[[3-[2-(6-oxa-9-azaspiro[3.5]nonan-9-yl)-2-oxo-éthyl]-4-oxo-phthalazin-1-yl]méthyl]-1H-benzimidazol-5-carbonitrile,
2-[4-[[5-cyano-6-(2,2-difluoroéthoxy)-1H-benzimidazol-2-yl]méthyl]-1-oxo-phthalazin-2-yl]-N-(3,3,3-trifluoro-2-hydroxy-2-méthyl-propyl)acétamide,
2-[4-[[5-cyano-6-(2,2-difluoroéthoxy)-1H-benzimidazol-2-yl]méthyl]-1-oxo-phthalazin-2-yl]-N-[(2S)-3,3,3-trifluoro-2-hydroxy-propyl]acétamide,
2-[4-[[5-cyano-6-(2,2-difluoroéthoxy)-1H-benzimidazol-2-yl]méthyl]-1-oxo-phthalazin-2-yl]-N-(2-hydroxy-3-méthoxy-propyl)acétamide,
6-(2,2-difluoroéthoxy)-2-[[3-[2-(4-hydroxy-1-pipéridyl)-2-oxo-éthyl]-4-oxo-phthalazin-1-yl]méthyl]-1H-benzimidazol-5-carbonitrile,
2-[4-[[5-cyano-6-(2,2-difluoroéthoxy)-1H-benzimidazol-2-yl]méthyl]-1-oxo-phthalazin-2-yl]-N-(2,2-difluoroéthyl)-N-(2-hydroxyéthyl)acétamide,
2-[4-[[5-cyano-6-(2,2-difluoroéthoxy)-1H-benzimidazol-2-yl]méthyl]-1-oxo-phthalazin-2-yl]-N-(2-cyanopropyl)-N-méthyl-acétamide,

2-[4-[[5-cyano-6-(2,2-difluoroéthoxy)-1H-benzimidazol-2-yl]méthyl]-1-oxo-phthalazin-2-yl]-N-(cyclopropylméthyl)-N-méthyl-acétamide,
2-[4-[[5-cyano-6-(2,2-difluoroéthoxy)-1H-benzimidazol-2-yl]méthyl]-1-oxo-phthalazin-2-yl]-N-(2,2-difluoroéthyl)-N-méthyl-acétamide,
6-(2,2-difluoroéthoxy)-2-[[3-[2-[(2S)-2-(hydroxyméthyl)pyrrolidin-1-yl]-2-oxo-éthyl]-4-oxo-phthalazin-1-yl]méthyl]-1H-benzimidazol-5-carbonitrile,
2-[4-[[5-cyano-6-(2,2-difluoroéthoxy)-1H-benzimidazol-2-yl]méthyl]-1-oxo-phthalazin-2-yl]-N-[[3-(hydroxyméthyl)oxétan-3-yl]méthyl]acétamide,
2-[[3-(2-morpholino-2-oxo-éthyl)-4-oxo-5,6,7,8-tétrahydrophthalazin-1-yl]méthyl]-6-(2,2,2-trifluoroéthoxy)-1H-benzimidazol-5-carbonitrile,
6-(2,2-difluoroéthoxy)-2-[[3-[2-(3-hydroxy-3-méthyl-pyrrolidin-1-yl)-2-oxo-éthyl]-4-oxo-phthalazin-1-yl]méthyl]-3H-benzimidazol-5-carbonitrile,
2-[4-[[6-cyano-5-(2,2-difluoroéthoxy)-1H-benzimidazol-2-yl]méthyl]-1-oxo-phthalazin-2-yl]-N-(2,2-diméthylpropyl)acétamide,
N-tert-butyl-2-[4-[[6-cyano-5-(2,2-difluoroéthoxy)-1H-benzimidazol-2-yl]méthyl]-1-oxo-5,6,7,8-tétrahydrophthalazin-2-yl]acétamide,
6-(2,2-difluoroéthoxy)-2-[[3-[2-[3-(diméthylamino)pyrrolidin-1-yl]-2-oxo-éthyl]-4-oxo-5,6,7,8-tétrahydrophthalazin-1-yl]méthyl]-1H-benzimidazol-5-carbonitrile,
5-(2,2-difluoroéthoxy)-2-[[3-(2-morpholino-2-oxo-éthyl)-4-oxo-phthalazin-1-yl]méthyl]indazol-6-carbonitrile,
5-(2,2-difluoroéthoxy)-2-[[3-[2-[(3R)-3-(diméthylamino)pyrrolidin-1-yl]-2-oxo-éthyl]-4-oxo-phthalazin-1-yl]méthyl]indazol-6-carbonitrile,
5-(2,2-difluoroéthoxy)-2-[[3-[2-[(3S)-3-(diméthylamino)pyrrolidin-1-yl]-2-oxo-éthyl]-4-oxo-phthalazin-1-yl]méthyl]indazol-6-carbonitrile,
2-[4-[[6-cyano-5-(2,2-difluoroéthoxy)indazol-2-yl]méthyl]-1-oxo-phthalazin-2-yl]-N-cyclopentyl-acétamide,
5-(cyclopropylméthoxy)-2-[[3-(2-morpholino-2-oxo-éthyl)-4-oxo-phthalazin-1-yl]méthyl]indazol-6-carbonitrile,
6-(cyclopropylméthoxy)-2-[[3-[2-(3-hydroxy-3-méthyl-pyrrolidin-1-yl)-2-oxo-éthyl]-4-oxo-phthalazin-1-yl]méthyl]imidazo[1,2-a]pyridin-7-carbonitrile,
6-(2,2-difluoroéthoxy)-2-[[3-[2-[(3S,4S)-3,4-dihydroxypyrrolidin-1-yl]-2-oxo-éthyl]-4-oxo-phthalazin-1-yl]méthyl]-3H-benzimidazol-5-carbonitrile,
6-(2,2-difluoroéthoxy)-2-[[3-[2-(3,3-difluoro-4-hydroxy-1-pipéridyl)-2-oxo-éthyl]-4-oxo-phthalazin-1-yl]méthyl]-3H-benzimidazol-5-carbonitrile,
2-[[3-[2-(4-cyclopropyl-4-hydroxy-1-pipéridyl)-2-oxo-éthyl]-4-oxo-phthalazin-1-yl]méthyl]-6-(2,2-difluoroéthoxy)-3H-benzimidazol-5-carbonitrile,
2-[[3-[2-(3-cyclopropyl-3-hydroxy-pyrrolidin-1-yl)-2-oxo-éthyl],
-4-oxo-phthalazin-1-yl]méthyl]-6-(2,2-difluoroéthoxy)-3H-benzimidazol-5-carbonitrile,
2-[[3-[2-(3-cyclopropyl-3-hydroxy-azétidin-1-yl)-2-oxo-éthyl]-4-oxo-phthalazin-1-yl]méthyl]-6-(2,2-difluoroéthoxy)-3H-benzimidazol-5-carbonitrile,
6-(2,2-difluoroéthoxy)-2-[[3-[2-[3-hydroxy-3-(trifluorométhyl)pyrrolidin-1-yl]-2-oxo-éthyl]-4-oxo-phthalazin-1-yl]méthyl]-3H-benzimidazol-5-carbonitrile,
1-[[6-bromo-5-(cyclopropylméthoxy)-1H-benzimidazol-2-yl]méthyl]-3-[2-[(3S,4S)-3,4-dihydroxypyrrolidin-1-yl]-2-oxo-éthyl]cinnolin-4-one,
2-[[3-[2-[4-(cyclopropylméthyl)pipérazin-1-yl]-2-oxo-éthyl]-4-oxo-phthalazin-1-yl]méthyl]-6-(2,2-difluoroéthoxy)-3H-benzimidazol-5-carbonitrile,
2-[[3-[2-(4-cyano-1-pipéridyl)-2-oxo-éthyl]-4-oxo-phthalazin-1-yl]méthyl]-6-(2,2-difluoroéthoxy)-3H-benzimidazol-5-carbonitrile,
2-[4-[[6-cyano-5-(2,2-difluoroéthoxy)-1H-benzimidazol-2-yl]méthyl]-1-oxo-phthalazin-2-yl]-N-méthyl-N-(2-méthylsulfonyléthyl)acétamide,
6-(2,2-difluoroéthoxy)-2-[[3-[2-(3-méthylsulfonylpyrrolidin-1-yl)-2-oxo-éthyl]-4-oxo-phthalazin-1-yl]méthyl]-3H-benzimidazol-5-carbonitrile,
2-[4-[[6-cyano-5-(2,2-difluoroéthoxy)-1H-benzimidazol-2-yl]méthyl]-1-oxo-phthalazin-2-yl]-N-(2-hydroxy-3,3-diméthyl-butyl)acétamide,
2-[4-[[6-cyano-5-(2,2-difluoroéthoxy)-1H-benzimidazol-2-yl]méthyl]-1-oxo-phthalazin-2-yl]-N-(2,2-difluoroéthyl)acétamide,
2-[4-[[6-cyano-5-(2,2-difluoroéthoxy)-1H-benzimidazol-2-yl]méthyl]-1-oxo-phthalazin-2-yl]-N-(3,3,3-trifluoropropyl)acétamide,
6-(2,2-difluoroéthoxy)-2-[[3-[2-[4-(2-hydroxyéthyl)pipérazin-1-yl]-2-oxo-éthyl]-4-oxo-phthalazin-1-yl]méthyl]-3H-benzimidazol-5-carbonitrile,
N-[cyano(cyclopropyl)méthyl]-2-[4-[[6-cyano-5-(2,2-difluoroéthoxy)-1H-benzimidazol-2-yl]méthyl]-1-oxo-phthalazin-2-yl]acétamide,
6-(2,2-difluoroéthoxy)-2-[[3-[2-(2,2-diméthylmorpholin-4-yl)-2-oxo-éthyl]-4-oxo-phthalazin-1-yl]méthyl]-3H-benzimidazol-5-carbonitrile,
2-[4-[[6-cyano-5-(2,2-difluoroéthoxy)-1H-benzimidazol-2-yl]méthyl]-1-oxo-phthalazin-2-yl]-N-(2,2-difluoro-3-hydroxy-propyl)acétamide,
2-[4-[[6-cyano-5-(2,2-difluoroéthoxy)-1H-benzimidazol-2-yl]méthyl]-1-oxo-phthalazin-2-yl]-N-(2-sulfamoyléthyl)acétamide,
2-[4-[[6-cyano-5-(2,2-difluoroéthoxy)-1H-benzimidazol-2-yl]méthyl]-1-oxo-phthalazin-2-yl]-N-(2,2,2-trifluoroéthyl)acétamide,
6-(2,2-difluoroéthoxy)-2-[[3-[2-(3-hydroxy-1-pipéridyl)-2-oxo-éthyl]-4-oxo-phthalazin-1-yl]méthyl]-3H-benzimidazol-5-carbonitrile,
2-[4-[[6-cyano-5-(2,2-difluoroéthoxy)-1H-benzimidazol-2-yl]méthyl]-1-oxo-phthalazin-2-yl]-N-[(3,3-difluorocyclobutyl)méthyl]acétamide,
6-(2,2-difluoroéthoxy)-2-[[3-[2-(3,3-diméthylpyrrolidin-1-yl)-2-oxo-éthyl]-4-oxo-phthalazin-1-yl]méthyl]-3H-benzimidazol-5-carbonitrile,
6-(2,2-difluoroéthoxy)-2-[[3-[2-(4,4-diméthyl-1-piperidyl)-2-oxo-éthyl]-4-oxo-phthalazin-1-yl]méthyl]-3H-benzimidazol-5-carbonitrile,
2-[[3-[2-(2,2-difluoro-5-azaspiro[2.4]heptan-5-yl)-2-oxo-éthyl]-4-oxo-phthalazin-1-yl]méthyl]-6-(2,2-difluoroéthoxy)-3H-benzimidazol-5-carbonitrile,
1-[2-[4-[[6-cyano-5-(2,2-difluoroethoxy)-1H-benzimidazol-2-yl]méthyl]-1-oxo-phthalazin-2-yl]acétyl]-N,N-diméthyl-piperidin-4-carboxamide,
6-(2,2-difluoroéthoxy)-2-[[3-[2-[3-(1-hydroxy-1-méthyl-éthyl)-1-pipéridyl]-2-oxo-éthyl]-4-oxo-phthalazin-1-yl]méthyl]-3H-benzimidazol-5-carbonitrile,
6-(2,2-difluoroéthoxy)-2-[[3-[2-(4-morpholino-1-pipéridyl)-2-oxo-éthyl]-4-oxo-phthalazin-1-yl]méthyl]-3H-benzimidazol-5-carbonitrile,
6-(2,2-difluoroéthoxy)-2-[[3-[2-(7-oxa-2-azaspiro[3.5]nonan-2-yl)-2-oxo-éthyl]-4-oxo-phthalazin-1-yl]méthyl]-3H-benzimidazol-5-carbonitrile,
6-(2,2-difluoroéthoxy)-2-[[3-[2-(4-éthylsulfonyl-1-pipéridyl)-2-oxo-éthyl]-4-oxo-phthalazin-1-yl]méthyl]-3H-benzimidazol-5-carbonitrile,
2-[4-[[6-cyano-5-(2,2-difluoroéthoxy)-1H-benzimidazol-2-yl]méthyl]-1-oxo-phthalazin-2-yl]-N-[2-(diméthylamino)-2-oxo-éthyl]-N-méthyl-acétamide,
2-[4-[[6-chloro-5-(cyclopropylméthoxy)-1H-benzimidazol-2-yl]méthyl]-1-oxo-phthalazin-2-yl]-N-méthylsulfonyl-acétamide,
2-[4-[[6-chloro-5-(cyclopropylméthoxy)indazol-2-yl]méthyl]-1-oxo-phthalazin-2-yl]-N-méthylsulfonyl-acétamide,
2-[4-[[6-chloro-5-(cyclopropylméthoxy)indazol-2-yl]méthyl]-1-oxo-phthalazin-2-yl]-N-cyclopropylsulfonyl-acétamide,
2-[4-[[6-chloro-5-(2,2-difluoroéthoxy)-1H-benzimidazol-2-yl]méthyl]-6-cyano-1-oxo-phthalazin-2-yl]-N-méthylsulfonyl-acétamide,
N-{2-[4-(6-Chloro-5-cyclopropylméthoxy-1H-benzimidazol-2-ylméthyl)-1-oxo-1H-phthalazin-2-yl]-propionyl} -méthanesulfonamide,
2-[4-[(5-éthoxy-6-fluoro-1H-benzimidazol-2-yl)méthyl]-1-oxo-phthalazin-2-yl]-N-méthylsulfonyl-acétamide,
2-[4-[[5-(2,2-difluoroéthoxy)-6-fluoro-1H-benzimidazol-2-yl]méthyl]-1-oxo-phthalazin-2-yl]-N-méthylsulfonyl-acétamide,
2-[4-[[6-chloro-5-[(1-cyanocyclopropyl)méthoxy]-1H-benzimidazol-2-yl]méthyl]-1-oxo-phthalazin-2-yl]-N-méthylsulfonyl-acétamide,
2-[4-[[6-chloro-5-[(1-méthylcyclopropyl)méthoxy]-1H-benzimidazol-2-yl]méthyl]-1-oxo-phthalazin-2-yl]-N-méthylsulfonyl-acétamide,
acide 2-[4-[(6-chloro-5-éthoxy-indazol-2-yl)méthyl]-1-oxo-phthalazin-2-yl]acétique,
acide 2-[4-[[6-chloro-5-(2,2,2-trifluoroéthoxy)indazol-2-yl]méthyl]-1-oxo-phthalazin-2-yl]acétique,
acide 2-[4-[[6-chloro-5-(2,2-difluoroéthoxy)indazol-2-yl]méthyl]-l-oxo-phthalazin-2-yl]acétique,
acide 2-[4-[(4-chloro-5-éthoxy-indazol-2-yl)méthyl]-1-oxo-phthalazin-2-yl]acétique,
acide 2-[4-[(5-chloro-6-éthoxy-indazol-2-yl)méthyl]-1-oxo-phthalazin-2-yl]acétique,
6-(2,2-difluoroéthoxy)-2-[[3-(2-morpholino-2-oxo-éthyl)-4-oxo-phthalazin-l-yl]méthyl]imidazo[1,2-a]pyridin-7-carbonitrile, et
acide 2-[4-[[6-chloro-5-(cyclopropylméthoxy)-1H-imidazo[4,5-b]pyridin-2-yl]méthyl]-1-oxo-phthalazin-2-yl]acétique.

**12.** Composition pharmaceutique comprenant un composé ou un sel pharmaceutiquement acceptable de celui-ci selon l'une quelconque des revendications 1-11, et un vecteur pharmaceutiquement acceptable.

**13.** Composition pharmaceutique selon la revendication 12 comprenant un autre agent thérapeutique.

**14.** Composé ou sel pharmaceutiquement acceptable de celui-ci, selon l'une quelconque des revendications 1-11, ou composition pharmaceutique selon la revendication 12 ou 13 destiné(e) à être utilisé(e) dans un médicament.

**15.** Composé ou sel pharmaceutiquement acceptable de celui-ci, selon l'une quelconque des revendications 1-11, ou composition pharmaceutique selon la revendication 12 ou 13 destiné(e) à être utilisé(e) en prophylaxie et/ou dans le traitement de maladies fibrotiques, maladies inflammatoires, maladies respiratoires, maladies auto-immunes, maladies métaboliques, maladies cardiovasculaires et/ou maladies prolifératives.

**16.** Composition pharmaceutique selon la revendication 13, dans lequel l'autre agent thérapeutique est un agent pour la prophylaxie et/ou le traitement de maladies fibrotiques, maladies inflammatoires, maladies respiratoires, maladies auto-immunes, maladies métaboliques, maladies cardiovasculaires et/ou maladies prolifératives.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014187550 A
- WO 2014139882 A
- CH 03605877535982


### Non-patent literature cited in the description


- Remington's Pharmaceutical Sciences. Mack Publishing Company, 1985
- *CHEMICAL ABSTRACTS,* 9041-93-4
- **ADADA, M. ; CANALS, D. ; HANNUN, Y.A. ; OBEID, L.M.** Sphingosine-1-phosphate receptor 2. *FEBS J.,* 2013, vol. 280, 6354-6366, https://doi.org/10.1111/febs.12446
- **ASHCROFT, T. ; SIMPSON, J.M. ; TIMBRELL, V.** Simple method of estimating severity of pulmonary fibrosis on a numerical scale. *J. Clin. Pathol.,* 1988, vol. 41, 467-470
- **BICKELHAUPT, S. ; ERBEL, C. ; TIMKE, C. ; WIRKNER, U. ; DADRICH, M. ; FLECHSIG, P. ; TIETZ, A. ; PFÖHLER, J. ; GROSS, W. ; PESCHKE, P.** Effects of CTGF Blockade on Attenuation and Reversal of Radiation-Induced Pulmonary Fibrosis. *JNCI J. Natl. Cancer Inst.,* 2017, vol. 109, https://doi.org/10.1093/jnci/djw339
- **BLANKENBACH, K.V. ; SCHWALM, S. ; PFEILSCHIFTER, J. ; MEYER ZU HERINGDORF, D.** Sphingosine-1-Phosphate Receptor-2 Antagonists: Therapeutic Potential and Potential Risks. *Front. Pharmacol.,* 2016, vol. 7, 167, https://doi.org/10.3389/fphar.2016.00167
- **BRUNNEMER, E. ; WÄLSCHER, J. ; TENENBAUM, S. ; HAUSMANNS, J. ; SCHULZE, K. ; SEITER, M. ; HEUSSEL, C.P. ; WARTH, A. ; HERTH, F.J.F. ; KREUTER, M.** Real-World Experience with Nintedanib in Patients with Idiopathic Pulmonary Fibrosis. *Respiration,* 2018, vol. 95, 301-309, https://doi.org/10.1159/000485933
- **CHEN, C.-W. ; BEYER, C. ; LIU, J. ; MAIER, C. ; LI, C. ; TRINH-MINH, T. ; XU, X. ; COLE, S.H. ; HSIEH, M.H. ; NG, N.** Pharmacological inhibition of porcupine induces regression of experimental skin fibrosis by targeting Wnt signalling. *Ann. Rheum. Dis.,* 2017, vol. 76, 773-778, https://doi.org/10.1136/annrheumdis-2016-210294
- **DEVOS, F.C. ; MAASKE, A. ; ROBICHAUD, A. ; POLLARIS, L. ; SEYS, S. ; LOPEZ, C.A. ; VERBEKEN, E. ; TENBUSCH, M. ; LORIES, R. ; NEMERY, B.** Forced expiration measurements in mouse models of obstructive and restrictive lung diseases. *Respir. Res.,* 2017, vol. 18, 123, https://doi.org/10.1186/s12931-017-0610-1
- **HOBSON, A.D. ; HARRIS, C.M. ; VAN DER KAM, E.L. ; TURNER, S.C. ; ABIBI, A. ; AGUIRRE, A.L. ; BOUSQUET, P. ; KEBEDE, T. ; KONOPACKI, D.B. ; GINTANT, G.** Discovery of A-971432, An Orally Bioavailable Selective Sphingosine-1-Phosphate Receptor 5 (S1P5) Agonist for the Potential Treatment of Neurodegenerative Disorders. *J. Med. Chem.,* 2015, vol. 58, 9154-9170, https://doi.org/10.1021/acs.jmedchem.5b00928
- **KITADA, Y. ; KAJITA, K. ; TAGUCHI, K. ; MORI, I. ; YAMAUCHI, M. ; IKEDA, T. ; KAWASHIMA, M. ; ASANO, M. ; KAJITA, T. ; ISHIZUKA, T.** Blockade of Sphingosine 1-Phosphate Receptor 2 Signaling Attenuates High-Fat Diet-Induced Adipocyte Hypertrophy and Systemic Glucose Intolerance in Mice. *Endocrinology,* 2016, vol. 157, 1839-1851, https://doi.org/10.1210/en.2015-1768
- **LANCASTER, L.H. ; ANDRADE, J.A. DE ; ZIBRAK, J.D. ; PADILLA, M.L. ; ALBERA, C. ; NATHAN, S.D. ; WIJSENBEEK, M.S. ; STAUFFER, J.L. ; KIRCHGAESSLER, K.-U. ; COSTABEL, U.** Pirfenidone safety and adverse event management in idiopathic pulmonary fibrosis. *Eur. Respir. Rev.,* 2017, vol. 26, 170057, https://doi.org/10.1183/16000617.0057-2017
- **MATSUSE, T. ; TERAMOTO, S. ; KATAYAMA, H. ; SUDO, E. ; EKIMOTO, H. ; MITSUHASHI, H. ; UEJIMA, Y. ; FUKUCHI, Y. ; OUCHI, Y.** ICAM-1 mediates lung leukocyte recruitment but not pulmonary fibrosis in a murine model of bleomycin-induced lung injury. *Eur. Respir. J.,* 1999, vol. 13, 71-77
- **MILSTIEN, S. ; SPIEGEL, S.** Targeting sphingosine-1-phosphate: A novel avenue for cancer therapeutics. *Cancer Cell,* 2006, vol. 9, 148-150, https://doi.org/10.1016/j.ccr.2006.02.025

- **NANTHAKUMAR, C.B. ; HATLEY, R.J.D. ; LEMMA, S. ; GAULDIE, J. ; MARSHALL, R.P. ; MACDONALD, S.J.F. ; 2015.** Dissecting fibrosis: therapeutic insights from the small-molecule toolbox. *Nat. Rev. Drug Discov.,* vol. 14, 693-720, https://doi.org/10.1038/nrd4592
- **RICHELDI, L. ; DU BOIS, R.M. ; RAGHU, G. ; AZUMA, A. ; BROWN, K.K. ; COSTABEL, U. ; COTTIN, V. ; FLAHERTY, K.R. ; HANSELL, D.M. ; INOUE, Y.** INPULSIS Trial Investigators, 2014. Efficacy and safety of nintedanib in idiopathic pulmonary fibrosis. *N. Engl. J. Med.,* vol. 370, 2071-2082, https://doi.org/10.1056/NEJMoa1402584
- **SOBEL, K. ; MENYHART, K. ; KILLER, N. ; RENAULT, B. ; BAUER, Y. ; STUDER, R. ; STEINER, B. ; BOLLI, M.H. ; NAYLER, O. ; GATFIELD, J.** Sphingosine 1-Phosphate (SIP) Receptor Agonists Mediate Pro-fibrotic Responses in Normal Human Lung Fibroblasts via S1P2 and S1P3 Receptors and Smad-independent Signaling. *J. Biol. Chem.,* 2013, vol. 288, 14839-14851, https://doi.org/10.1074/jbc.M112.426726
- **TAKABE, K. ; PAUGH, S.W. ; MILSTIEN, S. ; SPIEGEL, S.** Inside-Out'' Signaling of Sphingosine-1-Phosphate: Therapeutic Targets. *Pharmacol. Rev.,* 2008, vol. 60, 181-195, https://doi.org/10.1124/pr.107.07113
- **YUAN, S.Y. ; RIGOR, R.R.** Regulation of Endothelial Barrier Function, Integrated Systems Physiology: From Molecule to Function to Disease. *Morgan & Claypool Life Sciences,* 2010